(19) Europäisches Patentamt
European Patent Office
Office européen des brevets

(11) EP 4 406 948 A1

(12) # EUROPEAN PATENT APPLICATION
published in accordance with Art. 153(4) EPC

(43) Date of publication:
**31.07.2024 Bulletin 2024/31**

(21) Application number: **22872006.6**

(22) Date of filing: **21.09.2022**

(51) International Patent Classification (IPC):
***C07D 401/14** (2006.01)* ***C07D 239/72** (2006.01)*
***C07D 471/04** (2006.01)* ***C07D 403/14** (2006.01)*
***A61K 31/517** (2006.01)* ***A61P 35/00** (2006.01)*

(52) Cooperative Patent Classification (CPC):
**A61K 31/517; A61P 35/00; C07D 239/72; C07D 401/14; C07D 403/14; C07D 471/04**

(86) International application number:
**PCT/CN2022/120154**

(87) International publication number:
**WO 2023/045960 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.09.2021 CN 202111108176**
**25.01.2022 CN 202210089257**
**08.03.2022 CN 202210227315**

(71) Applicants:
- **Sichuan Huiyu Pharmaceutical Co., Ltd.
Neijiang, Sichuan 641000 (CN)**
- **Sichuan Huiyu Seacross Pharmaceutical Technology
Co., Ltd.
Chengdu, Sichuan 610200 (CN)**

(72) Inventors:
- **CHEN, Shoujun
Neijiang, Sichuan 641000 (CN)**
- **QIANG, Xiaoming
Neijiang, Sichuan 641000 (CN)**
- **DING, Zhao
Neijiang, Sichuan 641000 (CN)**
- **XIONG, Yong
Neijiang, Sichuan 641000 (CN)**
- **WANG, Haibo
Neijiang, Sichuan 641000 (CN)**
- **LIU, Ke
Neijiang, Sichuan 641000 (CN)**
- **LIU, Mingdeng
Neijiang, Sichuan 641000 (CN)**

(74) Representative: **Huang, Liwei
Cäcilienstraße 12
40597 Düsseldorf (DE)**

(54) **PYRIDINE DERIVATIVE AND USE THEREOF**

(57) A pyridine derivative and use thereof. The compound has an obvious inhibitory effect on the activity of KRAS and PI3K proteins, can be used as a KRAS and/or PI3K protein inhibitor, and can be used for preparing a drug for treatment of diseases such as KRAS or PI3K protein-mediated cancer. The compound has an extremely strong inhibitory effect on a KRAS G12C inhibitor (e.g. AMG-510) drug-resistant cancer cells , and has a wide application prospect thereof.

EP 4 406 948 A1

## Description

## Technical Field

The present disclosure relates to the field of medical technology, and in particular to a compound used as a KRAS G12C inhibitor and/or a PI3K protein inhibitor and its uses.

## Background

RAS protein is an important signaling molecule that participates in various processes such as cell proliferation, differentiation, survival and movement. The RAS family consists of KRAS, NRAS, and HRAS. They have two states in cells: an inactive state bound to GDP and an activated state bound to GTP. When RAS is activated, it can activate multiple downstream signaling pathways, including the MAPK signaling pathway, PI3K signaling pathway, and Ral-GEFs signaling pathway. These signaling pathways play an important role in promoting cell survival, proliferation, and cytokine release.

Activation of RAS relies on receptor phosphorylation after tyrosine kinase receptor binding to signaling molecules, exposing the binding site, Guanine Nucleotide Exchange Factor (GEF, such as SOS1) and growth factor receptor binding protein (Grb2) complex Binds to this site and binds to it through the SH2 domain of Grb2 to form an RTK/Grb2/SOS complex. SHP2 can further promote the binding of this complex to RAS, prompting it to release GDP and bind GTP, thereby activating RAS. In addition, it is worth noting that before activation, RAS needs to be bound to the inner side of the cell membrane to exert its physiological effects, in which farnesyl transferase plays a key role, and sphingomyelin synthase-1 may also be involved in regulating the localization of RAS. As the proto-oncogene with the highest mutation rate, the RAS family consists of KRAS, NRAS and HRAS respectively. 84% of patients have KRAS mutations, while only 4% and 12% have HRAS and NRAS mutations respectively. The KRAS (Kirsten Rat Sarcoma virus) gene is located on autosome 12, consists of 188-189 amino acids, and has a molecular weight of 21.7 kDa. RAS protein consists of 6 $\beta$-sheets and 5 $\alpha$-helices, of which the 166 amino acids at the N-terminus constitute the G domain, while the C-terminus is the membrane-binding region. The G domain (divided into G1-G5) is a functional domain that can directly bind to GDP/GTP. G2 (Switch I) binds to GTP, while G3 (Switch II) binds to GDP. KRAS is widely expressed in body tissues, and its mRNA levels can be detected in almost all tissues. KRAS functions in cells by participating in GTP hydrolysis. As a GTPase, it functions in catalyzing the conversion of guanine triphosphate (GTP) and guanine diphosphate (GDP), promoting Cell survival, migration and proliferation. In normal cells, KRAS mainly binds to GDP and is in an inactive state. However, Guanine Nucleotide Exchange Factor (GEF) such as SOS1 can promote the release of GDP and combine with GTP to become an activated state. The reverse transformation can be achieved through GTPase-activating protein (GAP), such as RasGAP. KRAS accounts for the majority of RAS mutations. KRAS mutations occur widely in cancer patients, including 5-30% of lung cancer patients, 36-40% of colon cancer patients, and about 90% of pancreatic cancer patients. In addition, KRAS mutations have also been found in patients with other tumors, such as endometrial cancer, skin cancer, and multiple myeloma.

The G12 mutations are the most common among KRAS mutations, accounting for 83%, followed by G13, which accounts for 14%, and Q61. G12V, G12D, and G12C are the most common among the G12 mutations,. G12C mutations account for 14% of patients with non-small cell lung cancer, 5% of colon cancer, and 2% of pancreatic ductal adenocarcinoma. Mutations in KRAS can promote its binding to GTP and remain in an activated state, continuously activating cell growth, leading to tumor occurrence. All these indicate that KRAS mutations have important value in clinical treatment. G12C mutation is a relatively common subtype of KRAS gene mutation, which refers to the mutation of glycine 12 to cysteine. KRAS G12C mutations are also the most common in lung cancer. According to data reported in the literature (Nat Rev Drug Discov 2014;13:828-851), KRAS G12C mutations account for about 10% of all lung cancer patients. For more than 30 years, there has been no breakthrough in the search for drugs that specifically target KRAS, so KRAS is generally considered an "Undruggable Target" protein target.

In recent years, the druggability of KRAS G12C has been discovered, and KRAS G12C inhibitors have become one of the current popular areas of drug research and development. The literature (Nature. 2013; 503: 548-551) reports a class of covalent binding inhibitors targeting the KRAS G12C mutation. However, these compounds have low enzymatic activity and do not show activity at the cellular level. Another type of compound reported in the literature (Science 2016; 351: 604-608, Cancer Discov 2016; 6: 316-29) shows $\mu$ M-level cellular anti-proliferative activity at the cellular level, but its metabolic stability is poor and its activity is difficult to improve further. Finding drugs that target RAS is very difficult. Because the binding ability of GTP and RAS is very strong, it is difficult to find small molecules that can competitively inhibit their binding; moreover, the surface of RAS protein is very smooth and lacks structural space for small molecules or drugs to bind. In 2013, the University of California discovered through protein crystallography research that a common mutant KRAS G12C protein among KRAS forms a new pocket on the surface of the molecule after binding to GDP. Small molecule inhibitors can cooperate with the KRAS G12C protein at this site. Valent binding locks the protein in an

inactive state. In recent years, AraxesPharma has applied for several patents targeting KRAS G12C inhibitors. For example, WO2016164675 and WO2016168540 reported a class of quinazoline derivatives with high enzyme-binding activity and demonstrated μM-level cell anti-proliferative activity. Its structure is stable and has certain selectivity. Amgen (WO2018119183) and AstraZeneca (WO2018206539A1) respectively disclosed patents on KRAS G12C inhibitors in 2018, and Amgen's KRASG12C inhibitor AMG-510 launched a phase I clinical study in July 2018.

Looking at the KRAS G12C inhibitors currently reported in the literature, they all have an acrylamide fragment, which acts as a Michael addition receptor and a cysteine residue on the KRAS G12C mutant protein to form a covalent binding complex. In 2018, LiuYi et al. published a report on Cell (Matthew, R. Janes, Yi Liu, et al., Cell, 2018, 172,578-589.), a covalent binding inhibitor ARS-1620 targeting the KRAS G12C mutation. The compound has good metabolic stability, exhibits nM-level cellular anti-proliferative activity at the cellular level, and can effectively inhibit tumor growth in the pancreatic cancer MIA-Paca2 cell subcutaneous xenograft tumor model. Currently, the KRAS G12C inhibitors under development that are progressing rapidly include Araxes' ARS-1620, Amgen's AMG-510, and Mirati's MRTX-849 (WO2020216190A1). Among them, AMG-510 is making the fastest progress and was approved for marketing in 2021. It started phase I clinical trials in 2018 and is the first KRAS G12C inhibitor to enter clinical trials.

However, resistance to KRAS inhibitors is a major problem in cancer treatment. Amgen's latest clinical trial results also show that some patients treated with the KRAS G12C inhibitor AMG-510 continued to progress after remission. Piro Lito et al. discovered rapid non-uniform adaptation to conformation-specific KRAS G12C inhibition. Because KRAS G12C cycles between active and inactive conformations, and inhibitors bind only to the latter, the researchers tested whether syngeneic cell populations responded in a non-uniform manner by studying the effects of treatment at single-cell resolution. The researchers found that shortly after treatment, some cancer cells were rendered quiescent and had lower KRAS activity, while others bypassed this effect to resume proliferation and develop drug resistance (XIE, J.Y; et al, Nature 2020, 577, 421-425). The latest research by Adachi and others shows that epithelial-mesenchymal transition (EMT) is the cause of intrinsic and acquired resistance to AMG510 (Adachi, Y, et al Clin Cancer Res 2020, Sep 8. doi: 10.1158/1078-0432.CCR-20 -2077. Online ahead of print.). Although there are other studies published on the resistance mechanism of AMG-510, there is still no good practical solution to the problem of resistance to KRAS inhibitors such as AMG-510 in clinical practice. Therefore, it is of great significance to develop new therapeutic agents that can overcome the drug resistance problem.

**Summary of the Invention**

The major technical problem solved by the present disclosure is to provide a pyridine derivative, which, as a selective inhibitor of KRAS mutation, has the advantages of high activity, good selectivity and low toxic and side effects. At the same time, it also has a good inhibitory effect on mutation sites that cause resistance to other KRAS inhibitors. In addition, it also shows a strong inhibitory effect on PI3K.

In order to solve the above technical problems, the present disclosure provides a compound of formula I'''' ,or a tautomer, meso isomer, racemate, enantiomer, diastereomer or mixture thereof, or a pharmaceutically acceptable hydrate, solvate or salt thereof:

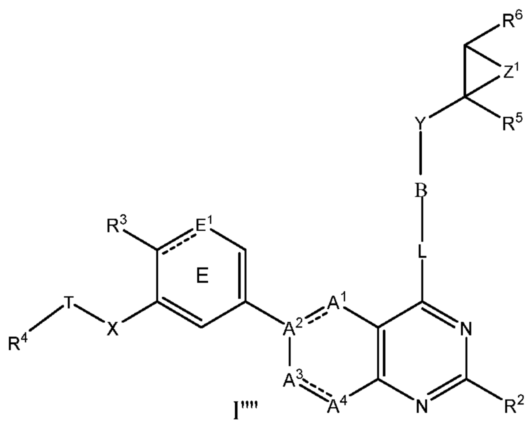

I''''

Wherein,

T is selected from $S(O)_2$, C=O, $CH_2$ or $NHS(O)_2$;

X is selected from $NR^1$, S or $S(O)_2$;

$A^1$ is selected from CH, $CH_2$, C=O or N, $A^2$ is selected from C, CH or N, $A^3$ is selected from $CR^8$, $CR^9R^{10}$ or N, $A^4$

is selected from $CR^{29}$, $CR^9R^{10}$ or N;
$Z^1$ is a bond or O;
E1 is $NR^{11}$, CH or $CH_2$;
B is

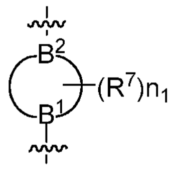

,

the ring where $B^1$, $B^2$ are located is a 5- to 10-membered nitrogen-containing heterocycloalkyl or a 5- to 10-membered nitrogen-containing heterocycloalkenyl, wherein the cycloalkyl or cycloalkenyl is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;
or, B is

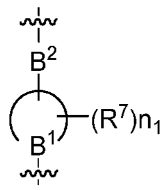

,

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;
or, B is

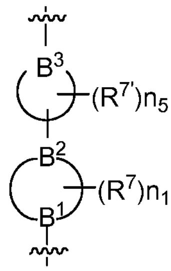

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13''}$ or N, and at least one of $B^1$ and $B^2$ is N; the ring where $B^3$ is located is a 4- to 10-membered nitrogen-containing heterocycloalkyl and is a monocyclic ring, spirocyclic ring or bridged cyclic ring, $B^3$ is selected from $CR^{12'}$ or N;
$B^1$ is connected to L;
L is selected from a bond or $NR^{15}$;
Y is selected from C=O or $S(O)_2$;
$R^5$, $R^6$ are independently selected from hydrogen, halogen, cyano, alkyl, aliphatic heterocyclyl, aryl, heteroaryl, -$OR^a$, -$NR^bR^c$, -$C(O)R^{16}$, -$S(O)R^{16}$, -$S(O)_2R^{16}$, -$P(O)R^{16}R^{17}$, -$C(O)NR^bR^c$ or -$C(O)OR^a$, wherein the alkyl, aliphatic heterocyclyl, aryl, heteroaryl are optionally substituted by one or more $R^{18}$;
each occurrence of $R^{18}$ is independently selected from halogen, cyano, alkyl, cycloalkyl, aliphatic heterocyclyl, -$OR^a$, -$NR^bR^c$, -$C(O)R^{16}$, -$C(O)NR^bR^c$ or -$C(O)OR^a$;
$R^1$, $R^{11}$, $R^{14}$, $R^{15}$ are independently selected from hydrogen, alkyl, cycloalkyl, aliphatic heterocyclyl or -$C(O)R^{19}$, wherein the alkyl, cycloalkyl, aliphatic heterocyclyl are optionally substituted by one or more substituents selected from the group consisting of: halogen, cyano, hydroxy, amino, alkyl, cycloalkyl and aliphatic heterocyclyl;
$n_1$, $n_5$ are independently selected from 0, 1, 2, 3 or 4;
each occurrence of $R^{12}$, $R^{12'}$, $R^{13}$, $R^{13'}$, $R^{13''}$, $R^7$, $R^{7'}$ is independently selected from hydrogen, halogen, cyano, alkyl, cycloalkyl, aliphatic heterocyclyl, aryl, heteroaryl, -$OR^n$, -$NR^oR^p$, -$C(O)R^{20}$, -$C(O)NR^oR^p$ or -$C(O)OR^n$, wherein the alkyl, cycloalkyl, aliphatic heterocyclyl, aryl, heteroaryl are optionally substituted by one or more $R^{21}$;

each occurrence of $R^{21}$ is independently selected from halogen, cyano, alkyl, cycloalkyl, aliphatic heterocyclyl, -$OR^n$, -$NR^oR^p$, -C(O)$R^{20}$, -C(O)$NR^oR^p$ or -C(O)$OR^n$;

or $Z^1$ is a bond, $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicyclyl, or $R^6$, $R^{14}$ together with the fragment to which $R^6$, $R^{14}$ are attached form a 4- to 9-membered aliphatic heterocyclyl, or $R^5$, $R^6$ together with the atoms to which $R^5$, $R^6$ are attached form a 6- to 10-membered aryl or 5- to 10-membered heteroaryl, wherein the cycloalkenyl, alicyclic heterocyclyl, alicyclyl, aryl and heteroaryl are optionally substituted by one or more substituents selected from the group consisting of: halogen, cyano, hydroxy, amino, alkyl, cycloalkyl and alicyclic heterocyclyl;

$R^2$ is selected from hydrogen, halogen, cyano, alkyl, cycloalkyl, alicyclic heterocyclyl, aryl, heteroaryl, -$OR^d$, - $NR^eR^f$, -C(O)$R^{22}$, -C(O)$R^eR^f$ or -C(O)$OR^d$, wherein the alkyl, cycloalkyl, alicyclic heterocyclyl, aryl, heteroaryl are optionally substituted by one or more $R^{23}$;

each occurrence of $R^{23}$ is independently selected from halogen, cyano, alkyl, cycloalkyl, aliphatic heterocyclyl, -$OR^d$, -$NR^eR^f$, -C(O)$R^{22}$, -C(O)$NR^eR^f$ or -C(O)$OR^d$;

$R^3$ is selected from hydrogen, halogen, cyano, alkyl, cycloalkyl, aliphatic heterocyclyl, aryl, heteroaryl, -$OR^g$, -$SR^g$, -$NR^hR^i$, -C(O)$R^{24}$, -C(O)$R^hR^i$ or -C(O)$OR^g$, when the ' -----' in ring E is absent, $R^3$ can also be a carbonyl group that together formed by the connected carbon atom, wherein the alkyl, cycloalkyl, aliphatic heterocyclyl, aryl, heteroaryl are optionally substituted by one or more $R^{25}$;

each occurrence of $R^{25}$ is independently selected from halogen, cyano, alkyl, cycloalkyl, aliphatic heterocyclyl, -$OR^g$, -$NR^hR^{i,}$ -C(O)$R^{24}$, -C(O)$NR^hR^i$ or -C(O)$OR^g$ ;

$R^4$ is selected from alkyl, cycloalkyl, aliphatic heterocyclyl, aryl or heteroaryl, wherein the alkyl, cycloalkyl, aliphatic heterocyclyl, aryl, heteroaryl are optionally substituted by one or more $R^{26}$;

each occurrence of $R^{26}$ is independently selected from halogen, cyano, alkyl, cycloalkyl, aliphatic heterocyclyl, aryl, heteroaryl, -$OR^j$, -$NR^kR^m$, -C(O)$R^{27}$, -C(O)$NR^kR^m$ or -C(O)$OR^j$, wherein the alkyl, cycloalkyl, aliphatic heterocyclyl, aryl, heteroaryl are optionally substituted by one or more $R^{28}$;

each occurrence of $R^{28}$ is independently selected from halogen, cyano, alkyl, cycloalkyl, aliphatic heterocyclyl, -$OR^j$, -$NR^kR^m$, -C(O)$R^{27}$, -C(O)$NR^kR^m$ or -C(O)$OR^j$;

each occurrence of $R^8$, $R^9$, $R^{10}$, $R^{29}$ is independently selected from hydrogen, halogen, cyano, alkyl, cycloalkyl, aliphatic heterocyclyl, aryl, heteroaryl, -$OR^q$, -$NR^rR^s$, -C(O)$R^{30}$, -C(O)$NR^rR^s$ or -C(O)$OR^q$; wherein the alkyl, cycloalkyl, aliphatic heterocyclyl, aryl, heteroaryl are optionally substituted by one or more $R^{31}$;

When the ' ----- ' in ring E is a bond, $R^{11}$ is absent;

each occurrence of $R^{31}$ is independently selected from halogen, cyano, alkyl, cycloalkyl, aliphatic heterocyclyl, -$OR^q$, -$NR^rR^s$, -C(O)$R^{30}$, -C(O)$NR^rR^s$ or -C(O)$OR^q$;

each occurrence of $R^a$, $R^b$, $R^c$, $R^d$, $R^e$, $R^f$, $R^g$, $R^h$, R', $R^j$, $R^k$, $R^m$, $R^n$, R°, $R^p$, $R^q$, $R^r$, $R^s$ is independently selected from H, alkyl, cycloalkyl, aliphatic heterocyclyl or -C(O)$R^{32}$; wherein the alkyl, cycloalkyl, alicyclic heterocyclyl are optionally substituted by one or more substituents selected from the group consisting of: halogen, cyano, hydroxy, amino, alkyl, cycloalkyl and alkyl substituted or unsubstituted aliphatic heterocyclyl;

each occurrence of $R^{16}$, $R^{17}$, $R^{19}$, $R^{20}$, $R^{22}$, $R^{24}$, $R^{27}$, $R^{30}$, $R^{32}$ is independently selected from H, alkyl, cycloalkyl or alicyclic heterocyclyl, the alkyl, cycloalkyl, alicyclic heterocyclyl are optionally substituted by one or more of the following substituents: halogen, cyano, hydroxy, amino, alkyl, cycloalkyl, aliphatic heterocyclyl.

In some embodiments of the present disclosure, the compound of the present disclosure has the structure shown in formula I''' ,or a tautomer, meso isomer, racemate, enantiomer, diastereomer or mixture thereof, or a pharmaceutically acceptable hydrate, solvate or salt thereof:

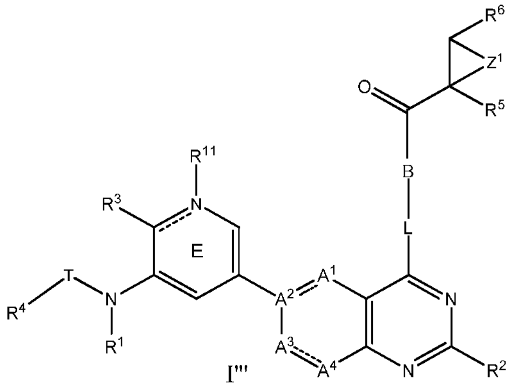

I'''

wherein,

T is selected from $S(O)_2$, C=O, $CH_2$ or $NHS(O)_2$;
$A^1$ is selected from CH, $CH_2$, C=O or N, $A^2$ is selected from C, CH or N, $A^3$ is selected from $CR^8$, $CR^9R^{10}$ or N, $A^4$ is selected from $CR^{29}$, $CR^9R^{10}$ or N;
$Z^1$ is a bond or O;
B is

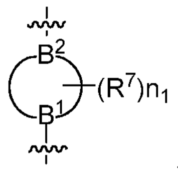
,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;
or, B is

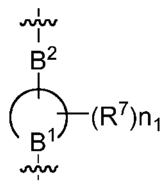
,

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;
or, B is

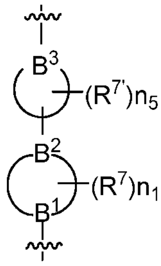
,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13''}$ or N, and at

least one of $B^1$ and $B^2$ is N; the ring where $B^3$ is located is a 4- to 10-membered nitrogen-containing heterocycloalkyl and is a monocyclic ring, spirocyclic ring or bridged cyclic ring, $B^3$ is selected from $CR^{12}$ or N;

$B^1$ is connected to L;

L is selected from a bond or $NR^{15}$;

$R^5$, $R^6$ are independently selected from hydrogen, halogen, cyano, alkyl, aliphatic heterocyclyl, aryl, heteroaryl, -$OR^a$, -$NR^bR^c$, -C(O)$R^{16}$, -S(O)$R^{16}$, -$S(O)_2R^{16}$, -P(O)$R^{16}R^{17}$, -C(O)$NR^bR^c$ or -C(O)$OR^a$, wherein the alkyl, aliphatic heterocyclyl, aryl, heteroaryl are optionally substituted by one or more $R^{18}$;

each occurrence of $R^{18}$ is independently selected from halogen, cyano, alkyl, cycloalkyl, aliphatic heterocyclyl, -$OR^a$, -$NR^bR^c$, -C(O)$R^{16}$, -C(O)$NR^bR^c$ or -C(O)$OR^a$;

$R^1$, $R^{11}$, $R^{14}$, $R^{15}$ are independently selected from hydrogen, alkyl, cycloalkyl, aliphatic heterocyclyl or -C(O)$R^{19}$, wherein the alkyl, cycloalkyl, aliphatic heterocyclyl are optionally substituted by one or more substituents selected from the group consisting of: halogen, cyano, hydroxy, amino, alkyl, cycloalkyl and aliphatic heterocyclyl;

$n_1$, $n_5$ are independently selected from 0, 1, 2, 3 or 4;

each occurrence of $R^{12}$, $R^{12'}$, $R^{13}$, $R^{13'}$, $R^{13''}$, $R^7$, $R^{7'}$ is independently selected from hydrogen, halogen, cyano, alkyl, cycloalkyl, aliphatic heterocyclyl, aryl, heteroaryl, -$OR^n$, -$NR^oR^p$, -C(O)$R^{20}$, -C(O)$NR^oR^p$ or -C(O)$OR^n$, wherein the alkyl, cycloalkyl, aliphatic heterocyclyl, aryl, heteroaryl are optionally substituted by one or more $R^{21}$;

each occurrence of $R^{21}$ is independently selected from halogen, cyano, alkyl, cycloalkyl, aliphatic heterocyclyl, -$OR^n$, -$NR^oR^p$, -C(O)$R^{20}$, -C(O)$NR^oR^p$ or -C(O)$OR^n$,

or $Z^1$ is a bond, $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicyclyl, or $R^6$, $R^{14}$ together with the fragment to which $R^6$, $R^{14}$ are attached form a 4- to 9-membered aliphatic heterocyclyl, or $R^5$, $R^6$ together with the atoms to which $R^5$, $R^6$ are attached form a 6- to 10-membered aryl or 5- to 10-membered heteroaryl, wherein the cycloalkenyl, alicyclic heterocyclyl, alicyclyl, aryl and heteroaryl are optionally substituted by one or more substituents selected from the group consisting of: halogen, cyano, hydroxy, amino, alkyl, cycloalkyl and alicyclic heterocyclyl;

$R^2$ is selected from hydrogen, halogen, cyano, alkyl, cycloalkyl, alicyclic heterocyclyl, aryl, heteroaryl, -$OR^d$, - $NR^eR^f$, -C(O)$R^{22}$, -C(O)$R^eR^f$ or -C(O)$OR^d$, wherein the alkyl, cycloalkyl, alicyclic heterocyclyl, aryl, heteroaryl are optionally substituted by one or more $R^{23}$;

each occurrence of $R^{23}$ is independently selected from halogen, cyano, alkyl, cycloalkyl, aliphatic heterocyclyl, -$OR^d$, -$NR^eR^f$, -C(O)$R^{22}$, -C(O)$NR^eR^f$ or -C(O)$OR^d$;

$R^3$ is selected from hydrogen, halogen, cyano, alkyl, cycloalkyl, aliphatic heterocyclyl, aryl, heteroaryl, -$OR^g$, - $NR^hR^i$, -C(O)$R^{24}$, -C(O)$R^hR^i$ or -C(O)$OR^g$, when the '----- ' in ring E is absent, $R^3$ can also be the carbonyl group that together formed by the connected carbon atom, wherein the alkyl, cycloalkyl, aliphatic heterocyclyl, aryl, heteroaryl are optionally substituted by one or more $R^{25}$;

each occurrence of $R^{25}$ is independently selected from halogen, cyano, alkyl, cycloalkyl, aliphatic heterocyclyl, -$OR^g$, -$NR^hR^{i,}$ -C(O)$R^{24}$, -C(O)$NR^hR^i$ or -C(O)$OR^g$;

$R^4$ is selected from alkyl, cycloalkyl, aliphatic heterocyclyl, aryl or heteroaryl, wherein the alkyl, cycloalkyl, aliphatic heterocyclyl, aryl, heteroaryl are optionally substituted by one or more $R^{26}$;

each occurrence of $R^{26}$ is independently selected from halogen, cyano, alkyl, cycloalkyl, aliphatic heterocyclyl, aryl, heteroaryl, -$OR^j$, -$NR^kR^m$, -C(O)$R^{27}$, -C(O)$NR^kR^m$ or -C(O)$OR^j$, wherein the alkyl, cycloalkyl, aliphatic heterocyclyl, aryl, heteroaryl are optionally substituted by one or more $R^{28}$;

each occurrence of $R^{28}$ is independently selected from halogen, cyano, alkyl, cycloalkyl, aliphatic heterocyclyl, -$OR^j$, -$NR^kR^m$, -C(O)$R^{27}$, -C(O)$NR^kR^m$ or -C(O)$OR^j$;

each occurrence of $R^8$, $R^9$, $R^{10}$, $R^{29}$ is independently selected from hydrogen, halogen, cyano, alkyl, cycloalkyl, aliphatic heterocyclyl, aryl, heteroaryl, -$OR^q$, -$NR^rR^s$, -C(O)$R^{30}$, -C(O)$NR^rR^s$ or -C(O)$OR^q$; wherein the alkyl, cycloalkyl, aliphatic heterocyclyl, aryl, heteroaryl are optionally substituted by one or more $R^{31}$;

When the ' ----- ' in ring E is a bond, $R^{11}$ is absent;

each occurrence of $R^{31}$ is independently selected from halogen, cyano, alkyl, cycloalkyl, aliphatic heterocyclyl, -$OR^q$, -$NR^rR^s$, -C(O)$R^{30}$, -C(O)$NR^rR^s$ or -C(O)$OR^q$;

each occurrence of $R^a$, $R^b$, $R^c$, $R^d$, $R^e$, $R^f$, $R^g$, $R^h$, R', $R^j$, $R^k$, $R^m$, $R^n$, R°, $R^p$, $R^q$, $R^r$, $R^s$ is independently selected from H, alkyl, cycloalkyl, aliphatic heterocyclyl or -C(O)$R^{32}$; wherein the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl;

each occurrence of $R^{16}$, $R^{17}$, $R^{19}$, $R^{20}$, $R^{22}$, $R^{24}$, $R^{27}$, $R^{30}$, $R^{32}$ is independently selected from H, alkyl, cycloalkyl or alicyclic heterocyclyl, wherein the alkyl, cycloalkyl, alicyclic heterocyclyl are optionally substituted by one or more substituents selected from the group consisting of: halogen, cyano, hydroxy, amino, alkyl, cycloalkyl and aliphatic

heterocyclyl.

In some embodiments of the present disclosure, the compound of the present disclosure has the structure shown in formula I' ,or a tautomer, meso isomer, racemate, enantiomer, diastereomer or mixture thereof, or a pharmaceutically acceptable hydrate, solvate or salt thereof:

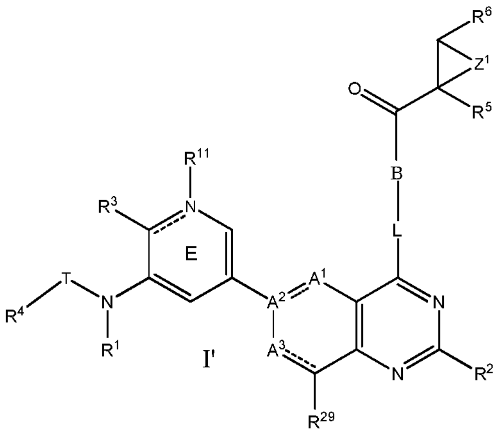

Wherein,

T is selected from $S(O)_2$, C=O, $CH_2$ or $NHS(O)_2$;
$A^1$ is selected from CH, $CH_2$, C=O or N, $A^2$ is selected from C or N, $A^3$ is selected from $CR^8$, $CR^9R^{10}$ or N; $Z^1$ is a bond or O;
B is

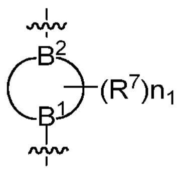

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;
or, B is

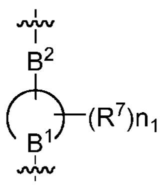

,

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;
or, B is

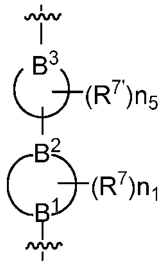

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13''}$ or N, and at least one of $B^1$ and $B^2$ is N; the ring where $B^3$ is located is a 4- to 10-membered nitrogen-containing heterocycloalkyl and is a monocyclic ring, spirocyclic ring or bridged cyclic ring, $B^3$ is selected from $CR^{12'}$ or N;

$B^1$ is connected to L;

L is selected from a bond or $NR^{15}$;

$R^5$, $R^6$ are independently selected from hydrogen, halogen, cyano, alkyl, aliphatic heterocyclyl, aryl, heteroaryl, -$OR^a$, -$NR^bR^c$, -C(O)$R^{16}$, -S(O)$R^{16}$, -$S(O)_2R^{16}$, -P(O)$R^{16}R^{17}$, -C(O)$NR^bR^c$ or -C(O)$OR^a$, wherein the alkyl, aliphatic heterocyclyl, aryl, heteroaryl are optionally substituted by one or more $R^{18}$;

each occurrence of $R^{18}$ is independently selected from halogen, cyano, alkyl, cycloalkyl, aliphatic heterocyclyl, -$OR^a$, -$NR^bR^c$, -C(O)$R^{16}$, -C(O)$NR^bR^c$ or -C(O)$OR^a$;

$R^1$, $R^{11}$, $R^{14}$, $R^{15}$ are independently selected from hydrogen, alkyl, cycloalkyl, aliphatic heterocyclyl or -C(O)$R^{19}$, wherein the alkyl, cycloalkyl, aliphatic heterocyclyl are optionally substituted by one or more substituents selected from the group consisting of: halogen, cyano, hydroxy, amino, alkyl, cycloalkyl and aliphatic heterocyclyl;

$n_1$, $n_5$ are independently selected from 0, 1, 2, 3 or 4;

each occurrence of $R^{12}$, $R^{12'}$, $R^{13}$, $R^{13'}$, $R^{13''}$, $R^7$, $R^{7'}$ is independently selected from hydrogen, halogen, cyano, alkyl, cycloalkyl, aliphatic heterocyclyl, aryl, heteroaryl, -$OR^n$, -$NR^oR^p$, -C(O)$R^{20}$, -C(O)$NR^oR^p$ or -C(O)$OR^n$, wherein the alkyl, cycloalkyl, aliphatic heterocyclyl, aryl, heteroaryl are optionally substituted by one or more $R^{21}$;

each occurrence of $R^{21}$ is independently selected from halogen, cyano, alkyl, cycloalkyl, aliphatic heterocyclyl, -$OR^n$, -$NR^oR^p$, -C(O)$R^{20}$, -C(O)$NR^oR^p$ or -C(O)$OR^n$,

or $Z^1$ is a bond, $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicyclyl, or $R^6$, $R^{14}$ together with the fragment to which $R^6$, $R^{14}$ are attached form a 4- to 9-membered aliphatic heterocyclyl, or $R^5$, $R^6$ together with the atoms to which $R^5$, $R^6$ are attached form a 6- to 10-membered aryl or 5- to 10-membered heteroaryl, wherein the cycloalkenyl, alicyclic heterocyclyl, alicyclyl, aryl and heteroaryl are optionally substituted by one or more substituents selected from the group consisting of: halogen, cyano, hydroxy, amino, alkyl, cycloalkyl and alicyclic heterocyclyl;

$R^2$ is selected from hydrogen, halogen, cyano, alkyl, cycloalkyl, alicyclic heterocyclyl, aryl, heteroaryl, -$OR^d$, - $NR^eR^f$, -C(O)$R^{22}$, -C(O)$R^eR^f$ or -C(O)$OR^d$, wherein the alkyl, cycloalkyl, alicyclic heterocyclyl, aryl, heteroaryl are optionally substituted by one or more $R^{23}$;

each occurrence of $R^{23}$ is independently selected from halogen, cyano, alkyl, cycloalkyl, aliphatic heterocyclyl, -$OR^d$, -$NR^eR^f$, -C(O)$R^{22}$, -C(O)$NR^eR^f$ or -C(O)$OR^d$;

$R^3$ is selected from hydrogen, halogen, cyano, alkyl, cycloalkyl, aliphatic heterocyclyl, aryl, heteroaryl, -$OR^g$, - $NR^hR^i$, -C(O)$R^{24}$, -C(O)$R^hR^i$ or -C(O)$OR^g$, when the '-----' in ring E is absent, $R^3$ can also be a carbonyl group that together formed by the connected carbon atom, wherein the alkyl, cycloalkyl, aliphatic heterocyclyl, aryl, heteroaryl are optionally substituted by one or more $R^{25}$;

each occurrence of $R^{25}$ is independently selected from halogen, cyano, alkyl, cycloalkyl, aliphatic heterocyclyl, -$OR^g$, -$NR^hR^{i,}$ -C(O)$R^{24}$, -C(O)$NR^hR^i$ or -C(O)$OR^g$;

$R^4$ is selected from alkyl, cycloalkyl, aliphatic heterocyclyl, aryl or heteroaryl, wherein the alkyl, cycloalkyl, aliphatic heterocyclyl, aryl, heteroaryl are optionally substituted by one or more $R^{26}$;

each occurrence of $R^{26}$ is independently selected from halogen, cyano, alkyl, cycloalkyl, aliphatic heterocyclyl, aryl, heteroaryl, -$OR^j$, -$NR^kR^m$, -C(O)$R^{27}$, -C(O)$NR^kR^m$ or -C(O)$OR^j$, wherein the alkyl, cycloalkyl, aliphatic heterocyclyl, aryl, heteroaryl are optionally substituted by one or more $R^{28}$;

each occurrence of $R^{28}$ is independently selected from halogen, cyano, alkyl, cycloalkyl, aliphatic heterocyclyl, -ORi, -$NR^kR^m$, -C(O)$R^{27}$, -C(O)$NR^kR^m$ or -C(O)$OR^j$;

each occurrence of $R^8$, $R^9$, $R^{10}$, $R^{29}$ is independently selected from hydrogen, halogen, cyano, alkyl, cycloalkyl,

aliphatic heterocyclyl, aryl, heteroaryl, $-OR^q$, $-NR^rR^s$, $-C(O)R^{30}$, $-C(O)NR^rR^s$ or $-C(O)OR^q$; wherein the alkyl, cycloalkyl, aliphatic heterocyclyl, aryl, heteroaryl are optionally substituted by one or more $R^{31}$;

When the ‘ ----- ’ in ring E is a bond, $R^{11}$ is absent;

each occurrence of $R^{31}$ is independently selected from halogen, cyano, alkyl, cycloalkyl, aliphatic heterocyclyl, $-OR^q$, $-NR^rR^s$, $-C(O)R^{30}$, $-C(O)NR^rR^s$ or $-C(O)OR^q$;

each occurrence of $R^a$, $R^b$, $R^c$, $R^d$, $R^e$, $R^f$, $R^g$, $R^h$, R', $R^j$, $R^k$, $R^m$, $R^n$, $R^o$, $R^p$, $R^q$, $R^r$, $R^s$ is independently selected from H, alkyl, cycloalkyl, aliphatic heterocyclyl or $-C(O)R^{32}$; wherein the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl;

each occurrence of $R^{16}$, $R^{17}$, $R^{19}$, $R^{20}$, $R^{22}$, $R^{24}$, $R^{27}$, $R^{30}$, $R^{32}$ is independently selected from H, alkyl, cycloalkyl or alicyclic heterocyclyl, the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl.

In some embodiments of the present disclosure, the compound of the present disclosure has the structure shown in formula I or a tautomer, meso isomer, racemate, enantiomer, diastereomer or mixture thereof, or a pharmaceutically acceptable hydrate, solvate or salt thereof:

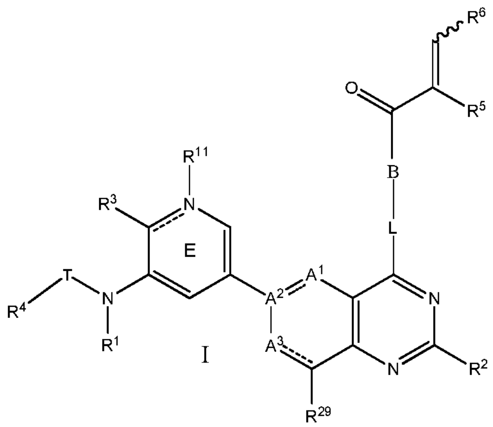

wherein,

T is selected from $S(O)_2$ or C=O;

$A^1$ is selected from CH, $CH_2$, C=O or N, $A^2$ is selected from C or N, $A^3$ is selected from $CR^8$, $CR^9R^{10}$ or N;

B is

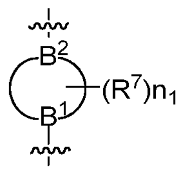

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;

or, B is

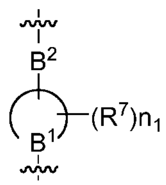

,

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;

$B^1$ is connected to L;

L is selected from a bond or $NR^{15}$;

$R^5$, $R^6$ are independently selected from hydrogen, halogen, cyano, alkyl, aliphatic heterocyclyl, aryl, heteroaryl, -$OR^a$, -$NR^bR^c$, -C(O)$R^{16}$, -S(O)$R^{16}$, -$S(O)_2R^{16}$, -P(O)$R^{16}R^{17}$, -C(O)$NR^bR^c$ or -C(O)$OR^a$, wherein the alkyl, aliphatic heterocyclyl, aryl, heteroaryl are optionally substituted by one or more $R^{18}$;

each occurrence of $R^{18}$ is independently selected from halogen, cyano, alkyl, cycloalkyl, aliphatic heterocyclyl, -$OR^a$, -$NR^bR^c$, -C(O)$R^{16}$, -C(O)$NR^bR^c$ or -C(O)$OR^a$;

$R^1$, $R^{11}$, $R^{14}$, $R^{15}$ are independently selected from hydrogen, alkyl, cycloalkyl, aliphatic heterocyclyl or -C(O)$R^{19}$, wherein the alkyl, cycloalkyl, aliphatic heterocyclyl are optionally substituted by one or more of the following substituents: halogen, cyano, hydroxy, amino, alkyl, cycloalkyl, aliphatic heterocyclyl;

When the ' ----- ' in ring E is a bond, $R^{11}$ is absent;

$n_1$ is selected from 0, 1, 2, 3 or 4;

each occurrence of $R^{12}$, $R^{13}$, $R^{13'}$, $R^7$ is independently selected from hydrogen, halogen, cyano, alkyl, cycloalkyl, aliphatic heterocyclyl, aryl, heteroaryl, -$OR^n$, -$NR^oR^p$, -C(O)$R^{20}$, -C(O)$NR^oR^p$ or -C(O)$OR^n$, wherein the alkyl, cycloalkyl, aliphatic heterocyclyl, aryl, heteroaryl are optionally substituted by one or more $R^{21}$;

each occurrence of $R^{21}$ is independently selected from halogen, cyano, alkyl, cycloalkyl, aliphatic heterocyclyl, -$OR^n$, -$NR^oR^p$, -C(O)$R^{20}$, -C(O)$NR^oR^p$ or -C(O)$OR^n$;

or $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicycyl, or $R^6$, $R^{14}$ together with the fragment to which $R^6$, $R^{14}$ are attached form a 4- to 9-membered aliphatic heterocyclyl,

$R^2$ is selected from hydrogen, halogen, cyano, alkyl, cycloalkyl, alicyclic heterocyclyl, aryl, heteroaryl, -$OR^d$, - $NR^eR^f$, -C(O)$R^{22}$, -C(O)$R^eR^f$ or -C(O)$OR^d$, wherein the alkyl, cycloalkyl, alicyclic heterocyclyl, aryl, heteroaryl are optionally substituted by one or more $R^{23}$;

each occurrence of $R^{23}$ is independently selected from halogen, cyano, alkyl, cycloalkyl, aliphatic heterocyclyl, -$OR^d$, -$NR^eR^f$, -C(O)$R^{22}$, -C(O)$NR^eR^f$ or -C(O)$OR^d$;

$R^3$ is selected from hydrogen, halogen, cyano, alkyl, cycloalkyl, aliphatic heterocyclyl, aryl, heteroaryl, -$OR^g$, - $NR^hR^i$, -C(O)$R^{24}$, -C(O)$R^hR^i$ or -C(O)$OR^g$, when the '-----' in ring E is absent, $R^3$ can also be a carbonyl group that together formed by the connected carbon atom, wherein the alkyl, cycloalkyl, aliphatic heterocyclyl, aryl, heteroaryl are optionally substituted by one or more $R^{25}$;

each occurrence of $R^{25}$ is independently selected from halogen, cyano, alkyl, cycloalkyl, aliphatic heterocyclyl, -$OR^g$, -$NR^hR^i$, -C(O)$R^{24}$, -C(O)$NR^hR^i$ or -C(O)$OR^g$;

$R^4$ is selected from alkyl, cycloalkyl, aliphatic heterocyclyl or aryl, heteroaryl, wherein the alkyl, cycloalkyl, aliphatic heterocyclyl, aryl, heteroaryl are optionally substituted by one or more $R^{26}$;

each occurrence of $R^{26}$ is independently selected from halogen, cyano, alkyl, cycloalkyl, aliphatic heterocyclyl, aryl, heteroaryl, -$OR^j$, -$NR^kR^m$, -C(O)$R^{27}$, -C(O)$NR^kR^m$ or -C(O)$OR^j$, wherein the alkyl, cycloalkyl, aliphatic heterocyclyl, aryl, heteroaryl are optionally substituted by one or more $R^{28}$;

each occurrence of $R^{28}$ is independently selected from halogen, cyano, alkyl, cycloalkyl, aliphatic heterocyclyl, -$OR^j$, -$NR^kR^m$, -C(O)$R^{27}$, -C(O)$NR^kR^m$ or -C(O)$OR^j$;

each occurrence of $R^8$, $R^9$, $R^{10}$, $R^{29}$ is independently selected from hydrogen, halogen, cyano, alkyl, cycloalkyl, aliphatic heterocyclyl, aryl, heteroaryl, -$OR^q$, -$NR^rR^s$, -C(O)$R^{30}$, -C(O)$NR^rR^s$ or -C(O)$OR^q$; wherein the alkyl, cycloalkyl, aliphatic heterocyclyl, aryl, heteroaryl are optionally substituted by one or more $R^{31}$;

each occurrence of $R^{31}$ is independently selected from halogen, cyano, alkyl, cycloalkyl, aliphatic heterocyclyl, -$OR^q$, -$NR^rR^s$, -C(O)$R^{30}$, -C(O)$NR^rR^s$ or -C(O)$OR^q$;

each occurrence of $R^a$, $R^b$, $R^c$, $R^d$, $R^e$, $R^f$, $R^g$, $R^h$, R', $R^j$, $R^k$, $R^m$, $R^n$, $R^o$, $R^p$, $R^q$, $R^r$, $R^s$ is independently selected from H, alkyl, cycloalkyl, aliphatic heterocyclyl or -C(O)$R^{32}$; wherein the alkyl, cycloalkyl, alicyclic heterocyclyl are optionally substituted by one or more of the following substituents: halogen, cyano, hydroxy, amino, alkyl, cycloalkyl, aliphatic heterocyclyl;

each occurrence of $R^{16}$, $R^{17}$, $R^{19}$, $R^{20}$, $R^{22}$, $R^{24}$, $R^{27}$, $R^{30}$, $R^{32}$ is independently selected from H, alkyl, cycloalkyl or alicyclic heterocyclyl, the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl.

$Z^1$ is a bond, which means that the two carbon atoms between $R^5$ and $R^6$ are connected by a double bond:

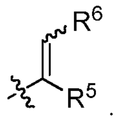

'Each occurrence of $R^{12}$ is independently selected from' means that when the number of $R^{12}$ is greater than 1, different $R^{12}$ can be selected from the same or different groups. For example, when the number of $R^{12}$ is 2, one $R^{12}$ can be selected from alkyl and the other $R^{12}$ can be selected from halogen; or, both $R^{12}$ can be selected from alkyl; the same applies to other similar situations.

In

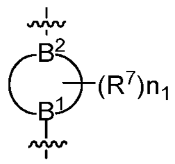

,

the connection point of $R^7$ is not fixed. It should be understood as that $R^7$ can be any substitutable position on ring B, and the same applies to other similar situations; but at here, since the substituents on $B^1$ and $B^2$ are limited to $R^{12}$ or $R^{13}$, then it should be understood as that the substitutable positions for $R^7$ do not include $B^1$ or $B^2$.

The connecting bond of $R^6$ is , which means that the configuration of $R^6$ on the alkenyl is not fixed and can be Z-configuration or E-configuration. The same applies to other similar situations.

'$R^5$, $R^6$ together with the atoms to which $R^5$, $R^6$ are attached form a 4- to 9-membered cycloalkenyl' means that $R^5$, $R^6$ and their commonly connected alkenyl fragment together form a 4- to 9-membered cycloalkenyl, that is,

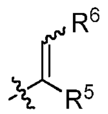

constitutes

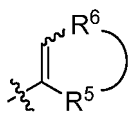

as a 4- to 9-membered cycloalkenyl. The same applies to other similar situations. '$R^5$, $R^6$ together with the atoms to which $R^5$, $R^6$ are attached form a 6- to 10-membered aryl or 5- to 10-membered heteroaryl' means that $R^5$, $R^6$ and their commonly connected alkenyl fragment together form a 6- to 10-membered aryl or 5- to 10-membered heteroaryl, that is,

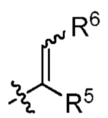

constitutes

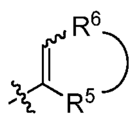

as a 6- to 10-membered aryl or 5- to 10-membered heteroaryl. The same applies to other similar situations.

'$R^5$, $R^{14}$ together with the fragment to which $R^5$, $R^{14}$ are attached form a 4- to 9-membered aliphatic heterocyclyl' means that $R^5$, $R^{14}$ and the $B^2$ ($NR^{14}$)and carbonyl groups between them together form a 4- to 9-membered aliphatic

heterocyclyl, that is,

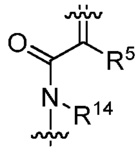

constitutes

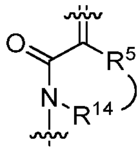

as a 4- to 9-membered aliphatic heterocyclyl. The same applies to other similar situations.

'$R^6$, $R^{13}$ together with the fragment to which $R^6$, $R^{13}$ are attached form a 4- to 9-membered cycloalkenyl' means that $R^6$, $R^{13}$ and the $B^2$ ($CR^{13}$ or $CR^{13}R^{13'}$), carbonyl, alkenyl fragment together form a 4- to 9-membered cycloalkenyl, that is,

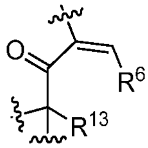

constitutes

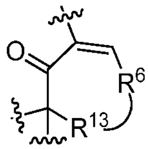

as a 4- to 9-membered cycloalkenyl. The same applies to other similar situations.

The '-----' in ring E represents '----- ' can be a bond or absent. When the ' ----- ' in ring E is a bond, $R^{11}$ is absent means ring E is

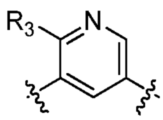

;

when the ' -----' is absent, means ring E is

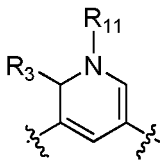

,

further, when the '-----' in ring E is absent, the carbonyl group which formed by $R^3$ and the attached carbon atoms is that, ring E is

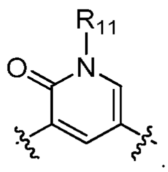

In some embodiments of the present disclosure, the compound of the present disclosure has the structure shown in formula I" or a tautomer, meso isomer, racemate, enantiomer, diastereomer or mixture thereof, or a pharmaceutically acceptable hydrate, solvate or salt thereof:

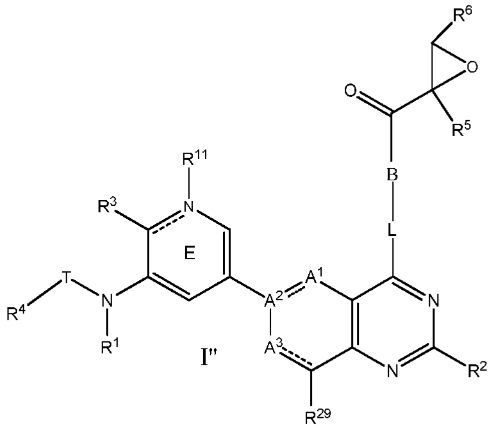

I"

,

wherein, T is selected from $S(O)_2$ or C=O.

In some embodiments of the present disclosure, the

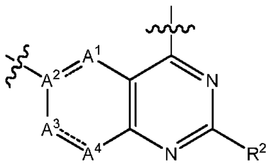

in the compound is selected from the following groups:

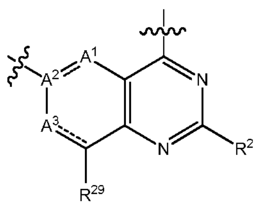
,
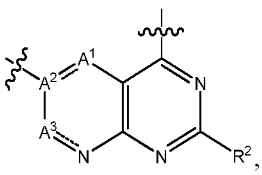
,
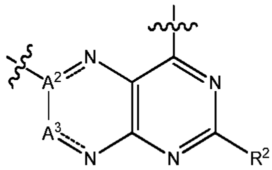
.

In some embodiments of the present disclosure,

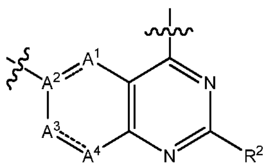

is selected from the following groups:

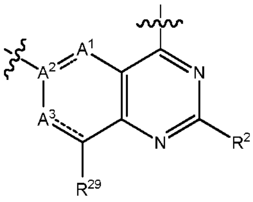
,
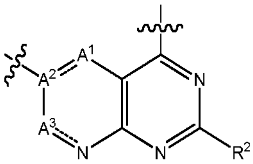
,
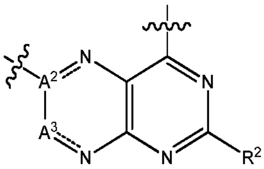
,
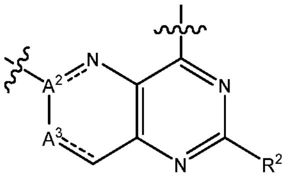
.

In some embodiments of the present disclosure, the

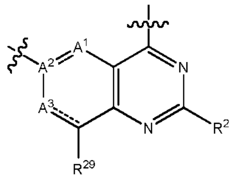

in the compound is selected from the following groups:

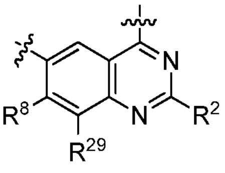
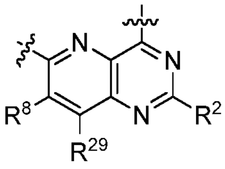
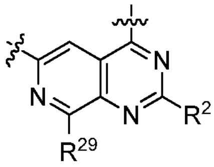
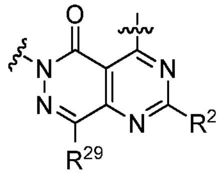
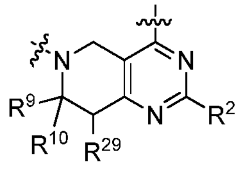

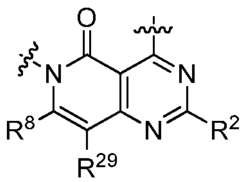
,

preferably

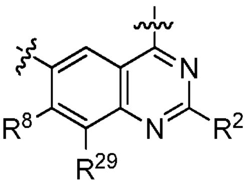
,
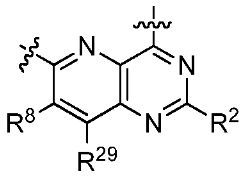
,
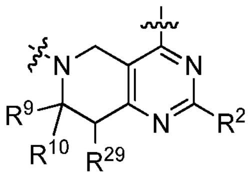
,

more preferably

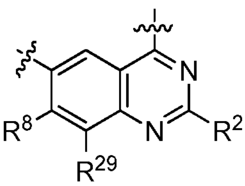
.

In some embodiments of the present disclosure, the compound of the present disclosure has the structure shown in formula (I‴-1) or formula (I‴-2), or a tautomer, meso isomer, racemate, enantiomer, diastereomer or mixture thereof, or a pharmaceutically acceptable hydrate, solvate or salt thereof:

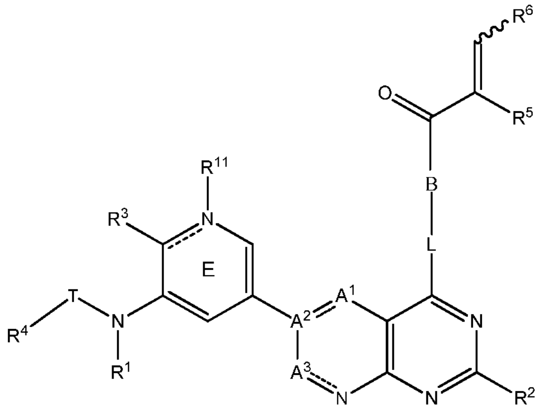

I'''-1

,

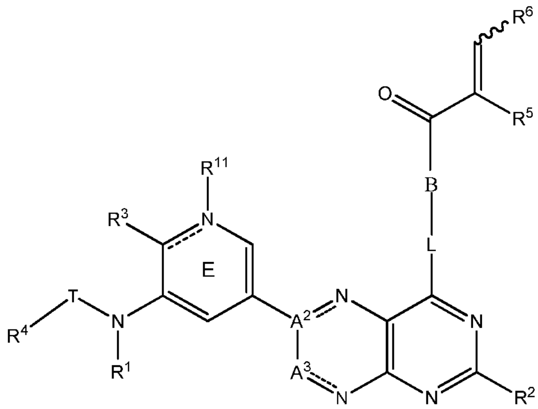

I'''-2

,

wherein, T is selected from $S(O)_2$ or C=O.

In some embodiments of the present disclosure, L is a bond, $B^1$ is N; or L is $NR^{15}$, $B^1$ is $CR^{12}$; further, when B is

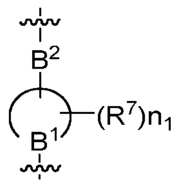

,

$B^1$ is N, $B^2$ is $NR^{14}$.

In some embodiments of the present disclosure, B is

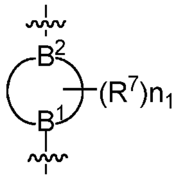

,

the ring where $B^1$, $B^2$ are located is a 6- to 9-membered nitrogen-containing heterocycloalkyl or a 6- to 9-membered nitrogen-containing heterocycloalkenyl, the cycloalkyl or cycloalkenyl is a monocyclic ring or spirocyclic ring; or, B is

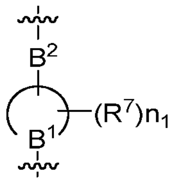

,

the ring where $B^1$ is located is a 4- to 6-membered nitrogen-containing monoheterocycloalkyl.

In some embodiments of the present disclosure, B is

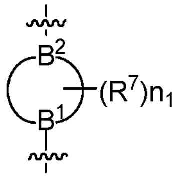

,

the ring where $B^1$, $B^2$ are located is a 6- to 9-membered nitrogen-containing heterocycloalkyl and is a monocyclic ring or spirocyclic ring; or, B is

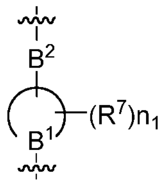

,

the ring where $B^1$ is located is a 4- to 6-membered nitrogen-containing monoheterocycloalkyl;

In some embodiments of the present disclosure, B is

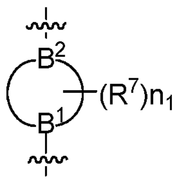

,

the ring where $B^1$, $B^2$ are located is a 5- to 6-membered nitrogen-containing monoheterocycloalkyl, 6-membered nitrogen-containing monoheterocycloalkenyl or 8- to 9-membered nitrogen-containing spiroheterocycloalkyl; or B is

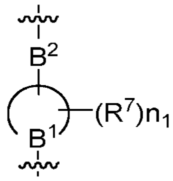

,

the ring where $B^1$ is located is a 4-membered nitrogen-containing monoheterocycloalkyl; further, B is

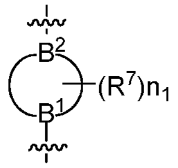
,

the ring where $B^1$, $B^2$ are located is a 6-membered nitrogen-containing monoheterocycloalkyl or 8- to 9-membered nitrogen-containing spiroheterocycloalkyl; or B is

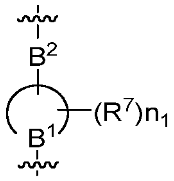
,

the ring where $B^1$ is located is a 4-membered nitrogen-containing monoheterocycloalkyl;
further,

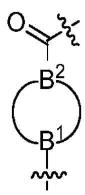
or
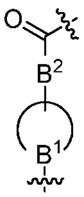

is selected from the following groups:

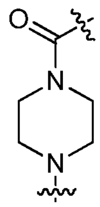 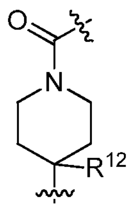 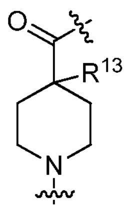 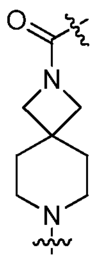 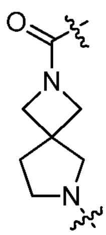 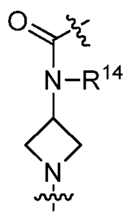 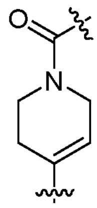 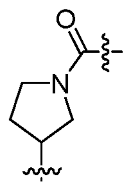

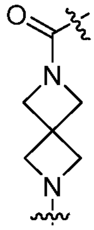
.

In some embodiments of the present disclosure,

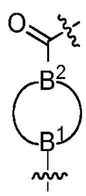 or 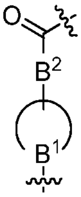

is selected from the following groups:

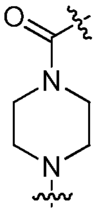 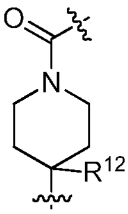 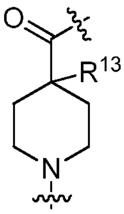 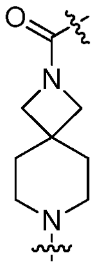 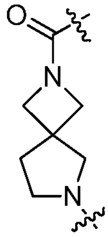 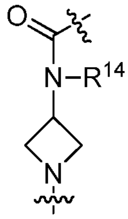 ,

$R^{12}$ is selected from hydrogen, halogen, C1~C6 alkyl, preferably hydrogen, halogen or C1~C3 alkyl, more preferably hydrogen;

$R^{13}$ is selected from hydrogen, halogenor C1~C6 alkyl, preferably hydrogen, halogen or C1~C3 alkyl, or $R^{13}$ and $R^6$ together with the fragment to which $R^{13}$ and $R^6$ are attached form a 4- to 6-membered cycloalkenyl, preferably $R^{13}$ and $R^6$ together with the fragment to which $R^{13}$ and $R^6$ are attached form a 5-membered cycloalkenyl;

$R^{14}$ is selected from hydrogen, C1~C6 alkyl, C3~C6 cycloalkyl, preferably hydrogen or C1~C3 alkyl, wherein the alkyl, cycloalkyl are optionally substituted by one or more substituents selected from the group consisting of: halogen, cyano, hydroxy, amino, C1-C6 alkyl, C3-C6 cycloalkyl and 3- to 6-membered aliphatic heterocyclyl, or $R^{14}$ and $R^5$ together with the fragment to which $R^{14}$ and $R^5$ are attached form a 4- to 6-membered aliphatic heterocyclyl, preferably $R^{14}$ and $R^5$ together with the fragment to which $R^{14}$ and $R^5$ are attached form a 5-membered aliphatic heterocyclyl;

further,

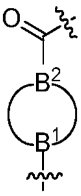

is selected from

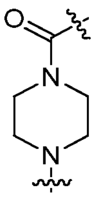 , 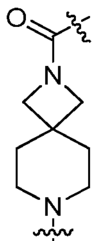 , 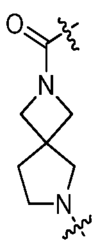 or 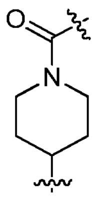 ;

preferably

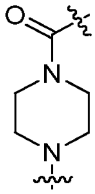,
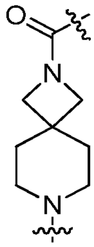,

or

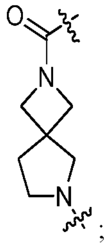;

more preferably,

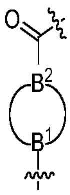 is 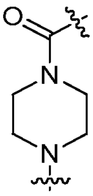.

In some embodiments of the present disclosure, B is

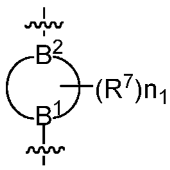,

the ring where $B^1$, $B^2$ are located is a 6- to 9-membered nitrogen-containing bridged heterocycloalkyl, preferably 7- to 8-membered nitrogen-containing bridged heterocycloalkyl, more preferably 8-membered nitrogen-containing bridged heterocycloalkyl; or B is

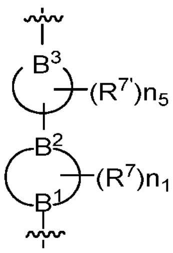,

the ring where $B^1$, $B^2$ are located is a 5- to 8-membered nitrogen-containing heterocycloalkyl and is a monocyclic ring, spirocyclic ring or bridged cyclic ring, preferably 5- to 8-membered nitrogen-containing monoheterocycloalkyl, more preferably 6- to 7-membered nitrogen-containing monoheterocycloalkyl; the ring where $B^3$ is located is a 4- to 8-membered nitrogen-containing heterocycloalkyl and is a monocyclic ring, spirocyclic ring or bridged cyclic ring, preferably 4- to 8-membered nitrogen-containing monoheterocycloalkyl, more preferably 4- to 6-membered nitrogen-containing monoheterocycloalkyl;

further, $R^{12}$, $R^{12}$, $R^{13"}$ are independently selected from hydrogen, halogen or C1-C6 alkyl, preferably hydrogen, halogenor C1-C3 alkyl, more preferably hydrogen;
further, each of $B^1$, $B^2$, $B^3$ is N;
further,

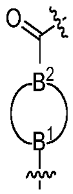 is 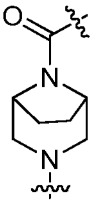, 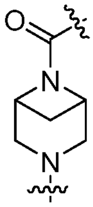, 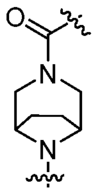, 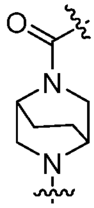, 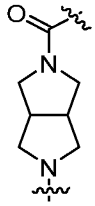; 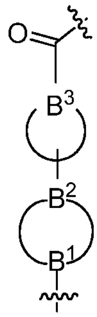 is 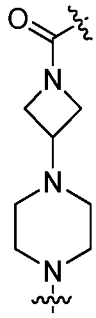.

further,

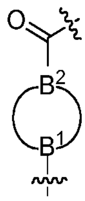 is 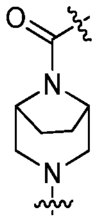; 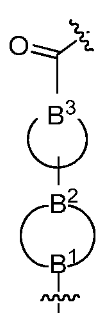 is 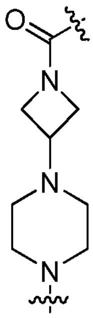.

In some embodiments of the present disclosure, the compound of the present disclosure has the structure shown in formula II or formula III, or a tautomer, meso isomer, racemate, enantiomer, diastereomer or mixture thereof, or a pharmaceutically acceptable hydrate, solvate or salt thereof:

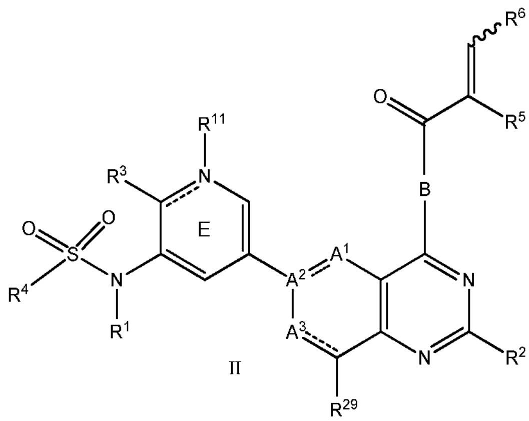

II

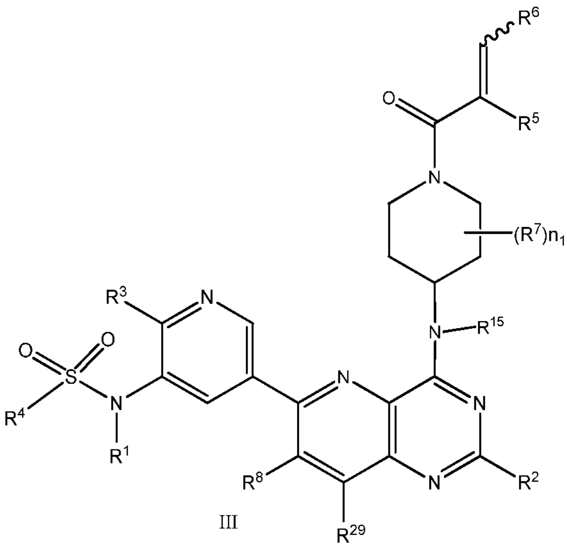

III

;

in formula II,

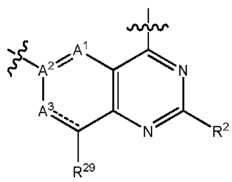

is not

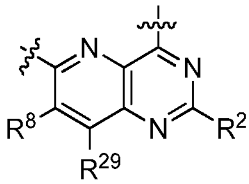

,

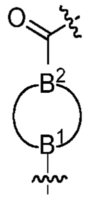

is not

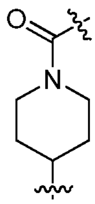

.

In some embodiments of the present disclosure, the compound of the present disclosure has the structure shown in formula II or formula III, or a tautomer, meso isomer, racemate, enantiomer, diastereomer or mixture thereof, or a pharmaceutically acceptable hydrate, solvate or salt thereof:

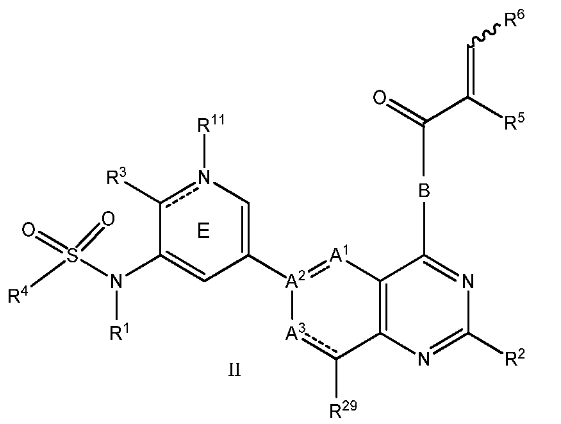

II

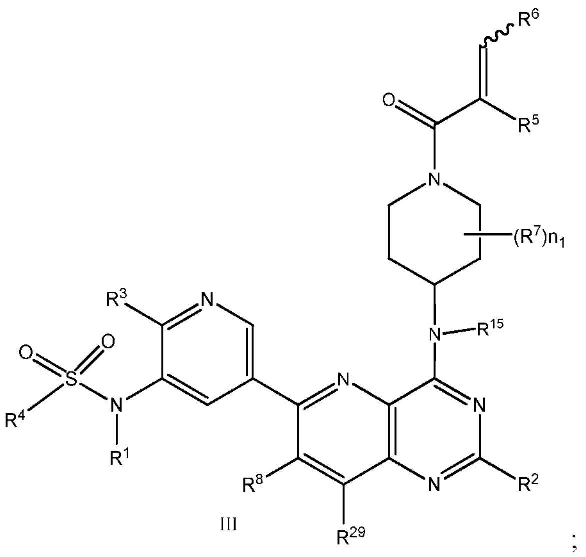

III

;

in formula II, when

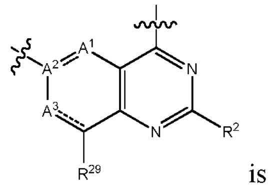 is 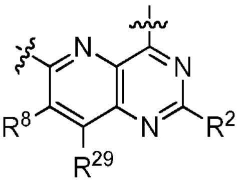 , 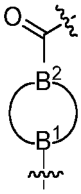

preferably

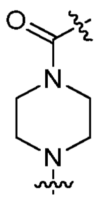 , 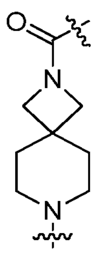

or

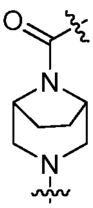

In some embodiments of the present disclosure, the compound of the present disclosure has the structure shown in formula IV or formula V, or a tautomer, meso isomer, racemate, enantiomer, diastereomer or mixture thereof, or a pharmaceutically acceptable hydrate, solvate or salt thereof:

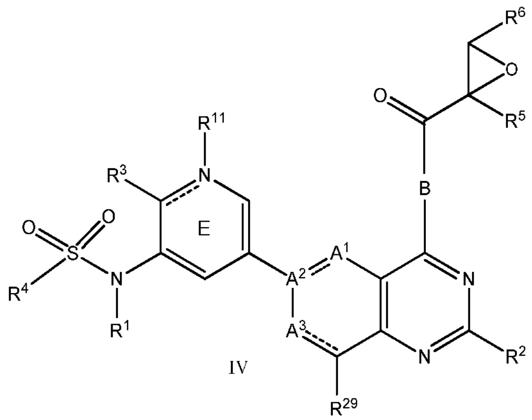

IV

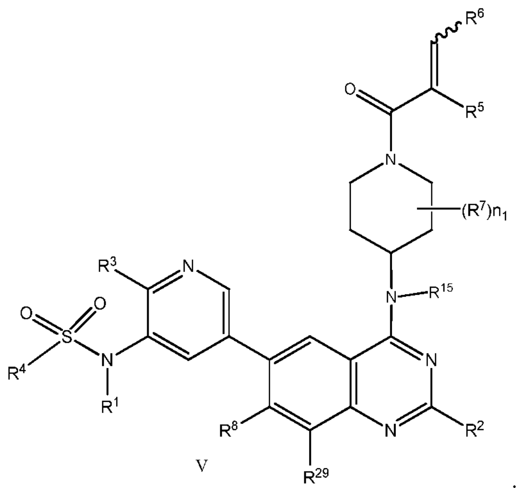

V

In some embodiments of the present disclosure, $n_1$ is selected from 0, 1, 2 or 3, preferably 0, 1 or 2, more preferably 0 or 1.

In some embodiments of the present disclosure, $R^1$, $R^{11}$, $R^{15}$ are independently selected from hydrogen, halogen, C1~C6 alkyl or C3-C6 cycloalkyl, wherein the alkyl, cycloalkyl are optionally substituted by one or more substituents selected from the group consisting of: halogen, cyano, hydroxy, amino, C1-C6 alkyl, C3-C6 cycloalkyl and 3- to 6-membered aliphatic heterocyclyl; when the ' -----' in ring E is a bond, $R^{11}$ is absent;

further, $R^1$, $R^{11}$, $R^{15}$ are independently selected from hydrogen, halogen or C1-C3 alkyl, wherein the alkyl, cycloalkyl are optionally substituted by one or more substituents selected from the group consisting of: halogen, cyano, hydroxy, amino, C1-C3 alkyl and C3-C6 cycloalkyl;

further, $R^1$, $R^{15}$ are independently selected from hydrogen or methyl, preferably hydrogen;

$R^{11}$ is selected from hydrogen or methyl, preferably methyl, when the ' -----' in ring E is a bond, $R^{11}$ is absent.

In some embodiments of the present disclosure, the compound of the present disclosure has the structure

shown in formula II' or formula III' or a tautomer, meso isomer, racemate, enantiomer, diastereomer or mixture thereof, or a pharmaceutically acceptable hydrate, solvate or salt thereof:

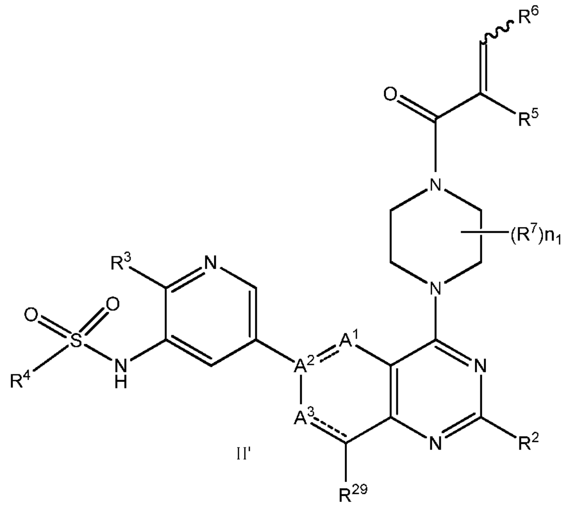

II'

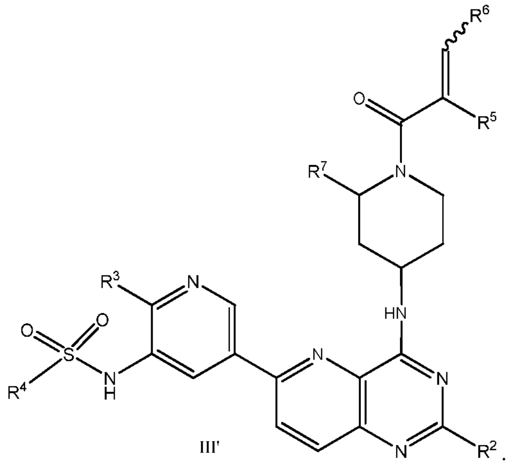

III'

In some embodiments of the present disclosure, the compound of the present disclosure has the structure shown in formula IV' or formula V' or a tautomer, meso isomer, racemate, enantiomer, diastereomer or mixture thereof, or a pharmaceutically acceptable hydrate, solvate or salt thereof:

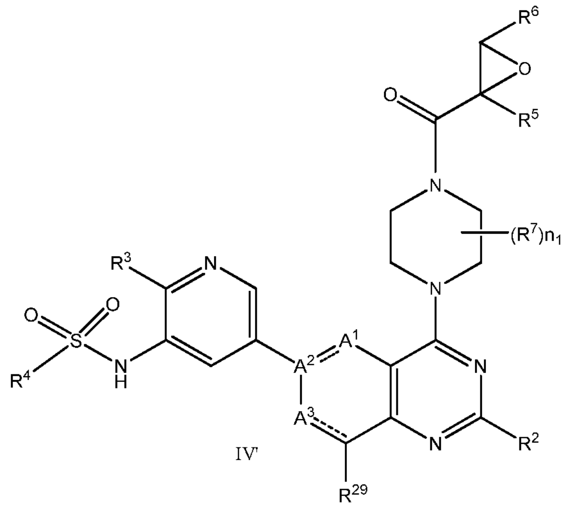

IV'

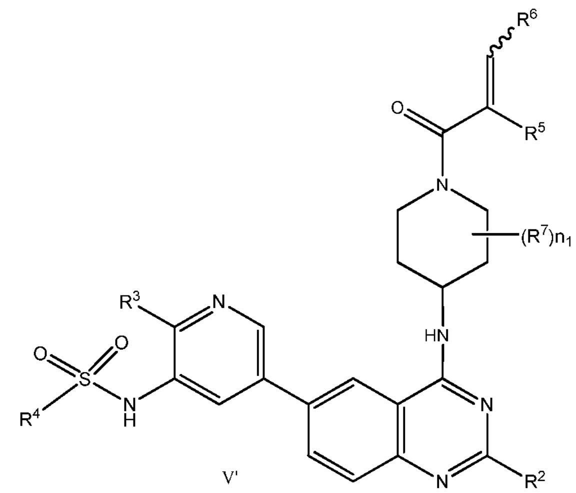

V'

.

In some embodiments of the present disclosure, the compound of the present disclosure has the structure shown in formula VI, VII, VIII or IX or a tautomer, meso isomer, racemate, enantiomer, diastereomer or mixture thereof, or a pharmaceutically acceptable hydrate, solvate or salt thereof:

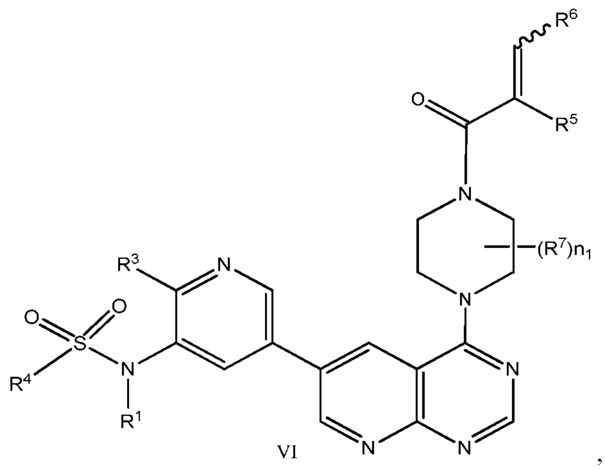

VI

,

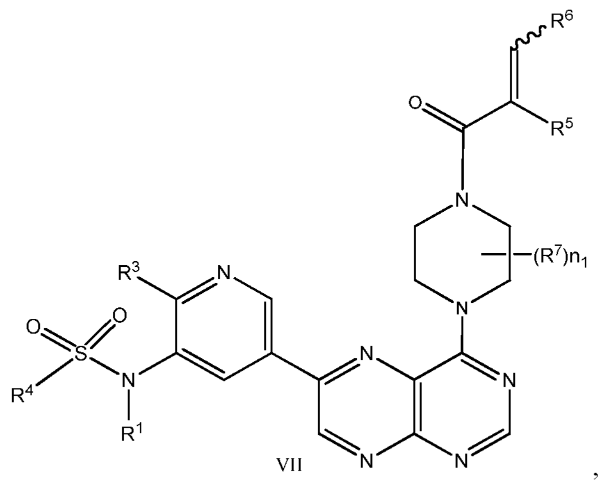

VII

,

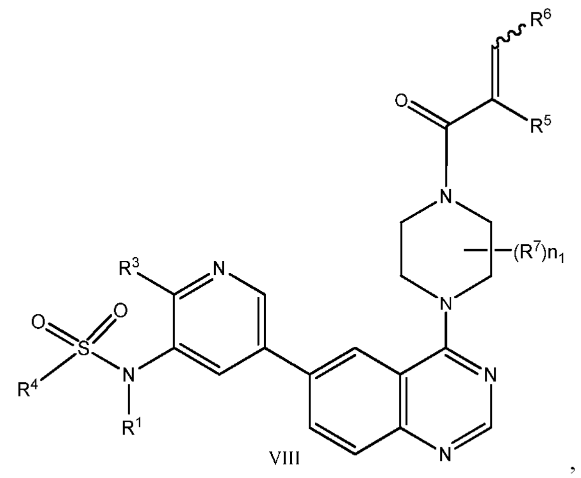

VIII

,

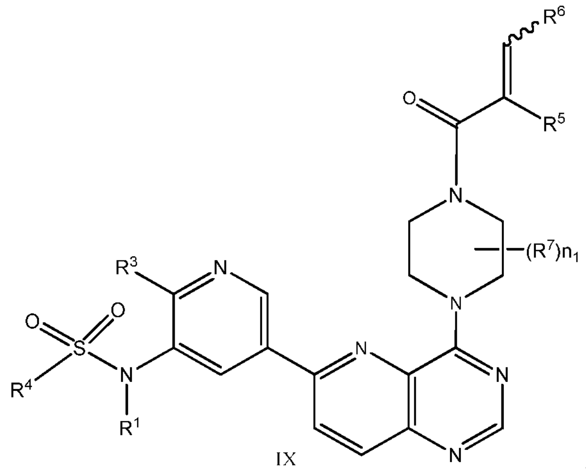

IX

,

wherein $n_1$ is selected from 0, 1, 2 or 3, preferably 0, 1 or 2, more preferably 0 or 1.

In some embodiments of the present disclosure, $R^5$, $R^6$ are independently selected from hydrogen, halogen, C1-C6 alkyl, C3-C6 cycloalkyl, 3- to 6-membered aliphatic heterocyclyl, 6- to 10-membered aryl, 5- to 10-membered heteroaryl, $-C(O)R^{16}$, $-S(O)R^{16}$, $-S(O)_2R^{16}$, $-P(O)R^{16}R^{17}$, $-C(O)NR^bR^c$ or $-C(O)OR^a$, wherein the alkyl, cycloalkyl, aliphatic heterocyclyl, aryl, heteroaryl are optionally substituted by one or more $R^{18}$;

each occurrence of $R^{18}$ is independently selected from halogen, cyano, C1-C6 alkyl, C3-C6 cycloalkyl, $-OR^a$ or $-NR^bR^c$;

or $R^5$, $R^6$ together with the atoms to which $R^5$, $R^6$ are attached form

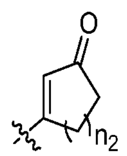

,

$n_2$ is selected from 0, 1, 2, 3 or 4;

or $R^5$, $R^{14}$ together with the fragment to which $R^5$, $R^{14}$ are attached form

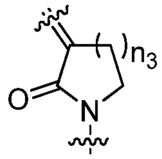

,

$n_3$ is selected from 0, 1, 2, 3 or 4;

or $R^6$, $R^{13}$ together with the fragment to which $R^6$, $R^{13}$ are attached form

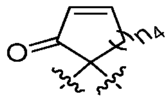

,

$n_4$ is selected from 0, 1, 2, 3 or 4;

further, $R^5$, $R^6$ are independently selected from hydrogen, halogen, C1~C3 alkyl, 5- to 6-membered heteroaryl, -C(O)$R^{16}$, -S(O)$_2R^{16}$ or -P(O)$R^{16}R^{17}$, wherein the alkyl, heteroaryl are optionally substituted by one or more $R^{18}$; each occurrence of $R^{18}$ is independently selected from halogen, C1~C3 alkyl, -$OR^a$ or -$NR^bR^c$;

or $R^5$, $R^6$ together with the atoms to which $R^5$, $R^6$ are attached form

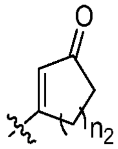

,

$n_2$ is selected from 1, 2 or 3;

or $R^5$, $R^{14}$ together with the fragment to which $R^5$, $R^{14}$ are attached form

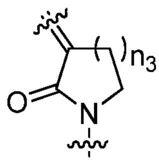

,

$n_3$ is selected from 1, 2 or 3;

or $R^6$, $R^{13}$ together with the fragment to which $R^6$, $R^{13}$ are attached form

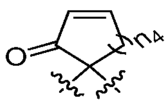

,

$n_4$ is selected from 1, 2 or 3;

further, $R^5$, $R^6$ are independently selected from H, F, -C(O)$CH_3$, -C(O)$CH_2CH_2CH_3$, -$CH_2N(CH_3)_2$, -$CH_2F$, -S(O)$_2CH_3$, -P(O)$(CH_3)_2$ or

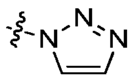
;

or $R^5$, $R^6$ together with the atoms to which $R^5$, $R^6$ are attached form

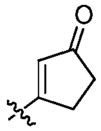 or 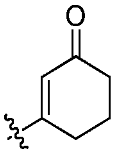,

or $R^5$, $R^{14}$ together with the fragment to which $R^5$, $R^{14}$ are attached form

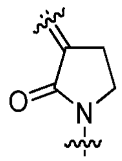,

or $R^6$, $R^{13}$ together with the fragment to which $R^6$, $R^{13}$ are attached form

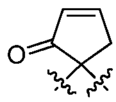;

further, $R^5$, $R^6$ are independently selected from H, F, $-C(O)CH_3$, $-C(O)CH_2CH_2CH_3$, $-CH_2F$ or

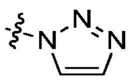;

further, $R^5$ is selected from H or F, $R^6$ is selected from H, $-C(O)CH_3$, $-C(O)CH_2CH_2CH_3$, $-CH_2F$ or

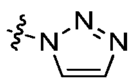;

further, the configuration of

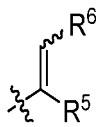 is 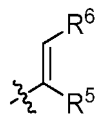;

In some embodiments of the present disclosure, $R^5$, $R^6$ are independently selected from hydrogen, halogen, C1~C6 alkyl, C3~C6 cycloalkyl, 3- to 6-membered aliphatic heterocyclyl, 6- to 10-membered aryl, 5- to 10-membered heteroaryl, $-C(O)R^{16}$, $-S(O)R^{16}$, $-S(O)_2R^{16}$, $-P(O)R^{16}R^{17}$, $-C(O)NR^bR^c$, $-C(O)OR^a$, $-OR^a$ or $-NR^bR^c$, wherein the alkyl, cycloalkyl, aliphatic heterocyclyl, aryl, heteroaryl are optionally substituted by one or more $R^{18}$;

each occurrence of $R^{18}$ is independently selected from halogen, cyano, C1~C6 alkyl, C3~C6 cycloalkyl, $-OR^a$ or $-NR^bR^c$;

or $Z^1$ is a bond, $R^5$, $R^6$ together with the atoms to which $R^5$, $R^6$ are attached form a 6- to 10-membered aryl, wherein the aryl is optionally substituted by one or more substituents selected from the group consisting of: halogen, cyano,

hydroxy, amino, C1~C6 alkyl, C3~C6 cycloalkyl and 3- to 6-membered aliphatic heterocyclyl;

further, $R^5$, $R^6$ are independently selected from hydrogen, halogen, C1~C3 alkyl, 5- to 6-membered heteroaryl, -C(O)$R^{16}$, -S(O)$_2R^{16}$, -P(O)$R^{16}R^{17}$, -C(O)N$R^bR^c$, -C(O)O$R^a$ or -O$R^a$, wherein the alkyl, heteroaryl are optionally substituted by one or more $R^{18}$;

further, $R^5$, $R^6$ are independently selected from hydrogen, halogen, C1~C3 alkyl, 5- to 6-membered heteroaryl, -C(O)$R^{16}$, -S(O)$_2R^{16}$, -P(O)$R^{16}R^{17}$, -C(O)O$R^a$ or -O$R^a$, wherein the alkyl, heteroaryl are optionally substituted by one or more $R^{18}$;

each occurrence of $R^{18}$ is independently selected from halogen, C1~C3 alkyl, -O$R^a$ or -N$R^bR^c$;

or $Z^1$ is a bond, $R^5$, $R^6$ together with the atoms to which $R^5$, $R^6$ are attached form a phenyl, wherein the phenyl is optionally substituted by 1~2 following substituents: halogen, cyano, hydroxy, amino, C1~C3 alkyl;

further, $R^5$, $R^6$ are independently selected from hydrogen, halogen, C1~C3 alkyl, -C(O)$R^{16}$, -S(O)$_2R^{16}$, -P(O)$R^{16}R^{17}$, -C(O)O-C1~C3 alkyl, -O-C1~C3 alkyl, 1H-1,2,3-triazolyl or oxazolyl, wherein the alkyl, 1H-1,2,3-triazolyl, oxazolyl are optionally substituted by 1~3 $R^{18}$;

each occurrence of $R^{18}$ is independently selected from halogen, C1~C3 alkyl, -O$R^a$ or -N$R^bR^c$;

or $Z^1$ is a bond, $R^5$, $R^6$ together with the atoms to which $R^5$, $R^6$ are attached form a phenyl substituted by one hydroxy;

further, $R^5$, $R^6$ are independently selected from H, F, CN, $-CH_2OCH_3$, $-C(CH_3)_3$, $-CH(OH)CH_3$, $C(O)CF_3$, $-C(O)CH_2CH_3$, $-C(O)CH_3$, $-C(O)NHCH_3$, $-C(O)CH_2CH_2CH_3$, $-CH_2N(CH_3)_2$, $-CH_2F$, $-S(O)_2CH_3$, $-P(O)(CH_3)_2$,

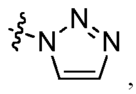

,

$-CHCH_3CH_3$, $-CH_2OCH_3$, $CF_3$,

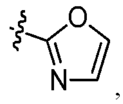

,

$-C(O)OCH_3$ or $-OCH_3$;

further, $R^5$, $R^6$ are independently selected from H, F, $-C(O)CH_3$, $-C(O)CH_2CH_2CH_3$, $-CH_2N(CH_3)_2$, $-CH_2F$, $-S(O)_2CH_3$, $-P(O)(CH_3)_2$,

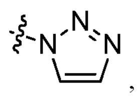

,

$-CHCH_3CH_3$, $-CH_2OCH_3$, $CF_3$,

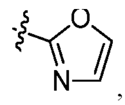

,

$-C(O)OCH_3$ or $-OCH_3$;

or $Z^1$ is a bond, $R^5$, $R^6$ together with the atoms to which $R^5$, $R^6$ are attached form

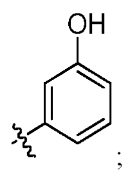

;

further, $R^5$ is selected from H, F, CN, $-CH(CH_3)_2$, $CH(OH)CH_3$, $-CH_2OCH_3$, $-OCH_3$ or $-C(O)CH_3$, $R^6$ is selected from H, $-C(CH_3)_3$, $-C(O)CH_3$, $-C(O)CH_2CH_2CH_3$, $-C(O)NHCH_3$, $-CH_2N(CH_3)_2$, $-CH_2F$,

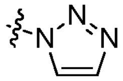,

$-CH_2OCH_3$, $CF_3$,

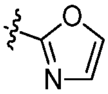,

$-C(O)OCH_3$, $-P(O)(CH_3)$, $-S(O)_2CH_3$, $-C(O)CF_3$ or $-C(O)CH_2CH_3$.

further, $R^5$ is selected from H, F, $-CHCH_3CH_3$, $-CH_2OCH_3$ or $-OCH_3$, $R^6$ is selected from H, $-C(O)CH_3$, $-C(O)CH_2CH_2CH_3$, $-CH_2F$,

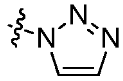,

$-CH_2OCH_3$, $CF_3$,

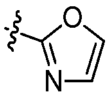

or $-C(O)OCH_3$.

In some embodiments, $Z^1$ is a bond; $R^5$ is H; $R^6$ is selected from H, $-C(CH_3)_3$, $-C(O)CH_3$, $-C(O)CH_2CH_2CH_3$, $-C(O)NHCH_3$, $-CH_2N(CH_3)_2$, $-CH_2F$,

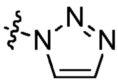,

$-CH_2OCH_3$, $CF_3$,

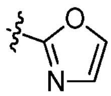,

$-C(O)OCH_3$, $-P(O)(CH_3)$, $-S(O)_2CH_3$ or $-C(O)CF_3$; preferably, $R^6$ is selected from H and $-C(O)CH_3$; further preferably,

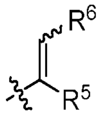 is 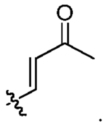.

In some embodiments of the present disclosure, each occurrence of $R^a$, $R^b$, $R^c$ is independently selected from H, C1-C6 alkyl or $-C(O)R^{13}$, wherein the alkyl is optionally substituted by one or more substituents selected from the group consisting of: halogen, cyano, hydroxy, amino and C1~C6 alkyl;

further, each occurrence of $R^a$, $R^b$, $R^c$ is independently selected from H or C1~C3 alkyl, wherein the alkyl is optionally substituted by one or more of the following substituents: halogen, C1~C3 alkyl;

further, each occurrence of $R^a$, $R^b$, $R^c$ is independently selected from H or C1~C3 alkyl, preferably H or methyl;

more preferably methyl.

In some embodiments of the present disclosure, $R^2$ is selected from hydrogen, halogen, cyano, C1~C6 alkyl, C3~C6 cycloalkyl, $-OR^d$ or $-NR^eR^f$, wherein the alkyl, cycloalkyl are optionally substituted by one or more $R^{14}$, each occurrence of $R^{14}$ is independently selected from halogen, cyano, C1~C6 alkyl, C3~C6 cycloalkyl, $-OR^d$ or $-NR^eR^f$; further, $R^2$ is selected from hydrogen, halogen, cyano, C1~C3 alkyl, $-OR^d$ or $-NR^eR^f$, wherein the alkyl is optionally substituted by one or more $R^{14}$, each occurrence of $R^{14}$ is independently selected from halogen, cyano, C1~C3 alkyl, $-OR^d$ or $-NR^eR^f$;
further, $R^2$ is hydrogen.

In some embodiments of the present disclosure, each occurrence of $R^d$, $R^e$, $R^f$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino, C1~C6 alkyl, C3~C6 cycloalkyl and alkyl substituted or unsubstituted C3~C6 heterocycloalkyl;
further, each occurrence of $R^d$, $R^e$, $R^f$ is independently selected from H or C1~C3 alkyl, wherein the alkyl is optionally substituted by one or more substituents selected from the group consisting of: halogen, C1~C3 alkyl and alkyl substituted or unsubstituted 5-membered nitrogen-containing heterocycloalkyl.

In some embodiments of the present disclosure, each occurrence of $R^d$, $R^e$, $R^f$ is independently selected from H, C1~C6 alkyl or C3~C6 cycloalkyl, wherein the alkyl, cycloalkyl are optionally substituted by one or more of the following substituents: halogen, cyano, hydroxy, amino, C1~C6 alkyl, C3~C6 cycloalkyl, C3~C6 heterocycloalky; further, each occurrence of $R^d$, $R^e$, $R^f$ is independently selected from H or C1~C3 alkyl, wherein the alkyl is optionally substituted by one or more of the following substituents: halogen, C1~C3 alkyl, 5-membered nitrogen-containing heterocycloalkyl;
further, each occurrence of $R^d$, $R^e$, $R^f$ is independently selected from H or C1~C3 alkyl, the alkyl is optionally substituted by one or more of the following substituents: halogen, C1~C3 alkyl,

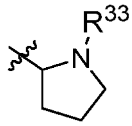
,

$R^{33}$ is selected from H or C1~C6 alkyl, preferably C1~C3 alkyl, more preferably methyl.

In some embodiments of the present disclosure, $R^3$ is selected from hydrogen, halogen, cyano, C1~C6 alkyl, C3~C6 cycloalkyl, 3- to 6-membered aliphatic heterocyclyl, $-OR^g$, $-C(O)OR^g$, $-SR^g$ or $-NR^hR^i$, when the ‘ ----- ’ in ring E is absent, $R^3$ can also be a carbonyl group that together formed by the connected carbon atom, wherein the alkyl, cycloalkyl, aliphatic heterocyclyl are optionally substituted by one or more $R^{25}$, each occurrence of the $R^{25}$ is independently selected from halogen, cyano, C1~C6 alkyl, C3~C6 cycloalkyl, $-OR^9$ or $-NR^hR^i$;

further, $R^3$ is selected from hydrogen, halogen, cyano, C1~C6 alkyl, C3~C6 cycloalkyl, 3- to 6-membered aliphatic heterocyclyl, $-OR^g$ or $-NR^hR^i$, when the ’-----’ in ring E is absent, $R^3$ can also be a carbonyl group that together formed by the connected carbon atom, wherein the alkyl, cycloalkyl, aliphatic heterocyclyl are optionally substituted by one or more $R^{25}$, each occurrence of the $R^{25}$ is independently selected from halogen, cyano, C1~C6 alkyl, C3~C6 cycloalkyl, $-OR^g$ or $-NR^hR^i$;
further, $R^3$ is selected from hydrogen, halogen, cyano, C1~C6 alkyl, $-OR^g$, $-C(O)OR^g$, $-SR^g$ or $-NR^hR^i$, when the ’-----’ in ring E is absent, $R^3$ can also be a carbonyl group that together formed by the connected carbon atom, wherein the alkyl is optionally substituted by one or more halogen or -OH;
further, $R^3$ is selected from hydrogen, halogen, C1~C3 alkyl, $-OR^8$ or $-NR^hR^i$, when the ’-----’ in ring E is absent, $R^3$ can also be a carbonyl group that together formed by the connected carbon atom, wherein the alkyl is optionally substituted by one or more $R^{25}$, each occurrence of $R^{25}$ is independently selected from halogen, cyano, C1~C3 alkyl, $-OR^g$ or $-NR^hR^i$;
further, $R^3$ is selected from hydrogen, F, Cl, Br, methyl, $-OR^g$ or $-NR^hR^i$, when the ’-----’ in ring E is absent, $R^3$ forms carbonyl group together with the connected carbon atom;
further, $R^3$ is selected from hydrogen, Cl, methyl or $-OR^g$, when the ’-----’ in ring E is absent, $R^3$ forms carbonyl group together with the connected carbon atom;
further, $R^3$ is selected from hydrogen, Cl or $-OR^g$;
further, $R^3$ is selected from hydrogen, Cl, $-CF_3$, $-OCH_3$, $-OCH_2CH_3$, $-OCF_3$, $-OCHF_2$, $-N(CH3)_2$, $-CH_3$, $-CH_2OH$, $-OCH_2CF_3$, -OH, $-NHCH_3$, $-SCH_3$, $-OCD_3$, -CN or $-C(O)OCH_3$.

Further, $R^3$ is selected from hydrogen, $-OCH_3$, $-OCH_2CH_3$, $-N(CH3)_2$, $-CH_2OH$, $-OCH_2CF_3$, $-SCH_3$, $-OCD_3$,

-CN or - C(O)$OCH_3$; preferably, $R^3$ is -$OCH_3$.

In some embodiments of the present disclosure, each occurrence of $R^g$, $R^h$, $R^i$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino and C1~C6 alkyl;

further, each occurrence of $R^g$, $R^h$, $R^i$ is independently selected from H, C1~C3 alkyl, cyclopropyl or cyclopentyl, wherein the alkyl, cyclopropyl are optionally substituted by one or more of the following substituents: halogen, C1~C3 alkyl;

further, each occurrence of $R^g$, $R^h$, $R^i$ is independently selected from H, methyl, cyclopropyl or cyclopentyl, preferably methyl.

In some embodiments of the present disclosure, each occurrence of $R^g$, $R^h$, $R^i$ is independently selected from H, methyl, cyclopropyl, cyclopentyl, ethyl, trifluoromethyl, trifluoroethyl, difluoromethyl or deuterated methyl.

In some embodiments of the present disclosure, $R^g$, $R^h$, $R^i$ is independently selected from H, methyl, cyclopropyl, cyclopentyl or ethyl;

preferably, each occurrence of $R^g$, $R^h$, $R^i$ is independently selected from H, methyl, cyclopropyl or ethyl.

In some embodiments of the present disclosure, $R^8$, $R^9$, $R^{10}$, $R^{29}$ are independently selected from hydrogen, halogen, cyano, C1~C6 alkyl, C3~C6 cycloalkyl, -$OR^q$ or -$NR^rR^s$, wherein the alkyl, cycloalkyl are optionally substituted by one or more $R^{31}$;

each occurrence of $R^{31}$ is independently selected from halogen, cyano, C1~C6 alkyl, C3~C6 cycloalkyl, -$OR^q$, -$NR^rR^s$, -C(O)$R^{15}$, -C(O)$NR^rR^s$ or -C(O)$OR^q$; further, $R^8$, $R^9$, $R^{10}$, $R^{29}$ are independently selected from hydrogen, halogen, cyano, C1~C3 alkyl or -$OR^q$, wherein the alkyl, cycloalkyl are optionally substituted by one or more $R^{31}$;

each occurrence of $R^{31}$ is independently selected from halogen, C1~C3 alkyl or -$OR^q$;

further, $R^8$, $R^{29}$ are independently selected from hydrogen, F, Cl, Br, C1~C3 alkyl, halogenated C1~C3 alkyl, -CN or -$OR^q$, preferably hydrogen, Cl or OH;

$R^9$, $R^{10}$ is hydrogen;

further, $R^8$, $R^9$, $R^{10}$, $R^{29}$ are independently selected from hydrogen, halogen, C1~C3 alkyl or -$OR^q$, wherein the alkyl, cycloalkyl are optionally substituted by one or more $R^{31}$;

each occurrence of $R^{31}$ is independently selected from halogen, C1~C3 alkyl or -$OR^q$;

further, $R^8$, $R^{29}$ are independently selected from hydrogen, F, Cl, Br, C1~C3 alkyl or -$OR^q$, preferably hydrogen, Cl, OH;

$R^9$, $R^{10}$ is hydrogen; further, $R^8$ is selected from hydrogen, Cl or -$CH_3$; preferably, $R^8$ is selected from hydrogen or Cl, preferably hydrogen; $R^{29}$ is selected from hydrogen, OH, -CN, For -$CF_3$, preferably, $R^{29}$ is selected from hydrogen, OH, -CN or F; preferably, $R^{29}$ is selected form hydrogen or OH, preferably hydrogen.

In some embodiments of the present disclosure, each occurrence of $R^q$, $R^r$, $R^s$ is independently selected from H, C1~C6 alkyl or C3~C6 cycloalkyl, wherein the alkyl, cycloalkyl are optionally substituted by one or more of the following substituents: halogen, cyano, hydroxy, amino, C1~C6 alkyl;

further, each occurrence of $R^q$, $R^r$, $R^s$ is independently selected from H or C1~C3 alkyl, preferably H.

In some embodiments of the present disclosure, $R^4$ is selected from C1~C6 alkyl, C3~C9 cycloalkyl, 3- to 6-membered heterocycloalkyl, 6- to 10-membered aryl or 5- to 10-membered heteroaryl, wherein the alkyl, cycloalkyl, heterocycloalkyl, aryl, heteroaryl are optionally substituted by one or more $R^{26}$;

each occurrence of $R^{26}$ is independently selected from halogen, cyano, C1~C6 alkyl, C3~C6 cycloalkyl, -$OR^j$, -$NR^kR^m$, -C(O)$R^{27}$, -C(O)$NR^kR^m$ or -C(O)$OR^j$, wherein the alkyl, cycloalkyl are optionally substituted by one or more $R^{28}$;

each occurrence of $R^{28}$ is independently selected from halogen, cyano, alkyl, cycloalkyl, -$OR^j$ or -$NR^kR^m$;

further, $R^4$ is selected from C1~C3 alkyl, C3~C6 cycloalkyl, phenyl, 5- to 6-membered nitrogen-containing heteroaryl or 5- to 6-membered sulphur-containing heteroaryl, wherein the alkyl, cycloalkyl, aryl, nitrogen-containing heteroaryl, sulphur-containing heteroaryl are optionally substituted by 1~3 $R^{26}$;

each occurrence of $R^{26}$ is independently selected from halogen, cyano, C1~C3 alkyl, C3~C6 cycloalkyl, -$OR^j$ or -$NR^kR^m$;

In some embodiments, $R^4$ is selected from C1~C3 alkyl, C3~C6 cycloalkyl, phenyl, naphthyl, 5- to 6-membered nitrogen-containing heteroaryl or 5- to 6-membered sulphur-containing heteroaryl, wherein the alkyl, cycloalkyl, aryl, nitrogen-containing heteroaryl, sulphur-containing heteroaryl are optionally substituted by 1~3 $R^{26}$;

each occurrence of $R^{26}$ is independently selected from halogen, cyano, C1~C3 alkyl, halogenated C1~C3 alkyl, -

O(C1~C3 alkyl) or -C(O)OH.

In some embodiments, $R^4$ is selected from C1~C3 alkyl, cyclohexyl, phenyl, pyridyl, thienyl, naphthyl, pyrrolyl or thiazolyl, wherein the alkyl, cyclohexyl, phenyl, pyridyl, thienyl, naphthyl, pyrrolyl, thiazolyl are optionally substituted by 1~2 $R^{26}$;

each occurrence of $R^{26}$ is independently selected from F, Cl, Br, cyano, unsubstituted C1~C3 alkyl or C1~C3 alkyl substituted by 1 to 3 halogen, -$OR^j$ or -C(O)$OR^j$, more preferably F, Cl, methyl, methoxy, cyano, trifluoromethyl or - COOH;

preferably, each occurrence of $R^{26}$ is independently selected from F, Cl, Br, cyano, unsubstituted C1~C3 alkyl or C1~C3 alkyl substituted by 1 to 3 halogen or -$OR^j$, more preferably F, Cl, methyl, methoxy, cyano or trifluoromethyl.

In some embodiments, $R^4$ is selected from C1~C3 alkyl, cyclohexyl, phenyl, pyrrolyl or thiazolyl, wherein the alkyl, cyclohexyl, phenyl, pyrrolyl, thiazolyl are optionally substituted by 1~2 $R^{26}$; each occurrence of $R^{26}$ is independently selected from F, Cl, Br, C1~C3 alkyl, -$OR^j$, preferably F, Cl, methyl or methoxy.

In some embodiments, $R^4$ is selected from C1~C3 alkyl, cyclohexyl, phenyl, pyrrolyl or thiazolyl, wherein the alkyl, cyclohexyl, phenyl, pyrrolyl, thiazolyl are optionally substituted by 1-2 $R^{26}$;
each occurrence of $R^{26}$ is independently selected from F, Cl, Br, C1~C3 alkyl or -$OR^j$, preferably F, Cl, methyl or methoxy;

In some embodiments of the present disclosure, $R^4$ is selected from methyl, cyclohexyl,

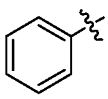 , 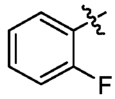 ,

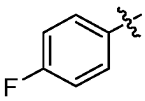 , 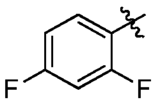 , 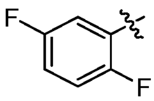 , 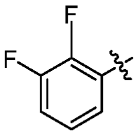 , 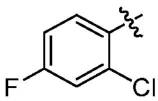 , 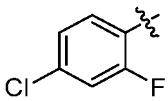 , 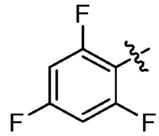 ,

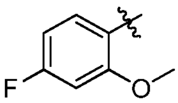 , 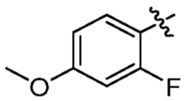 , 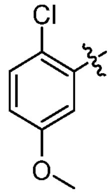 , 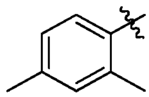 , 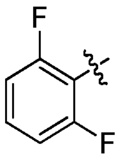 , 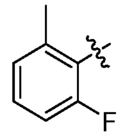 , 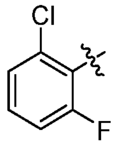 ,

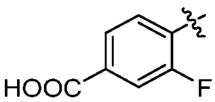 , 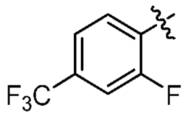 , 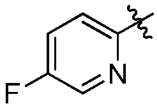 , 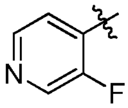 , 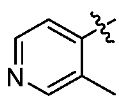 , 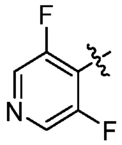 ,

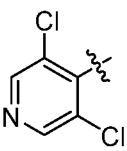 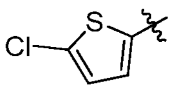 , 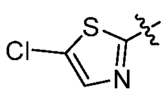 , 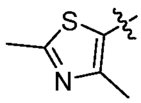 or 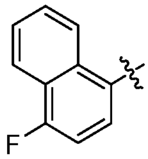 .

Further, $R^4$ is selected from methyl, cyclohexyl,

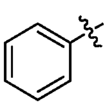 , 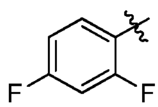 , 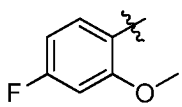 , 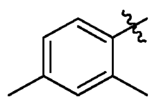 ,

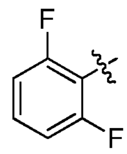 , 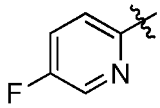 , 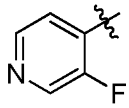 or 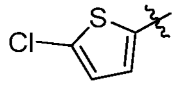 ,

preferably methyl, cyclohexyl,

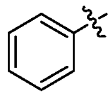 ,

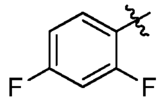 , 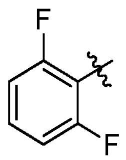 or 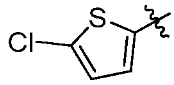 ,

more preferably

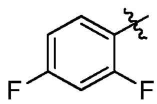 or 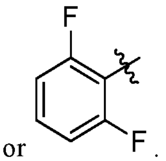 .

In some embodiments of the present disclosure, , $R^4$ is selected from

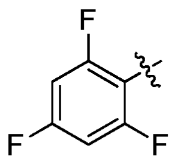 , 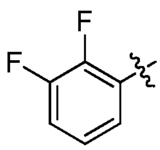 , 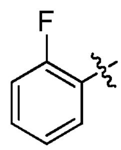 ,

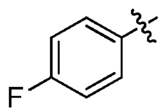 , 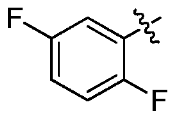 , 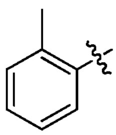 , 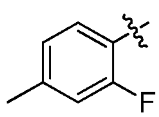 , 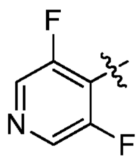 or 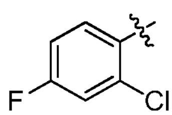 .

In some embodiments of the present disclosure, each occurrence of $R^{26}$ is independently selected from halogen, cyano, C1~C3 alkyl, C3~C6 cycloalkyl, $-OR^j$ or $-NR^kR^m$, wherein the alkyl, cycloalkyl are optionally substituted by 1~3 $R^{28}$;

each occurrence of $R^{28}$ is independently selected from halogen, cyano, C1~C3 alkyl, $-OR^j$ or $-NR^kR^m$;
further, each occurrence of $R^{26}$ is independently selected from F, Cl, Br, cyano, unsubstituted C1~C3 alkyl or C1~C3 alkyl substituted by 1 to 3 halogen, $-OR^j$, more preferably F, Cl, methyl, methoxy, cyano or trifluoromethyl;
further, $R^4$ is selected from

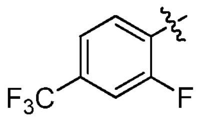, 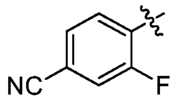 or 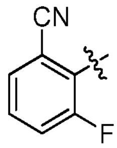.

In some embodiments of the present disclosure, $R^4$ is selected from naphthyl or pyrrolyl, wherein the naphthyl, pyrrolyl are optionally substituted by 1~3 $R^{26}$; $R^{26}$ is as defined above;

further, the $R^4$ is selected from unsubstituted following group or following group substituted by 1 to 3 $R^{26}$:

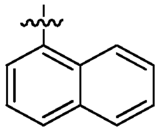, 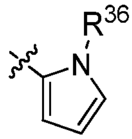,

$R^{36}$ is selected from H or C1~C6 alkyl, preferably C1~C3 alkyl, more preferably methyl;

further, the $R^4$ is selected from

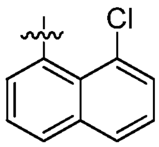 or 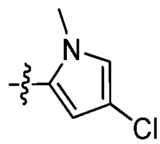.

In some embodiments of the present disclosure, each occurrence of $R^j$, $R^k$, $R^m$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino and C1~C6 alkyl;
further, each occurrence of $R^j$, $R^k$, $R^m$ is independently selected from H or C1~C3 alkyl, preferably C1~C3 alkyl, more preferably methyl.

In some embodiments of the present disclosure, each occurrence of $R^7$ is independently selected from halogen, cyano, C1~C6 alkyl, $-OR^n$, $-NR^oR^p$, $-C(O)R^{20}$, $-C(O)NR^oR^p$ or $-C(O)OR^n$, wherein the alkyl is optionally substituted by one or more $R^{21}$;

each occurrence of $R^{21}$ is independently selected from halogen, cyano, C1~C6 alkyl, $-OR^n$, $-NR^oR^p$, $-C(O)R^{20}$, $-C(O)NR^oR^p$ or $-C(O)OR^n$;
further, each occurrence of $R^7$ is independently selected from halogen, cyano, C1~C3 alkyl, $-OR^n$ or $-NR^oR$, wherein the alkyl is optionally substituted by one or more $R^{21}$;
each occurrence of $R^{21}$ is independently selected from halogen, cyano, C1~C3 alkyl, $-OR^n$ or $-NR^oR^p$;
further, each occurrence of $R^7$ is independently selected from C1~C3 alkyl, wherein the alkyl is optionally substituted by one or more $R^{21}$;
each occurrence of $R^{21}$ is independently selected from halogen, cyano or C1~C3 alkyl, preferably cyano;
further, $R^7$ is selected from hydrogen, methyl or $-CH_2CN$;
further,

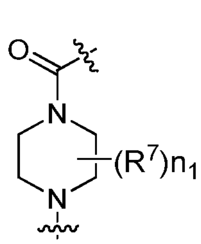

is selected from

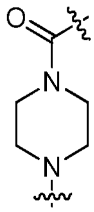 , 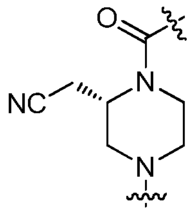 , 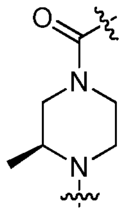 or 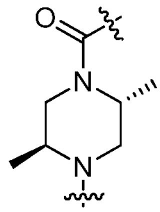 ;

preferably,

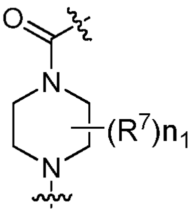 is 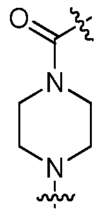 .

In some embodiments of the present disclosure, each occurrence of $R^{7'}$ is independently selected from halogen, cyano, C1~C6 alkyl, $-OR^n$, $-NR^oR^p$, $-C(O)R^{20}$ or $-C(O)OR^n$, wherein the alkyl is optionally substituted by 1~3 $R^{21}$; each occurrence of $R^{21}$ is independently selected from halogen, cyano, C1~C6 alkyl, $-OR^n$ or $-NR^oR^p$;

further, each occurrence of $R^{7'}$ is independently selected from halogen, cyano, C1~C3 alkyl, $-OR^n$ or $-NR^oR$, wherein the alkyl is optionally substituted by 1~2 $R^{21}$;
each occurrence of $R^{21}$ is independently selected from halogen, cyano, C1~C3 alkyl, $-OR^n$ or $-NR^oR^p$;
further, $n_5$ is selected from 0, 1, 2 or 3, preferably 0, 1 or 2, more preferably 0 or 1.

In some embodiments of the present disclosure, each occurrence of $R^n$, $R^o$, $R^p$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino and C1~C6 alkyl;
further, each occurrence of $R^n$, $R^o$, $R^p$ is independently selected from H or C1~C3 alkyl.
In some embodiments of the present disclosure, each occurrence of $R^{16}$, $R^{17}$, $R^{19}$, $R^{20}$, $R^{22}$, $R^{24}$, $R^{27}$, $R^{30}$, $R^{32}$ is independently selected from H or C1~C6 alkyl, wherein the alkyl is optionally substituted by one or more substituents selected from the group consisting of: halogen, cyano, hydroxy, amino and C1~C6 alkyl;

further, each occurrence of $R^{16}$, $R^{17}$, $R^{19}$, $R^{20}$, $R^{22}$, $R^{24}$, $R^{27}$, $R^{30}$, $R^{32}$ is independently selected from H or C1~C3 alkyl, wherein the alkyl is optionally substituted by one or more substituents selected from the group consisting of: halogen, hydroxy, amino and C1~C3 alkyl;
further, each occurrence of $R^{16}$, $R^{17}$, $R^{19}$, $R^{20}$, $R^{22}$, $R^{24}$, $R^{27}$, $R^{30}$, $R^{32}$ is independently selected from C1~C3 alkyl, preferably methyl or propyl.

In some embodiments, the compound of the present disclosure has the structure shown in formula II or a tautomer, meso isomer, racemate, enantiomer, diastereomer or mixture thereof, or a pharmaceutically acceptable hydrate, solvate or salt thereof:

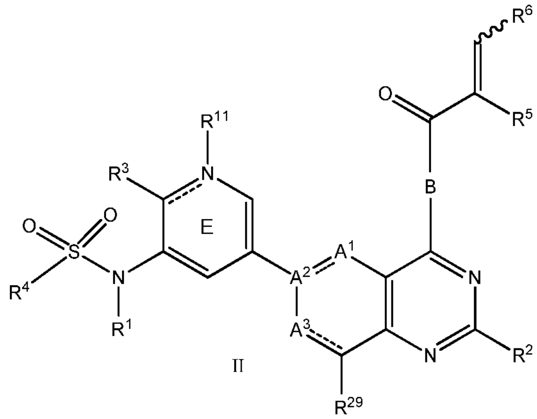

II

wherein,

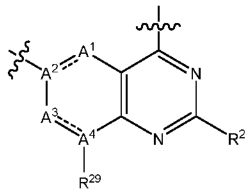 is 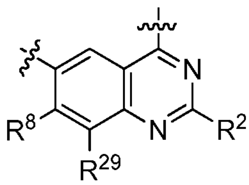 ;

B is

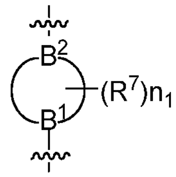

,

the ring where $B^1$, $B^2$ are located is a 6- to 9-membered nitrogen-containing heterocycloalkyl or a 6- to 9-membered nitrogen-containing heterocycloalkenyl, wherein the cycloalkyl or cycloalkenyl is a monocyclic ring, spirocyclic ring or bridged cyclic ring;

$R^1$ is H;

$R^2$ is H;

the '-----' in ring E represemts '-----' is a bond or absent, when '-----' is a bond, $R^{11}$ is absent, ring E is

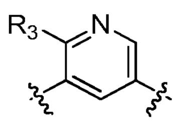

;

when ' ----- ' is absent, ring E is

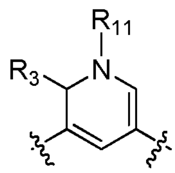

;

when $R^{11}$ exists, $R^{11}$ is selected from hydrogen or methyl;

$R^8$ is selected from hydrogen, Cl or $-CH_3$; preferably $R^8$ is hydrogen;

$R^{29}$ is selected from hydrogen, OH, -CN, For $-CF_3$; preferably $R^{29}$ is hydrogen;

$R^3$ is selected from hydrogen, halogen, cyano, C1~C6 alkyl, -$OR^g$, -C(O)$OR^g$, -$SR^g$ or -$NR^hR^i$, when the ' ----- ' in ring E is absent, $R^3$ can also be a carbonyl group that together formed by the connected carbon atom, wherein the alkyl is optionally substituted by one or more halogen or -OH;

each occurrence of $R^g$, $R^h$, $R^i$ is independently selected from H, methyl, cyclopropyl, cyclopentyl or ethyl; preferably methyl;

$R^4$ is selected from C1~C3 alkyl, C3~C6 cycloalkyl, phenyl, naphthyl, 5- to 6-membered nitrogen-containing heteroaryl or 5- to 6-membered sulphur-containing heteroaryl, wherein the alkyl, cycloalkyl, aryl, nitrogen-containing heteroaryl, sulphur-containing heteroaryl are optionally substituted by 1~3 $R^{26}$;

each occurrence of $R^{26}$ is independently selected from halogen, cyano, C1~C3 alkyl, halogenated C1~C3 alkyl, -O(C1~C3 alkyl) or -C(O)OH;

$R^5$, $R^6$ are independently selected from hydrogen, halogen, C1~C3 alkyl, 5- to 6-membered heteroaryl, -C(O)$R^{16}$, - $S(O)_2R^{16}$, -P(O)$R^{16}R^{17}$, -C(O)$NR^bR^c$, -C(O)$OR^a$ or -$OR^a$, wherein the alkyl, heteroaryl are optionally substituted by one or more $R^{18}$;

$R^{16}$, $R^{17}$ are independently selected from C1~C3 alkyl;

each occurrence of $R^a$, $R^b$, $R^c$ is independently selected from H and C1~C3 alkyl;

each occurrence of $R^{18}$ is independently selected from halogen, C1~C3 alkyl, -$OR^a$ or -$NR^bR^c$;

each occurrence of $R^7$ is independently selected from C1~C3 alkyl, wherein the alkyl is optionally substituted by one or more substituents independently selected from halogen, cyano or C1~C3 alkyl;

$n_1$ is selected from 0 or 1;

preferably,

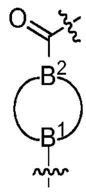

is selected from the following group:

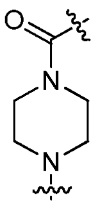, 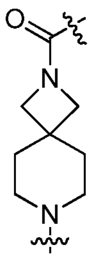, 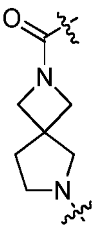, 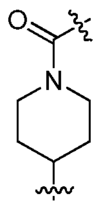, 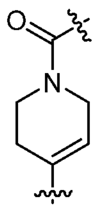 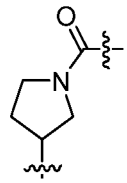, 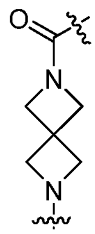, 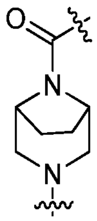, 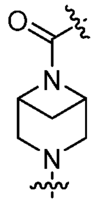, 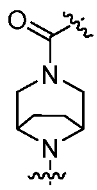,

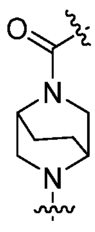 or 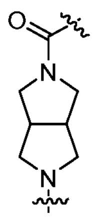.

In some embodiments, the compound of the present disclosure has the structure shown in formula VIII or a tautomer, meso isomer, racemate, enantiomer, diastereomer or mixture thereof, or a pharmaceutically acceptable hydrate, solvate or salt thereof:

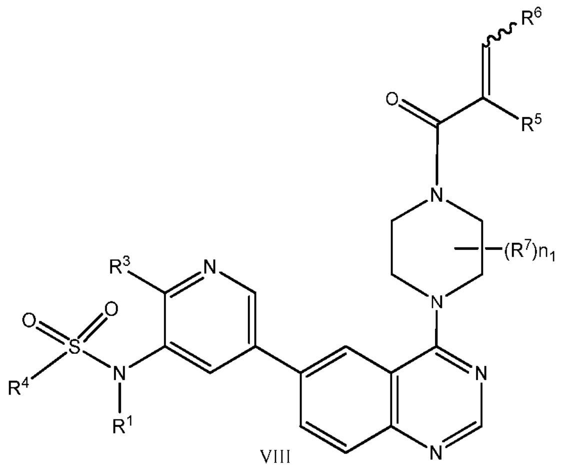

VIII

Wherein,

$R^1$ is H;
$R^3$ is selected from hydrogen, halogen, cyano, C1~C6 alkyl, $-OR^g$, $-C(O)OR^g$, $-SR^g$, $-NR^hR^i$ or wherein the alkyl is optionally substituted by one or more halogen or -OH;
each occurrence of $R^g$, $R^h$, $R^i$ is independently selected from H, methyl, cyclopropyl, cyclopentyl or ethyl; preferably methyl;
$R^4$ is selected from C1~C3 alkyl, cyclohexyl, phenyl, pyridyl, thienyl, naphthyl, pyrrolyl or thiazolyl, wherein alkyl, cyclohexyl, phenyl, pyridyl, thienyl, naphthyl, pyrrolyl, thiazolyl are optionally substituted by 1~2 $R^{26}$;
each occurrence of $R^{26}$ is independently selected from F, Cl, Br, cyano, unsubstituted C1~C3 alkyl or C1~C3 alkyl substituted by 1 to 3 halogen, $-OR^j$ or $-C(O)OR^j$;
$R^j$ is selected from H and C1~C3 alkyl;
$R^5$, $R^6$ are independently selected from hydrogen, halogen, C1~C3 alkyl, 5- to 6-membered heteroaryl, $-C(O)R^{16}$, - $S(O)_2R^{16}$, $-P(O)R^{16}R^{17}$, $-C(O)NR^bR^c$, $-C(O)OR^a$ or $-OR^a$, wherein the alkyl, heteroaryl are optionally substituted by one or more $R^{18}$;
$R^{16}$, $R^{17}$ is independently selected from C1~C3 alkyl;
each occurrence of $R^{18}$ is independently selected from halogen, C1~C3 alkyl, $-OR^a$ or $-NR^bR^c$;
each occurrence of $R^a$, $R^b$, $R^c$ is independently selected from H and C1~C3 alkyl;
each occurrence of $R^7$ is independently selected from C1~C3 alkyl, wherein the alkyl is optionally substituted by one or more substituents independently selected from halogen, cyano or C1~C3 alkyl;
$n_1$ is selected from 0 or 1.

In some embodiments, the compound of the present disclosure has the structure shown in formula VIII or a tautomer, meso isomer, racemate, enantiomer, diastereomer or mixture thereof, or a pharmaceutically acceptable hydrate, solvate or salt thereof:
wherein,

$R^1$ is H;
$R^3$ is selected from hydrogen, Cl, $-CF_3$, $-OCH_3$, $-OCH_2CH_3$, $-OCF_3$, $-OCHF_2$, $-N(CH3)_2$, $-CH_3$, $-CH_2OH$, $-OCH_2CF_3$, -OH, $-NHCH_3$, $-SCH_3$, $-OCD_3$, -CN or $-C(O)OCH_3$;
$R^4$ is selected from methyl, cyclohexyl,

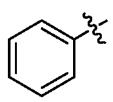 , 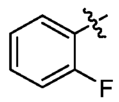 , 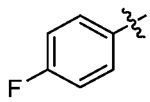 , 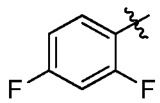 , 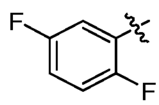 ,

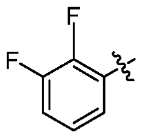, 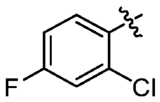, 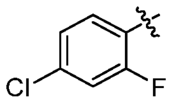, 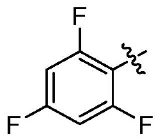, 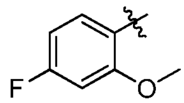, 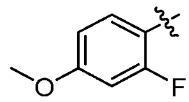, 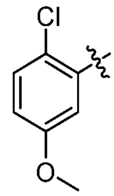,

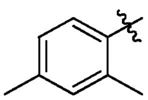, 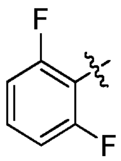, 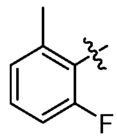, 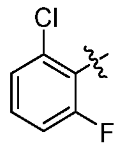, 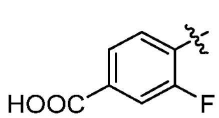, 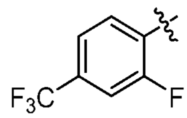, 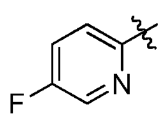,

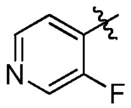, 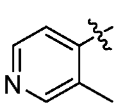, 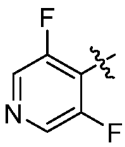, 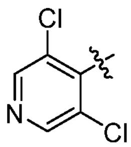 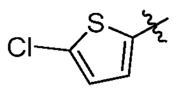, 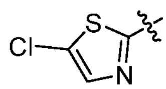, 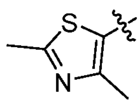 or 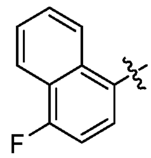;

$R^5$ is selected from H, F, CN, $-CH(CH_3)_2$, $-CH(OH)CH_3$, $-CH_2OCH_3$, $-OCH_3$ or $-C(O)CH_3$;
$R^6$ is selected from H, $-C(CH_3)_3$, $-C(O)CH_3$, $-C(O)CH_2CH_2CH_3$, $-C(O)NHCH_3$, $-CH_2N(CH_3)_2$, $-CH_2F$,

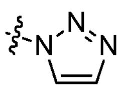,

$CH_2OCH_3$, $CF_3$,

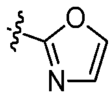,

$-C(O)OCH_3$, $-P(O)(CH_3)$, $-S(O)_2CH_3$, $-C(O)CF_3$ or $-C(O)CH_2CH_3$.
$R^7$ is selected from hydrogen, methyl or $-CH_2CN$;
$n_1$ is selected from 0 or 1.

In some embodiments of the present disclosure, the compound has the structure shown as followed:

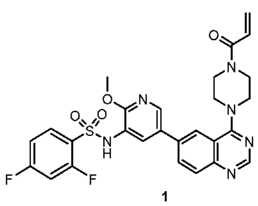
1

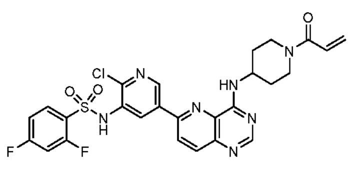
2

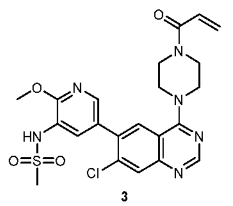
3

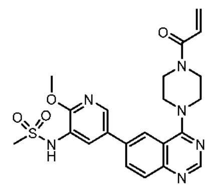
4

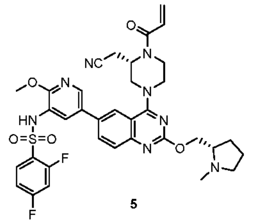
5

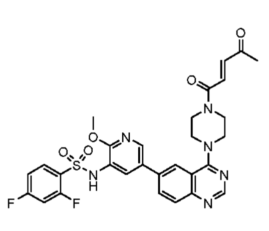
6

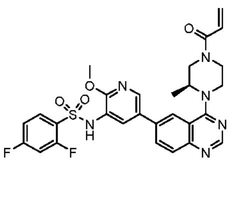
7

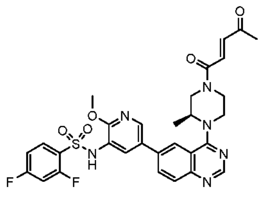
8

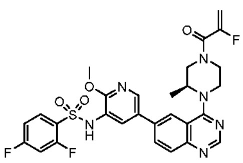
9

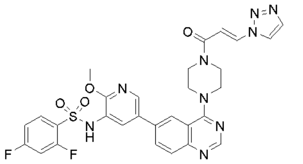
10

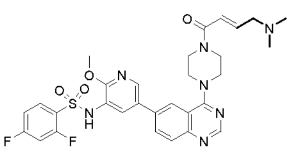
11

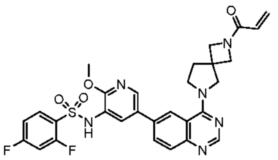
12

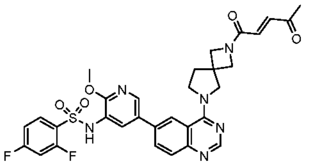
13

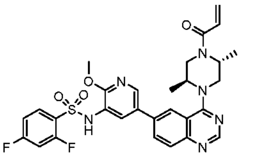
14

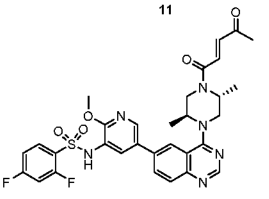
15

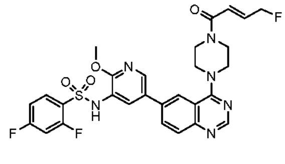
16

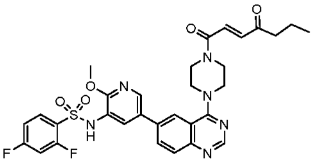
17

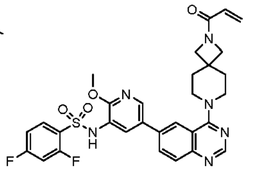
18

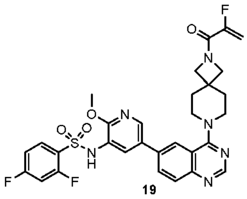
19

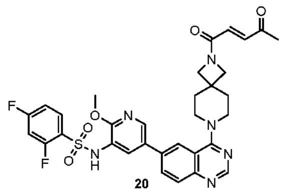
20

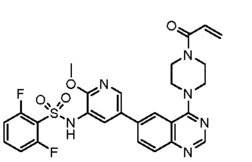
21

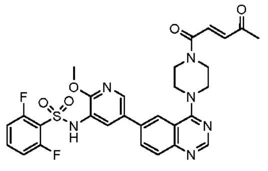
22

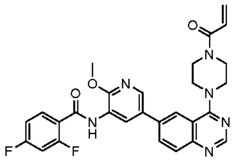
23

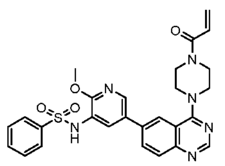
24

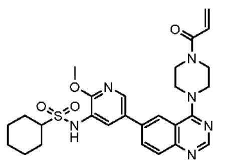
25

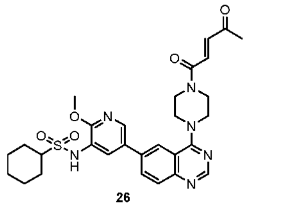
26

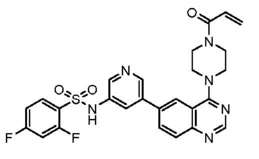
27

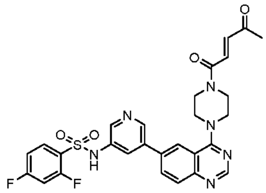
28

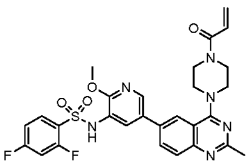
29

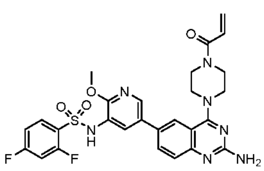
30

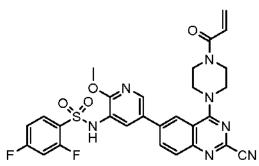
31

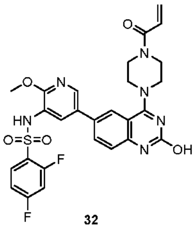
32

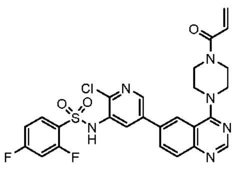
33

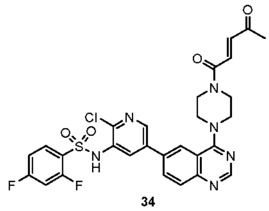
34

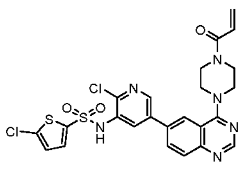
35

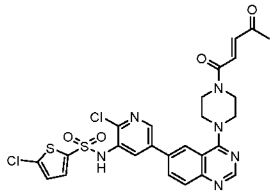
36

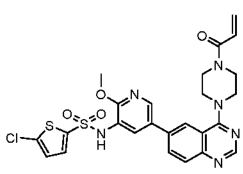
37

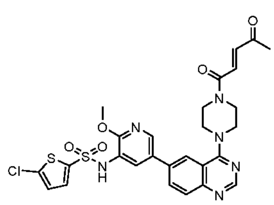
38

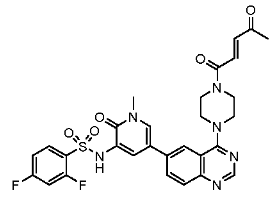
39

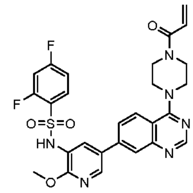
40

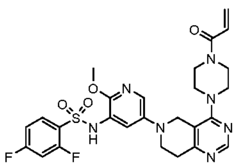
41

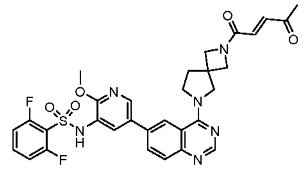
42

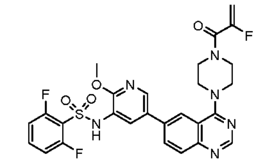
43

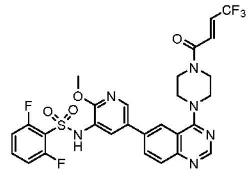
44

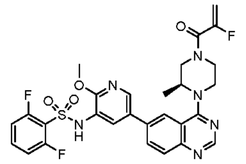
45

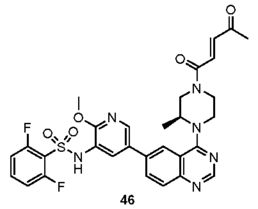
46

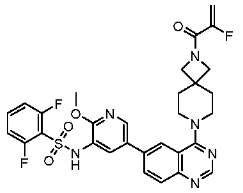
47

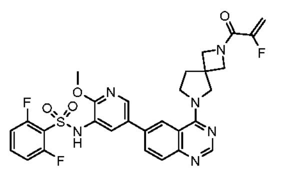
48

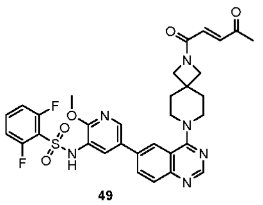
49

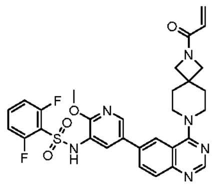
50

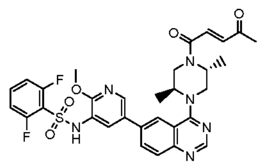
51

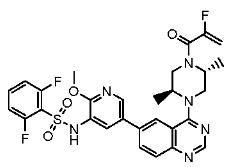
52

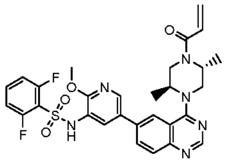
53

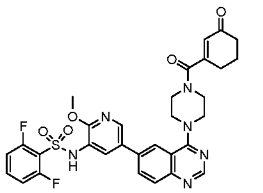
54

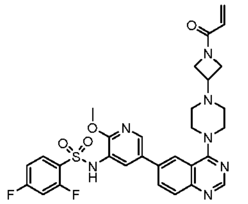
55

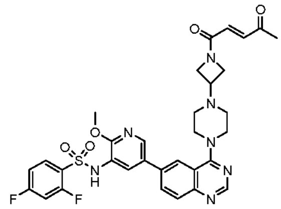
56

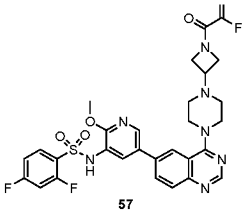
57

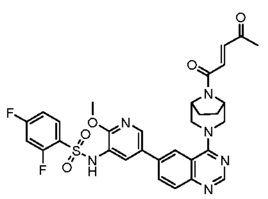
58

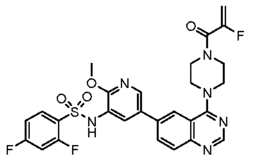
59

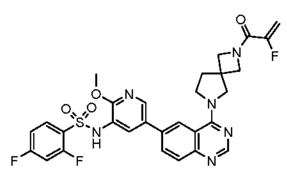
60

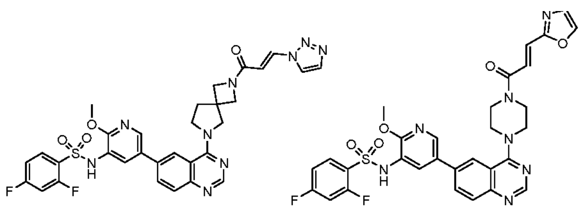

61

62

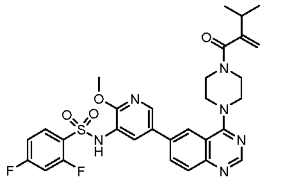

63

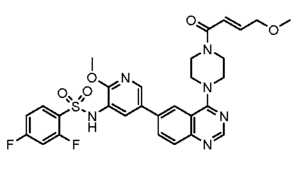

64

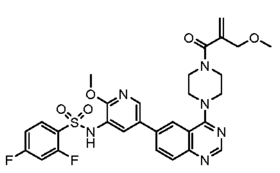

65

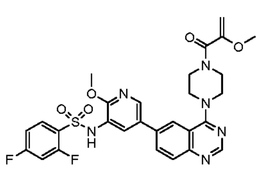

66

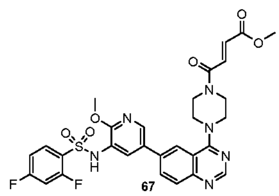

67

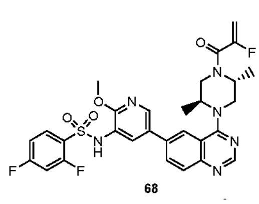

68

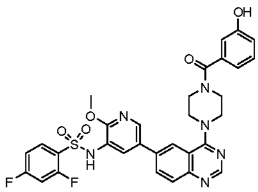

69

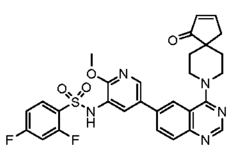

70

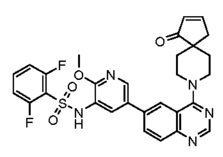

71

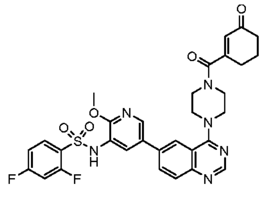

72

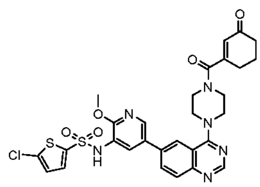

73

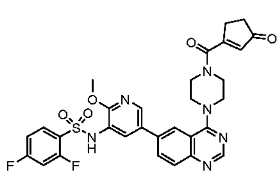

74

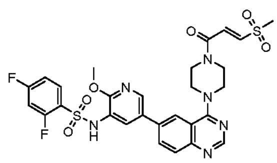

75

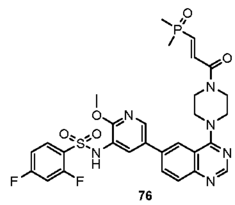

76

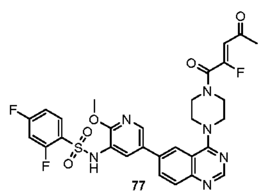

77

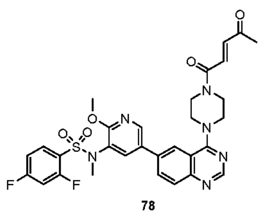

78

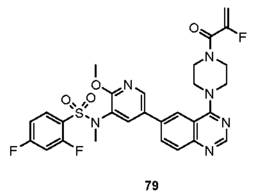

79

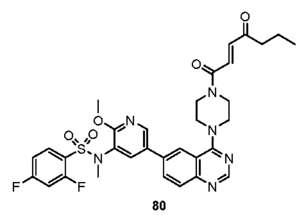

80

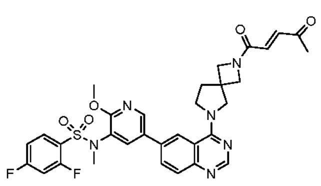

81

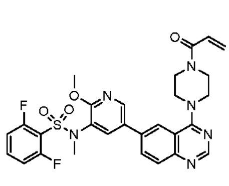

82

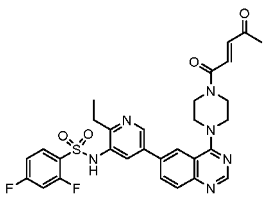

83

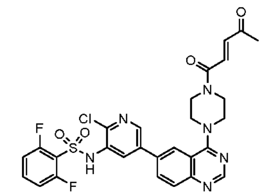

84

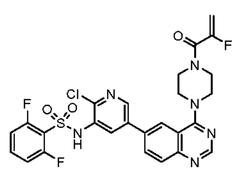
85

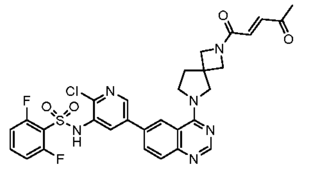
86

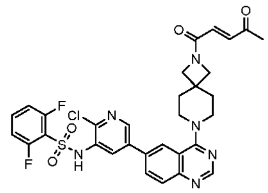
87

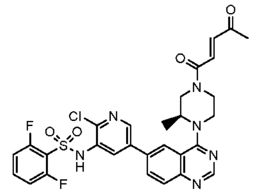
88

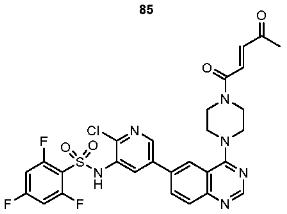
89

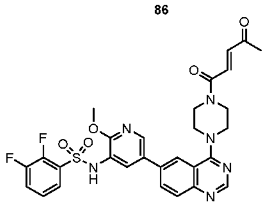
90

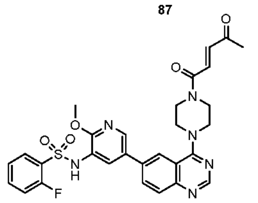
91

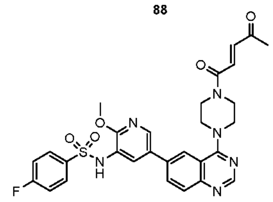
92

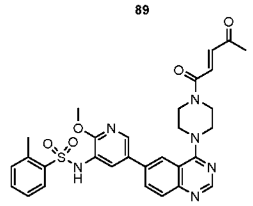
93

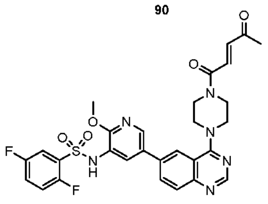
94

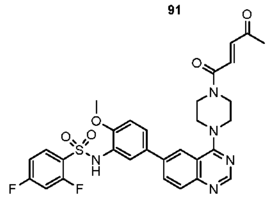
95

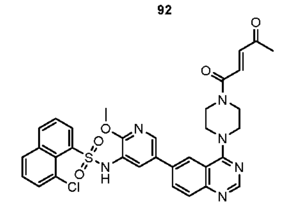
96

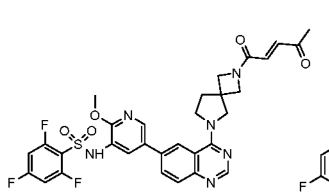
97

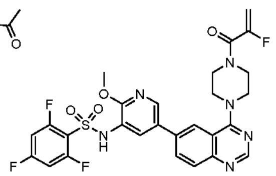
98

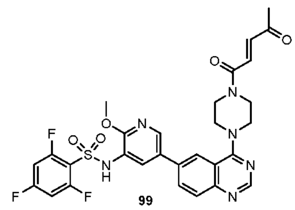
99

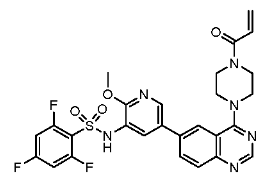
100

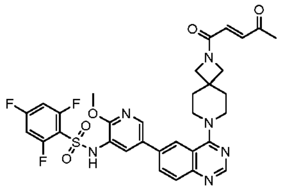
101

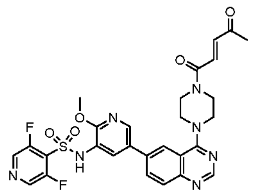
102

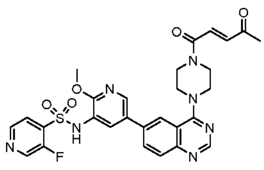
103

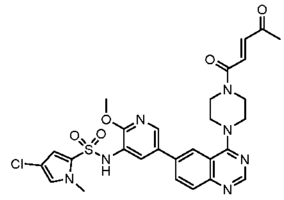
104

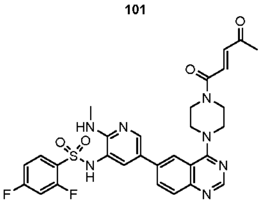
105

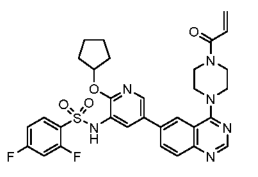
106

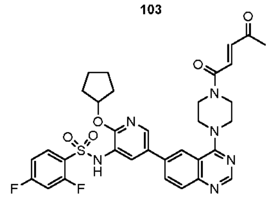
107

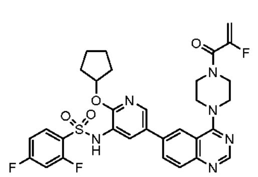
108

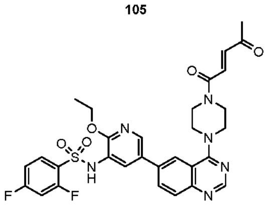
109

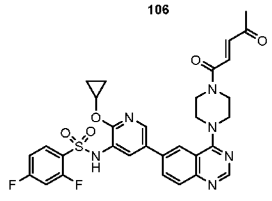
110

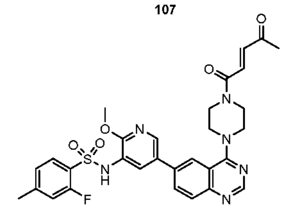
111

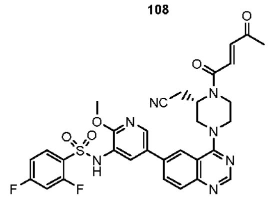
112

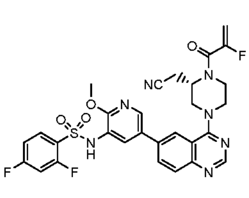
113

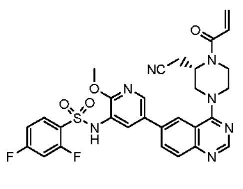
114

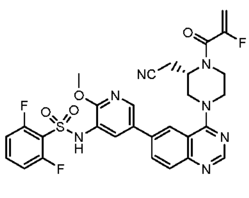
115

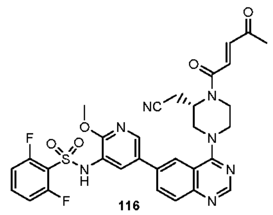
116

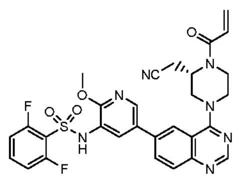
117

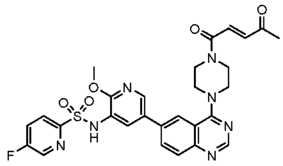
118

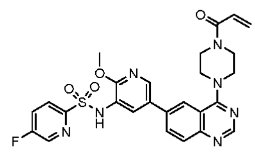
119

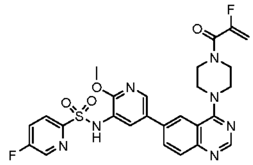
120

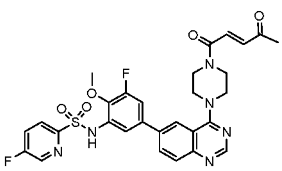
121

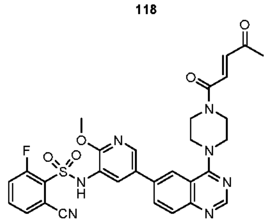
122

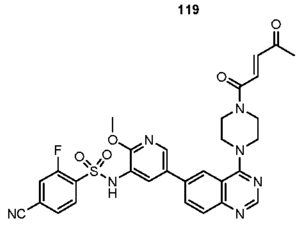
123

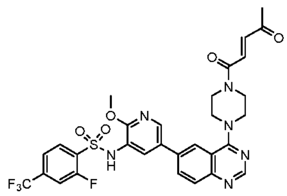
124

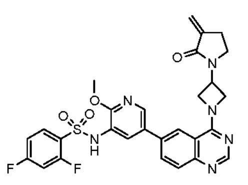
125

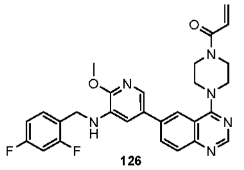
126

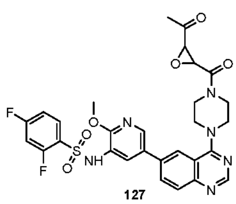
127

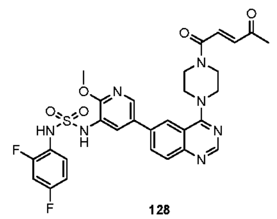
128

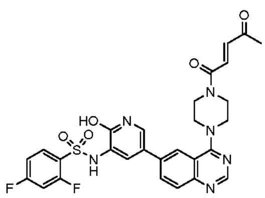
129

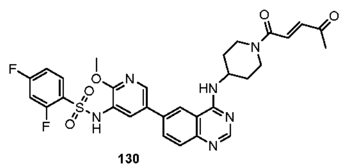
130

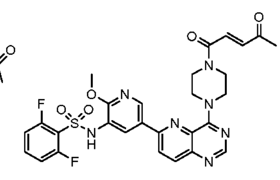
131

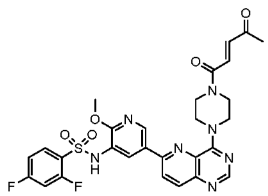
132

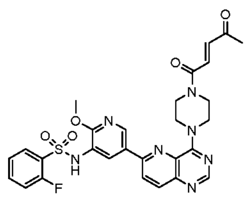
133

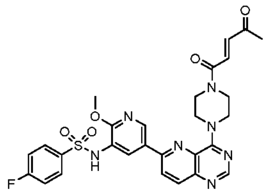
134

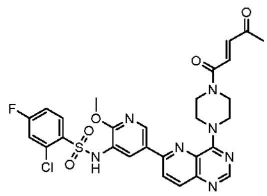
135

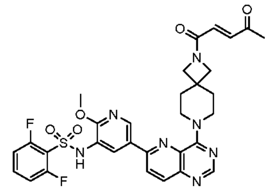
136

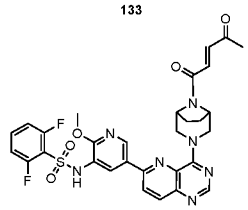
137

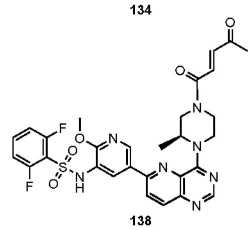
138

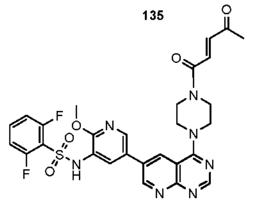
139

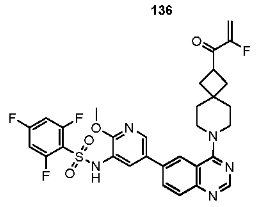
140

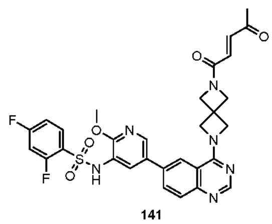
141

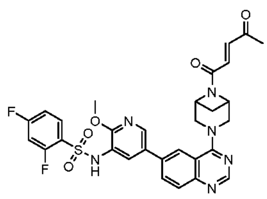
142

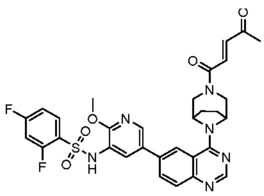
144

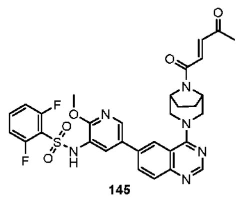
145

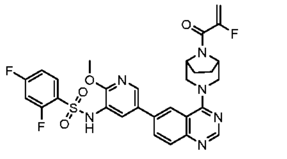
146

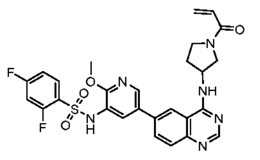
147

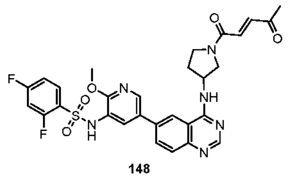
148

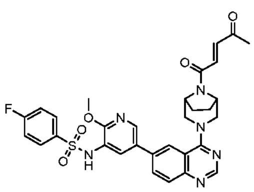
149

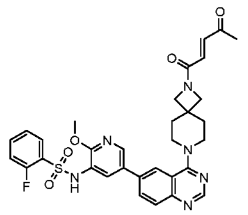
150

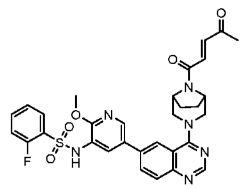
151

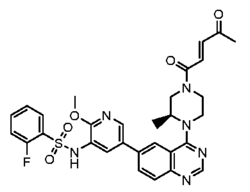
152

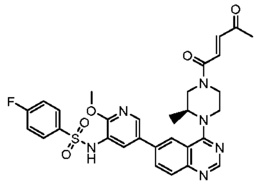
153

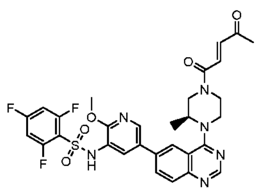
154

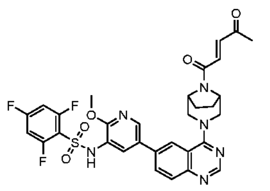
155

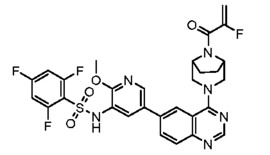
156

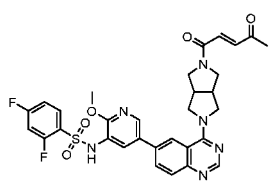
157

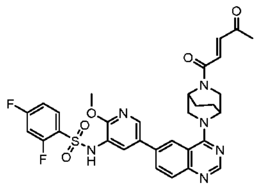
158

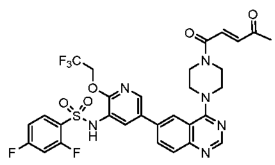
159

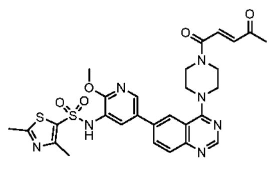
160

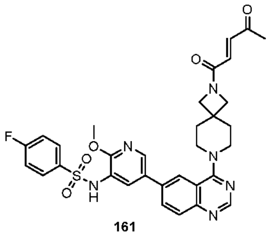
161

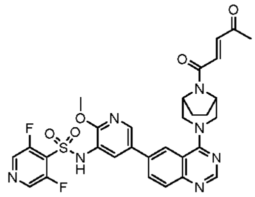
162

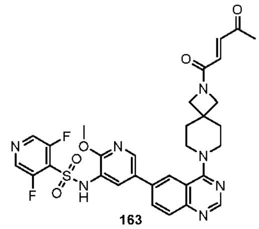
163

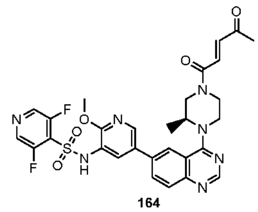
164

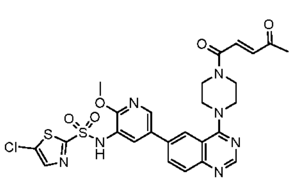
165

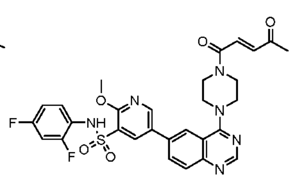
166

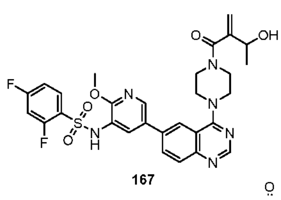
167

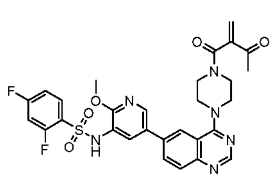
168

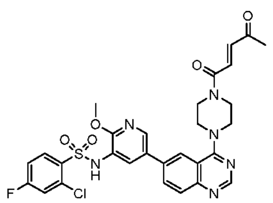
169

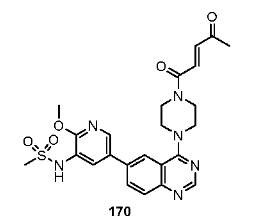
170

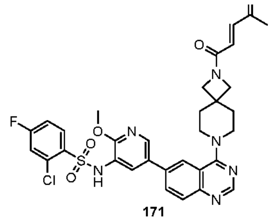
171

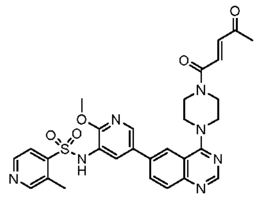
172

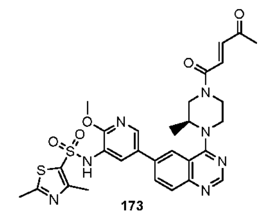
173

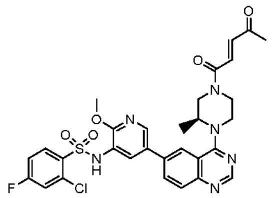
174

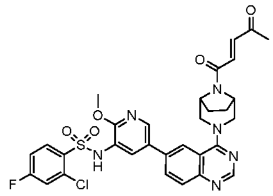
175

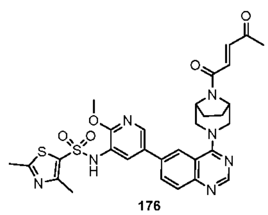
176

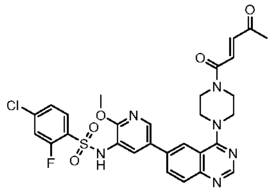
177

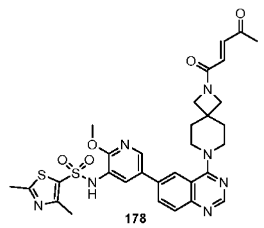
178

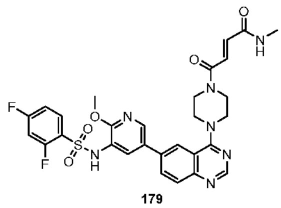
179

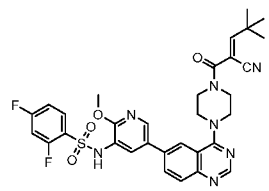
180

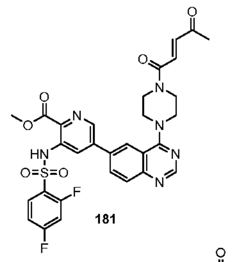
181

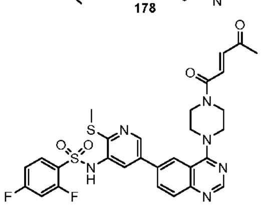
182

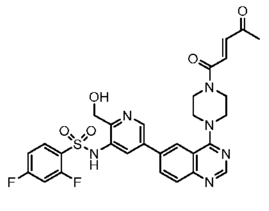
183

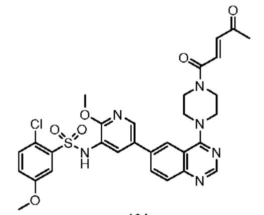
184

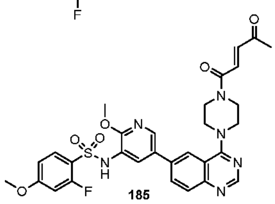
185

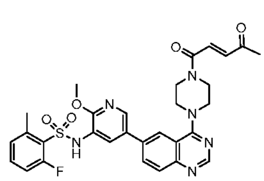
186

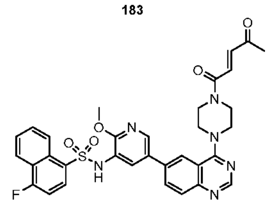
187

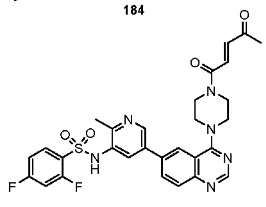
188

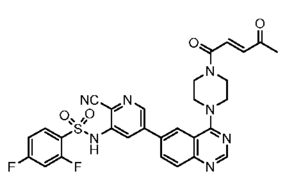
189

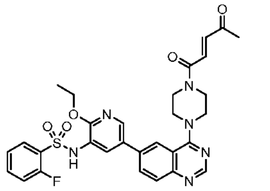
190

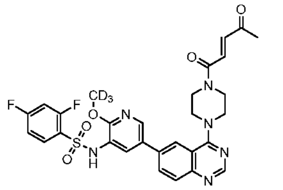
191

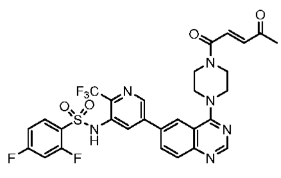
192

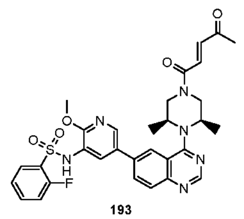
193

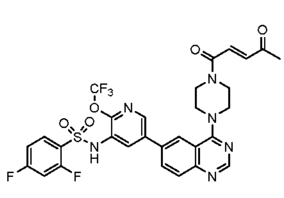
194

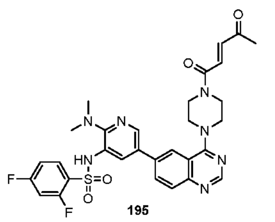
195

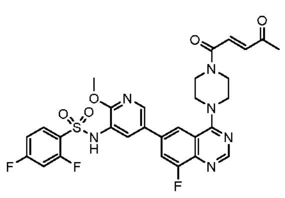
196

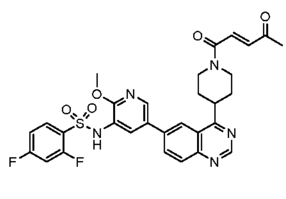
197

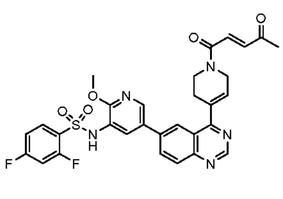
198

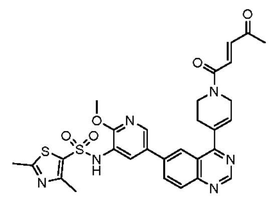
199

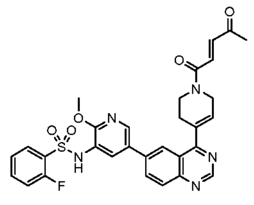
200

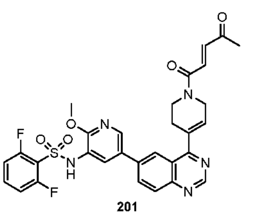
201

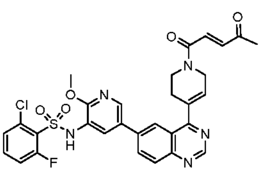
202

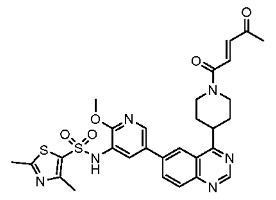
203

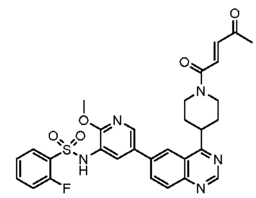
204

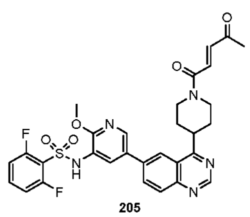
205

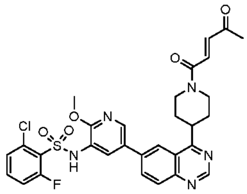
206

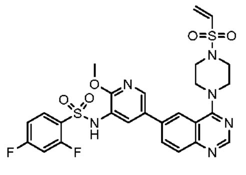
207

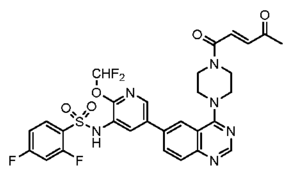
208

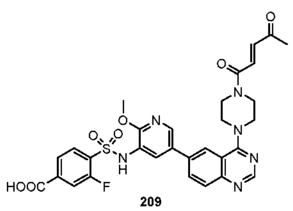
209

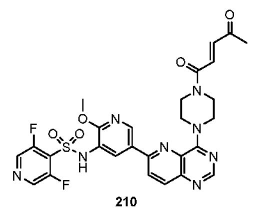
210

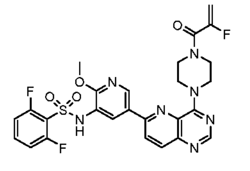
211

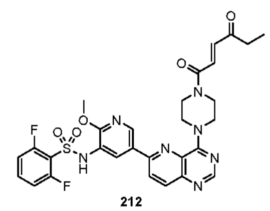
212

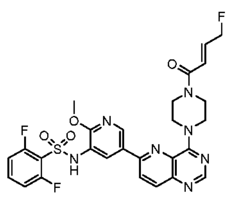
213

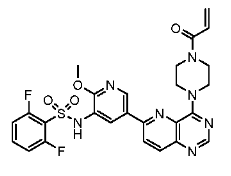
214

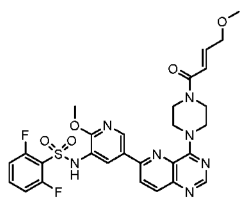
215

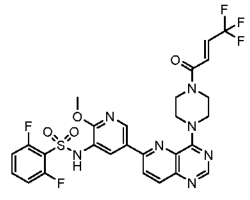
216

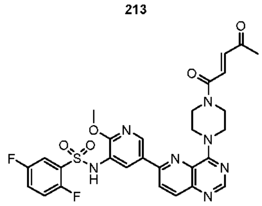
217

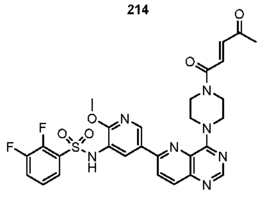
218

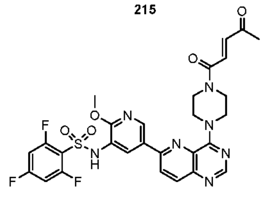
219

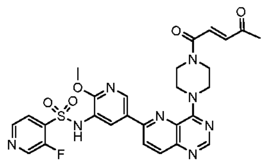
220

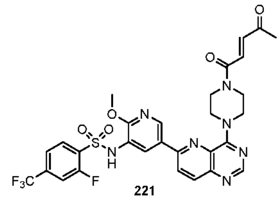
221

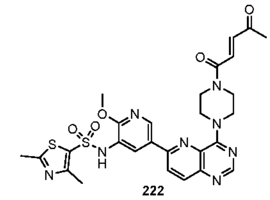
222

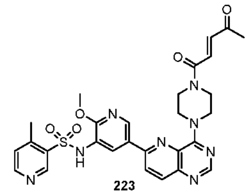
223

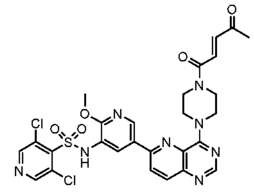
224

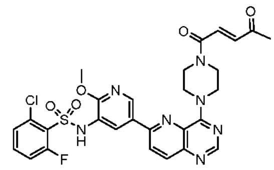
225

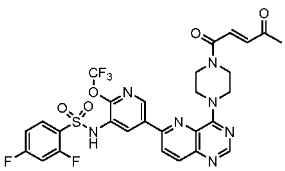
226

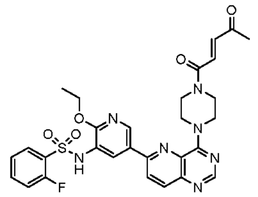
227

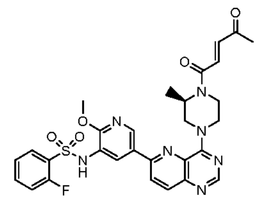
228

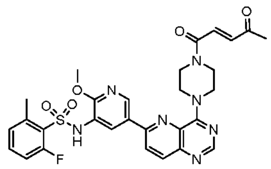
229

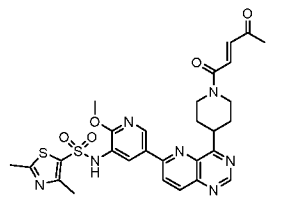
230

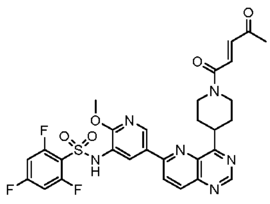
231

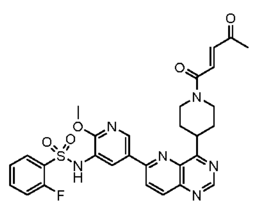
232

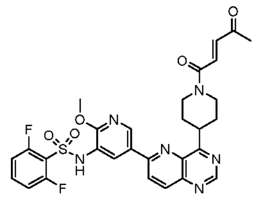
233

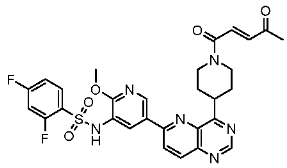
234

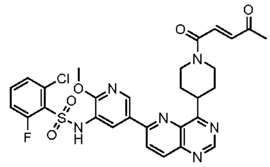
235

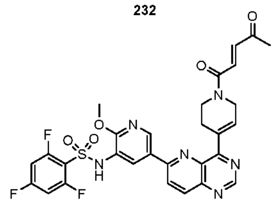
236

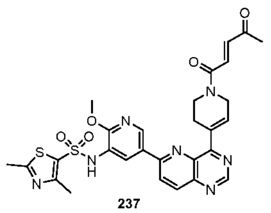
237

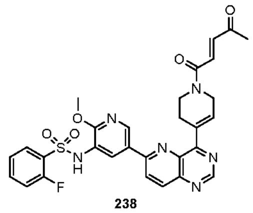
238

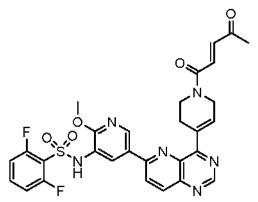
239

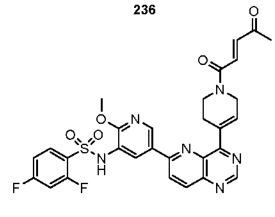
240

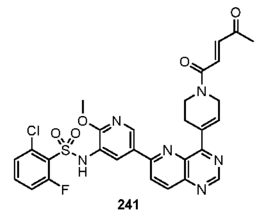
241

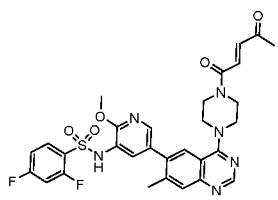
242

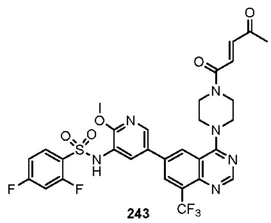
243

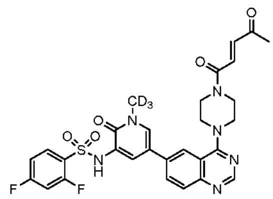
244

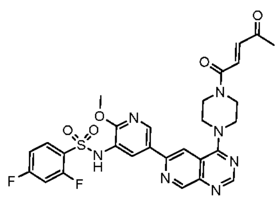
245

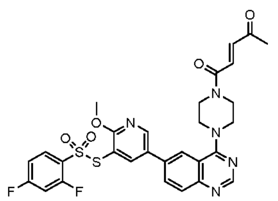
246

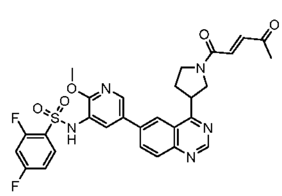
247

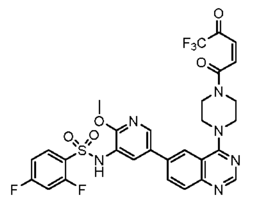
248

The present disclosure also provides a preparation method of the above compound, which includes the following steps:

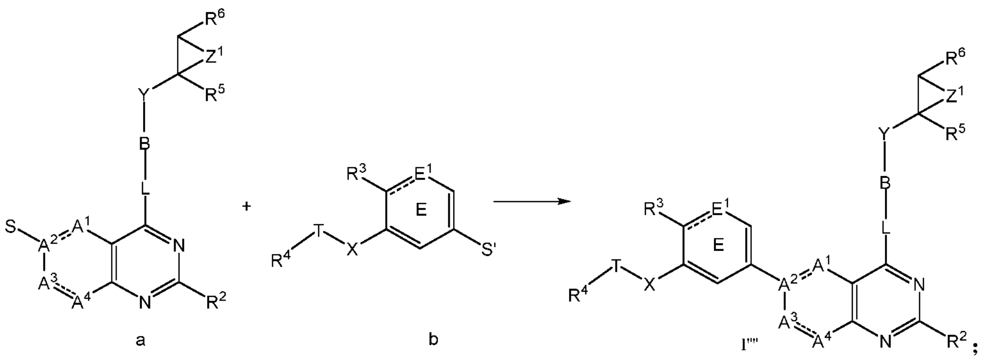
a b I''''

Wherein the definitions of each group in formulas (a) and (b) are as described above.

S and S' are selected from halogen, boronic acid or boronic ester; provided that, when S is selected from halogen, S' is selected from boronic acid or boronic ester; when S' is selected from halogen, S is selected from boronic acid or boronic ester.

The compound of formula I'''' are prepared by Suzuki coupling reaction with the compound of formula (a) and formula (b) .

The compounds of formula (a) and formula (b) can be prepared with reference to the examples. On the basis of the compound core (e.g.

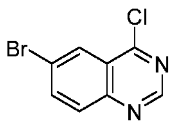, 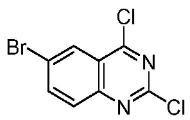, 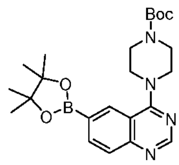,

etc.) provided in the examples, according to the methods described in the examples and/or by using the intermediate raw materials (e.g.

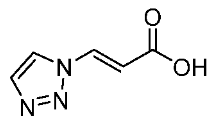,

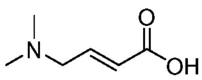, 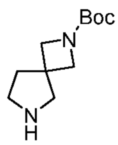,

etc.) provided in the examples.

The present disclosure also provides a pharmaceutical composition, wherein the active ingredient of the pharmaceutical composition is selected from one or a combination of two or more of the compound above ,or a tautomer, meso isomer, racemate, enantiomer, diastereomer or mixture thereof, or a pharmaceutically acceptable hydrate, solvate or salt thereof.

The present disclosure also provides use of the compound above, or a tautomer, meso isomer, racemate, enantiomer, diastereomer or mixture thereof, or a pharmaceutically acceptable hydrate, solvate or salt thereof or the pharmaceutical composition of above in the preparation of KRAS inhibitors and/or PI3K inhibitors.

Further, the KRAS inhibitors is selected from KRAS G12C inhibitors, KRAS G12V inhibitors, KRAS G12D inhibitors, KRAS G12S inhibitors, preferably KRAS G12C inhibitors; the PI3K inhibitors is PI3Kα inhibitors and/or PI3Kδ inhibitors.

The present disclosure also provides use of the compound above, or a tautomer, meso isomer, racemate, enantiomer, diastereomer or mixture thereof, or a pharmaceutically acceptable hydrate, solvate or salt thereof or the pharmaceutical composition of above in the preparation of a medicament for the treatment of a disease mediated by

KRAS and/or PI3K.

The present disclosure provides the compound above, or a tautomer, meso isomer, racemate, enantiomer, diastereomer or mixture thereof, or a pharmaceutically acceptable hydrate, solvate or salt thereof or the pharmaceutical composition of above, for use in the treatment of a disease mediated by KRAS and/or PI3K.

The present disclosure provides a method in the treatment of a disease mediated by KRAS and/or PI3K, comprising administering to said individual an effective amount of a compound described above, or a tautomer, meso isomer, racemate, enantiomer, diastereomer or mixture thereof, or a pharmaceutically acceptable hydrate, solvate or salt thereof or the pharmaceutical composition of above.

Futher, the present disclosure provides use of the compound above, or a tautomer, meso isomer, racemate, enantiomer, diastereomer or mixture thereof, or a pharmaceutically acceptable hydrate, solvate or salt thereof or the pharmaceutical composition of above in the preparation of medicament for the treatment of a disease mediated by one or more of KRAS G12C, PI3Kα, and PI3Kδ.

The present disclosure provides the compound above, or a tautomer, meso isomer, racemate, enantiomer, diastereomer or mixture thereof, or a pharmaceutically acceptable hydrate, solvate or salt thereof or the pharmaceutical composition of above, for use in the treatment of a disease mediated by one or more of KRAS G12C, PI3Kα, and PI3Kδ.

A method in the treatment of a disease mediated by one or more of KRAS G12C, PI3Kα, and PI3Kδ, comprising administering to said individual an effective amount of a compound described above, or a tautomer, meso isomer, racemate, enantiomer, diastereomer or mixture thereof, or a pharmaceutically acceptable hydrate, solvate or salt thereof or the pharmaceutical composition of above.

Futher, the disease is cancer or an autoimmune disease.

Futher, the cancer is selected from: non-small cell lung cancer, lung cancer, pancreatic cancer, ovarian cancer, bladder cancer, prostate cancer, chronic myelogenous leukemia, colorectal cancer, brain cancer, liver cancer, kidney cancer, stomach cancer, breast cancer, triple negative breast cancer, skin cancer, melanin Cancer, head and neck cancer, bone cancer, cervical cancer, pelvic cancer, vaginal cancer, oral cancer, lymphoma, blood cancer, esophageal cancer, urethra cancer, nasal cavity cancer.

The present disclosure also provides use of the compound above, or a tautomer, meso isomer, racemate, enantiomer, diastereomer or mixture thereof, or a pharmaceutically acceptable hydrate, solvate or salt thereof, or the pharmaceutical composition of above in the preparation of medicament for the treatment of a disease that is resistant to anticancer agents.

The present disclosure also provides the compound above, or a tautomer, meso isomer, racemate, enantiomer, diastereomer or mixture thereof, or a pharmaceutically acceptable hydrate, solvate or salt thereof or the pharmaceutical composition of above, for use in the treatment of a disease that is resistant to anticancer agents.

A method in the treatment of a disease that is resistant to anticancer agents, comprising administering to said individual an effective amount of a compound described above or a tautomer, meso isomer, racemate, enantiomer, diastereomer or mixture thereof, or a pharmaceutically acceptable hydrate, solvate or salt thereof or the pharmaceutical composition of above.

Further, the anticancer agent is selected from KRAS G12C inhibitors, KRAS G12V inhibitors, KRAS G12D inhibitors, KRAS G12S inhibitors, preferably KRAS G12C inhibitors;

further, the KRAS G12C inhibitors is selected from AMG-510, MRTX-849, preferably AMG-510.

The present disclosure also provides use of the compound above, or a tautomer, meso isomer, racemate, enantiomer, diastereomer or mixture thereof, or a pharmaceutically acceptable hydrate, solvate or salt thereof or the pharmaceutical composition of above in the preparation of medicament for the treatment of a disease causing overexpression of PI3K protein and/or KRAS G12C protein.

The present disclosure also provides use of the compound above, or a tautomer, meso isomer, racemate, enantiomer, diastereomer or mixture thereof, or a pharmaceutically acceptable hydrate, solvate or salt thereof or the pharmaceutical composition of above in the preparation of medicament for the treatment of a disease caused by overexpression of PI3K protein and/or KRAS G12C protein.

The present disclosure provides the compound above, or a tautomer, meso isomer, racemate, enantiomer, diastereomer or mixture thereof, or a pharmaceutically acceptable hydrate, solvate or salt thereof or the pharmaceutical composition of above, for use in the treatment of a disease caused by overexpression of PI3K protein and/or KRAS G12C protein.

The present disclosure provides a method in the treatment of a disease caused by overexpression of PI3K protein and/or KRAS G12C protein, including administering to said individual an effective amount of a compound described above, or a tautomer, meso isomer, racemate, enantiomer, diastereomer or mixture thereof, or a pharmaceutically acceptable hydrate, solvate or salt thereof or the pharmaceutical composition of above.

The pharmaceutical composition containing compound or a tautomer, meso isomer, racemate, enantiomer, diastereomer or mixture thereof, or a pharmaceutically acceptable hydrate, solvate or salt thereof in the present disclosure may contain pharmaceutically acceptable excipients.

"Pharmaceutically acceptable" as used herein refers to any substance that does not interfere with the effectiveness of the biological activity of the active ingredient and is non-toxic to the host to which it is administered.

Pharmaceutically acceptable excipients as claimed in the present disclosure are the general term for all additional materials in drugs other than the main drug. The excipients should have the following properties: (1) No toxic effects on the human body and almost no side effects; (2) Stable chemical properties, not easily affected by temperature, pH, storage time, etc.; (3) It has no incompatibility with the main drug and does not affect the efficacy and quality inspection of the main drug; (4) It does not interact with packaging materials. The excipients in the present disclosure include but are not limited to fillers (diluents), lubricants (glidants or anti-adhesive agents), dispersants, wetting agents, adhesives, regulators, solubilizers, antioxidants, and bacteriostatic agents, emulsifiers, disintegrants, etc. Adhesives include syrup, gum arabic, gelatin, sorbitol, tragacanth, cellulose and its derivatives (such as microcrystalline cellulose, sodium carboxymethylcellulose, ethylcellulose or hydroxypropylmethylcellulose, etc.) , gelatin slurry, syrup, starch slurry or polyvinylpyrrolidone, etc.; Lubricants include micronized silica gel, magnesium stearate, talc, aluminum hydroxide, boronic acid, hydrogenated vegetable oil, polyethylene glycol, etc.; disintegrants include starch and its derivatives (such as sodium carboxymethyl starch, sodium starch glycolate , pregelatinized starch, modified starch, hydroxypropyl starch, corn starch, etc.), polyvinylpyrrolidone or microcrystalline cellulose, etc.; wetting agents include sodium lauryl sulfate, water or alcohol, etc.; antioxidants include sodium sulfite, sodium bisulfite, sodium metabisulfite, dibutylbenzoic acid, etc.; bacteriostatic agents include 0.5% phenol, 0.3% cresol, 0.5% chlorobutanol, etc.;

The term "pharmaceutically acceptable salt" refers to salts of compounds of the disclosure with acids or bases suitable for use as pharmaceuticals. The above acids and bases are generalized Lewis acids and bases. Suitable salt-forming acids include, but are not limited to: Hydrochloric acid, hydrobromic acid, hydrofluoric acid, sulfuric acid, nitric acid, phosphoric acid and other inorganic acids, formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenesulfonic acid, benzenesulfonic acid and other organic acids; and acidic amino acids such as aspartic acid and glutamic acid.

The administration mode of the compound or pharmaceutical composition in the present disclosure is not particularly limited. Representative administration modes include (but are not limited to): oral, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) Fillers or compatibilizers, such as starch, lactose, sucrose, glucose, mannitol and silicic acid; (b) Adhesives such as hydroxymethylcellulose, alginate, gelatin, polyvinylpyrrolidone, sucrose and gum arabic; (c) Humectants such as glycerin; (d) Disintegrants such as agar, calcium carbonate, potato or tapioca starch, alginic acid, certain complex silicates, and sodium carbonate; (e) Retarders, such as paraffin; (f) Absorption accelerators, such as quaternary ammonium compounds; (g) Wetting agents, such as cetyl alcohol and glyceryl monostearate; (h) Adsorbent, uch as kaolin; and (i) Lubricants, such as talc, calcium stearate, magnesium stearate, solid polyethylene glycol, sodium lauryl sulfate, or mixtures thereof. In capsules, tablets and pills, the dosage form may also contain buffering agents.

Solid dosage forms such as tablets, dragees, capsules, pills and granules may be prepared using coatings and shell materials such as enteric casings and other materials well known in the art. They may contain opacifying agents and the release of the active compound or compounds in such compositions may be in a delayed manner in a certain part of the digestive tract. Examples of embedding components that can be used are polymeric substances and waxy substances. If necessary, the active compounds can also be in microencapsulated form with one or more of the above-mentioned excipients.

Liquid dosage forms for oral administration include pharmaceutically acceptable emulsions, solutions, suspensions, syrups or tinctures. Liquid dosage forms may contain, in addition to the active compound, inert diluents conventionally employed in the art, such as water or other solvents, solubilizers and emulsifiers, for example, ethanol, isopropyl alcohol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances, etc.

Besides these inert diluents, the compositions may also contain adjuvants such as wetting agents, emulsifying and suspending agents, sweetening, flavoring and perfuming agents.

Suspensions may contain, in addition to the active compound, suspending agents, for example, ethoxylated isostearyl alcohols, polyoxyethylene sorbitol and sorbitan esters, microcrystalline cellulose, aluminum methoxide and agar or mixtures of these substances and the like.

Compositions for parenteral injection may contain physiologically acceptable sterile aqueous or anhydrous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

Dosage forms for topical administration of the compounds of this disclosure include ointments, powders, patches, sprays and inhalants. The active ingredient is mixed under sterile conditions with a physiologically acceptable carrier and any preservatives, buffers, or propellants that may be required.

The compounds in the present disclosure may also be used in injectable preparations. Wherein, the injection is selected from liquid injection (water injection), sterile powder for injection (powder injection) or injection tablets (Refers to the molded tablets or machine-pressed tablets made by aseptic methods, which are dissolved in water for injection for subcutaneous or intramuscular injection).

Wherein, in addition to the above compounds, the injection powder also contains at least excipients. The excipients mentioned in the present disclosure are ingredients intentionally added to the medicine, and they should not have pharmacological properties in the amount used. However, excipients may aid in processing, dissolution or dissolution of the drug, delivery through a targeted route of administration, or aid in stability.

‘optionally substituted by one or more’ means it can be substituted by one or more specified substituents, or it can be unsubstituted; if ’more’ in ’one or more’ is not limited, the minimum value is 2, and the maximum value is the possible number of the substitution site of substituted groups.

If a substituent is described as being ‘independently selected from’ a group, each substituent is selected independently of the other. Thus, each substituent may be the same as or different from another (or other) substituent. ’Substituted’ means that a hydrogen atom in a molecule is replaced by a different group.

’Membered’ refers to the number of skeleton atoms constituting the ring.

The ’bond’ in the present disclosure means that there is only one bond, which can also be understood as ‘none’.

As used herein, alkyl as optional substituents for $R^g$ and $R^{11}$ include deuterated alkyl.

’Alkyl’ refers to an aliphatic hydrocarbon group and refers to a saturated hydrocarbon group. The alkyl may be a straight chain alkyl group or a branched chain alkyl group. Typical alkyl groups include, but are not limited to, methyl, ethyl, propyl, isopropyl, butyl, isobutyl, tert-butyl, pentyl, hexyl, etc.

As used in the present disclosure, C1~Cn includes C1-C2, C1~C3 ...... C1~Cn with n as an integer greater than one. The prefix used as a substituent indicates the minimum and maximum number of carbon atoms in the substituent. For example, ’C1~C6 alkyl’ refers to a straight-chain or branched alkyl group containing 1 to 6 carbon atoms. ’Heteroalkyl’ refers to an alkyl group containing heteroatoms.

’Ring’ refers to any covalently closed structure, including, for example, carbocycles (e.g., aryl or cycloalkyl), heterocycles (e.g., heteroaryl or heterocycloalkyl), aryls (e.g., aryl or heteroaryl), non-aromatic groups (such as cycloalkyl or heterocycloalkyl). The ’ring’ in the present disclosure can be a monocyclic ring, as well as a polycyclic ring and also parallel ring, spirocyclic ring or bridged cyclic ring

’Cycloalkyl’ refers to a saturated cyclic hydrocarbon substituent.

‘Cycloalkenyl’ refers to a cyclic substituent containing at least one carbon-carbon double bond in the ring skeleton.

‘Heterocycloalkyl’ refers to a saturated ring substituent containing heteroatoms in the ring skeleton. ‘Nitrogen-containing heterocycloalkyl’ refers to a cycloalkyl group containing nitrogen atoms in the ring skeleton, and the same applies to other similar situations.

‘Nitrogen-containing monoheterocycloalkyl’ refers to nitrogen-containing heterocycloalkyl with a monocyclic structure, and the same applies to other similar situations.

‘Alicyclyl’ refers to a cyclic substituent without aromaticity, which may be a cycloalkyl group, a cycloalkenyl group or an alicyclic heterocyclic group.

‘Aliphatic heterocyclyl’ refers to a substituent group formed by a non-aromatic heterocyclic compound containing at least one heteroatom on the ring skeleton, and ‘aliphatic heterocyclyl’ includes ’heterocycloalkyl’.

Typical aliphatic heterocyclyl include but are not limited to:

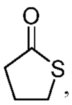, 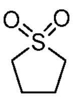, 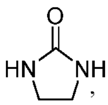, 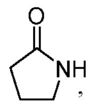, 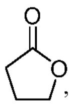, 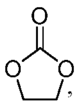, 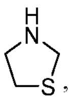, 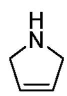, 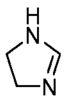, 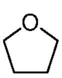, 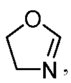, 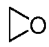, 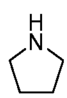, 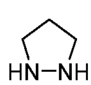,

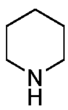, 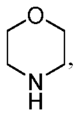, 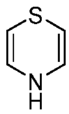, 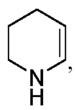, 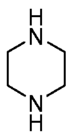, 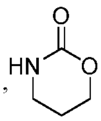, 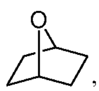, 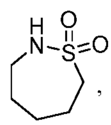, 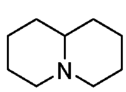, 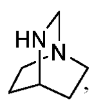, 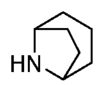

’Aryl’ refers to an aromatic monocyclic or polycyclic group whose planar ring has a delocalized π electron system and contains 4n+2 π electrons, where n is an integer; typical aryl include but not limited to phenyl, naphthyl, phenanthrenyl, anthracenyl, fluorenyl and indenyl, etc.

'Heteroaryl' refers to a monocyclic or polycyclic group containing heteroatoms and having aromatic properties. Typical heteroaryl include but are not limited to:

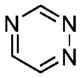, 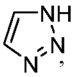, 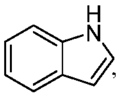, 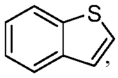, 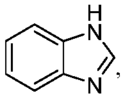, 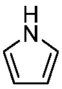, 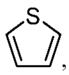, 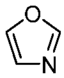, 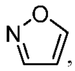, 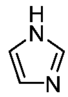, 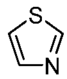, 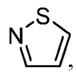,

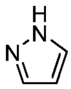, 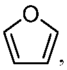, 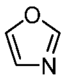, 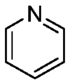, 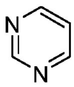, 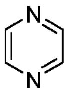, 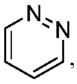, 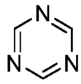, 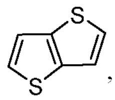, 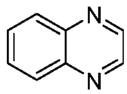, 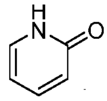.

As used herein, the alkyl, cycloalkyl, cycloalkenyl, aliphatic cycloalkyl, heterocyclyl, heterocycloalkyl, aryl and heteroaryl, etc. can be unsubstituted alkyl, cycloalkyl, cycloalkenyl, aliphatic cycloalkyl, heterocyclyl, heterocycloalkyl, aryl and heteroaryl, etc., as well as alkyl, cycloalkyl, cycloalkenyl, aliphatic cycloalkyl, heterocyclyl, heterocycloalkyl, aryl and heteroaryl, etc.

As used herein, unless otherwise specified, 'substituted' means that the mentioned group may be substituted by one or more additional groups, each of which is independently selected from substituent groups common in the art, such as halogen, cyano, hydroxy, amino, carboxyl, alkyl, alkoxy, alkylamino, alkylthio, haloalkyl, cycloalkyl, heterocycloalkyl, aryl, heteroaryl, etc.

'Alkoxy' is -O-alkyl.

'Alkylamino' is -NH-alkyl or -N-$(alkyl)_2$

'Alkoxy' is -S-alkyl.

'Halogen' or 'Halo' is F, Cl, Br, or I.

'Cyano' is -CN.

'Amino' is $-NH_2$.

'Hydroxy' is -OH.

'(O)' is =O, as $-C(O)R^{13}$ is

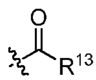,

and the same applies to other similar situations.

C=O is

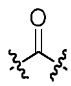.

The structural formula of pyridine is

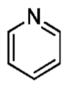.

The structural formula of thiophene is

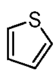.

The structural formula of pyrrole is

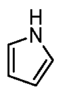.

The structural formula of 1H-1,2,3-triazole is

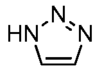

The structural formula of oxazole is

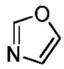

As used herein, an 'individual' includes a human or non-human animal. Exemplary human subjects include human subjects (referred to as patients) suffering from a disease, such as those described herein, or normal subjects. 'Non-human animals' include all vertebrate animals, such as non-mammals and mammals, such as non-human primates, domestic animals and/or domesticated animals.

As used herein, an 'effective amount' refers to an amount of a compound that, when administered, alleviates to a certain extent one or more symptoms of the disease being treated. Dosage regimens can be adjusted to provide the best desired response.

The beneficial effects of the present disclosure are:

(1) The present disclosure provides a series of compounds with obvious inhibitory effects on KRAS and PI3K proteins, provides new solutions for the treatment of diseases such as cancer with KRAS or PI3K as the target, and can be used to prepare medicines for treating related diseases, with broad application prospects.

(2) The compound of the present disclosure has a strong inhibitory effect on AMG-510-resistant cancer cells, overcomes the resistance problem caused by simple G12C inhibitors, and is expected to prolong the survival period of patients.

**Detailed Description of the Invention**

The technical solution of the present disclosure is clearly and completely described below. Obviously, the described embodiments are some of the embodiments of the present disclosure, rather than all the embodiments. Based on the embodiments of the present disclosure, all other embodiments obtained by those of ordinary skill in the art without creative efforts fall within the scope of protection of the present disclosure.

The structure of the compound of the present disclosure is determined by Nuclear Magnetic Resonance (NMR) or/and Liquid Chromatography-Mass Spectrometry (LC-MS). Chemical shifts ($\delta$) for NMR are given in units of parts per million (ppm). NMR was measured using an AVANCE NEO 400 MHz Bruker instrument. The solvents used for the determination were deuterated dimethyl sulfoxide (DMSO-$d_6$), deuterated chloroform ($CDCl_3$), and deuterated methanol ($CD_3OD$). The internal standard was tetramethylsilane (TMS). MS was measured using an ISQ-EC Thermo Fisher LC-MS instrument. Prep-HPLC is a GX-281 Gilson chromatograph. The separation methods used are: (Method 1) Sun Fire Prep C18 OBDTM 5$\mu$m, 30 $\times$ 150 mm Column, 0.04% HCl aqueous solution/acetonitrile; (Method 2) Xbridge Prep C18 OBDTM 5$\mu$m, 30 $\times$ 150 mm Column, 10 mM $NH_4HCO_3$ aqueous solution/acetonitrile.

The starting materials in the examples of the present disclosure are known and can be purchased on the market, or can be synthesized according to methods known in the art.

The solvents used in the present disclosure, unless otherwise specified, are commercially available.

The reaction temperature in the examples, unless otherwise specified, is room temperature, which is 20°C to 30°C. The chemical abbreviations involved in the present disclosure have the following meanings:

TFA: trifluoroacetic acid
DMF: *N*,*N*-dimethylformamide
DMSO: dimethyl sulfoxide
THF: tetrahydrofuran
HOBT: 1-hydroxybenzotriazole
EDCI: 1-(3-dimethylaminopropyl)-3-ethylcarbodiimide hydrochloride
DIPEA: *N*,*N*-diisopropylethylamine
HATU: O-(7-azabenzotriazol-1-yl)-*N*,*N*,*N'*,*N'*-tetramethylurea
$T_3P$: 1-propylphosphonic anhydride
BINAP: 1,1'-binaphthyl-2,2'-bisdiphenylphosphine

Prep-HPLC: preparative High-Performance Liquid Chromatography

**Example 1**

**Preparation of *N*-(5-(4-(4-acryloylpiperazin-1-yl)quinazolin-6-yl)-2-methoxypyridin-3-yl)-2,4-difluorobenzenesulfonamide**

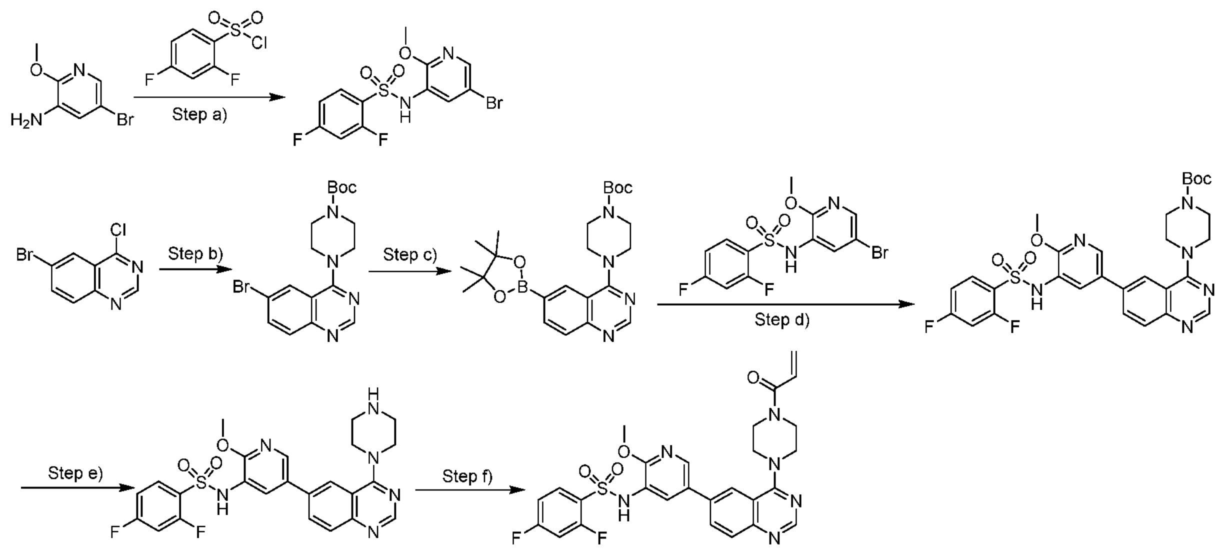


Step a): Preparation of *N*-(5-bromo-2-methoxypyridin-3-yl)-2,4-difluorobenzenesulfonamide

5-Bromo-2-methoxypyridin-3-amine (2.0 g, 9.850 mmol), 2,4-difluorobenzenesulfonyl chloride (2.3 g, 10.835 mmol), 4-dimethylaminopyridine (60 mg, 0.493 mmol) and pyridine (1.2 g, 14.775 mmol) were dissolved in dichloromethane (20 mL). After the addition was completed, the reaction mixture was stirred at room temperature for 18 h. Upon completion of the reaction, the reaction was quenched by adding water (50 mL), and the reaction mixture was extracted with dichloromethane (100 mL×2). The organic phases were combined, dried over anhydrous sodium sulfate and filtered. The filtrate was concentrated in vacuo. The crude product obtained was purified by silica gel column chromatography (Eluent: petroleum ether/ethyl acetate = 3/1 to 1/1). *N*-(5-bromo-2-methoxypyridin-3-yl)-2,4-difluorobenzene sulfonamide was obtained, yield: 37.3%; ESI-MS(m/z): 381.0 $[M+H]^+$

Step b): Preparation of tert-butyl 4-(6-bromoquinazolin-4-yl)piperazine-1-carboxylate

6-Bromo-4-chloroquinazoline (1.9 g, 7.820 mmol) and tert-butyl piperazine-1-carboxylate (1.8 g, 9.380 mmol) were dissolved in dimethyl sulfoxide (30 mL), with triethyl amine (2.4 g, 23.470 mmol) added . After the addition was completed, the reaction mixture was heated to 60°C and stirred for 6 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated in vacuo. The crude product obtained was purified by silica gel column chromatography (Eluent: dichloromethane/methanol = 50/1 to 10/1), tert-butyl 4-(6-bromoquinazolin-4-yl)piperazine-1-carboxylate was obtained, yield: 65.4%; ESI-MS(m/z): 393.1 $[M+H]^+$.

Step c): Preparation of tert-butyl 4-(6-(4,4,5,5-tetramethyl-1,3,2-dioxaborolan-2-yl)quinazolin-4-yl)piperazine-1-carboxylate

tert-butyl 4-(6-bromoquinazolin-4-yl)piperazine-1-carboxylate (2.0 g, 5.10 mmol), bis(pinacolato)diboron (1.9 g, 7.640 mmol), $Pd(dppf)Cl_2$ (373 mg, 0.510 mmol) and potassium acetate (1.5 g, 15.310 mmol) were dissolved in dioxane (30 mL). After the addition was completed, in the presense of protective nitrogen, the reaction mixture was kept at 110°C and stirred for 4 h. Upon completion of the reaction, the reaction mixture was filtered, and the filtrate was concentrated under reduced pressure to provide the crude residue. The crude product obtained was purified by silica

gel column chromatography (Eluent: dichloromethane/methanol = 50/1 to 10/1), tert-butyl 4-(6-(4,4,5,5-tetramethyl-1,3,2-dioxaborolan-2-yl)quinazolin-4-yl)piperazine-1-carboxylate was obtained, yield: 62.5%; ESI-MS(m/z): 441.3 $[M+H]^+$.

Step d): Preparation of tert-butyl 4-(6-(5-((2,4-difluorophenyl)sulfonamido)-6-methoxypyridin-3-yl)quinazolin-4-yl)piperazine-1-carboxylate

*N*-(5-bromo-2-methoxypyridin-3-yl)-2,4-difluorobenzenesulfonamide (300 mg, 0.790 mmol), tert-butyl 4-(6-(4,4,5,5-tetramethyl-1,3,2-dioxaborolan-2-yl)quinazolin-4-yl)piperazine-1-carboxylate (698 mg, 1.580 mmol), $Pd(dppf)Cl_2$ ( 58 mg, 0.080 mmol) and cesium carbonate (779 mg, 2.390 mmol) were dissolved in dioxane/water (4:1, ,10 mL). After the addition was completed, in the presense of protective nitrogen, the reaction mixture was kept at 110°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure . The crude product obtained was purified by silica gel column chromatography (Eluent: petroleum ether/ethyl acetate = 1/1 to 1/3), tert-butyl 4-(6-(5-((2,4-difluorophenyl)sulfonamido)-6-methoxypyridin-3-yl)quinazolin-4-yl)piperazine-1-carboxylate was obtained, yield: 82.5%. ESI-MS(m/z): 613.1 $[M+H]^+$.

Step e): Preparation of 2,4-difluoro-*N*-(2-methoxy-5-(4-(piperazin-1-yl)quinazolin-6-yl)pyridin-3-yl)benzenesulfonamide

tert-butyl 4-(6-(5-((2,4-difluorophenyl)sulfonamido)-6-methoaypyridin-3-yl)quinazolin-4-yl)piperazine-1-carboxylate (400 mg, 0.650 mmol) were dissolved in dichloromethane (8 mL), under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure, 2.4-difluoro-*N*-(2-methoxy-5-(4-(piperazin-1-yl)quinazolin-6-yl)pyridin-3-yl)benzenesulfonamide was obtained. ESI-MS(m/z): 513.1 $[M+H]^+$.

Step f): Preparation of *N*-(5-(4-(4-acryloylpiperazin-1-yl)quinazolin-6-yl)-2-methoxypyridin-3-yl)-2,4-difluorobenzenesulfonamide

To a solution of 2,4-difluoro-*N*-(2-methoxy-5-(4-(piperazin-1-yl)quinazolin-6-yl)pyridin-3-yl)benzenesulfonamide (170 mg, 0.330 mmol), HOBT (54 mg, 0.400 mmol), and EDCI (76 mg, 0.400 mmol) in dichloromethane (10 mL) was added DIEA (215 mg, 1.650 mmol) and acrylic (36 mg, 0.500 mmol) slowly at -78°C. After the addition was completed, the reaction mixture was kept at -78°C and stirred for 1 h. Upon completion of the reaction, the reaction was quenched by adding water (50 mL), and the reaction mixture was extracted with dichloromethane (100 mL×2). The organic phases were combined, dried over anhydrous sodium sulfate and filtered. The filtrate was concentrated under reduced pressure. The crude product obtained was purified by Prep-HPLC (Method 2), N-(5-(4-(4-acryloylpiperazin-1-yl)quinazolin-6-yl)-2-methoxypyridin-3-yl)-2,4-difluorobenzenesulfonamide was obtained, yield: 10.2%; $^1H$ NMR (400 MHz, DMSO-$d_6$) δ 10.34 (s, 1H), 8.66 (s, 1H), 8.47 (s, 1H), 8.09-8.07 (m, 2H), 8.02 (s, 1H), 7.93-7.91 (m, 1H), 7.80-7.77 (m, 1H), 7.60-7.55 (m, 1H), 7.24-7.20 (m, 1H), 6.87-6.81 (m, 1H), 6.19 (d, *J* = 8.0 Hz, 1H), 5.76-5.73 (m, 1H), 3.90-3.79 (m, 8H), 3.69 (s, 3H); ESI-MS(m/z): 567.0 $[M+H]^+$.

**Example 2**

***N*-(5-(4-((1-acryloylpiperidin-4-yl)amino)pyrido[3,2-*d*]pyrimidin-6-yl)-2-chloropyridin-3-yl)-2,4-difluorobenzenesulfonamide**

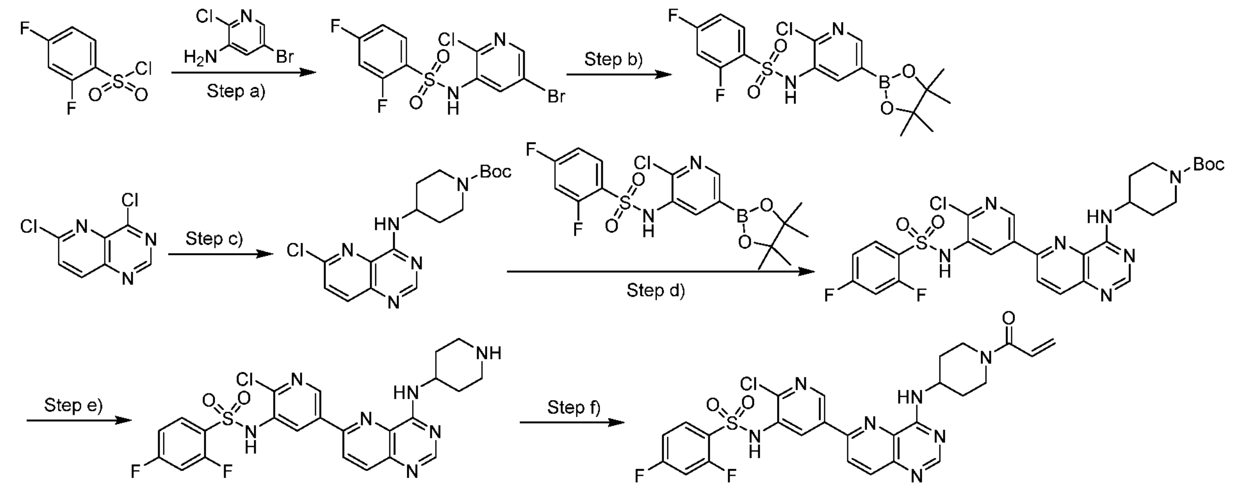


Step a): Preparation of N-(5-bromo-2-chloropyridin-3-yl)-2,4-difluorobenzenesulfonamide

5-Bromo-2-chloropyridin-3-amine (2.0 g, 9.640 mmol), 2,4-difluorobenzenesulfonyl chloride (2.3 g, 10.819 mmol) were added to pyridine (20 mL). After the addition was completed, the reaction mixture was heated to 80°C and stirred for 18 h. Upon completion of the reaction, the reaction was quenched by adding water (50 mL), and the reaction mixture was extracted with dichloromethane (100 mL×2). The organic phases were combined, washed with saturated brine (50 mL×1), dried over anhydrous sodium sulfate and filtered. The filtrate was concentrated under reduced pressure. The crude product obtained was purified by silica gel column chromatography (Eluent: dichloromethane/methanol=100/1 to 50/1), N-(5-bromo-2-chloropyridin-3-yl)-2,4-difluorobenzenesulfonamide was obtained, yield: 31.2%; ESI-MS(m/z): 384.9 $[M+H]^+$.

Step b): Preparation of *N*-(2-chloro-5-(4,4,5,5-tetramethyl-1,3,2-dioxaborolan-2-yl)pyridin-3-yl)-2,4-difluorobenzenesulfonamide

N-(5-bromo-2-chloropyridin-3-yl)-2,4-difluorobenzenesulfonamide (450 mg, 1.173mmol), bis(pinacolato)diboron (298 mg, 1.173mmol) , Pd(dppf)$Cl_2$ (172 mg, 0.235 mmol) and potassium acetate (345 mg, 3.519 mmol) were dissolved in dioxane (10 mL). After the addition was completed, in the presense of protective nitrogen, the reaction mixture was heated to 100°C and stirred for 4 h. Upon completion of the reaction, the reaction was quenched by adding water (30 mL). The reaction mixture was extracted with ethyl acetate (50 mL×2). The organic phases were combined, washed with saturated brine (50 mL×1), dried over anhydrous sodium sulfate and filtered. The filtrate was concentrated through vacuum concentration. The crude product obtained was purified by silica gel column chromatography (Eluent: petroleum ether/ethyl acetate = 10/1 to 5/1), *N*-(2-chloro-5-(4,4,5,5-tetramethyl-1,3,2-dioxaborolan-2-yl)pyridin-3-yl)-2,4-difluorobenzenesulfonamide was obtained, yield: 58.8%; ESI-MS(m/z): 431.1 $[M+H]^+$.

Step c): Preparation of tert-butyl 4-((6-chloropyrido[3,2-*d*]pyrimidin-4-yl)amino)piperidine-1-carboxylate

4,6-Dichloropyrido[3,2-d]pyrimidine (500 mg, 2.500 mmol), 4-aminopiperidine-1-carboxylic acid tert-butyl ester (551 mg, 2.750 mmol) were dissolved in dimethyl sulfoxide (50 mL), with *N*,*N*-diisopropylethylamine (969 mg, 7.500 mmol) added. After the addition was completed, the reaction mixture was heated to 55°C and stirred for 2 h. Upon completion of the reaction, the reaction was quenched by adding water (50 mL). The reaction mixture was extracted with ethyl acetate (100 mL×2). The organic phases were combined and washed with saturated brine (50 mL×1), dried over anhydrous sodium sulfate and filtered. The filtrate was concentrated under reduced pressure. The crude product obtained was purified by silica gel column chromatography (Eluent: petroleum ether/ethyl acetate = 10/1 to 2/1), tert-butyl 4-((6-chloropyrido[3,2-*d*]pyrimidin-4-yl)amino)piperidine-1-carboxylate was obtained, yield: 87.9%; ESI-MS(m/z): 364.2 $[M+H]^+$.

Step d): Preparation of tert-butyl 4-((6-(6-chloro-5-((2,4-difluorophenyl)sulfonamido)pyridin-3-yl)pyrido[3,2-*d*]pyrimidin-4-yl)amino)piperidine-1-carboaylate

tert-butyl 4-((6-chloropyrido[3,2-*d*]pyrimidin-4-yl)amino)piperidine-1-carboxylate (420 mg, 1.154 mmol), N-(2-chloro-5-(4,4,5,5-tetramethyl-1,3,2-dioxaborolan-2-yl)pyridin-3-yl)-2,4-difluorobenzenesulfonamide (497 mg, 1.154

mmol), $Pd(dppf)Cl_2$ (169 mg, 0.231 mmol) and cesium carbonate (1.1 g, 3.376 mmol) were added to dioxane/water mixed solvent (10 mL, v/v=4:1). After the addition was completed, in the presense of protective nitrogen, the reaction mixture was heated to 100°C and stirred for 4 h. Upon completion of the reaction, the reaction was quenched by adding water (50 mL). The reaction mixture was extracted with ethyl acetate (100 mL×2). The organic phases were combined and washed with saturated brine (50 mL×1), dried over anhydrous sodium sulfate and filtered. The filtrate was concentrated under reduced pressure. The crude product obtained was purified by silica gel column chromatography (Eluent: dichloromethane/methanol=50/1 to 30/1), tert-butyl 4-((6-(6-chloro-5-((2,4-difluorophenyl)sulfonamido)pyridin-3 -yl)pyrido[3,2-*d*]pyrimidin-4-yl)amino)piperidine-1-carboxylate was obtained, yield: 37.0%; ESI-MS(m/z): 632.2 $[M+H]^+$.

Step e): *N*-(2-chloro-5-(4-(piperidin-4-ylamino)pyrido[3,2-*d*]pyrimidin-6-yl)pyridin-3-yl)-2,4-difluorobenzenesulfonamide

tert-butyl 4-((6-(6-chloro-5-((2,4-difluorophenyl)sulfonamido)pyridin-3-yl)pyrido[3,2-*d*]pyrimidin-4-yl)amino)piperidine-1-carboxylate (70 mg, 0.132 mmol) was dissolved in hydrogen chloride dioxane solution (10 mL, 4M), and the reaction mixture was stirred at room temperature for 1 h.Upon completion of the reaction, the reaction mixture was concentrated under reduced pressure, quenched by adding saturated sodium bicarbonate solution (20 mL), extracted with ethyl acetate (50 mL×2). The organic phases were combined and washed with saturated brine (50 mL×1), dried over anhydrous sodium sulfate and filtered. The filtrate was concentrated under reduced pressure, *N*-(2-chloro-5-(4-(piperidin-4-ylamino)pyrido[3,2-*d*]pyrimidin-6-yl)pyridin-3-yl)-2,4-difluorobenzenesulfonamide was obtained. The product can be used directly in the next reaction without purification. ESI-MS(m/z): 532.2 $[M+H]^+$.

Step f): *N*-(5-(4-((1-acryloylpiperidin-4-yl)amino)pyrido[3,2-*d*]pyrimidin-6-yl)-2-chloropyridin-3-yl)-2,4-difluorobenzenesulfonamide

*N*-(2-chloro-5-(4-(piperidin-4-ylamino)pyrido[3,2-*d*]pyrimidin-6-yl)pyridin-3-yl)-2,4-difluorobenzenesulfonamide (50 mg, 0.094 mmol), 1-hydroxybenzotriazole (15 mg, 0.113 mmol), 1-ethyl-(3-dimethylaminopropyl)carbodiimide hydrochloride (22 mg, 0.113 mmol) were dissolved in dichloromethane (5 mL), with *N*,*N*-diisopropylethylamine (36 mg, 0.282 mmol) and acrylic acid (8 mg, 0.113 mmol) slowly added under -78°C. After the addition was completed, the reaction mixture was kept at -78°C and stirred for 1 h. Upon completion of the reaction, the reaction was quenched by adding water (20 mL), and the reaction mixture was extracted with dichloromethane (50 mL). The organic phases were combined, concentrated under reduced pressure. The crude product obtained was purified by Prep-HPLC (Method 2), *N*-(5-(4-((1-acryloylpiperidin-4-yl)amino)pyrido[3,2-*d*]pyrimidin-6-yl)-2-chloropyridin-3-yl)-2,4-difluorobenzenesulfonamide was obtained, yield: 15.8%; $^1$H NMR (400 MHz, DMSO-$d_6$) δ 9.25-9.05 (m, 1H), 8.53-8.51 (m, 2H), 8.33-8.19 (m, 3H), 7.85-7.79 (m, 1H), 7.46-7.31 (m, 1H), 7.26-7.18 (m, 1H), 6.92-6.85 (m, 1H), 6.18-6.13 (m, 1H), 5.72-5.69 (m, 1H), 4.54-4.41(m, 2H), 4.24-4.11 (m, 2H), 2.85-2.67 (m, 1H), 2.01-1.97 (m, 2H), 1.73-1.67 (m, 2H); ESI-MS(m/z): 586.0 $[M+H]^+$.

## Example 3

### Preparation of *N*-(5-(4-(4-acryloylpiperazin-1-yl)-7-chloroquinazolin-6-yl)-2-methoxypylidin-3-yl)methanesulfonamide

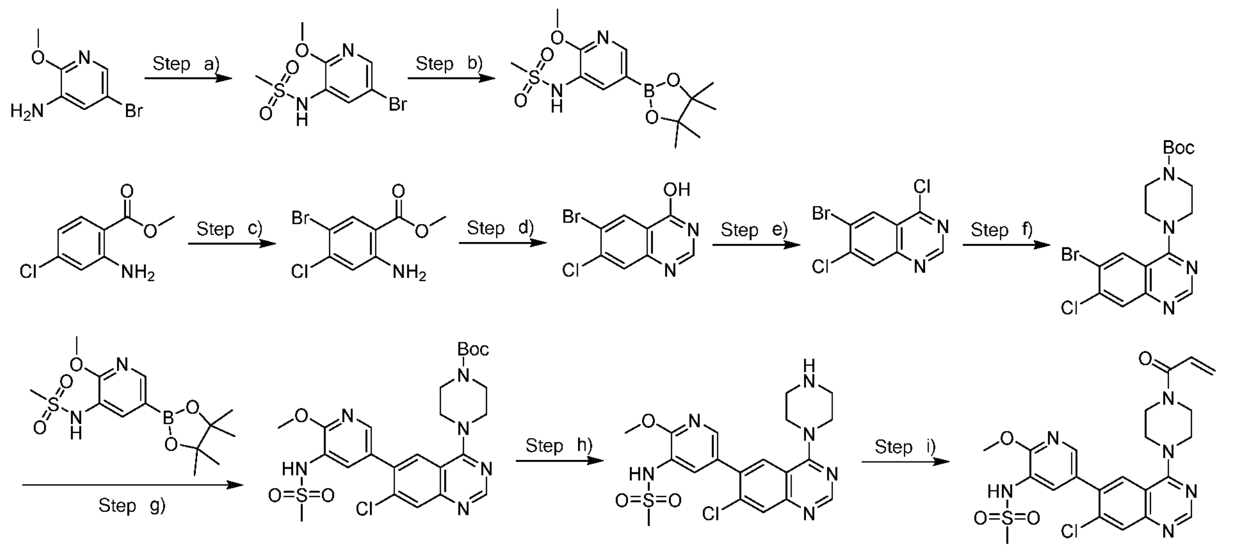


Step a): Preparation of *N*-(5-bromo-2-methoxypyridin-3-yl)methanesulfonamide

Methanesulfonyl chloride (2.3 g, 20.087 mmol) and pyridine (4.8 g, 60.261 mmol) were dissolved in acetonitrile (40 mL), with 5-bromo-2-methoxypyridin-3-amine (3.4 g, 16.739 mmol) added. After the addition was completed, the reaction mixture was stirred at room temperature for 6 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=5/1 to 1:1). *N*-(5-bromo-2-methoxypyridin-3-yl)methanesulfonamide was obtained, yield: 42.3%; ESI-MS(m/z): 280.9 $[M+H]^+$.

Step b): Preparation of *N*-(2-methoxy-5-(4,4,5,5-tetramethyl-1,3,2-dioxaborolan-2-yl)pyridin-3-yl)methanesulfonamide

*N*-(5-bromo-2-methoaypyridin-3-yl)methanesulfonamide (500 mg, 1.779 mmol), bis(pinacolato)diboron (677.93 mg, 2.669 mmol), Pd(dppf)$Cl_2$ (130.25 mg, 0.178 mmol) and potassium acetate (523.13 mg, 5.339 mmol) were added to dioxane (15 mL). After the addition was completed, in the presense of protective nitrogen, the reaction mixture was kept at 110°C and stirred for 4 h. Upon completion of the reaction, the reaction mixture was filtered. The filtrate was concentrated under reduced pressure. The crude product obtained was purified by silica gel column chromatography (Eluent: petroleum ether/ethyl acetate=5/1 to 1:1). *N*-(2-methoxy-5-(4,4,5,5-tetramethyl-1,3,2-dioxaborolan-2-yl)pyridin-3-yl)methanesulfonamide was obtained, yield: 85.7%; ESI-MS(m/z): 329.0 $[M+H]^+$.

Step c): Preparation of methyl 2-amino-5-bromo-4-chlorobenzoate

Methyl 2-amino-4-chlorobenzoate (5.0 g, 26.940 mmol) was dissolved in *N*,*N*-dimethylformamide (60 mL). Under the ice bath condition, *N*-bromosuccinimide (4.8 g, 26.940 mmol) was added to the system. After the addition was completed, the reaction mixture was stirred at room temperature overnight. Upon completion of the reaction, the reaction was quenched by adding water (80 mL), and the reaction mixture was extracted with dichloromethane (200 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=20/1 to 1/1). methyl 2-amino-5-bromo-4-chlorobenzoate was obtained, yield: 91.2%; ESI-MS(m/z): 265.9 $[M+H]^+$.

Step d): Preparation of 6-bromo-7-chloroquinazolin-4-ol

Methyl 2-amino-5-bromo-4-chlorobenzoate (1.0 g, 3.781 mmol) was added to methylamide (10 mL). The reaction mixture was heated to 200°C and stirred for 3 h. Upon completion of the reaction, the reaction mixture was cooled to room temperature, quenched by adding water (50 mL). The solution was filtered after solid precipitation appeared. The filter cake was dried to obtain 6-Bromo-7-chloroquinazolin-4-ol, yield: 67.2%; ESI-MS(m/z): 260.9 $[M+H]^+$.

Step e): Preparation of 6-bromo-4,7-dichloroquinazoline

6-bromo-7-chloroquinazolin-4-ol (660 mg, 2.543 mmol) and *N*,*N*-diisopropylethylamine (0.5 mL) were dissolved in thionyl chloride (10 mL). The reaction mixture was heated to 100°C and stirred for 3 h. Upon completion of the reaction, the reaction mixture was concentrated under reduced pressure, the reaction was quenched by adding water (30 mL). The reaction mixture was extracted with ethyl acetate (60 mL×2). The organic phases were combined, dried over anhydrous sodium sulfate and then filtered. The filtrate was concentrated under reduced pressure. The crude product obtained was purified by silica gel column chromatography (Eluent: petroleum ether/ethyl acetate=30/1 to 10/1). 6-Bromo-4,7-dichloroquinazoline was obtained, yield: 38.2%; ESI-MS(m/z): 278.9$[M+H]^+$.

Step f): Preparation of tert-butyl 4-(6-bromo-7-chloroquinazolin-4-yl)piperazine-1-carboxylate

6-Bromo-4,7-dichloroquinazoline (270 mg, 0.972 mmol), 1-tert-butoxycarbonylpiperazine (271.5 mg, 1.458 mmol) and triethylamine (294.9 mg, 2.916 mmol) were added to dichloromethane (5 mL) under the condition of stirring. The reaction mixture was stirred at room temperature for 1 h. Upon completion of the reaction, the reaction was quenched by adding water (20 mL). The reaction mixture was extracted with ethyl acetate (50 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=10/1 to 3/1). Tert-butyl 4-(6-bromo-7-chloroquinazolin-4-yl)piperazine-1-carboxylate was obtained, yield: 67.3%; ESI-MS(m/z): 429.0$[M+H]^+$.

Step g): Preparation of tert-butyl 4-(7-chloro-6-(6-methoxy-5-(methylsulfonamido)pyridin-3-yl)quinazolin-4-yl)piperazine-1-carboxylate

Tert-butyl 4-(6-bromo-7-chloroquinazolin-4-yl)piperazine-1-carboxylate (280 mg, 0.655 mmol), *N*-(2-methoxy-5-(4,4,5,5-tetramethyl-1,3,2-dioxaborolan-2-yl)pyridin-3-yl)methanesulfonamide (429.7 mg, 1.310 mmol), Pd(dppf)$Cl_2$ (47.9 mg, 0.065 mmol) and potassium carbonate (181 mg, 1.310 mmol) were added to dioxane (4 mL) and water (0.8 mL) under the condition of stirring. After the addition was completed, in the presense of protective nitrogen, the reaction mixture was heated to reflux and stirred for 2 h. Upon completion of the reaction, the reaction was quenched by adding water (20 mL), and the reaction mixture was extracted with dichloromethane (50 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=3/1 to 1/1). 4-(7-chloro-6-(6-methoxy-5-(methylsulfonamido)pyridin-3-yl)quinazolin-4-yl)piperazine-1-carboxylate was obtained, yield: 61.2%; ESI-MS(m/z): 549.2 $[M+H]^+$.

Step h): Preparation of *N*-(5-(7-chloro-4-(piperazin-1-yl)quinazolin-6-yl)-2-methoxypyridin-3-yl)methanesulfonamide

Tert-butyl 4-(7-chloro-6-(6-methoxy-5-(methylsulfonamido)pyridin-3-yl)quinazolin-4-yl)piperazine-1-carboxylate (120 mg, 0.219 mmol) was dissolved in dichloromethane (3 mL). Under the ice bath condition, trifluoroacetic acid (1 mL) was slowly added to the system. After the addition was completed, the reaction mixture was stirred at room temperature for 1 h. Upon completion of the reaction, under the ice bath condition, the reaction was quenched by adding saturated sodium bicarbonate solution (20 mL), and the reaction mixture was extracted with dichloromethane (30 mL×2). The organic phases were combined, dried over anhydrous sodium sulfate and filtered. The filtrate was concentrated under reduced pressure. *N*-(5-(7-chloro-4-(piperazin-1-yl)quinazolin-6-yl)-2-methoxypyridin-3-yl)methanesulfonamide was obtained. The product can be used directly in the next reaction without further purification; ESI-MS(m/z): 449.1 $[M+H]^+$.

Step i): Preparation of *N*-(5-(4-(4-acryloylpiperazin-1-yl)-7-chloroquinazolin-6-yl)-2-methoxypyridin-3-yl)methanesulfonamide

*N*-(5-(7-chloro-4-(piperazin-1-yl)quinazolin-6-yl)-2-methoxypyridin-3-yl)methanesulfonamide (95 mg, 0.212 mmol) and acrylic acid (15.3 mg, 0.212 mmol) were dissolved in dichloromethane (3 mL). At -78°C, *N,N*-diisopropylethylamine (137 mg, 1.060 mmol) and HATU (89 mg, 0.233 mmol) were slowly added to the system. After the addition was completed, the reaction mixture was kept at -78°C and stirred for 0.5 h. Upon completion of the reaction, the reaction was quenched by adding water (10 mL), and the reaction mixture was extracted with dichloromethane (50 mL×2). The organic phases were combined, dried over anhydrous sodium sulfate and filtered. The filtrate was concentrated under reduced pressure. The crude product obtained was purified by Prep-HPLC (Method 2). *N*-(5-(4-(4-acryloylpiperazin-1-yl)-7-chloroquinazolin-6-yl)-2-methoxypyridin-3-yl)methanesulfonamide was obtained, yield 4.9%; $^1H$ NMR (400 MHz,

DMSO-$d_6$) δ 9.38 (s, 1H), 8.66 (s, 1H), 8.16 (d, *J* = 2.4 MHz, 1H), 8.02 (s, 2H), 7.83 (d, *J* = 2.4 Hz, 1H), 6.84-6.78 (m, 1H), 6.18-6.13 (m, 1H), 5.74-5.71 (m, 1H), 3.99 (s, 3H), 3.92-3.89 (m, 4H), 3.80 (s, 2H), 3.74 (s, 2H), 3.07 (s, 3H); ESI-MS(m/z): 503.0 [M+H]$^+$.

**Example 4**

**Preparation of *N*-(5-(4-(4-acryloylpiperazin-1-yl)quinazolin-6-yl)-2-methoxypyridin-3-yl)methanesulfonamide**

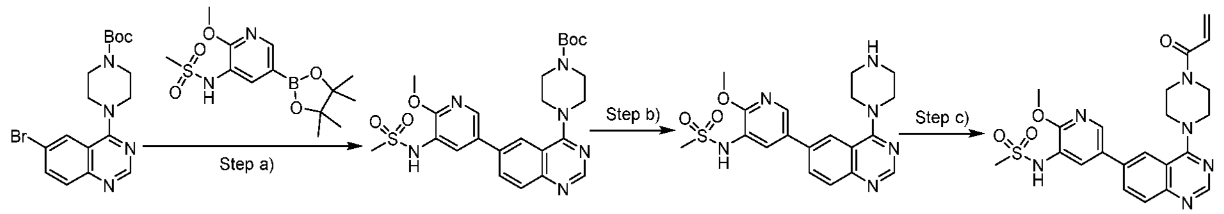


Step a): Preparation of tert-butyl 4-(6-(6-methoxy-5-(methylsulfonamido)pyridin-3-yl)quinazolin-4-yl)piperazine-1-carboxylate

Tert-butyl 4-(6-bromoquinazolin-4-yl)piperazine-1-carboxylate (300 mg, 0.763 mmol), *N*-(2-methoxy-5-(4,4,5,5-tetramethyl-1,3,2-dioxaborolan-2-yl)pyridin-3-yl)methanesulfonamide (300 mg, 0.916 mmol), Pd(dppf)$Cl_2$ (56 mg, 0.076 mmol) and cesium carbonate (751 mg, 2.290 mmol) were added to dioxane/water (10 mL, v/v=4:1). After the addition was completed, in the presense of protective nitrogen, the reaction mixture was kept at 110°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: dichloromethane/methanol=50/1 to 20/1). Tert-butyl 4-(6-(6-methoxy-5-(methylsulfonamido)pyridin-3-yl)quinazolin-4-yl)piperazine-1-carboxylate was obtained, yield: 63.7%; ESI-MS(m/z): 515.2 [M+H]$^+$.

Step b): Preparation of N-(2-methoay-5-(4-(piperazin-1-yl)quinazolin-6-yl)pyridin-3-yl)methanesulfonamide

Tert-butyl 4-(6-(6-methoxy-5-(methylsulfonamido)pyridin-3-yl)quinazolin-4-yl)piperazine-1-carboxylate (250 mg, 0.486 mmol) was dissolved in dichloromethane (8 mL). Under the ice bath condition, TFA (2 mL) was slowly added dropwise. After the addition was completed, the reaction mixture was kept at room temperature and reacted for 1h. Upon completion of the reaction, the reaction mixture was concentrated under reduced pressure. *N*-(2-methoxy-5-(4-(piperazin-1-yl)quinazolin-6-yl)pyridin-3-yl)methanesulfonamide was obtained. The product can be used directly in the next reaction without further purification. ESI-MS(m/z): 415.1 [M+H]$^+$.

Step c): Preparation of *N*-(5-(4-(4-acryloylpiperazin-1-yl)quinazolin-6-yl)-2-methoxypyridin-3-yl)methanesulfonamide

*N*-(2-methoxy-5-(4-(piperazin-1-yl)quinazolin-6-yl)pyridin-3-yl)methanesulfonamide (200 mg, 0.483 mmol) and HATU (367 mg, 0.966 mmol) were dissolved in dichloromethane (10 mL). At -78°C, *N*,*N*-diisopropylethylamine (251 mg, 1.932 mmol) and acrylic acid (52 mg, 0.725 mmol) were slowly added to the system. After the addition was completed, the reaction mixture was kept at -78°C and stirred for 2 h. Upon completion of the reaction, the reaction was quenched by adding water (50 mL), and the reaction mixture was extracted with dichloromethane (100 mL×2). The organic phases were combined, dried over anhydrous sodium sulfate and filtered. The filtrate was concentrated under reduced pressure. The crude product obtained was purified by Prep-HPLC. *N*-(5-(4-(4-acryloylpiperazin-1-yl)quinazolin-6-yl)-2-methoxypyridin-3-yl)methanesulfonamide was obtained, yield: 21.8%; $^1$H NMR (400 MHz, DMSO-$d_6$) δ 9.40 (s, 1H), 8.66 (s, 1H), 8.46 (s, 1H), 8,15-8.12 (m, 2H), 8.02 (m, 1H), 7.93-7.91 (m, 1H), 6.87-6.80 (m, 1H), 6.15 (d, *J* = 20.0 Hz, 1H), 5.76 (d, *J* = 12.0 Hz, 1H), 4.00 (s, 3H), 3.89-3.79 (m, 8H), 3.13 (s, 3H); ESI-MS(m/z): 469.0 [M+H]$^+$.

**Example 5**

**Preparation of *N*-(5-(4-((S)-4-acryloyl-3-(cyanomethyl)piperazin-1-yl)-2-(((*S*)-1-methylpyrrolidin-2-yl)methoxy)quinazolin-6-yl)-2-methoxypyridin-3-yl)-2,4-difluorobenzenesulfonamide**

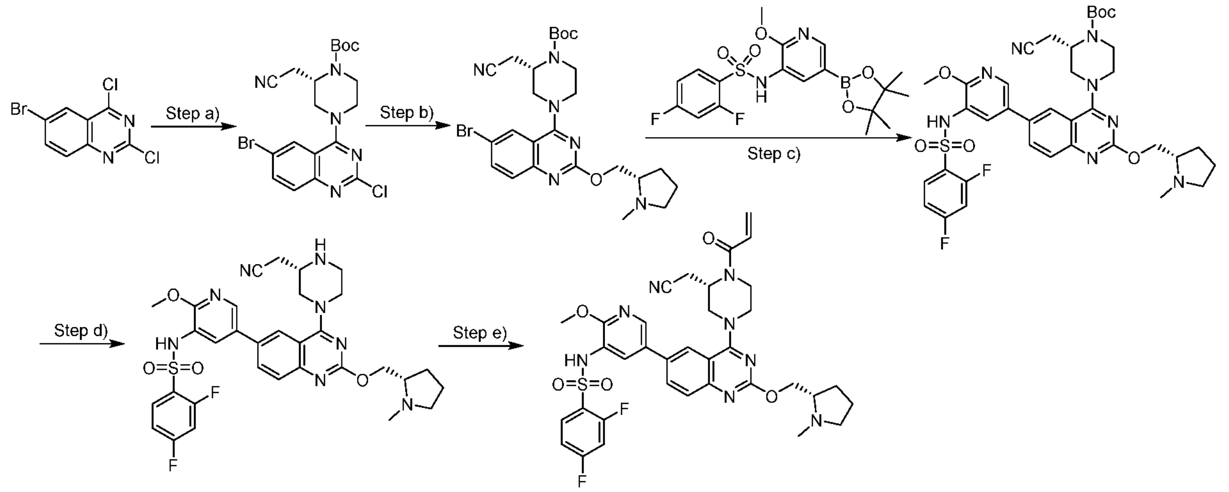


Step a): Preparation of tert-butyl (*S*)-4-(6-bromo-2-chloroquinazolin-4-yl)-2-(cyanomethyl)piperazine-1-carboxylate

6-Bromo-2,4-dichloroquinazoline (800 mg, 2.878 mmol), (S)-2-(piperazin-2-yl)acetonitrile (431.65 g, 3.453 mmol) and *N*,*N*-diisopropylethylamine (1.87 g, 14.388 mmol) were dissolved in dimethyl sulfoxide (20 mL). After the addition was completed, the reaction mixture was heated to 60°C and stirred for 1 h, with di-tert-butyl dicarbonate (1.26 g, 5.755 mmol) added. Upon completion of the reaction, the reaction was quenched by adding water (100 mL). The reaction mixture was extracted with ethyl acetate (150 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=5/1 to 3/1). Tert-butyl (*S*)-4-(6-bromo-2-chloroquinazolin-4-yl)-2-(cyanomethyl)piperazine-1-carboxylate was obtained, yield: 74.4%; ESI-MS(m/z): 466.0$[M+H]^+$,

Step b): Preparation of tert-butyl (*S*)-4-(6-bromo-2-(((*S*)-1-methylpyrrolidin-2-yl)methoxy)quinazolin-4-yl)-2-(cyanomethyl)piperazine-1-carboxylate

Sodium hydride (256.96 mg, 6.424 mmol) was suspended in tetrahydrofuran (30 mL) under nitrogen atmosphere. Under the ice bath condition, (*S*)-(1-methylpyrrolidin-2-yl)methanol (295.50 mg, 2.57 mmol) was added to the system. After the addition was completed, under the ice bath condition, the reaction mixture was stirred for 1 h. Tert-butyl (*S*)-4-(6-bromo-2-chloroquinazolin-4-yl)-2-(cyanomethyl)piperazine-1-carboxylate (300 mg, 0.642 mmol) was then added to the system. After the addition was completed, the reaction mixture was slowly heated to the room temperature and stirred for 2 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: dichloromethane/methanol=50/1 to 20/1). Tert-butyl (*S*)-4-(6-bromo-2-(((*S*)-1-methylpyrrolidin-2-yl)methoxy)quinazolin-4-yl)-2-(cyanomethyl)piperazine-1-carboxylate was obtained, yield: 85.7%; ESI-MS(m/z): 545.2 $[M+H]^+$.

Step c): Preparation of tert-butyl (S)-2-(cyanomethyl)-4-(6-(5-((2,4-difluorophenyl)sulfonamido)-6-methoxypyridin-3-yl)-2-(((*S*)-1-methylpyrrolidin-2-yl)methoxy)quinazolin-4-yl)piperazine-1-carboxylate

Tert-butyl (*S*)-4-(6-bromo-2-(((*S*)-1-methylpyrrolidin-2-yl)methoxy)quinazolin-4-yl)-2-(cyanomethyl)piperazine-1-carboxylate (300.00 mg, 0.550 mmol), 2,4-difluoro-*N*-(2-methoxy-5-(4,4,5,5-tetramethyl-1,3,2-dioxaborolan-2-yl)pyridin-3-yl)benzenesulfonamide (351.74 mg, 0.826 mmol), Pd(dppf)$Cl_2$ (40.29 mg, 0.055 mmol) and cesium carbonate (361.10 mg, 1.101mmol) were added to dioxane/water (10 mL, v/v=4:1). After the addition was completed, in the presense

of protective nitrogen, the reaction mixture was kept at 110°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: dichloromethane/methanol=50/1 to 20/1). Tert-butyl (*S*)-2-(cyanomethyl)-4-(6-(5-((2,4-difluorophenyl)sulfonamido)-6-methoxypyridin-3-yl)-2-(((*S*)-1-methylpyrrolidin-2-yl)methoxy)quinazolin-4-yl)piperazine-1-carboxylate was obtained, yield: 83.1%; ESI-MS(m/z): 765.3 $[M+H]^+$.

Step d): Preparation of *N*-(5-(4-((*S*)-3-(cyanomethyl)piperazin-1-yl)-2-(((*S*)-1-methylpyrrolidin-2-yl)methoxy)quinazolin-6-yl)-2-methoxypyridin-3-yl)-2,4-difluorobenzenesulfonamide

Tert-butyl (*S*)-2-(cyanomethyl)-4-(6-(5-((2,4-difluorophenyl)sulfonamido)-6-methoxypyridin-3-yl)-2-(((*S*)-1-methylpyrrolidin-2-yl)methoxy)quinazolin-4-yl)piperazine-1-carboxylate (350.00 mg, 0.458 mmol) was dissolved in dichloromethane (8 mL). Under the ice bath condition, TFA (2 mL) was slowly added dropwise. After the addition was completed, the reaction mixture was stirred at room temperature for 1 h. Upon completion of the reaction, the reaction mixture was concentrated under reduced pressure. *N*-(5-(4-((*S*)-3-(cyanomethyl)piperazin-1-yl)-2-(((*S*)-1-methylpyrrolidin-2-yl)methoxy)quinazolin-6-yl)-2-methoxypyridin-3-yl)-2,4-difluorobenzenesulfonamide was obtained. The product can be used directly in the next reaction without purification. ESI-MS(m/z): 665.3 $[M+H]^+$.

Step e): Preparation of *N*-(5-(4-((*S*)-4-acryloyl-3-(cyanomethyl)piperazin-1-yl)-2-(((*S*)-1-methylpyrrolidin-2-yl)methoxy)quinazolin-6-yl)-2-methoxypyridin-3-yl)-2,4-difluorobenzenesulfonamide

*N*-(5-(4-((*S*)-3-(cyanomethyl)piperazin-1-yl)-2-(((*S*)-1-methylpyrrolidin-2-yl)methoxy)quinazolin-6-yl)-2-methoxypyridin-3-yl)-2,4-difluorobenzenesulfonamide (250.00 mg, 0.375 mmol) and HATU (285.71 mg, 0.750 mmol) were dissolved in dichloromethane (10 mL). At -78°C *N*,*N*-diisopropylethylamine (244.36 mg, 1.125 mmol) and acrylic acid (40.60 mg, 0.564 mmol) were slowly added to the system. After the addition was completed, the reaction mixture was kept at -78°C and stirred for 2 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by Prep-HPLC. *N*-(5-(4-((*S*)-4-acryloyl-3-(cyanomethyl)piperazin-1-yl)-2-(((*S*)-1-methylpyrrolidin-2-yl)methoxy)quinazolin-6-yl)-2-methoxypyridin-3-yl)-2,4-difluorobenzenesulfonamide was obtained, yield: 10.4%; $^1H$ NMR (400 MHz, DMSO-$d_6$) δ 10.52-9.59 (m, 1H), 8.45 (s, 1H), 8.12 (s, 1H), 7.96-7.91 (m, 2H), 7.78-7.70 (m, 2H), 7.52-7.48 (m, 1H), 7.23 -7.18 (m, 1H), 6.98-6.70 (m, 1H), 6.22 (d, *J* = 8.0 Hz, 1H), 5.73-5.71 (m, 1H), 5.08-4.71 (m, 1H), 4.44-4.03 (m, 5H), 3.75-3.71 (m, 1H), 3.69 (s, 3H), 3.53-3.35 (m, 4H), 3.10-2.91 (m, 4H), 2.70 (s, 1H), 2.32-2.40 (m, 1H), 2.10-1.98 (m, 1H), 1.77-1.72 (m, 3H); ESI-MS(m/z): 719.5 $[M+H]^+$.

**Example 6**

**Preparation of (E)-2,4-difluoro-*N*-(2-methoxy-5-(4-(4-(4-oxopent-2-enoyl)piperazin-1-yl)quinazoline-6-yl)pyridin-3-yl)benzenesulfonamide**

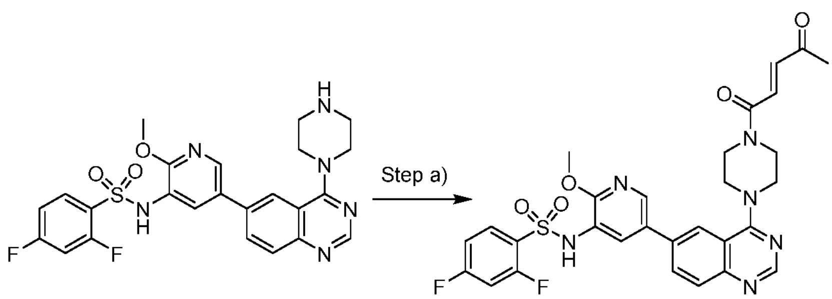


2,4-difluoro-*N*-(2-methoxy-5-(4-(piperazin-1-yl)quinazolin-6-yl)pyridin-3-yl)benzenesulfonamide (40 mg, 0.078 mmol) and (*E*)-4-oxopent-2-enoic acid (9 mg, 0.079 mmol) were dissolved in tetrahydrofuran (2 mL). At -78°C, *N,N*-diisopropylethylamine (101 mg, 0.781 mmol) and 1-propylphosphonic anhydride (75 mg, 0.236 mmol, 50% wt) were slowly added to the system. After the addition was completed, the reaction mixture was kept at -78°C and stirred for 1 h. Upon

completion of the reaction, the reaction was quenched by adding water (10 mL), and the reaction mixture was extracted with dichloromethane (30 mL×2). The organic phases were combined, dried over anhydrous sodium sulfate and filtered. The filtrate was concentrated under reduced pressure to provide the crude residue. The crude product obtained was purified by Prep-HPLC. (*E*)-2,4-difluoro-*N*-(2-methoxy-5-(4-(4-(4-oxopent-2-enoyl)piperazin-1-yl)quinazoline-6-yl)pyridin-3-yl)benzenesulfonamide was obtained, yield: 38.6%; $^1H$ NMR (400 MHz, DMSO-$d_6$) $\delta$ 10.34 (s, 1H), 8.67 (s, 1H), 8.46 (s, 1H), 8.14-8.08 (m, 2H), 8.03-7.99 (m, 1H), 7.95-7.90 (m, 1H), 7.81-7.74 (m, 1H), 7.61-7.52 (m, 1H), 7.45 (d,*J* = 16.0 Hz, 1H), 7.25-7.18 (m, 1H), 6.74 (d, *J* = 16.0 Hz, 1H), 3.93-3.87 (m, 6H), 3.83-3.79 (m, 2H), 3.68 (s, 3H), 2.37 (s, 3H); ESI-MS(m/z): 609.0 $[M+H]^+$.

**Example 7**

**Preparation of (*S*)-N-(5-(4-(4-acryloyl-2-methylpiperazin-1-yl)quinazolin-6-yl)-2-methoxypyridin-3-yl)-2,4-difluorobenzenesulfonamide**

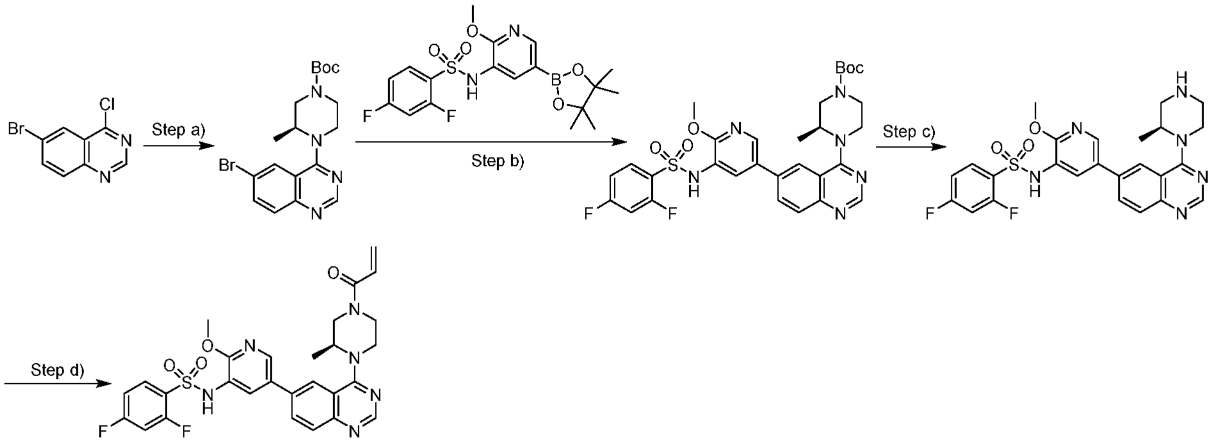


Step a): Preparation of tert-butyl (*S*)-4-(6-bromoquinazolin-4-yl)-3-methylpiperazine-1-carboxylate

6-Bromo-4-chloroquinazoline (1.0 g, 4.133 mmol) and tert-butyl (*S*)-3-methylpiperazine-1-carboxylate (827,0 g, 4.133 mmol) were dissolved in dimethyl sulfoxide (10 mL), with N,N-diisopropylethylamine (1.1 g, 8.266 mmol) added. After the addition was completed, the reaction mixture was heated to 50°C and stirred for 1 h. Upon completion of the reaction, the reaction was quenched by adding water (50 mL). The reaction mixture was extracted with ethyl acetate (100 mL×2 times). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 10/1 to 3/1). (S)-4-(6-bromoquinazolin-4-yl)-3-methylpiperazine-1-carboxylate was obtained, yield: 51.8%; ESI-MS(m/z): 407.10 $[M+H]^+$.

Step b): Preparation of tert-butyl (S)-4-(6-(5-((2,4-difluorophenyl)sulfonamido)-6-methoxypyridin-3-yl)quinazolin-4-yl)-3-methylpiperazine-1-carboxylate

(S)-4-(6-bromoquinazolin-4-yl)-3-methylpiperazine-1-carboxylate (377 mg, 0.929 mmol), 2,4-difluoro-N-(2-methoxy-5-(4,4,5,5-tetramethyl-1,3,2-dioxaborolan-2-yl)pyridin-3-yl)benzenesulfonamide (330 mg, 0.774 mmol), Pd(dppf)$Cl_2$ (113 mg, 0.155 mmol) and cesium carbonate (503 mg, 1.548 mmol) were added to dioxane/water mixed solvent (10 mL, v/v=10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2 times). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl (*S*)-4-(6-(5-((2,4-difluorophenyl)sulfonamido)-6-methoxypyridin-3-yl)quinazolin-4-yl)-3-methylpiperazine-1-carboxylate was obtained, yield: 54.8%; ESI-MS(m/z): 613.20 $[M+H]^+$.

Step c): Preparation of (*S*)-2,4-difluoro-*N*-(2-methoxy-5-(4-(2-methylpiperazin-1-yl)quinazolin-6-yl)pyridin-3-yl)benzenesulfonamide

Tert-butyl (*S*)-4-(6-(5-((2,4-difluorophenyl)sulfonamido)-6-methoxypyridin-3-yl)quinazolin-4-yl)-3-methylpiperazine-1-carboxylate (260.0mg, 0.415 mmol) was dissolved in dichloromethane (4 mL). Under the ice bath condition, trifluoroacetic acid (4 mL) was slowly added dropwise. After the addition was completed, the reaction mixture was stirred at room temperature for 1 h. Upon completion of the reaction, the reaction mixture was concentrated under reduced pressure. (*S*)-2,4-difluoro-N-(2-methoxy-5-(4-(2-methylpipemzin-1-yl)quinazolin-6-yl)pyridin-3-yl)benzenesulfonamide was obtained. ESI-MS(m/z): 527.2 $[M+H]^+$.

Step d): Preparation of (*S*)-*N*-(5-(4-(4-acryloyl-2-methylpiperazin-1-yl)quinazolin-6-yl)-2-methoxypyridin-3-yl)-2,4-difluorobenzenesulfonamide

(*S*)-2,4-difluoro-*N*-(2-methoxy-5-(4-(2-methylpiperazin-1-yl)quinazolin-6-yl)pyridin-3-yl)benzenesulfonamide (130.0 mg, 0.247 mmol) was dissolved in dichloromethane (10 mL). The reaction system was cooled to -78°C, with *N,N*-diisopropylethylamine (127.5 mg, 0.988 mmol), acrylic acid (17.8 mg, 0.247mmol) and HATU (112.7 mg, 0.296 mmol) added successively. After the addition was completed, the reaction mixture was kept at -78°C and stirred for 1 h. Upon completion of the reaction, the reaction was quenched by adding water (5 mL). The reaction mixture was extracted by adding dichloromethane (10 mL×2 times). The organic phases were combined, dried over anhydrous sodium sulfate and filtered. The filtrate was concentrated under reduced pressure. The crude product obtained was purified by Prep-HPLC. (*S*)-*N*-(5-(4-(4-acryloyl-2-methylpiperazin-1-yl)quinazolin-6-yl)-2-methoxypyridin-3-yl)-2,4-difluorobenzenesulfonamide was obtained, yield: 10.5%; $^1$H NMR (400 MHz, DMSO-$d_6$) δ 10.5 (s, 1H), 8.74 (s, 1H), 8.53 (s, 1H), 8.19-7.87 (m, 4H), 7.85 (s, 1H), 7.65 (s, 1H), 7.30 (s, 1H), 7.0 (s, 1H), 6.30-6.28 (m, 1H), 5.85-5.77 (m, 1H),4.84 (s, 1H),4.53 (d, *J* = 12.0 Hz, 1H), 4.44-4.21 (m, 2H), 4.05 (d, *J* =16.0 Hz,1H), 3.78-3.70 (m, 4H), 3.25-3.03 (m, 1H),1.0 (t, *J* = 12.0 Hz, 3H); ESI-MS(m/z): 581.2 $[M+H]^+$.

**Example 8**

**Preparation of (*S*,*E*)-2,4-difluoro-*N*-(2-methoxy-5-(4-(2-methyl-4-(4-oxopent-2-enoyl)piperazin-1-yl)quinazolin-6-yl)pyridin-3-yl)benzenesulfonamide**

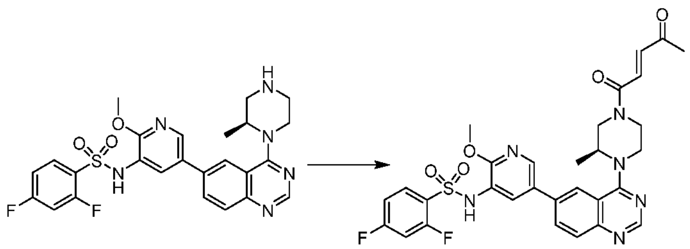

(*S*)-2,4-difluoro-*N*-(2-methoxy-5-(4-(2-methylpiperazin-1-yl)quinazolin-6-yl)pyridin-3-yl)benzenesulfonamide

(60.0 mg, 0.114 mmol) was dissolved in tetrahydrofuran (2 mL). The reaction system was cooled to -78°C, with *N,N*-diisopropylethylamine (73.53 mg, 0.570 mmol), 3-acetylacrylic acid (13.0 mg, 0.114 mmol) and 50% $T_3P$ ethyl acetate solution (72.5 mg, 0.114 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (*S*,*E*)-2,4-difluoro-N-(2-methoxy-5-(4-(2-methyl-4-(4-oxopent-2-enoyl)piperazin-1-yl)quinazolin-6-yl)pyridin-3-yl)benzenesulfonamide was obtained, yield: 28.2%; $^1$H NMR (400 MHz, DMSO-$d_6$) δ 10.5 (s, 1H), 8.67 (s, 1H), 8.46 (s, 1H), 8.11-8.01 (m, 3H), 7.92 (d, *J* =8.0 Hz, 1H), 7.79-7.77 (m, 1H), 7.57-7.48 (m, 2H), 7.22-7.21 (m, 1H), 6.79-6.70 (m, 1H), 4.81-4.76 (m, 1H), 4.43 (d, *J* = 12.0 Hz, 1H), 4.30-4.20 (m, 2H), 4.01 (d, *J* = 12.0 Hz, 1H), 3.70-3.67 (m, 5H), 2.38-2.21 (m, 3H), 1.43-1.31 (m, 3H); ESI-MS(m/z): 622.6 $[M+H]^+$.

**Example 9**

**Preparation of (*S*)-2,4-difluoro-*N*-(5-(4-(4-(2-fluoroacryloyl)-2-methylpiperazin-1-yl)quinazolin-6-yl)-2-methoxypyridin-3-yl)benzenesulfonamide**

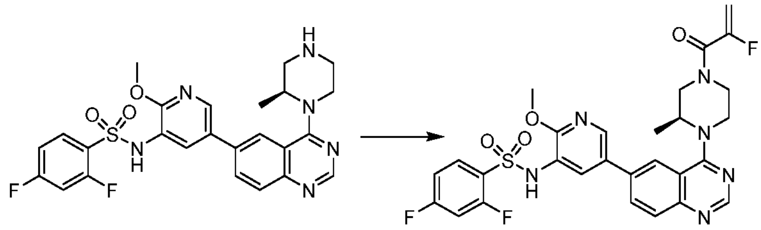

(*S*)-2,4-difluoro-*N*-(2-methoxy-5-(4-(2-methylpiperazin-1-yl)quinazolin-6-yl)pyridin-3-yl)benzenesulfonamide

(75.0 mg, 0.143 mmol) was dissolved in tetrahydrofuran (2 mL). The reaction system was cooled to -78°C, with *N,N*-diisopropylethylamine (91.9 mg, 0.713 mmol), 2-fluoroacrylic acid (12.9 mg, 0.143 mmol) and 50% $T_3P$ ethyl acetate solution (91.0 mg, 0.143 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (S)-2,4-difluoro-M-(5-(4-(4-(2-fluoroacryloyl)-2-methylpiperazin-1-yl)quinazolin-6-yl)-2-methoxypyridin-3-yl)benzenesulfonamide was obtained, yield: 25.8%; $^1$H NMR (400 MHz, DMSO-*d*$_6$) δ 10.3 (brs, 1H), 8.64 (s, 1H), 8.44 (s, 1H), 8.08-8.00 (m, 3H), 7.99-7.88 (m, 1H), 7.88-7.73 (m, 1H), 7.54-7.52 (m, 1H), 7.19 (d, *J* = 4.0 Hz, 1H), 5.34-5.18 (m, 2H), 4.80 (s, 1H), 4.40-3.85 (m, 3H), 3.83-3.49 (m, 6H), 1.30 (d, *J*= 8.0 Hz, 3H); ESI-MS(m/z): 599.6 $[M+H]^+$.

**Example 10**

**Preparation of (*E*)-*N*-(5-(4-(4-(3-(1*H*-1,2,3-triazol-1-yl)acryloyl)piperazin-1-yl)quinazolin-6-yl)-2-methoxypyridin-3-yl)-2,4-difluorobenzenesulfonamide**

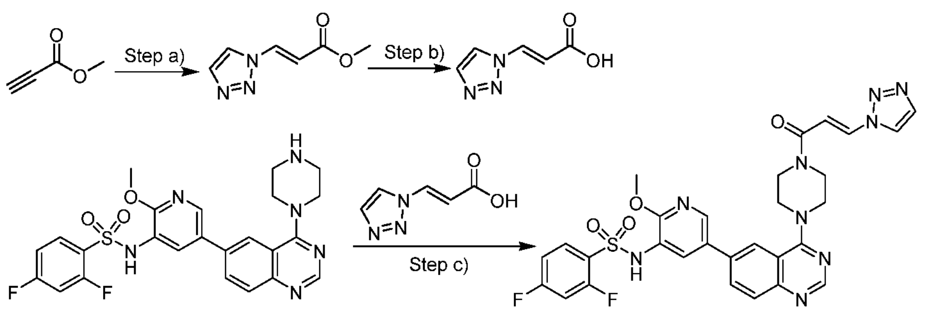


Step a): Preparation of methyl (*E*)-3-(1*H*-1,2,3-triazol-1-yl)acrylate

Triazole (5 g, 72.359 mmol) was dissolved in methyl propiolate (8 mL), the reaction solution was heated to 100°C and stirred for 12 h. Upon completion of the reaction, add ethyl acetate to the reaction solution (50 mL). After yellow solid precipitation appeared, the solution was filtered. The filter cake was collected and eluted with appropriate amount of ethyl acetate, vacuum dried to obtain methyl (*E*)-3-(1*H*-1,2,3-triazol-1-yl)acrylate, yield: 23.5%; ESI-MS(m/z): 154.1 $[M+H]^+$.

Step b): Preparation of (*E*)-3-(1*H*-1,2,3-triazol-1-yl)acrylic acid

Methyl (*E*)-3-(1*H*-1,2,3-triazol-1-yl)acrylate (500 mg, 3.266 mmol) was dissolved in dilute $H_2SO_4$ (1 M, 10 mL). The reaction solution was heated to reflux, stirred and reacted for 3 h. Upon completion of the reaction, the solution was

filtered. The filter cake was collected and washed with appropriate amount of water, vacuum dried to obtain (*E*)-3-(1*H*-1,2,3-triazol-1-yl)acrylic acid, yield: 66.1%; ESI-MS(m/z): 138.0 $[M+H]^+$.

Step c): Preparation of (*E*)-*N*-(5-(4-(4-(3-(1*H*-1,2,3-triazol-1-yl)acryloyl)piperazin-1-yl)quinazolin-6-yl)-2-methoxypyridin-3-yl)-2,4-difluorobenzenesulfonamide

(*E*)-3-(1*H*-1,2,3-triazol-1-yl)acrylic acid (50 mg, 0.359 mmol) and HATU (71 mg, 0.187 mmol) were dissolved in N,N-dimethylformamide (2 mL). The reaction system was cooled to -41°C, with *N*,*N*-diisopropylethylamine (101 mg, 0.781 mmol) and 2,4-difluoro-N (2-methoay-5-(4-(piperazin-1-yl)quinazolin-6-yl)pyridin-3-yl)benzenesulfonamide (80 mg, 0.156 mmol) added successively. After the addition was completed, the reaction mixture was kept at -41°C and stirred for 1 h. Upon completion of the reaction, the reaction solution was purified by Prep-HPLC. (*E*)-*N*-(5-(4-(4-(3-(1*H*-1,2,3-triazol-1-yl)acryloyl)piperazin-1-yl)quinazolin-6-yl)-2-methoxypyridin-3-yl)-2,4-difluorobenzenesulfonamide was obtained, yield: 43.3%; $^1$H NMR (400 MHz, DMSO-$d_6$) δ 10.33 (s, 1H), 8.75 (s, 1H), 8.67 (s, 1H), 8.49-8.48 (m, 1H), 8.25-8.21 (m, 1H), 8.16-8.09 (m, 2H), 8.03 (d, *J* = 8.0 Hz, 1H), 7.94-7.91 (m, 2H), 7.81-7.75 (m, 1H), 7.60-7.54 (m, 1H), 7.49-7.44 (m, 1H), 7.25-7.19 (m, 1H), 3.97-3.93 (m, 6H), 3.87-3.84 (m, 2H), 3.68 (s, 3H); ESI-MS(m/z): 634.0 $[M+H]^+$.

**Example 11**

**Preparation of (*E*)-*N*-(5-(4-(4-(4-(dimethylamino)but-2-enoyl)piperazin-1-yl)quinazolin-6-yl)-2-methoxypyridin-3-yl)-2,4-difluorobenzenesulfonamide**

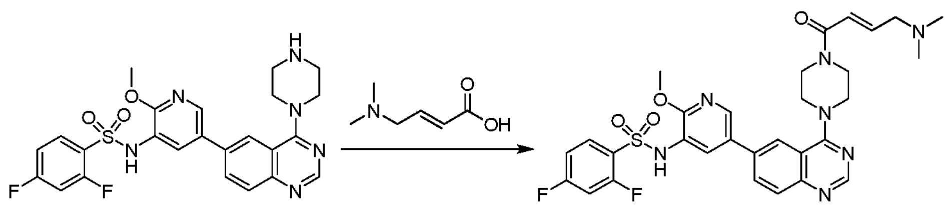

(*E*)-4-(dimethylamino)but-2-enoic acid (40 mg, 0.310 mmol) and HATU (71 mg, 0.187 mmol) were dissolved in *N,N*-dimethylformamide (2 mL). The reaction system was cooled to 0°C, with *N*,*N*-diisopropylethylamine (101 mg, 0.781 mmol) and 2,4-difluoro-*N*-(2-methoxy-5-(4-(piperazin-1-yl)quinazolin-6-yl)pyridin-3-yl)benzenesulfonamide (80 mg, 0.156 mmol) added successively. After the addition was completed, the reaction mixture was kept at 0°C and stirred for 1 h. Upon completion of the reaction, the reaction solution was purified by Prep-HPLC. (*E*)-*N*-(5-(4-(4-(4-(dimethylamino)but-2-enoyl)piperazin-1-yl)quinazolin-6-yl)-2-methoxypyridin-3-yl)-2,4-difluorobenzenesulfonamide was obtained, yield: 47.6%; $^1$H NMR (400 MHz, DMSO-$d_6$) δ 8.65 (s, 1H), 8.38-8.36 (m, 1H), 8.13-8.02 (m, 3H), 7.96 (d, *J* =8.0 Hz, 1H), 7.92-7.88 (m, 1H), 7.82-7.75 (m, 1H), 7.54-7.48 (m, 1H), 7.22-7.16 (m, 1H), 6.68-6.65 (m, 2H), 3.88-3.85 (m, 4H), 3.84-3.81 (m, 2H), 3.79-3.76 (m, 2H), 3.70 (s, 3H), 3.22-3.19 (m, 2H), 2.28 (s, 6H); ESI-MS(m/z): 624.0 $[M+H]^+$.

**Example 12**

**Preparation of *N*-(5-(4-(2-acryloyl-2,6-diazaspiro[3.4]octan-6-yl)quinazolin-6-yl)-2-methoxypyridin-3-yl)-2,4-difluorobenzenesulfonamide**

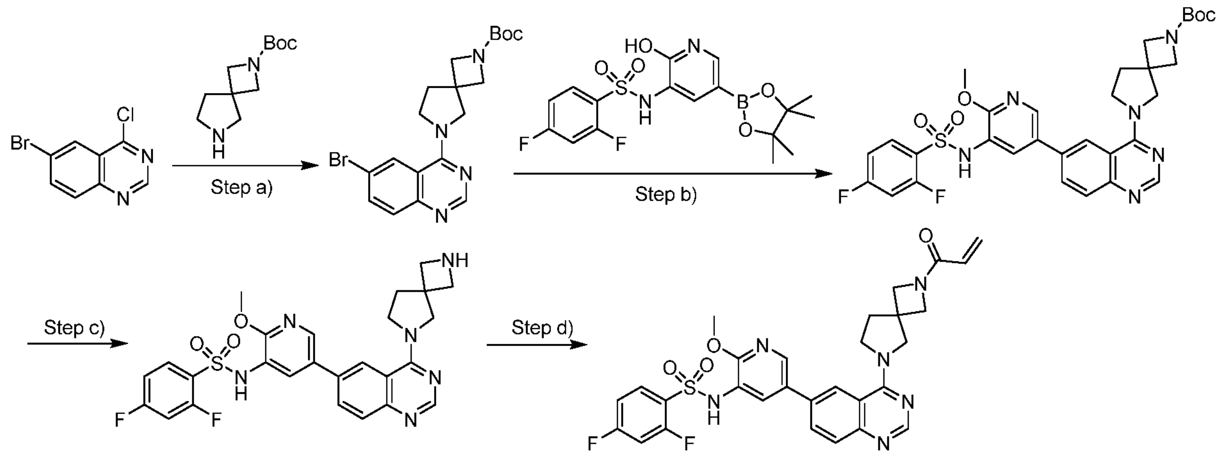


Step a): Preparation of tert-butyl 6-(6-bromoquinazolin-4-yl)-2,6-diazaspiro[3.4]octane-2-carboxylate

6-Bromo-4-chloroquinazoline (500 mg, 2.06 mmol) and tert-butyl 2,6-diazaspiro[3.4]octane-2-carboxylate (523 mg, 2.46 mmol) were dissolved in dimethyl sulfoxide (10 mL), with *N*,*N*-diisopropylethylamine (799 mg, 6.18 mmol) added. After the addition was completed, the reaction mixture was heated to 50°C and stirred for 1 h. Upon completion of the reaction, the reaction solution was cooled to 25°C and added to methyl tert-butyl ether (120 mL) dropwise.The mixture was stirred for 20 min. After solid precipitation appeared, the solution was filtered. The solid was vacuum dried to obtain tert-butyl 6-(6-bromoquinazolin-4-yl)-2,6-diazaspiro[3.4]octane-2-carboxylate, yield: 46.4%; ESI-MS(m/z): 419.1[M+H]$^+$.

Step b): Preparation of tert-butyl 6-(6-(5-((2,4-difluorophenyl)sulfonamido)-6-methoxypyridin-3-yl)quinazolin-4-yl)-2,6-diazaspiro[3.4]octane-2-carboxylate

Tert-butyl 6-(6-bromoquinazolin-4-yl)-2,6-diazaspiro[3.4]octane-2-carboxylate (350 mg, 0.835 mmol), 2,4-difluoro-*N*-(2-hydroxy-5-(4,4,5,5-tetramethyl-1,3,2-dioxaborolan-2-yl)pyridin-3-yl)benzenesulfonamide (350 mg, 0.835 mmol), Pd(dppf)$Cl_2$ (120 mg, 0.167 mmol) and cesium carbonate (540 mg, 1.67 mmol) were added to dioxane/water mixed solvent (16.5 mL, v/v=10:1). After the addition was completed, in the presense of protective nitrogen, the reaction mixture was heated to 90°C and stirred for 0.5 h. Upon completion of the reaction, the reaction mixture was cooled to 25°C, with reaction quenched by adding water (20 mL), extracted with ethyl acetate (20 mL×3). The organic phase was washed with saturated brine (15 mL), and concentrated under reduced pressure. The crude product obtained was purified by silica gel column chromatography (Eluent: methanol/dichloromethane=100/1 to 96/4). Tert-butyl 6-(6-(5-((2,4-difluorophenyl)sulfonamido)-6-methoxypyridin-3-yl)quinazolin-4-yl)-2,6-diazaspiro[3.4]octane-2-carboxylate (280 mg) was obtained, yield: 52.4%; ESI-MS(m/z): 639.21 [M+H]$^+$.

Step c): Preparation of *N*-(5-(4-(2,6-diazaspiro[3.4]octan-6-yl)quinazolin-6-yl)-2-methoxypyfidin-3-yl)-2,4-difluorobenzenesulfonamide

Tert-butyl 6-(6-(5-((2,4-difluorophenyl)sulfonamido)-6-methoxypyridin-3-yl)quinazolin-4-yl)-2,6-diazaspiro[3.4] octane-2-carboxylate (280 mg, 0.5 mmol) was dissolved in dichloromethane (10 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. The crude product obtained was added to methyl tert-butyl ether and slurried for 20 min to precipitate solid and filtered. *N*-(5-(4-(2,6-diazaspiro[3.4]octan-6-yl)quinazolin-6-yl)-2-methoxypyridin-3-yl)-2,4-difluorobenzenesulfonamide trifluoroacetate was obtained. ESI-MS(m/z): 539.16 [M+H]$^+$.

Step d): Preparation of *N*-(5-(4-(2-acryloyl-2,6-diazaspiro[3.4]octan-6-yl)quinazolin-6-yl)-2-methoxypyridin-3-yl)-2,4-difluorobenzenesulfonamide

*N*-(5-(4-(2,6-diazaspiro[3.4]octan-6-yl)quinazolin-6-yl)-2-methoxypyridin-3-yl)-2,4-difluorobenzenesulfonamide trifluoroacetate (100 mg, 0.153 mmol) was added to tetrahydrofuran (2 mL). In the presense of protective nitrogen, the reaction system was cooled to -70°C, with *N*,*N*-diisopropylethylamine (118 mg, 0.918 mmol), acrylic acid (11 mg, 0.153 mmol) and $T_3P$ (195 mg, 0.307 mmol) added successively. After the addition was completed, the reaction mixture

was kept at -70°C and stirred for 0.5 h. Upon completion of the reaction, the product was purified by Prep-HPLC. M-(5-(4-(2-acryloyl-2,6-diazaspiro[3.4]octan-6-yl)quinazolin-6-yl)-2-methoxypyridin-3-yl)-2,4-difluorobenzenesulfonamide (20 mg) was obtained, yield: 22.0%; $^1$H NMR (400 MHz, DMSO-$d_6$) δ 10.33 (brs, 1H), 8.50-8.47 (s, 2H), 8.46 (s, 1H), 8.03-8.00 (m, 2H), 7.82-7.75 (m, 2H), 7.59-7.54 (m, 1H), 7.24-7.19 (m, 1H), 6.35-6.28 (m, 1H), 6.13 (d, *J* = 16.0 Hz, 1H),5.69 (d, *J* = 12.0 Hz, 1H), 4.32 (d, *J* = 8.0 Hz, 1H), 4.21-4.19 (m, 3H), 4.05-4.00 (m, 3H), 3.94 (d, *J* = 12 Hz, 1H), 3.69 (s, 3H), 2.27-2.23 (m, 2H); ESI-MS(m/z): 593.0 [M+H]$^+$.

**Example 13**

**Preparation of (*E*)-2,4-difluoro-*N*-(2-methoxy-5-(4-(2-(4-oxopent-2-enoyl)-2,6-diazaspiro[3.4]octan-6-yl)quinazolin-6-yl)pyridin-3-yl)benzenesulfonamide**

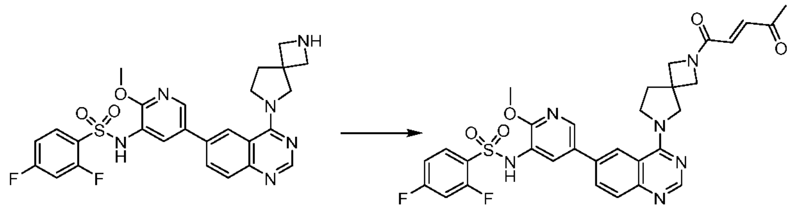

*N*-(5-(4-(2,6-diazaspiro[3.4]octan-6-yl)quinazolin-6-yl)-2-methoxypyridin-3-yl)-2,4-difluorobenzenesulfonamide trifluoroacetate (100.0 mg, 0.154 mmol) was added to tetrahydrofuran (2 mL). The reaction system was cooled to - 70°C, with *N*,*N*-diisopropylethylamine (121 mg, 0.924 mmol), acrylic acid (18 mg, 0.154 mmol) and $T_3P$ (170 mg, 0.278 mmol) added successively. After the addition was completed, the reaction mixture was kept at -70°C and stirred for 0.5 h. Upon completion of the reaction, the reaction mixture was filtered and purified by Prep-HPLC. (*E*)-2,4-difluoro-*N*-(2-methoxy-5-(4-(2-(4-oxopent-2-enoyl)-2,6-diazaspiro[3.4]octan-6-yl)quinazolin-6-yl)pyridin-3-yl)benzenesulfonamide was obtained, yield: 34.0%; $^1$H NMR (400 MHz, DMSO-$d_6$) δ 10.32 (br, 1H), 8.49 (s, 1H), 8.45 (s, 1H), 8.37 (s, 1H), 8.03-7.99 (m, 2H), 7.82 (d, *J* = 8.0 Hz, 1H), 7.81-7.75 (m, 1H), 7.59-7.53 (m, 1H), 7.23-7.19 (m, 1H), 6.90 (d, *J* = 16.0 Hz, 1H),6.74 (d, *J* = 12.0 Hz, 1H), 4.42 (d, *J* = 8.0 Hz, 1H), 4.31 (m, *J* = 8.0 Hz, 1H), 4.30-4.20 (m, 2H), 4.15-4.05 (m, 1H), 4.03-3.97 (m, 3H), 3.69 (s, 3H), 2.34 (s, 3H), 2.28-2.25 (m, 2H); ESI-MS(m/z): 635.0 [M+H]$^+$.

**Example 14**

**Preparation of *N*-(5-(4-((2*S*,5*R*)-4-acryloyl-2,5-dimethylpiperazin-1-yl)quinazolin-6-yl)-2-methoxypyridin-3-yl)-2,4-difluorobenzenesulfonamide**

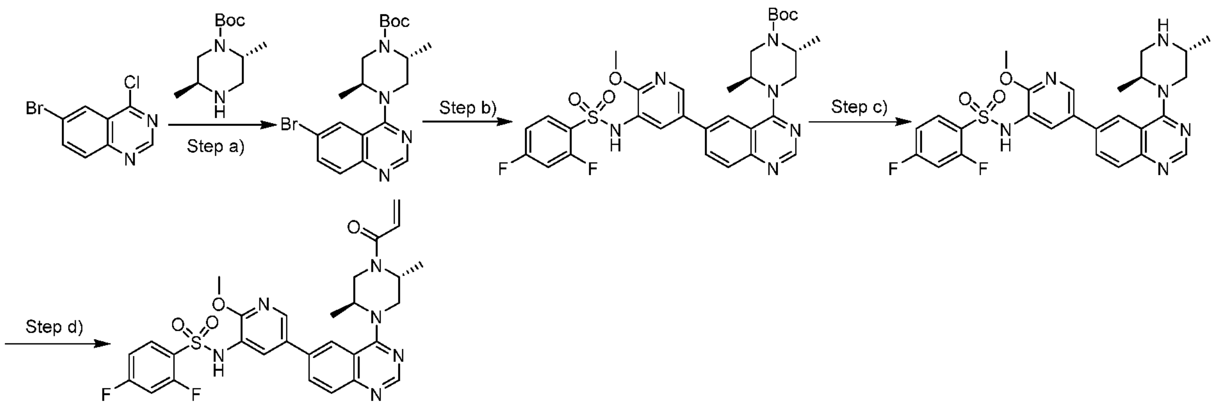


Step a): Preparation of tert-butyl (2*R*,5*S*)-4-(6-bromoquinazolin-4-yl)-2,5-dimethylpiperazine-1-carboxylate

6-Bromo-4-chloroquinazoline (300 mg, 1.23 mmol) and tert-butyl (2*R*,5*S*)-2,5-dimethylpiperazine-1-carboxylate (396 mg, 1.84 mmol) were dissolved in dimethyl sulfoxide (5.0 mL), with *N*,*N*-diisopropylethylamine (476 mg, 3.69 mmol)

added. After the addition was completed, the reaction mixture was heated to 50°C and stirred for 0.5 h. Upon completion of the reaction, the reaction mixture was cooled to 25°C, with reaction quenched by adding water (20 mL), extracted with ethyl acetate (20 mL×2). The organic phases were combined, washed with saturated brine (15 mL×3), concentrated under reduced pressure. The crude product obtained was purified by silica gel column chromatography (Eluent: petroleum ether/ethyl acetate = 10/1 to 3/1). Tert-butyl (2*R*,5*S*)-4-(6-bromoquinazolin-4-yl)-2,5-dimethylpiperazine-1-carboxylate was obtained, yield: 96.3%; ESI-MS(m/z): 421.1 $[M+H]^+$.

Step b): Preparation of tert-butyl (2*R*,5*S*)-4-(6-(5-((2,4-difluorophenyl)sulfonamido)-6-methoxypyridin-3-yl)quinazolin-4-yl)-2,5-dimethylpiperazine-1-carboxylate

Tert-butyl (2*R*,5*S*)-4-(6-bromoquinazolin-4-yl)-2,5-dimethylpiperazine-1-carboxylate (450 mg, 1.068 mmol), 2,4-difluoro-*N*-(2-hydroxy-5-(4,4,5,5-tetramethyl-1,3,2-dioxaborolan-2-yl)pyridin-3-yl)benzenesulfonamide (450 mg, 1.068 mmol), Pd(dppf)$Cl_2$ (153 mg, 0.209 mmol) and cesium carbonate (693 mg, 2.132 mmol) were added to dioxane/water mixed solvent (22 mL, v/v=10:1). After the addition was completed, in the presense of protective nitrogen, the reaction mixture was heated to 100°C and stirred for 0.5 h. Upon completion of the reaction, the reaction mixture was cooled to 25°C. The reaction was quenched by adding water (60 mL). The reaction mixture was extracted with ethyl acetate (40 mL×2 times). The organic phases were combined, washed with saturated brine (15 mL×1), dried over anhydrous sodium sulfate and filtered. The filtrate was concentrated under reduced pressure. The crude product obtained was purified by silica gel column chromatography (Eluent: petroleum ether/ethyl acetate=20/1 to 1/1). Tert-butyl (2*R*,5*S*)-4-(6-(5-((2,4-difluorophenyl)sulfonamido)-6-methoxypyridin-3-yl)quinazolin-4-yl)-2,5-dimethylpiperazine-1-carboxylate was obtained, yield: 70.1%; ESI-MS(m/z): 641.2 $[M+H]^+$.

Step c): Preparation of *N*-(5-(4-((2*S*,5*R*)-2,5-dimethylpiperazin-1-yl)quinazolin-6-yl)-2-methoxypyridin-3-yl)-2,4-difluorobenzenesulfonamide trifluoroacetate

Tert-butyl (2*R*,5*S*)-4-(6-(5-((2,4-difluorophenyl)sulfonamido)-6-methoxypyridin-3-yl)quinazolin-4-yl)-2,5-dimethylpiperazine-1-carboxylate (400 mg, 0.624 mmol) was dissolved in dichloromethane (10 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. The oily crude product was obtained, added to methyl tert-butyl ether (20 mL) and slurried for 10 min. The solution was filtered after white solid precipitation appeared. *N*-(5-(4-((2*S*,5*R*)-2,5-dimethylpiperazin-1-yl)quinazolin-6-yl)-2-methoxypyridin-3-yl)-2,4-difluorobenzenesulfonamide trifluoroacetate was obtained; yield: 98.0%; ESI-MS(m/z): 541.2 $[M+H]^+$.

Step d): Preparation of *N*-(5-(4-((2*S*,5*R*)-4-acryloyl-2,5-dimethylpiperazin-1-yl)quinazolin-6-yl)-2-methoxypyridin-3-yl)-2,4-difluorobenzenesulfonamide

*N*-(5-(4-((2*S*,5*R*)-2,5-dimethylpiperazin-1-yl)quinazolin-6-yl)-2-methoxypyridin-3-yl)-2,4-difluorobenzenesulfonamide trifluoroacetate (100.0 mg, 0.153 mmol) was dissolved in tetrahydrofuran (2 mL). The reaction system was cooled to -70°C, with *N*,*N*-diisopropylethylamine (118 mg, 0.918 mmol), acrylic acid (12 mg, 0.153 mmol) and $T_3P$ (194 mg, 0.306 mmol) added successively. After the addition was completed, the reaction mixture was kept at -70°C and stirred for 0.5 h. Upon completion of the reaction, the product was purified by Prep-HPLC to obtained *N*-(5-(4-((2*S*,5*R*)-4-acryloyl-2,5-dimethylpiperazin-1-yl)quinazolin-6-yl)-2-methoxypyridin-3-yl)-2,4-difluorobenzenesulfonamide, yield: 18.9%; $^1$H NMR (400 MHz, DMSO-$d_6$) δ 10.34 (brs, 1H), 8.65 (s, 1H), 8.46 (s, 1H), 8.09-8.08 (m, 2H), 8.00 (s, 1H), 7.92 (d, *J* = 8.0 Hz, 1H), 7.81-7.75 (m, 1H), 7.59-7.54 (m, 1H), 7.24-7.20 (m, 1H), 6.84-6.77 (m, 1H),6.18 (d, *J* = 16.0 Hz, 1H), 5.74 (d, *J* = 12.0 Hz, 1H), 4.79-4.44 (m, 2H), 4.12-4.08 (m,2H), 3.88-3.80 (m, 2H), 3.69 (s, 3H),1.30-1.15 (m, 6H); ESI-MS(m/z): 595.0 $[M+H]^+$.

**Example 15**

**Preparation of *N*-(5-(4-((2*S*,5*R*)-2,5-dimethyl-4-((*E*)-4-oxopent-2-enoyl)piperazin-1-yl)quinazolin-6-yl)-2-methoxypyridin-3-yl)-2,4-difluorobenzenesulfonamide**

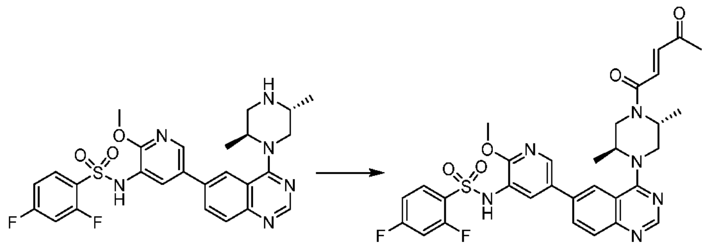

*N*-(5-(4-((2*S*,5*R*)-2,5-dimethylpiperazin-1-yl)quinazolin-6-yl)-2-methoxypyridin-3-yl)-2,4-difluorobenzenesulfonamide trifluoroacetate (100.0 mg, 0.157 mmol) was added to tetrahydrofuran (2 mL). The reaction system was cooled to -70°C, with *N*,*N*-diisopropylethylamine (121 mg, 0.942 mmol), 3-acetylacrylic acid (18 mg, 0.157 mmol) and $T_3P$ (180 mg, 0.283 mmol) added successively. After the addition was completed, the reaction mixture was kept at -70°C and stirred for 1 h. Upon completion of the reaction, the product was purified by Prep-HPLC. *N*-(5-(4-((2*S*,5*R*)-2,5-dimethyl-4-((E)-4-oxopent-2-enoyl)piperazin-1-yl)quinazolin-6-yl)-2-methoxypyridin-3-yl)-2,4-difluorobenzenesulfonamide was obtained, yield: 29.0%; $^1H$ NMR (400 MHz, DMSO-$d_6$) δ 10.34 (brs, 1H), 8.66 (s, 1H), 8.47 (s, 1H), 8.10-8.07 (m, 2H), 8.02-8.00 (m, 1H), 7.93 (d, *J* = 8.0 Hz, 1H), 7.81-7.75 (m, 1H), 7.59-7.55 (m, 1H), 7.49 (d, *J* =16.0 Hz, 1H), 7.25-7.20 (m, 1H),6.78 (d, *J* = 16.0 Hz, 1H), 4.86-4.48 (m, 2H), 4.21-4.10 (m, 2H), 3.90-3.83 (m, 2H), 3.69 (s, 3H), 2.38 (s, 3H),1.31-1.17 (m, 6H); ESI-MS(m/z): 637.0 $[M+H]^+$.

**Example 16**

**Preparation of (*E*)-2,4-difluoro-*N*-(5-(4-(4-(4-fluorobut-2-enoyl)piperazin-1-yl)quinazolin-6-yl)-2-methoxypyridin-3-yl)benzenesulfonamide**

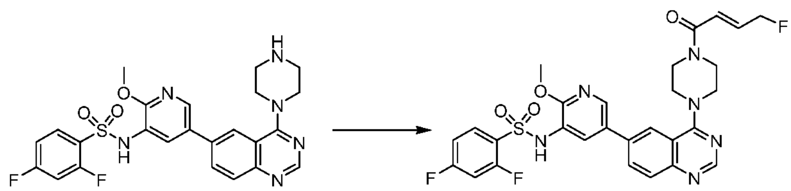

2,4-difluoro-*N*-(2-methoay-5-(4-(piperazin-1-yl)quinazolin-6-yl)pyridin-3-yl)benzenesulfonamide trifluoroacetate (70 mg, 0.112 mmol) was added to tetrahydrofuran (2 mL), in the presense of protective nitrogen. The reaction system was cooled to -70°C, with *N*,*N*-diisopropylethylamine (87 mg, 0.670 mmol), (*E*)-4-fluorobut-2-enoic acid (12 mg, 0.112 mmol) and $T_3P$ (142 mg, 0.224 mmol) added successively. After the addition was completed, the reaction mixture was kept at -70°C and stirred for 0.5 h. Upon completion of the reaction, the reaction mixture was filtered and purified by Prep-HPLC. (E)-2,4-difluoro-N-(5-(4-(4-(4-fluorobut-2-enoyl)piperazin-1-yl)quinazolin-6-yl)-2-methoxypyridin-3-yl)benzenesulfonamide was obtained, yield: 37.4%; $^1H$ NMR (400 MHz, DMSO-$d_6$) δ 10.33 (brs, 1H), 8.65 (s, 1H), 8.46 (s, 1H), 8.13-8.07 (m, 2H), 8.01 (s, 1H), 7.92-7.90 (d, *J* = 8.0 Hz, 1H), 7.80-7.74 (m, 1H), 7.58-7.53 (m, 1H), 7.23-7.18 (m, 1H), 6.85-6.79 (m, 1H), 6.77-6.71 (m, 1H), 5.20 (d, *J* = 4.0 Hz, 1H), 5.09 (d, *J* = 4.0 Hz, 1H), 3.88-3.78 (m, 8H), 3.68 (s, 3H); ESI-MS(m/z): 599.0 $[M+H]^+$.

**Example 17**

**Preparation of (*E*)-2,4-difluoro-*N*-(2-methoxy-5-(4-(4-(4-oxohept-2-enoyl)piperazin-1-yl)quinazolin-6-yl)pyridin-3-yl)benzenesulfonamide**

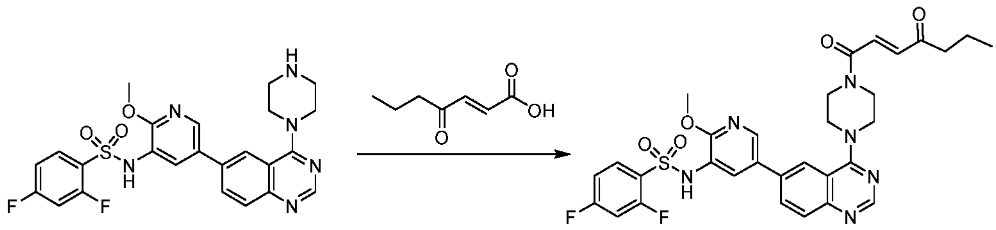

2,4-difluoro-*N*-(2-methoxy-5-(4-(piperazin-1-yl)quinazolin-6-yl)pyridin-3-yl)benzenesulfonamide trifluoroacetate (80 mg, 0.128 mmol) was added to tetrahydrofuran (2 mL). In the presense of protective nitrogen, the reaction system was cooled to -70°C, with *N*,*N*-diisopropylethylamine (99 mg, 0.768 mmol), (*E*)-4-oxohept-2-enoic acid (18 mg, 0.128 mmol) and $T_3P$ (163 mg, 0.256 mmol) added successively. After the addition was completed, the reaction mixture was kept at -70°C and stirred for 0.5 h. Upon completion of the reaction, the reaction mixture was filtered and purified by Prep-HPLC. (E)-2,4-difluoro-N-(2-methoxy-5-(4-(4-(4-oxohept-2-enoyl)pipemzin-1-yl)quinazolin-6-yl)pyridin-3-yl)benzenesulfonamide was obtained, yield: 32.5%; $^1H$ NMR (400 MHz, DMSO-$d_6$) $\delta$ 10.33 (brs, 1H), 8.66 (s, 1H), 8.46 (s, 1H), 8.13-8.08 (m, 2H), 8.01 (d, *J*= 4.0 Hz, 1H), 7.92 (d, *J*= 8.0 Hz, 1H), 7.80-7.74 (m, 1H), 7.58-7.53 (m, 1H), 7.44 (d, *J*= 16.0 Hz, 1H), 7.23-7.18 (m, 1H),6.83 (d, *J*= 16.0 Hz, 1H),3.90-3.86 (m, 6H), 3.81-3.80 (m, 2H), 3.68 (s, 3H), 2.74-2.70 (m, 2H), 1.58-1.52 (m, 2H), 0.90-0.87 (m, 3H); ESI-MS(m/z): 637.0 $[M+H]^+$.

**Example 18**

**Preparation of *N*-(5-(4-(2-acryloyl-2,7-diazaspiro[3.5]nonan-7-yl)quinazolin-6-yl)-2-methoxypyridin-3-yl)-2,4-difluorobenzenesulfonamide**

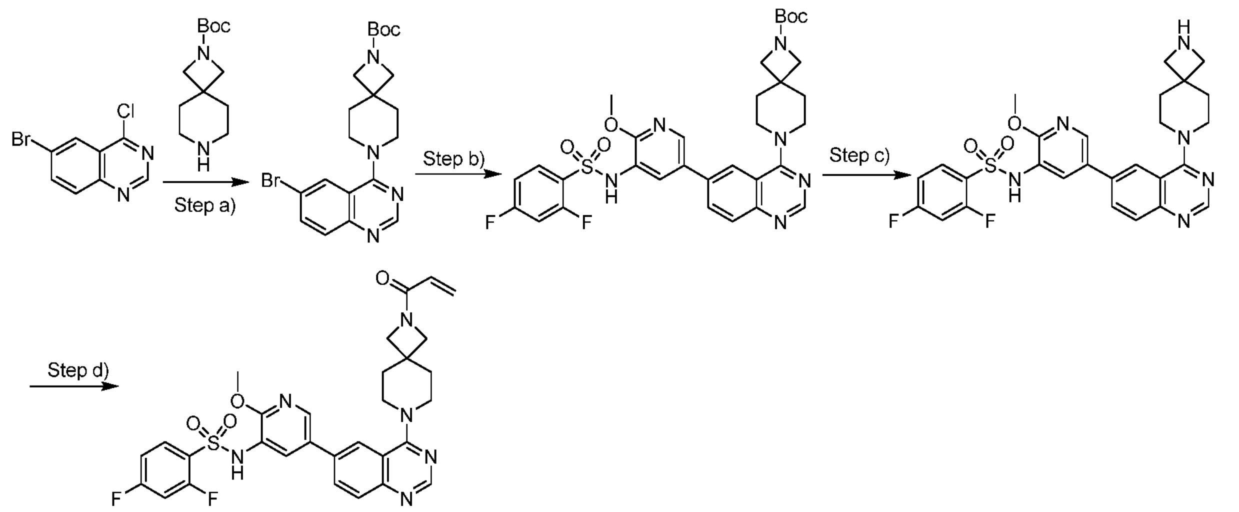


Step a): Preparation of tert-butyl 7-(6-bromoquinazolin-4-yl)-2,7-diazaspiro[3.5]nonane-2-carboxylate

6-bromo-4-chloroquinazoline (600.0 mg, 2.480 mmol), tert-butyl 2,7-diazaspiro[3.5]nonane-2-carboxylate (560.9 mg, 2.480 mmol) were dissolved in dimethyl sulfoxide (30 mL), with triethylamine (501.1 mg, 4.960 mmol) added. After the addition was completed, the reaction mixture was heated to 60°C and stirred for 2 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 2/1 to 1/1). Tert-butyl 7-(6-bromoquinazolin-4-yl)-2,7-diazaspiro[3.5]nonane-2-carboxylate was obtained, yield: 74.4%; ESI-MS(m/z): 433.3 $[M+H]^+$.

Step b): Preparation of tert-butyl 7-(6-(5-((2,4-difluorophenyl)sulfonamido)-6-methoxypyridin-3-yl)quinazolin-4-yl)-2,7-diazaspiro[3.5]nonane-2-carboxylate

Tert-butyl 7-(6-bromoquinazolin-4-yl)-2,7-diazaspiro[3.5]nonane-2-carboxylate (400.0 mg, 0.925 mmol), 2,4-difluoro-*N*-(2-hydroxy-5-(4,4,5,5-tetramethyl-1,3,2-dioxaborolan-2-yl)pyridin-3-yl)benzenesulfonamide (394.5mg, 0.925mmol), Pd(dppf)$Cl_2$ (135.4 mg, 0.185 mmol) and cesium carbonate (601.6 mg, 1.851 mmol) were added to dioxane/water mixed solvent (10 mL, v/v=10:1) successively. After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl 7-(6-(5-((2,4-difluorophenyl)sulfonamido)-6-methoxypyridin-3-yl)quinazolin-4-yl)-2,7-diazaspiro[3.5]nonane-2-carboxylate was obtained, yield: 33.1%; ESI-MS(m/z): 653.7 $[M+H]^+$.

Step c): Preparation of *N*-(5-(4-(2,7-diazaspiro[3.5]nonan-7-yl)quinazolin-6-yl)-2-methoxypyridin-3-yl)-2,4-difluorobenzenesulfonamide

Tert-butyl 7-(6-(5-((2,4-difluorophenyl)sulfonamido)-6-methoxypyridin-3-yl)quinazolin-4-yl)-2,7-diazaspiro[3.5]nonane-2-carboxylate (200.0 mg, 0.306 mmol) was dissolved in dichloromethane (2 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. *N*-(5-(4-(2,7-diazaspiro[3.5]nonan-7-yl)quinazolin-6-yl)-2-methoxypyridin-3-yl)-2,4-difluorobenzenesulfonamide was obtained. The product can be used directly in the next reaction without purification. ESI-MS(m/z): 553.6 $[M+H]^+$.

Step d): Preparation of *N*-(5-(4-(2-acryloyl-2,7-diazaspiro[3.5]nonan-7-yl)quinazolin-6-yl)-2-methoxypyridin-3-yl)-2,4-difluorobenzenesulfonamide

*N*-(5-(4-(2,7-diazaspiro[3.5]nonan-7-yl)quinazolin-6-yl)-2-methoxypyridin-3-yl)-2,4-difluorobenzenesulfonamide (60.0 mg, 0.109 mmol) was dissolved in tetrahydrofuran (2 mL). The reaction system was cooled to -78°C, with N,N-diisopropylethylamine (70.1 mg, 0.543 mmol), acrylic acid (7.8 mg, 0.109 mmol) and 50% $T_3P$ ethyl acetate solution (69.4 mg, 0.218 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. *N*-(5-(4-(2-acryloyl-2,7-diazaspiro[3.5]nonan-7-yl)quinazolin-6-yl)-2-methoxypyridin-3-yl)-2,4-difluorobenzenesulfonamide was obtained; yield: 10.1%; $^1H$ NMR (400 MHz, DMSO-$d_6$) δ 10.25 (s, 1H), 8.62 (s, 1H), 8.38 (s, 1H), 8.07-8.02 (m, 2H), 7.96 (s, 2H), 7.89-7.88 (m, 1H), 7.55 (s, 1H), 7.21 (d, *J*= 4.0 Hz, 1H), 6.33-6.31 (m, 1H), 6.14 (s, 1H), 5.69-5.66 (m, 1H), 4.03 (s, 2H), 3.75-3.70 (m, 9H), 1.96-1.93 (m, 4H); ESI-MS(m/z): 607.6 $[M+H]^+$.

## Example 19

### Preparation of 2,4-difluoro-*N*-(5-(4-(2-(2-fluoroacryloyl)-2,7-diazaspiro[3.5]nonan-7-yl)quinazolin-6-yl)-2-methoxypyridin-3-yl)benzenesulfonamide

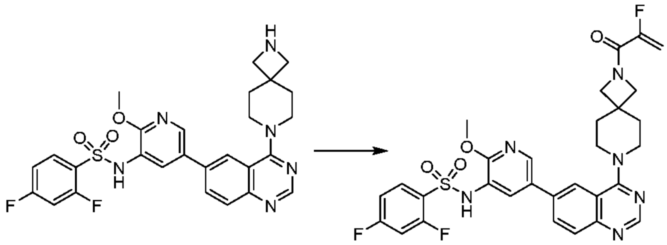

***N*-(5-(4-(2,7-diazaspiro[3.5]nonan-7-yl)quinazolin-6-yl)-2-methoxypyridin-3-yl)-2,4-difluorobenzenesulfonamide** (60.0 mg, 0.109 mmol) was dissolved in tetrahydrofuran (2 mL). The reaction system was cooled to -78°C, with N,N-diisopropylethylamine (70.1 mg, 0.543 mmol), 2-fluoroacrylic acid (9.8 mg, 0.109 mmol) and 50% $T_3P$ ethyl acetate solution (69.4 mg, 0.218 mmol) added successively. After the addition was completed, the reaction mixture was

kept at -78°C and stirred for 1 h. Upon completion of the reaction, the reaction solution was diluted by adding acetonitrile (1 mL) and purified by Prep-HPLC. 2,4-difluoro-*N*-(5-(4-(2-(2-fluoroacryloyl)-2,7-diazaspiro[3.5]nonan-7-yl)quinazolin-6-yl)-2-methoxypyridin-3-yl)benzenesulfonamide was obtained; yield: 19.1%; $^1$H NMR (400 MHz, DMSO-$d_6$) δ 8.61 (s, 1H), 8.15(s, 1H), 7.98-7.93 (m, 2H), 7.87-7.75 (m, 3H), 7.35 (s, 1H), 7.14-7.13 (m, 1H), 5.55-5.42 (m, 1H), 5.32-5.27 (m, 1H), 4.18 (d, *J* =4.0 Hz, 2H), 3.80-3.69 (m, 9H), 1.95 (t, *J* =8.0 Hz, 4H); ESI-MS(m/z): 625.6 [M+H]$^+$.

**Example 20**

**Preparation of (*E*)-2,4-difluoro-*N*-(2-methoxy-5-(4-(2-(4-oxopent-2-enoyl)-2,7-diazaspiro[3.5]nonan-7-yl)quinazolin-6-yl)pyridin-3-yl)benzenesulfonamide**

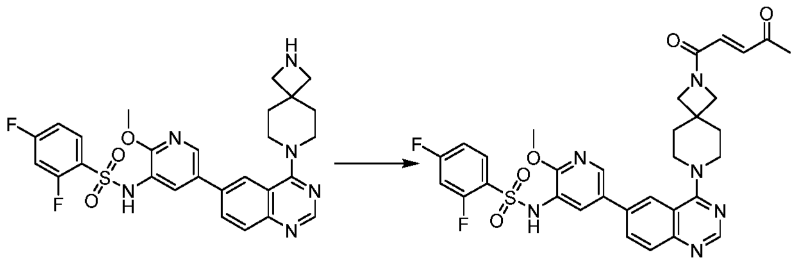

*N*-(5-(4-(2,7-diazaspiro [3.5] nonan-7-yl)quinazolin-6-yl)-2-methoaypyridin-3-yl)-2,4-difluorobenzenesulfonamide (60.0 mg, 0.109 mmol) was dissolved in tetrahydrofuran (2 mL). The reaction system was cooled to -78°C, with N,N-diisopropylethylamine (70.1 mg, 0.543 mmol), 3-acetylacrylic acid(12.4 mg, 0.109 mmol) and 50% $T_3P$ ethyl acetate solution (69.4 mg, 0.218 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (*E*)-2,4-difluoro-N-(2-methoxy-5-(4-(2-(4-oxopent-2-enoyl)-2,7-diazaspiro[3.5]nonan-7-yl)quinazolin-6-yl)pyridin-3-yl)benzenesulfonamide was obtained; yield: 28.6%; $^1$H NMR (400 MHz, DMSO-$d_6$) δ 10.35 (s, 1H), 8.63 (s, 1H), 8.4 (s, 1H), 8.09-8.07 (m, 2H), 8.05 (s, 1H), 7.99-7.91 (m, 1H), 7.89-7.78 (m, 1H), 7.57 (s, 1H), 7.22 (s, 1H), 6.93 (d, *J* =16.0 Hz, 1H), 6.75 (d, *J* =16.0 Hz, 1H), 4.15 (s, 2H), 3.80-3.70 (m, 9H), 2.35 (s, 3H), 1.97 (t, *J*=8.0 Hz, 4H); ESI-MS(m/z): 649.7 [M+H]$^+$.

**Example 21**

**Preparation of *N*-(5-(4-(4-acryloylpiperazin-1-yl)quinazolin-6-yl)-2-methoxypyridin-3-yl)-2,6-difluorobenzenesulfonamide**

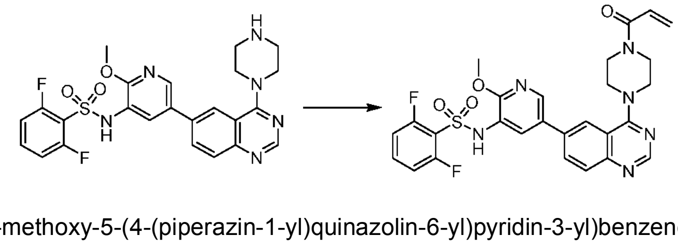

2,6-difluoro-*N*-(2-methoxy-5-(4-(piperazin-1-yl)quinazolin-6-yl)pyridin-3-yl)benzenesulfonamide (100.0 mg, 0.195 mmol) was dissolved in dichloromethane (10 mL). The reaction system was cooled to -78°C, with *N,N*-diisopropylethylamine (126.7 mg, 0.975 mmol), acrylic acid (21.1 mg, 0.293 mmol) and HATU (111.1 mg, 0.293 mmol) added successively. After the addition was completed, the reaction mixture was kept at -78°C and stirred for 1 h. Upon completion of the reaction. The reaction was quenched by adding water (5 mL). The reaction mixture was extracted by adding dichloromethane (10 mL×2 times). The organic phases were combined, dried over anhydrous sodium sulfate and filtered. The filtrate was concentrated under reduced pressure. The crude product obtained was purified by Prep-HPLC. *N*-(5-(4-(4-acryloylpiperazin-1-yl)quinazolin-6-yl)-2-methoxypyfidin-3-yl)-2,6-difluorobenzenesulfonamide was obtained, yield: 15.0%; $^1$H NMR (400 MHz, DMSO-$d_6$) δ 10.61 (s, 1H), 8.67 (s, 1H), 8.50 (s, 1H), 8.15 (s, 1H), 8.14-8.11 (m, 1H), 8.11 (s, 1H), 7.92 (d, *J* = 8 Hz, 1H), 7.75-7.71 (m, 1H), 7.31-7.26 (m, 2H), 6.21 (d, *J*= 20 MHz, 1H), 6.87-6.81

(m, 1H), 5.75 (d, *J*= 12.0 MHz, 1H), 3.90-3.79 (m, 8H), 3.65 (s, 3H); ESI-MS(m/z): 567.0 $[M+H]^+$.

**Example 22**

**Preparation of (*E*)-2,6-difluoro-*N*-(2-methoxy-5-(4-(4-(4-oxopent-2-enoyl)piperazin-1-yl)quinazolin-6-yl)pyridin-3-yl)benzenesulfonamide**

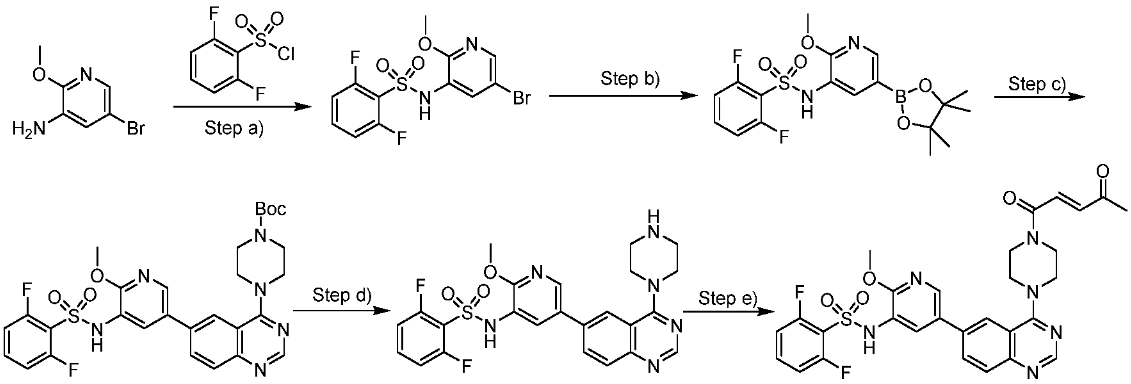


Step a): Preparation of *N*-(5-bromo-2-methoxypyridin-3-yl)-2,6-difluorobenzenesulfonamide

5-bromo-2-methoxypyridin-3-amine (500 mg, 2.463 mmol), 2,6-difluorobenzenesulfonyl chloride (783.3 mg, 3.695 mmol), 4-dimethylaminopyridine (15.0 mg, 0.123 mmol) and pyridine (746.3 mg, 7.389 mmol) were dissolved in dichloromethane (20 mL). The reaction mixture was stirred at room temperature overnight. Upon completion of the reaction, the reaction was quenched by adding water (50 mL). The reaction mixture was extracted by adding dichloromethane (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=3/1 to 1/1). was obtained *N*-(5-bromo-2-methoxypyiidin-3-yl)-2,6-difluorobenzenesulfonamide, yield: 69.7%; ESI-MS(m/z): 379.3 $[M+H]^+$.

Step b): Preparation of 2,6-difluoro-*N*-(2-methoxy-5-(4,4,5,5-tetramethyl-1,3,2-dioxaborolan-2-yl)pyridin-3-yl)benzenesulfonamide

*N*-(5-bromo-2-methoxypyridin-3-yl)-2,6-difluorobenzenesulfonamide (650 mg, 1.715 mmol), bis(pinacolato)diboron (653.4 mg, 2.573 mmol), Pd(dppf)$Cl_2$ (125.5 mg, 0.172 mmol) and potassium acetate (504.2 mg, 5.145 mmol) were dissolved in dioxane (30 mL). After the addition was completed, in the presense of protective nitrogen, the reaction mixture was heated to 110°C and stirred for 2 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=1/1 to 1/2). 2,6-difluoro-N-(2-methoxy-5-(4,4,5,5-tetramethyl-1,3,2-dioxaborolan-2-yl)pyridin-3-yl)benzenesulfonamide was obtained, yield: 95.8%; ESI-MS(m/z): 427.1 $[M+H]^+$.

Step c): Preparation of tert-butyl 4-(6-(5-((2,6-difluorophenyl)sulfonamido)-6-methoxypyridin-3-yl)quinazolin-4-yl)piperazine-1-carboxylate

2,6-difluoro-*N*-(2-methoxy-5-(4,4,5,5-tetramethyl-1,3,2-dioxaborolan-2-yl)pyridin-3-yl)benzenesulfonamide (400 mg, 0.939 mmol), tert-butyl 4-(6-bromoquinazolin-4-yl)piperazine-1-carboxylate (553.5 mg, 1.409 mmol), Pd(dppf)$Cl_2$ (68.7 mg, 0.094 mmol) and cesium carbonate (918.3 g, 2.817 mmol) were dissolved in dioxane/water (4:1,10 mL). After the addition was completed, the reaction mixture was heated to 110°C and stirred for 3 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl 4-(6-(5-((2,6-difluorophenyl)sulfonamido)-6-methoxypyridin-3-yl)quinazolin-4-yl)piperazine-1-carboxylate was obtained, yield: 69.4%; ESI-MS(m/z): 613.2 $[M+H]^+$.

Step d): Preparation of 2,6-difluoro-*N*-(2-methoxy-5-(4-(piperazin-1-yl)quinazolin-6-yl)pyridin-3-yl)benzenesulfonamide

Tert-butyl 4-(6-(5-((2,6-difluorophenyl)sulfonamido)-6-methoxypyridin-3-yl)quinazolin-4-yl)piperazine-1-carboxylate (400 mg, 0.435 mmol) was dissolved in dichloromethane (4 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. 2,6-difluoro-*N*-(2-methoxy-5-(4-(piperazin-1-yl)quinazolin-6-yl)pyridin-3-yl)benzenesulfonamide was obtained. ESI-MS(m/z): 513.1$[M+H]^+$,

Step e): Preparation of (*E*)-2,6-difluoro-*N*-(2-methoxy-5-(4-(4-(4-oxopent-2-enoyl)piperazin-1-yl)quinazolin-6-yl)pyridin-3-yl)benzenesulfonamide

2,6-difluoro-*N*-(2-methoxy-5-(4-(piperazin-1-yl)quinazolin-6-yl)pyridin-3-yl)benzenesulfonamide (100.0 mg, 0.195 mmol) was dissolved in tetrahydrofuran (10 mL). The reaction system was cooled to -78°C, with *N,N*-diisopropylethylamine (126.7 mg, 0.975 mmol), (*E*)-4-oxopent-2-enoic acid (33.3 mg, 0.293 mmol) and $T_3P$ (186.0 mg, 0.293 mmol) added successively. After the addition was completed, the reaction mixture was kept at -78°C and stirred for 1 h. Upon completion of the reaction, the reaction was quenched by adding water (5 mL). The reaction mixture was extracted by adding dichloromethane (10 mL×2 times). The organic phases were combined, dried over anhydrous sodium sulfate and filtered. The filtrate was concentrated under reduced pressure. The crude product obtained was purified by Prep-HPLC. (*E*)-2,6-difluoro-*N*-(2-methoxy-5-(4-(4-(4-oxopent-2-enoyl)piperazin-1-yl)quinazolin-6-yl)pyridin-3-yl)benzenesulfonamide was obtained, yield: 9.8%; $^1H$ NMR (400 MHz, DMSO-$d_6$) δ 10.55 (s, 1H), 8.67 (s, 1H), 8.44 (s, 1H), 8.13-8.10 (m, 2H), 8.09 (s, 1H), 7.92 (d, *J* = 8 Hz, 1H), 7.71-7.68 (m, 1H), 7.45 (d, *J*= 16 Hz, 1H), 7.28-7.23 (m, 2H), 6.78-6.73 (m, 1H), 3.90-3.80 (m, 8H), 3.63 (s, 3H), 2.37 (s, 3H); ESI-MS(m/z): 609.0 $[M+H]^+$.

**Example 23**

**Preparation of *N*-(5-(4-(4-acryloylpiperazin-1-yl)quinazolin-6-yl)-2-methoxypyridin-3-yl)-2,4-difluorobenzamide**

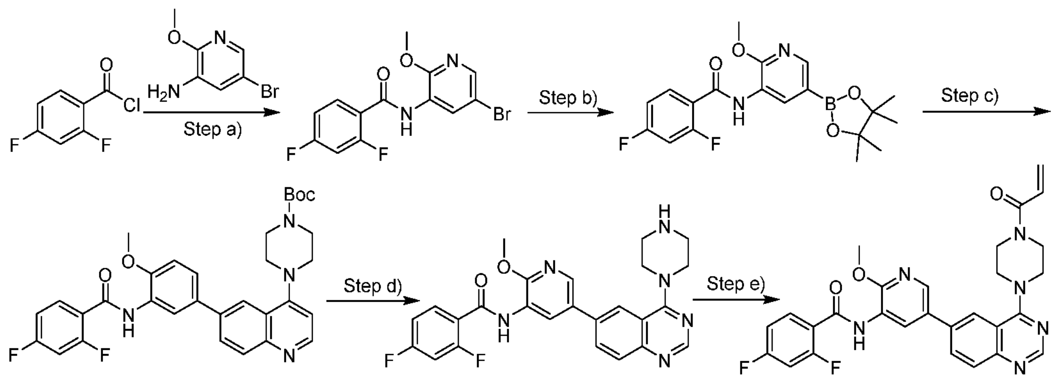


Step a): Preparation of *N*-(5-bromo-2-methoxypyridin-3-yl)-2,4-difluorobenzamide

2,4-difluorobenzoyl chloride (3.0 g, 11.111 mmol) was dissolved in dichloromethane (10 mL). Under the ice bath condition, triethylamine (3.0 g, 11.111 mmol) and 5-bromo-2-methoxypyridin-3-amine (3.0 g, 11.111 mmol) were added to the system. After the addition was completed, the reaction mixture was heated to room temperature and stirred overnight. Upon completion of the reaction, the reaction was quenched by adding water (50 mL). The reaction mixture was extracted by adding dichloromethane (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=3/1 to 1/1). *N*-(5-bromo-2-methoxypyridin-3-yl)-2,4-difluorobenzamide was obtained, yield: 51.3%; ESI-MS(m/z): 343.1 $[M+H]^+$.

Step b): Preparation of 2,4-difluoro-N-(2-methoxy-5-(4,4,5,5-tetramethyl-1,3,2-dioxaborolan-2-yl)pyridin-3-yl)benzamide

*N*-(5-bromo-2-methoxypyridin-3-yl)-2,4-difluorobenzamide (500 mg, 1.458 mmol), bis(pinacolato)diboron (555.4 mg, 2.187 mmol), Pd(dppf)$Cl_2$ (106.7 mg, 0.146 mmol) and potassium acetate (428.6 mg, 4.374 mmol) were dissolved in dioxane (30 mL). After the addition was completed, in the presense of protective nitrogen, the reaction mixture was heated to 110°C and stirred for 2 h. Upon completion of the reaction. The reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=1/1 to 1/2). 2,4-difluoro-*N*-(2-methoxy-5-(4,4,5,5-tetramethyl-1,3,2-dioxaborolan-2-yl)pyridin-3-yl)benzamide was obtained, yield: 87.9%; ESI-MS(m/z): 391.3 $[M+H]^+$.

Step c): Preparation of tert-butyl 4-(6-(3-(2,4-difluorobenzamido)-4-methoxyphenyl)quinolin-4-yl)piperazine-1-carboxylate

2,4-difluoro-*N*-(2-methoay-5-(4,4,5,5-tetramethyl-1,3,2-dioxaborolan-2-yl)pyridin-3-yl)benzamide (400 mg, 1.026 mmol), tert-butyl 4-(6-bromoquinazolin-4-yl)piperazine-1-carboxylate (603.1 mg, 1.539 mmol), Pd(dppf)$Cl_2$ (75.1 mg, 0.103 mmol) and cesium carbonate (1.0 g, 3.078 mmol) were dissolved in dioxane/water (4:1, ,10 mL). After the addition was completed, the reaction mixture was heated to 110°C and stirred for 3 h. Upon completion of the reaction. The reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl 4-(6-(3 -(2,4-difhiorobenzamido)-4-methoxyphenyl)quinolin-4-yl)piperazine-1 -carboxylate was obtained, yield: 42.4%; ESI-MS(m/z): 577.2 $[M+H]^+$.

Step d): Preparation of 2,4-difluoro-*N*-(2-methoxy-5-(4-(piperazin-1-yl)quinazolin-6-yl)pyridin-3-yl)benzamide

Tert-butyl 4-(6-(3-(2,4-difluorobenzamido)-4-methoxyphenyl)quinolin-4-yl)piperazine-1-carboxylate (250 mg, 0.435 mmol) was dissolved in dichloromethane (4 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. 2,4-difluoro-*N*-(2-methoxy-5-(4-(piperazin-1-yl)quinazolin-6-yl)pyridin-3-yl)benzamide was obtained. The product can be used directly in the next reaction without further purification. ESI-MS(m/z): 477.3 $[M+H]^+$.

Step e): Preparation of *N*-(5-(4-(4-acryloylpiperazin-1-yl)quinazolin-6-yl)-2-methoxypyridin-3-yl)-2,4-difluorobenzamide

2,4-difluoro-*N*-(2-methoxy-5-(4-(piperazin-1-yl)quinazolin-6-yl)pyridin-3-yl)benzamide (150.0 mg, 0.314 mmol) was dissolved in dichloromethane (10 mL). The reaction system was cooled to -78°C, with *N,N*-diisopropylethylamine (204.4 mg, 1.570 mmol), acrylic acid (34.0 mg, 0.471 mmol) and HATU (179.2 mg, 0.471 mmol) added successively. After the addition was completed, the reaction mixture was kept at -78°C and stirred for 1 h. Upon completion of the reaction. The reaction was quenched by adding water (5 mL). The reaction mixture was extracted by adding dichloromethane (10 mL×2 times). The organic phases were combined, dried over anhydrous sodium sulfate and filtered. The filtrate was concentrated under reduced pressure. The crude product obtained was purified by Prep-HPLC. *N*-(5-(4-(4-acryloylpiperazin-1-yl)quinazolin-6-yl)-2-methoxypyfidin-3-yl)-2,4-difluorobenzamide was obtained, yield: 54.9%; $^1$H NMR (400 MHz, DMSO-$d_6$) $\delta$ 9.09-9.04 (m, 2H), 8.70 (s, 1H), 8.18-8.13 (m, 2H), 8.05-8.98 (m, 3H), 7.04-7.00 (m, 1H), 6.94-6.89 (m, 1H), 6.60-6.53 (m, 1H), 6.33-6.28 (m, 1H), 5.72 (d, *J*= 12.0 Hz, 1H), 4.08 (s, 3H), 3.88-3.82 (m, 8H); ESI-MS(m/z): 531.0 $[M+H]^+$.

**Example 24**

**Preparation of *N*-(5-(4-(4-acryloylpiperazin-1-yl)quinazolin-6-yl)-2-methoxypyridin-3-yl)benzenesulfonamide**

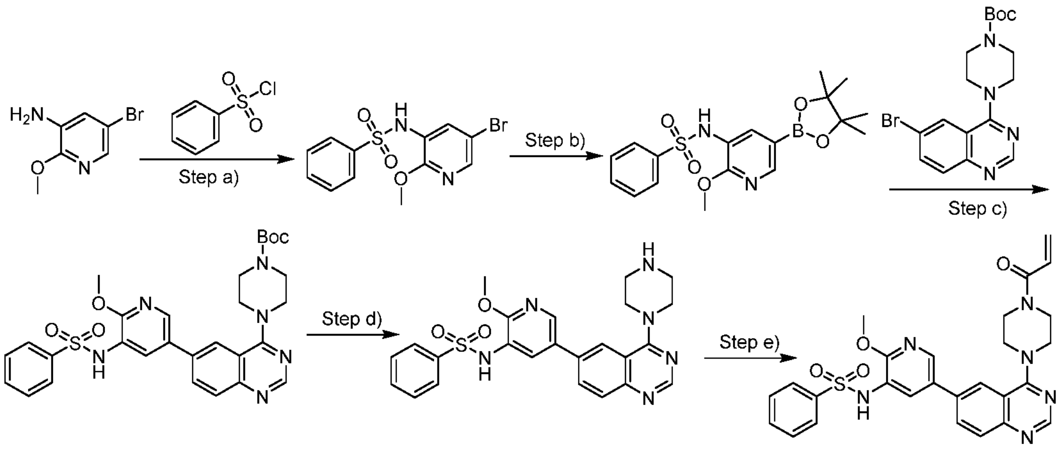


Step a): Preparation of *N*-(5-bromo-2-methoxypyridin-3-yl)benzenesulfonamide

5-bromo-2-methoxypyridin-3-amine (600 mg, 2.970 mmol), benzenesulfonyl chloride (522.8 mg, 2.970 mmol), 4-dimethylaminopyridine (36.3 mg, 0.297 mmol), Pyridine (352.5 mg, 4.456 mmol) and dichloromethane (12 mL) were added to the reaction flask successively. After the addition was completed, the reaction mixture was stirred at room temperature for 4 h. Upon completion of the reaction, the reaction was quenched by adding water (50 mL). The reaction system was extracted with dichloromethane (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 2/1 to 1/1). *N*-(5-bromo-2-methoxypyridin-3-yl)benzenesulfonamide was obtained, yield: 49.2%; ESI-MS(m/z): 343.2 $[M+H]^+$.

Step b): Preparation of *N*-(2-methoxy-5-(4,4,5,5-tetramethyl-1,3,2-dioxaborolan-2-yl)pyridin-3-yl)benzenesulfonamide

*N*-(5-bromo-2-methoxypyridin-3-yl)benzenesulfonaniide (600.0 mg, 1.755 mmol), bis(pinacolato)diboron (534.7 mg, 1.755mmol), Pd(dppf)$Cl_2$ (256.5 mg, 0.351 mmol) and potassium acetate (792.1 mg, 8.070 mmol) were dissolved in dioxane (12 mL) successively. After the addition was completed, in the presense of protective nitrogen, the reaction mixture was kept at 100°C and stirred for 4 h. Upon completion of the reaction, the reaction mixture was filtered. The filtrate was concentrated under reduced pressure. The crude product obtained was purified by silica gel column chromatography (Eluent: petroleum ether/ethyl acetate = 2/1 to 1/1). *N*-(2-methoxy-5-(4,4,5,5-tetramethyl-1,3,2-dioxaborolan-2-yl)pyridin-3-yl)benzenesulfonamide was obtained, yield: 87.7%; ESI-MS(m/z): 391.2 $[M+H]^+$.

Step c): Preparation of tert-butyl 4-(6-(6-methoxy-5-(phenylsulfonamido)pyridin-3-yl)quinazolin-4-yl)piperazine-1-carboxylate

Tert-butyl 4-(6-bromoquinazolin-4-yl)piperazine-1-carboaylate (490.0 mg, 1.256 mmol), *N*-(2-methoxy-5-(4,4,5,5-tetramethyl-1,3,2-dioxaborolan-2-yl)pyridin-3-yl)benzenesulfonamide (492.2 mg, 1.256 mmol), Pd(dppf)$Cl_2$ (275.5 mg, 0.377 mmol) and cesium carbonate (780.3 mg, 2.512 mmol) were added to dioxane/water mixed solvent (10 mL, v/v=10: 1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1). Tert-butyl 4-(6-(6-methoxy-5-(phenylsulfonamido)pyridin-3-yl)quinazolin-4-yl)piperazine-1-carboxylate was obtained, yield: 23.5%; ESI-MS(m/z): 577.2 $[M+H]^+$.

Step d): Preparation of *N*-(2-methoxy-5-(4-(piperazin-1-yl)quinazolin-6-yl)pyridin-3-yl)benzenesulfonamide

Tert-butyl 4-(6-(6-methoxy-5-(phenylsulfonamido)pyridin-3-yl)quinazolin-4-yl)piperazine-1-carboxylate (75.0 mg, 0.130 mmol) was dissolved in dichloromethane (4 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. *N*-(2-methoxy-5-(4-(piperazin-1-yl)quinazolin-6-yl)pyridin-3-yl)benzenesulfonamide was obtained. The product can be used directly in the next

reaction without further purification. ESI-MS(m/z): 477.6 $[M+H]^+$.

Step e): Preparation of *N*-(5-(4-(4-acryloylpiperazin-1-yl)quinazolin-6-yl)-2-methoxypyfidin-3-yl)benzenesulfonamide

*N*-(2-methoxy-5-(4-(piperazin-1-yl)quinazolin-6-yl)pyridin-3-yl)benzenesulfonamide (62.0 mg, 0.130 mmol) was dissolved in dichloromethane (2 mL). The reaction system was cooled to -78°C, with *N,N*-diisopropylethylamine (134.3 mg, 1.041 mmol), acrylic acid (9.4 mg, 0.130 mmol) and HATU (74.2 mg, 0.195 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. *N*-(5-(4-(4-acryloylpiperazin-1-yl)quinazolin-6-yl)-2-methoxypyridin-3-yl)benzenesulfonamide was obtained, yield: 10.1%; $^1$H NMR (400 MHz, DMSO-$d_6$) δ 10.07 (s, 1H), 8.78 (s, 1H), 8.44 (s, 1H), 8.21 (s, 2H), 7.99-7.94 (m, 2H), 7.79-7.77 (m, 2H), 7.65-7.55 (m, 3H), 6.81 (s, 1H), 6.18 (d, *J*= 4.0 Hz, 1H), 5.78 (d, *J*= 4.0 Hz, 1H), 4.01-4.08 (m, 4H), 3.88-3.81 (m, 4H), 3.67 (s, 3H); ESI-MS(m/z): 531.6 $[M+H]^+$.

**Example 25**

**Preparation of *N*-(5-(4-(4-acryloylpiperazin-1-yl)quinazolin-6-yl)-2-methoxypyridin-3-yl)cyclohexanesulfonamide**

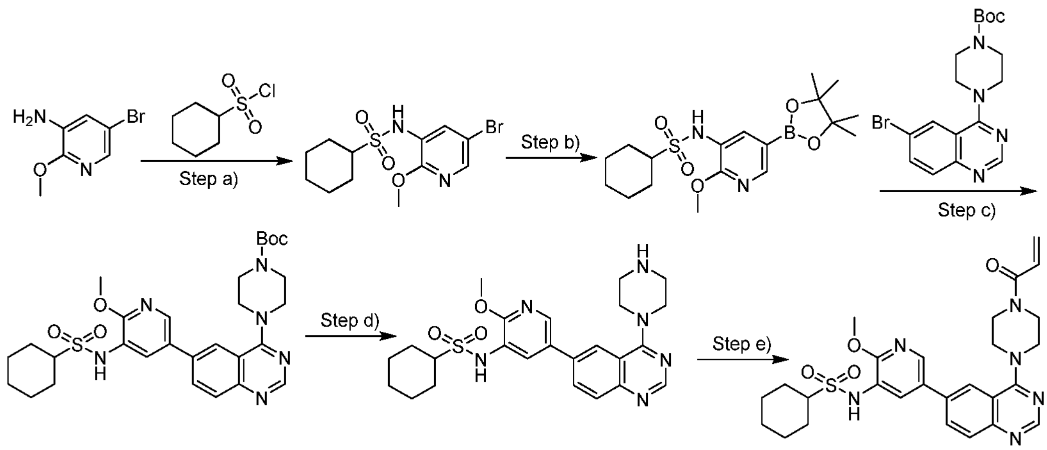


Step a): Preparation of *N*-(5-bromo-2-methoxypyridin-3-yl)cyclohexanesulfonamide

5- bromo-2-methoxypyridin-3-amine (500 mg, 2.476 mmol) and tetrahydrofuran (10mL) were added successively to the reaction flask. Potassium bistrimethylsilylamide (5.0 mL, 4.951 mmol) was slowly added to the system. After the addition was completed, the mixture was stirred for 30min, with cyclohexanesulfonyl chloride (675.9 mg, 3.713 mmol) added. After the addition was completed, the reaction mixture was stirred at room temperature for 12 h. Upon completion of the reaction, the reaction was quenched by adding water (50 mL) slowly. The reaction system was extracted with ethyl acetate (50 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 3/1 to 1/1). *N*-(5-bromo-2-methoxypyridin-3-yl)cyclohexanesulfonamide was obtained, yield: 58.0%; ESI-MS(m/z): 349.2 $[M+H]^+$

Step b): Preparation of *N*-(2-methoxy-5-(4,4,5,5-tetrainethyl-1,3,2-dioxaborolan-2-yl)pyridin-3-yl)cyclohexanesulfonamide

*N*-(5-bromo-2-methoxypyridin-3-yl)cyclohexanesulfonamide (500 mg, 1.437 mmol), bis(pinacolato)diboron (437.8 mg, 1.724mmol), Pd(dppf)$Cl_2$ (210.0 mg, 0.287 mmol) and potassium acetate (647.7 mg, 6.610 mmol) were added to dioxane (10 mL) successively. After the addition was completed, the reaction mixture was stireed at 100°C for 4 h under an atmosphere of nitrogen. Upon completion of the reaction, the reaction mixture was filtered. The filtrate was concentrated under reduced pressure. The crude product obtained was purified by silica gel column chromatography (Eluent: petroleum ether/ethyl acetate = 2/1 to 1/1). *N*-(2-methoxy-5-(4,4,5,5-tetramethyl-1,3,2-dioxaborolan-2-yl)pyridin-3-yl)cyclohexanesulfonamide was obtained, yield: 70.3%; ESI-MS(m/z): 396.2 $[M+H]^+$.

Step c): Preparation of tert-butyl 4-(6-(5-(cyclohexanesulfonamido)-6-methoxypyridin-3-yl)quinazolin-4-yl)piperazine-1-carboxylate

Tert-butyl 4-(6-bromoquinazolin-4-yl)piperazine-1-carboxylate (395.9 mg, 1.010 mmol), *N*-(2-methoxy-5-(4,4,5,5-tetramethyl-1,3,2-dioxaborolan-2-yl)pyridin-3-yl)cyclohexanesulfonamide (400 mg, 1.010 mmol) ) $Pd(dppf)Cl_2$ (147.6 mg, 0.202 mmol) and cesium carbonate (656.2 mg, 2.020 mmol) were added to dioxane/water mixed solvent (8 mL, v/v=10: 1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 0/1). Tert-butyl 4-(6-(5-(cyclohexanesulfonamido)-6-methoxypyridin-3-yl)quinazolin-4-yl)piperazine-1-carboxylate was obtained, yield: 34.0%; ESI-MS(m/z): 583.2 $[M+H]^+$.

Step d): Preparation of *N*-(2-methoxy-5-(4-(piperazin-1-yl)quinazolin-6-yl)pyridin-3-yl)cyclohexanesulfonamide

Tert-butyl 4-(6-(5-(cyclohexanesulfonamido)-6-methoxypyridin-3-yl)quinazolin-4-yl)piperazine-1-carboxylate (80.0mg, 0.137mmol) was dissolved in dichloromethane (2 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. *N*-(2-methoxy-5-(4-(piperazin-1-yl)quinazolin-6-yl)pyridin-3-yl)cyclohexanesulfonamide was obtained. The product can be used directly in the next reaction without further purification. ESI-MS(m/z): 483.1 $[M+H]^+$.

Step e): Preparation of *N*-(5-(4-(4-acryloylpiperazin-1-yl)quinazolin-6-yl)-2-methoxypyridin-3-yl)cyclohexanesulfonamide

*N*-(2-methoxy-5-(4-(piperazin-1-yl)quinazolin-6-yl)pyridin-3-yl)cyclohexanesulfonamide (60 mg, 0.124 mmol) was dissolved in tetrahydrofuran (2 mL). The reaction system was cooled to -78°C, with N,N-diisopropylethylamine (96 mg, 0.744 mmol), acrylic acid (8.9 mg, 0.124mmol) and 50% $T_3P$ ethyl acetate solution (79.1mg, 0.248 mmol) added successively. After the addition was completed, the reaction mixture wasstirred at - 78°C for 1 h. Upon completion of the reaction, the reaction was quenched by adding acetonitrile (1 mL). The reaction solution was purified by Prep-HPLC. *N*-(5-(4-(4-acryloylpiperazin-1-yl)quinazolin-6-yl)-2-methoxypyridin-3-yl)cyclohexanesulfonamide was obtained, yield: 40.7%; $^1H$ NMR (400 MHz, DMSO-$d_6$) $\delta$ 9.36 (s, 1H), 8.66 (s, 1H), 8.45 (s, 1H), 8.13-8.11 (m, 2H), 8.02 (d, *J* =4.0 Hz, 1H), 7.92 (d, *J* =8.0 Hz, 1H), 6.81 (t, *J* = 16.0 Hz, 1H), 6.19-6.14 (m, 1H), 5.75-5.72 (m, 1H), 3.99 (s, 3H), 3.89-3.79 (m, 8H), 3.10 (s,1H), 2.13(d, J=12.0 Hz, 1H), 1.80-1.77(m, 2H), 1.62 (d, *J* =12.0 Hz, 1H), 1.44-1.37(m, 2H), 1.31-1.21(m, 2H), 1.18-1.12(m, 1H); ESI-MS(m/z): 537.5 $[M+H]^+$.

**Example 26**

**Preparation of (*E*)-*N*-(2-methoxy-5-(4-(4-(4-oxopent-2-enoyl)piperazin-1-yl)quinazolin-6-yl)pyridin-3-yl)cyclohexanesulfonamide**

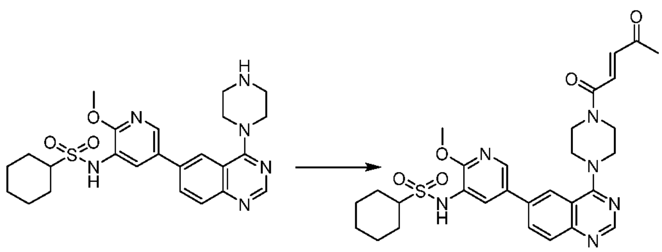

(*E*)-*N*-(2-methoxy-5-(4-(4-(4-oxopent-2-enoyl)piperazin-1-yl)quinazolin-6-yl)pyridin-3-yl)cyclohexanesulfonamide (60.0 mg, 0.124 mmol) was dissolved in tetrahydrofuran (2 mL). The reaction system was cooled to -78°C, with *N,N*-diisopropylethylamine (96.0 mg, 0.744 mmol), 3-acetylacrylic acid (14.1 mg, 0.124mmol) and 50% $T_3P$ ethyl acetate

solution (79.1mg, 0.248 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (*E*)-*N*-(2-methoxy-5-(4-(4-(4-oxopent-2-enoyl)piperazin-1-yl)quinazolin-6-yl)pyridin-3-yl)cyclohexanesulfonamide was obtained, yield: 45.9%; $^1$H NMR (400 MHz, DMSO-$d_6$) δ 9.35 (s, 1H), 8.67 (s, 1H), 8.45 (s, 1H), 8.14-8.11 (m, 2H), 8.02 (d, *J* =4.0 Hz, 1H), 7.92 (d, *J* =8.0 Hz, 1H), 7.44 (d, *J* =16.0 Hz, 1H), 6.73 (d, *J* =16.0 Hz, 1H), 3.99 (s, 3H), 3.89-3.80 (m, 8H), 3.10 (s,1H), 2.37 (s,3H), 2.13 (d, J=16.0 Hz, 2H), 1.62-1.59 (m, 1H), 1.80-1.77 (m, 2H), 1.44-1.40 (m, 2H), 1.27-1.24 (m, 2H), 1.18-1.12 (m, 1H); ESI-MS(m/z): 579.2 [M+H]$^+$.

**Example 27**

**Preparation of *N*-(5-(4-(4-acryloylpiperazin-1-yl)quinazolin-6-yl)pyridin-3-yl)-2,4-difluorobenzenesulfonamide**

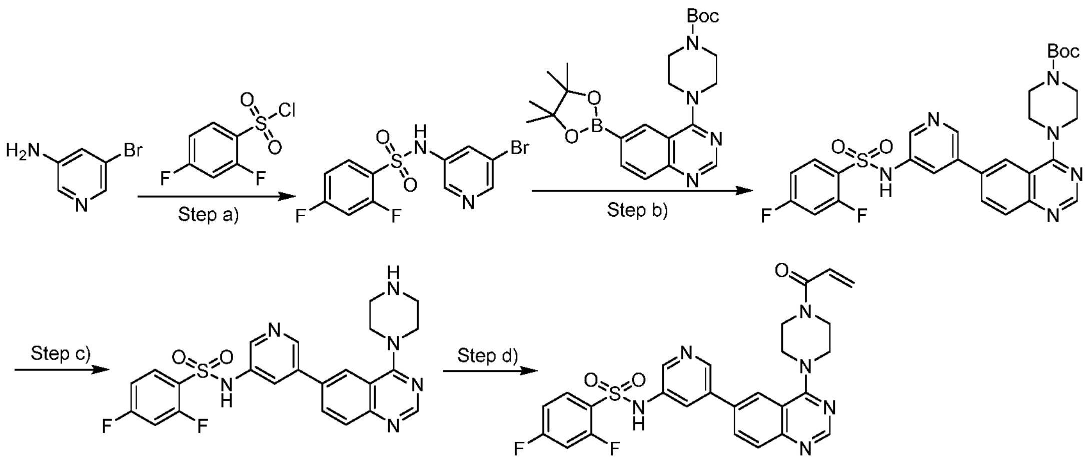


Step a): Preparation of *N*-(5-bromopyridin-3-yl)-2,4-difluorobenzenesulfonamide

5-bromopyridin-3-amine (600.0 mg, 3.489 mmol), 2,4-difluorobenzenesulfonyl chloride (739.5m g, 3.489 mmol), 4-dimethylaminopyridine (42.6 mg, 0.349 mmol), pyridine (413.5 mg, 5.233 mmol) and dichloromethane (12 mL) were added to the reaction flask successively. After the addition was completed, the reaction mixture was stirred at room temperature for 4 h. Upon completion of the reaction, the reaction was quenched by adding water (50 mL). The reaction system was extracted with dichloromethane (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=3/1 to 1/1). *N*-(5-bromopyridin-3-yl)-2,4-difluorobenzenesulfonamide was obtained, yield: 65.9%; ESI-MS(m/z): 349.2 [M+H]$^+$.

Step b): Preparation of tert-butyl 4-(6-(5-((2,4-difluorophenyl)sulfonamido)pyridin-3-yl)quinazolin-4-yl)piperazine-1-carboxylate

Tert-butyl 4-(6-(4,4,5,5-tetramethyl-1,3,2-dioxaborolan-2-yl)quinazolin-4-yl)piperazine-1-carboxylate (252.4 mg, 0.573 mmol), *N*-(5-bromopyridin-3-yl)-2,4-difluorobenzenesulfonamide (200 mg, 0.573 mmol), Pd(dppf)$Cl_2$ (167.4 mg, 0.115 mmol) and cesium carbonate (372.3 mg, 1.145 mmol) were added to dioxane/water mixed solvent (10 mL, v/v=10: 1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl 4-(6-(5-((2,4-difluorophenyl)sulfonamido)pyridin-3-yl)quinazolin-4-yl)piperazine-1-carboxylate was obtained, yield: 39.0%; ESI-MS(m/z): 583.6 [M+H]$^+$.

Step c): Preparation of 2,4-difluoro-*N*-(5-(4-(piperazin-1-yl)quinazofin-6-yl)pyridin-3-yl)benzenesulfonamide

Tert-butyl 4-(6-(5-((2,4-difluorophenyl)sulfonamido)pyridin-3-yl)quinazolin-4-yl)piperazine-1-carboxylate

(130.0mg, 0.223mmol) was dissolved in dichloromethane (4 mL). Under the ice bath condition, trifluoroacetic acid (4 mL) was slowly added dropwise. After the addition was completed, the reaction mixture was stirred at room temperature for 1 h. Upon completion of the reaction, the reaction mixture was concentrated under reduced pressure. 2,4-difluoro-*N*-(5-(4-(piperazin-1-yl)quinazolin-6-yl)pyridin-3-yl)benzenesulfonamide was obtained. The product can be used directly in the next reaction without further purification. ESI-MS(m/z): 483.1 $[M+H]^+$.

Step d): Preparation of *N*-(5-(4-(4-acryloylpiperazin-1-yl)quinazolin-6-yl)pyridin-3-yl)-2,4-difluorobenzenesulfonamide

2,4-difluoro-*N*-(5-(4-(piperazin-1-yl)quinazolin-6-yl)pyridin-3-yl)benzenesulfonamide (60.0 mg, 0.124 mmol) was dissolved in tetrahydrofuran (2 mL). The reaction system was cooled to -78°C, with *N,N*-diisopropylethylamine (96.0 mg, 0.744 mmol), acrylic acid (8.9 mg, 0.124mmol) and 50% $T_3P$ ethyl acetate solution (79.1mg, 0.248 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. *N*-(5-(4-(4-acryloylpiperazin-1-yl)quinazolin-6-yl)pyridin-3-yl)-2,4-difluorobenzenesulfonamide was obtained, yield: 12.0%; $^1$H NMR (400 MHz, DMSO-$d_6$) $\delta$ 11.2 (s, 1H), 8.78-8.77 (m, 2H), 8.39-8.38 (m, 1H), 8.24 (s, 1H), 8.13-8.01 (m, 1H), 7.95-7.86 (m, 2H),7.57 (s, 1H), 7.30 (s, 1H), 6.83-6.78 (m, 1H), 6.21-6.17 (m, 1H), 5.77-5.74 (m, 1H), 4.09-4.06 (m, 4H), 3.88-3.80 (m, 4H); ESI-MS(m/z): 537.5 $[M+H]^+$.

**Example 28**

**Preparation of (*E*)-2,4-difluoro-*N*-(5-(4-(4-(4-oxopent-2-enoyl)piperazin-1-yl)quinazolin-6-yl)pyridin-3-yl)benzenesulfonamide**

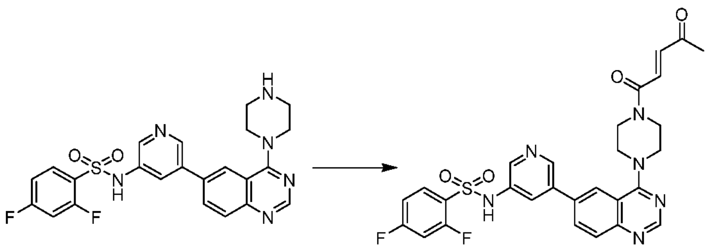

2,4-difluoro-*N*-(5-(4-(piperazin-1-yl)quinazolin-6-yl)pyridin-3-yl)benzenesulfonamide (60.0 mg, 0.124 mmol) was dissolved in tetrahydrofuran (2 mL). The reaction system was cooled to -78°C, with *N,N*-diisopropylethylamine (96.0 mg, 0.744 mmol), 3-acetylacrylic acid (14.1 mg, 0.124mmol) and 50% $T_3P$ ethyl acetate solution (79.1mg, 0.248 mmol) added successively. After the addition was completed, the reaction mixture was kept at -78°C and stirred for 1 h. Upon completion of the reaction, the reaction was quenched by adding acetonitrile (1 mL), the reaction solution was purified by Prep-HPLC. (*E*)-2,4-difluoro-*N*-(5-(4-(4-(4-oxopent-2-enoyl)piperazin-1-yl)quinazolin-6-yl)pyridin-3-yl)benzenesulfonamide was obtained, yield: 21.8%; $^1$H NMR (400 MHz, DMSO-$d_6$) $\delta$ 11.2 (s, 1H), 8.79-8.77 (m, 2H), 8.38-8.24 (m, 1H), 8.15 (d, *J* = 8.0 Hz,1H), 8.01 (s, 2H), 7.95 (d, *J* = 8.0 Hz, 1H),7.87 (s, 1H), 7.57 (s, 1H), 7.42 (d, *J* = 16.0 Hz, 1H),7.29 (s, 1H), 6.76 (d, *J* = 16.0 Hz, 1H), 4.12-4.11 (m, 4H), 3.93-3.80 (m, 4H), 2.37 (s, 3H); ESI-MS(m/z): 579.5 $[M+H]^+$.

**Example 29**

**Preparation of *N*-(5-(4-(4-acryloylpiperazin-1-yl)-2-methylquinazolin-6-yl)-2-methoxypyridin-3-yl)-2,4-difluorobenzenesulfonamide**

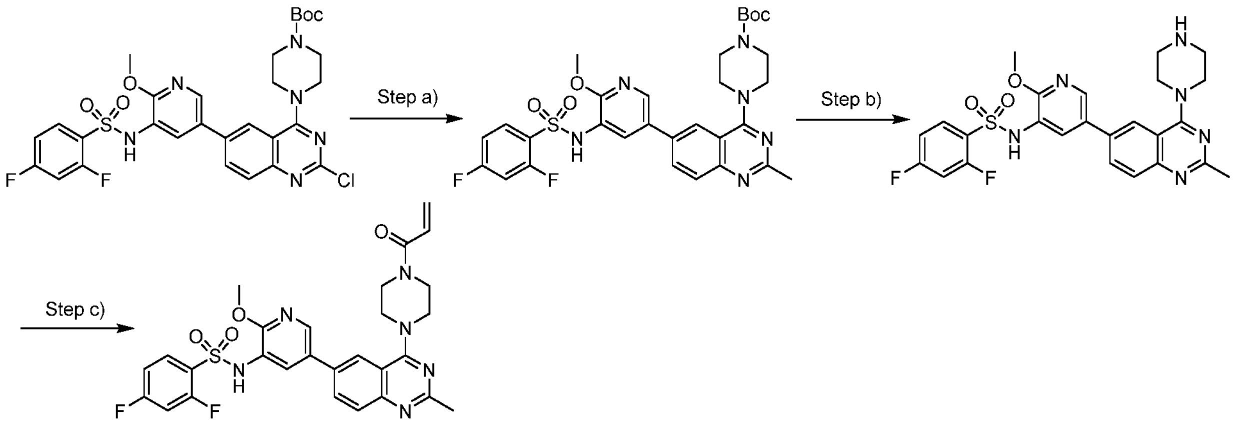


Step a): Preparation of tert-butyl 4-(6-(5-((2,4-difluorophenyl)sulfonamido)-6-methoxypyridin-3-yl)-2-methylquinazolin-4-yl)piperazine-1-carboxylate

Tert-butyl 4-(2-chloro-6-(5-((2,4-difluorophenyl)sulfonamido)-6-methoxypyridin-3-yl)quinazolin-4-yl)piperazine-1-carboxylate (200 mg, 0.309 mmol), pinacol methylborate (88 mg, 0.620 mmol), Pd(dppf)$Cl_2$ (23 mg, 0.031 mmol) and potassium carbonate (128 mg, 0.926 mmol) were added to *N,N*-dimethylformamide/water mixed solvent (5 mL, v/v=10: 1). After the addition was completed, in the presense of protective nitrogen, the reaction mixture was heated to 100°C and stirred overnight. Upon completion of the reaction, the reaction was quenched by adding water (30 mL). The reaction mixture was extracted with ethyl acetate (60 mL×2). The organic phases were combined, washed with saturated brine (50 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl 4-(6-(5-((2,4-difluorophenyl)sulfonamido)-6-methoxypyridin-3-yl)-2-methylquinazolin-4-yl)piperazine-1-carboxylate was obtained, yield: 36.3%; ESI-MS(m/z): 627.20 $[M+H]^+$.

Step b): Preparation of 2,4-difluoro-*N*-(2-methoxy-5-(2-methyl-4-(piperazin-1-yl)quinazolin-6-yl)pyridin-3-yl)benzenesulfonamide

Tert-butyl 4-(6-(5-((2,4-difluorophenyl)sulfonamido)-6-methoxypyridin-3-yl)-2-methylquinazolin-4-yl)piperazine-1-carboxylate (70 mg, 0.112 mmol) was dissolved in dichloromethane (2 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. 2,4-difluoro-*N*-(2-methoxy-5-(2-methyl-4-(piperazin-1-yl)quinazolin-6-yl)pyridin-3-yl)benzenesulfonamide was obtained. The product can be used directly in the next reaction without further purification. ESI-MS(m/z): 527.2 $[M+H]^+$.

Step c): Preparation of *N*-(5-(4-(4-acryloylpiperazin-1-yl)-2-methylquinazolin-6-yl)-2-methoxypyfidin-3-yl)-2,4-difluorobenzenesulfonamide

2,4-difluoro-*N*-(2-methoxy-5-(2-methyl-4-(piperazin-1-yl)quinazolin-6-yl)pyridin-3-yl)benzenesulfonamide (50 mg, 0.095 mmol) and acrylic acid (34 mg, 0.472 mmol) were dissolved in tetrahydrofuran (2 mL). The reaction system was cooled to -78°C, with *N,N*-diisopropylethylamine (61 mg, 0.472 mmol) and 1-propylphosphonic anhydride (91 mg, 0.286mmol) added successively. After the addition was completed, the reaction mixture was kept at -78°C and stirred for 1 h. Upon completion of the reaction, the reaction system was diluted by adding acetonitrile (1 mL). The reaction solution was purified by Prep-HPLC. *N*-(5-(4-(4-acryloylpiperazin-1-yl)-2-methylquinazolin-6-yl)-2-methoxypyridin-3-yl)-2,4-difluorobenzenesulfonamide was obtained, yield: 24.3%; $^1H$ NMR (400 MHz, DMSO-$d_6$) δ 10.3 (s, 1H), 8.42-8.38 (m, 1H), 8.04-7.93 (m, 3H), 7.79-7.69 (m, 2H), 7.56-7.49 (m, 1H), 7.19-7.14 (m, 1H), 6.83-6.75 (m, 1H), 6.16-6.09 (m, 1H), 5.72-5.66 (m, 1H), 3.81-3.71 (m, 8H), 3.63 (s, 3H), 2.51 (s, 3H); ESI-MS(m/z): 581.0 $[M+H]^+$.

**Example 30**

**Preparation of *N*-(5-(4-(4-acryloylpiperazin-1-yl)-2-aminoquinazolin-6-yl)-2-methoxypyridin-3-yl)-2,4-difluorobenzenesulfonamide**

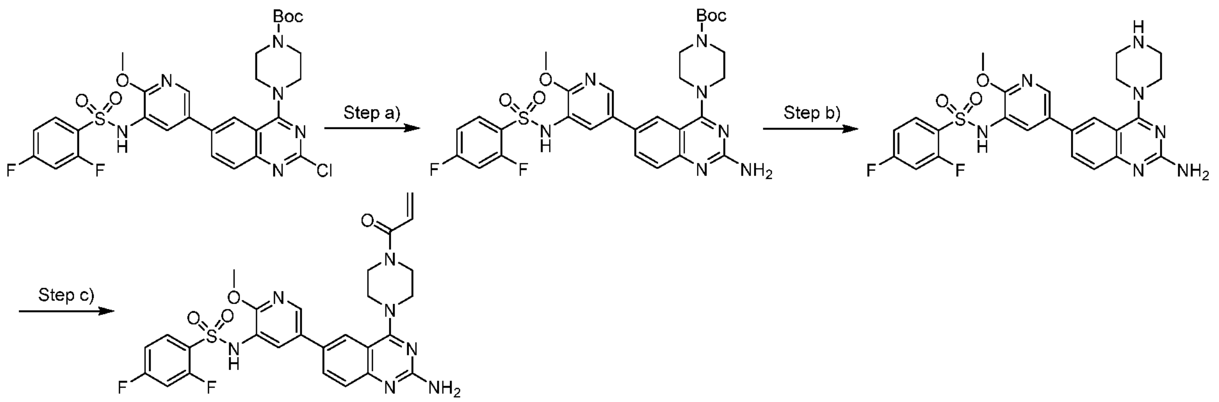


Step a): Preparation of tert-butyl 4-(2-amino-6-(5-((2,4-difluorophenyl)sulfonamido)-6-methoxypyridin-3-yl)quinazolin-4-yl)piperazine-1-carboxylate

Tert-butyl 4-(2-chloro-6-(5-((2,4-difluorophenyl)sulfonamido)-6-methoxypyfidin-3-yl)quinazolin-4-yl)piperazine-1-carboxylate (250 mg, 0.386 mmol) were dissolved in $NH_3$/methanol solution (6 mL), the reaction solution was placed in a sealed tube, heated to 100°C and stirred overnight. Upon completion of the reaction, the reaction solution was purified by reversed-phase column chromatography (mobile phase: acetonitrile/water (0.1%TFA) = 0 to 50%). Tert-butyl 4-(2-amino-6-(5-((2,4-difluorophenyl)sulfonamido)-6-methoxypyridin-3-yl)quinazolin-4-yl)piperazine-1-carboxylate was obtained, yield: 20.7%; ESI-MS(m/z): 628.2 $[M+H]^+$.

Step b): Preparation of *N*-(5-(2-amino-4-(piperazin-1-yl)quinazolin-6-yl)-2-methoaypyridin-3-yl)-2,4-difluorobenzenesulfonamide

Tert-butyl 4-(2-amino-6-(5-((2,4-difluorophenyl)sulfonamido)-6-methoxypyridin-3-yl)quinazolin-4-yl)piperazine-1-carboxylate (50 mg, 0.080 mmol) was dissolved in dichloromethane (2 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. *N*-(5-(2-amino-4-(piperazin-1 -yl)quinazolin-6-yl)-2-metho xypyridin-3 -yl)-2,4-difluorobenzenesulfonamide was obtained. The product can be used directly in the next reaction without further purification. ESI-MS(m/z): 528.2 $[M+H]^+$.

Step c): Preparation of *N*-(5-(4-(4-acryloylpiperazin-1-yl)-2-aminoquinazolin-6-yl)-2-methoxypyridin-3-yl)-2,4-difluorobenzenesulfonamide

*N*-(5-(2-amino-4-(piperazin-1-yl)quinazolin-6-yl)-2-methoaypyridin-3-yl)-2,4-difluorobenzenesulfonamide (40 mg, 0.076 mmol) was dissolved in dichloromethane (1.5 mL). The reaction system was cooled to -78°C, with *N,N*-diisopropylethylamine (49 mg, 0.379 mmol), acrylic acid (11 mg, 0.153 mmol) and HATU (35 mg, 0.092 mmol) added successively. After the addition was completed, the reaction mixture was kept at -78°C and stirred for 1 h. Upon completion of the reaction, the reaction system was diluted by adding acetonitrile (1 mL). The reaction solution was purified by Prep-HPLC. *N*-(5-(4-(4-acryloylpiperazin-1-yl)-2-aminoquinazolin-6-yl)-2-methoxypyridin-3-yl)-2,4-difluorobenzenesulfonamide was obtained, yield: 4.3%; $^1H$ NMR (400 MHz, DMSO-$d_6$) $\delta$ 11.84 (s, 1H), 10.26 (s, 1H), 9.25-8.95 (m, 1H), 8.55-8.46 (m, 1H), 8.44-8.40 (m, 1H), 8.17-8.06 (m, 1H), 8.05-8.02 (m, 1H), 7.74-7.60 (m, 2H), 7.56-7.49 (m, 1H), 7.18-7.12 (m, 1H), 6.89-6.78 (m, 1H), 6.18-6.10 (m, 1H), 5.73-5.69 (m, 1H), 3.85 (s, 4H), 3.77-3.65 (m, 4H), 3.57 (s, 3H); ESI-MS(m/z): 582.0 $[M+H]^+$.

**Example 31**

**Preparation of *N*-(5-(4-(4-acryloylpiperazin-1-yl)-2-cyanoquinazolin-6-yl)-2-methoxypyridin-3-yl)-2,4-difluorobenzenesulfonamide**

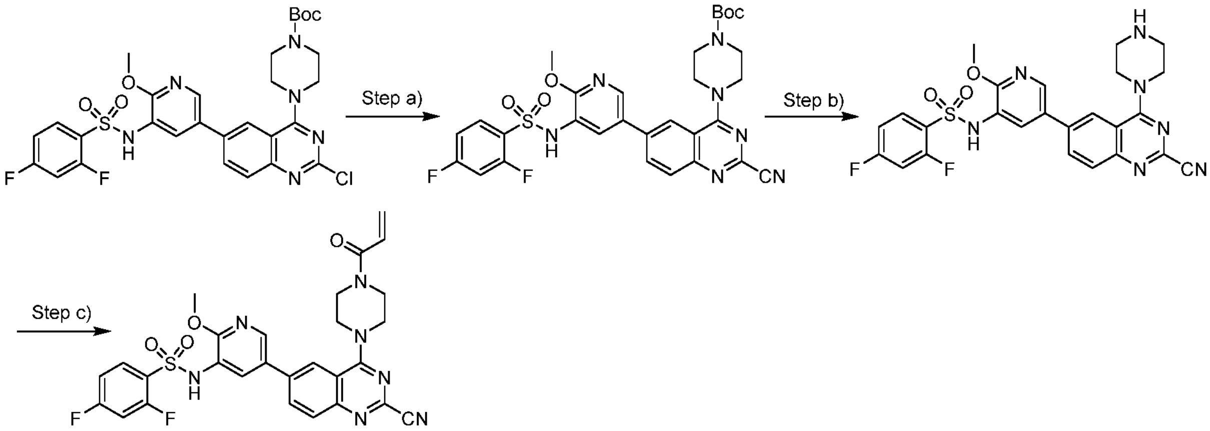


Step a): Preparation of tert-butyl 4-(2-cyano-6-(5-((2,4-difluorophenyl)sulfonamido)-6-methoxypyridin-3-yl)quinazolin-4-yl)piperazine-1-carboxylate

Tert-butyl 4-(2-chloro-6-(5-((2,4-difluorophenyl)sulfonamido)-6-methoxypyridin-3-yl)quinazolin-4-yl)piperazine-1-carboxylate (150 mg, 0.232 mmol), Zinc cyanide (55 mg, 0.464 mmol), Pd(dppf)$Cl_2$ (37 mg, 0.046 mmol) and cesium carbonate (227 mg, 0.696 mmol) were added to N-methylpyrrolidone (5 mL). After the addition was completed, under nitrogen atmosphere, the reaction mixture was microwave heated to 150°C and reacted for 2 h. Upon completion of the reaction, the reaction was quenched by adding water (30 mL). The reaction mixture was extracted with ethyl acetate (100 mL×2 times). The organic phases were combined, washed with saturated brine (50 mL×1 time), dried over anhydrous sodium sulfate and filtered. The filtrate was concentrated under reduced pressure. The crude product obtained was purified by silica gel column chromatography (Eluent: petroleum ether/ethyl acetate=5/1 to 3/1). Tert-butyl 4-(2-cyano-6-(5-((2,4-difluorophenyl)sulfonamido)-6-methoxypyridin-3-yl)quinazolin-4-yl)piperazine-1-carboxylate was obtained, yield: 48.6%; ESI-MS(m/z): 638.2 $[M+H]^+$.

Step b): Preparation of *N*-(5-(2-cyano-4-(piperazin-1-yl)quinazolin-6-yl)-2-methoxypyridin-3-yl)-2,4-difluorobenzenesulfonamide

Tert-butyl 4-(2-cyano-6-(5-((2,4-difluorophenyl)sulfonamido)-6-methoxypyridin-3-yl)quinazolin-4-yl)piperazine-1-carboxylate (50 mg, 0.078 mmol) was dissolved in dichloromethane (2 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. *N*-(5-(2-cyano-4-(piperazin-1-yl)quinazolin-6-yl)-2-methoxypyridin-3-yl)-2,4-difluorobenzenesulfonamide was obtained: 538.1 $[M+H]^+$.

Step c): Preparation of *N*-(5-(4-(4-acryloylpiperazin-1-yl)-2-cyanoquinazolin-6-yl)-2-methoxypyridin-3-yl)-2,4-difluorobenzenesulfonamide

N-(5-(2-cyano-4-(piperazin-1-yl)quinazolin-6-yl)-2-methoxypyridin-3-yl)-2,4-difluorobenzenesulfonamide (30 mg, 0.056 mmol) was dissolved in tetrahydrofuran (2 mL). The reaction system was cooled to -78°C, with *N,N*-diisopropylethylamine (36 mg, 0.280 mmol), acrylic acid (4 mg, 0.056mmol) and 50% $T_3P$ ethyl acetate solution (36 mg, 0.112 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. *N*-(5-(4-(4-acryloylpiperazin-1-yl)-2-cyanoquinazolin-6-yl)-2-methoxypyridin-3-yl)-2,4-difluorobenzenesulfonamide was obtained, yield: 18.5%; $^1H$ NMR (400 MHz, DMSO-$d_6$) δ 10.36 (brs, 1H), 8.53-8.52 (m, 1H), 8.26-8.21 (m, 1H), 8.07-8.06 (m, 1H), 8.00-7.98 (m, 1H), 7.80-7.74 (m, 1H), 7.61-7.55 (m, 1H), 7.24-7.19 (m, 1H), 6.89-6.79 (m, 1H), 6.21-6.16 (m, 1H), 5.77-5.74 (m, 1H),4.06-4.03(m, 4H), 3.87-3.79 (m, 4H), 3.68 (s, 3H); ESI-MS(m/z): 592.2 $[M+H]^+$.

**Example 32**

**Preparation of *N*-(5-(4-(4-acryloylpiperazin-1-yl)-2-hydroxyquinazolin-6-yl)-2-methoxypyridin-3-yl)-2,4-difluorobenzenesulfonamide**

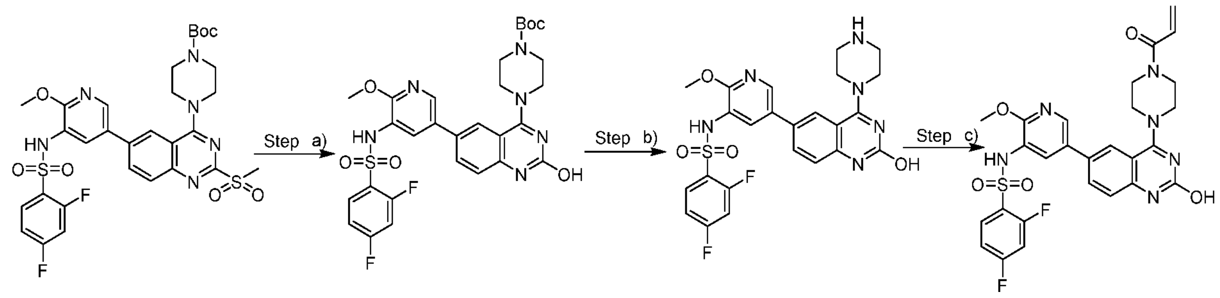


Step a): Preparation of tert-butyl 4-(6-(5-((2,4-difluorophenyl)sulfonamido)-6-methoxypyridin-3-yl)-2-hydroxyquinazolin-4-yl)piperazine-1 -carboxylate

Tert-butyl 4-(6-(5-((2,4-difluorophenyl)sulfonamido)-6-methoxypyridin-3-yl)-2-(methylsulfonyl)quinazolin-4-yl)piperazine-1-carboxylate (170.0 mg, 0.246 mmol), potassium hydroxide (55.3 mg, 0.985 mmol) were dissolved in dimethyl sulfoxide (10 mL) and water (2mL) successively. The reaction mixture was heated to 100°C and stirred for 2 h. Upon completion of the reaction, the reaction solution was adjust to pH = 2~3 with 1M hydrogen chloride aqueous solution and extracted with dichloromethane (10 mL×3).The organic phase was concentrated under reduced pressure. Tert-butyl 4-(6-(5-((2,4-difluorophenyl)sulfonamido)-6-methoxypyridin-3-yl)-2-hydroxyquinazolin-4-yl)piperazine-1-carboxylate was obtained, crude product yield: 99.9%; ESI-MS(m/z): 629.6 $[M+H]^+$.

Step b): Preparation of 2,4-difluoro-*N*-(5-(2-hydroxy-4-(piperazin-1-yl)quinazolin-6-yl)-2-methoxypyfidin-3-yl)benzenesulfonamide

Tert-butyl 4-(6-(5-((2,4-difluorophenyl)sulfonamido)-6-methoxypyridin-3-yl)-2-hydroxyquinazolin-4-yl)piperazine-1-carboxylate (170.0 mg, 0.072 mmol) was dissolved in dichloromethane (4 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. 2,4-difluoro-*N*-(5-(2-hydroxy-4-(piperazin-1-yl)quinazolin-6-yl)-2-methoxypyridin-3-yl)benzenesulfonamide was obtained. The product can be used directly in the next reaction without further purification. ESI-MS(m/z): 529.5 $[M+H]^+$.

Step c): Preparation of *N*-(5-(4-(4-acryloylpiperazin-1-yl)-2-hydroxyquinazolin-6-yl)-2-methoxypyridin-3-yl)-2,4-difluorobenzenesulfonamide

2,4-difluoro-*N*-(5-(2-hydroxy-4-(piperazin-1-yl)quinazolin-6-yl)-2-methoxypyridin-3-yl)benzenesulfonamide (80.0 mg, 0.151 mmol) was dissolved in dichloromethane (5 mL). The reaction system was cooled to -78°C, with *N,N*-diisopropylethylamine (97.7 mg, 0.757 mmol), acrylic acid (10.9 mg, 0.151 mmol) and HATU (69.1 mg, 0.182 mmol) added successively. After the addition was completed, the reaction mixture was kept at -78°C and stirred for 1 h. Upon completion of the reaction, acetonitrile (1 mL) was added for dissolution. The reaction solution was purified by Prep-HPLC. *N*-(5-(4-(4-acryloylpiperazin-1-yl)-2-hydroxyquinazolin-6-yl)-2-methoxypyridin-3-yl)-2,4-difluorobenzenesulfonamide was obtained; yield: 8.0%; $^1H$ NMR (400 MHz, DMSO-$d_6$) $\delta$ 11.07 (s, 1H), 10.30 (s, 1H), 8.32 (d, *J* = 4.0 Hz, 1H), 7.90-7.82 (m, 3H), 7.79-7.73 (m, 1H), 7.58-7.53 (m, 1H), 7.31 (d, *J* = 8.0 Hz, 1H), 7.23-7.18 (m, 1H), 6.83-6.76 (m,1H), 6.17-6.12 (m,1H), 5.73-5.70 (m,1H), 3.82-3.72 (m, 8H), 3.63 (s, 3H); ESI-MS(m/z): 583.0 $[M+H]^+$.

**Example 33**

**Preparation of *N*-(5-(4-(4-acryloylpiperazin-1-yl)quinazolin-6-yl)-2-chloropyridin-3-yl)-2,4-difluorobenzenesulfonamide**

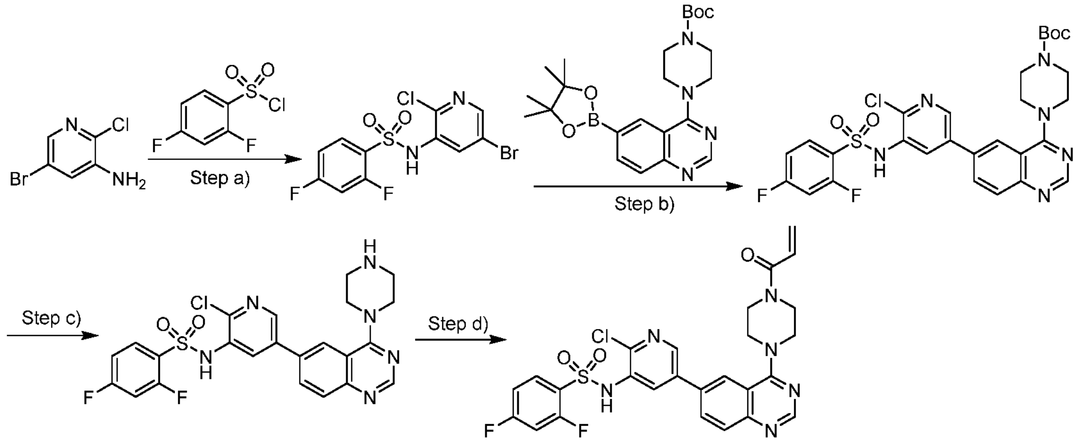


Step a): Preparation of *N*-(5-bromo-2-chloropyridin-3-yl)-2,4-difluorobenzenesulfonamide

5-bromo-2-chloropyridin-3-amine (1.5 g, 7.230 mmol), 2,4-difluorobenzenesulfonyl chloride (1.69 g, 7.950 mmol) were dissolved in pyridine (20 mL). After the addition was completed, the reaction mixture was heated to 80°C, stirred and reacted overnight. Upon completion of the reaction, the reaction was quenched by adding water (50 mL). The reaction mixture was extracted with ethyl acetate (200 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=10/1 to 3/1). *N*-(5-bromo-2-chloropyridin-3-yl)-2,4-difluorobenzenesulfonamide was obtained. ESI-MS(m/z): 384.9 $[M+H]^+$.

Step b): Preparation of tert-butyl 4-(6-(6-chloro-5-((2,4-difluorophenyl)sulfonamido)pyridin-3-yl)quinazolin-4-yl)piperazine-1-carboxylate

*N*-(5-bromo-2-chloropyridin-3-yl)-2,4-difluorobenzenesulfonamide (300 mg, 0.782 mmol), tert-butyl 4-(6-(4,4,5,5-tetramethyl-1,3,2-dioxaborolan-2-yl)quinazolin-4-yl)piperazine-1-carboxylate (379 mg, 0.860 mmol), $Pd(dppf)Cl_2$ (114 mg, 0.156 mmol) and cesium carbonate (764 mg, 2.346 mmol) were added to dioxane/water mixed solvent (10 mL, v/v=10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (150 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=5/1 to 1/1). Tert-butyl 4-(6-(6-chloro-5-((2,4-difluorophenyl)sulfonamido)pyridin-3-yl)quinazolin-4-yl)piperazine-1-carboxylate was obtained, yield: 51.8%; ESI-MS(m/z): 617.2 $[M+H]^+$.

Step c): Preparation of *N*-(2-chloro-5-(4-(piperazin-1-yl)quinazolin-6-yl)pyridin-3-yl)-2,4-difluorobenzenesulfonamide

Tert-butyl 4-(6-(6-chloro-5-((2,4-difluorophenyl)sulfonamido)pyridin-3-yl)quinazolin-4-yl)piperazine-1-carboxylate (100 mg, 0.162 mmol) was dissolved in dichloromethane (5 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. *N*-(2-chloro-5-(4-(piperazin-1-yl)quinazolin-6-yl)pyridin-3-yl)-2,4-difluorobenzenesulfonamide was obtained. The product can be used directly in the next reaction without further purification. ESI-MS(m/z): 517.1 $[M+H]^+$.

Step d): Preparation of *N*-(5-(4-(4-acryloylpiperazin-1-yl)quinazolin-6-yl)-2-chloropyridin-3-yl)-2,4-difluorobenzenesulfonamide

*N*-(2-chloro-5-(4-(piperazin-1-yl)quinazolin-6-yl)pyridin-3-yl)-2,4-difluorobenzenesulfonamide (50 mg, 0.097 mmol) was dissolved in tetrahydrofuran (2 mL). The reaction system was cooled to -78°C, with *N,N*-diisopropylethylamine (63 mg, 0.485 mmol), acrylic acid (4 mg, 0.097mmol) and 50% $T_3P$ ethyl acetate solution (123 mg, 0.194 mmol) added successively. After the addition was completed, the reaction mixture was kept at - 78°C and stirred for 1 h. Upon completion of the reaction, the reaction solution was diluted by adding acetonitrile (1 mL) and purified by Prep-HPLC. *N*-(5-(4-(4-acryloylpiperazin-1-yl)quinazolin-6-yl)-2-chloropyridin-3-yl)-2,4-difluorobenzenesulfonamide was obtained, yield: 17.6%; $^1$H NMR (400 MHz, DMSO-$d_6$) δ8.69 (s, 2H), 8.23-8.13 (m, 3H), 7.95-7.93 (m, 1H), 7.85-7.79 (m, 1H), 7.58-7.50 (m, 1H), 7.26-7.19 (m, 1H), 6.86-6.80 (m, 1H), 6.20-6.15 (m, 1H), 5.75-5.73 (m, 1H), 3.98-3.92(m, 4H), 3.85-3.79 (m, 4H); ESI-MS(m/z): 571.0 [M+H]$^+$.

**Example 34**

**Preparation of (*E*)-*N*-(2-chloro-5-(4-(4-(4-oxopent-2-enoyl)piperazin-1-yl)quinazolin-6-yl)pyridin-3-yl)-2,4-difluorobenzenesulfonamide**

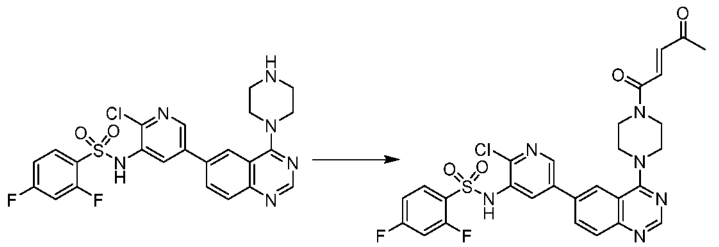

Step a): Preparation of (*E*)-*N*-(2-chloro-5-(4-(4-(4-oxopent-2-enoyl)piperazin-1-yl)quinazolin-6-yl)pyridin-3-yl)-2,4-difluorobenzenesulfonamide

*N*-(2-chloro-5-(4-(piperazin-1-yl)quinazolin-6-yl)pyridin-3-yl)-2,4-difluorobenzenesulfonamide (50 mg, 0.097 mmol) was dissolved in tetrahydrofuran (2 mL). The reaction system was cooled to -78°C, with *N,N*-diisopropylethylamine (63 mg, 0.485 mmol), (E)-4-oxopent-2-enoic acid (11 mg, 0.097mmol) and 50% $T_3P$ ethyl acetate solution (123 mg, 0.194 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (*E*)-*N*-(2-chloro-5-(4-(4-(4-oxopent-2-enoyl)piperazin-1-yl)quinazolin-6-yl)pyridin-3-yl)-2,4-difluorobenzenesulfonamide was obtained, yield: 16.9%; $^1$H NMR (400 MHz, DMSO-$d_6$) δ 8.68 (s, 1H), 8.31 (s, 1H), 8.12 (s, 1H), 8.04-7.99 (m, 2H), 7.93 (d, *J*= 8.0 MHz, 1H), 7.86-7.80 (m, 1H), 7.45 (d, *J*= 16.0 MHz, 1H), 7.41-7.35 (m, 1H), 7.18-7.13 (m, 2H), 6.74 (d, *J*= 16.0 MHz, 1H), 3.89-3.78 (m, 8H), 2.37 (s, 3H); ESI-MS(m/z): 613.0 [M+H]$^+$.

**Example 35**

**Preparation of *N*-(5-(4-(4-acryloylpiperazin-1-yl)quinazolin-6-yl)-2-chloropyridin-3-yl)-5-chlorothiophene-2-sulfonamide**

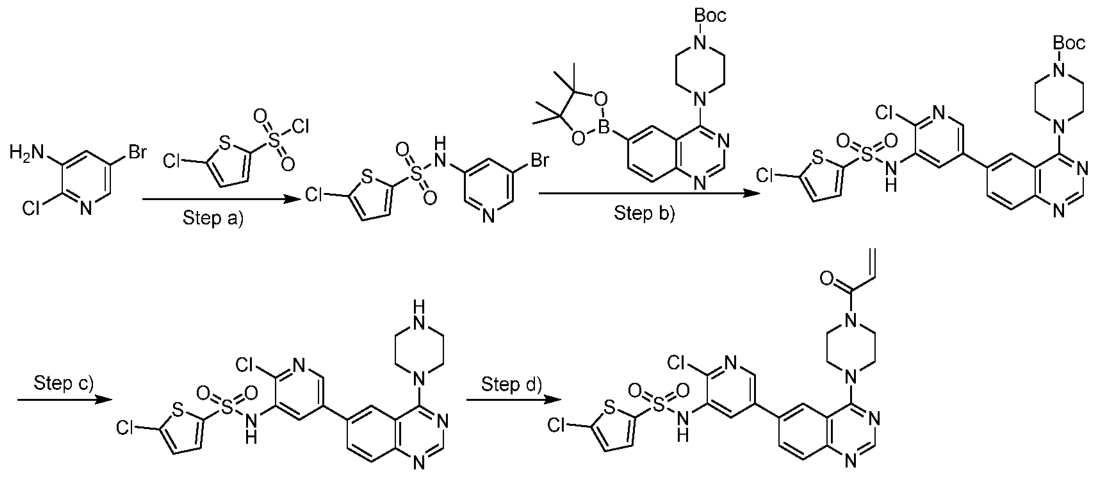


Step a): Preparation of *N*-(5-bromopyridin-3-yl)-5-chlorothiophene-2-sulfonamide

5-bromo-2-chloropyridin-3-amine (1.0 g, 4.820 mmol), 5-chlorothiophene-2-sulfonyl chloride (994 mg, 4.579 mmol) were dissolved in pyridine (10 mL). After the addition was completed, at 80°C, the reaction mixture was stirred and reacted overnight. Upon completion of the reaction, the reaction was quenched by adding water (50 mL). The reaction mixture was extracted with ethyl acetate (200 mL×2). The organic phases were combined, washed with saturated brine (100 mL xl), dried over anhydrous sodium sulfate and filtered. The filtrate was concentrated under reduced pressure. The crude product obtained was purified by silica gel column chromatography (Eluent: petroleum ether/ethyl acetate=10/1 to 3/1). *N*-(5-bromopyridin-3-yl)-5-chlorothiophene-2-sulfonamide was obtained, yield: 49.7%. ESI-MS(m/z): 387.8 $[M+H]^+$.

Step b): Preparation of tert-butyl 4-(6-(6-chloro-5-((5-chlorothiophene)-2-sulfonamido)pyridin-3-yl)quinazolin-4-yl)piperazine-1-carboxylate

N-(5-bromopyridin-3-yl)-5-chlorothiophene-2-sulfonamide (500 mg, 1.289 mmol), tert-butyl 4-(6-(4,4,5,5-tetramethyl-1,3,2-dioxaborolan-2-yl)quinazolin-4-yl)piperazine-1-carboxylate (624 mg, 1.418 mmol), Pd(dppf)$Cl_2$ (189 mg, 0.258 mmol) and cesium carbonate (1.26 g, 3.867 mmol) were added to dioxane/water mixed solvent (10 mL, v/v=10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (200 mL×2). The organic phases were combined, washed with saturated brine (100 mL xl), dried over anhydrous sodium sulfate and filtered. The filtrate was concentrated under reduced pressure. The crude product obtained was purified by silica gel column chromatography (Eluent: dichloromethane/methanol=100/1 to 40/1). Tert-butyl 4-(6-(6-chloro-5-((5-chlorothiophene)-2-sulfonamido)pyridin-3-yl)quinazolin-4-yl)piperazine-1-carboxylate was obtained, yield: 55.2%; ESI-MS(m/z): 621.1 $[M+H]^+$.

Step c): Preparation of 5-chloro-*N*-(2-chloro-5-(4-(piperazin-1-yl)quinazolin-6-yl)pyridin-3-yl)thiophene-2-sulfonamide

Tert-butyl 4-(6-(6-chloro-5-((5-chlorothiophene)-2-sulfonamido)pyridin-3-yl)quinazolin-4-yl)piperazine-1-carboxylate (500 mg, 0.804 mmol) was dissolved in dichloromethane (5 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. 5-chloro-*N*-(2-chloro-5-(4-(piperazin-1-yl)quinazolin-6-yl)pyridin-3-yl)thiophene-2-sulfonamide was obtained. The product can be used directly in the next reaction without further purification. ESI-MS(m/z): 521.0 $[M+H]^+$.

Step d): Preparation of *N*-(5-(4-(4-acryloylpiperazin-1-yl)quinazolin-6-yl)-2-chloropyridin-3-yl)-5-chlorothiophene-2-sulfonamide

5-chloro-*N*-(2-chloro-5-(4-(piperazin-1-yl)quinazolin-6-yl)pyridin-3-yl)thiophene-2-sulfonamide (100 mg, 0.192 mmol) was dissolved in tetrahydrofuran (2 mL). The reaction system was cooled to -78°C, with *N,N*-diisopropylethylamine (124 mg, 0.960 mmol), acrylic acid (14 mg, 0.192mmol) and 50% $T_3P$ ethyl acetate solution (244 mg, 0.384 mmol) added successively. After the addition was completed, the reaction mixture was kept at - 78°C and stirred for 1 h. Upon completion of the reaction, the reaction solution was diluted by adding acetonitrile (1 mL) and purified by Prep-HPLC.

*N*-(5-(4-(4-acryloylpiperazin-1-yl)quinazolin-6-yl)-2-chloropyridin-3-yl)-5-chlorothiophene-2-sulfonamide was obtained, yield: 15.3%; $^1$H NMR (400 MHz, DMSO-$d_6$) δ 8.70 (s, 1H), 8.63-8.62 (m, 1H), 8.23-8.22 (m, 1H), 8.15-8.14 (m, 1H), 8.13-8.12 (m, 1H), 7.96-7.93 (m, 1H), 7.39-7.37 (m, 1H), 7.21-7.20 (m, 1H), 6.87-6.80 (m, 1H),6.20-6.15(m, 4H), 5.76-5.73 (m, 4H), 3.97-3.79(m, 8H); ESI-MS(m/z): 575.0 [M+H]$^+$.

**Example 36**

**Preparation of (*E*)-5-chloro-*N*-(2-chloro-5-(4-(4-(4-oxopent-2-enoyl)piperazin-1-yl)quinazolin-6-yl)pyridin-3-yl)thiophene-2-sulfonamide**

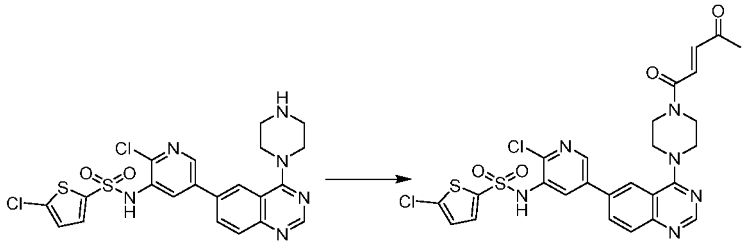

5-chloro-*N*-(2-chloro-5-(4-(piperazin-1-yl)quinazolin-6-yl)pyridin-3-yl)thiophene-2-sulfonamide (100 mg, 0.192 mmol) was dissolved in tetrahydrofuran (2 mL). The reaction system was cooled to -78°C, with *N,N*-diisopropylethylamine (124 mg, 0.960 mmol), (*E*)-4-oxopent-2-enoic acid (22 mg, 0.192mmol) and 50% $T_3P$ ethyl acetate solution (244 mg, 0.384 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (*E*)-5-chloro-*N*-(2-chloro-5-(4-(4-(4-oxopent-2-enoyl)piperazin-1-yl)quinazolin-6-yl)pyridin-3-yl)thiophene-2-sulfonamide was obtained, yield: 14.4%; $^1$H NMR (400 MHz, DMSO-$d_6$) δ 8.71 (s, 1H), 8.61-8.60 (m, 1H), 8.23-8.22 (m, 1H), 8.15-8.14 (m, 1H), 8.13-8.11 (m, 1H), 7.95 (d, *J* = 8.0 MHz, $^1$H ), 7.47-7.37 (m, 2H), 7.21-7.20 (m, 1H), 6.75 (d, *J*= 16.0 MHz, 1H), 3.98-3.80 (m, 8H), 2.37(s, 3H); ESI-MS(m/z): 617.0 [M+H]$^+$.

**Example 37**

**Preparation of *N*-(5-(4-(4-acryloylpiperazin-1-yl)quinazolin-6-yl)-2-methoxypyridin-3-yl)-5-chlorothiophene-2-sulfonamide**

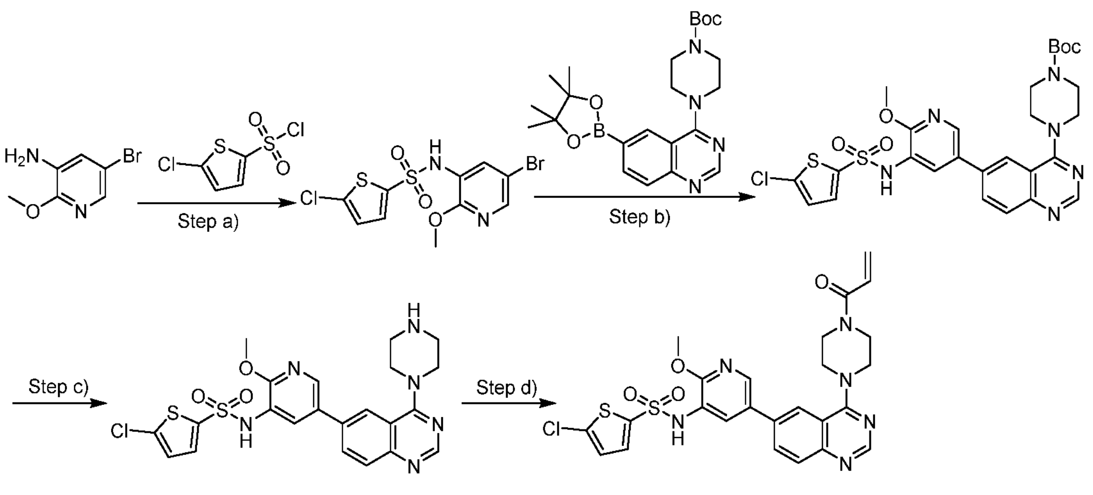


Step a): Preparation of *N*-(5-bromo-2-methoxypyridin-3-yl)-5-chlorothiophene-2-sulfonamide

5-bromo-2-methoxypyridin-3-amine (1.0 g, 4.925 mmol), 5-chlorothiophene-2-sulfonyl chloride (1.02 g, 4.679

mmol) were dissolved in pyridine (10 mL). After the addition was completed, at 80°C, the reaction mixture was stirred and reacted overnight. Upon completion of the reaction, the reaction was quenched by adding water (50 mL). The reaction mixture was extracted with ethyl acetate (200 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=10/1 to 3/1). *N*-(5-bromo-2-methoxypyridin-3-yl)-5-chlorothiophene-2-sulfonamide was obtained, yield: 78.5%. ESI-MS(m/z): 384.9 $[M+H]^+$.

Step b): Preparation of tert-butyl 4-(6-(5-((5-chlorothiophene)-2-sulfonamido)-6-methoxypyridin-3-yl)quinazolin-4-yl)piperazine-1-carboxylate

*N*-(5-bromo-2-methoxypyridin-3-yl)-5-chlorothiophene-2-sulfonamide (300 mg, 0.782 mmol), tert-butyl 4-(6-(4,4,5,5-tetramethyl-1,3,2-dioxaborolan-2-yl)quinazolin-4-yl)piperazine-1-carboxylate (370 mg, 0.782 mmol), Pd(dppf)$Cl_2$ (114 mg, 0.156 mmol) and cesium carbonate (750 mg, 2.346 mmol) were added to dioxane/water mixed solvent (10 mL, v/v=10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (200 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: dichloromethane/methanol=100/1 to 40/1). Tert-butyl 4-(6-(5-((5-chlorothiophene)-2-sulfonamido)-6-methoxypyridin-3-yl)quinazolin-4-yl)piperazine-1-carboxylate was obtained, yield: 50.7%; ESI-MS(m/z): 617.1 $[M+H]^+$.

Step c): Preparation of 5-chloro-*N*-(2-methoxy-5-(4-(piperazin-1-yl)quinazolin-6-yl)pyridin-3-yl)thiophene-2-sulfonamide

Tert-butyl 4-(6-(5-((5-chlorothiophene)-2-sulfonamido)-6-methoxypyridin-3-yl)quinazolin-4-yl)piperazine-1-carboxylate (200 mg, 0.804 mmol) was dissolved in dichloromethane (3 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. 5-chloro-*N*-(2-methoxy-5-(4-(piperazin-1-yl)quinazolin-6-yl)pyridin-3-yl)thiophene-2-sulfonamide was obtained. ESI-MS(m/z): 517.1 $[M+H]^+$.

Step d): Preparation of *N*-(5-(4-(4-acryloylpiperazin-1-yl)quinazolin-6-yl)-2-methoxypyridin-3-yl)-5-chlorothiophene-2-sulfonamide

5-chloro-*N*-(2-methoxy-5-(4-(piperazin-1-yl)quinazolin-6-yl)pyridin-3-yl)thiophene-2-sulfonamide (100 mg, 0.193 mmol) was dissolved in tetrahydrofuran (2 mL). The reaction system was cooled to -78°C, with *N,N*-diisopropylethylamine (124 mg, 0.960 mmol), acrylic acid (14 mg, 0.193mmol) and 50% $T_3P$ ethyl acetate solution (245 mg, 0.386 mmol) added successively. After the addition was completed, the reaction mixture was kept at - 78°C and stirred for 1 h. Upon completion of the reaction, the reaction solution was diluted by adding acetonitrile (1 mL) and purified by Prep-HPLC. *N*-(5-(4-(4-acryloylpiperazin-1-yl)quinazolin-6-yl)-2-methoxypyridin-3-yl)-5-chlorothiophene-2-sulfonamide was obtained, yield: 16.6%; $^1$H NMR (400 MHz, DMSO-$d_6$) δ 8.66 (s, 1H), 8.51-8.50 (m, 1H), 8.15-8.09 (m, 1H), 8.02-8.01 (m, 1H), 7.93-7.91 (m, 1H), 7.42-7.41 (m, 1H), 7.23-7.21 (m, 1H), 6.86-6.80 (m, 1H), 6.19-6.14 (m, 4H), 5.75-5.72 (m, 1H), 3.91-3.79 (m, 8H), 3.78 (s, 3H); ESI-MS(m/z): 571.0 $[M+H]^+$.

**Example 38**

**Preparation of (*E*)-5-chloro-*N*-(2-methoxy-5-(4-(4-(4-oxopent-2-enoyl)piperazin-1-yl)quinazolin-6-yl)pyridin-3-yl)thiophene-2-sulfonamide**

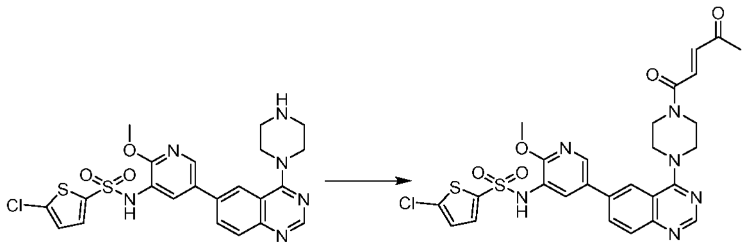

5-chloro-*N*-(2-methoxy-5-(4-(piperazin-1-yl)quinazolin-6-yl)pyridin-3-yl)thiophene-2-sulfonamide (100 mg, 0.193 mmol) was added to tetrahydrofuran (2 mL). The reaction system was stirred to mix evenly and cooled to -78°C, with *N,N*-diisopropylethylamine (124 mg, 0.960 mmol), (E)-4-oxopent-2-enoic acid (22 mg, 0.193mmol) and 50% $T_3P$ ethyl acetate solution (245 mg, 0.386 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC. (*E*)-5-chloro-*N*-(2-methoxy-5-(4-(4-(4-oxopent-2-enoyl)piperazin-1-yl)quinazolin-6-yl)pyridin-3-yl)thiophene-2-sulfonamide was obtained, yield: 18.1%; $^1$H NMR (400 MHz, DMSO-$d_6$) δ 10.42 (s, 1H), 8.69 (s, 1H), 8.53-8.52 (m, 1H), 8.17-8.11 (m, 2H), 8.03 (d, *J*= 4.0 Hz, 1H), 7.93 (d, *J* = 8.0 Hz, 1H), 7.47-7.41 (m, 2H), 7.24 (d, *J* = 4.0 Hz, 1H), 6.77-6.73 (d, *J* = 16.0 Hz, 1H), 3.96-3.80 (m, 8H), 3.78 (s, 3H), 2.37 (s, 3H); ESI-MS(m/z): 613.0 $[M+H]^+$.

**Example 39**

**Preparation of (*E*)-2,4-difluoro-*N*-(1-methyl-2-oxo-5-(4-(4-(4-oxopent-2-enoyl)piperazin-1-yl)quinazolin-6-yl)-1,2-dihydropyridin-3-yl)benzenesulfonamide**

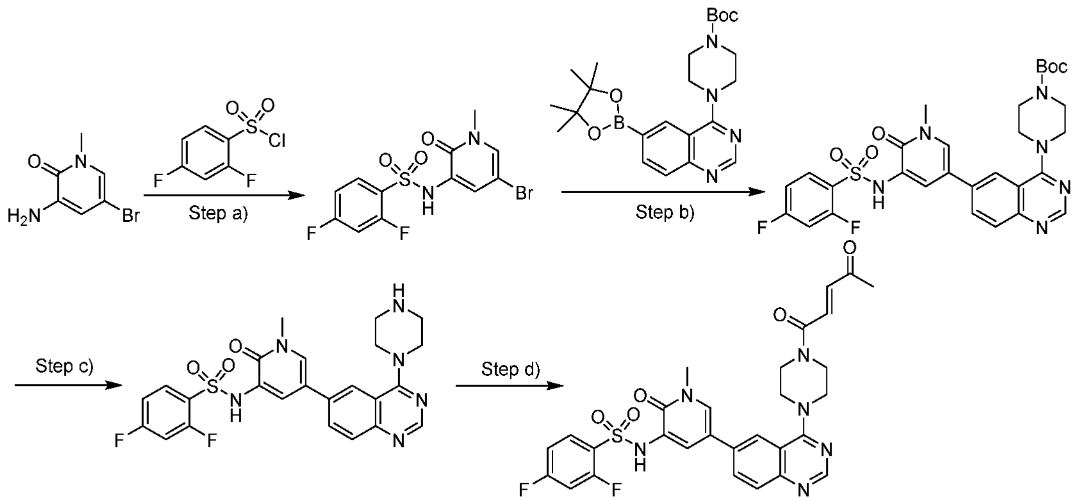


Step a): Preparation of *N*-(5-bromo-1-methyl-2-oxo-1,2-dihydropyridin-3-yl)-2,4-difluorobenzenesulfonamide

3-amino-5-bromo-1-methylpyridin-2(1*H*)-one (500 mg, 2.46 mmol), 4-dimethylaminopyridine (30 mg, 0.25 mmol) and pyridine (600 mg, 7.38 mmol) were dissolved in dichloromethane (15.0 mL), the reaction mixture was cooled to 0°C, with 2,4-difluorobenzenesulfonyl chloride (1.57 g, 7.38 mmol) added. The reaction mixture was heated to 25°C and stirred for 4 h. Upon completion of the reaction, the reaction mixture was diluted by adding dichloromethane (20 mL). The organic phase was washed with water (15 mL×1), 5% citric acid aqueous solution (30 mL×1), and saturated brine (15 mL×1) successively, concentrated under reduced pressure. With acetonitrile (8 mL) added, the crude product was kept at 45°C and stirred for 1 h, then kept at 25°C and stirred for 20 h. The solution was filtered after solid precipitation appeared. *N*-(5-bromo-1-methyl-2-oxo-1,2-dihydropyridin-3-yl)-2,4-difluorobenzenesulfonamide was obtained, yield: 70.0%; ESI-MS(m/z): 378.9 $[M+H]^+$.

Step b): Preparation of tert-butyl 4-(6-(5-((2,4-difluorophenyl)sulfonamido)-1-methyl-6-oxo-1,6-dihydropyridin-3-yl)quinazolin-4-yl)piperazine-1-carboxylate

*N*-(5-bromo-1-methyl-2-oxo-1,2-dihydropyridin-3-yl)-2,4-difluorobenzenesulfonamide (156 mg, 0.413 mmol), tert-butyl 4-(6-(4,4,5,5-tetramethyl-1,3,2-dioxaborolan-2-yl)quinazolin-4-yl)piperazine-1-carboxylate (200 mg, 0.454 mmol), Pd(dppf)$Cl_2$ (45 mg, 0.06 mmol) and cesium carbonate (402 mg, 1.24 mmol) were added to dioxane/water mixed solvent (10 mL, v/v=4:1). After the addition was completed, in the presense of protective nitrogen, the reaction mixture was heated to 95°C and stirred for 0.5 h. Upon completion of the reaction, the reaction mixture was cooled to 25°C, with reaction quenched by adding water (15 mL), extracted with ethyl acetate (20 mL×2). The organic phases were combined, dried over anhydrous sodium sulfate and filtered. The filtrate was concentrated under reduced pressure. The crude product obtained was purified by silica gel column chromatography (Eluent: dichloromethane/methanol=100/1 to 50/1). Tert-butyl 4-(6-(5-((2,4-difluorophenyl)sulfonamido)-1-methyl-6-oxo-1,6-dihydropyridin-3-yl)quinazolin-4-yl)piperazine-1-carboxylate was obtained, yield: 31.7%; ESI-MS(m/z): 613.2 $[M+H]^+$.

Step c): Preparation of 2,4-difluoro-*N*-(1-methyl-2-oxo-5-(4-(piperazin-1-yl)quinazolin-6-yl)-1,2-dihydropyridin-3-yl)benzenesulfonamide trifluoroacetate

Tert-butyl 4-(6-(5-((2,4-difluorophenyl)sulfonamido)-1-methyl-6-oxo-1,6-dihydropyridin-3 -yl)quinazolin-4-yl)piperazine-1-carboxylate (80 mg, 13 mmol) was dissolved in dichloromethane (2.5 mL). Under the ice bath condition, trifluoroacetic acid (0.5 mL) was slowly added dropwise. After the addition was completed, the reaction mixture was stirred at room temperature for 3 h. Upon completion of the reaction, the reaction mixture was concentrated under reduced pressure. Oily crude product was obtained, with methyl tert-butyl ether (5 mL) added, and stirred for 30 min. The solution was filtered after solid precipitation appeared. 2,4-difluoro-*N*-(1-methyl-2-oxo-5-(4-(piperazin-1-yl)quinazolin-6-yl)-1,2-dihydropyridin-3-yl)benzenesulfonamide trifluoroacetate was obtained, yield: 73.7%. ESI-MS(m/z): 513.14 $[M+H]^+$.

Step d): Preparation of (*E*)-2,4-difluoro-*N*-(1-methyl-2-oxo-5-(4-(4-(4-oxopent-2-enoyl)piperazin-1-yl)quinazolin-6-yl)-1,2-dihydropyridin-3 -yl)benzenesulfonamide

2,4-difluoro-*N*-(1-methyl-2-oxo-5-(4-(piperazin-1-yl)quinazolin-6-yl)-1,2-dihydropyridin-3-yl)benzenesulfonamide trifluoroacetate (60 mg, 0.096 mmol) was added to tetrahydrofuran (2 mL). The reaction system was cooled to -70°C, with *N,N*-diisopropylethylamine (75 mg, 0.575 mmol), 3-acetylacrylic acid (11 mg, 0.096 mmol) and $T_3P$ (122 mg, 0.193 mmol) added successively. After the addition was completed, the reaction mixture was kept at -70°C and stirred for 0.5 h. Upon completion of the reaction, the reaction mixture was filtered and purified by Prep-HPLC. (*E*)-2,4-difluoro-*N*-(1-methyl-2-oxo-5-(4-(4-(4-oxopent-2-enoyl)piperazin-1-yl)quinazolin-6-yl)-1,2-dihydropyridin-3-yl)benzenesulfonamide was obtained, yield39.6%; $^1H$ NMR (400 MHz, DMSO-$d_6$) $\delta$ 9.86(brs, 1H), 8.66 (s, 1H), 8.11 (s, 1H), 8.01-7.98 (m, 2H), 7.96-7.92 (m, 1H), 7.91-7.85 (m, 2H), 7.54-7.49 (m, 1H), 7.46 (d, *J*= 16 Hz, 1H), 7.27-7.22 (m, 1H), 6.76 (d, *J*= 16.0 Hz ,1H), 3.87-3.82 (m, 8H), 3.53 (s, 3H), 2.36 (s, 3H); ESI-MS(m/z): 609.0 $[M+H]^+$.

## Example 40

**Preparation of *N*-(5-(4-(4-acryloylpiperazin-1-yl)quinazolin-7-yl)-2-methoxypyridin-3-yl)-2,4-difluorobenzenesulfonamide**

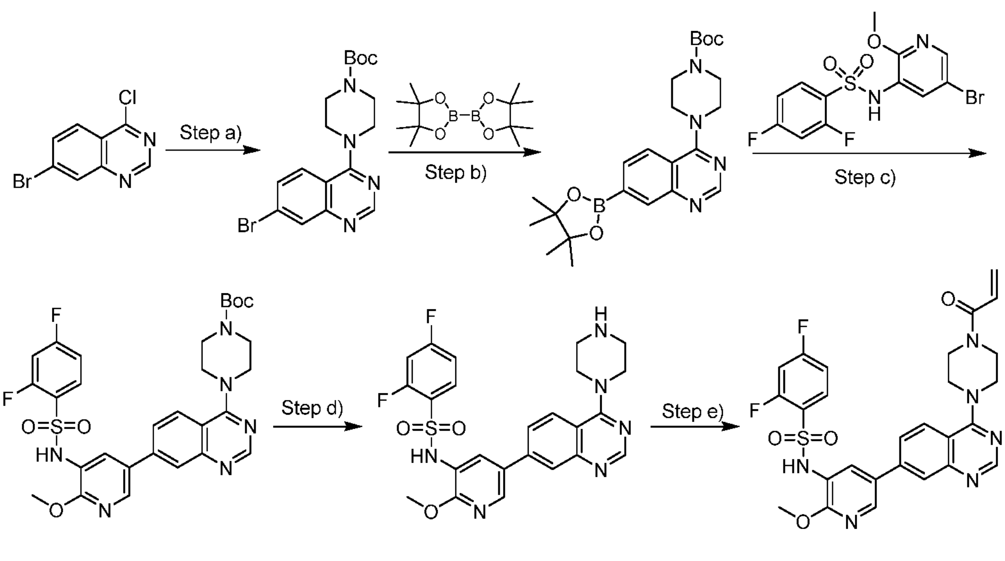


Step a): Preparation of tert-butyl 4-(7-bromoquinazolin-4-yl)piperazine-1-carboxylate

7-Bromo-4-chloroquinazoline (500 mg, 2.053 mmol) and tert-butyl piperazine-1-carboxylate (420 mg, 2.255 mmol) were dissolved in dimethyl sulfoxide (10 mL), with N,N-diisopropylethylamine (796 mg, 6.159 mmol) added. After the addition was completed, the reaction mixture was heated to 55°C and stirred for 1 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 10/1 to 3/1). Tert-butyl 4-(7-bromoquinazolin-4-yl)piperazine-1-carboxylate was obtained, yield: 82.6%; ESI-MS(m/z): 393.1 $[M+H]^+$.

Step b): Preparation of tert-butyl 4-(7-(4,4,5,5-tetramethyl-1,3,2-dioxaborolan-2-yl)quinazolin-4-yl)piperazine-1-carboxylate

Tert-butyl 4-(7-bromoquinazolin-4-yl)piperazine-1-carboxylate (800 mg, 2.034 mmol), bis(pinacolato)diboron (775 mg, 3.051 mmol), Pd(dppf)$Cl_2$ (149 mg, 0.203 mmol) and potassium acetate (599 mg, 6.102 mmol) were dissolved in dioxane (20 mL). After the addition was completed, in the presense of protective nitrogen, the reaction mixture was kept at 110°C and stirred for 4 h. Upon completion of the reaction, the reaction mixture was filtered, and the filtrate was concentrated through pressure concentration. The crude product obtained was purified by silica gel column chromatography (Eluent: dichloromethane/methanol = 50/1 to 10/1). Tert-butyl 4-(7-(4,4,5,5-tetramethyl-1,3,2-dioxaborolan-2-yl)quinazolin-4-yl)piperazine-1-carboxylate was obtained, yield: 66.6%; ESI-MS(m/z): 441.3 $[M+H]^+$.

Step c): Preparation of tert-butyl 4-(7-(5-((2,4-difluorophenyl)sulfonamido)-6-methoxypyridin-3-yl)quinazolin-4-yl)piperazine-1-carboxylate

Tert-butyl 4-(7-(4,4,5,5-tetramethyl-1,3,2-dioxaborolan-2-yl)quinazolin-4-yl)piperazine-1-carboxylate (735 mg, 1.668 mmol), *N*-(5-bromo-2-methoxypyridin-3-yl)-2,4-difluorobenzenesulfonamide (500 mg, 1.319 mmol), Pd(dppf)$Cl_2$ (96 mg, 0.132 mmol) and cesium carbonate (1.289 g, 3.957 mmol) were added to dioxane/water mixed solvent (25 mL, v/v=10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (150 mL×2 times). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Tert-butyl 4-(7-(5-((2,4-difluorophenyl)sulfonamido)-6-methoxypyridin-3-yl)quinazolin-4-yl)piperazine-1-carboxylate was obtained, yield: 37.8%; ESI-MS(m/z): 613.2 $[M+H]^+$.

Step d): Preparation of 2,4-difluoro-N (2-methoay-5-(4-(piperazin-1-yl)quinazolin-7-yl)pyridin-3-yl)benzenesulfonamide

Tert-butyl 4-(7-(5-((2,4-difluorophenyl)sulfonamido)-6-methoxypyridin-3-yl)quinazolin-4-yl)piperazine-1-carboxylate (200 mg, 0.326 mmol) was dissolved in dichloromethane (4 mL). Under the ice bath condition, trifluoroacetic

acid (1 mL) was slowly added dropwise. After the addition was completed, the reaction mixture was stirred at room temperature for 1 h. Upon completion of the reaction, the reaction mixture was concentrated under reduced pressure. 2,4-difluoro-*N*-(2-methoxy-5-(4-(piperazin-1-yl)quinazolin-7-yl)pyridin-3-yl)benzenesulfonamide was obtained. ESI-MS(m/z): 513.1 $[M+H]^+$.

Step e): Preparation of *N*-(5-(4-(4-acryloylpiperazin-1-yl)quinazofin-7-yl)-2-methoxypyridin-3-yl)-2,4-difluorobenzenesulfonamide

2,4-difluoro-*N*-(2-methoxy-5-(4-(piperazin-1-yl)quinazolin-7-yl)pyridin-3-yl)benzenesulfonamide (100 mg, 0.195 mmol) was dissolved in tetrahydrofuran (3 mL). The reaction system was cooled to -78°C, with *N,N*-diisopropylethylamine (126 mg, 0.975 mmol), acrylic acid (14.0 mg, 0.195mmol) and 50% $T_3P$ ethyl acetate solution (124 mg, 0.390 mmol) added successively. After the addition was completed, the reaction mixture was kept at -78°C and stirred for 1 h. Upon completion of the reaction, the reaction was quenched by adding acetonitrile (2 mL), the reaction solution was purified by Prep-HPLC. *N*-(5-(4-(4-acryloylpiperazin-1-yl)quinazolin-7-yl)-2-methoxypyridin-3-yl)-2,4-difluorobenzenesulfonamide was obtained, yield: 13.1%; $^1H$ NMR (400 MHz, DMSO-$d_6$) $\delta$ 10.37 (brs, 1H), 8.67 (s, 1H), 8.51 (s, 1H), 8.24 (s, 1H), 8.16-8.13 (m, 1H), 8.04-8.02 (m, 1H), 7.84-7.76 (m, 2H), 7.60-7.54 (m, 1H), 7.24-7.19(m, 1H), 6.89-6.82 (m, 1H), 6.20-6.15 (m, 1H), 5.76-5.73 (m, 1H),3.84-3.77 (m, 8H), 3.70 (s, 3H); ESI-MS(m/z): 567.0 $[M+H]^+$.

**Example 41**

**Preparation of *N*-(5-(4-(4-acryloylpiperazin-1-yl)-7,8-dihydropyrido[4,3-*d*]pyrimidin-6(5*H*)-yl)-2-methoxypyridin-3-yl)-2,4-difluorobenzenesulfonamide**

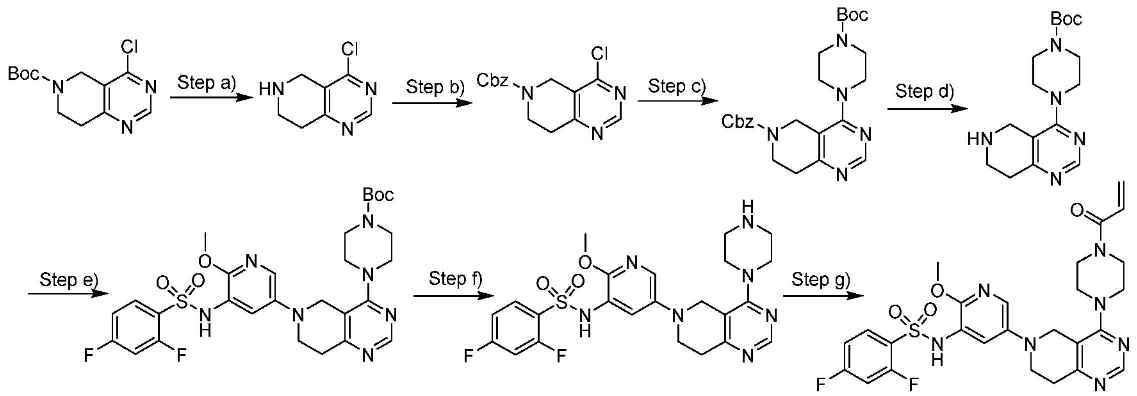


Step a): Preparation of 4-chloro-5,6,7,8-tetrahydropyrido[4,3-*d*]pyrimidine

Tert-butyl 4-chloro-7,8-dihydropyrido[4,3-*d*]pyrimidine-6(5*H*)-carboxylate (3.0 g, 11.111 mmol) was dissolved in dichloromethane (10 mL). Under the ice bath condition, HCl dioxane solution (4M, 10 mL) was added to the system. After the addition was completed, the reaction mixture was heated to room temperature and stirred for 1 h. Upon completion of the reaction, the reaction mixture was concentrated under reduced pressure. 4-chloro-5,6,7,8-tetrahydropyrido[4,3-*d*]pyrimidine was obtained, yield: 90.3%; ESI-MS(m/z): 170.1 $[M+H]^+$.

Step b): Preparation of benzyl 4-chloro-7,8-dihydropyrido[4,3-*d*]pyrimidine-6(5*H*)-carboaylate

4-chloro-5,6,7,8-tetrahydropyrido[4,3-*d*]pyrimidine (1.7 g, 1.003 mmol)was dissolved in dichloromethane (30 mL). Under the ice bath condition, triethylamine (5.1 g, 5.015 mmol) and benzyl chloroformate (2.6 g, 1.505 mmol) were added to the system. After the addition was completed, the reaction mixture was heated to room temperature and reacted for 2 h. Upon completion of the reaction, the reaction was quenched by adding water (50 mL). The reaction mixture was extracted by adding dichloromethane (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=3/1 to 1/1). Benzyl 4-chloro-7,8-dihydropyrido[4,3-*d*]pyrimidine-6(5*H*)-carboxylate was obtained, yield: 98.6%; ESI-MS(m/z): 304.3 $[M+H]^+$.

Step c): Preparation of benzyl 4-(4-(tert-butoaycarbonyl)piperazin-1-yl)-7,8-dihydropyrido[4,3-*d*]pyrimidine-6(5H)-carboxylate

Benzyl 4-chloro-7,8-dihydropyrido[4,3-*d*]pyrimidine-6(5*H*)-carboxylate (2.9 g, 9.539 mmol) were dissolved in *N*-methylpyrrolidone (30 mL), with triethylamine (2.9 g, 28.617 mmol) and tert-butyl piperazine-1-carboxylate (2.7 g, 14.309 mmol) added. After the addition was completed, the reaction mixture was heated to 130°C and stirred for 3 h. Upon completion of the reaction, the reaction was quenched by adding water (50 mL). The reaction mixture was extracted with ethyl acetate (100 mL×2). The organic phases were combined, washed with saturated brine (100 mL×1), dried over anhydrous sodium sulfate and filtered. The filtrate was concentrated under reduced pressure. The crude product obtained was purified by silica gel column chromatography (Eluent: petroleum ether/ethyl acetate=2/1 to 1/1). Benzyl 4-(4-(tert-butoxycarbonyl)piperazin-1-yl)-7,8-dihydropyrido[4,3-*d*]pyriniidine-6(5*H*)-carboxylate was obtained, yield: 92.4%; ESI-MS(m/z): 454.1 $[M+H]^+$.

Step d): Preparation of tert-butyl 4-(5,6,7,8-tetrahydropyrido[4,3-*d*]pyrimidin-4-yl)piperazine-1-carboaylate

Benzyl 4-(4-(tert-butoxycarbonyl)piperazin-1-yl)-7,8-dihydropyrido[4,3-*d*]pyrimidine-6(5*H*)-carboxylate (500 mg, 1.101 mmol) were dissolved in isopropanol (10 mL), with Pd/C (500 mg, 10%) added. After the addition was completed, the reaction mixture was kept at room temperature and stirred at for 3 h. Upon completion of the reaction, the reaction mixture was filtered. The filtrate was concentrated under reduced pressure. The crude product obtained was purified by silica gel column chromatography (Eluent: petroleum ether/ethyl acetate=2/1 to 1/1). Tert-butyl 4-(5,6,7,8-tetrahydropyrido[4,3-*d*]pyrimidin-4-yl)piperazine-1-carboxylate was obtained, yield: 85.5%; ESI-MS(m/z): 320.1 $[M+H]^+$.

Step e): Preparation of tert-butyl 4-(6-(5-((2,4-difluorophenyl)sulfonamido)-6-methoxypyridin-3-yl)-5,6,7,8-tetrahydropyrido [4,3-*d*]pyrimidin-4-yl)piperazine-1-carboxylate

Tert-butyl 4-(5,6,7,8-tetrahydropyrido[4,3-*d*]pyrimidin-4-yl)piperazine-1-carboxylate (300 mg, 0.940 mmol), *N*-(5-bromo-2-methoxypyridin-3-yl)-2,4-difluorobenzenesulfonamide (427.7 mg, 1.128 mmol), $Pd_2(dba)_3$ (172.3 mg, 0.188 mmol), RuPhos (131.8 mg, 0.282 mmol) and cesium carbonate (919.7 mg, 2.821 mmol) were added to toluene (10 mL). After the addition was completed, in the presense of protective nitrogen, the reaction mixture was heated to 110°C and stirred for 2 h. Upon completion of the reaction, the reaction was quenched by adding water (50 mL). The reaction mixture was extracted with ethyl acetate (100 mL×2) The organic phases were combined, washed with saturated brine (100 mL xl), dried over anhydrous sodium sulfate and filtered. The filtrate was concentrated under reduced pressure. The crude product obtained was purified by silica gel column chromatography (Eluent: petroleum ether/ethyl acetate=1/1 to 1/2). Tert-butyl 4-(6-(5-((2,4-difluorophenyl)sulfonamido)-6-methoxypyridin-3-yl)-5,6,7,8-tetrahydropyrido[4,3-*d*]pyrimidin-4-yl)piperazine-1-carboxylate was obtained, yield: 34.4%; ESI-MS(m/z): 618.3 $[M+H]^+$.

Step f): Preparation of 2,4-difluoro-*N*-(2-methoxy-5-(4-(piperazin-1-yl)-7,8-dihydropyrido[4,3-*d*]pyrimidin-6(5*H*)-yl)pyridin-3-yl)benzenesulfonamide

Tert-butyl 4-(6-(5-((2,4-difluorophenyl)sulfonamido)-6-methoxypyridin-3-yl)-5,6,7,8-tetrahydropyrido[4,3-*d*]pyrimidin-4-yl)piperazine-1-carboxylate (200 mg, 0.324 mmol) was dissolved in dichloromethane (4 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. 2,4-difluoro-*N*-(2-methoxy-5-(4-(piperazin-1-yl)-7,8-dihydropyrido[4,3-*d*]pyrimidin-6(5*H*)-yl)pyridin-3-yl)benzenesulfonamide was obtained. ESI-MS(m/z): 518.2 $[M+H]^+$.

Step g): Preparation of *N*-(5-(4-(4-acryloylpiperazin-1-yl)-7,8-dihydropyrido[4,3-*d*]pyrimidin-6(5*H*)-yl)-2-methoxypyridin-3-yl)-2,4-difluorobenzenesulfonamide

2,4-difluoro-*N*-(2-methoxy-5-(4-(piperazin-1-yl)-7,8-dihydropyrido[4,3-*d*]pyrimidin-6(5*H*)-yl)pyridin-3-yl)benzenesulfonamide (150.0 mg, 0.289 mmol) was dissolved in dichloromethane (10 mL). The reaction system was cooled to -78°C, with *N,N*-diisopropylethylamine (188.2 mg, 1.445 mmol), acrylic acid (31.3 mg, 0.434 mmol) and HATU (165.1 mg, 0.434 mmol) added successively. After the addition was completed, the reaction mixture was kept at -78°C and stirred for 1 h. Upon completion of the reaction, the reaction was quenched by adding water (5 mL). The reaction mixture was extracted by adding dichloromethane (10 mL×2). The organic phases were combined, dried over anhydrous sodium sulfate and filtered. The filtrate was concentrated under reduced pressure. The crude product obtained was purified by Prep-HPLC. N (5-(4-(4-acryloylpiperazin-1-yl)-7,8-dihydropyrido[4,3-*d*]pyrimidin-6(5*H*)-yl)-2-methoxypyridin-3-yl)-2,4-difluorobenzenesulfonamide was obtained, yield: 13.1%; $^1$H NMR (400 MHz, DMSO-$d_6$) δ 8.48 (s, 1H), 7.75-7.73 (m,

1H), 7.25-7.20 (m, 1H), 7.10 (s, 2H), 6.88-6.80 (m, 3H), 6.15 (d, *J*= 16.0 Hz, 1H), 5.72 (d, *J* =16.0 Hz, 1H), 4.01 (s, 2H), 3.70-3.64 (m, 7H), 3.40-3.36 (m, 6H), 2.77-2.74 (m, 2H); ESI-MS(m/z): 572.0 $[M+H]^+$.

**Example 42**

**Preparation of (*E*)-2,6-difluoro-*N*-(2-methoxy-5-(4-(2-(4-oxopent-2-enoyl))-2,6-diazaspiro[3.4]octane-6-yl)quinazolin-6-yl)pyridin-3-yl)benzenesulfonamide**

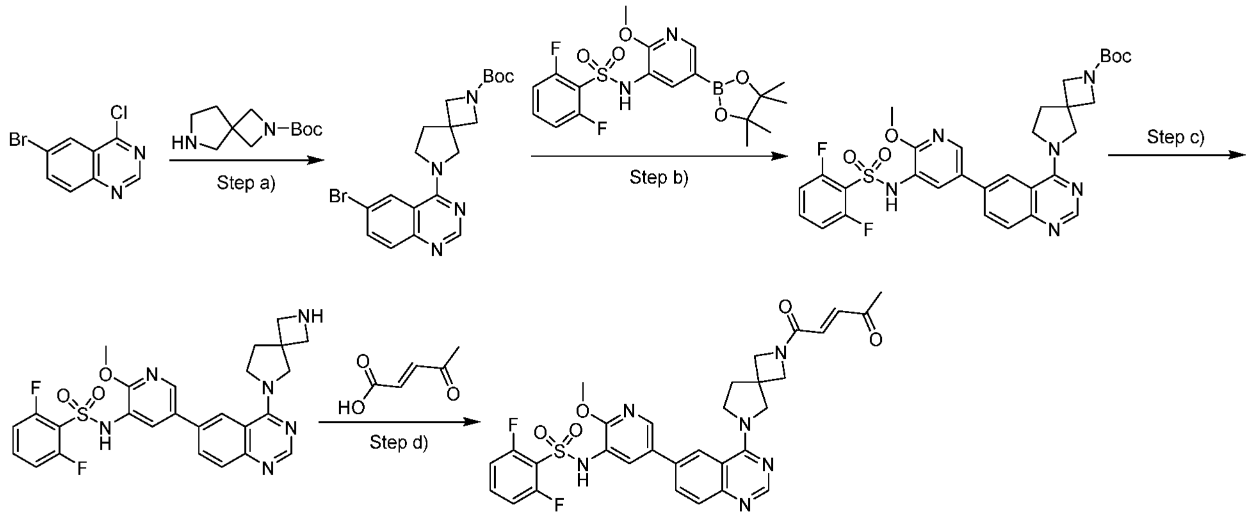


Step a): Preparation of Tert-butyl 6-(6-bromoquinazolin-4-yl)-2,6-diazaspiro[3.4]octane-2-carboxylate

6-bromo-4-chloroquinazoline (500 mg, 2.053 mmol), tert-butyl 2,6-diazaspiro[3.4]octane-2-carboxylate (436 mg, 2.464 mmol) and triethylamine (623 mg, 6.159 mmol) were added to *N,N*-dimethylformamide (10 mL). After the addition was completed, the reaction mixture was heated to 60°C, stirred and reacted for 2 h. Upon completion of the reaction, the reaction was quenched by adding water (50 mL). The reaction mixture was extracted by adding dichloromethane (150 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 50/1). Tert-butyl 6-(6-bromoquinazolin-4-yl)-2,6-diazaspiro[3.4]octane-2-carboxylatewas obtained, yield: 83.9%; ESI-MS(m/z): 419.1 $[M+H]^+$.

Step b): Preparation of tert-butyl 6-(6-(5-((2,6-difluorophenyl)sulfonamido)-6-methoxypyridin-3-yl)quinazolin-4-yl)-2,6-diazaspiro[3.4]octane-2-carboxylate

Tert-butyl 6-(6-bromoquinazolin-4-yl)-2,6-diazaspiro[3.4]octane-2-carboxylate (400 mg, 0.954 mmol), 2,6-difluoro-*N*-(2-methoxy-5-(4,4,5,5-tetramethyl-1,3,2-dioxaboran-2-yl)pyridine-3-benzenesulfonamide (447 mg, 1.049 mmol), $Pd(dppf)Cl_2$ (140 mg, 0.191 mmol) and cesium carbonate (932 mg, 2.862 mmol) were added to dioxane/water mixed solvent (10 mL, v/v = 10: 1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (200 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol=100/1 to 50/1). tert-butyl 6-(6-(5-((2,6-difluorophenyl)sulfonamido)-6-methoxypyridin-3-yl)quinazolin-4-yl)-2,6-diazaspiro[3.4]octane-2-carboxylate was obtained, yield: 90.5%; ESI-MS(m/z): 639.2 $[M+H]^+$.

Step c): Preparation of *N*-(5-(4-(2,6-diazaspiro[3.4]octane-6-yl)quinazolin-6-yl)-2-methoxypyridin-3-yl)-2,6-difluorobenzenesulfonamide trifluoroacetate

tert-butyl 6-(6-(5-((2,6-difluorophenyl)sulfonamido)-6-methoxypyridin-3-yl)quinazolin-4-yl)-2,6-diazaspiro[3.4]octane-2-carboxylate (500 mg, 0.783 mmol) was dissolved in dichloromethane (4 mL). Under the ice bath condition, TFA (1.5 mL) was slowly added dropwise. After the addition was completed, the reaction mixture was stirred at room temperature for 1 h. Upon completion of the reaction, the reaction mixture was concentrated under reduced pressure. Methyl tert-butyl ether was added to the crude product. The mixture was stirred to precipitate solid and filtered. *N*-(5-(4-(2,6-diazaspiro[3.4]octane-6-yl)quinazolin-6-yl)-2-methoxypyridin-3-yl)-2,6-difluorobenzenesulfonamide trifluoroacetate was obtained, yield: 92.8%; ESI-MS(m/z): 539.2 $[M+H]^+$.

Step d): Preparation of (*E*)-2,6-difluoro-*N*-(2-methoxy-5-(4-(2-(4-oxopent-2-enoyl))-2,6-diazaspiro[3.4]octane-6-yl)quinazolin-6-yl)pyridin-3-yl)benzenesulfonamide

*N*-(5-(4-(2,6-diazaspiro[3.4]octane-6-yl)quinazolin-6-yl)-2-methoxypyridin-3-yl)-2,6-difluorobenzenesulfonamide trifluoroacetate (150 mg, 0.230 mmol) was dissolved in tetrahydrofuran (2 mL). The reaction system was cooled to -78°C, with *N,N*-diisopropylethylamine (178 mg, 1.380 mmol), (E)-4-oxopent-2-enoic acid (26 mg, 0.230 mmol) and 50% $T_3P$ ethyl acetate solution (293 mg, 0.460 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). (*E*)-2,6-difluoro-*N*-(2-methoxy-5-(4-(2-(4-oxopent-2-enoyl))-2,6-diazaspiro[3.4]octane-6-yl)quinazolin-6-yl)pyridin-3-yl)benzenesulfonamide was obtained, yield: 19.1%; $^1H$ NMR (400 MHz, DMSO-$d_6$) δ 8.48 (s, 1H), 8.43 (s, 1H), 8.36 (s, 1H), 8.01-7.99 (m, 2H), 7.80 (d, *J*= 8.0 Hz, 1H), 7.71-7.65 (m, 1H), 7.27-7.22 (m,2H), 6.88 (d, *J*= 16.0 Hz, 1H), 6.71 (d, *J*= 16.0 Hz, 1H), 4.41 (d, *J* = 8.0 Hz, 1H), 4.31 (d, *J* = 8.0 Hz, 1H), 4.24-4.17 (m, 2H), 4.10-4.07 (m, 1H), 4.02-3.96 (m, 3H), 3.65 (s, 3H), 2.83 (s, 3H), 2.28-2.24 (m, 2H); ESI-MS(m/z): 635.0 $[M+H]^+$.

Referring to the preparation method of Example 42 and using the corresponding raw materials, the compounds in the following examples were prepared.

| Number | Name | Structure | $^1H$ NMR and MS |
|---|---|---|---|
| **Example 43** | 2,6-difluoro-*N*-(5-(4-(4-(2-fluoroacryloyl)pipe razin-1-yl) quinazolin-6-yl)-2-methoxypyridin-3-yl) benzenesulfona mide | 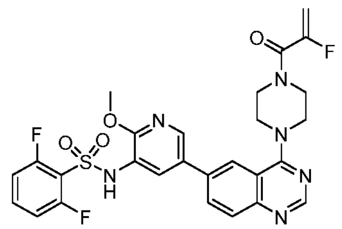 | $^1H$ NMR (400 MHz, DMSO-$d_6$) δ 8.66 (s, 1H), 8.49 (s, 1H), 8.13-8.03 (m, 3H), 7.93-7.91 (m, 1H), 7.71-7.69 (m, 1H), 7.30-7.24 (m, 2H), 5.38-5.20 (m, 2H), 3.92-3.69 (m, 8H), 3.65 (s, 3H); ESI-MS(m/z): 585.0 $[M+H]^+$. |
| **Example 44** | (*E*)-2,6-difluoro-*N*-(2-methoxy-5-(4-(4-(4,4,4-trifluorobut-2-enoyl) piperazin-1-yl)quinazolin-6-yl)pyridin-3-yl) benzenesulfona mide | 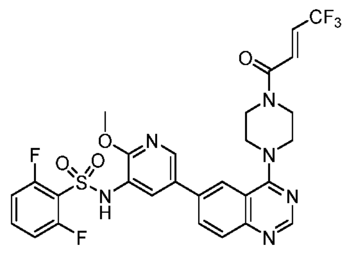 | $^1H$ NMR (400 MHz, DMSO-$d_6$) δ 10.59 (brs, 1H), 8.67 (s, 1H), 8.48 (s, 1H), 8.13-8.08 (m, 2H), 8.04 (d, *J* = 4.0 Hz, 1H), 7.93 (d, *J* = 8.0 Hz, 1H), 7.75-7.68 (m, 1H), 7.43-7.38 (m, 1H), 7.30-7.25 (t, 2H), 6.86-6.77 (m, 1H), 3.91-3.80 (m, 8H), 3.65 (s, 3H); ESI-MS (m/z): 635.0 $[M+H]^+$. |
| **Example 45** | (*S*)-2,6-difluoro-*N*-(5-(4-(4-(2-fluoroacryloyl)-2-methylpiperazin-1-yl) quinazolin-6-yl)-2-methoxypyridin-3-yl) benzenesulfona mide | 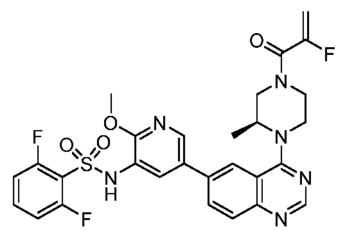 | $^1H$ NMR (400 MHz, DMSO-$d_6$) δ 10.61 (s, 1H), 8.69 (s, 1H), 8.47 (s, 1H), 8.11-8.04 (m, 3H), 7.94 (d, *J* = 8.0 Hz, 1H), 7.71 (s, 1H), 7.27 (s, 2H), 5.39-5.35 (m, 1H), 5.34-5.22 (m, 1H), 4.81 (s, 1H), 4.25-4.09 (m, 3H), 3.69 (s, 4H), 3.20 (d, *J* = 4.0 Hz, 2H), 1.35 (d, *J* = 4.0 Hz, 3H); ESI-MS(m/z): 599.2 $[M+H]^+$. |

(continued)

| Number | Name | Structure | $^1$H NMR and MS |
|---|---|---|---|
| **Example 46** | (*S*,*E*)-2,6-difluoro-*N*-(2-methoxy-5-(4-(2-methyl-4-(4-oxopent-2-enoyl)piperazin-1-yl)quinazolin-6-yl)pyridin-3-yl)benzenesulfona mide | 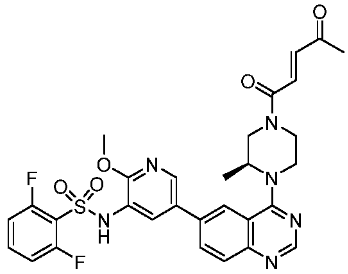 | $^1$H NMR (400 MHz, DMSO-$d_6$) δ 8.63 (s, 1H), 8.35 (s, 1H), 8.06-8.02 (m, 2H), 7.96 (s, 1H), 7.90 (d, *J* = 8.0 Hz, 1H), 7.68-7.61 (m, 1H), 7.52-7.30 (m, 1H), 7.21 (t, *J* =8.0 Hz, 2H), 6.76-5.70 (m, 1H), 4.79-4.73 (m, 1H), 4.41-4.14 (m, 3H), 3.96 (d, *J* = 16.0 Hz, 1H), 3.65 (s, 4H), 3.22-3.11 (m, 1H), 2.36 (d, *J* = 4.0 Hz, 3H), 1.31-1.28 (m, 3H); ESI-MS(m/z): 623.2 [M+H]$^+$. |
| **Example 47** | 2,6-difluoro-*N*-(5-(4-(2-(2-fluoroacryloyl)-2,7-diazaspiro [3.5] non an-7-yl)quinazolin-6-yl)-2-methoxypyridin-3-yl) benzenesulfona mide | 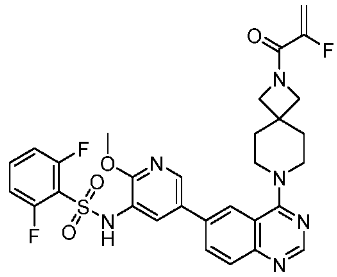 | $^1$H NMR (400 MHz, DMSO-$d_6$) δ 10.59 (s, 1H), 8.62 (s, 1H), 8.40 (s, 1H), 8.09-8.01 (m, 2H), 7.98-7.97 (m, 1H), 7.88 (d, *J*= 8.4 Hz, 1H), 7.71-7.67 (m, 1H), 7.25 (t, *J* = 9.2 Hz, 2H), 5.47 (dd, *J* = 48.4, 3.6 Hz, 1H), 5.29 (dd, *J* = 16.4, 3.6 Hz, 1H), 4.17 (d, *J* = 3.2 Hz, 2H), 3.79 (s, 2H), 3.75-3.73 (m, 4H), 3.66 (s, 3H), 1.95 (t, *J* = 5.2 Hz, 4H); ESI-MS(m/z): 625.0 [M+H]$^+$. |
| **Example 48** | 2,6-difluoro-*N*-(5-(4-(2-(2-fluoroacryloyl)-2,6-diazaspiro [3.4]octa n-6-yl)quinazolin-6-yl)-2-methoxypyridin-3-yl) benzenesulfona mide | 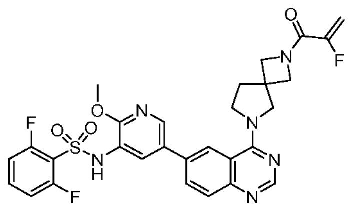 | $^1$H NMR (400 MHz, DMSO-$d_6$) δ 10.58 (s, 1H), 8.47 (s, 1H), 8.42 (s, 1H), 8.35 (s, 1H), 8.01-7.98 (m, 2H), 7.80 (d, *J* = 8.0 Hz, 1H), 7.72-7.65 (m, 1H), 7.27-7.22 (m, 2H), 5.52-5.39 (m, 1H), 5.30-5.25 (m, 1H), 4.48-4.45 (m, 1H), 4.36-4.32 (m, 1H), 4.23-4.16 (m, 2H), 4.10-4.07 (m, 1H), 4.01-3.96 (m, 3H), 3.66 (s, 3H), 2.29-2.20 (m, 2H); ESI-MS (m/z): 611.0 [M+H]$^+$. |
| **Example 49** | (*E*)-2,6-difluoro-*N*-(2-methoxy-5-(4-(2-(4-oxopent-2-enoyl)-2,7-diazaspiro [3.5] non an-7-yl)quinazolin-6-yl) pyridin-3-yl)benzenesulfona mide | 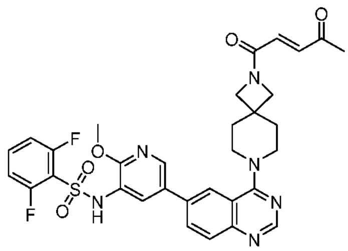 | $^1$H NMR (400 MHz, DMSO-$d_6$) δ 10.56 (s, 1H), 8.62 (s, 1H), 8.35 (s, 1H), 8.06-8.03 (m, 2H), 7.97-7.95 (m, 1H), 7.88 (d, *J* = 12.0 Hz, 1H), 7.67-7.65 (m, 1H), 7.23 (t, *J* = 12.0 Hz, 2H), 6.93 (d, *J* = 16.0 Hz, 1H), 6.74 (d, *J* =16.0 Hz, 1H), 4.14 (s, 2H), 3.79 (s, 2H), 3.76-7.72 (m,4H), 3.67 (s, 3H), 2.34 (s, 3H), 1.96 (t, *J* = 5.2 Hz, 4H); ESI-MS(m/z): 649.0 [M+H]$^+$. |

(continued)

| Number | Name | Structure | $^1$H NMR and MS |
|---|---|---|---|
| **Example 50** | *N*-(5-(4-(2-acryloyl-2,7-diazaspiro [3.5] non an-7-yl) quinazolin-6-yl)-2-methoxypyridin-3-yl)-2,6-difluorobenzenesul fonamide | 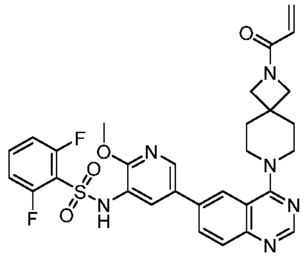 | $^1$H NMR (400 MHz, DMSO-$d_6$) δ 10.59 (s, 1H), 8.62 (s, 1H), 8.42 (s, 1H), 8.11-7.95 (m, 3H), 7.89 (d, *J* = 8.4 Hz, 1H), 7.74-7.7.67 (m, 1H), 7.27 (t, *J* = 9.6 Hz, 2H), 6.34 (dd, *J* = 17.2, 10.4 Hz, 1H), 6.11 (dd, *J* = 16.8, 2.4 Hz, 1H), 5.67 (dd, *J* = 10.4, 2.4 Hz, 1H), 4.02 (s, 2H), 3.75-3.73 (m, 6H), 3.66 (s, 3H), 1.94 (t, *J* = 5.2 Hz, 4H); ESI-MS(m/z): 607.0 [M+H]$^+$. |
| **Example 51** | *N*-(5-(4-((2*S*,5*R*)-2,5-dimethyl-4-((*E*)-4-oxopent-2-enoyl)piperazin-1-yl) quinazolin-6-yl)-2-methoxypyridin-3-yl)-2,6-difluorobenzenesul fonamide | 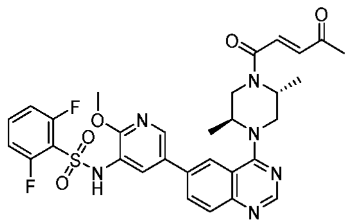 | $^1$H NMR (400 MHz, DMSO-$d_6$) δ 10.59 (s, 1H), 8.66 (s, 1H), 8.45 (s, 1H), 8.10-8.05 (m, 2H), 8.03-7.99 (m, 1H), 7.92 (d, *J* = 12.0 Hz, 1H), 7.74-7.66 (m, 1H), 7.49-7.35 (m, 1H), 7.26 (t, *J* = 12.0 Hz, 2H), 6.78-6.72 (m, 1H), 4.88-4.78 (m, 1H), 4.77-4.44 (m, 1H), 4.21-4.08 (m, 1H), 3.91-3.81 (m, 2H), 3.65 (s, 3H), 3.54-3.42 (m, 1H), 2.37 (s, 3H), 1.31-1.15 (m, 6H); ESI-MS(m/z): 637.0 [M+H]$^+$. |
| **Example 52** | 2,6-difluoro-*N*-(5-(4-((2*S*, 5*R*)-4-(2-fluoroacryloyl)-2,5-dimethylpiperazin-1-yl) quinazolin-6-yl)-2-methoxypyridin-3-yl) benzenesulfona mide | 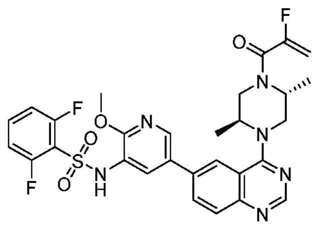 | $^1$H NMR (400 MHz, DMSO-$d_6$) δ 10.60 (s, 1H), 8.65 (s, 1H), 8.46 (s, 1H), 8.09-8.06 (m, 2H), 8.03-8.01 (m, 1H), 7.92 (d, *J* = 12.0 Hz, 1H), 7.74-7.66 (m, 1H), 7.26 (t, *J* = 8.0 Hz, 2H), 5.36-5.16 (m, 2H), 4.95-4.60 (m, 2H), 4.33-4.06 (m, 2H), 3.90-3.71 (m, 2H), 3.65 (s, 3H), 1.30-1.21 (m, 6H); ESI-MS(m/z): 613.0 [M+H]$^+$. |
| **Example 53** | *N*-(5-(4-((2*S*,5*R*)-4-acryloyl-2,5-dimethylpiperazin-1-yl) quinazolin-6-yl)-2-methoxypyridin-3-yl)-2,6-difluorobenzenesul fonamide | 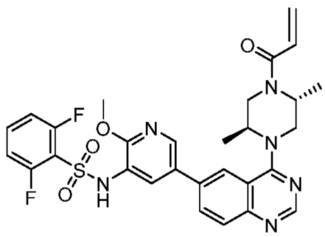 | $^1$H NMR (400 MHz, DMSO-$d_6$) δ 10.60 (s, 1H), 8.65 (s, 1H), 8.49-8.47 (m, 1H), 8.10-8.06 (m, 2H), 8.05-8.01 (m, 1H), 7.93-7.89 (m, 1H), 7.76-7.68 (m, 1H), 7.28 (t, *J* = 8.0 Hz, 2H), 6.88-6.76 (m, 1H), 6.20-6.14 (m, 1H), 5.76-5.71 (m, 1H), 4.85-4.40 (m, 2H), 4.20-3.75 (m, 4H), 3.65 (s, 3H), 1.29-1.20 (m, 6H); ESI-MS(m/z): 595.0 [M+H]$^+$. |

(continued)

| Number | Name | Structure | $^1$H NMR and MS |
|---|---|---|---|
| **Example 54** | 2,6-difluoro-*N*-(2-methoxy-5-(4-(4-(3-oxocyclohex-1-ene-1-carbonyl)piperazin-1-yl)quinazolin-6-yl)pyridin-3-yl)benzenesulfona mide | 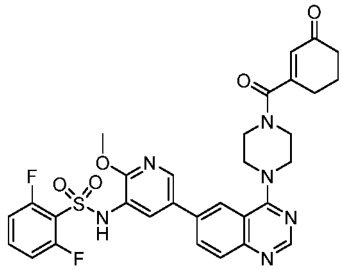 | $^1$HNMR (400 MHz, DMSO-$d_6$) δ 10.59 (s, 1H), 8.68 (s, 1H), 8.49 (s, 1H), 8.11-8.09 (m, 2H), 8.05 (s, 1H), 7.92 (d, *J* = 8 Hz, 1H), 7.75-7.72 (m, 1H), 7.27 (t, *J* = 12 Hz, 2H), 5.94 (s, 1H), 3.95-3.90 (m, 4H), 3.75-3.69 (m, 4H), 3.64 (s, 3H), 3.35-3.34 (m, 2H), 2.41-22.40 (m, 2H), 2.07-2.01 (m, 2H); ESI-MS(m/z): 635.0 [M+H]$^+$. |
| **Example 55** | *N*-(5-(4-(4-(1-acryloylazetidin-3 - yl) piperazin-1-yl)quinazolin-6-yl)-2-methoxypyridin-3-yl)-2,4-difluorobenzenesulfonamide | 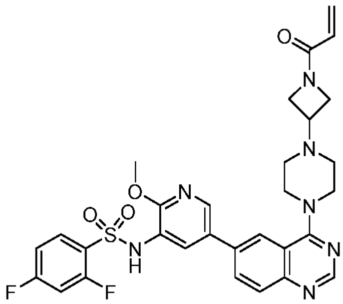 | $^1$H NMR (400 MHz, DMSO-$d_6$) δ 10.41 (s, 1H), 8.64 (s, 1H), 8.45 (s, 1H), 8.06-8.05 (m, 2H), 7.95 (s, 1H), 7.83-7.80 (m, 1H), 7.78-7.76 (m, 1H), 7.53-7.51 (m, 1H), 7.25-7.20 (m, 1H), 6.35-6.31 (m, 1H), 6.08-6.07 (m, 1H), 5.67 (d, *J* = 12.0 Hz, 1H), 4.26-4.23 (m, 1H), 4.13-4.10 (m, 1H), 4.01-3.97 (m, 1H), 3.83-3.79 (m, 5H), 3.69 (s, 3H), 3.28-3.25 (m, 1H), 2.58-2,52 (m, 4H); ESI-MS(m/z): 622.0 [M+H]$^+$. |
| **Example 56** | (*E*)-2,4-difluoro-*N*-(2-methoxy-5-(4-(4-(1-(4-oxopent-2-enoyl)azetidin-3-yl)piperazin-1-yl)quinazolin-6-yl)pyridin-3-yl) benzenesulfona mide | 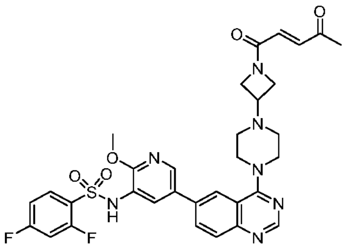 | $^1$HNMR (400 MHz, DMSO-$d_6$) δ 10.41 (s, 1H), 8.64 (s, 1H), 8.45 (s, 1H), 8.06-8.05 (m, 2H), 7.91-7.80 (m, 2H), 7.78-7.76 (m, 1H), 7.53-7.51 (m, 1H), 7.25-7.20 (m, 1H), 6.91 (d, *J* = 12 Hz, 1H), 6.71 (d, *J* = 12 Hz, 1H), 4.38-4.36 (m, 1H), 4.28-4.24 (m, 1H), 4.02-3.97 (m, 1H), 3.89-3.70 (m, 5H), 3.69 (s, 3H), 2.83-2.80 (m, 1H), 2.58-2,52 (m, 4H), 2.23 (s, 3H); ESI-MS(m/z): 664.0 [M+H]$^+$. |
| **Example 57** | 2,4-difluoro-*N*-(5-(4-(4-(1-(2-fluoroacryloyl)azet idin-3-yl) piperazin-1-yl)quinazolin-6-yl)-2-methoxypyridin-3-yl) benzenesulfona mide | 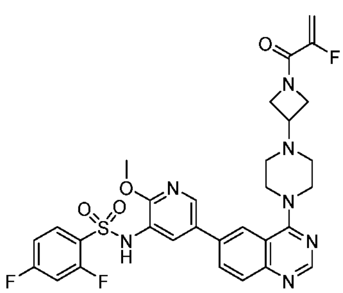 | $^1$HNMR (400 MHz, DMSO-$d_6$) δ 10.41 (s, 1H), 8.64 (s, 1H), 8.45 (s, 1H), 8.06-8.05 (m, 2H), 7.91-7.80 (m, 2H), 7.78-7.76 (m, 1H), 7.53-7.51 (m, 1H), 7.25-7.20 (m, 1H), 5.53-5.40 (m, 1H), 5.31-5.20 (m, 1H), 4.90-4.81 (m, 1H), 4.76-4.73 (m, 1H), 4.07-4.03 (m, 1H), 3.87-3.81 (m, 5H), 3.69 (s, 3H), 3.28-3.25 (m, 1H), 2.58-2,52 (m, 4H); ESI-MS(m/z): 640.0 [M+H]$^+$. |

(continued)

| Number | Name | Structure | $^1$H NMR and MS |
|---|---|---|---|
| **Example 58** | 2,4-difluoro-*N*-(2-methoxy-5-(4-(8-((*E*)-4-oxopent-2-enoyl)-3,8-diazabicyclo [3.2.1] octan-3-yl)quinazolin-6-yl)pyridin-3-yl) benzenesulfona mide | 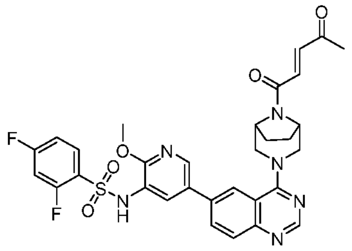 | $^1$H NMR (400 MHz, DMSO-$d_6$) δ 10.31 (brs, 1H), 8.60 (s, 1H), 8.45 (s, 1H), 8.09-8.05 (m, 2H), 8.00-7.98 (m, 1H), 7.88 (d, *J* = 8.0 Hz, 1H), 7.79-7.73 (m, 1H), 7.60-7.55 (m, 1H), 7.41 (d, *J* = 16.0 Hz, 1H), 7.24-7.19 (m, 1H), 6.84 (d, *J* = 16.0 Hz, 1H), 4.80-4.74 (m, 2H), 4.55-4.46 (m, 2H), 3.67 (s, 3H), 3.63-3.57 (m, 2H), 2.93 (s, 3H), 1.96-1.79 (m, 4H); ESI-MS(m/z): 635.0 [M+H]$^+$. |

**Example 59**

**Preparation of 2,4-difluoro-*N*-(5-(4-(4-(2-fluoroacryloyl)piperazin-1-yl)quinazolin-6-yl)-2-methoxypyridin-3-yl)benzenesulfonamide**

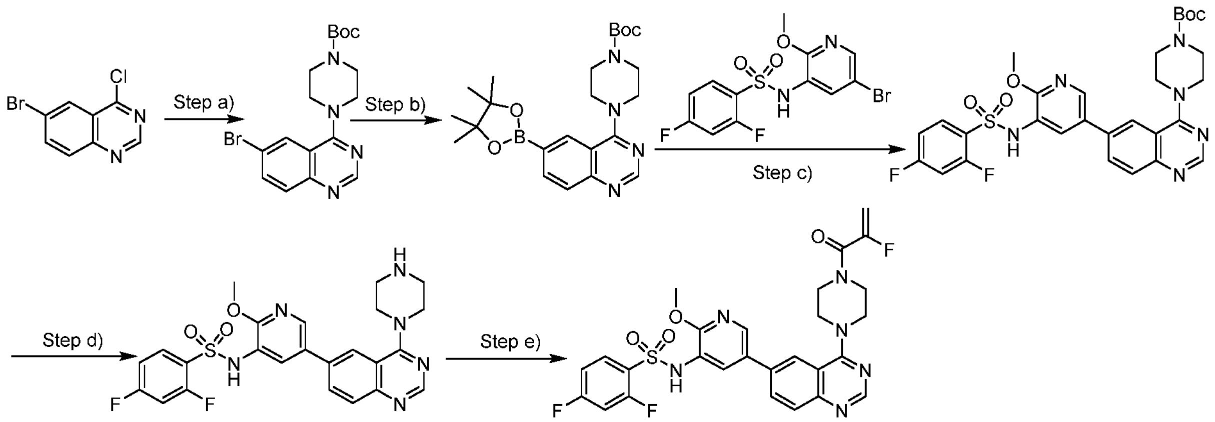


Step a): Preparation of tert-butyl 4-(6-bromoquinazolin-4-yl)piperazine-1-carboxylate

6-Bromo-4-chloroquinazoline (1.9 g, 7.820 mmol) and tert-butyl piperazine-1-carboxylate (1.8 g, 9.380 mmol) were dissolved in dimethyl sulfoxide (30 mL), with triethylamine (2.4 g, 23.470 mmol) added. After the addition was completed, the reaction mixture was heated to 60°C and stirred for 6 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: dichloromethane/methanol = 50/1 to 10/1). Tert-butyl 4-(6-bromoquinazolin-4-yl)piperazine-1-carboxylate was obtained, yield: 65.4%; ESI-MS(m/z): 393.1 [M+H]$^+$.

Step b): Preparation of tert-butyl 4-(6-(4,4,5,5-tetramethyl-1,3,2-dioxaborolan-2-yl)quinazolin-4-yl)piperazine-1-carboxylate

Tert-butyl 4-(6-bromoquinazolin-4-yl)piperazine-1-carboxylate (2.0 g, 5.10 mmol), bis(pinacolato)diboron (1.9 g, 7.640 mmol), Pd(dppf)$Cl_2$ (373 mg, 0.510 mmol) and potassium acetate (1.5 g, 15.310 mmol) were dissolved in dioxane (30 mL). After the addition was completed, in the presense of protective nitrogen, the reaction mixture was kept

at 110°C and stirred for 4 h. Upon completion of the reaction, the reaction mixture was filtered. The filtrate was concentrated through pressure concentration. The crude product obtained was purified by silica gel column chromatography (Eluent: dichloromethane/methanol = 50/1 to 10/1). Tert-butyl 4-(6-(4,4,5,5-tetramethyl-1,3,2-dioxaborolan-2-yl)quinazolin-4-yl)piperazine-1-carboxylate was obtained, yield: 62.5%; ESI-MS(m/z): 441.3 $[M+H]^+$.

Step c): Preparation of tert-butyl 4-(6-(5-((2,4-difluorophenyl)sulfonaniido)-6-methoxypyfidin-3-yl)quinazolin-4-yl)piperazine-1-carboxylate

*N*-(5-bromo-2-methoxypyridin-3-yl)-2,4-difluorobenzenesulfonamide (300 mg, 0.790 mmol), tert-butyl 4-(6-(4,4,5,5-tetramethyl-1,3,2-dioxaborolan-2-yl)quinazolin-4-yl)piperazine-1-carboxylate (698 mg, 1.580 mmol), Pd(dppf)$Cl_2$ (58 mg, 0.080 mmol) and cesium carbonate (779 mg, 2.390 mmol) were dissolved in dioxane/water (4:1, ,10 mL). After the addition was completed, in the presense of protective nitrogen, the reaction mixture was kept at 110°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 1/1 to 1/3). Tert-butyl 4-(6-(5-((2,4-difluorophenyl)sulfonamido)-6-methoxypyridin-3-yl)quinazolin-4-yl)piperazine-1-carboxylate was obtained, yield: 82.5%. ESI-MS(m/z): 613.1 $[M+H]^+$.

Step d): Preparation of 2,4-difluoro-*N*-(2-methoxy-5-(4-(piperazin-1-yl)quinazofin-6-yl)pyridin-3-yl)benzenesulfonamide

Tert-butyl 4-(6-(5-((2,4-difluorophenyl)sulfonamido)-6-methoxypyridin-3-yl)quinazolin-4-yl)piperazine-1-carboxylate (400 mg, 0.650 mmol) was dissolved in dichloromethane (8 mL). Under the ice bath condition, trifluoroacetic acid (2 mL) was slowly added dropwise. After the addition was completed, the reaction mixture was stirred at room temperature for 1 h. Upon completion of the reaction, the reaction mixture was concentrated under reduced pressure. 2.4-difluoro-*N*-(2-methoxy-5-(4-(piperazin-1-yl)quinazolin-6-yl)pyridin-3-yl)benzenesulfonamide was obtained. ESI-MS(m/z): 513.1 $[M+H]^+$.

Step e): Preparation of 2,4-difluoro-*N*-(5-(4-(4-(2-fluoroacryloyl)piperazin-1-yl)quinazolin-6-yl)-2-methoxypyridin-3 -yl)benzenesulfonamide

2-Fluoroacrylic acid (35 mg, 0.388 mmol) and 2,4-difluoro-N-(2-methoxy-5-(4-(piperazin-1-yl)quinazolin-6-yl)pyridin-3-yl)benzenesulfonamide trifluoroacetate (80 mg, 0.128 mmol) were dissolved in THF (2 mL). The reaction system was cooled to -78°C, with *N,N*-diisopropylethylamine (99 mg, 0.766 mmol) and $T_3P$ (244 mg, 0.383 mmol, 50% wt) added successively. After the addition was completed, the reaction mixture was kept at - 78°C and stirred for 1 h. Upon completion of the reaction, the reaction was quenched by adding water (10 mL), and the reaction mixture was extracted with dichloromethane (30 mL×2 times). The organic phases were combined, dried over anhydrous sodium sulfate and filtered. The filtrate was concentrated under reduced pressure. The crude product obtained was purified by Prep-HPLC (Method 2). 2,4-difluoro-*N*-(5-(4-(4-(2-fluoroacryloyl)piperazin-1-yl)quinazolin-6-yl)-2-methoxypyridin-3-yl)benzenesulfonamide was obtained, yield: 12.9%; $^1H$ NMR (400 MHz, DMSO-$d_6$) δ 10.32 (s, 1H), 8.66 (s, 1H), 8.46 (s, 1H), 8.13-8.07 (m, 2H), 8.02-7.99 (m, 1H), 7.91 (d, *J*= 12.0 Hz, 1H), 7.81-7.74 (m, 1H), 7.58-7.51 (m, 1H), 7.24-7.18 (m, 1H), 5.38-5.19 (m, 2H), 3.92-3.88 (m, 4H), 3.82-3.76 (m, 4H), 3.68 (s, 3H); ESI-MS(m/z): 585.0 $[M+H]^+$.

Referring to the preparation method of Example 59 and using the corresponding raw materials, the compounds in the following examples were prepared.

| Number | Name | Structure | $^1$H NMR and MS |
|---|---|---|---|
| **Example 60** | 2,4-difluoro-*N*-(5-(4-(2-(2-fluoroacryloyl)-2,6-diazaspiro[3.4]octa n-6-yl) quinazolin-6-yl)-2-methoxypyridin-3-yl) benzenesulfonam ide | 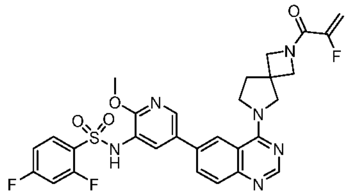 | $^1$H NMR (400 MHz, DMSO-$d_6$) δ 10.30 (brs, 1H), 8.48 (s, 1H), 8.46 (s, 1H), 8.36 (s, 1H), 8.02 (s, 1H), 8.00 (s, 1H), 7.81-7.74 (m, 2H), 7.56 (t, *J* = 12.0 Hz, 1H), 7.20 (t, *J* = 12.0 Hz, 1H), 5.52 (d, *J* = 48.0 Hz, 1H), 5.30 (d, *J* = 16 Hz, 1H), 4.48-4.44 (m, 1H), 4.36-4.32 (m, 1H), 4.24-4.17 (m, 2H), 4.10 (d, *J* = 12.0 Hz, 1H), 4.02-3.96 (m, 3H), 3.68 (s, 3H), 2.28-2.23 (m, 2H); ESI-MS(m/z): 611.0 [M+H]$^+$. |
| **Example 61** | (*E*)-*N*-(5-(4-(2-(3-(1*H*-1,2,3-triazol-1-yl)acryloyl)-2,6-diazaspiro[3.4]octa n-6-yl) quinazolin-6-yl)-2-methoxypyridin-3-yl)-2,4-difluorobenzenesulf onamide | 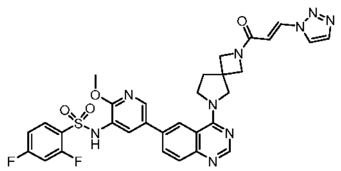 | $^1$H NMR (400 MHz, DMSO-$d_6$) δ 10.31 (s, 1H), 8.67 (s, 1H), 8.48 (s, 1H), 8.38-8.35 (m, 2H), 8.13 (d, *J* = 16.0 Hz, 1H), 8.00-7.92 (m, 3H), 7.81-7.74 (m, 2H), 7.53-7.48 (m, 1H), 7.21-7.16 (m, 1H), 6.90 (d, *J* = 12.0 Hz, 1H), 4.41-4.36 (m, 1H), 4.30 (d, *J* = 8.0 Hz, 1H), 4.25-4.19 (m, 2H), 4.10 (d, *J* = 8.0 Hz, 1H), 4.02-3.98 (m, 3H), 3.69 (s, 3H), 2.29-2.26 (m, 2H); ESI-MS(m/z): 660.0 [M+H]$^+$. |
| **Example 62** | (*E*)-2,4-difluoro-*N*-(2-methoxy-5-(4-(4-(3-(oxazol-2-yl)acryloyl)piperazi n-1-yl) quinazolin-6-yl)pyridin-3-yl) benzenesulfonam ide | 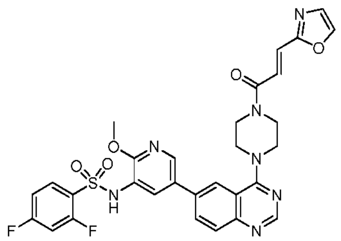 | $^1$H NMR (400 MHz, DMSO-$d_6$) δ 10.33 (s, 1H), 8.66 (s, 1H), 8.48 (d, *J* = 2.4 Hz, 1H), 8.24 (s, 1H), 8.14 (d, *J* = 2.0 Hz, 1H), 8.09 (dd, *J* = 8.4, 2.0 Hz, 1H), 8.02 (d, *J* = 2.4 Hz, 1H), 7.91 (d, *J* = 8.8 Hz, 1H), 7.77 (td, *J* = 8.4, 6.4 Hz, 1H), 7.59-7.75 (m, 1H), 7.46-7.43 (m, 2H), 7.30-7.17 (m, 2H), 3.93-3.84 (m, 8H), 3.68 (s, 3H); ESI-MS (m/z): 634.0 [M+H]$^+$. |
| **Example 63** | 2,4-difluoro-*N*-(2-methoxy-5-(4-(4-(3-methyl-2-methylenebutanoyl) piperazin-1-yl)quinazolin-6-yl)pyridin-3-yl) benzenesulfonamide | 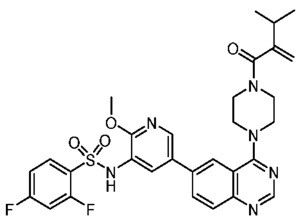 | $^1$H NMR (400 MHz, DMSO-$d_6$) δ 10.33 (brs, 1H), 8.66 (s, 1H), 8.46 (s, 1H), 8.10 (s, 1H), 8.08 (d, *J* = 2.0 Hz, 1H), 8.02 (d, *J* = 2.4 Hz, 1H), 7.92 (d, *J* = 8.4 Hz, 1H), 7.80-7.74 (m, 1H), 7.59-7.53 (t, 1H), 7.24-7.19 (t, 1H), 5.18 (s, 1H), 5.04 (s, 1H), 3.82-3.75 (m, 8H), 3.68 (s, 3H), 2.62-2.55 (m, 1H), 1.05 (d, *J* = 6.8 Hz, 6H); ESI-MS (m/z): 609.0 [M+H]$^+$. |

(continued)

| Number | Name | Structure | $^1$H NMR and MS |
|---|---|---|---|
| **Example 64** | (*E*)-2,4-difluoro-*N*-(2-methoxy-5-(4-(4-(4-methoxybut-2-enoyl) piperazin-1-yl)quinazolin-6-yl)pyridin-3-yl) benzenesulfonam ide | 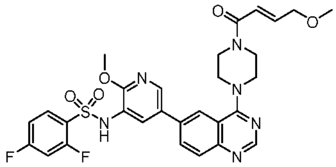 | $^1$H NMR (400 MHz, DMSO-$d_6$) δ 10.34 (brs, 1H), 8.65 (s, 1H), 8.47 (s, 1H), 8.13-8.08 (t, 2H), 8.022 (d, *J* = 4.0 Hz, 1H), 7.92 (d, *J* = 8.0 Hz, 1H), 7.80-7.74 (m, 1H), 7.59-7.54 (t, 1H), 7.24-7.19 (t, 1H), 6.76-6.70 (m, 1H), 6.66-6.61 (m, 1H), 4.09 (d, *J* = 4.0 Hz, 2H), 3.87-3.78 (m, 8H), 3.68 (s, 3H), 3.31(s, 3H); ESI-MS (m/z): 611.0 $[M+H]^+$. |
| **Example 65** | 2,4-difluoro-*N*-(2-methoxy-5-(4-(4-(2-(methoxymethyl) ac ryloyl)piperazin-1-yl) quinazolin-6-yl)pyridin-3-yl) benzenesulfonam ide | 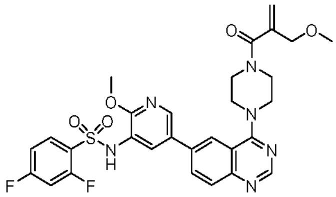 | $^1$H NMR (400 MHz, DMSO-$d_6$) δ 8.64 (s, 1H), 7.93-7.89 (m, 1H), 7.89-7.78 (m, 3H), 7.78-7.75 (m, 1H), 7.57 (d, *J* = 2.0 Hz, 1H), 7.23-7.11 (m, 1H), 7.09-6.98 (m, 1H), 6.06 (s, 1H), 5.53-5.40 (m, 1H), 5.36-5.23 (m, 1H), 4.05 (s, 2H), 3.80 (s, 3H), 3.75 (s, 8H), 3.28 (s, 3H); ESI-MS (m/z): 611.2 $[M+H]^+$. |
| **Example 66** | 2,4-difluoro-*N*-(2-methoxy-5-(4-(4-(2-methoxyacryloyl) pi perazin-1-yl)quinazolin-6-yl)pyridin-3-yl) benzenesulfonam ide | 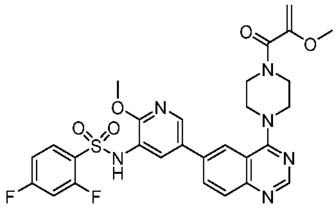 | $^1$H NMR (400 MHz, DMSO-$d_6$) δ 10.34 (s, 1H), 8.66 (s, 1H), 8.47 (s, 1H), 8.12-8.07 (m, 2H), 8.02 (d, *J* = 8.0 Hz, 1H), 7.93-7.89 (m, 1H), 7.80-7.73 (m, 1H), 7.60-7.53 (m, 1H), 7.24-7.18 (m, 1H), 4.47-4.40 (m, 2H), 3.86-3.82 (m, 4H), 3.72-3.67 (m, 7H), 3.60 (s, 3H); ESI-MS (m/z): 597.0 $[M+H]^+$. |
| **Example 67** | methyl (*E*)-4-(4-(6-(5-((2,4-difluorophenyl)sulf onamido)-6-methoxypyridin-3-yl) quinazolin-4-yl)piperazin-1-yl)-4-oxobut-2-enoate | 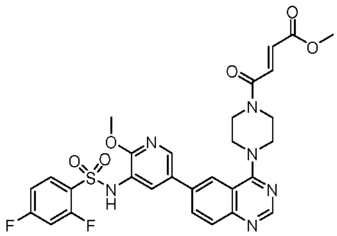 | $^1$H NMR (400 MHz, DMSO-d6) δ 10.33 (s, 1H), 8.67 (s, 1H), 8.45 (s, 1H), 8.14-8.05 (m, 2H), 8.01 (d, *J* = 2.0 Hz, 1H), 7.92 (d, *J* = 8.4 Hz, 1H), 7.83-7.71 (m, 1H), 7.62-7.50 (m, 1H), 7.26-7.15 (m, 1H), 6.85 (d, *J* = 12.0 Hz, 1H), 6.12 (d, *J* = 12.0 Hz, 1H), 3.89-3.79 (m, 4H), 3.79-3.71 (m, 2H), 3.70-3.64 (m, 6H), 3.63-3.55 (m, 2H); ESI-MS (m/z): 625.2 $[M+H]^+$. |

(continued)

| Number | Name | Structure | $^1$H NMR and MS |
|---|---|---|---|
| **Example 68** | 2,4-difluoro-*N*-(5-(4-((2*S*, 5*R*)-4-(2-fluoroacryloyl)-2,5-dimethylpiperazin-1-yl) quinazolin-6-yl)-2-methoxypyridin-3-yl) benzenesulfonam ide | 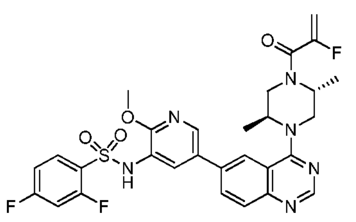 | $^1$H NMR (400 MHz, DMSO-$d_6$) δ 10.33 (brs, 1H), 8.65 (s, 1H), 8.49 (d, *J* = 4.0 Hz, 1H), 8.10-8.07 (m, 2H), 8.02 (d, *J* = 4.0 Hz, 1H), 7.93-7.90 (m, 1H), 7.80-7.74 (m, 1H), 7.62 (t, *J* = 8.0 Hz,1H), 7.20 (t, *J* = 8.0 Hz, 1H), 5.34-5.18 (m, 2H), 4.85 (m, 1H), 4.63-4.30 (m, 1H), 3.81-3.77 (m, 1H), 3.72-3.49 (m, 1H), 3.68 (s, 3H), 1.29-1.23 (m, 6H); ESI-MS(m/z): 613.0 [M+H]$^+$. |
| **Example 69** | 2,4-difluoro-*N*-(5-(4-(4-(3-hydroxybenzoyl)pip erazin-1-yl)quinazolin-6-yl)-2-methoxypyridin-3-yl) benzenesulfonam ide | 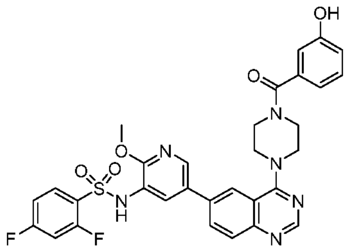 | $^1$H NMR (400 MHz, DMSO-$d_6$) δ 10.33 (s, 1H), 9.72 (s, 1H), 8.66 (s, 1H), 8.46 (d, *J* = 4.0 Hz, 1H), 8.10-8.07 (m, 2H), 8.00 (s, 1H), 7.91 (d, *J* = 8.0 Hz, 1H), 7.80-7.74 (m, 1H), 7.58-7.52 (m, 1H), 7.28-7.18 (m, 2H), 6.88-6.82 (m, 3H), 3.96-3.75 (m, 6H), 3.68-3.62 (m, 5H), ESI-MS(m/z) : 633.0 [M+H]$^+$, |

**Example** 70

**Preparation of 2,4-difluoro-*N*-(2-methoxy-5-(4-(1-oxo-8-azaspiro[4.5]dec-2-en-8-yl)quinazolin-6-yl)pyridin-3-yl)benzenesulfonamide**

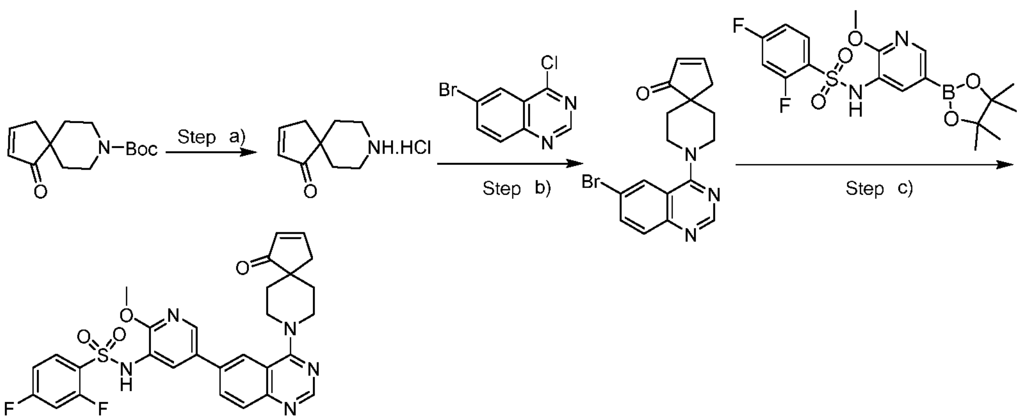


Step a): Preparation of 8-azaspiro[4.5]dec-2-en-1-one hydrochloride

Tert-butyl 1-oxo-8-azaspiro[4.5]dec-2-ene-8-carboxylate (260mg, 1.032 mmol) and tetrahydrofuran (2 mL) were added to the reaction flask, stirred to dissolve. At 0°C, under the condition of stirring, HCl/1,4-dioxane solution (4 M, 4mL) was slowly added to the system. After the addition was completed, the reaction mixture was naturally heated to room temperature and reacted for 1 h. After completion of the reaction, the reaction mixture was concentrated under reduced pressure. The residue was used directly in the next reaction without purification. ESI-MS (m/z): 152.2 [M+H]$^+$.

Step b-c): Preparation of 2,4-difluoro-*N*-(2-methoxy-5-(4-(1-oxo-8-azaspiro[4.5]dec-2-en-8-yl)quinazolin-6-yl)pyridin-3-yl)benzenesulfonamide

Referring to the preparation method of Example 42, Step a-b, 2,4-difluoro-*N*-(2-methoxy-5-(4-(1-oxo-8-azaspiro[4.5]dec-2-en-8-yl)quinazolin-6-yl)pyridin-3-yl)benzenesulfonamide was obtained, yield: 69.8 %; $^1$H NMR (400 MHz, DMSO-$d_6$) δ 10.32 (s, 1H), 8.64 (s, 1H), 8.38 (s, 1H), 8.08-8.04 (m, 2H), 7.95-7.88 (m, 3H), 7.80-7.75 (m, 1H), 7.56-7.51 (m, 1H), 7.23-7.18 (m, 1H), 6.23-6.20 (m, 1H), 4.36 (d, *J*= 12.0 Hz, 2H), 3.69 (s, 3H), 3.42 (d, *J*= 12.0 Hz, 2H), 2.78 (s, 2H), 1.89-1.81 (m, 2H), 1.43 (d, *J*= 12.0 Hz, 2H); ESI-MS (m/z): 578.6 $[M+H]^+$.

**Example 71**

**Preparation of 2,6-difluoro-*N*-(2-methoxy-5-(4-(1-oxo-8-azaspiro[4.5]dec-2-en-8-yl)quinazolin-6-yl)pyridin-3-yl)benzenesulfonamide**

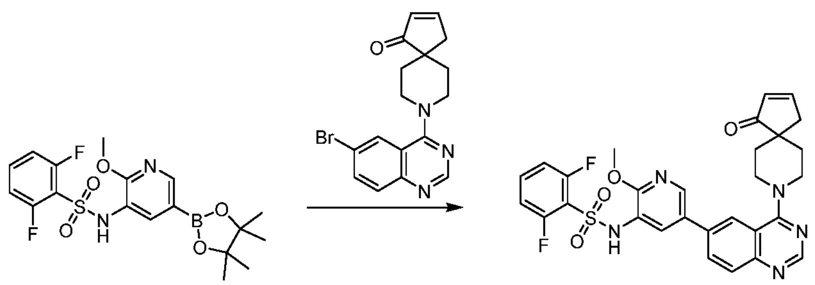

Referring to the preparation method of Example 42, Step b, 2,6-difluoro-*N*-(2-methoxy-5-(4-(1-oxo-8-azaspiro[4.5]dec-2-en-8-yl)quinazolin-6-yl)pyridin-3-yl)benzenesulfonamide was obtained, yield: 42.2%; $^1$H NMR (400 MHz, DMSO-$d_6$) δ 10.57 (s, 1H), 8.68 (s, 1H), 8.39 (s, 1H), 8.09-8.07 (m, 2H), 7.99-7.92 (m, 3H), 7.72-7.69 (m, 1H), 7.30-7.25 (m, 2H), 7.28 (t, *J* = 8.0 Hz, 1H), 6.26-6.24 (m, 1H), 4.41-4.38 (m, 2H), 3.70 (s, 3H), 3.50-3.44 (m, 2H), 2.82 (t, *J*= 4.0 Hz, 1H), 1.89-1.86 (m, 2H), 1.48-1.45 (m, 2H); ESI-MS(m/z): 578.1 $[M+H]^+$.

**Example 72**

**Preparation of 2,4-difluoro-*N*-(2-methoxy-5-(4-(4-(3-oxocyclohex-1-ene-1-carbonyl)piperazin-1-yl)quinazolin-6-yl)pyridin-3-yl)benzenesulfonamide**

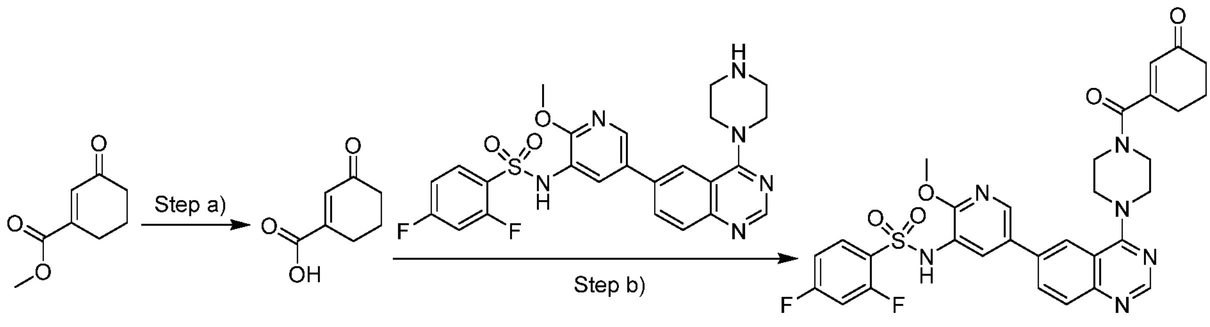


Step a): Preparation of 3-oxocyclohex-1-ene-1-carboxylic acid

Methyl 3-oxocyclohex-1-ene-1-carboxylate (300 mg, 1.948 mmol), sodium carbonate (309.7 mg, 2.922 mmol) were dissolved in tetrahydrofuran/methanol/water (4:1:1, 6 mL). The reaction mixture was stirred at room temperature and reacted overnight. Upon completion of the reaction, the reaction was quenched by adding water (50 mL). The reaction mixture was adjusted to pH = 3 ~ 4 with hydrochloric acid (2 M) and extracted with ethyl acetate (100 mL×2 times). The organic phases were combined, washed with saturated brine (100 mL), dried over anhydrous sodium sulfate

and filtered. The filtrate was concentrated under reduced pressure. 3-oxocyclohex-1-ene-1-carboxylic acid was obtained, yield: 55.1%.

Step b): Preparation of 2,4-difluoro-*N*-(2-methoay-5-(4-(4-(3-oxocyclohex-1-ene-1-carbonyl)piperazin-1-yl)quinazolin-6-yl)pyridin-3-yl)benzenesulfonamide

2.4-difluoro-*N*-(2-methoxy-5-(4-(piperazin-1-yl)quinazolin-6-yl)pyridin-3-yl)benzenesulfonamide (200.0 mg, 0.390 mmol) and HATU (222.2 mg, 0.585 mmol) were dissolved in dichloromethane (10 mL). The reaction system was cooled to -78°C, with *N,N*-diisopropylethylamine (253.4 mg, 0.975 mmol), 3-oxocyclohex-1-ene-1-carboxylic acid (81.9 mg, 1.950 mmol) added successively. After the addition was completed, the reaction mixture was kept at -78°C and stirred for 1 h. Upon completion of the reaction, the reaction was quenched by adding water (5 mL). The reaction mixture was extracted by adding dichloromethane (10 mL×2 times). The organic phases were combined, dried over anhydrous sodium sulfate and filtered. The filtrate was concentrated under reduced pressure. The crude product obtained was purified by Prep-HPLC (Method 2). 2,4-difluoro-*N*-(2-methoxy-5-(4-(4-(3-oxocyclohex-1-ene-1-carbonyl)piperazin-1-yl)quinazolin-6-yl)pyridin-3-yl)benzenesulfonamide was obtained, yield: 15.0%; $^1$H NMR (400 MHz, DMSO-$d_6$) δ 10.32 (s, 1H), 8.67 (s, 1H), 8.45 (s, 1H), 8.09-8.07 (m, 2H), 8.00 (s, 1H), 7.92 (d, *J* = 8.0 Hz, 1H), 7.79-7.76 (m, 1H), 7.55-7.54 (m, 1H), 7.21-7.18 (m, 1H), 5.93 (s, 1H), 3.86-3.68 (m, 11H), 2.53-2.52 (m, 2H), 2.41-2.38 (m, 2H), 2.04-2.01 (m, 2H); ESI-MS(m/z): 635.0 $[M+H]^+$.

**Example 73**

**Preparation of 5-chloro-*N*-(2-methoxy-5-(4-(4-(3-oxocyclohexane-1-en-1-carbonyl)piperazin-1-yl)quinazoline-6-yl)pyridin-3-yl)thiophene-2-sulfonamide**

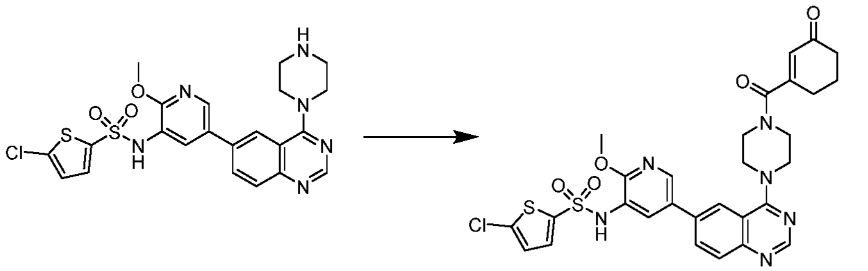

Referring to the preparation method of Example 72, 5-chloro-*N*-(2-methoxy-5-(4-(4-(3-oxocyclohexane-1-en-1-carbonyl)piperazin-1-yl)quinazoline-6-yl)pyridin-3-yl)thiophene-2-sulfonamide was obtained, yield: 16.6%; $^1$H NMR (400 MHz, DMSO-$d_6$) δ 10.41 (s, 1H), 8.67 (s, 1H), 8.48 (d, *J* = 4.0 Hz, 1H), 8.11-8.09 (m, 2H), 8.01 *(d, J*= 4.0 Hz, 1H), 7.94-7.91 (m, 1H), 7.39 (d, J = 4.0 Hz, 1H), 7.21 (d, *J*= 4.0 Hz, 1H), 5.94 (s, 1H), 3.90-3.84 (m, 4H), 3.78 (s, 3H), 3.71-3.68 (m, 4H), 3.28-3.25 (m, 2H), 2.49-2.38 (m, 2H), 2.06-1.99 (m, 2H); ESI-MS(m/z): 639.0 $[M+H]^+$.

**Example 74**

**Preparation of 2,4-difluoro-*N*-(2-methoxy-5-(4-(4-(3-oxocyclopent-1-ene-1-carbonyl)piperazin-1-yl)quinazolin-6-yl)pyridin-3-yl)benzenesulfonamide**

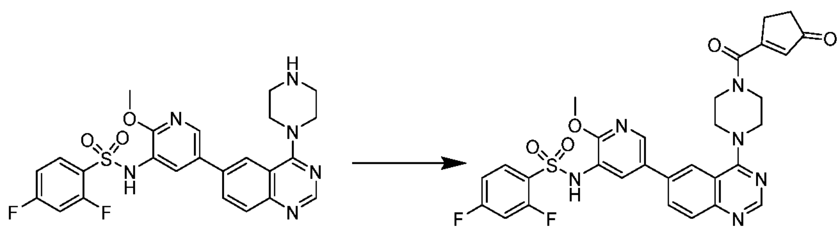

Referring to the preparation method of Example 72, 2,4-difluoro-*N*-(2-methoxy-5-(4-(4-(3-oxocyclopent-1-ene-1-carbonyl)piperazin-1-yl)quinazolin-6-yl)pyridin-3-yl)benzenesulfonamide was obtained, yield: 10.6%; $^1$H NMR (400 MHz, DMSO-$d_6$) δ 10.36 (s, 1H), 8.85 (s, 1H), 8.51 (s, 1H), 8.27-8.26 (m, 2H), 8.05 (s, 1H), 7.93 (d, *J*= 8.0 Hz, 1H), 7.77-7.74 (m, 1H), 7.60-7.55 (m, 1H), 7.24-7.20 (m, 1H), 6.31 (s, 1H), 4.24-4.10 (m, 3H), 3.77 (s, 2H), 3.74 (s, 2H), 3.67 (s, 3H), 3.48-3.46 (m, 1H), 2.86-2.84 (m, 2H), 2.45-2.42 (m, 2H); ESI-MS(m/z): 621.0 $[M+H]^+$.

**Example 75**

**Preparation of (*E*)-2,4-difluoro-*N*-(2-methoxy-5-(4-(4-(3-(methylsulfonyl)acryloyl)piperazin-1-yl)quinazolin-6-yl)pyridin-3-yl)benzenesulfonamide**

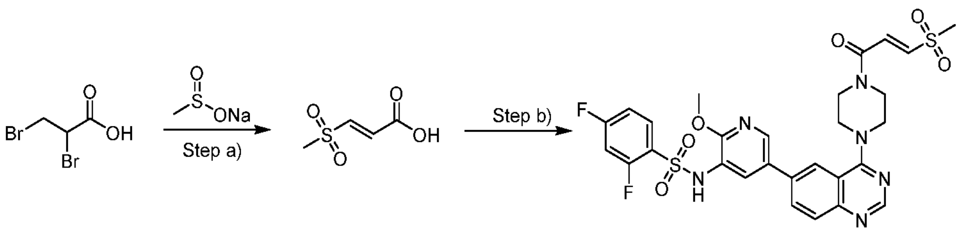


Step a): Preparation of (*E*)-3-(methylsulfonyl)acrylic acid

2,3-dibromopropanoic acid (500 mg, 2.155 mmol) and sodium methanesulfinate (263.8 mg, 2.586 mmol) were dissolved in *N,N*-dimethylformamide/water (4: 1, ,10 mL). At 80°C, the reaction mixture was stirred and reacted overnight. Upon completion of the reaction, the reaction was quenched by adding water (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. (*E*)-3-(methylsulfonyl)acrylic acid was obtained, yield: 61.9%. The product can be used directly in the next reaction without further purification.

Step b): Preparation of (*E*)-2,4-difluoro-*N*-(2-methoxy-5-(4-(4-(3-(methylsulfonyl)acryloyl)piperazin-1-yl)quinazolin-6-yl)pyridin-3-yl)benzenesulfonamide

Referring to the preparation method of Example 72, Step b, (*E*)-2,4-difluoro-*N*-(2-methoxy-5-(4-(4-(3-(methylsulfonyl)acryloyl)piperazin-1-yl)quinazolin-6-yl)pyridin-3-yl)benzenesulfonamide was obtained, yield: 10.7%; $^1$H NMR (400 MHz, DMSO-$d_6$) δ 8.63 (s, 1H), 7.92-7.78 (m, 5H), 7.56 (s, 1H), 7.55-7.37 (m, 2H), 7.20-7.18 (m, 1H), 7.09-7.05 (m, 1H), 3.85-3.79 (m, 11H), 3.17 (s, 3H); ESI-MS(m/z): 645.0 $[M+H]^+$.

**Example 76**

**Preparation of (*E*)-N-(5-(4-(4-(3-(dimethylphosphoryl)acryloyl)piperazin-1-yl)quinazolin-6-yl)-2-methoxypyridin-3-yl)-2,4-difluorobenzenesulfonamide**

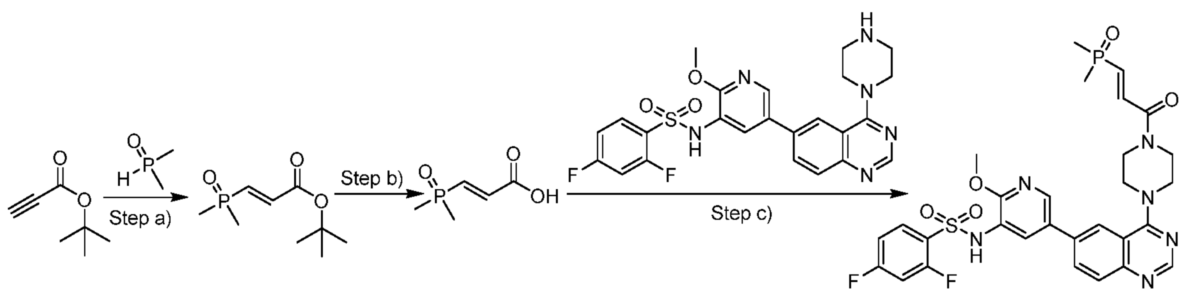

Step a): Preparation of tert-butyl (*E*)-3-(dimethylphosphoryl)acrylate

Tert-butyl propiolate (1.0 g, 9.730 mmol) was dissolved in tetrahydrofuran (10 mL), with dimethylphosphine oxide (620 mg, 9.730 mmol) added. After the addition was completed, the reaction mixture was heated to 50°C and stirred for 12 h. Upon completion of the reaction, the reaction mixture was concentrated under reduced pressure. The crude product obtained was purified by silica gel column chromatography (Eluent: petroleum ether/ethyl acetate = 20/1 to 10/1). Tert-butyl (*E*)-3-(dimethylphosphoryl)acrylate was obtained, yield: 58.7%.

Step b): Preparation of (*E*)-3-(dimethylphosphoryl)acrylic acid

Tert-butyl (*E*)-3-(dimethylphosphoryl)acrylate (50 mg, 0.240 mmol)was dissolved in dichloromethane (3 mL). After the addition was completed, the reaction mixture was cooled to 0°C, with TFA (1 mL) added slowly. After the addition was completed, the reaction mixture was kept at current temperature and stirred for 30min. Upon completion of the reaction, the reaction mixture was concentrated under reduced pressure. (E)-3-(dimethylphosphoryl)acrylic acid was obtained. ESI-MS(m/z): 147 $[M-H]^+$.

Step c): Preparation of (*E*)-N-(5-(4-(4-(3-(dimethylphosphoryl)acryloyl)piperazin-1-yl)quinazolin-6-yl)-2-methoxypyridin-3-yl)-2,4-difluorobenzenesulfonamide

2,4-difluoro-*N*-(2-methoxy-5-(4-(piperazin-1-yl)quinazolin-6-yl)pyridin-3-yl)benzenesulfonamide trifluoroacetate (60 mg, 0.098 mmol) and tetrahydrofuran (2 mL) were added to the reaction flask. The reaction system was cooled to -78°C, with *N,N*-diisopropylethylamine (76 mg, 0.590 mmol), (*E*)-3-(dimethylphosphoryl)acrylic acid (15 mg, 0.098 mmol) and 50% $T_3P$ ethyl acetate solution (62 mg, 0.098 mmol) added successively. After the addition was completed, the reaction mixture was kept at -78°C and stirred for 1 h. Upon completion of the reaction, the reaction was quenched by adding acetonitrile (1 mL), the reaction solution was purified by Prep-HPLC (Method 2). (*E*)-*N*-(5-(4-(4-(3-(dimethylphosphoryl)acryloyl)piperazin-1-yl)quinazolin-6-yl)-2-methoxypyridin-3-yl)-2,4-difluorobenzenesulfonamide was obtained, yield: 21.9%: $^1$H NMR (400 MHz, DMSO-$d_6$) δ 10.27 (s, 1H), 8.72 (s, 1H), 8.50 (d, *J* =4.0 Hz, 1H), 8.19-8.14 (m, 2H), 8.03 (s, 1H), 7.92 (d, *J* =8.0 Hz, 1H), 7.79-7.74 (m, 1H), 7.61-7.55 (m, 1H), 7.24-7.03 (m, 3H), 4.01 (m, 4H), 3.95-3.80 (m, 2H), 3.82-3.80 (m, 2H), 3.69 (s, 3H), 1.55 (s, 3H), 1.51 (s, 3H); ESI-MS(m/z): 643.0 $[M+H]^+$.

**Example 77**

**Preparation of (*Z*)-2,4-difluoro-*N*-(5-(4-(4-(2-fluoro-4-oxopent-2-enoyl)piperazin-1-yl)quinazolin-6-yl)-2-methoxypyridin-3-yl)benzenesulfonamide**

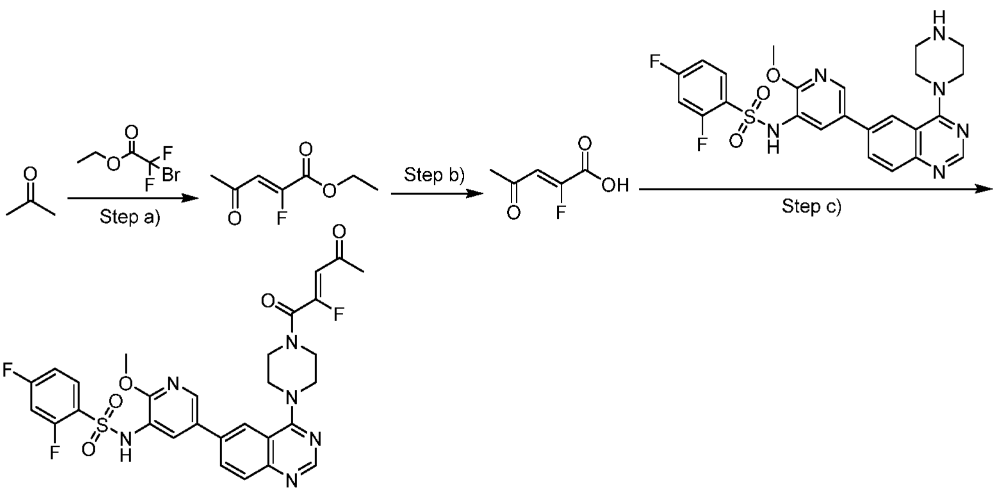


Step a): Preparation of ethyl (Z)-2-fluoro-4-oxopent-2-enoate

Acetone (1.0 g, 17.218 mmol), cuprous iodide (328 mg, 1.722 mmol) and acetonitrile (20 mL) were added to the reaction flask. Under the ice bath condition, pentamethyldiethylenetriamine (4.48 g, 25.827 mmol), trimethylsilyl

iodide (5.17 g, 25.827 mmol) and ethyl difluorobromoacetate (8.74 g, 43.045 mmol) were then added successively. After the addition was completed, the reaction mixture was kept at room temperature and reacted for 10 min, then heated to 60°C and reacted overnight. Upon completion of the reaction, under the ice bath condition, the reaction was quenched by adding water (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: ethyl acetate/petroleum ether = 1/100 to 1/20). Ethyl (*Z*)-2-fluoro-4-oxopent-2-enoate was obtained, yield: 34.2%; $^1$H NMR (400 MHz, $CDCl_3$) δ 6.40 (d, *J* = 34.0 Hz, 1H), 4.36 (q, *J* = 7.2 Hz, 2H), 2.46 (d, *J* = 2.8 Hz, 3H), 1.37 (t, *J* = 7.2 Hz, 3H).

Step b): Preparation of (*Z*)-2-fluoro-4-oxopent-2-enoic acid

Ethyl (*Z*)-2-fluoro-4-oxopent-2-enoate (150 mg, 0.937 mmol) was added to tetrahydrofuran (2 mL), with sodium bicarbonate solution (2 mL, 8.4% w/w) added. After the addition was completed, the reaction mixture was heated to 70°C and reacted for 2 h. Upon completion of the reaction, the reaction mixture was adjusted to pH = 1 with dilute hydrochloric acid (2M) under the ice bath condition, extracted with ethyl acetate (50 mL×2 times). The organic phases were combined, washed with saturated brine (50 mL), dried over anhydrous sodium sulfate and filtered. The filtrate was concentrated under reduced pressure. (*Z*)-2-fluoro-4-oxopent-2-enoic acid was obtained, yield: 58.8%; ESI-MS(m/z): 131.0 $[M-H]^-$.

Step c): Preparation of (*Z*)-2,4-difluoro-*N*-(5-(4-(4-(2-fluoro-4-oxopent-2-enoyl)piperazin-1-yl)quinazolin-6-yl)-2-methoxypyridin-3-yl)benzenesulfonamide

Referring to the preparation method of Example 72, Step b, (Z)-2,4-difluoro-N-(5-(4-(4-(2-fluoro-4-oxopent-2-enoyl)piperazin-1-yl)quinazolin-6-yl)-2-methoxypyridin-3-yl)benzenesulfonamide was obtained, yield: 20.1%; $^1$H NMR (400 MHz, DMSO-$d_6$) δ 10.36 (s, 1H), 8.68 (s, 1H), 8.49 (d, *J* = 4.0 Hz, 1H), 8.16-8.08 (m, 2H), 8.03 (d, *J* = 4.0 Hz, 1H), 7.93 (d, *J* = 8.0 Hz, 1H), 7.83-7.72 (m, 1H), 7.63-7.53 (m, 1H), 7.27-7.17 (m, 1H), 6.12 (d, *J* = 40 0Hz, 1H), 3.95-3.79 (m, 8H), 3.68 (s, 3H), 2.37 (d, *J* = 4.0 Hz, 3H); ESI-MS(m/z): 627.0 $[M+H]^+$.

**Example 78**

**Preparation of (*E*)-2,4-difluoro-*N*-(2-methoxy-5-(4-(4-(4-oxopent-2-enoyl)piperazin-1-yl)quinazolin-6-yl)pyridin-3-yl)-*N*-methylbenzenesulfonamide**

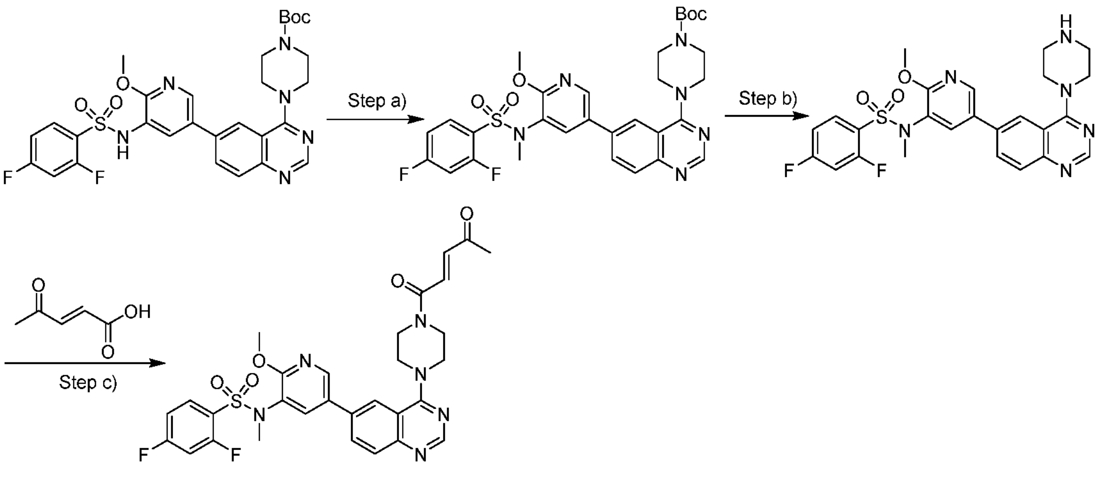


Step a): Preparation of tert-butyl 4-(6-(5-((2,4-difluoro-N-methylphenyl)sulfonamido)-6-methoaypyridin-3-yl)quinazolin-4-yl)piperazine-1-carboxylate

Tert-butyl 4-(6-(5-((2,4-difluorophenyl)sulfonamido)-6-methoxypyridin-3-yl)quinazolin-4-yl)piperazine-1-carboxylate (500 mg, 0.817 mmol), methyl iodide (580 mg, 4.085 mmol), cesium carbonate (1.34g, 4.085 mmol) and *N,N*-dimethylformamide (10 mL) were added to the reaction flask. After the addition was completed, the reaction mixture was stirred at room temperature and reacted for 2 h. Upon completion of the reaction, the reaction was quenched by

adding water (50 mL). The reaction mixture was extracted with ethyl acetate (200 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 50/1). Tert-butyl 4-(6-(5-((2,4-difluoro-*N*-methylphenyl)sulfonamido)-6-methoxypyridin-3-yl)quinazolin-4-yl)piperazine-1-carboxylate was obtained, yield: 45.2%; ESI-MS(m/z): 627.2 $[M+H]^+$.

Step b): Preparation of 2,4-difluoro-*N*-(2-methoxy-5-(4-(piperazin-1-yl)quinazolin-6-yl)pyridin-3-yl)-*N*-methylbenzenesulfonamide trifluoroacetate

Tert-butyl 4-(6-(5-((2,4-difluoro-*N*-methylphenyl)sulfonamido)-6-methoxypyridin-3-yl)quinazolin-4-yl)piperazine-1-carboxylate (200 mg, 0.319 mmol) was dissolved in dichloromethane (3 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2,4-difluoro-*N*-(2-methoxy-5-(4-(piperazin-1-yl)quinazolin-6-yl)pyridin-3-yl)-*N*-methylbenzenesulfonamide trifluoroacetate was obtained, yield: 95.6%; ESI-MS(m/z): 513.1 $[M+H]^+$.

Step c): Preparation of (*E*)-2,4-difluoro-*N*-(2-methoxy-5-(4-(4-(4-oxopent-2-enoyl)piperazin-1-yl)quinazolin-6-yl)pyridin-3-yl)-*N*-methylbenzenesulfonamide

2,4-difluoro-*N*-(2-methoxy-5-(4-(piperazin-1-yl)quinazolin-6-yl)pyridin-3-yl)-*N*-methylbenzenesulfonamide (85 mg, 0.133 mmol) was dissolved in tetrahydrofuran (2 mL). The reaction system was cooled to -78°C, with *N,N*-diisopropylethylamine (86 mg, 0.665 mmol), (E)-4-oxopent-2-enoic acid (15 mg, 0.133mmol) and 50% $T_3P$ ethyl acetate solution (169 mg, 0.266 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). (E)-2,4-difluoro-N-(2-methoxy-5-(4-(4-(4-oxopent-2-enoyl)piperazin-1-yl)quinazolin-6-yl)pyridin-3-yl)-*N*-methylbenzenesulfonamide was obtained, yield: 15.2%; $^1$H NMR (400 MHz, DMSO-$d_6$ + $D_2O$) δ 8.65 (s, 1H), 8.63 (d, *J* = 4.0 Hz, 1H), 8.18-8.13 (m, 3H), 7.94 (d, *J*= 12.0 Hz, 1H), 7.79-7.73 (m, 1H), 7.61-7.56 (m, 1H), 7.43 (d, *J*= 16.0 Hz, 1H), 7.28-7.24 (m, 1H), 6.74 (d, *J*= 16.0 Hz, 1H), 3.96-3.80 (m, 8H), 3.65 (s, 3H), 3.29 (s, 3H), 2.37 (s, 3H); ESI-MS(m/z): 623.0 $[M+H]^+$.

## Example 79

**Preparation of 2,4-difluoro-*N*-(5-(4-(4-(2-fluoroacryloyl)piperazin-1-yl)quinazolin-6-yl)-2-methoxypyridin-3-yl)-*N*-methylbenzenesulfonamide**

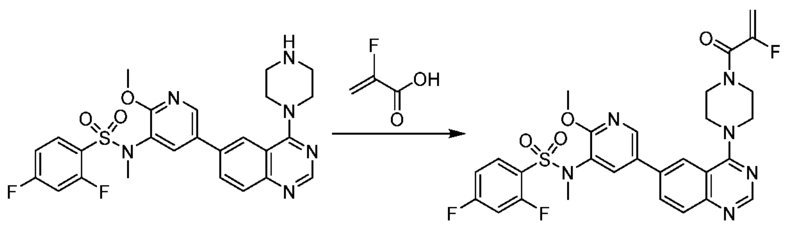

Referring to the preparation method of Example 78, Step c, 2,4-difluoro-*N*-(5-(4-(4-(2-fluoroacryloyl)piperazin-1-yl)quinazolin-6-yl)-2-methoxypyridin-3-yl)-*N*-methylbenzenesulfonamide was obtained, yield: 14.3%; $^1$H NMR (400 MHz, DMSO-$d_6$ + $D_2O$) δ 8.66-8.63 (m, 2H), 8.18-8.13 (m, 3H), 7.93 (d, *J*= 8.0 Hz, 1H), 7.79-7.73 (m, 1H), 7.61-7.56 (m, 1H), 7.28-7.24 (m, 1H), 5.38-5.20 (m, 2H), 3.96-3.81 (m, 8H), 3.65 (s, 3H), 3.29 (s, 3H), 2.37 (s, 3H); ESI-MS(m/z): 623.0 $[M+H]^+$.

**Example 80**

**Preparation of (*E*)-2,4-difluoro-N-(2-methoxy-5-(4-(4-(4-oxohept-2-enoyl)piperazin-1-yl)quinazolin-6-yl)pyridin-3-yl)-*N*-methylbenzenesulfonamide**

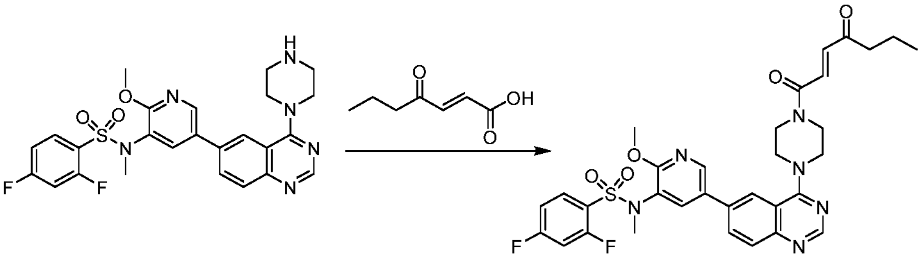

Referring to the preparation method of Example 78, Step c, (*E*)-2,4-difluoro-*N*-(2-methoxy-5-(4-(4-(4-oxohept-2-enoyl)piperazin-1-yl)quinazolin-6-yl)pyridin-3-yl)-*N*-methylbenzenesulfonamide was obtained, yield: 18.8%; $^1$H NMR (400 MHz, DMSO-$d_6$) δ 8.66 (s, 1H), 8.64 (d, *J*= 8.0 Hz, 1H), 8.18-8.13 (m, 3H), 7.92 (d, *J*= 8.0 Hz, 1H), 7.78-7.72 (m, 1H), 7.67-7.61 (m, 1H), 7.43 (d, *J* = 16.0 Hz, 1H), 7.28-7.23 (m, 1H), 6.82 (d, *J* = 16.0 Hz, 1H), 3.94-3.79 (m, 8H), 3.79 (s, 3H), 3.32-3.29 (m, 3H), 2.73 (t, *J*= 8.0 Hz, 2H), 1.60-1.51 (m, 2H), 0.89.(t, *J*= 12.0 Hz, 3H); ESI-MS(m/z): 651.0 [M+H]$^+$.

**Example 81**

**Preparation of (*E*)-2,4-difluoro-*N*-(2-methoxy-5-(4-(2-(4-oxopent-2-enoyl)-2,6-diazaspiro[3.4]octan-6-yl)quinazolin-6-yl)pyridin-3-yl)-*N*-methylbenzenesulfonamide**

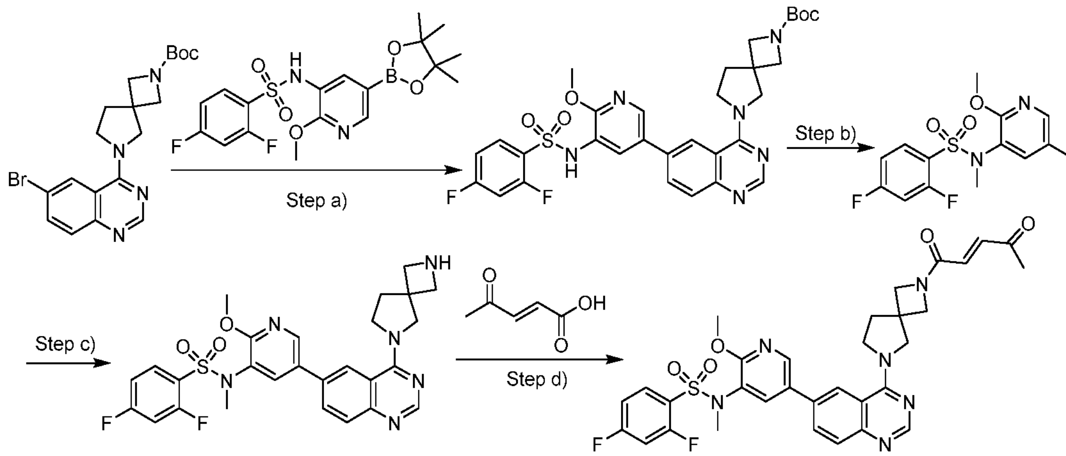


Step a): Preparation of tert-butyl 6-(6-(5-((2,4-difluorophenyl)sulfonamido)-6-methoxypyridin-3-yl)quinazolin-4-yl)-2,6-diazaspiro[3.4]octane-2-carboxylate

Tert-butyl 6-(6-bromoquinazolin-4-yl)-2,6-diazaspiro[3.4]octane-2-carboxylate (200 mg, 0.478 mmol), 2,4-difluoro-*N*-(2-hydroxy-5-(4,4,5,5-tetramethyl-1,3,2-dioxaborolan-2-yl)pyridin-3-yl)benzenesulfonamide (204 mg, 0.478 mmol), Pd(dppf)$Cl_2$ (70 mg, 0.096 mmol) and cesium carbonate (311 mg, 0.956 mmol) were added to dioxane/water mixed solvent (v/v = 10:1, 4.4 mL) successively. After the addition was completed, in the presense of protective nitrogen, the reaction mixture was kept at 100°C and stirred for 3h. Upon completion of the reaction, the reaction mixture was filtered. The filtrate was concentrated under reduced pressure. The crude product obtained was purified by silica gel column chromatography (Eluent: petroleum ether/ethyl acetate = 10/1 to 0/1). Tert-butyl 6-(6-(5-((2,4-difluorophenyl)sulfonamido)-6-methoxypyridin-3-yl)quinazolin-4-yl)-2,6-diazaspiro[3.4]octane-2-carboxylate was obtained, yield: 65.6%; ESI-MS(m/z): 639.2 [M+H]$^+$.

Step b-d): Preparation of (*E*)-2,4-difluoro-*N*-(2-methoxy-5-(4-(2-(4-oxopent-2-enoyl)-2,6-diazaspiro[3.4]octan-6-yl)quinazolin-6-yl)pyridin-3-yl)-*N*-methylbenzenesulfonamide

Referring to the preparation method of Example 78, Step a-c, (*E*)-2,4-difluoro-*N*-(2-methoxy-5-(4-(2-(4-oxopent-2-enoyl)-2,6-diazaspiro[3.4]octan-6-yl)quinazolin-6-yl)pyridin-3-yl)-N-methylbenzenesulfonamide was obtained, yield: 48.4%; $^{1}$H NMR (400 MHz, DMSO-$d_6$) $\delta$ 8.65 (d, *J* =4.0 Hz, 1H), 8.49 (s, 1H), 8.41 (d, *J* =4.0 Hz, 1H), 8.15 (d, *J* =4.0 Hz, 1H), 8.06 (t, *J* =4.0 Hz, 1H), 7.81(d, *J* =8.0 Hz, 1H), 7.77-7.71 (m, 1H), 7.66-7.61 (m, 1H), 7.28-7.23 (m, 1H), 6.89 (d, *J* =12.0 Hz, 1H), 6.73 (d, *J* =16.0 Hz, 1H), 4.41 (d, *J* =8.0 Hz, 1H), 4.32 (d, *J* =12.0 Hz, 1H), 4.22 (m, 2H), 4.10-3.97 (m, 4H), 3.63 (s, 3H), 3.29 (s, 3H), 2.32-2.24 (m, 5H); ESI-MS(m/z): 649.2 [M+H]$^+$.

**Example 82**

**Preparation of *N*-(5-(4-(4-acryloylpiperazin-1-yl)quinazolin-6-yl)-2-methoxypyridin-3-yl)-2,6-difluoro-*N*-methylbenzenesulfonamide**

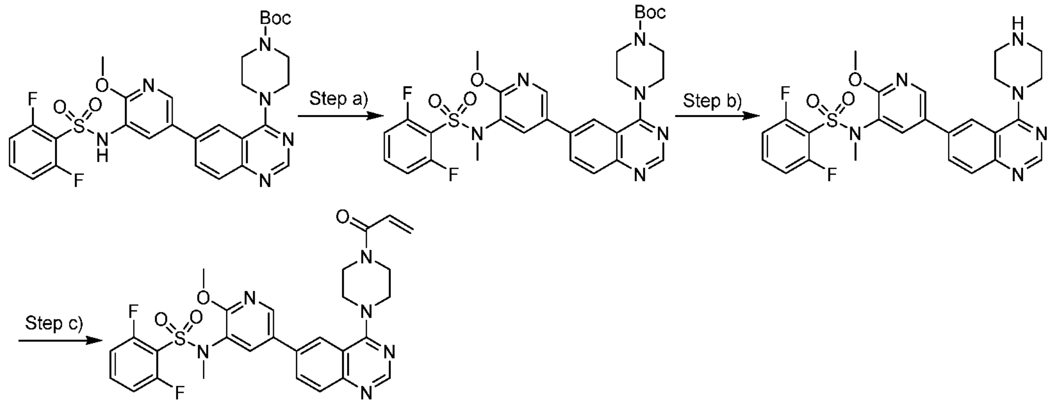


Step a): Preparation of tert-butyl 4-(6-(5-((2,6-difluoro-*N*-methylphenyl)sulfonamido)-6-methoxypyridin-3-yl)quinazolin-4-yl)piperazine-1-carboxylate

Tert-butyl 4-(6-(5-((2,6-difluorophenyl)sulfonamido)-6-methoxypyridin-3-yl)quinazolin-4-yl)piperazine-1-carboxylate (240 mg, 0.392 mmol) and cesium carbonate (255 mg, 0.784 mmol) were dissolved in tert-butyl 6-(6-(5-((2,4-difluorophenyl)sulfonamido)-6-methoaypyridin-3-yl)quinazolin-4-yl)-2,6-diazaspiro [3.4] octane-2-carboxylate (8 mL), with methyl iodide (111 mg, 0.784 mmol) added. After the addition was completed, the reaction mixture was stirred at room temperature for 2 h. Upon completion of the reaction, the reaction was quenched by adding water (5 mL). The reaction mixture was extracted with ethyl acetate (100 mL×2 times). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 2/1 to 1/2). Tert-butyl 4-(6-(5-((2,6-difluoro-N-methylphenyl)sulfonamido)-6-methoxypyridin-3-yl)quinazolin-4-yl)piperazine-1-carboxylate was obtained, yield: 61.2%; ESI-MS(m/z): 627.1 [M+H]$^+$.

Step b): Preparation of 2,6-difluoro-*N*-(2-methoxy-5-(4-(piperazin-1-yl)quinazolin-6-yl)pyridin-3-yl)-*N*-methylbenzenesulfonamide trifluoroacetate

Tert-butyl 4-(6-(5-((2,6-difluoro-*N*-methylphenyl)sulfonamido)-6-methoxypyridin-3-yl)quinazolin-4-yl)piperazine-1-carboxylate (150 mg, 0.239 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. 2,6-difluoro-*N*-(2-methoxy-5-(4-(piperazin-1-yl)quinazolin-6-yl)pyridin-3-yl)-*N*-methylbenzenesulfonamide trifluoroacetate was obtained; ESI-MS(m/z): 527.2 [M+H]$^+$.

Step c): Preparation of N-(5-(4-(4-acryloylpiperazin-1-yl)quinazolin-6-yl)-2-methoxypyridin-3-yl)-2,6-difluoro-N-methylbenzenesulfonamide

2,6-difluoro-*N*-(2-methoxy-5-(4-(piperazin-1-yl)quinazolin-6-yl)pyridin-3-yl)-*N*-methylbenzenesulfonamide (120 mg, 0.187 mmol) was dissolved in dichloromethane (5 mL). The reaction system was cooled to -78°C, with *N,N*-diisopropylethylamine (148 mg, 1.140 mmol), acrylic acid (25 mg, 0.342 mmol) and HATU (130 mg, 0.342 mmol) added successively. After the addition was completed, the reaction mixture was kept at -78°C and stirred for 1 h. Upon completion of the reaction, the reaction was quenched by adding water (5 mL). The reaction mixture was extracted by adding dichloromethane (100 mL×2 times). The organic phases were combined, dried over anhydrous sodium sulfate and filtered. The filtrate was concentrated under reduced pressure. The crude product obtained was purified by Prep-HPLC (Method 2). *N*-(5-(4-(4-acryloylpiperazin-1-yl)quinazolin-6-yl)-2-methoxypyridin-3-yl)-2,6-difluoro-*N*-methylbenzenesulfonamide was obtained, yield: 24.9%; $^1$H NMR (400 MHz, DMSO-$d_6$) δ 8.65 (s, 2H), 8.20-8.15 (m, 3H), 7.93-7.90 (m, 1H), 7.80-7.75 (m, 1H), 7.35-7.30 (m, 2H), 6.84-6.80 (m, 1H), 6.20-6.15 (m, 1H), 5.74 (d, *J* =8.0 Hz, 1H), 3.90-3.75 (m, 8H), 3.61 (s, 3H), 3.34 (s, 3H); ESI-MS(m/z): 581.0 $[M+H]^+$.

**Example 83**

**Preparation of (*E*)-*N*-(2-ethyl-5-(4-(4-(4-oxopent-2-enoyl)piperazin-1-yl)quinazolin-6-yl)pyridin-3-yl)-2,4-difluorobenzenesulfonamide**

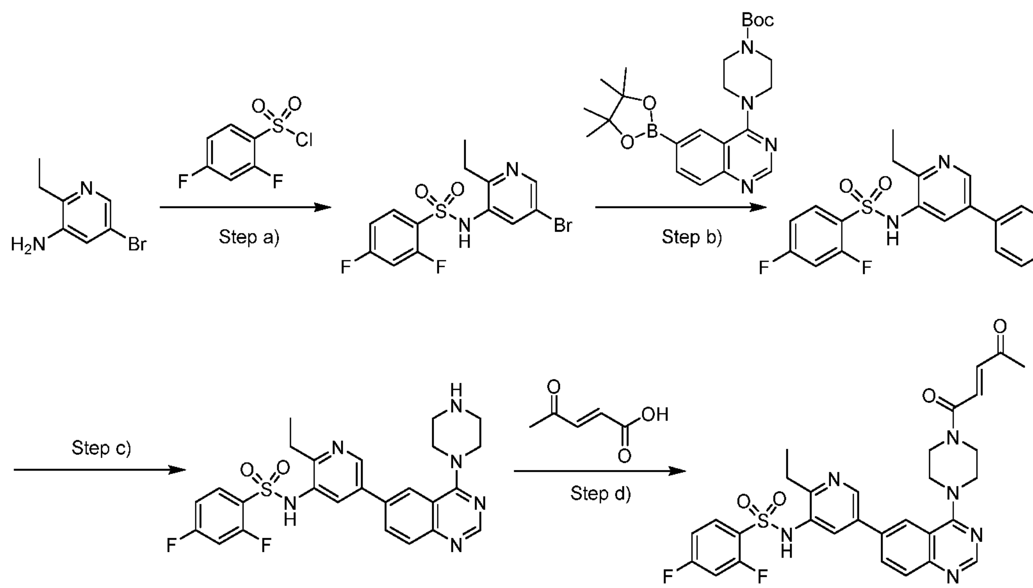


Step a): Preparation of *N*-(5-bromo-2-ethylpyridin-3-yl)-2,4-difluorobenzenesulfonamide

5-Bromo-2-ethylpyridin-3-amine (550 mg, 2.736 mmol) and 2,4-difluorobenzenesulfonyl chloride (582 mg, 2.737 mmol) were dissolved in dichloromethane (6 mL), with 4-dimethylaminopyridine (33 mg, 0.270 mmol) and pyridine (433 mg, 5.474 mmol) added successively. After the addition was completed, the reaction mixture was kept at room temperature and stirred for 1 h. Upon completion of the reaction, the reaction was quenched by adding water (50 mL), and the reaction mixture was extracted with dichloromethane (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 5/1 to 2/1). *N*-(5-bromo-2-ethylpyridin-3-yl)-2,4-difluorobenzenesulfonamide was obtained, yield: 92.1%; ESI-MS(m/z): 377.0 $[M+H]^+$.

Step b): Preparation of Tert-butyl 4-(6-(5-((2,4-difluorophenyl)sulfonamido)-6-ethylpyridin-3-yl)quinazolin-4-yl)piperazine-1-carboxylate

*N*-(5-bromo-2-ethylpyridin-3-yl)-2,4-difluorobenzenesulfonamide (550 mg, 1.458 mmol), tert-butyl 4-(6-(4,4,5,5-tetramethyl-1,3,2-dioxaborolan-2-yl)quinazolin-4-yl)piperazine-1-carboxylate (706 mg, 1.603 mmol), Pd(dppf)$Cl_2$ (107

mg, 0.146 mmol) and cesium carbonate (1.4 g, 4.297 mmol) were added to dioxane/water mixed solvent (6 mL, v/v = 5:1). After the addition was completed, in the presense of protective nitrogen, the reaction mixture was kept at 100°C and stirred for 1 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 1/1 to 1/3). Tert-butyl 4-(6-(5-((2,4-difluorophenyl)sulfonamido)-6-ethylpyridin-3-yl)quinazolin-4-yl)piperazine-1-carboxylate was obtained, yield: 78.7%. ESI-MS(m/z): 611.2 $[M+H]^+$.

Step c): Preparation of *N*-(2-ethyl-5-(4-(piperazin-1-yl)quinazolin-6-yl)pyridin-3-yl)-2,4-difluorobenzenesulfonamide trifluoroacetate

Tert-butyl 4-(6-(5-((2,4-difluorophenyl)sulfonamido)-6-ethylpyridin-3-yl)quinazolin-4-yl)piperazine-1-carboxylate (150 mg, 0.246 mmol) was dissolved in dichloromethane (3 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. Methyl tert-butyl ether was added to the crude product. The mixture was stirred to precipitate solid and filtered. *N*-(2-ethyl-5-(4-(piperazin-1-yl)quinazolin-6-yl)pyridin-3-yl)-2,4-difluorobenzenesulfonamide trifluoroacetate was obtained, yield: 93.8%; ESI-MS(m/z): 511.2 $[M+H]^+$.

Step d): Preparation of (*E*)-*N*-(2-ethyl-5-(4-(4-(4-oxopent-2-enoyl)piperazin-1-yl)quinazolin-6-yl)pyridin-3-yl)-2,4-difluorobenzenesulfonamide

3-Acetylacrylic acid (44 mg, 0.386 mmol) and *N*-(2-ethyl-5-(4-(piperazin-1-yl)quinazolin-6-yl)pyridin-3-yl)-2,4-difluorobenzenesulfonamide trifluoroacetate (120 mg, 0.192 mmol) were dissolved in THF (2 mL). The reaction system was cooled to -78°C, with *N,N*-diisopropylethylamine (124 mg, 0.959 mmol) and 1-propylphosphonic anhydride (367 mg, 0.576 mmol, 50%wt) added successively. After the addition was completed, the reaction mixture was kept at -78°C and stirred for 1 h. Upon completion of the reaction, the reaction was quenched by adding water (10 mL), and the reaction mixture was extracted with dichloromethane (30 mL×2). The organic phases were combined, dried over anhydrous sodium sulfate and filtered. The filtrate was concentrated under reduced pressure. The crude product obtained was purified by Prep-HPLC (Method 2). (*E*)-*N*-(2-ethyl-5-(4-(4-(4-oxopent-2-enoyl)piperazin-1-yl)quinazolin-6-yl)pyridin-3-yl)-2,4-difluorobenzenesulfonamide was obtained, yield: 30.4%; $^1$H NMR (400 MHz, DMSO-$d_6$) δ 10.54 (s, 1H), 8.80 (s, 1H), 8.67 (s, 1H), 8.14 (s, 1H), 8.06-8.01 (m, 1H), 7.95-7.91 (m, 1H), 7.85-7.77 (m, 2H), 7.60-7.53 (m, 1H), 7.44 *(d, J*= 16.0 Hz, 1H), 7.27-7.20 (m, 1H), 6.74 *(d, J*= 16.0 Hz, 1H), 3.93-3.87 (m, 6H), 3.82-3.77 (m, 2H), 2.72-2.65 (m, 2H), 2.36 (s, 3H), 1.08-1.02 (m, 3H); ESI-MS(m/z): 607.0 $[M+H]^+$.

Referring to the preparation method of Example 83 and using the corresponding raw materials, the compounds in the following examples were prepared.

| Number | Name | Structure | $^1$H NMR and MS |
|---|---|---|---|
| **Example 84** | (*E*)-*N*-(2-chloro-5-(4-(4-(4-oxopent-2-enoyl)piperazin-1 - yl) quinazolin-6-yl)pyridin-3-yl)-2,6-difluorobenzenesulfonamide | 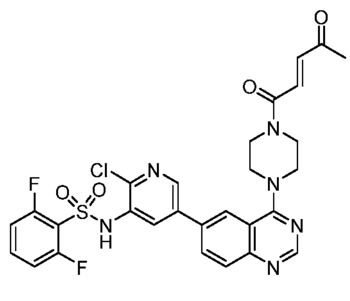 | $^1$H NMR (400 MHz, DMSO-$d_6$) δ 11.45 (brs, 1H), 8.70 (s, 1H), 8.60 (d, *J* = 4.0 Hz, 1H), 8.22-8.18 (m, 2H), 8.12 (dd, $J_1$ = 8.0 Hz, $J_2$ = 4.0 Hz, 1H), 7.94 (d, *J* = 8.0 Hz, 1H), 7.70-7.63 (m, 1H), 7.45 (d, *J* = 16.0 Hz, 1H), 7.26-7.21 (m, 2H), 6.74 (d, J = 16.0 Hz, 1H), 3.96-3.88 (m, 6H), 3.81-3.79 (m, 2H), 2.37 (s, 3H); ESI-MS(m/z): 613.0 $[M+H]^+$. |

(continued)

| Number | Name | Structure | $^1H$ NMR and MS |
|---|---|---|---|
| **Example 85** | *N*-(2-chloro-5-(4-(4-(2-fluoroacryloyl)pipera zin-1-yl)quinazolin-6-yl) pyridin-3-yl)-2,6-difluorobenzenesulfo namide | 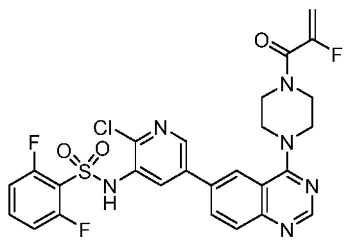 | $^1H$ NMR (400 MHz, DMSO-$d_6$) δ 11.46 (brs, 1H), 8.70 (s, 1H), 8.61 (d, *J* = 4.0 Hz, 1H), 8.21-8.18 (m, 2H), 8.12 (dd, $J_1$ = 8.0 Hz, $J_2$ = 4.0 Hz, 1H), 7.94 (d, *J* = 8.0 Hz, 1H), 7.71-7.64 (m, 1H), 7.26-7.21 (m, 2H), 5.35-5.33 (m, 1H), 5.30 (dd, $J_1$ = 68.0 Hz, $J_2$ = 4.0 Hz, 1H), 3.96-3.94 (m, 4H), 3.87-3.77 (m, 4H); ESI-MS(m/z): 589.0 $[M+H]^+$. |
| **Example 86** | (*E*)-*N*-(2-chloro-5-(4-(2-(4-oxopent-2-enoyl)-2,6-diazaspiro[3.4] octan-6-yl)quinazolin-6-yl)pyridin-3-yl)-2,6-difluorobenzenesulfo namide | 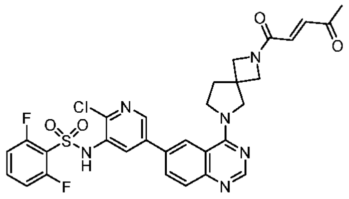 | $^1H$ NMR (400 MHz, DMSO-$d_6$) δ 8.55 (s, 1H), 8.40 (s, 1H), 8.35 (s, 1H), 8.07 (d, *J* = 4.0 Hz, 1H), 8.01-7.99 (m, 1H), 7.83 (d, *J* = 8.0 Hz, 1H), 7.60-7.52 (m, 1H), 7.27-7.22 (m, 2H), 6.88 (d, *J* = 16.0 Hz, 1H), 6.71 (d, *J* = 16.0 Hz, 1H), 4.41 (d, *J* = 8.0 Hz, 1H), 4.3 1 (d, *J* = 8.0 Hz, 1H), 4.24-4.17 (m, 2H), 4.10-4.07 (m, 1H), 4.03-3.97 (m, 3H), 2.32 (s, 3H), 2.28-2.24 (m, 2H); ESI-MS(m/z): 639.0 $[M+H]^+$. |
| **Example 87** | (*E*)-*N*-(2-chloro-5-(4-(2-(4-oxopent-2-enoyl)-2,7-diazaspiro [3.5] nonan -7-yl) quinazolin-6-yl)pyridin-3-yl)-2,6-difluorobenzenesulfo namide | 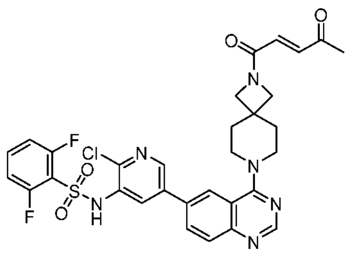 | $^1H$ NMR (400 MHz, DMSO-$d_6$) δ 11.62 (s, 1H), 8.68 (s, 1H), 8.59 (s, 1H), 8.20-8.08 (m, 3H), 7.92 (d, *J* = 8.8 Hz, 1H), 7.74-7.64 (m, 1H), 7.29-7.18 (m, 2H), 6.94 (d, *J* = 16.0 Hz, 1H), 6.74 (d, *J* = 16.0 Hz, 1H), 4.15 (s, 2H), 3.89-3.72 (m, 6H), 2.35 (s, 3H), 1.97 (t, *J* = 5.6 Hz, 4H); ESI-MS(m/z): 653.0 $[M+H]^+$. |
| **Example 88** | (*S,E*)-*N*-(2-chloro-5-(4-(2-methyl-4-(4-oxopent-2-enoyl)piperazin-1 - yl) quinazolin-6-yl)pyridin-3-yl)-2,6-difluorobenzenesulfo namide | 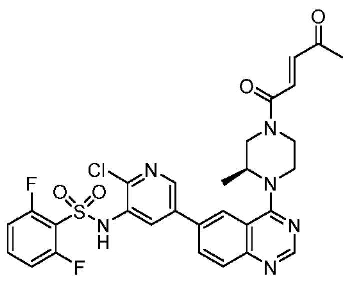 | $^1H$ NMR (400 MHz, DMSO-$d_6$) δ 8.65 (s, 1H), 8.51 (s, 1H), 8.13-8.07 (m, 3H), 7.94-7.91 (m, 1H), 7.67-7.58 (m, 1H), 7.48-7.32 (m, 1H), 7.18 (t, *J* = 12.0 Hz, 2H), 6.73 (dd, $J_1$ = 16.0 Hz, $J_2$ = 12.0 Hz, 1H), 4.86-4.76 (m, 1H), 4.41-4.10 (m, 3H), 4.02-3.88 (m, 1H), 3.53-3.44 (m, 1H), 3.23-3.10 (m, 1H), 2.36-2.33 (m, 3H), 1.31 (t, *J* = 4.0 Hz, 3H); ESI-MS(m/z): 627.0 $[M+H]^+$. |

(continued)

| Number | Name | Structure | $^1$H NMR and MS |
|---|---|---|---|
| **Example 89** | (*E*)-*N*-(2-chloro-5-(4-(4-(4-oxopent-2-enoyl)piperazin-1 - yl) quinazolin-6-yl)pyridin-3-yl)-2,4,6-trifluorobenzenesulfo namide | 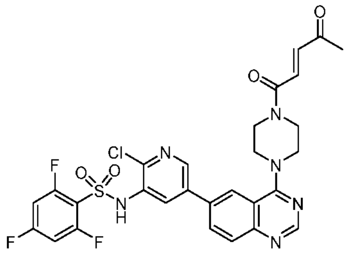 | $^1$H NMR (400 MHz, DMSO-$d_6$) δ 11.65 (s, 1H), 8.71 (s, 1H), 8.65-8.56 (m, 1H), 8.26-8.10 (m, 3H), 7.95 (d, *J* = 8.4 Hz, 1H), 7.49-7.34 (m, 3H), 6.74 (d, *J* = 15.6 Hz, 1H), 4.01-3.77 (m, 8H), 2.37 (s, 3H); ESI-MS(m/z): 631.0 $[M+H]^+$. |
| **Example 90** | (*E*)-2,3-difluoro-*N*-(2-methoxy-5-(4-(4-(4-oxopent-2-enoyl) piperazin-1-yl)quinazolin-6-yl)pyridin-3-yl) benzenesulfonami de | 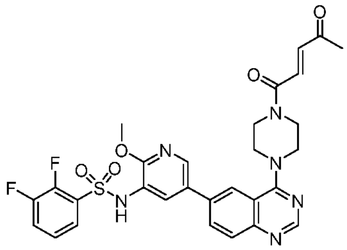 | $^1$H NMR (400 MHz, DMSO-$d_6$) δ 10.50 (brs, 1H), 8.66 (s, 1H), 8.39 (s, 1H), 8.11-8.06 (m, 2H), 7.97 (d, *J* = 8.0 Hz, 1H), 7.91 (d, *J* = 8.0 Hz, 1H), 7.75-7.68 (m, 1H), 7.54-7.51 (m, 1H), 7.45 (d, *J* = 16.0 Hz, 1H), 7.35-7.30 (m, 1H), 6.74 (d, *J* = 16.0 Hz, 1H), 3.89-3.79 (m, 8H), 3.67 (s, 3H), 2.37 (s, 3H); ESI-MS(m/z): 609.0 $[M+H]^+$. |
| **Example 91** | (*E*)-2-fluoro-*N*-(2-methoxy-5-(4-(4-(4-oxopent-2-enoyl) piperazin-1-yl)quinazolin-6-yl)pyridin-3-yl) benzenesulfonami de | 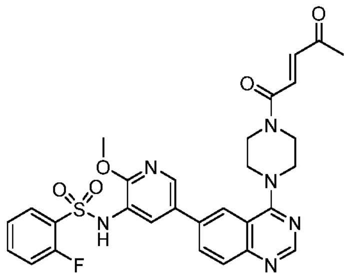 | $^1$H NMR (400 MHz, DMSO-$d_6$) δ 10.28 (s, 1H), 8.67 (s, 1H), 8.45 (d, J = 4.0 Hz, 1H), 8.12-8.06 (m, 2H), 7.99 (d, *J* = 8.0 Hz, 1H), 7.92 (d, *J* = 8.0 Hz, 1H), 7.74-7.68 (m, 2H), 7.48-7.46 (s, 2H), 7.34-7.30 (m, 1H), 6.74 (d, *J* = 16.0 Hz, 1H), 3.90-3.88 (m, 6H), 3.81-3.80 (m, 2H), 3.65 (s, 3H), 2.37 (s, 3H); ESI-MS(m/z): 591.0 $[M+H]^+$. |
| **Example 92** | (*E*)-4-fluoro-*N*-(2-methoxy-5-(4-(4-(4-oxopent-2-enoyl) piperazin-1-yl)quinazolin-6-yl)pyridin-3-yl) benzenesulfonami de | 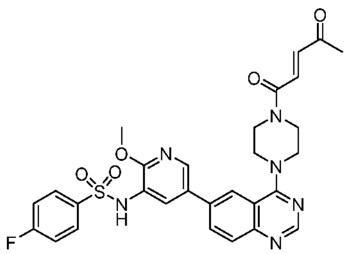 | $^1$H NMR (400 MHz, DMSO-$d_6$) δ 10.11 (s, 1H), 8.67 (s, 1H), 8.45-8.43 (m, 1H), 8.12-8.11 (m, 1H), 8.10-8.06 (m, 1H), 8.00-7.98 (m, 1H), 7.92 (d, J = 8.0 Hz, 1H), 7.85-7.83 (m, 1H), 7.83-7.80 (m, 1H), 7.48-7.43 (m, 1H), 7.43-7.39 (m, 2H), 6.74 (d, *J* = 16.0 Hz, 1H), 3.91-3.88 (m, 6H), 3.82-3.79 (m, 2H), 3.68 (s, 3H), 2.37 (s, 3H); ESI-MS(m/z): 591.0 $[M+H]^+$. |
| **Example 93** | (*E*)-*N*-(2-methoxy-5-(4-(4-(4-oxopent-2-enoyl)piperazin-1-yl) quinazolin-6-yl)pyridin-3-yl)-2-methylbenzenesulfon amide | 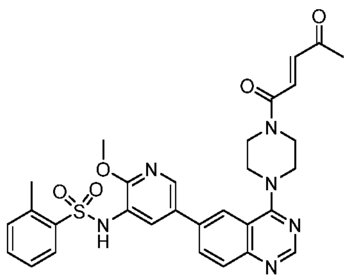 | $^1$HNMR (400 MHz, DMSO-$d_6$) δ 10.11 (s, 1H), 8.67 (s, 1H), 8.40-8.39 (m, 1H), 8.07-8.01 (m, 2H), 7.92-7.90 (m, 2H), 7.72 (d, *J* = 8.0 Hz, 1H), 7.75-7.41 (m, 4H), 6.74 (d, *J* = 16.0 Hz, 1H), 3.89 (s, 6H), 3.82-3.81 (m, 2H), 3.71 (s, 3H), 2.67 (s, 3H), 2.37 (s, 3H); ESI-MS(m/z): 587.0 $[M+H]^+$. |

(continued)

| Number | Name | Structure | $^1H$ NMR and MS |
|---|---|---|---|
| **Example 94** | (*E*)-2,5-difluoro-*N*-(2-methoxy-5-(4-(4-(4-oxopent-2-enoyl) piperazin-1-yl)quinazolin-6-yl)pyridin-3-yl) benzenesulfonami de | 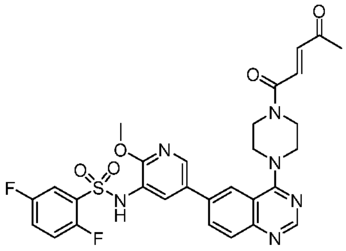 | $^1H$ NMR (400 MHz, DMSO-$d_6$) δ 10.46 (s, 1H), 8.66 (s, 1H), 8.47 (s, 1H), 8.14-8.08 (m, 2H), 8.03-8.01 (m, 1H), 7.92 (d, *J* = 12.0 Hz, 1H), 7.59-7.51 (m, 3H), 7.45 (d, *J* = 16.0 Hz, 1H), 6.74 (d, *J* = 16.0 Hz, 1H), 3.92-3.87 (m, 6H), 3.82-3.78 (m, 2H), 3.68 (s, 3H), 2.37 (s, 3H); ESI-MS(m/z): 609.0 [M+H]$^+$. |
| **Example 95** | (*E*)-2,4-difluoro-*N*-(2-methoxy-5-(4-(4-(4-oxopent-2-enoyl) piperazin-1-yl)quinazolin-6-yl)phenyl)benzenesul fonamide | 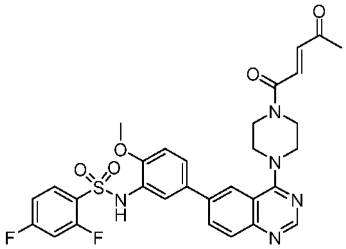 | $^1H$ NMR (400 MHz, DMSO-$d_6$) δ 9.99 (s, 1H), 8.67 (s, 1H), 8.08-8.06 (m, 2H), 7.92-7.90 (m, 1H), 7.76-7.70 (m, 1H), 7.68-7.64 (m, 2H), 7.59-7.53 (m, 1H), 7.47 (d, *J* = 16.0 Hz, 1H), 7.22-7.15 (m, 1H), 7.07 (d, *J* = 8.0 Hz, 1H), 6.75 (d, *J* =16.0 Hz, 1H), 4.07-3.81 (m, 8H), 3.56 (s, 3H), 2.38 (s, 3H); ESI-MS (m/z): 608.0 [M+H]$^+$. |

**Example 96**

**Preparation of (*E*)-8-chloro-*N*-(2-methoxy-5-(4-(4-(4-oxopent-2-enoyl)piperazin-1-yl)quinazolin-6-yl)pyridin-3-yl)naphthalene-1-sulfonamide**

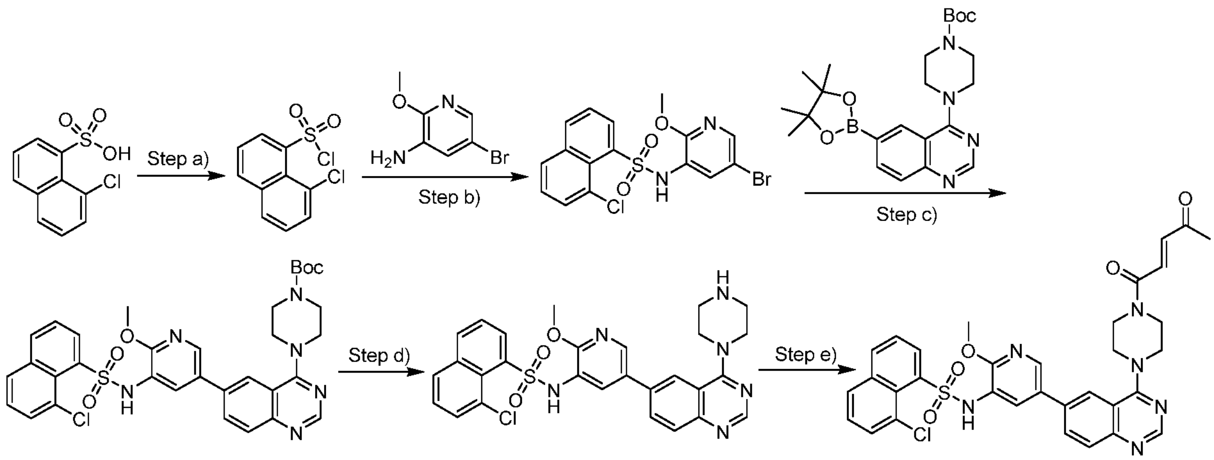


Step a): Preparation of 8-chloronaphthalene-1-sulfonyl chloride

8-chloronaphthalene-1-sulfonic acid (200 mg, 0.820 mmol) was dissolved in dichloromethane (4 mL), with DMF (0.6 mg, 0.008 mmol) added. After the addition was completed, the reaction mixture was cooled to 0°C, with oxalyl chloride (210 mg, 1.640 mmol) added dropwise. After the addition was completed, the reaction mixture was heated to 40°C and stirred for 3 h. Upon completion of the reaction, the reaction mixture was concentrated under reduced pressure. 8-chloronaphthalene-1-sulfonyl chloride was obtained, which can be used directly in the next reaction.

Step b-e): Preparation of (*E*)-8-chloro-*N*-(2-methoxy-5-(4-(4-(4-oxopent-2-enoyl)piperazin-1-yl)quinazolin-6-yl)pyridin-3-yl)naphthalene-1-sulfonamide

Referring to the preparation method of Example 83, (*E*)-8-chloro-*N*-(2-methoxy-5-(4-(4-(4-oxopent-2-enoyl)piperazin-1-yl)quinazolin-6-yl)pyridin-3-yl)naphthalene-1-sulfonamide was obtained, yield: 20.9%; $^1$H NMR (400 MHz, DMSO-$d_6$) δ 10.13 (s, 1H), 8.64 (s, 1H), 8.45 (d, *J* =8.0 Hz, 1H), 8.39 (s, 1H), 8.29 (d, *J* =8.0 Hz, 1H), 8.11 (d, *J* =8.0 Hz, 1H), 7.95-7.85 (m, 4H), 7.76 (s, 1H), 7.70-7.62 (m, 2H), 7.45 (d, *J* =16.0 Hz, 1H), 6.77 (d, *J* =16.0 Hz, 1H), 3.86-3.71 (m, 11H), 2.38 (s, 3H); ESI-MS(m/z): 657.0 [M+H]$^+$.

**Example 97**

**Preparation of (*E*)-2,4,6-trifluoro-*N*-(2-methoxy-5-(4-(2-(4-oxopent-2-enoyl)-2,6-diazaspiro[3.4]octan-6-yl)quinazolin-6-yl)pyridin-3-yl)benzenesulfonamide**

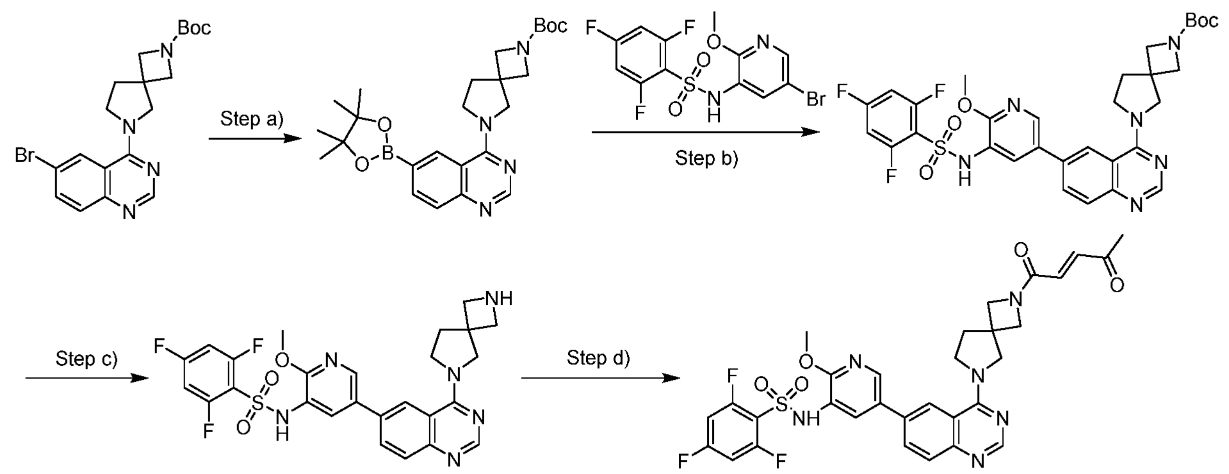


Step a): Preparation of tert-butyl 6-(6-(4,4,5,5-tetramethyl-1,3,2-dioxaborolan-2-yl)quinazolin-4-yl)-2,6-diazaspiro [3.4] octane-2-carboxylate

Tert-butyl 6-(6-bromoquinazolin-4-yl)-2,6-diazaspiro[3.4]octane-2-carboxylate (725 mg, 2.864 mmol), Pd(dppf)$Cl_2$ (174 mg, 0.239 mmol) and potassium acetate (701 mg, 7.161 mmol) were added to dioxane (20 mL). After the addition was completed, in the presense of protective nitrogen, the reaction mixture was heated to 110°C and stirred for 2 h. Upon completion of the reaction, the reaction was quenched by adding water (50 mL). The reaction mixture was extracted with ethyl acetate (150 mL×2 times). The organic phases were combined, washed with saturated brine (100 mL×1 time), dried over anhydrous sodium sulfate and filtered. The filtrate was concentrated under reduced pressure. The crude product obtained was purified by silica gel column chromatography (Eluent: dichloromethane/methanol = 100/1 to 10/1). Tert-butyl 6-(6-(4,4,5,5-tetramethyl-1,3,2-dioxaborolan-2-yl)quinazolin-4-yl)-2,6-diazaspiro[3.4]octane-2-carboxylate was obtained, yield: 81.1%; ESI-MS(m/z): 467.0 [M+H]$^+$.

Step b): Preparation of tert-butyl 6-(6-(6-methoxy-5-((2,4,6-trifluorophenyl)sulfonamido)pyridin-3-yl)quinazolin-4-yl)-2,6-diazaspiro [3.4]octane-2-carboxylate

N-(5-bromo-2-methoxypyridin-3-yl)-2,4,6-trifluorobenzenesulfonamide (300 mg, 0.756 mmol), tert-butyl 6-(6-(4,4,5,5-tetramethyl-1,3,2-dioxaborolan-2-yl)quinazolin-4-yl)-2,6-diazaspiro[3,4]octane-2-carboxylate (425 mg, 0.907 mmol), Pd(dppf)$Cl_2$ (56 mg, 0.076 mmol) and cesium carbonate (743 g, 2.268 mmol) were added to dioxane/water mixed solvent (10 mL, v/v=4:1). After the addition was completed, in the presense of protective nitrogen, the reaction mixture was heated to 110°C and stirred for 2 h. Upon completion of the reaction, the reaction was quenched by adding water (50 mL). The reaction mixture was extracted with ethyl acetate (150 mL×2 times). The organic phases were combined, washed with saturated brine (100 mL×1 time), dried over anhydrous sodium sulfate and filtered. The filtrate was concentrated under reduced pressure. The crude product obtained was purified by silica gel column chromatography (Eluent: dichloromethane/methanol = 100/1 to 10/1). Tert-butyl 6-(6-(6-methoxy-5-((2,4,6-trifluorophenyl)sulfonamido)pyridin-3-yl)quinazolin-4-yl)-2,6-diazaspiro[3.4]octane-2-carboxylate was obtained, yield: 50.4%; ESI-MS(m/z): 657.6

[M+H]+.

Step c): Preparation of *N*-(5-(4-(2,6-diazaspiro[3.4]octan-6-yl)quinazolin-6-yl)-2-methoxypyridin-3-yl)-2,4,6-trifluorobenzenesulfonamide

Tert-butyl 6-(6-(6-methoxy-5-((2,4,6-trifluorophenyl)sulfonamido)pyridin-3-yl)quinazolin-4-yl)-2,6-diazaspiro[3.4]octane-2-carboxylate (250 mg, 0.381 mmol) was dissolved in dichloromethane (8 mL). Under the ice bath condition, TFA (2 mL) was slowly added dropwise. After the addition was completed, the reaction mixture was stirred at room temperature for 1 h. Upon completion of the reaction, the solvent was directly spinned dry. The product was added to 5mL methyl tert-butyl ether to slurry, filtered. The solid was collected to obtain *N*-(5-(4-(2,6-diazaspiro[3.4]octan-6-yl)quinazolin-6-yl)-2-methoxypyridin-3-yl)-2,4,6-trifluorobenzenesulfonamide. ESI-MS(m/z): 557.2 [M+H]+.

Step d): Preparation of (*E*)-2,4,6-tfifluoro-*N*-(2-methoxy-5-(4-(2-(4-oxopent-2-enoyl)-2,6-diazaspiro[3.4]octan-6-yl)quinazolin-6-yl)pyridin-3-yl)benzenesulfonamide

*N*-(5-(4-(2,6-diazaspiro[3.4]octan-6-yl)quinazolin-6-yl)-2-methoxypyridin-3-yl)-2,4,6-trifluorobenzenesulfonamide (100 mg, 0.180mmol) and (*E*)-4-oxopent-2-enoic acid (31 mg, 0.270 mmol) were dissolved in tetrahydrofuran (2 mL). The reaction system was cooled to -78°C, with *N*,*N*-diisopropylethylamine (116 mg, 0.900 mmol) and 1-propylphosphonic anhydride (115 mg, 0.360 mmol) added successively. After the addition was completed, the reaction mixture was kept at -78°C and stirred for 1 h. Upon completion of the reaction, with acetonitrile (2 ml) added, the reaction solution was purified by Prep-HPLC (Method 2). (*E*)-2,4,6-trifluoro-*N*-(2-methoxy-5-(4-(2-(4-oxopent-2-enoyl)-2,6-diazaspiro[3.4]octan-6-yl)quinazolin-6-yl)pyridin-3-yl)benzenesulfonamidewas obtained, yield: 20.4%; $^1$HNMR (400 MHz, DMSO-$d_6$) δ 10.66 (s, 1H), 8.48 (s, 1H), 8.47-8.34 (m, 2H), 7.98-7.91 (m, 2H), 7.80 (d, *J* = 12 Hz, 1H), 7.30-7.25 (m, 2H), 6.88 (d, *J* = 16 Hz, 1H), 6.71 (d, *J* = 16 Hz, 1H), 4.43-4.41 (m, 1H), 4.32-4.30 (m, 1H), 4.21-4.19 (m, 2H), 4.11-4.08 (m, 1H), 4.05-3.96 (m, 3H), 3.71 (s, 3H), 2.32 (s, 3H), 2.28-2.24 (m, 2H); ESI-MS(m/z): 653.0 [M+H]+.

Referring to the preparation method of Example 97 and using the corresponding raw materials, the compounds in the following examples wereprepared.

| Number | Name | Structure | $^1$H NMR and MS |
|---|---|---|---|
| **Example 98** | 2,4,6-trifluoro-*N*-(5-(4-(4-(2-fluoroacryloyl)pipera zin-1-yl)quinazolin-6-yl)-2-methoxypyridin-3-yl) benzenesulfonami de | 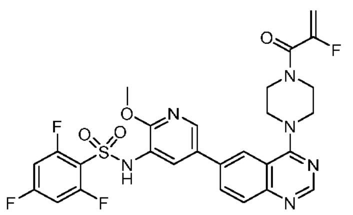 | $^1$H NMR (400 MHz, DMSO-$d_6$) δ 10.66 (s, 1H), 8.67 (s, 1H), 8.50 (s, 1H), 8.17-8.08 (m, 2H), 8.04 (d, *J* = 2.0 Hz, 1H), 7.92 (d, *J* = 8.8 Hz, 1H), 7.41 (t, *J* = 9.2 Hz, 2H), 5.33 (dd, $J_1$ = 4.0, $J_2$ = 2.0 Hz, 1H), 5.29 (dd, $J_1$ = 66.0 Hz, $J_2$ = 4.0 Hz, 1H), 3.96-3.86 (m, 4H), 3.80-3.78 (m, 4H), 3.69 (s, 3H); ESI-MS(m/z): 603.0 [M+H]+. |
| **Example 99** | (*E*)-2,4,6-trifluoro-*N*-(2-methoxy-5-(4-(4-(4-oxopent-2-enoyl)piperazin-1-yl)quinazolin-6-yl)pyridin-3-yl)benzenesulfonami de | 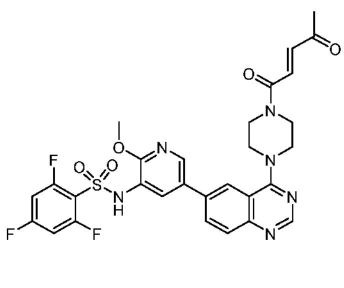 | $^1$H NMR (400 MHz, DMSO-$d_6$) δ 10.66 (s, 1H), 8.67 (s, 1H), 8.54-8.44 (m, 1H), 8.16-8.08 (m, 2H), 8.05 (d, *J* = 2.4 Hz, 1H), 7.92 (d, *J* = 8.8 Hz, 1H), 7.50-7.37 (m, 3H), 6.74 (d, *J* = 15.6 Hz, 1H), 3.90-3.88 (m, 6H), 3.82-3.79 (m, 2H), 3.68 (s, 3H), 2.37 (s, 3H); ESI-MS(m/z): 627.0 [M+H]+. |

(continued)

| Number | Name | Structure | $^1$H NMR and MS |
|---|---|---|---|
| **Example 100** | *N*-(5-(4-(4-acryloylpiperazin-1-yl) quinazolin-6-yl)-2-methoxypyridin-3-yl)-2,4,6-trifluorobenzenesulfo namide | 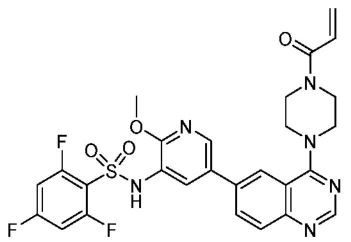 | $^1$H NMR (400 MHz, DMSO-$d_6$) δ 10.67 (s, 1H), 8.66 (s, 1H), 8.51 (d, *J* = 2.4 Hz, 1H), 8.19-8.08 (m, 2H), 8.05 (d, *J* = 2.4 Hz, 1H), 7.91 (d, *J* = 8.8 Hz, 1H), 7.54-7.34 (m, 2H), 6.83 (dd, *J* = 16.8, 10.4 Hz, 1H), 6.17 (dd, *J* = 16.8, 2.4 Hz, 1H), 5.74 (dd, *J* = 10.4, 2.4 Hz, 1H), 3.96-3.72 (m, 8H), 3.68 (s, 3H); ESI-MS(m/z): 585.0 [M+H]$^+$. |
| **Example 101** | (*E*)-2,4,6-trifluoro-*N*-(2-methoxy-5-(4-(2-(4-oxopent-2-enoyl)-2,7-diazaspiro[3.5]nonan -7-yl) quinazolin-6-yl)pyridin-3-yl) benzenesulfonami de | 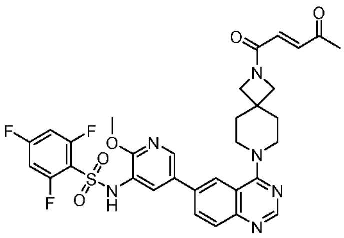 | $^1$H NMR (400 MHz, DMSO-$d_6$) δ 10.68 (s, 1H), 8.64 (s, 1H), 8.46 (s, 1H), 8.12-8.00 (m, 3H), 7.90 (d, *J* = 8.4 Hz, 1H), 7.43 (t, *J* = 9.2 Hz, 2H), 6.93 (d, *J* = 15.6 Hz, 1H), 6.74 (d, *J* = 15.6 Hz, 1H), 4.15 (s, 2H), 3.83-3.71 (m, 6H), 3.69 (s, 3H), 2.35 (s, 3H), 1.98-1.95 (m, 4H); ESI-MS(m/z): 667.0 [M+H]$^+$. |

**Example 102**

**Preparation of (E)-3,5-difluoro-N-(2-methoxy-5-(4-(4-(4-oxopent-2-enoyl)piperazin-1-yl)quinazolin-6-yl)pyridin-3-yl)pyridine-4-sulfonamide**

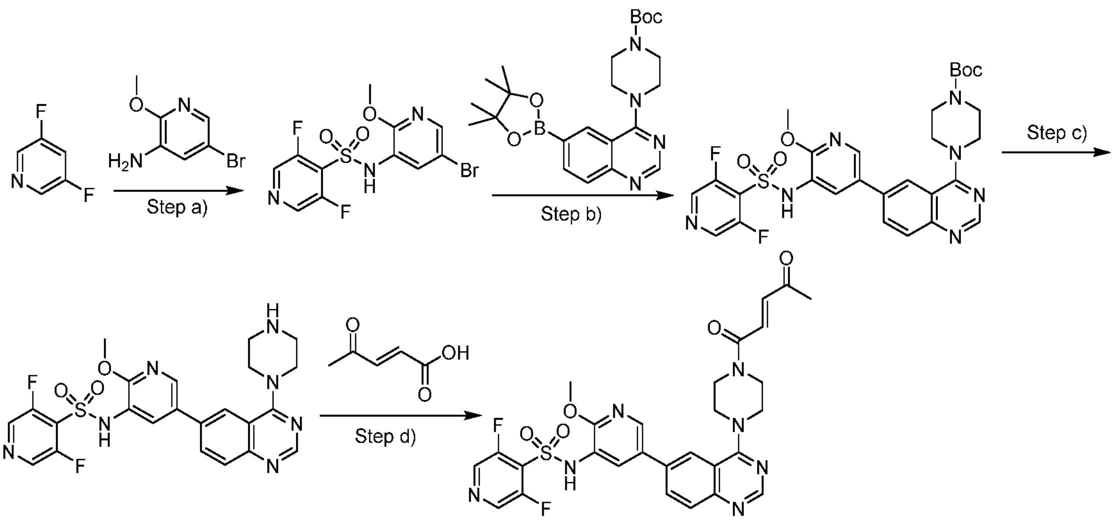


Step a): Preparation of *N*-(5-bromo-2-methoxypyridin-3-yl)-3,5-difluoropyridine-4-sulfonamide

3,5-difluoropyridine (500 mg, 4.345 mmol) was added to tetrahydrofuran (20 mL), in the presense of protective nitrogen, the reaction solution was cooled to -78°C, with butyllithium solution (1.54 g, 18% wt) added. After the addition was completed, the reaction mixture was kept at -78°C and reacted for 1 h. Then at -78°C, sulfur dioxide gas was bubble into the reaction solution for 10 min. After that, the reaction mixture was heated to room temperature and reacted for 1 h. Upon completion of the reaction, the reaction mixture was concentrated under reduced pressure. The residue was dissolved in dichloromethane (20 mL), with N-chlorosuccinimide (2.35 g, 17.600 mmoL) added, at room temperature,

reacted for 2 h. Upon completion of the reaction, under the ice bath condition, the reaction solution was quenched by adding saturated sodium sulfite solution (30 mL). The reaction mixture was extracted by adding dichloromethane (100 mL×2 times). The organic phases were combined, washed with saturated brine (100 mL), dried over anhydrous sodium sulfate, filtered, concentrated through pressure concentration. The residue was dissolved in pyridine (5 mL), with 5-bromo-2-methoxypyridin-3-amine (882 mg, 4.345 mmol) added. The reaction mixture was kept at room temperature and reacted overnight. Upon completion of the reaction, the reaction was quenched by adding water (30 mL). The reaction mixture was extracted by adding dichloromethane (50 mL×2 times). The organic phases were combined, washed with saturated brine (50 mL), dried over anhydrous sodium sulfate, filterd, and concentrated under reduced pressure. The crude product obtained was purified by silica gel column chromatography (Eluent: methanol/dichloromethane = 1/100 to 1/50). *N*-(5-bromo-2-methoxypyridin-3-yl)-3,5-difluoropyridine-4-sulfonamide was obtained, yield: 12.8%; ESI-MS(m/z): 382.0 $[M+H]^+$.

Step b-d): Preparation of (*E*)-3,5-difluoro-*N*-(2-methoxy-5-(4-(4-(4-oxopent-2-enoyl)piperazin-1-yl)quinazolin-6-yl)pyridin-3-yl)pyridine-4-sulfonamide

Referring to the preparation method of Example 83, Step b-d, (*E*)-3,5-difluoro-*N*-(2-methoxy-5-(4-(4-(4-oxopent-2-enoyl)piperazin-1-yl)quinazolin-6-yl)pyridin-3-yl)pyridine-4-sulfonamide was obtained, yield: 23.8%; $^1H$ NMR (400 MHz, DMSO-$d_6$) δ 11.19 (brs, 1H), 8.72 (s, 2H), 8.67 (s, 1H), 8.44 (s, 1H), 8.14-8.09 (m, 2H), 8.03-8.02 (m, 1H), 7.92 (d, *J* = 16.0 Hz, 1H), 7.46 (d, *J* = 16.0 Hz, 1H), 6.74 (d, *J* = 16.0 Hz, 1H), 3.91-3.80 (m, 8H), 3.62 (s, 3H), 2.37 (s, 3H); ESI-MS(m/z): 610.0 $[M+H]^+$.

**Example 103**

**Preparation of (*E*)-3-fluoro-*N*-(2-methoxy-5-(4-(4-(4-oxopent-2-enoyl)piperazin-1-yl)quinazolin-6-yl)pyridin-3-yl)pyridine-4-sulfonamide**

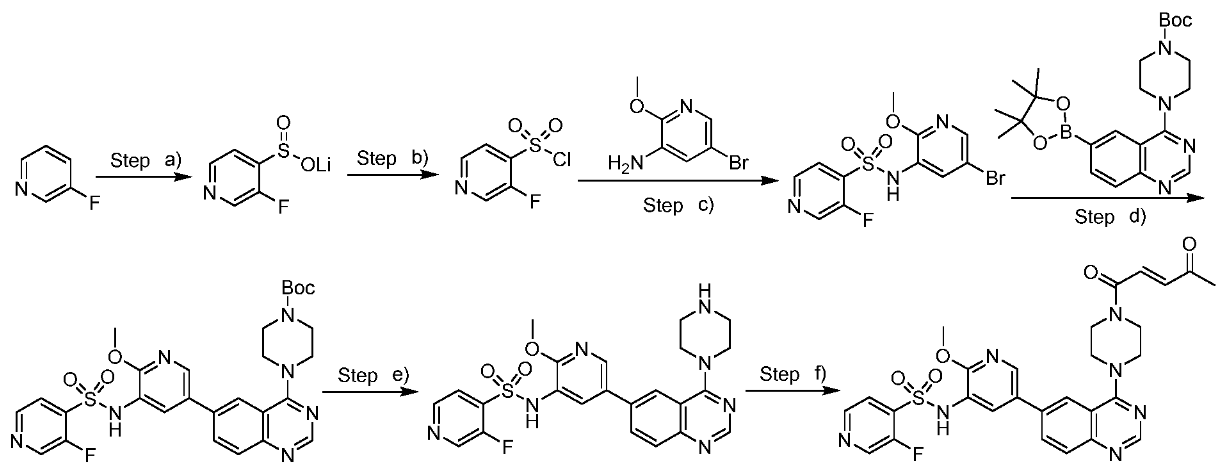


Step a): Preparation of lithium 3-fluoropyridine-4-sulfinate

3-fluoropyridine (600 mg, 6.184 mmol) was placed in a three-necked bottle. In the presense of protective nitrogen, tetrahydrofuran (25 mL) was added. The reaction mixture was cooled to -78°C, with n-butyllithium (3.23 mL, 6.184 mmol, 1.6 M n-hexane solution) slowly added. After the addition was completed, the reaction mixture was kept at current temperature and reacted for 0.5 h, after that, with sulfur dioxide gas bubbled for 10 minutes, continued the reaction at room temperature for 2 h. Upon completion of the reaction, the reaction solution was directly spinned dry. Lithium 3-fluoropyridine-4-sulfinate was obtained. The product can be used directly in the next reaction without purification; $^1H$ NMR (400 MHz, DMSO-$d_6$) δ 8.46 (dd, *J* = 4.8, 2.0 Hz, 1H), 8.41 (s, 1H), 7.60 (t, *J* = 5.2 Hz, 1H); ESI-MS(m/z): 162.0 $[M-Li+H+H]^+$.

Step b): Preparation of 3-fluoropyridine-4-sulfonyl chloride

Lithium 3-fluoropyridine-4-sulfinate (2945 mg) was dispersed in dichloromethane (20 mL), with N-bromosuccinimide (1.7 g, 12.364 mmol) added in portions. After the addition was completed, in the presense of protective nitrogen, the reaction mixture was kept at room temperature and reacted for 2 h. Upon completion of the reaction, the reaction was quenched by adding water (30 mL), and the reaction mixture was extracted with dichloromethane (10 mL×2). The organic phases were combined, wased with water (40 mL), dried over anhydrous sodium sulfate, filtered. The filtrate was used directly in the next reaction.

Step c): Preparation of *N*-(5-bromo-2-methoxypyridin-3-yl)-3-fluoropyridine-4-sulfonamide

5-bromo-2-methoxypyridin-3-amine (1.4 g, 6.800 mmol), 4-dimethylaminopyridine (76 mg, 0.620 mmol) and pyridine (2.4 g, 30.854 mmol) were added to the reaction flask which contained 3-fluoropyridine-4-sulfonyl chloride filtrate. In the presense of protective nitrogen, the reaction mixture was kept at room temperature and reacted for 15 h. Upon completion of the reaction, the reaction was quenched by adding saturated aqueous ammonium chloride solution (80 mL), and the reaction mixture was extracted with dichloromethane (50 mL×2). The organic phases were combined, dried over anhydrous sodium sulfate and filtered. The filtrate was concentrated under reduced pressure. The crude product obtained was purified by silica gel column chromatography (Eluent: dichloromethane/methanol = 1/0 to 10/1). *N*-(5-bromo-2-methoxypyridin-3-yl)-3-fluoropyridine-4-sulfonaniide was obtained, three-step yield: 16.8%; $^1$H NMR (400 MHz, DMSO-$d_6$) δ 10.88 (s, 1H), 8.89 (d, *J* = 2.0 Hz, 1H), 8.64 (dd, *J* = 5.2, 0.8 Hz, 1H), 8.18 (d, *J* = 2.0 Hz, 1H), 7.83 (d, *J* = 2.4 Hz, 1H), 7.69 (t, *J* = 6.0, 4.8 Hz, 1H), 3.55 (s, 3H); ESI-MS(m/z): 361.9 [M+H]$^+$.

Step d-f): Preparation of (*E*)-3-fluoro-*N*-(2-methoxy-5-(4-(4-(4-oxopent-2-enoyl)piperazin-1-yl)quinazolin-6-yl)pyridin-3-yl)pyridine-4-sulfonamide

Referring to the preparation method of Example 83, Step b-d, (*E*)-3-fluoro-*N*-(2-methoxy-5-(4-(4-(4-oxopent-2-enoyl)piperazin-1-yl)quinazolin-6-yl)pyridin-3-yl)pyridine-4-sulfonamide was obtained, yield: 52.2%; $^1$H NMR (400 MHz, DMSO-$d_6$) δ 10.81 (s, 1H), 8.89 (d, *J* = 1.6 Hz, 1H), 8.67 (s, 1H), 8.63 (d, *J* = 4.8 Hz, 1H), 8.53-8.49 (m, 1H), 8.17-8.10 (m, 2H), 8.06 (d, *J* = 2.4 Hz, 1H), 7.92 (d, *J* = 8.8 Hz, 1H), 7.72-7.67 (m, 1H), 7.47 (d, *J* = 16.0 Hz, 1H), 6.74 (d, *J* = 16.0 Hz, 1H), 3.96-3.78 (m, 8H), 3.61 (s, 3H), 2.38 (s, 3H); ESI-MS(m/z): 592.0 [M+H]$^+$.

**Example 104**

**Preparation of (*E*)-4-Chloro-*N*-(2-methoxy-5-(4-(4-(4-oxopent-2-enoyl)piperazin-1-yl)quinazoline-6-yl)pyridin-3-yl)-1-methyl-1*H*-pyrrole-2-sulfonamide**

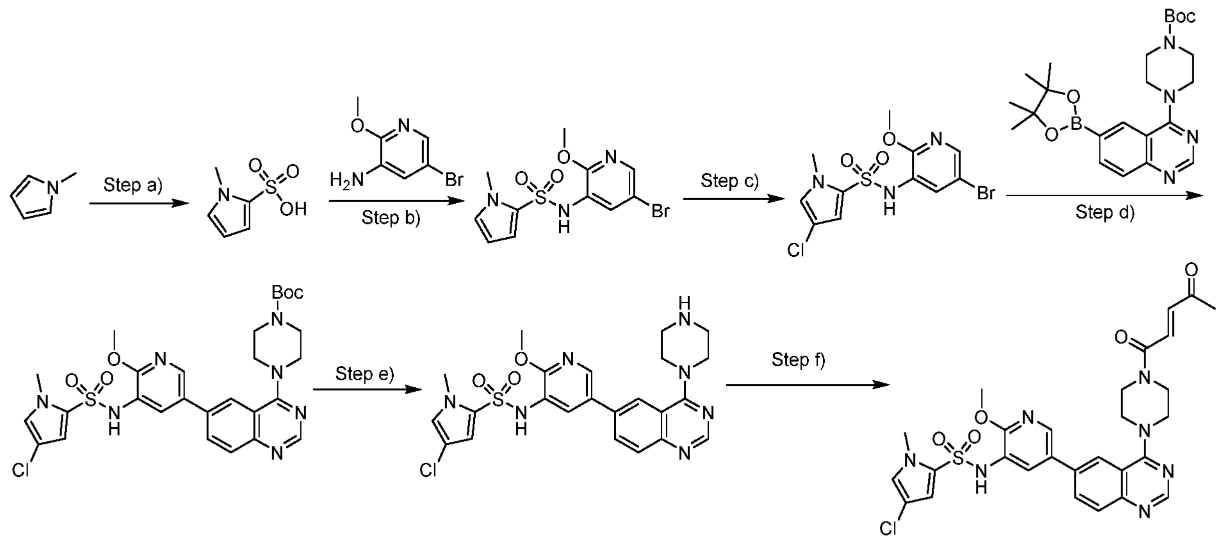


Step a): Preparation of 1-methyl-1*H*-pyrrole-2-sulfonic acid

1-methyl-1*H*-pyrrole (300 mg, 3.704 mmol) was dissolved in dichloromethane (30 mL). After the reaction system was cooled to 0°C, chlorosulfonic acid (433 mg, 3.704 mmol) was diluted and slowly added dropwise to the reaction

system. After the addition was completed, the reaction system was kept at 0°C and stirred for 2 h. Upon completion of the reaction, the reaction solution can be used directly in the next reaction; ESI-MS(m/z): 160.1 $[M-H]^-$.

Step b): Preparation of *N*-(5-bromo-2-methoxypyridin-3-yl)-1-methyl-1*H*-pyrrole-2-sulfonamide

After the reaction solution in the previous step was cooled to 0°C, oxalyl chloride (1.9 g, 14.816 mmol) and *N*,*N*-dimethylformamide (8 mg, 0.111 mmol) were added to the system. After the addition was completed, the reaction mixture was heated to room temperature and reacted for 2 h. Upon completion of the reaction, the reaction solution was concentrated under reduced pressure. The residue was then dissolved in dichloromethane (20 mL). 4-dimethylaminopyridine (23 mg, 0.185 mmol), pyridine (878 mg, 11.112 mmol) and 5-bromo-2-methoxypyridin-3-amine (1.1 g, 5.556mmol) were added to the system at room temperature. After the addition was completed, the reaction mixture was kept at room temperature and reacted for 3 h. Upon completion of the reaction, the reaction was quenched by adding water (50 mL). The reaction mixture was extracted by adding dichloromethane (100 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate = 2/1 to 1/2). *N*-(5-bromo-2-methoaypyridin-3-yl)-1-methyl-1*H*-pyrrole-2-sulfonamide was obtained, yield: 19.5%; ESI-MS(m/z): 346.0 $[M+H]^+$.

Step c): Preparation of *N*-(5-bromo-2-methoxypyridin-3-yl)-4-chloro-1-methyl-1*H*-pyrrole-2-sulfonamide

*N*-(5-bromo-2-methoxypyridin-3-yl)-1-methyl-1*H*-pyrrole-2-sulfonamide (180 mg, 0.520 mmol) was dissolved in tetrahydrofuran/acetic acid mixed solvent (10 mL, v/v=1:1), with *N*-chlorosuccinimide (59 mg, 0.572 mmol) added. After the addition was completed, the reaction mixture was kept at 80°C and stirred for 6 h. Upon completion of the reaction, the reaction was quenched by adding water (50 mL). The reaction mixture was extracted by adding dichloromethane (100 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate = 2/1 to 1/2). *N*-(5-bromo-2-methoaypyridin-3-yl)-4-chloro-1-methyl-1*H*-pyrrole-2-sulfonamidewas obtained, yield: 60.6%; ESI-MS(m/z): 380.8 $[M+H]^+$.

Step d-f): Preparation of (*E*)-4-chloro-*N*-(2-methoxy-5-(4-(4-(4-oxopent-2-enoyl)piperazin-1-yl)quinazoline-6-(yl)pyridin-3-yl)-1-methyl-1*H*-pyrrole-2-sulfonamide

Referring to the preparation method of Example 83, Step b-d, (*E*)-4-chloro-*N*-(2-methoxy-5-(4-(4-(4-oxopent-2-enoyl)piperazin-1-yl)quinazoline-6-(yl)pyridin-3-yl)-1-methyl-1H-pyrrole-2-sulfonamide was obtained, yield: 20.3%; $^1$HNMR (400 MHz, DMSO-$d_6$) $\delta$ 9.43 (s, 1H), 8.67 (s, 1H), 8.39 (s, 1H), 8.11 (s, 1H), 8.07 (d, *J* = 12.0 Hz, 1H), 7.97-7.92 (m, 2H), 7.48-7.44 (m, 2H), 6.74 (d, *J* = 16.0 Hz, 1H), 6.40 (s, 1H), 3.92-3.89 (m, 6H), 3.86 (s, 3H), 3.84-3.80 (m, 2H), 3.56 (s, 3H), 2.37 (s, 3H); ESI-MS(m/z): 610.0 $[M+H]^+$.

## Example 105

### Preparation of (*E*)-2,4-difluoro-*N*-(2-(methylamino)-5-(4-(4-(4-oxopent-2-enoyl)piperazin-1-yl)quinazolin-6-yl)pyridin-3-yl)benzenesulfonamide

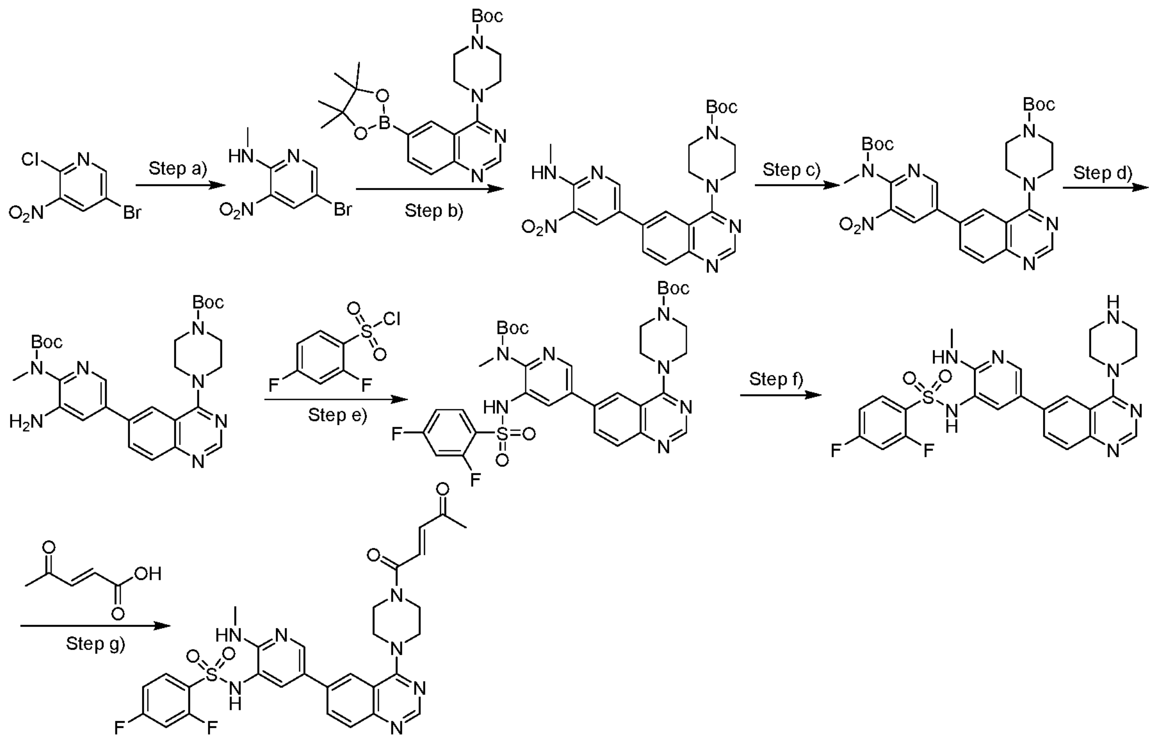


Step a): Preparation of 5-bromo-*N*-methyl-3-nitropyridin-2-amine

5-bromo-2-chloro-3-nitropyridine (5.0 g, 21.058 mmol), methylamine hydrochloride (4.3 g, 62.963 mmol), potassium carbonate (14.55 g, 105.290 mmol) were added to DMF (150 mL). After the addition was completed, the reaction mixture was stirred at room temperature and reacted overnight. Upon completion of the reaction, the reaction was quenched by adding water (100 mL). The reaction mixture was extracted with ethyl acetate (200 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 50/1). 5-Bromo-*N*-methyl-3-nitropyridin-2-amine was obtained, yield: 96.2%; ESI-MS(m/z): 232.0 $[M+H]^+$.

Step b): Preparation of tert-butyl 4-(6-(6-(methylamino)-5-nitropyridin-3-yl)quinazolin-4-yl)piperazine-1-carboxylate

5-bromo-*N*-methyl-3-nitropyridin-2-amine (500 mg, 2.155 mmol), tert-butyl 4-(6-(4,4,5,5-tetramethyl-1,3,2-dioxaborolan-2-yl)quinazolin-4-yl)piperazine-1-carboxylate (949 mg, 2.155 mmol), Pd(dppf)$Cl_2$ (315 mg, 0.431 mmol) and cesium carbonate (2.1 g, 6.465 mmol) were added to dioxane/water mixed solvent (20 mL, v/v = 10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (150 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 40/1). Tert-butyl 4-(6-(6-(methylamino)-5-nitropyridin-3-yl)quinazolin-4-yl)piperazine-1-carboxylate was obtained, yield: 88.5%; ESI-MS(m/z): 466.2 $[M+H]^+$.

Step c): Preparation of tert-butyl 4-(6-(6-((tert-butoxycarbonyl)(methyl)amino)-5-nitropyridin-3-yl)quinazolin-4-yl)piperazine-1-carboxylate

Tert-butyl 4-(6-(6-(methylamino)-5-nitropyridin-3-yl)quinazolin-4-yl)piperazine-1-carboxylate (500 mg, 1.075 mmol), di-tert-butyl dicarbonate (704 mg, 3.225 mmol), 4-dimethylaminopyridine (6 mg, 0.054 mmol) and *N,N*-diisopropylethylamine (693 mg, 5.375 mmol) were added to DMF (10 mL). After the addition was completed, at 60°C, the reaction mixture was stirred and reacted for 2 h. Upon completion of the reaction, the reaction was quenched by adding water (50 mL). The reaction mixture was extracted with ethyl acetate (150 mL×2 times). The organic phases were combined, washed with saturated brine (100 mL), dried over anhydrous sodium sulfate and filtered. The filtrate was concentrated

under reduced pressure. The crude product obtained was purified by silica gel column chromatography (Eluent: dichloromethane/methanol = 100/1 to 50/1). Tert-butyl 4-(6-(6-((tertbutoxycarbonyl)(methyl)amino)-5-nitropyridin-3-yl)quinazolin-4-yl)piperazine-1-carboxylate was obtained, yield: 90.5%; ESI-MS(m/z): 566.3 $[M+H]^+$.

Step d): Preparation of tert-butyl 4-(6-(5-amino-6-((tert-butoxycarbonyl)(methyl)amino)pyridin-3-yl)quinazolin-4-yl)piperazine-1-carboxylate

Tert-butyl 4-(6-(6-((tert-butoxycarbonyl)(methyl)amino)-5-nitropyridin-3-yl)quinazolin-4-yl)piperazine-1-carboxylate (500 mg, 0.884 mmol), Fe powder (494 mg, 8.840 mmol), ammonium chloride (47 mg, 0.884 mmol) were added to ethanol/water = 5/1 mixed solvent (30 mL) . After the addition was completed, at 90°C, the reaction mixture was stirred and reacted for 4 h. Upon completion of the reaction. The reaction was quenched by adding water (50 mL). The reaction mixture was extracted with ethyl acetate (200 mL×2 times). The organic phases were combined, washed with saturated brine (100 mL), dried over anhydrous sodium sulfate and filtered. The filtrate was concentrated under reduced pressure. Tert-butyl 4-(6-(5-amino-6-((tertbutoxycarbonyl)(methyl)amino)pyridin-3-yl)quinazolin-4-yl)piperazine-1-carboxylate was obtained, yield: 68.6%; ESI-MS(m/z): 536.3 $[M+H]^+$.

Step e): *tert-butyl* 4-(6-(6-((tert-butoxycarbonyl)(methyl)amino)-5-((2,4-difluorophenyl)sulfonamido)pyridin-3-yl)quinazolin-4-yl)piperazine-1-carboxylate

Tert-butyl 4-(6-(5-amino-6-((tert-butoxycarbonyl)(methyl)amino)pyridin-3-yl)quinazolin-4-yl)piperazine-1-carboxylate (300 mg, 0.560 mmol), 2,4-difluorobenzenesulfonyl chloride (357 mg, 1.680 mmol) were added to pyridine (5 mL). After the addition was completed, at 80°C, the reaction mixture was stirred and reacted overnight. Upon completion of the reaction, the reaction solution was diluted by adding water (50 mL) and extracted with ethyl acetate (200 mL×2 times). The organic phases were combined, washed with saturated brine (100 mL), dried over anhydrous sodium sulfate and filtered. The filtrate was concentrated under reduced pressure until completely dried. The crude product obtained was purified by silica gel column chromatography (Eluent: dichloromethane/methanol = 100/1 to 50/1). *Tert-butyl* 4-(6-(6-((tert-butoxycarbonyl)(methyl)amino)-5-((2,4-difluorophenyl)sulfonamido)pyridin-3-yl)quinazolin-4-yl)piperazine-1-carboxylate was obtained, yield: 42.6%; ESI-MS(m/z): 712.3 $[M+H]^+$.

Step f): Preparation of 2,4-difluoro-*N*-(2-(methylamino)-5-(4-(piperazin-1-yl)quinazolin-6-yl)pyridin-3-yl)benzenesulfonamide trifluoroacetate

*Tert-butyl* 4-(6-(6-((tert-butoxycarbonyl)(methyl)amino)-5-((2,4-difluorophenyl)sulfonamido)pyridin-3-yl)quinazolin-4-yl)piperazine-1-carboxylate (170 mg, 0.239 mmol) was dissolved in dichloromethane (3 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. Solid phase 2,4-difluoro-*N*-(2-(methylamino)-5-(4-(piperazin-1-yl)quinazolin-6-yl)pyridin-3-yl)benzenesulfonamide trifluoroacetate was obtained, yield: 90.2%; ESI-MS(m/z): 512.2 $[M+H]^+$.

Step g): Preparation of (*E*)-2,4-difluoro-*N*-(2-(methylamino)-5-(4-(4-(4-oxopent-2-enoyl)piperazin-1-yl)quinazolin-6-yl)pyridin-3-yl)benzenesulfonamide

2,4-difluoro-*N*-(2-(methylamino)-5-(4-(piperazin-1-yl)quinazolin-6-yl)pyridin-3-yl)benzenesulfonamide trifluoroacetate (110 mg, 0.176 mmol) was dissolved in tetrahydrofuran (2 mL). The reaction system was cooled to -78°C, with *N,N*-diisopropylethylamine (86 mg, 1.056 mmol), (*E*)-4-oxopent-2-enoic acid (22 mg, 0.194 mmol) and 50% $T_3P$ ethyl acetate solution (224 mg, 0.352 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). (*E*)-2,4-Difluoro-N-(2-(methylamino)-5-(4-(4-(4-oxopent-2-enoyl)piperazin-1-yl)quinazolin-6-yl)pyridin-3-yl)benzenesulfonamide was obtained, yield: 16.7%; $^1H$ NMR (400 MHz, DMSO-$d_6$) $\delta$ 8.64 (s, 1H), 8.23 (s, 1H), 7.94 (s, 1H), 7.89-7.79 (m, 3H), 7.54-7.43 (m, 3H), 7.23-7.19 (m, 1H), 6.74 (d, *J* = 16.0 Hz, 1H), 3.89-3.78 (m, 8H), 2.86 (s, 3H), 2.37 (s, 3H); ESI-MS(m/z): 608.0 $[M+H]^+$.

Referring to the preparation method of Example 105 and using the corresponding raw materials, the compounds in the following examples were prepared.

| Number | Name | Structure | $^1$H NMR and MS |
|---|---|---|---|
| **Example 106** | *N*-(5-(4-(4-acryloylpiperazin-1-yl) quinazolin-6-yl)-2-(cyclopentyloxy)pyri din-3-yl)-2,4-difluorobenzenesulfo namide | 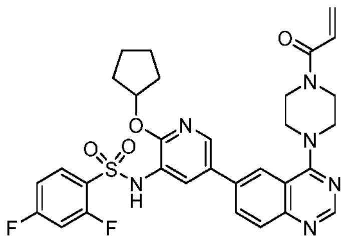 | $^1$H NMR (400 MHz, DMSO-$d_6$) δ 8.63 (s, 1H), 8.06-7.97 (m, 3H), 7.89-7.77 (m, 3H), 7.34-7.29 (m, 1H), 7.11-7.07 (m, 1H), 6.86-6.79 (m, 1H), 6.17 (d, *J* = 16.0 Hz, 1H), 5.73 (d, *J* = 16.0 Hz, 1H), 5.33-5.28 (m, 1H), 3.83-3.78 (m, 9H), 1.94-1.87 (m, 2H), 1.70-1.53 (m, 6H); ESI-MS(m/z): 621.7 [M+H]$^+$. |
| **Example 107** | (*E*)-*N*-(2-(cyclopentyloxy)-5-(4-(4-(4-oxopent-2-enoyl)piperazin-1-yl) quinazolin-6-yl)pyridin-3-yl)-2,4-difluorobenzenesulfo namide | 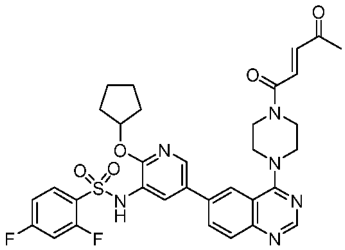 | $^1$H NMR (400 MHz, DMSO-$d_6$) δ 8.66 (s, 1H), 8.46 (s, 1H), 8.14-8.09 (m, 2H), 8.04-7.91 (m, 1H), 7.92 (d, *J* = 8.0 Hz, 1H), 7.81-7.75 (m, 1H), 7.58-7.52 (m, 1H), 7.44 (d, *J* = 16.0 Hz, 1H), 7.23-7.19 (m, 1H), 6.74 (d, *J* = 16.0 Hz, 1H), 5.28-5.23 (m, 1H), 3.90-3.80 (m, 9H), 2.37 (s, 3H), 1.87-1.78 (m, 2H), 1.64-1.51 (m, 2H), 1.49-1.45 (m, 4H); ESI-MS (m/z): 663.7 [M+H]$^+$. |
| **Example 108** | *N*-(2-(cyclopentyloxy)-5-(4-(4-(2-fluoroacryloyl) pipera zin-1-yl)quinazolin-6-yl)pyridin-3 -yl)-2,4-difluorobenzenesulfo namide | 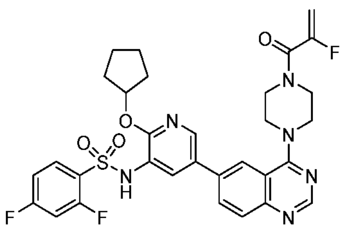 | $^1$H NMR (400 MHz, DMSO-$d_6$) δ 10.15 (s, 1H), 8.65 (s, 1H), 8.44 (s, 1H), 8.12-8.09 (m, 2H), 8.02 (d, *J* = 4.0 Hz, 1H), 7.91 (d, *J* = 8.0 Hz, 1H), 7.79-7.74 (m, 1H), 7.56-7.50 (m, 1H), 7.22-7.17 (m, 1H), 5.36-7.31 (m, 1H), 5.25-5.19 (m, 1H), 3.91-3.79 (m, 9H), 1.86-1.78 (m, 2H), 1.61-1.51 (m, 2H), 1.49-1.44 (m, 4H); ESI-MS(m/z): 639.7 [M+H]$^+$. |
| **Example 109** | (*E*)-*N*-(2-ethoxy-5-(4-(4-(4-oxopent-2-enoyl) piperazin-1-yl)quinazolin-6-yl)pyridin-3-yl)-2,4-difluorobenzenesulfo namide | 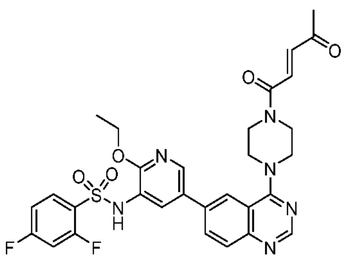 | $^1$H NMR (400 MHz, DMSO-$d_6$) δ 10.26 (s, 1H), 8.66 (s, 1H), 8.45 (d, *J* = 4.0 Hz, 1H), 8.14 (s, 1H), 8.10 (d, *J* = 8.0 Hz, 1H), 8.03 (d, *J* = 4.0 Hz, 1H), 7.92 (d, *J* = 8.0 Hz, 1H), 7.81-7.75 (m, 1H), 7.59-7.54 (m, 1H), 7.45 (d, *J* = 16.0 Hz, 1H), 7.23-7.18 (m, 1H), 6.74 (d, *J* = 16.0 Hz, 1H), 4.19-4.13 (m, 2H), 3.90-3.81 (m, 8H), 2.37 (s, 3H), 1.13 (t, *J* = 8.0 Hz, 3H); ESI-MS (m/z): 623.5 [M+H]$^+$. |
| **Example 110** | (*E*)-*N*-(2-cyclopropoxy-5-(4-(4-(4-oxopent-2-enoyl)piperazin-1-yl) quinazolin-6-yl)pyridin-3-yl)-2,4-difluorobenzenesulfo | 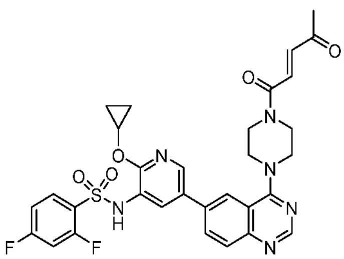 | $^1$H NMR (400 MHz, DMSO-$d_6$) δ 10.25 (s, 1H), 8.67 (s, 1H), 8.51 (d, *J* = 4.0 Hz, 1H), 8.15 (s, 1H), 8.11 (d, *J* = 4.0 Hz, 1H), 8.03 (d, *J* = 4.0 Hz, 1H), 7.92 (d, *J* = 8.0 Hz, 1H), 7.76-7.70 (m, 1H), 7.61-7.56 (m, 1H), 7.45 (d, *J* = 16.0 Hz, 1H), 7.23-7.18 (m, 1H), 6.74 (d, *J* = 16.0 Hz, 1H), 4.17-4.13 (m, 1H), 3.90- |

(continued)

| Number | Name | Structure | $^1$H NMR and MS |
|---|---|---|---|
| | namide | | 3.81 (m, 8H), 2.37 (s, 3H), 0.69-. 0.64 (m, 2H), 0.35-0.31 (m, 2H); ESI-MS(m/z): 635.5 [M+H]$^+$. |

**Example 111**

**Preparation of (*E*)-2-fluoro-*N*-(2-methoxy-5-(4-(4-(4-oxopent-2-enoyl)piperazin-1-yl)quinazoline-6-(yl)pyridin-3-yl)-4-methylbenzenesulfonamide**

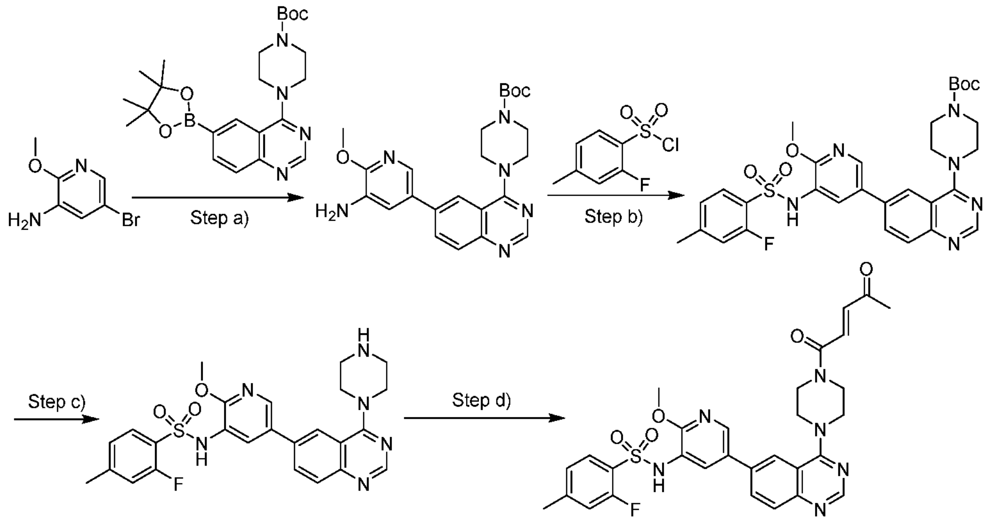


Step a): Preparation of tert-butyl 4-(6-(5-amino-6-methoxypyridin-3-yl)quinazolin-4-yl)piperazine-1-carboxylate

5-bromo-2-methoxypyridin-3-amine (1.0 g, 4.932 mmol), tert-butyl 4-(6-(4,4,5,5-tetramethyl-1,3,2-dioxaborolan-2-yl)quinazolin-4-yl)piperazine-1-carboxylate (2.2 g, 4.932 mmol), Pd(dppf)$Cl_2$ (361 mg, 0.493 mmol) and cesium carbonate (4.8 g, 14.795 mmol) were added to dioxane (45 mL) and water (15 mL) mixed solvent. After the addition was completed, in the presense of protective nitrogen, the reaction mixture was heated to 100°C and stirred for 13 h. Upon completion of the reaction, the reaction mixture was cooled to room temperature and the reaction was quenched by adding saturated ammonium chloride (80 mL). The reaction mixture was extracted with ethyl acetate (80 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 5/1 to 1/2). Tert-butyl 4-(6-(5-amino-6-methoxypyridin-3-yl)quinazolin-4-yl)piperazine-1-carboxylate was obtained, yield: 77.4%; ESI-MS(m/z): 437.1 [M+H]$^+$.

Step b): Preparation of tert-butyl 4-(6-(5-((3-fluoro-5-methyl-2-sulfamoylbenzyl)oxy)pyridin-3-yl)quinazolin-4-yl)piperazine-1-carboxylate

Tert-butyl 4-(6-(5-amino-6-methoxypyridin-3-yl)quinazolin-4-yl)piperazine-1-carboxylate (150 mg, 0.340 mmol), 2-fluoro-4-methylbenzenesulfonyl chloride (78 mg, 0.372 mmol), 4-dimethylaminopyridine (4 mg, 0.034 mmol) and pyridine (81 mg, 1.023 mmol) were dissolved in dichloromethane (10 mL). After the addition was completed, the reaction mixture was stirred at room temperature for 18 h. Upon completion of the reaction, the reaction was quenched by adding saturated aqueous ammonium chloride solution (30 mL) and the reaction mixture was extracted with dichloromethane (30 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 10/1 to 0/1). Tert-butyl 4-(6-(5-((3-fluoro-5-methyl-2-sulfamoylbenzyl)oxy)pyridin-3-yl)quinazolin-4-yl)piperazine-1-carboxylate was obtained, yield: 38.3%; ESI-MS(m/z): 609.2 [M+H]$^+$.

Step c): Preparation of 2-fluoro-*N*-(2-methoxy-5-(4-(piperazin-1-yl)quinazolin-6-yl)pyridin-3-yl)-4-methylbenzenesulfonamide trifluoroacetate

Tert-butyl 4-(6-(5-((3-fluoro-5-methyl-2-sulfamoylbenzyl)oxy)pyridin-3-yl)quinazolin-4-yl)piperazine-1-carboxylate (90 mg, 0.132 mmol) was dissolved in dichloromethane (6 mL). Under the ice bath condition, TFA (2 mL) was slowly added dropwise. After the addition was completed, the reaction mixture was stirred at room temperature for 1 h. Upon completion of the reaction, the reaction mixture was concentrated under reduced pressure. The product obtained was dissolved in 4 mL methyl tert-butyl ether. The reaction mixture was stirred at room temperature for 0.5 h, after solid precipitation appeared, filtered to obtain 2-fluoro-*N*-(2-methoxy-5-(4-(piperazin-1-yl)quinazolin-6-yl)pyridin-3-yl)-4-methylbenzenesulfonamide trifluoroacetate, yield: 97.3%; ESI-MS(m/z): 509.3 $[M+H]^+$.

Step d): Preparation of (*E*)-2-fluoro-*N*-(2-methoxy-5-(4-(4-(4-oxopent-2-enoyl)piperazin-1-yl)quinazoline-6-(yl)pyridin-3-yl)-4-methylbenzenesulfonamide

2-fluoro-*N*-(2-methoxy-5-(4-(piperazin-1-yl)quinazolin-6-yl)pyridin-3-yl)-4-methylbenzenesulfonamide trifluoroacetate (122 mg, 0.132 mmol), (*E*)-4-oxopent-2-enoic acid (16 mg, 0.143 mmol), *N*,*N*-diisopropylethylamine (84 mg, 0.654 mmol) were dissolved in tetrahydrofuran (2 mL), at -78°C, with 1-propylphosphonic anhydride (91 mg, 0.143 mmol, 50% wt%) slowly added. Upon completion of the reaction, with 1 mL acetonitrile added, the reaction solution was filtered and purified by Prep-HPLC (Method 2). (*E*)-2-fluoro-N-(2-methoxy-5-(4-(4-(4-oxopent-2-enoyl)piperazin-1-yl)quinazoline-6-(yl)pyridin-3-yl)-4-methylbenzenesulfonamide was obtained, yield: 45.8%; $^1$H NMR (400 MHz, DMSO-$d_6$) δ 10.17 (s, 1H), 8.67 (s, 1H), 8.46-8.40 (m, 1H), 8.13-8.03 (m, 2H), 7.98 (d, *J* = 2.4 Hz, 1H), 7.92 (d, *J* = 8.8 Hz, 1H), 7.59 (t, *J* = 7.9 Hz, 1H), 7.46 (d, *J* = 15.6 Hz, 1H), 7.29 (d, *J* = 11.2 Hz, 1H), 7.16-7.09 (m, 1H), 6.74 (d, *J* = 15.6 Hz, 1H), 3.93-3.78 (m, 8H), 3.69 (s, 3H), 2.37-2.35 (m, 6H); ESI-MS(m/z): 605.0 $[M+H]^+$.

## Example 112

### Preparation of (*S*,*E*)-*N*-(5-(4-(3-(cyanomethyl)-4-(4-oxopent-2-enoyl)piperazin-1-yl)quinazolin-6-yl)-2-methoxypyridin-3-yl)-2,4-difluorobenzenesulfonamide

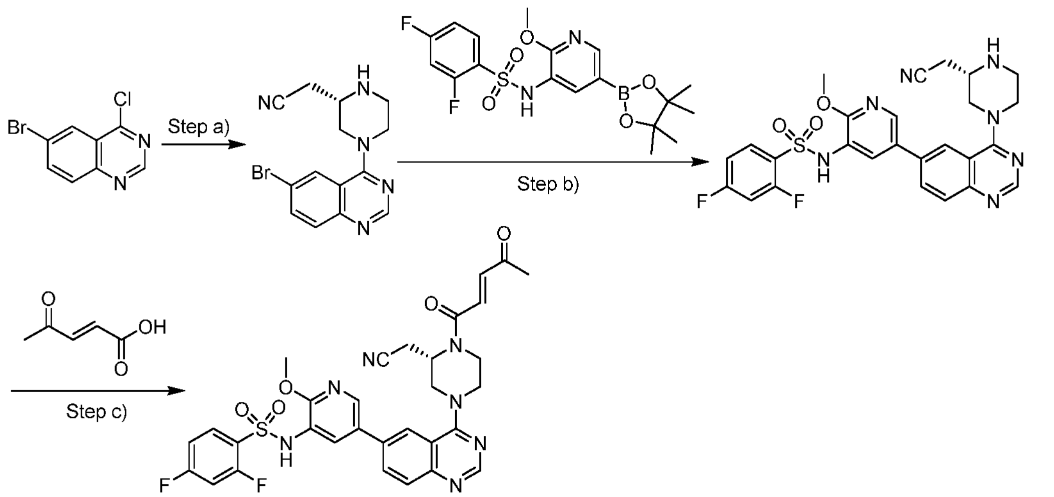


Step a) Preparation of (*S*)-2-(4-(6-bromoquinazolin-4-yl)piperazin-2-yl)acetonitrile

6-bromo-4-chloroquinazoline (600 mg, 2.464 mmol) and (*S*)-2-(piperazin-2-yl)acetonitrile dihydrochloride (537 mg, 2.711 mmol) were dissolved in DMF (8 mL), with *N*,*N*-diisopropylethylamine (955 mg, 7.389 mmol) added. After the addition was completed, the reaction mixture was kept at 50°C and stirred for 1 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 1/1 to 1/5). (*S*)-2-(4-(6-bromoquinazolin-4-yl)piperazin-2-yl)acetonitrile was obtained, yield: 95.2%; ESI-MS(m/z): 332.0 $[M+H]^+$.

Step b): Preparation of (*S*)-*N*-(5-(4-(3-(cyanomethyl)piperazin-1-yl)quinazolin-6-yl)-2-methoxypyridin-3-yl)-2,4-difluorobenzenesulfonamide

(*S*)-2-(4-(6-bromoquinazolin-4-yl)piperazin-2-yl)acetonitrile (780 mg, 2.350 mmol), 2,4-difluoro-*N*-(2-hydroxy-5-(4,4,5,5-tetramethyl-1,3,2-dioxaborolan-2-yl)pyridin-3-yl)benzenesulfonamide (1.1 g, 2.581 mmol), Pd(dppf)$Cl_2$ (172 mg, 0.235 mmol) and cesium carbonate (2.3 g, 7.059 mmol) were dissolved in dioxane/water mixed solvent (8 mL, v/v = 5:1). After the addition was completed, in the presense of protective nitrogen, the reaction mixture was heated to 100°C and stirred for 1 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The crude product obtained was purified by silica gel column chromatography (Eluent: dichloromethane/methanol = 50/1 to 20/1). (*S*)-*N*-(5-(4-(3-(cyanomethyl)piperazin-1-yl)quinazolin-6-yl)-2-methoxypyridin-3-yl)-2,4-difluorobenzenesulfonamide was obtained, yield: 77.2%; ESI-MS(m/z): 552.2 $[M+H]^+$.

Step c): Preparation of (*S*,*E*)-*N*-(5-(4-(3-(cyanomethyl)-4-(4-oxopent-2-enoyl)piperazin-1-yl)quinazolin-6-yl)-2-methoxypyridin-3-yl)-2,4-difluorobenzenesulfonamide

(*S*)-*N*-(5-(4-(3-(cyanomethyl)piperazin-1-yl)quinazolin-6-yl)-2-methoxypyridin-3-yl)-2,4-difluorobenzenesulfonamide (120 mg, 0.217 mmol) and 3-acetylacrylic acid (50 mg, 0.438 mmol) were dissolved in THF (2 mL). The reaction system was cooled to -78°C, with *N*,*N*-diisopropylethylamine (85 mg, 0.658 mmol) and 1-propylphosphonic anhydride (415 mg, 0.653 mmol, 50%wt) added successively. After the addition was completed, the reaction mixture was kept at -78°C and stirred for 1 h. Upon completion of the reaction, the reaction was quenched by adding water (10 mL), and the reaction mixture was extracted with dichloromethane (30 mL×2). The organic phases were combined, dried over anhydrous sodium sulfate and filtered. The filtrate was concentrated under reduced pressure. The crude product obtained was purified by Prep-HPLC (Method 2). (*S,E*)-*N*-(5-(4-(3-(cyanomethyl)-4-(4-oxopent-2-enoyl)piperazin-1-yl)quinazolin-6-yl)-2-methoxypyridin-3-yl)-2,4-difluorobenzenesulfonamide was obtained, yield: 33.0%; $^1$H NMR (400 MHz, DMSO-$d_6$) δ 10.30 (s, 1H), 8.70-8.66 (m, 1H), 8.49 (d, *J* = 8.0 Hz, 1H), 8.21 (s, 1H), 8.13-8.07 (m, 1H), 8.05-8.00 (m, 1H), 7.93 (d, *J* = 8.0 Hz, 1H), 7.81-7.73 (m, 1H), 7.59-7.40 (m, 2H), 7.24-7.17 (m, 1H), 6.82-6.69 (m, 1H), 5.07-4.89 (m, 1H), 4.49-4.25 (m, 2H), 4.18-3.85 (m, 1H), 3.68 (s, 3H), 3.60-3.37 (m, 3H), 3.25-3.00 (m, 2H), 2.40-2.35 (m, 3H); ESI-MS(m/z): 648.0 $[M+H]^+$.

Referring to the preparation method of Example 112 and using the corresponding raw materials, the compounds in the following examples were prepared.

| Number | Name | Structure | $^1$H NMR and MS |
|---|---|---|---|
| **Example 113** | (*S*)-*N*-(5-(4-(3-(cyanomethyl)-4-(2-fluoroacryloyl)pipera zin-1-yl)quinazolin-6-yl)-2-methoxypyridin-3-yl)-2,4-difluorobenzenesulfo namide | 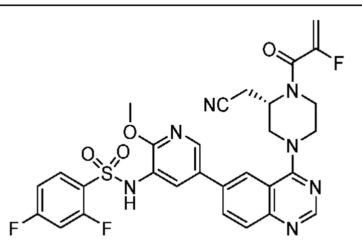 | $^1$H NMR (400 MHz, DMSO-$d_6$) δ 10.33 (s, 1H), 8.69 (s, 1H), 8.52-8.50 (m, 1H), 8.21-8.18 (m, 1H), 8.12-8.08 (m, 1H), 8.05-8.03 (m, 1H), 7.94 (d, J = 8.0 Hz, 1H), 7.81-7.73 (m, 1H), 7.60-7.53 (m, 1H), 7.24-7.18 (m, 1H), 5.44-5.37 (m, 1H), 5.36-5.21 (m, 1H), 4.92 (s, 1H), 4.37-4.27 (m, 2H), 4.17-3.78 (m, 2H), 3.68 (s, 3H), 3.50-3.44 (m, 1H), 3.41-3.31 (m, 2H), 3.10-3.03 (m, 1H); ESI-MS (m/z): 624.0 [M+H]+. |

(continued)

| Number | Name | Structure | $^1$H NMR and MS |
|---|---|---|---|
| **Example 114** | (*S*)-*N*-(5-(4-(4-acryloyl-3-(cyanomethyl)pipera zin-1-yl)quinazolin-6-yl)-2-methoxypyridin-3-yl)-2,4-difluorobenzenesulfo namide | 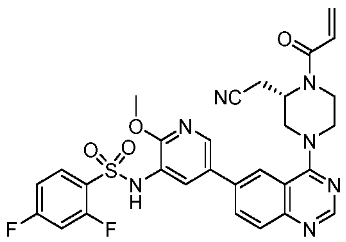 | $^1$H NMR (400 MHz, DMSO-$d_6$) δ 10.31 (s, 1H), 8.67 (s, 1H), 8.42 (m, 1H), 8.20-8.17 (m, 1H), 8.07 (d, *J* = 8.0 Hz, 1H), 8.01-7.97 (m, 1H), 7.92 (d, *J* = 8.0 Hz, 1H), 7.81-7.74 (m, 1H), 7.55-7.49 (m, 1H), 7.22-7.16 (m, 1H), 6.95-6.78 (m, 1H), 6.22-6.16 (m, 1H), 5.78 (d, *J* = 8.0 Hz, 1H), 5.07-4.82 (m, 1H), 4.49-4.20 (m, 3H), 4.15-3.79 (m, 1H), 3.69 (s, 3H), 3.60-3.41 (m, 2H), 3.19-2.98 (m, 2H); ESI-MS(m/z): 606.0 [M+H]$^+$. |
| **Example 115** | (*S*)-*N*-(5-(4-(3-(cyanomethyl)-4-(2-fluoroacryloyl)pipera zin-1-yl)quinazolin-6-yl)-2-methoxypyridin-3-yl)-2,6-difluorobenzenesulfo namide | 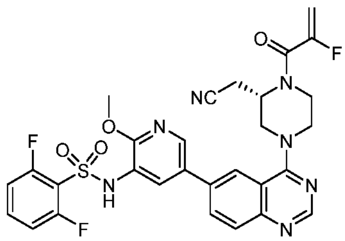 | $^1$H NMR (400 MHz, DMSO-$d_6$) δ 10.59 (s, 1H), 8.69 (s, 1H), 8.54 (d, *J* = 2.4 Hz, 1H), 8.21 (d, *J* = 2.0 Hz, 1H), 8.14-8.04 (m, 2H), 7.95 (d, *J* = 8.8 Hz, 1H), 7.76-7.68 (m, 1H), 7.32-7.24 (m, 2H), 5.48-5.20 (m, 2H), 4.94-4.92 (m, 1H), 4.40-4.24 (m, 2H), 4.09-3.96 (m, 2H), 3.65 (s, 3H), 3.54-3.35 (m, 3H), 3.08-3.03 (m, 1H); ESI-MS (m/z): 624.0 [M+H]+. |
| **Example 116** | *(S,E)-N*-(5-(4-(3-(cyanomethyl)-4-(4-oxopent-2-enoyl)piperazin-1 -yl)quinazolin-6-yl)-2-methoxypyridin-3-yl)-2,6-difluorobenzenesulfo namide | 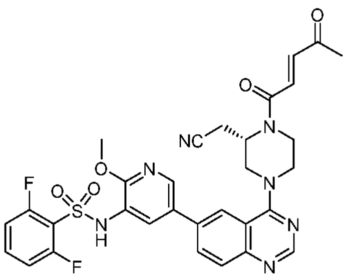 | $^1$H NMR (400 MHz, DMSO-$d_6$) δ 10.58 (s, 1H), 8.69 (d, *J* = 4.8 Hz, 1H), 8.52 (d, *J* = 10.4 Hz, 1H), 8.22 (s, 1H), 8.15-8.01 (m, 2H), 7.95-7.93 (m, 1H), 7.75-7.68 (m, 1H), 7.56-7.42 (m, 1H), 7.27 (t, *J* = 9.6 Hz, 2H), 6.81-6.70 (m, 1H), 5.03-4.91 (m, 1H), 4.49-4.24 (m, 3H), 4.20-3.82 (m, 1H), 3.65 (d, *J* = 4.4 Hz, 3H), 3.60-3.35 (m, 2H), 3.24-3.17 (m, 1H), 3.08-3.01 (m, 1H), 2.38 (d, *J* = 12.4 Hz, 3H); ESI-MS(m/z): 648.0 [M+H]$^+$. |

(continued)

| Number | Name | Structure | $^1H$ NMR and MS |
|---|---|---|---|
| **Example 117** | (*S*)-*N*-(5-(4-(4-acryloyl-3-(cyanomethyl)pipera zin-1-yl)quinazolin-6-yl)-2-methoxypyridin-3-yl)-2,6-difluorobenzenesulfo namide | 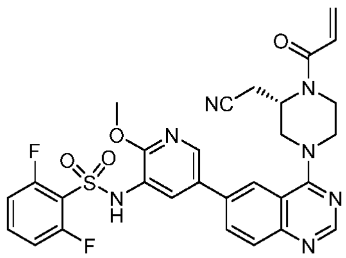 | $^1H$ NMR (400 MHz, DMSO-$d_6$) δ 10.58 (s, 1H), 8.68 (s, 1H), 8.46-8.39 (m, 1H), 8.26-8.14 (m, 1H), 8.12-7.98 (m, 2H), 7.93 (d, *J* = 8.8 Hz, 1H), 7.72-7.68 (m, 1H), 7.25 (t, *J* = 9.2 Hz, 2H), 6.95-6.82 (m, 1H), 6.26-6.15 (m, 1H), 5.78 (d, *J* = 10.4 Hz, 1H), 5.05-4.76 (m, 1H), 4.53-4.18 (m, 3H), 4.10-3.78 (m, 1H), 3.66 (s, 3H), 3.61-3.38 (m, 2H), 3.21-2.94 (m, 2H); ESI-MS(m/z): 606.0 [M+H]+. |

**Example 118**

**Preparation of (*E*)-5-fluoro-*N*-(2-methoxy-5-(4-(4-(4-oxopent-2-enoyl)piperazin-1-yl)quinazolin-6-yl)pyridin-3-yl)pyridine-2-sulfonamide**

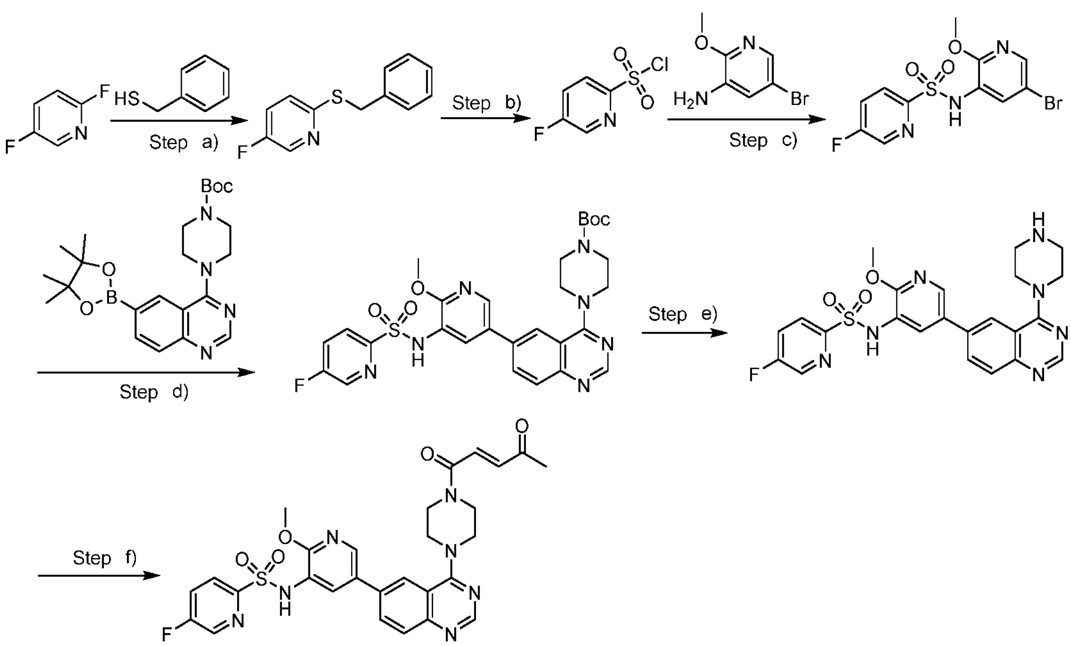


Step a): Preparation of 2-(benzylthio)-5-fluoropyridine

2,5-difluoropyridine (1.15g, 10.000 mmol), phenylmethanethiol (1.118g, 9.000 mmol), $Cs_2CO_3$ (3.26g, 10.000 mmol) and DMSO (30 mL) were added to the reaction flask. The reaction mixture was stirred and reacted for 2 h at room temperature. Upon completion of the reaction, the reaction was quenched by adding water (150 mL). The reaction mixture was extracted with ethyl acetate(100 mL×3). The organic phases were combined, washed with saturated brine (100 mL), dried over anhydrous sodium sulfate, completely concentrated under reduced pressure. The residue was purified by silica gel column chromatography (Eluent: petroleum ether/ethyl acetate = 100/1). 2-(Benzylthio)-5-fluoropyridine was obtained, yield: 55.5%; ESI-MS (m/z): 220.2 [M+H]+.

Step b): Preparation of 5-fluoropyridine-2-sulfonyl chloride

2-(Benzylthio)-5-fluoropyridine (1.217g, 5.550 mmol) and acetonitrile (20 mL) were added to the reaction flask, stirred to dissolve, with glacial acetic acid (1.2mL) and water (700 uL) added. At 0°C, under the condition of stirring, 1,3-dichloro-5,5-dimethylimidazolidine-2,4-dione (2.187g, 11.100 mmol) was slowly added to the system. After the addition was completed, the reaction mixture was stirred and reacted for 2 h at 0°C. After completion of the reaction, the reaction was quenched with saturated brine (100 mL). The reaction mixture was extracted with ethyl acetate (100 mL×3). The organic phases were combined, dried over anhydrous sodium sulfate, completely concentrated under reduced pressure. 5-fluoropyridine-2-sulfonyl chloride was obtained. The product can be used directly in the next reaction without further purification.

Step c): Preparation of *N*-(5-bromo-2-methoxypyridin-3-yl)-5-fluoropyridine-2-sulfonamide

5-fluoropyridine-2-sulfonyl chloride (1085 mg, 5.550 mmol), 5-bromo-2-methoxypyridin-3-amine (1127 mg, 5.550 mmol), pyridine (1305mg, 16.65mmol) and DMAP (870 mg, 6.720 mmol) were added to dichloromethane (20 mL). The reaction solution was stirred at room temperature for 16 h. Upon completion of the reaction, the reaction was quenched by adding water (50 mL). The reaction mixture was extracted with ethyl acetate (50 mL×3). The organic phases were combined, washed with saturated brine (50 mL), dried over anhydrous sodium sulfate and filtered. The filtrate was concentrated under reduced pressure. The residue was purified by silica gel column chromatography (Eluent: petroleum ether/ethyl acetate = 5/1). *N*-(5-bromo-2-methoxypyridin-3-yl)-5-fluoropyridine-2-sulfonamide was obtained, yield: 59.0 %; ESI-MS (m/z): 362.2 $[M+H]^+$.

Step d): Preparation of tert-butyl 4-(6-(5-((5-fluoropyridine)-2-sulfonamido)-6-methoxypyridin-3-yl)quinazolin-4-yl)piperazine-1-carboxylate

Tert-butyl 4-(6-(4,4,5,5-tetramethyl-1,3,2-dioxaborolan-2-yl)quinazolin-4-yl)piperazine-1-carboxylate (594 mg, 1.350 mmol), *N*-(5-bromo-2-methoxypyridin-3-yl)-5-fluoropyridine-2-sulfonamide (489 mg, 1.350 mmol), $Cs_2CO_3$ (880 mg, 2.700 mmol), $Pd(dppf)Cl_2$ (98 mg, 0.135 mmol), water (1 mL) and 1,4-dioxane (10 mL) were added to the reaction flask. With nitrogen purged three times, the reaction mixture was heated to 90°C, stirred and reacted for 2 h. Upon completion of the reaction, the reaction was quenched by adding water (100 ml). The reaction mixture was extracted with ethyl acetate (50 mL×3). The organic phases were combined, washed with saturated brine (50 mL), concentrated under reduced pressure. The residue was purified by silica gel column chromatography (Eluent: dichloromethane/methanol = 50/1). Tert-butyl 4-(6-(5-((5-fluoropyridine)-2-sulfonamido)-6-methoxypyridin-3-yl)quinazolin-4-yl)piperazine-1-carboxylate was obtained, yield: 72.1 %; ESI-MS (m/z): 596.5 $[M+H]^+$.

Step e): Preparation of 5-fluoro-*N*-(2-methoay-5-(4-(piperazin-1-yl)quinazolin-6-yl)pyridin-3-yl)pyridine-2-sulfonamide trifluoroacetate

Tert-butyl 4-(6-(5-((5-fluoropyridine)-2-sulfonamido)-6-methoxypyridin-3-yl)quinazolin-4-yl)piperazine-1-carboxylate (476mg, 0.800 mmol) and dichloromethane (5 mL) were added to the reaction flask, stirred to dissolve. At 0°C, under the condition of stirring, HCl/1,4-dioxane solution (4 M, 5 mL) was slowly added to the system. After the addition was completed, the reaction mixture was naturally heated to room temperature and reacted for 1 h. After completion of the reaction, the reaction mixture was concentrated under reduced pressure. The product can be used directly in the next reaction without further purification; ESI-MS (m/z): 496.2 $[M+H]^+$.

Step f): Preparation of (*E*)-5-fluoro-*N*-(2-methoxy-5-(4-(4-(4-oxopent-2-enoyl)piperazin-1-yl)quinazolin-6-yl)pyridin-3-yl)pyridine-2-sulfonamide

5-fluoro-*N*-(2-methoxy-5-(4-(piperazin-1-yl)quinazolin-6-yl)pyridin-3-yl)pyridine-2-sulfonamide trifluoroacetate (119 mg, 0.200 mmol), (*E*)-4-oxopent-2-enoic acid (23 mg, 0.200 mmol), *N*,*N*-diisopropylethylamine (129 mg, 1.000 mmol) and tetrahydrofuran (2 mL) were added to the reaction flask. In the dry ice/ethanol bath, at -78°C, $T_3P$ (254 mg, 0.400 mmol, 50% ethyl acetate solution) was added to the reaction system under the condition of stirring. The reaction system was kept at -78°C and reacted for 1 h. Upon completion of the reaction, the reaction was quenched by adding water (20 mL). The reaction mixture was extracted with ethyl acetate (20 mL×2). The organic phases were combined and washed with saturated sodium bicarbonate aqueous solution (20 mL), saturated brine (10 mL×2) successively, dried over anhydrous sodium sulfate and filtered. The filtrate was concentrated under reduced pressure. The residue was purified by silica gel column chromatography (Eluent: dichloromethane/methanol = 20/1). The crude product was then purified by Prep-HPLC (Method 2). (E)-5-fluoro-N-(2-methoxy-5-(4-(4-(4-oxopent-2-enoyl)piperazin-1-yl)quinazolin-6-yl)pyridin-3-yl)pyridine-2-sulfonaniide was obtained, yield: 68.7%; $^1$H NMR (400 MHz, DMSO-$d_6$) δ 10.16 (s, 1H), 8.75 (d, *J* = 4.0 Hz, 1H), 8.67 (s, 1H), 8.41 (s, 1H), 8.13-7.92 (m, 6H), 7.43 (d, *J* = 16.0 Hz, 1H), 6.73 (d, *J* = 16.0 Hz, 1H), 3.90-3.80 (m, 8H), 3.72 (s, 3H), 2.36 (s, 3H); ESI-MS(m/z): 592.6 [M+H]$^+$.

Referring to the preparation method of Example 118 and using the corresponding raw materials, the compounds in the following examples were obtained.

| Number | Name | Structure | $^1$H NMR and MS |
|---|---|---|---|
| **Example 119** | *N*-(5-(4-(4-acryloylpiperazin-1-yl) quinazolin-6-yl)-2-methoxypyridin-3-yl)-5-fluoropyridine-2-sulfonamide | 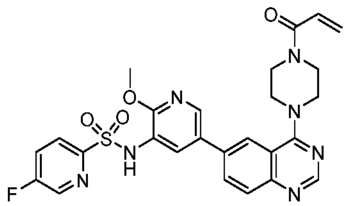 | $^1$H NMR (400 MHz, DMSO-$d_6$) δ 10.21 (s, 1H), 8.75 (d, *J* = 4.0 Hz, 1H), 8.66 (s, 1H), 8.41 (s, 1H), 8.13-7.91 (m, 6H), 6.85-6.78 (m, 1H), 6.16 (d, *J* = 16.0 Hz, 1H), 5.71 (d, *J* = 16.0 Hz, 1H), 3.89-3.78 (m, 8H), 3.72 (s, 3H); ESI-MS(m/z): 550.6 [M+H]$^+$. |
| **Example 120** | 5-fluoro-*N*-(5-(4-(4-(2-fluoroacryloyl)piperazin-1-yl)quinazolin-6-yl)-2-methoxypyridin-3-yl)pyridine-2-sulfonamide | 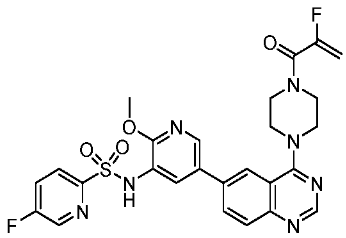 | $^1$H NMR (400 MHz, DMSO-$d_6$) δ 10.20 (s, 1H), 8.75 (d, *J* = 4.0 Hz, 1H), 8.67 (s, 1H), 8.42 (s, 1H), 8.13-7.92 (m, 6H), 5.36-5.19 (m, 2H), 3.92-3.79 (m, 8H), 3.72 (s, 3H); ESI-MS(m/z): 568.6 [M+H]$^+$. |
| **Example 121** | (*E*)-5-fluoro-*N*-(3-fluoro-2-methoxy-5-(4-(4-(4-oxopent-2-enoyl)piperazin-1- yl) quinazolin-6-yl)phenyl) pyridine-2-sulfonamide | 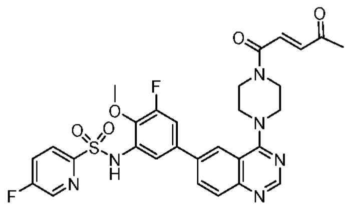 | $^1$H NMR (400 MHz, DMSO-$d_6$) δ 10.39 (s, 1H), 8.84 (s, 1H), 8.79 (s, 1H), 8.24 (s, 1H), 8.22-8.20 (m, 1H), 8.17-8.10 (m, 1H), 8.04-7.94 (m, 1H), 7.92 (d, *J* = 4.0 Hz, 1H), 7.64-7.61 (m, 2H), 7.42 (d, *J* = 16.0 Hz, 1H), 6.76 (d, *J* = 16.0 Hz, 1H), 4.25-4.20 (m, 4H), 4.19-3.93 (m, 2H), 3.84-3.81 (m, 2H), 3.66 (s, 3H), 2.36 (s, 3H); ESI-MS(m/z): 609.0 [M+H]$^+$. |

**Example 122**

**Preparation of (*E*)-2-cyano-6-fluoro-*N*-(2-methoxy-5-(4-(4-(4-oxopent-2-enoyl)piperazin-1-yl)quinazolin-6-yl)pyridin-3-yl)benzenesulfonamide**

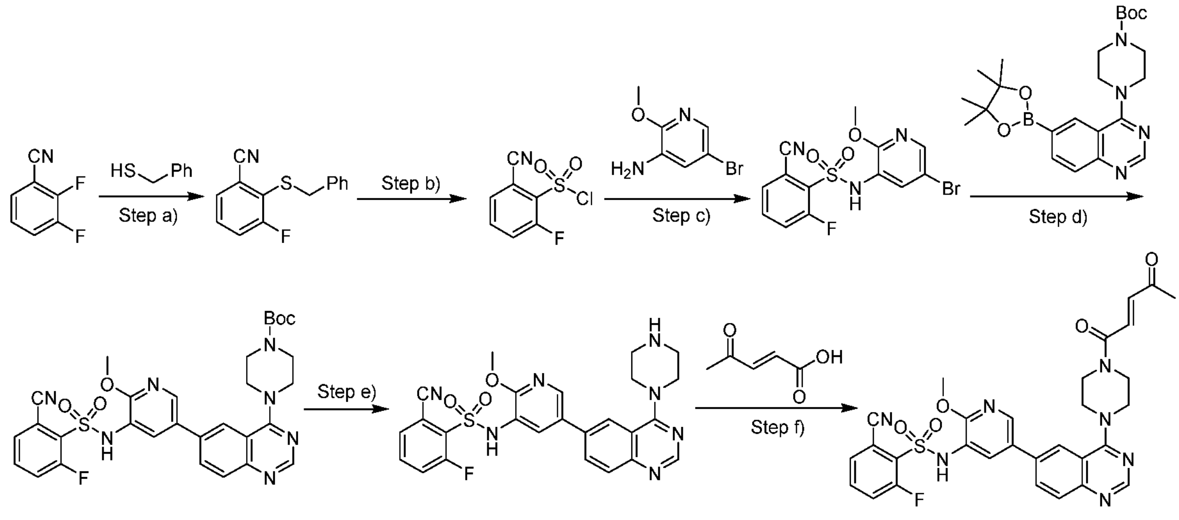


Referring to the preparation method of Example 118, (*E*)-2-cyano-6-fluoro-*N*-(2-methoxy-5-(4-(4-(4-oxopent-2-enoyl)piperazin-1-yl)quinazolin-6-yl)pyridin-3-yl)benzenesulfonamide was obtained, yield: 9.3%; $^1$HNMR (400 MHz, DMSO-$d_6$) $\delta$ 10.89 (s, 1H), 8.68 (s, 1H), 8.42-8.36 (m, 1H), 8.15-8.06 (m, 3H), 7.93-7.82 (m, 4H), 7.46 (d, *J* = 16 Hz, 1H), 6.73 (d, *J* = 16 Hz, 1H), 3.94-3.90 (m, 6H), 3.88-3.81 (m, 2H), 3.59 (s, 3H), 2.37 (s, 3H); ESI-MS(m/z): 616.1 $[M+H]^+$.

**Example 123**

**Preparation of (*E*)-4-cyano-2-fluoro-*N*-(2-methoxy-5-(4-(4-(4-oxopent-2-enoyl)piperazin-1-yl)quinazolin-6-yl)pyridin-3-yl)benzenesulfonamide**

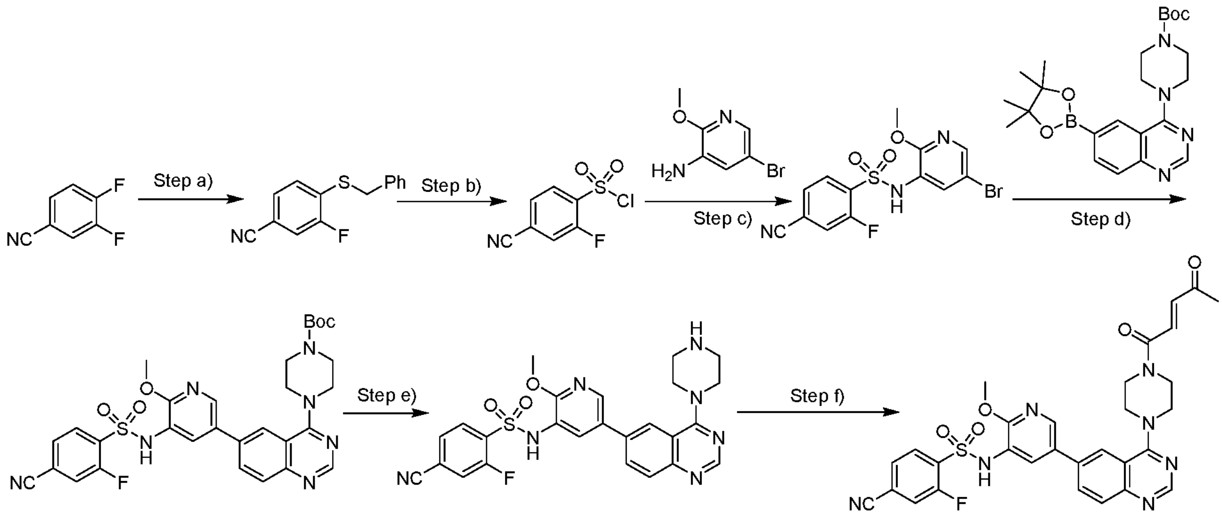


Referring to the preparation method of Example 118, (*E*)-4-cyano-2-fluoro-*N*-(2-methoxy-5-(4-(4-(4-oxopent-2-enoyl)piperazin-1-yl)quinazolin-6-yl)pyridin-3-yl)benzenesulfonamide was obtained, yield: 14.0%; $^1$H NMR (400 MHz, DMSO-$d_6$) $\delta$ 10.67 (s, 1H), 8.67 (s, 1H), 8.52-8.50 (m, 1H), 8.20-8.10 (m, 3H), 8.06 (d, *J* = 8.0 Hz, 1H), 7.93-7.89 (m, 1H), 7.88-7.82 (m, 2H), 7.45 (d, *J* = 16.0 Hz, 1H), 6.74 (d, *J* = 16.0 Hz, 1H), 3.94-3.88 (m, 6H), 3.83-3.78 (m, 2H), 3.61 (s, 3H), 2.37 (s, 3H); ESI-MS(m/z): 616.2 $[M+H]^+$.

**Example 124**

**Preparation of (*E*)-2-fluoro-*N*-(2-methoxy-5-(4-(4-(4-oxopent-2-enoyl)piperazin-1-yl)quinazolin-6-yl)pyridin-3-yl)-4-(trifluoromethyl)benzenesulfonamide**

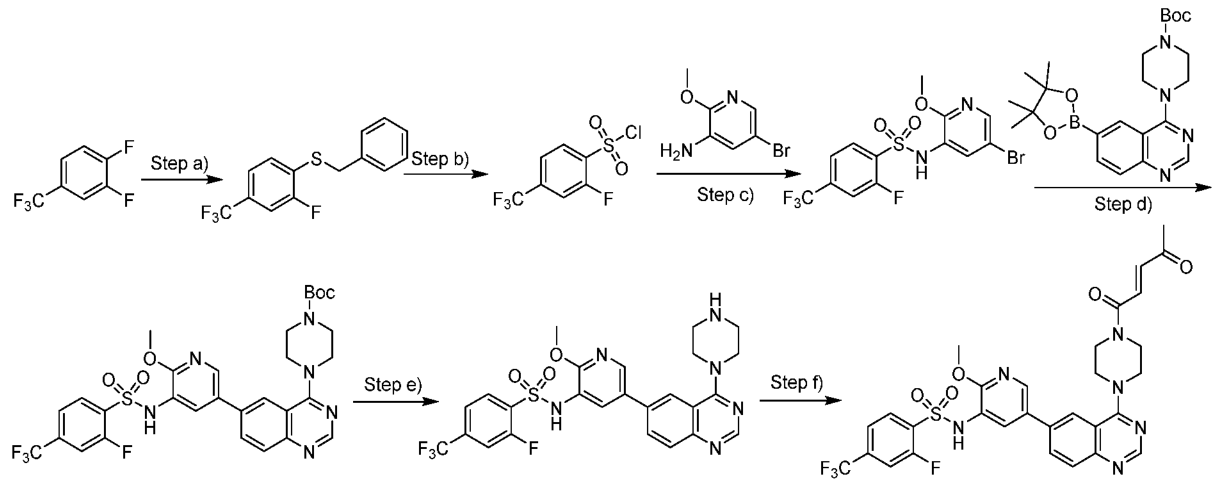


Referring to the preparation method of Example 118, (*E*)-2-fluoro-N-(2-methoxy-5-(4-(4-(4-oxopent-2-enoyl)piperazin-1-yl)quinazolin-6-yl)pyridin-3-yl)-4-(trifluoromethyl)benzenesulfonamide was obtained, yield: 10.9 %; $^1$H NMR (400 MHz, DMSO-$d_6$) δ 8.61 (s, 1H), 8.27 (s, 1H), 8.05-7.99 (m, 2H), 7.91-7.84 (m, 4H), 7.65 (d, *J* =8.0 Hz, 1H), 7.40 (d, *J* =16.0 Hz, 1H), 6.79 (d, *J* =12.0 Hz, 1H), 3.84-3.74 (m, 8H), 3.59 (s, 3H), 2.32 (m, 3H); ESI-MS(m/z): 659.0 [M+H]$^+$.

**Example 125**

**Preparation of 2,4-difluoro-N-(2-methoxy-5-(4-(3-(3-methylene-2-oxopyrrolidin-1-yl))azetidine-1-yl)quinazolin-6-yl)pyridin-3-yl)benzenesulfonamide**

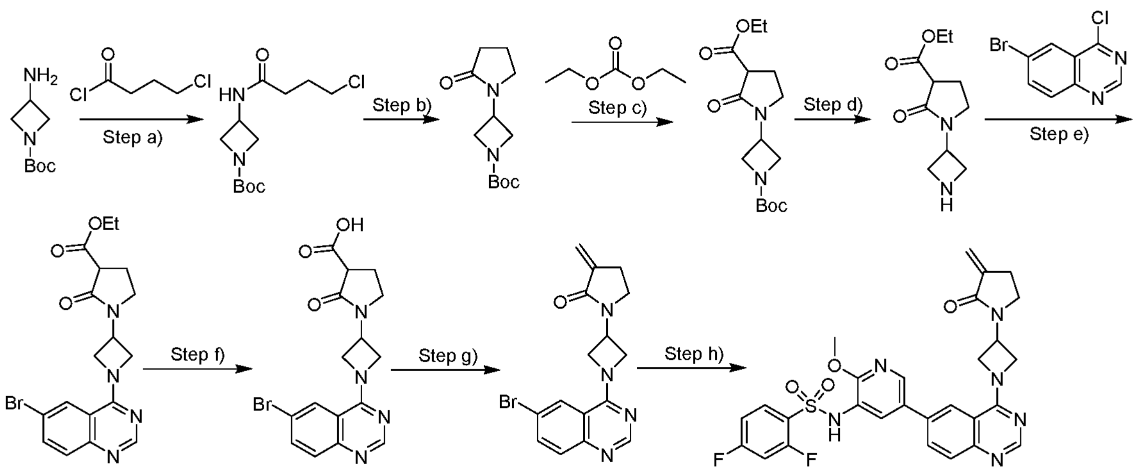


Step a): Preparation of tert-butyl 3-(4-chlorobutanamido)azetidine-1-carboxylate

Tert-butyl 3-aminoazetidine-1-carboxylate (2.0 g, 11.627 mmol) and triethylamine (3.5 g, 34.881 mmol) were dissolved in dichloromethane (30 mL), with 4-chlorobutyryl chloride (2.4 g, 17.441 mmol) added. After the addition was completed, the reaction mixture was kept at room temperature and reacted overnight. Upon completion of the reaction, the reaction was quenched by adding water (50 mL). The reaction mixture was extracted with ethyl acetate (100 mL×2

times). The organic phases were combined, dried over anhydrous sodium sulfate and filtered. The filtrate was concentrated under reduced pressure. The crude product obtained was purified by silica gel column chromatography (Eluent: dichloromethane/methanol = 30/1 to 10/1). Tert-butyl 3-(4-chlorobutanamido)azetidine-1-carboxylate was obtained, yield: 31.1%.

Step b): Preparation of tert-butyl 3-(2-oxopyrrolidin-1-yl)azetidine-1-carboxylate

Tert-butyl 3-(4-chlorobutanamido)azetidine-1-carboxylate (950 mg, 3.430 mmol) was dissolved in tetrahydrofuran (15 mL), with sodium hydride (206 mg, 5.145 mmol) added at 0°C. After the addition was completed, the reaction mixture was slowly heated to room temperature and reacted overnight. Upon completion of the reaction, the reaction was quenched by adding saturated ammonium chloride solution (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. The crude product obtained was purified by chromatography plate (Eluent: petroleum ether/ethyl acetate= 1/1). Tert-butyl 3-(2-oxopyrrolidin-1-yl)azetidine-1-carboxylate was obtained, yield: 97.2%.

Step c): Preparation of ethyl 1-(1-(tert-butoxycarbonyl)azetidin-3-yl)-2-oxopyrrolidine-3-carboxylate

Tert-butyl 3-(2-oxopyrrolidin-1-yl)azetidine-1-carboxylate (500 mg, 2.083 mmol) was dissolved in tetrahydrofuran (12 mL). At -78°C, lithium hexamethyldisilazide (4.2 mL, 4.166 mmol) was slowly added dropwise. After the addition was completed, the reaction mixture was kept at -78°C and stirred for 1 h. Tetrahydrofuran (3 mL) solution of diethyl carbonate (492 mg, 4.166 mmol) was then slowly added dropwise. After the addition was completed, the reaction mixture was kept at -78°C and stirred for 1 h, then slowly heated to 0°C and stirred for 30 min. Upon completion of the reaction, the reaction was quenched by adding saturated ammonium chloride solution (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. Ethyl 1-(1-(tert-butoxycarbonyl)azetidin-3-yl)-2-oxopyrrolidine-3-carboxylate was obtained, yield: 76.9%.

Step d): Preparation of Ethyl 1-(azetidin-3-yl)-2-oxopyrrolidine-3-carboxylate

Ethyl 1-(1-(tert-butoxycarbonyl)azetidin-3-yl)-2-oxopyrrolidine-3-carboxylate (950 mg, 3.430 mmol) was dissolved in dichloromethane (5 mL). At 0°C, HCl dioxane solution (5 mL) was added to the system. After the addition was completed, the reaction mixture was slowly heated to room temperature and reacted for 2 h. Upon completion of the reaction, the reaction mixture was concentrated under reduced pressure. Ethyl 1-(azetidin-3-yl)-2-oxopyrrolidine-3-carboxylate was obtained.

Step e): Preparation of ethyl 1-(1-(6-bromoquinazolin-4-yl)azetidin-3-yl)-2-oxopyrrolidine-3-carboxylate

Ethyl 1-(azetidin-3-yl)-2-oxopyrrolidine-3-carboxylate (400 mg, 1.887 mmol) and *N,N*-diisopropylethylamine (1.2 g, 9.435 mmol) were dissolved in *N,N*-dimethylformamide (30 mL), with 6-bromo-4-chloroquinazoline (688 mg, 2.831 mmol) added. After the addition was completed, the reaction mixture was kept at 60°C and reacted for 3 h. Upon completion of the reaction, the reaction was quenched by adding water (50 mL). The reaction mixture was extracted with ethyl acetate (100 mL×2 times). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 2/1 to 1/2). Ethyl 1-(1-(6-bromoquinazolin-4-yl)azetidin-3-yl)-2-oxopyrrolidine-3-carboxylate was obtained, yield: 63.2%; ESI-MS(m/z): 419.1 $[M+H]^+$.

Step f): Preparation of 1-(1-(6-bromoquinazolin-4-yl)azetidin-3-yl)-2-oxopyrrolidine-3-carboxylic acid

Ethyl 1-(1-(6-bromoquinazolin-4-yl)azetidin-3-yl)-2-oxopyrrolidine-3-carboxylate (400 mg, 1.887 mmol) and lithium hydroxide (501.2 mg, 18.870 mmol) were dissolved in tetrahydrofuran/methanol/water mixed solvent (4:1:1, 10 mL). After the addition was completed, the reaction mixture was kept at room temperature and reacted for 3 h. Upon completion of the reaction, the pH of the reaction solution was adjusted to 3-4 by adding 2 M hydrogen chloride aqueous solution and extracted with dichloromethane (100 mL×2 times). The organic phases were combined, dried over anhydrous sodium sulfate and filtered. The filtrate was concentrated under reduced pressure. The crude product obtained was purified by silica gel column chromatography (Eluent: dichloromethane/methanol = 20/1 to 10/1). 1-(1-(6-Bromoquinazolin-4-yl)azetidin-3-yl)-2-oxopyrrolidine-3-carboxylic acid was obtained, yield: 27.9%; ESI-MS(m/z): 391.2 $[M+H]^+$.

Step g): Preparation of 1-(1-(6-bromoquinazolin-4-yl)azetidin-3-yl)-3-methylenepyrrolidin-2-one

1-(1-(6-Bromoquinazolin-4-yl)azetidin-3-yl)-2-oxopyrrolidine-3-carboxylic acid (100 mg, 0.256 mmol) and paraformaldehyde (34.5 mg, 0.384 mmol) were dissolved in ethyl acetate (5 mL), with dimethylamine tetrahydrofuran solution (0.1 mL, 0.184 mmol) added at 0°C. After the addition was completed, the reaction mixture was kept at 80°C and reacted for 3 h. Upon completion of the reaction, the reaction was quenched by adding water (50 mL). The reaction mixture was extracted with ethyl acetate (100 mL×2 times). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 2/1 to 1/2). 1-(1-(6-Bromoquinazolin-4-yl)azetidin-3-yl)-3-methylenepyrrolidin-2-one was obtained, yield: 43.5%; ESI-MS(m/z): 359.3 $[M+H]^+$.

Step h): Preparation of 2,4-difluoro-*N*-(2-methoxy-5-(4-(3-(3-methylene-2-oxopyrrolidin-1-yl))azetidine-1-yl)quinazolin-6-yl)pyridin-3-yl)benzenesulfonamide

1-(1-(6-Bromoquinazolin-4-yl)azetidin-3-yl)-3-methylenepyrrolidin-2-one (40 mg, 0.111 mmol), 2,4-difluoro-N-(2-hydroxy-5-(4,4,5,5-tetramethyl-1,3,2-dioxaborol-2-yl)pyridin-3-yl)benzene sulfonamide (57.0 mg, 0.133 mmol), $Pd(dppf)Cl_2$ (8.2 mg, 0.011 mmol) and cesium carbonate (109.0 mg, 0.333 mmol) were dissolved in dioxane/water (4:1, 10 mL). After the addition was completed, the reaction mixture was heated to 110°C and stirred for 3 h. Upon completion of the reaction, the reaction was quenched by adding water (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. The crude product obtained was purified by Prep-HPLC (Method 2). 2,4-difluoro-*N*-(2-methoxy-5-(4-(3-(3-methylene-2-oxopyrrolidin-1-yl))azetidine-1-yl)quinazolin-6-yl)pyridin-3-yl)benzenesulfonamide was obtained, yield: 27.7%; $^1H$ NMR (400 MHz, DMSO-$d_6$) δ 10.31 (s, 1H), 8.50 (s, 1H), 8.42 (s, 1H), 8.05-8.01 (m, 2H), 7.95 (s, 1H), 7.82-7.76 (m, 2H), 7.56-7.53 (m, 1H), 7.19-7.16 (m, 1H), 5.79 (s, 1H), 5.38 (s, 1H), 5.18-5.15 (m, 1H), 4.85-4.75(m, 4H), 3.73-3.67(m, 5H), 2.80-2.77 (m, 2H); ESI-MS(m/z): 579.0 $[M+H]^+$.

**Example 126**

**Preparation of 1-(4-(6-(5-((2,4-difluorobenzyl)amino)-6-methoxypyridin-3-yl)quinazolin-4-yl)piperazin-1-yl)prop-2-en-1-one**

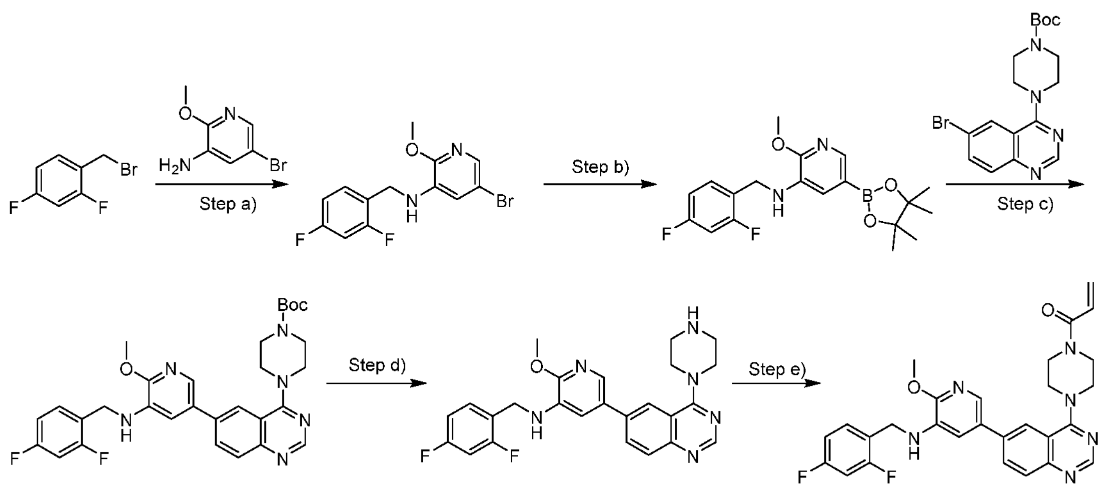


Step a): Preparation of 5-bromo-*N*-(2,4-difluorobenzyl)-2-methoxypyridin-3-amine

1-(Bromomethyl)-2,4-difluorobenzene (500 mg, 2.415 mmol), 5-bromo-2-methoxypyridin-3-amine (491 mg, 2.419 mmol) and potassium carbonate (668 mg, 4.833 mmol) were added to *N*,*N*-dimethylformamide (5 mL). After the addition was completed, the reaction mixture was heated to 50°C and stirred for 2 h. Upon completion of the reaction, the reaction was quenched by adding water (50 mL). The reaction mixture was extracted with ethyl acetate (60 mL×2 times). The organic phases were combined, washed with saturated brine (50 mL), dried over anhydrous sodium sulfate

and filtered. The filtrate was concentrated under reduced pressure. The crude product obtained was purified by silica gel column chromatography (Eluent: petroleum ether/ethyl acetate = 5/1 to 2/1). 5-Bromo-*N*-(2,4-difluorobenzyl)-2-methoxypyridin-3-amine was obtained, yield: 75.5%; ESI-MS(m/z): 329.0 $[M+H]^+$.

Step b): Preparation of *N*-(2,4-difluorobenzyl)-2-methoxy-5-(4,4,5,5-tetramethyl-1,3,2-dioxaborolan-2-yl)pyridin-3-amine

5-Bromo-*N*-(2,4-difluorobenzyl)-2-methoxypyridin-3-amine (600 mg, 1.823 mmol), pinacol diborate (695 mg, 2.737 mmol), $Pd(dppf)Cl_2$ (134 mg, 0.183 mmol) and potassium acetate (358 mg, 3.648 mmol) were added to dioxane (5 mL). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate = 2/1 to 1/1). *N*-(2,4-difluorobenzyl)-2-methoxy-5-(4,4,5,5-tetramethyl-1,3,2-dioxaborolan-2-yl)pyridin-3-amine was obtained, yield: 87.5%; ESI-MS(m/z): 377.2 $[M+H]^+$.

Step c): Preparation of tert-butyl 4-(6-(5-((2,4-difluorobenzyl)amino)-6-methoxypyridin-3-yl)quinazolin-4-yl)piperazine-1-carboxylate

*N*-(2,4-difluorobenzyl)-2-methoxy-5-(4,4,5,5-tetramethyl-1,3,2-dioxaborolan-2-yl)pyridin-3-amine (600 mg, 1.595 mmol), tert-butyl 4-(6-bromoquinazolin-4-yl)piperazine-1-carboxylate (690 mg, 1.754 mmol), $Pd(dppf)Cl_2$ (117 mg, 0.160 mmol) and cesium carbonate (1.0 g, 3.069 mmol) were added to dioxane/water mixed solvent (10 mL, v/v = 5:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2 times). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate = 2/1 to 1/2). Tert-butyl 4-(6-(5-((2,4-difluorobenzyl)amino)-6-methoxypyridin-3-yl)quinazolin-4-yl)piperazine-1-carboxylate was obtained, yield: 22.3%; ESI-MS(m/z): 563.3 $[M+H]^+$.

Step d): Preparation of *N*-(2,4-difluorobenzyl)-2-methoxy-5-(4-(piperazin-1-yl)quinazolin-6-yl)pyridin-3-amine- trifluoroacetate

Tert-butyl 4-(6-(5-((2,4-difluorobenzyl)amino)-6-methoxypyridin-3-yl)quinazolin-4-yl)piperazine-1-carboxylate (70 mg, 0.124 mmol) was dissolved in dichloromethane (2 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. Methyl tert-butyl ether was added to the crude product. The mixture was stirred to precipitate solid and filtered. *N*-(2,4-difluorobenzyl)-2-methoxy-5-(4-(piperazin-1-yl)quinazolin-6-yl)pyridin-3-amine· trifluoroacetate was obtained, yield: 98.5%. ESI-MS(m/z): 463.2 $[M+H]^+$.

Step e): Preparation of 1-(4-(6-(5-((2,4-difluorobenzyl)amino)-6-methoxypyridin-3-yl)quinazolin-4-yl)piperazin-1-yl)prop-2-en-1 -one

Acrylic acid (21 mg, 0.291 mmol) and HATU (44 mg, 0.116 mmol) were dissolved in DMF (2 mL). With the reaction system cooled to -41°C, *N,N*-diisopropylethylamine (62 mg, 0.480 mmol) and *N*-(2,4-difluorobenzyl)-2-methoxy-5-(4-(piperazin-1-yl)quinazolin-6-yl)pyridin-3-amine· trifluoroacetate (55 mg, 0.095 mmol) were added successively. After the addition was completed, the reaction mixture was kept at -41°C and stirred for 1 h. Upon completion of the reaction, the reaction solution was purified by Prep-HPLC (Method 2). 1-(4-(6-(5-((2,4-Difluorobenzyl)amino)-6-methoxypyridin-3-yl)quinazolin-4-yl)piperazin-1-yl)prop-2-en-1-one was obtained, yield: 12.1%; $^1$H NMR (400 MHz, DMSO-$d_6$) $\delta$ 8.63 (s, 1H), 8.05-7.97 (m, 2H), 7.88-7.79 (m, 2H), 7.46-7.39 (m, 1H), 7.23-7.15 (m, 1H), 7.07-6.99 (m, 2H), 6.86-6.78 (m, 1H), 6.21-6.14 (dd, $J_1$ = 16.0 Hz, $J_2$ = 4.0 Hz, 1H), 6.11-5.92 (m, 1H), 5.76-5.72 (dd, $J_1$ = 12.0 Hz, $J_2$ = 4.0 Hz, 1H), 4.51-4.46 (m, 2H), 3.97 (s, 3H), 3.83-3.73 (m, 8H); ESI-MS(m/z): 517.0 $[M+H]^+$.

**Example 127**

**Preparation of *N*-(5-(4-(4-(3-acetyloxirane-2-carbonyl)piperazin-1-yl)quinazolin-6-yl)-2-methoxypyridin-3-yl)-2,4-difluorobenzenesulfonamide**

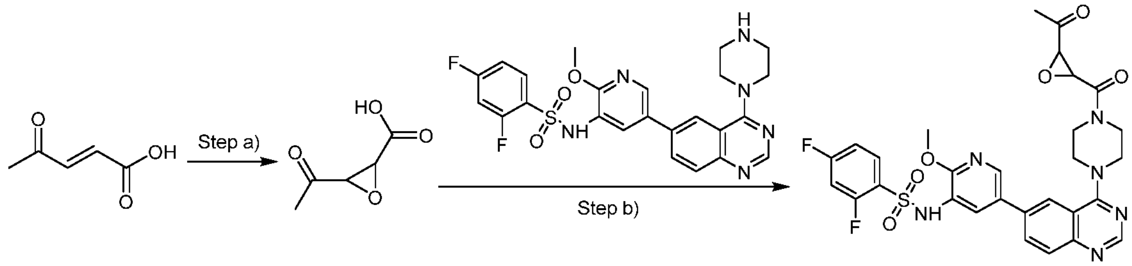


Step a): Preparation of 3-acetyloxirane-2-carboxylic acid

(*E*)-4-oxopent-2-enoic acid (100 mg, 0.876 mmol), potassium carbonate (484 mg, 3.504 mmol) were added to tertbutanol (0.5 ml) and water (0.5 ml) mixed solvent. Under the ice bath condition, hydrogen peroxide (855 mg, 35% wt%) was slowly added to the system. After the addition was completed, the reaction mixture was kept at room temperature and reacted overnight. Upon completion of the reaction, the reaction mixture was adjusted to pH = 1 with hydrochloric acid (2M) under the ice bath condition and extracted with ethyl acetate (50 mL×2 times). The organic phases were combined, washed with saturated brine (50 mL), dried over anhydrous sodium sulfate and filtered. The filtrate was concentrated under reduced pressure. 3-Acetyloxirane-2-carboxylic acid was obtained, yield: 37.3%, ESI-MS(m/z): 129.0 $[M-H]^-$.

Step b): Preparation of *N*-(5-(4-(4-(3-acetyloxirane-2-carbonyl)piperazin-1-yl)quinazolin-6-yl)-2-methoxypyridin-3 -yl)-2,4-difluorobenzenesulfonamide

2,4-difluoro-*N*-(2-methoxy-5-(4-(piperazin-1-yl)quinazolin-6-yl)pyridin-3-yl)benzenesulfonamide trifluoroacetate (80 mg, 0.128 mmol) was dissolved in tetrahydrofuran (2 mL). The reaction system was cooled to -78°C, with *N,N*-diisopropylethylamine (99 mg, 0.768 mmol), 3-acetyloxirane-2-carboxylic acid (17 mg, 0.128 mmol) and 50% $T_3P$ ethyl acetate solution (163 mg, 0.256 mmol) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). *N*-(5-(4-(4-(3-acetyloxirane-2-carbonyl)piperazin-1-yl)quinazolin-6-yl)-2-methoxypyridin-3-yl)-2,4-difluorobenzenesulfonamide was obtained, yield: 25.1%; $^1$H NMR (400 MHz, DMSO-$d_6$) δ 10.37 (brs, 1H), 8.75 (s, 1H), 8.50 (d, *J* = 8.0 Hz, 1H), 8.20-8.16 (m, 2H), 8.04 (d, *J* = 8.0 Hz, 1H), 7.93 (d, *J* = 8.0 Hz, 1H), 7.80-7.74 (m, 1H), 7.62-7.56 (m, 1H), 7.25-7.20 (m, 1H), 4.26 (d, *J* = 8.0 Hz, 1H), 4.05-3.72 (m, 8H), 3.67 (s, 3H), 3.65 (d, *J* = 8.0 Hz, 1H), 2.13 (s, 3H); ESI-MS(m/z): 625.0 $[M+H]^+$.

**Example 128**

***N*-(2,4-difluorophenyl)[(2-methoxy-5-(4-(4-[(2*E*)-4-oxopent-2-enoyl]piperazin-1-yl)quinazolin-6-yl)pyridin-3-yl)amino] sulfonamide**

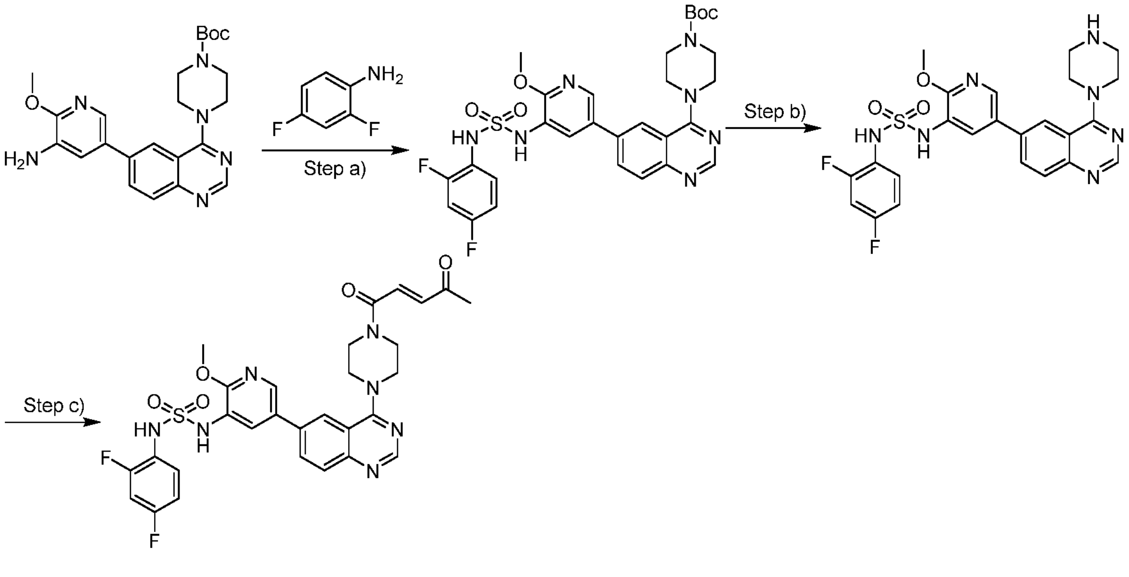


Step a): Preparation of tert-butyl 4-(6-(5-((*N*-(2,4-difluorophenyl)sulfamoyl)amino)-6-methoxypyridin-3-yl)quinazolin-4-yl)piperazine-1-carboxylate

2,4-Difluoroaniline (130 mg, 1.007 mmol), triethylamine (408 mg, 4.028 mmol) were added to dichloromethane (5 mL). At -78°C, sulfonyl chloride (136 mg, 1.007 mmol) was added to the reaction solution dropwise. After the addition was completed, the reaction mixture was heated to room temperature and reacted for 5 h. At the same time, tert-butyl 4-(6-(5-amino-6-methoxypyridin-3-yl)quinazolin-4-yl)piperazine-1-carboxylate (132 mg, 0.302mmol), pyridine (72 mg, 0.906 mmol), 4-dimethylaminopyridine (12 mg, 0.100 mmol) and dichloromethane (5 mL) were added to another reaction flask. After the solution was stirred to dissolve, the sulfonyl chloride reaction solution was added to the newly prepared solution. After the addition was completed, the reaction mixture was kept at room temperature and reacted overnight. Upon completion of the reaction, the reaction was quenched by adding water (50 mL). The reaction mixture was extracted with ethyl acetate (100 mL×2 times). The organic phases were combined, washed with saturated brine (50 mL), dried over anhydrous sodium sulfate, concentrated under reduced pressure. The residue was purified by silica gel column chromatography (Eluent: ethyl acetate/petroleum ether = 1/10 to 1/1). Tert-butyl 4-(6-(5-((*N*-(2,4-difluorophenyl)sulfamoyl)amino)-6-methoxypyridin-3-yl)quinazolin-4-yl)piperazine-1-carboxylatewas obtained, yield: 15.3%; ESI-MS(m/z): 628.2 $[M+H]^+$.

Step b): Preparation of *N*-(2,4-difluorophenyl)[(2-methoxy-5-(4-(piperazin-1-yl)quinazolin-6-yl)pyridin-3-yl)amino]sulfonateamide trifluoroacetate

Tert-butyl 4-(6-(5-((N (2,4-difluorophenyl)sulfamoyl)amino)-6-methoaypyridin-3-yl)quinazolin-4-yl)piperazine-1-carboxylate (90 mg, 0.143 mmol) was dissolved in dichloromethane (3 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. N-(2,4-difluorophenyl)[(2-methoxy-5-(4-(piperazin-1-yl)quinazolin-6-yl)pyridin-3-yl)amino]sulfonateamide trifluoroacetate was obtained, yield: 92.5%; ESI-MS(m/z): 528.2 $[M+H]^+$.

Step c): Preparation of *N*-(2,4-difluorophenyl)[(2-methoxy-5-(4-(4-[(2*E*)-4-oxopent-2-enoyl]piperazin-1-yl)quinazolin-6-yl)pyridin-3-yl)amino] sulfonamide

Referring to the preparation method of Example 109 Step e, N-(2,4-difluorophenyl)[(2-methoxy-5-(4-(4-[(2E)-4-oxopent-2-enoyl]piperazin-1-yl)quinazolin-6-yl)pyridin-3-yl)amino]sulfonamide was obtained, yield: 22.2%; $^1H$ NMR (400 MHz, DMSO-$d_6$) δ 10.07 (brs, 1H), 9.65 (brs, 1H), 8.67 (s, 1H), 8.37 (d, *J* = 4.0 Hz, 1H), 8.17-8.11 (m, 3H), 7.94 (d, *J* = 16.0 Hz, 1H), 7.54-7.48 (m, 1H), 7.44 (d, *J* = 16.0 Hz, 1H), 7.30-7.24 (m, 1H), 7.13-7.07 (m, 1H), 6.74 (d, *J* = 16.0 Hz, 1H), 3.91-3.88 (m, 5H), 3.87 (s, 3H), 3.81-3.80 (m, 3H), 2.37 (s, 3H); ESI-MS(m/z): 624.0 $[M+H]^+$.

**Example 129**

**Preparation of (*E*)-2,4-difluoro-*N*-(2-hydroxy-5-(4-(4-(4-oxopent-2-enoyl)piperazin-1-yl)quinazolin-6-yl)pyridin-3-yl)benzenesulfonamide**

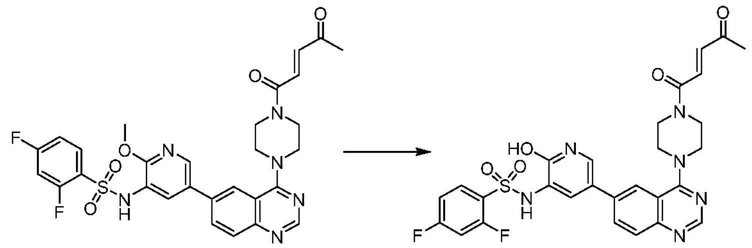

(*E*)-2,4-difluoro-*N*-(2-methoxy-5-(4-(4-(4-oxopent-2-enoyl)piperazin-1-yl)quinazolin-6-yl)pyridin-3-yl)benzenesulfonamide (120 mg, 0.197 mmol) were dissolved in acetonitrile (1 mL) and distilled water (2 mL), with 2 M dilute hydrochloric acid (2 mL) added dropwise to the reaction system. After the addition was completed, the reaction solution was heated to 45°C, stirred for 2 h and freeze dried. The crude product obtained was purified by Prep-HPLC (Method 2). (*E*)-2,4-difluoro-*N*-(2-hydroxy-5-(4-(4-(4-oxopent-2-enoyl)piperazin-1-yl)quinazolin-6-yl)pyridin-3-yl)benzenesulfonamide was obtained, yield: 8.5%; $^1H$ NMR (400 MHz, DMSO-$d_6$) δ 12.26 (s, 1H), 9.86 (s, 1H), 8.65 (s, 1H), 8.02-7.97 (m, 2H), 7.96-7.89 (m, 1H), 7.88-7.85 (m, 1H), 7.85-7.71 (m, 2H), 7.54-7.48 (m, 1H), 7.45 (d, *J* = 16.0 Hz, 1H), 7.27-7.20 (m, 1H), 6.73 (d, *J* = 16.0 Hz, 1H), 3.89-3.84 (m, 6H), 3.82-3.78 (m, 2H), 2.37 (s, 3H); ESI-MS(m/z): 595.0 $[M+H]^+$.

**Example 130**

**Preparation of (*E*)-2,4-difluoro-*N*-(2-methoxy-5-(4-((1-(4-oxopent-2-enoyl)piperidin-4-yl)amino)quinazolin6-yl)pyridin-3-yl)benzenesulfonamide**

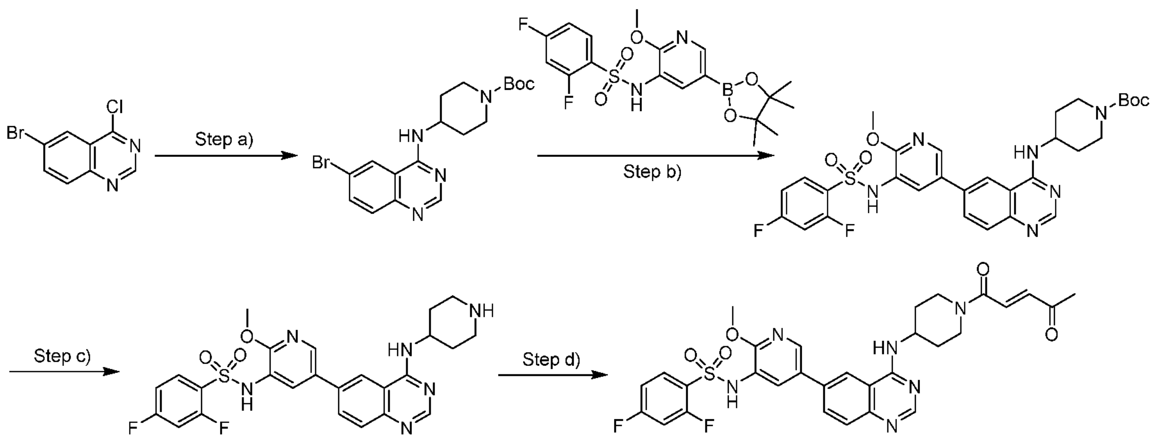


Step a): Preparation of tert-butyl 4-((6-bromoquinazolin-4-yl)amino)piperidine-1-carboaylate

6-Bromo-4-chloroquinazoline (500.0 mg, 2.053 mmol) and tert-butyl 4-aminopiperidine-1-carboxylate (492.8 mg, 2.464 mol) were added to *N*,*N*-dimethylformamide (25 mL). The reaction mixture was stirred at room temperature, with *N*,*N*-diisopropylethylamine (533.8 mg, 4.106 mmol) added. After the addition was completed, the reaction mixture was heated to 80°C and stirred for 1 h. Upon completion of the reaction, the reaction solution was cooled to room temperature, with water (100 mL) and ethyl acetate (100 mL) added, stirred for 10 min. The liquids were separated and the organic phase was washed with water (100 mL×3), dried over anhydrous sodium sulfate and filtered. The filtrate was concentrated under reduced pressure. The residue was purified by silica gel column chromatography (Eluent: petroleum ether/ethyl acetate = 3/1 to 1/1). Tert-butyl 4-((6-bromoquinazolin-4-yl)amino)piperidine-1-carboxylate was obtained, yield: 98.1 %; ESI-MS (m/z): 407.1 $[M+H]^+$.

Step b): Preparation of tert-butyl 4-((6-(5-((2,4-difluorophenyl)sulfonamido)-6-methoxypyridin-3-yl)quinazolin-4-yl)amino)piperidine-1 -carboxylate

Tert-butyl 4-((6-bromoquinazolin-4-yl)amino)piperidine-1-carboxylate (400.0 mg, 0.982 mmol), 2,4-difluoro-N-(2-methoxy-5-(4,4,5,5-tetramethyl-1,3,2-dioxaborolan-2-yl)pyridin-3-yl)benzenesulfonamide (586.2 mg, 1.375 mmol), Pd(dppf)$Cl_2$ (115.0 mg, 0.098 mmol), cesium carbonate (640.5 mg, 1.963 mmol), 1,4-dioxane (5 mL) and water (1 mL) were added to the reaction flask, with nitrogen purged three times, the reaction mixture was heated to 85°C, stirred and reacted for 2 h. Upon completion of the reaction, the reaction solution was concentrated under reduced pressure. The residue was purified by silica gel column chromatography (Eluent: petroleum ether/ethyl acetate = 30/1 to 10/1). Tert-butyl 4-((6-(5-((2,4-difluorophenyl)sulfonamido)-6-methoxypyridin-3-yl)quinazolin-4-yl)amino)piperidine-1-carboxylate was obtained, yield 63.3 %; ESI-MS (m/z): 627.2 $[M+H]^+$.

Step c): Preparation of 2,4-difluoro-*N*-(2-methoxy-5-(4-(piperidin-4-ylamino)quinazolin-6-yl)pyridin-3-yl)benzenesulfonamide

Tert-butyl 4-((6-(5-((2,4-difluorophenyl)sulfonamido)-6-methoxypyridin-3-yl)quinazolin-4-yl)amino)piperidine-1-carboxylate (200.0 mg, 0.380 mmol) was dissolved in dichloromethane (1 mL), with trifluoroacetic acid (1 mL) added. The reaction mixture was stirred at room temperature for 1 h, concentrated under reduced pressure, then with methyl tert-butyl ether (5 mL) added, stirred at room temperature for 30 min and filtered. The filter cake was dried to obtain 2,4-difluoro-*N*-(2-methoxy-5-(4-(piperidin-4-ylamino)quinazolin-6-yl)pyridin-3-yl)benzenesulfonamide, yield: 98.4 %; ESI-MS (m/z): 527.6 $[M+H]^+$.

Step d): Preparation of (*E*)-2,4-difluoro-*N*-(2-methoxy-5-(4-((1-(4-oxopent-2-enoyl)piperidin-4-yl)amino)quinazolin-6-yl)pyridin-3-yl)benzenesulfonamide

2,4-difluoro-*N*-(2-methoxy-5-(4-(piperidin-4-ylamino)quinazolin-6-yl)pyfidin-3-yl)benzenesulfonamide (160.0 mg, 0.257 mmol), acetylacrylic acid (29.2 mg, 0.257 mmol), *N*,*N*-diisopropylethylamine (100.0 mg, 0.769 mmol) and tetrahydrofuran (2 mL) were added to the reaction flask. At -78°C, under the condition of stirring, 1-propylphosphonic anhydride (123.2 mg, 0.385 mmol) was slowly added to the system. After the addition was completed, the reaction mixture was kept at -78°C and reacted for 30 min. Upon completion of the reaction, water (5 mL) and ethyl acetate (10 mL) were added to the reaction solution. The reaction solution was naturally heated to room temperature and stirred for 10 min. The liquids were separated and the organic phase was dried over anhydrous sodium sulfate and filtered. The filtrate was concentrated under reduced pressure. The residue was purified by Prep-HPLC (Method 2). (*E*)-2,4-difluoro-*N*-(2-methoxy-5-(4-((l-(4-oxopent-2-enoyl)piperidin-4-yl)amino)quinazolin-6-yl)pyridin-3-yl)benzenesulfonamide was obtained, yield: 39.8%; $^1H$ NMR (400 MHz, DMSO-$d_6$) $\delta$ 10.32 (s, 1H), 8.57-8.51 (m, 1H), 8.49 (s, 1H), 8.45-8.37 (m, 1H), 8.08 (d, *J* = 8.0 Hz, 1H), 8.05-7.98 (m, 2H), 7.79-7.68 (m, 2H), 7.57-7.43 (m, 2H), 7.22-7.13 (m, 1H), 6.69 (d, *J* = 16.0 Hz, 1H), 4.63-4.44 (m, 2H), 4.18 (d, *J* = 14.0 Hz, 1H), 3.64 (s, 3H), 3.28 (s, 1H), 2.94-2.87 (m, 1H), 2.37 (s, 3H), 2.08 (s, 2H), 1.63-1.51 (m, 2H); ESI-MS (m/z): 623.2 [M+H]+.

**Example 131**

**Preparation of (*E*)-2,6-difluoro-*N*-(2-methoxy-5-(4-(4-(4-oxopent-2-enoyl)piperazin-1-yl)pyrido[3,2-d]pyrimidin-6-yl)pyridin-3-yl)benzenesulfonamide**

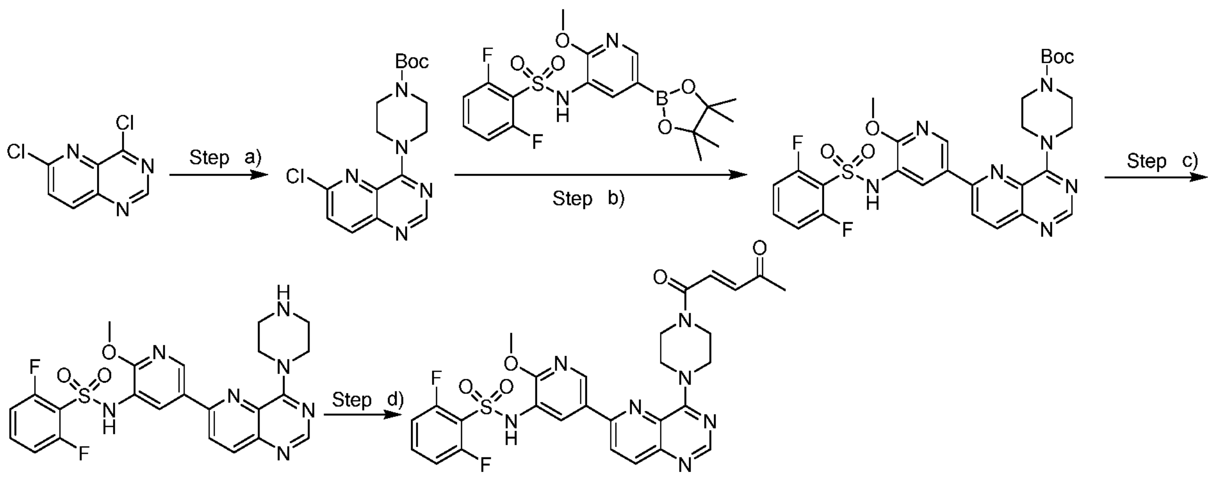


Step a): Preparation of tert-butyl 4-(6-chloropyrido[3,2-d]pyrimidin-4-yl)piperazine-1-carboxylate

4,6-Dichloropyrido[3,2-*d*]pyrimidine (250 mg, 1.250mmol), tert-butyl piperazine-1-carboxylate (350 mg, 1.875 mmol), *N*,*N*-diisopropylethylamine (485 mg, 3.750 mmol) and *N*,*N*-dimethylformamide (5 mL) were added to the reaction flask. The reaction mixture was heated to 55°C and reacted for 2 h. Upon completion of the reaction, the reaction solution was cooled to room temperature, with water (20 mL) and ethyl acetate (20 mL) added, stirred for 10 min. The liquids were separated and the organic phase was dried over anhydrous sodium sulfate and filtered. The filtrate was concentrated under reduced pressure. The residue was purified by silica gel column chromatography (Eluent: petroleum ether/ethyl acetate = 1/1). Tert-butyl 4-(6-chloropyrido[3,2-*d*]pyrimidin-4-yl)piperazine-1-carboxylate was obtained, yield: 99.1%; ESI-MS(m/z): 350.0 $[M+H]^+$.

Step b): Preparation of tert-butyl 4-(6-(5-((2,6-difluorophenyl)sulfonamido)-6-methoxypyridin-3-yl)pyrido[3,2-*d*]pyrimidin-4-yl)piperazine-1-carboxylate

Tert-butyl 4-(6-chloropyrido[3,2-*d*]pyrimidin-4-yl)piperazine-1-carboxylate (430 mg, 1.230 mmol), 2,6-difluoro-*N*-(2-methoxy-5-(4,4,5,5-tetramethyl-1,3,2-dioxaborolan-2-yl)pyridin-3-yl)benzenesulfonamide (550 mg, 1.290 mmol), $Pd(dppf)Cl_2$ (47 mg, 0.065 mmol), cesium carbonate (840 mg, 2.50mmol), 1,4-dioxane (10 mL) and water (1.5 mL) were added to the reaction flask. With nitrogen purged three times, in the presense of protective nitrogen, the reaction mixture was heated to 85°C and stirred for 1 h. Upon completion of the reaction, the reaction solution was concentrated under reduced pressure. The residue was purified by silica gel column chromatography (Eluent: petroleum ether/ethyl acetate = 1/3). Tert-butyl 4-(6-(5-((2,6-difluorophenyl)sulfonamido)-6-methoxypyridin-3-yl)pyrido[3,2-*d*]pyrimidin-4-yl)piperazine-1-carboaylate was obtained, yield: 95.2%; ESI-MS(m/z): 614.0$[M+H]^+$.

Step c): Preparation of 2,6-difluoro-*N*-(2-methoxy-5-(4-(piperizin-1-yl)pyrido[3,2-*d*]pyrimidin-6-yl)pyridin-3-yl)benzenesulfonamide trifluoroacetate

Tert-butyl 4-(6-(5-((2,6-difluorophenyl)sulfonamido)-6-methoxypyridin-3-yl)pyrido[3,2-*d*]pyrimidin-4-yl)piperazine-1-carboxylate (160 mg, 0.260mmol) and dichloromethane (1 mL) were added to the reaction flask and stirred to mix. After that, with trifluoroacetic acid (1 mL) added, the reaction mixture was stirred at room temperature for 1 h, concentrated under reduced pressure, then with methyl tert-butyl ether (5 mL) added, stirred at room temperature for 30 min and filtered. The filter cake was vacuum dried to obtain 2,6-difluoro-*N*-(2-methoxy-5-(4-(piperazin-1-yl)pyrido[3,2-*d*]pyrimidin-6-yl)pyridin-3-yl)benzenesulfonamide trifluoroacetate, yield: 99.9%; ESI-MS (m/z): 514.2 $[M+H]^+$.

Step d): Preparation of (*E*)-2,6-difluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide

2,6-Difluoro-*N*-(2-methoxy-5-(4-(piperazin-1-yl)pyrido[3,2-*d*]pyrimidin-6-yl)pyridin-3-yl)benzenesulfonamide trifluoroacetate (220 mg, 0.260 mmol), (*E*)-acrylic acid (40 mg, 0.350 mmol) and tetrahydrofuran (2 mL) were added to the reaction flask. The reaction solution was kept at -78°C and stirred for 10 min. Then *N*,*N*-diisopropylethylamine (189.8 mg, 1.460 mmol) was added. After that, the reaction solution was still kept at -78°C and stirred for 10 min. After that, 50% 1-propylphosphonic anhydride (185.7 mg, 0.584 mmol) was slowly added to the system. After the addition was

completed, the reaction solution was kept at -78°C and reacted for 30 min. Upon completion of the reaction, the reaction system was quenched by adding water (10 mL). The reaction mixture was extracted with ethyl acetate (20 mL×4). The organic phases were combined, dried over anhydrous sodium sulfate and filtered. The filtrate was concentrated under reduced pressure. The residue was purified by Prep-HPLC (Method 2). (*E*)-2,6-difluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide was obtained, yield: 68.8%; $^1H$ NMR (400 MHz, DMSO) δ 8.56 (s, 1H), 8.55(s, 1H), 8.39 (s, 2H), 8.18 (d, *J* = 8.8 Hz, 1H), 7.56 (s, 1H), 7.46 (d, *J* = 16.0 Hz, 1H), 7.15 (t, *J* = 9.2 Hz, 3H), 6.71 (d, *J* = 15.6 Hz, 1H), 4.56-4.43(m, 4H), 3.94-3.91 (m, 2H), 3.82-3.79 (m, 2H),3.75 (s, 3H), 2.33 (s, 3H); ESI-MS (m/z): 610.0 $[M+H]^+$.

## Example 132

**Preparation of (*E*)-2,4-difluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide**

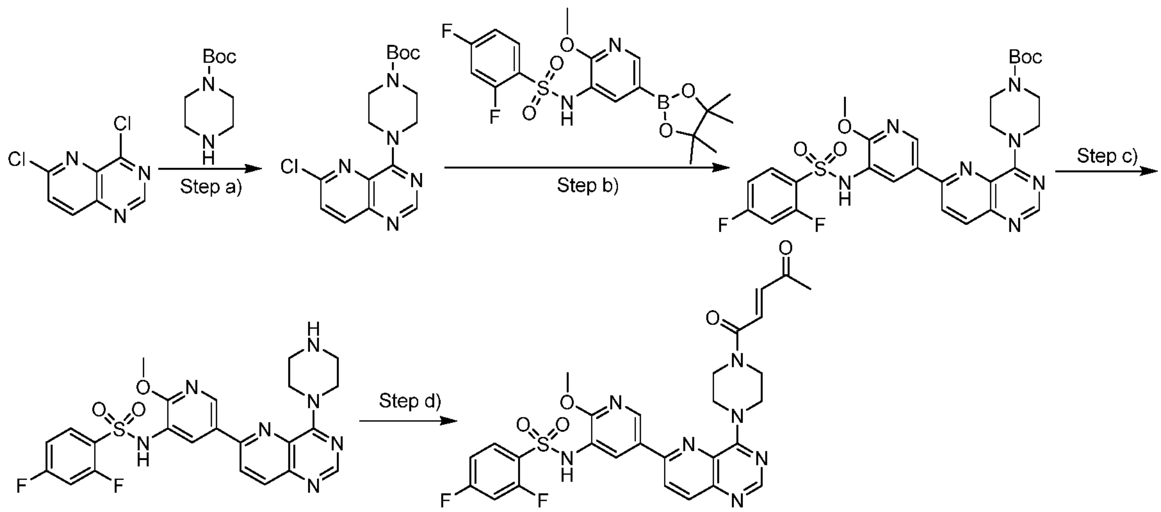


Step a): Preparation of tert-butyl 4-(6-chloropyrido[3,2-*d*]pyrimidin-4-yl)piperazine-1-carboxylate

4,6-Dichloropyrido[3,2-*d*]pyrimidine (145 mg, 0.725 mmol), tert-butyl piperazine-1-carboxylate (135 mg, 0.725 mmol), diisopropylethylamine (281 mg, 2.175 mmol) were added to *N*,*N*-dimethylformamide (3 mL). The reaction mixture was kept at 60°C and reacted for 2 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/50). Tert-butyl 4-(6-chloropyrido[3,2-*d*]pyrimidin-4-yl)piperazine-1-carboxylate was obtained, yield: 90.8%; ESI-MS(m/z): 350.1 $[M+H]^+$.

Step b): Preparation of tert-butyl 4-(6-(5-((2,4-difluorophenyl)sulfonamido)-6-methoxypyridin-3-yl)pyrido[3,2-*d*]pyrimidin-4-yl)piperazine-1-carboxylate

Tert-butyl 4-(6-chloropyrido[3,2-*d*]pyrimidin-4-yl)piperazine-1-carboxylate (130 mg, 0.372 mmol), 2,4-difluoro-*N*-(2-methoxy-5-(4,4,5,5-tetramethyl-1,3,2-dioxaborolan-2-yl)pyridin-3-yl)benzenesulfonamide (190 mg, 0.446 mmol), Pd(dppf)$Cl_2$ (27 mg, 0.037 mmol) and cesium carbonate (364 mg, 1.116 mmol) were added to dioxane/water mixed solvent (5 mL, v/v = 10:1). After the addition was completed, in the presense of protective nitrogen, the reaction mixture was heated to 100°C, stirred and reacted for 3 h. Upon completion of the reaction, the reaction was quenched by adding water (30 mL). The reaction mixture was extracted with ethyl acetate (50 mL×2 times). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/30). Tert-butyl 4-(6-(5-((2,4-difluorophenyl)sulfonamido)-6-methoxypyridin-3-yl)pyrido[3,2-*d*]pyrimidin-4-yl)piperazine-1-carboxylate was obtained, yield: 79.0%; ESI-MS(m/z): 614.2 $[M+H]^+$.

Step c): Preparation of 2,4-difluoro-*N*-(2-methoxy-5-(4-(piperazin-1-yl)pyrido[3,2-*d*]pyrimidin-6-yl)pyridin-3-yl)benzenesulfonamide trifluoroacetate

Tert-butyl 4-(6-(5-((2,4-difluorophenyl)sulfonamido)-6-methoxypyridin-3-yl)pyrido[3,2-*d*]pyrimidin-4-yl)piperazine-1-carboxylate (130 mg, 0.212 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2,4-difluoro-*N*-(2-methoxy-5-(4-(piperazin-1-yl)pyrido[3,2-*d*]pyrimidin-6-yl)pyridin-3-yl)benzenesulfonamide trifluoroacetate was obtained, yield: 97.1%; ESI-MS(m/z): 514.2 $[M+H]^+$.

Step d): Preparation of (*E*)-2,4-difluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide

Referring to the preparation method of Example 131, Step d, (*E*)-2,4-difluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide was obtained, yield: 21.9%; $^1$H NMR (400 MHz, DMSO-$d_6$) δ 10.36 (brs, 1H), 8.79 (d, *J* = 2.4 Hz, 1H), 8.58 (s, 1H), 8.45-8.38 (m, 2H), 8.22 (d, *J* = 8.8 Hz, 1H), 7.81-7.72 (m, 1H), 7.60-7.51 (m, 1H), 7.47 (d, *J* = 15.6 Hz, 1H), 7.24-7.16 (m, 1H), 6.73 (d, *J* = 15.6 Hz, 1H), 4.75-4.30 (m, 4H), 4.00-3.80 (m, 4H), 3.75 (s, 3H), 2.36 (s, 3H); ESI-MS(m/z): 610.0 $[M+H]^+$.

**Example 133**

**Preparation of (*E*)-2-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide**

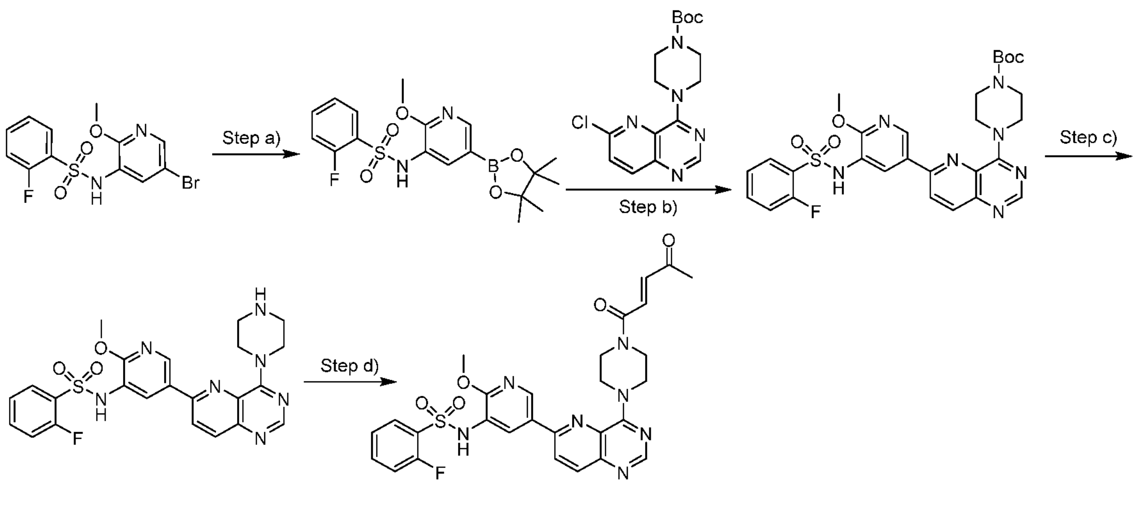


Step a): Preparation of 2-fluoro-*N*-(2-methoxy-5-(4,4,5,5-tetramethyl-1,3,2-dioxaborolan-2-yl)pyridin-3-yl)benzenesulfonamide

*N*-(5-bromo-2-methoxypyridin-3-yl)-2-fluorobenzenesulfonamide (2.16 g, 5.980 mmol), pinacol diborate (2.28 g, 8.970 mmol), Pd(dppf)$Cl_2$ (438 mg, 0.598 mmol) and potassium acetate (1.76 g, 17.940 mmol) were added to 1,4-dioxane (20 mL). In the presense of protective nitrogen, the reaction mixture was kept at 90°C and reacted for 3 h. Upon completion of the reaction, the reaction was quenched by adding water (50 mL). The reaction mixture was extracted with ethyl acetate (150 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: methanol/dichloromethane = 1/100 to 1/50). 2-Fluoro-N-(2-methoxy-5-(4,4,5,5-tetramethyl-1,3,2-dioxaborolan-2-yl)pyridin-3-yl)benzenesulfonamide was obtained, yield: 97.2%; ESI-MS(m/z): 409.2 $[M+H]^+$.

Step b): Preparation of tert-butyl 4-(6-(5-((2-fluorophenyl)sulfonamido)-6-methoxypyridin-3-yl)pyrido[3,2-*d*]pyrimidin-4-yl)piperazine-1-carboxylate

2-Fluoro-*N*-(2-methoxy-5-(4,4,5,5-tetramethyl-1,3,2-dioxaborolan-2-yl)pyridin-3-yl)benzenesulfonamide (152 mg, 0.372 mmol), tert-butyl 4-(6-chloropyrido[3,2-*d*]pyrimidin-4-yl)piperazine-1-carboxylate (130 mg, 0.372 mmol), Pd(dppf)$Cl_2$ (27 mg, 0.037 mmol) and cesium carbonate (364 mg, 1.116 mmol) were added to dioxane/water mixed solvent (5 mL, v/v = 10:1). After the addition was completed, in the presense of protective nitrogen, the reaction mixture was heated to 100°C, stirred and reacted for 3 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/30). Tert-butyl 4-(6-(5-((2-fluorophenyl)sulfonamido)-6-methoxypyridin-3-yl)pyrido[3,2-*d*]pyrimidin-4-yl)piperazine-1-carboxylate was obtained, yield: 45.4%; ESI-MS(m/z): 596.2 $[M+H]^+$.

Step c): Preparation of 2-fluoro-*N*-(2-methoxy-5-(4-(piperazin-1-yl)pyrido[3,2-*d*]pyrimidin-6-yl)pyridin-3-yl)benzenesulfonamide trifluoroacetate

Tert-butyl 4-(6-(5-((2-fluorophenyl)sulfonamido)-6-methoxypyridin-3-yl)pyrido[3,2-*d*]pyrimidin-4-yl)piperazine-1-carboxylate (100 mg, 0.168 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2-Fluoro-*N*-(2-methoxy-5-(4-(piperazin-1-yl)pyrido[3,2-*d*]pyrimidin-6-yl)pyridin-3-yl)benzenesulfonamide trifluoroacetate was obtained, yield: 98.6%; ESI-MS(m/z): 496.2 $[M+H]^+$.

Step d): Preparation of (*E*)-2-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide

Referring to the preparation method of Example 131, Step d, (*E*)-2-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide was obtained, yield: 29.7%; $^1H$ NMR (400 MHz, DMSO-$d_6$) δ 10.28 (brs, 1H), 8.78 (d, *J* = 2.0 Hz, 1H), 8.58 (s, 1H), 8.45-8.35 (m, 2H), 8.22 (d, *J* = 8.8 Hz, 1H), 7.74-7.64 (m, 2H), 7.51-7.40 (m, 2H), 7.34-7.26 (m, 1H), 6.73 (d, *J* = 16.0 Hz, 1H), 4.73-4.30 (m, 4H), 3.99-3.88 (m, 2H), 3.88-3.78 (m, 2H), 3.73 (s, 3H), 2.37 (s, 3H); ESI-MS(m/z): 592.0 $[M+H]^+$.

**Example 134**

**Preparation of (*E*)-4-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide**

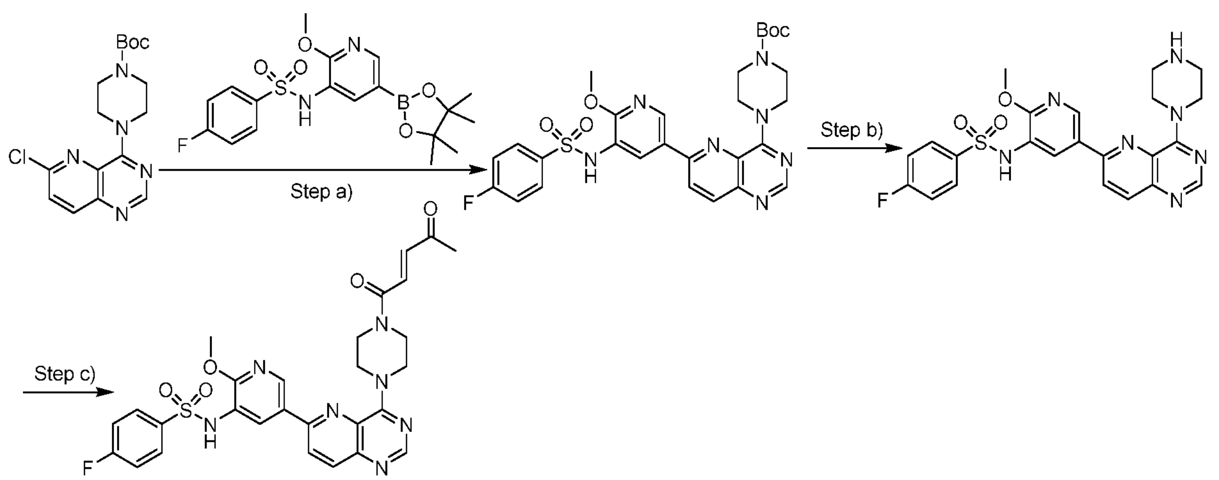

Referring to the preparation method of Example 3, After purified by Prep-HPLC (Method 2), (*E*)-4-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide was obtained, yield: 15.5%; $^1$H NMR (400 MHz, DMSO-$d_6$) δ 10.07 (brs, 1H), 8.76 (d, *J* = 2.4 Hz, 1H), 8.58 (s, 1H), 8.46-8.38 (m, 2H), 8.22 (d, *J* = 8.8 Hz, 1H), 7.86-7.76 (m, 2H), 7.47 (d, *J* = 16.0 Hz, 1H), 7.42-7.33 (m, 2H), 6.73 (d, *J* = 15.6 Hz, 1H), 4.54 (s, 4H), 4.00-3.89 (m, 2H), 3.89-3.79 (m, 2H), 3.75 (s, 3H), 2.36 (s, 3H); ESI-MS(m/z): 592.0 [M+H]$^+$.

**Example 135**

**Preparation of (*E*)-2-chloro-4-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide**

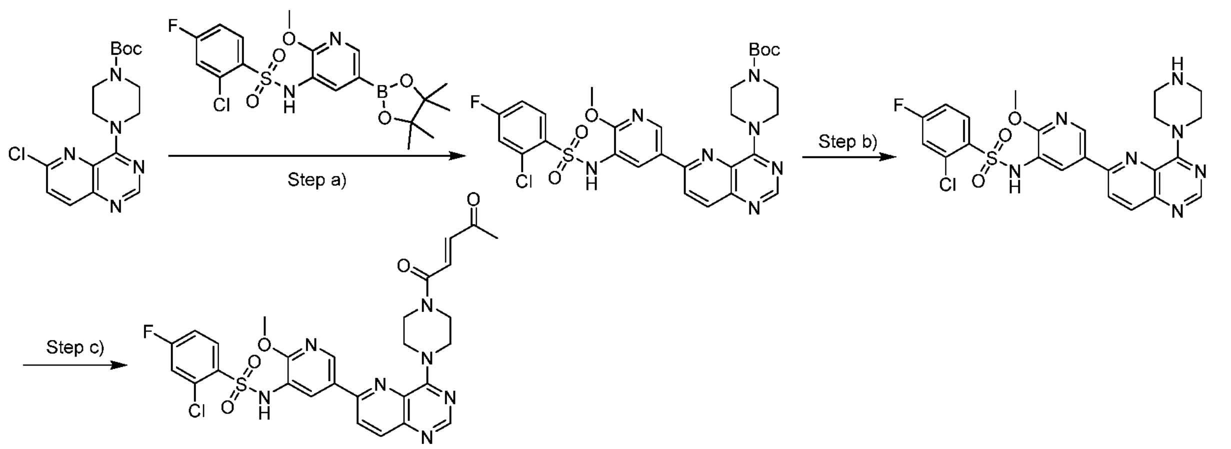


Step a): Preparation of tert-butyl 4-(6-(5-((2-chloro-4-fluorophenyl)sulfonamido)-6-methoxypyridin-3-yl)pyrido[3,2-*d*]pyrimidin-4-yl)piperazine-1-carboaylate

Tert-butyl 4-(6-chloropyrido[3,2-*d*]pyrimidin-4-yl)piperazine-1-carboxylate (100 mg, 0.286 mmol), 2-chloro-4-fluoro-N-(2-methoxy-5-(4,4,5,5-tetramethyl-1,3,2-dioxaborolan-2-yl)pyridin-3-yl)benzenesulfonamide (152 mg, 0.343 mmol), Pd(dppf)$Cl_2$ (21 mg, 0.029 mmol) and cesium carbonate (280 mg, 0.858 mmol) were added to dioxane/water mixed solvent (10 mL, v/v = 10:1). After the addition was completed, in the presense of protective nitrogen, the reaction mixture was heated to 100°C, stirred and reacted for 3 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/30). Tert-butyl 4-(6-(5-((2-chloro-4-fluorophenyl)sulfonamido)-6-methoxypyridin-3-yl)pyrido[3,2-*d*]pyrimidin-4-yl)piperazine-1-carboxylate was obtained, yield: 87.6%; ESI-MS(m/z): 630.2 [M+H]$^+$.

Step b): Preparation of 2-chloro-4-fluoro-*N*-(2-methoxy-5-(4-(piperazin-1-yl)pyrido[3,2-*d*]pyrimidin-6-yl)pyridin-3-yl)benzenesulfonamide trifluoroacetate

Tert-butyl 4-(6-(5-((2-chloro-4-fluorophenyl)sulfonamido)-6-methoxypyridin-3-yl)pyrido[3,2-*d*]pyrimidin-4-yl)piperazine-1-carboxylate (160 mg, 0.254 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2-Chloro-4-fluoro-*N*-(2-methoxy-5-(4-(piperazin-1-yl)pyrido[3,2-*d*]pyrimidin-6-yl)pyridin-3-yl)benzenesulfonamide trifluoroacetate was obtained trifluoroacetate, yield: 74.5%; ESI-MS(m/z): 530.2 [M+H]$^+$.

Step c): Preparation of (*E*)-2-chloro-4-fluoro-*N*-(2-methoxy-5-(4-(4-(4-oxopent-2-enoyl)piperazin-1-yl)pyrido[3,2-*d*]pyrimidin-6-yl)pyridin-3-yl)benzenesulfonamide

Referring to the preparation method of Example 131 Step d, (*E*)-2-chloro-4-fluoro-M-(2-methoxy-5-(4-(4-(4-oxopent-2-enoyl)piperazin-1-yl)pyrido[3,2-*d*]pyrimidin-6-yl)pyridin-3-yl)benzenesulfonamide was obtained, yield: 18.6%; $^1$H NMR (400 MHz, DMSO-$d_6$) δ 10.23 (brs, 1H), 8.77 (d, *J* = 2.4 Hz, 1H), 8.57 (s, 1H), 8.44-8.34 (m, 2H), 8.21 (d, *J* = 8.8 Hz, 1H), 7.95-7.91 (m, 1H), 7.73-7.71 (m, 1H), 7.47 (d, *J* = 16.0 Hz, 1H), 7.38-7.28 (m, 1H), 6.74 (d, *J* = 15.6 Hz, 1H), 4.51 (s, 4H), 3.99-3.89 (m, 2H), 3.88-3.80 (m, 2H), 3.77 (s, 3H), 2.37 (s, 3H); ESI-MS(m/z): 626.0 [M+H]$^+$.

**Example 136**

**Preparation of (*E*)-2,6-difluoro-*N*-(2-methoxy-5-(4-(2-(4-oxopent-2-enoyl)-2,7-diazaspiro[3.5]nonan-7-yl)pyrido[3,2-*d*]pyrimidin-6-yl)pyridin-3-yl)benzenesulfonamide**

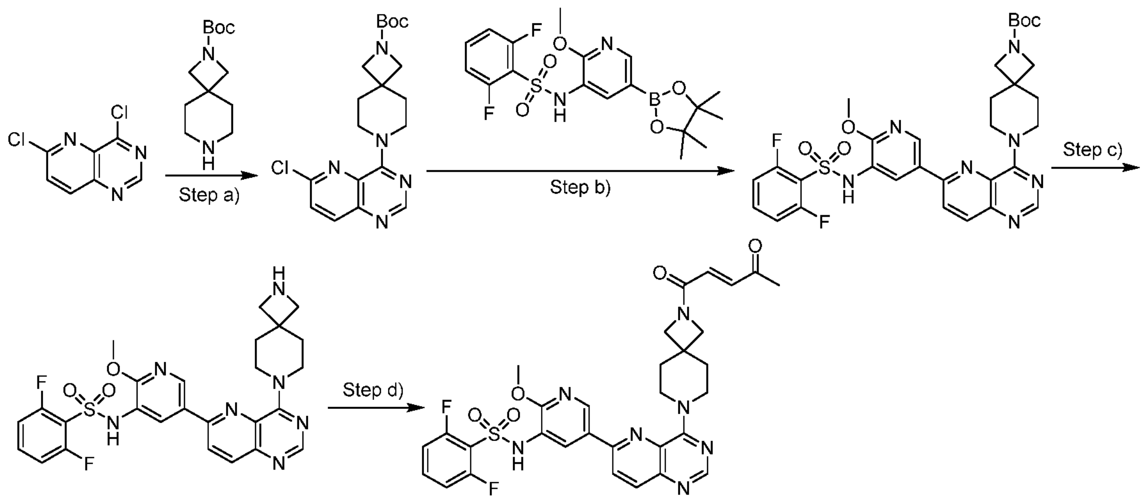


Step a): Preparation of tert-butyl 7-(6-chloropyrido[3,2-*d*]pyrimidin-4-yl)-2,7-diazaspiro[3.5]nonane-2-carboxylate

4,6-Dichloropyrido[3,2-*d*]pyrimidine (150 mg, 0.750 mmol), tert-butyl 2,7-diazaspiro[3.5]nonane-2-carboxylate (186 mg, 0.825 mmol) was dissolved in *N*,*N*-dimethylformamide (5 mL), with *N*,*N*-diisopropylethylamine (290 mg, 2.250 mmol) added. After the addition was completed, the reaction mixture was heated to 40°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The residue was purified by silica gel column chromatography (Eluent: dichloromethane/methanol = 100/1 to 10/1). Tert-butyl 7-(6-chloropyrido[3,2-*d*]pyrimidin-4-yl)-2,7-diazaspiro[3.5]nonane-2-carboxylate was obtained, yield: 85.5%; ESI-MS(m/z): 390.5 [M+H]$^+$.

Step b): Preparation of tert-butyl 7-(6-(5-((2,6-difluorophenyl)sulfonamido)-6-methoxypyridin-3-yl)pyrido[3,2-*d*]pyrimidin-4-yl)-2,7-diazaspiro[3.5]nonane-2-carboxylate

Tert-butyl 7-(6-chloropyrido[3,2-*d*]pyrimidin-4-yl)-2,7-diazaspiro[3.5]nonane-2-carboxylate (250 mg, 0.641 mmol), 2,6-difluoro-N-(2-methoxy-5-(4,4,5,5-tetramethyl-1,3,2-dioxaborolan-2-yl)pyridin-3-yl)benzenesulfonamide (328 mg, 0.769 mmol), Pd(dppf)$Cl_2$ (47 mg, 0.064 mmol) and cesium carbonate (627 mg, 1.923 mmol) were added to dioxane/water mixed solvent (10 mL, v/v = 4:1). After the addition was completed, in the presense of protective nitrogen, the reaction mixture was heated to reflux and stirred for 2 h. Upon completion of the reaction, the reaction was quenched by adding water (50 mL). The reaction mixture was extracted with ethyl acetate (200 mL×2). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 10/1). Tert-butyl 7-(6-(5-((2,6-difluorophenyl)sulfonamido)-6-methoxypyridin-3-yl)pyrido[3,2-*d*]pyrimidin-4-yl)-2,7-diazaspiro[3.5]nonane-2-carboxylate was obtained, yield: 95.5%; ESI-MS(m/z): 654.2 [M+H]$^+$.

Step c): Preparation of *N*-(5-(4-(2,7-diazaspiro[3.5]nonan-7-yl)pyrido[3,2-*d*]pyrimidin-6-yl)-2-methoxypyridin-3-yl)-2,6-difluorobenzenesulfonamide trifluoroacetate

Tert-butyl 7-(6-(5-((2,6-difluorophenyl)sulfonamido)-6-methoxypyridin-3-yl)pyrido[3,2-*d*]pyrimidin-4-yl)-2,7-diazaspiro[3.5]nonane-2-carboxylate (400 mg, 0.612 mmol) was dissolved in dichloromethane (6 mL). Under the ice bath condition, trifluoroacetic acid (1.5 mL) was slowly added dropwise. After the addition was completed, the reaction mixture was stirred at room temperature for 1 h. Upon completion of the reaction, the reaction mixture was concentrated under reduced pressure. Methyl tert-butyl ether was added to the crude product. The mixture was stirred to precipitate solid and filtered. *N*-(5-(4-(2,7-diazaspiro[3.5]nonan-7-yl)pyrido[3,2-*d*]pyrimidin-6-yl)-2-methoxypyridin-3-yl)-2,6-difluorobenzenesulfonamide trifluoroacetate was obtained; ESI-MS(m/z): 545.5 [M+H]$^+$. Step d): Preparation of (*E*)-2,6-difluoro-*N*-(2-methoxy-5-(4-(2-(4-oxopent-2-enoyl)-2,7-diazaspiro[3.5]nonan-7-yl)pyrido[3,2-*d*]pyrimidin-6-yl)pyridin-3-yl)benzenesulfonamide *N*-(5-(4-(2,7-diazaspiro[3.5]nonan-7-yl)pyrido[3,2-*d*]pyrimidin-6-yl)-2-methoxypyridin-3-yl)-2,6-difluorobenzenesulfonamide trifluoroacetate (150 mg, 0.230 mmol) and tetrahydrofuran (6 mL) were added to the reaction flask. The reaction system was cooled to -78°C, with *N,N*-diisopropylethylamine (150 mg, 1.150 mmol), (*E*)-4-oxopent-2-enoic acid (29 mg, 0.253 mmol) and 50% $T_3P$ ethyl acetate solution (293 mg, 0.460 mmol) added successively. After the addition was completed, the reaction mixture was kept at -78°C and stirred for 1 h. Upon completion of the reaction, at -78°C, the reaction was quenched using saturated aqueous ammonium chloride solution (50 mL). The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). (*E*)-2,6-difluoro-*N*-(2-methoxy-5-(4-(2-(4-oxopent-2-enoyl)-2,7-diazaspiro[3.5]nonan-7-yl)pyrido[3,2-*d*]pyrimidin-6-yl)pyridin-3-yl)benzenesulfonamide was obtained, yield: 16.8%; $^1$H NMR (400 MHz, DMSO-$d_6$) δ 10.51 (s, 1H), 8.82-8.71 (m, 1H), 8.51 (s, 1H), 8.43-8.34 (m, 2H), 8.17 (d, *J* = 8.8 Hz, 1H), 7.74-7.63 (m, 1H), 7.25 (t, *J* = 9.2 Hz, 2H), 6.95 (d, *J* = 15.6 Hz, 1H), 6.75 (d, *J* = 15.6 Hz, 1H), 4.49-4.32 (m, 4H), 4.15 (s, 2H), 3.79 (s, 2H), 3.71 (s, 3H), 2.35 (s, 3H), 1.97-1.87 (m, 4H); ESI-MS(m/z): 650.0 [M+H]$^+$.

**Example 137**

**Preparation of 2,6-difluoro-*N*-(2-methoxy-5-(4-(8-((*E*)-4-oxopent-2-enoyl)-3,8-diazabicyclo[3.2.1]octan-3-yl)pyrido[3,2-*d*]pyrimidin-6-yl)pyridin-3-yl)benzenesulfonamide**

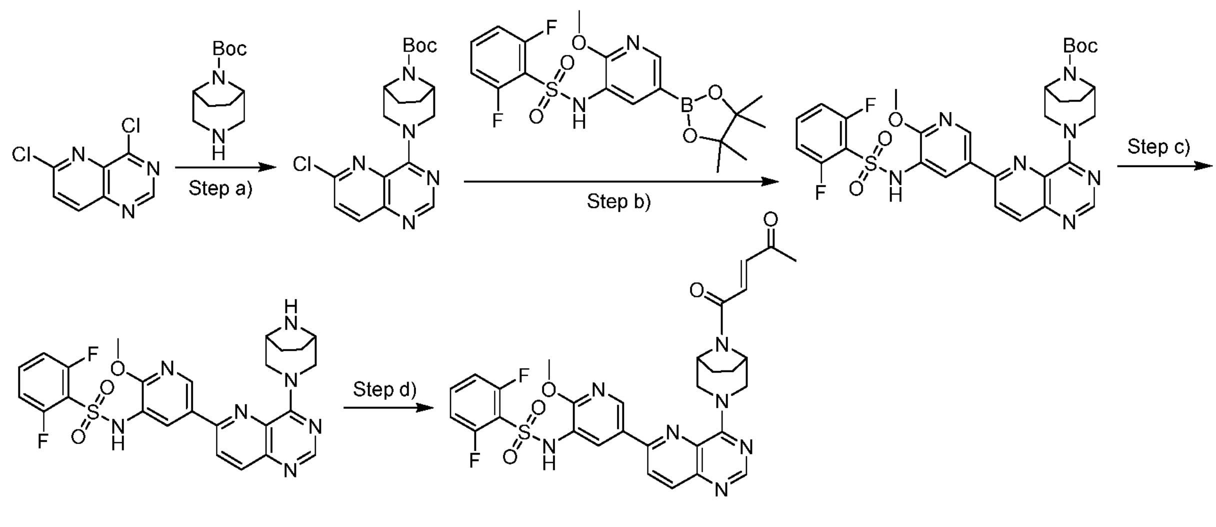


Step a): Preparation of tert-butyl 3-(6-chloropyrido[3,2-*d*]pyrimidin-4-yl)-3,8-diazabicyclo[3.2.1]octane-8-carboxylate

4,6-Dichloropyrido[3,2-*d*]pyrimidine (150 mg, 0.750 mmol), tert-butyl 3,8-diazabicyclo[3.2.1]octane-8-carboxylate (175 mg, 0.825 mmol) were dissolved in *N,N*-dimethylformamide (5 mL), with *N,N*-diisopropylethylamine (290 mg, 2.250 mmol) added. After the addition was completed, the reaction mixture was heated to 40°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The residue was purified by silica gel column chromatography (Eluent: dichloromethane/methanol = 100/1 to 10/1). Tert-butyl 3-(6-chloropyrido[3,2-*d*]pyrimidin-4-yl)-3,8-diazabicyclo[3.2.1]octane-8-carboxylate was obtained, yield: 92.2%; ESI-MS(m/z): 376.2 $[M+H]^+$.

Step b): Preparation of tert-butyl 3-(6-(5-((2,6-difluorophenyl)sulfonamido)-6-methoxypyridin-3-yl)pyrido[3,2-*d*]pyrimidin-4-yl)-3,8-diazabicyclo[3.2.1]octane-8-carboxylate

Tert-butyl 3-(6-chloropyrido[3,2-*d*]pyrimidin-4-yl)-3,8-diazabicyclo[3.2.1]octane-8-carboxylate (260 mg, 0.691 mmol), 2,6-difluoro-*N*-(2-methoxy-5-(4,4,5,5-tetramethyl-1,3,2-dioxaborolan-2-yl)pyridin-3-yl)benzenesulfonamide (353 mg, 0.830 mmol), $Pd(dppf)Cl_2$ (51 mg, 0.069 mmol) and cesium carbonate (676 mg, 2.073 mmol) were added to dioxane/water mixed solvent (10 mL, v/v = 4:1). After the addition was completed, in the presense of protective nitrogen, the reaction mixture was heated to 110°C and stirred for 2 h. Upon completion of the reaction, the reaction was quenched by adding water (50 mL). The reaction mixture was extracted with ethyl acetate (200 mL×2). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 10/1). Tert-butyl 3-(6-(5-((2,6-difluorophenyl)sulfonamido)-6-methoxypyridin-3-yl)pyrido[3,2-*d*]pyrimidin-4-yl)-3,8-diazabicyclo[3.2.1]octane-8-carboxylate was obtained, yield: 90.3%; ESI-MS(m/z): 640.2 $[M+H]^+$.

Step c): Preparation of *N*-(5-(4-(3,8-diazabicyclo[3.2.1]octan-3-yl)pyrido[3,2-*d*]pyrimidin-6-yl)-2-methoxypyridin-3 -yl)-2,6-difluorobenzenesulfonamide trifluoroacetate

Tert-butyl 3-(6-(5-((2,6-difluorophenyl)sulfonamido)-6-methoxypyridin-3-yl)pyrido[3,2-*d*]pyrimidin-4-yl)-3,8-diazabicyclo[3.2.1]octane-8-carboxylate (400 mg, 0.612 mmol) was dissolved in dichloromethane (6 mL). Under the ice bath condition, trifluoroacetic acid (1.5 mL) was slowly added dropwise. After the addition was completed, the reaction mixture was stirred at room temperature for 1 h. Upon completion of the reaction, the reaction mixture was concentrated under reduced pressure. Methyl tert-butyl ether was added to the crude product. The mixture was stirred to precipitate solid and filtered. *N*-(5-(4-(3,8-diazabicyclo[3.2.1]octan-3-yl)pyrido[3,2-*d*]pyrimidin-6-yl)-2-methoxypyridin-3-yl)-2,6-difluorobenzenesulfonamide trifluoroacetate was obtained; ESI-MS(m/z): 540.5 $[M+H]^+$. Step d): Preparation of 2,6-difluoro-*N*-(2-methoxy-5-(4-(8-((*E*)-4-oxopent-2-enoyl)-3,8-diazabicyclo[3.2.1]octan-3-yl)pyrido[3,2-*d*]pyrimidin-6-yl)pyridin-3-yl)benzenesulfonamide *N*-(5-(4-(3,8-diazabicyclo[3.2.1]octan-3-yl)pyrido[3,2-*d*]pyrimidin-6-yl)-2-methoxypyridin-3-yl)-2,6-difluorobenzenesulfonamide trifluoroacetate (150 mg, 0.235 mmol) and tetrahydrofuran (6 mL) were added to the reaction flask. The reaction system was cooled to -78°C, with *N*,*N*-diisopropylethylamine (152 mg, 1.175 mmol), (*E*)-4-oxopent-2-enoic acid (29 mg, 0.259 mmol) and 50% $T_3P$ ethyl acetate solution (299 mg, 0.470 mmol, 50%wt) added successively. After the addition was completed, the reaction mixture was kept at -78°C and stirred for 1 h. Upon completion of the reaction, at -78°C, the reaction was quenched using saturated aqueous ammonium chloride solution (50 mL). The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). 2,6-Difluoro-*N*-(2-methoxy-5-(4-(8-((*E*)-4-oxopent-2-enoyl)-3,8-diazabicyclo[3.2.1]octan-3-yl)pyrido[3,2-*d*]pyrimidin-6-yl)pyridin-3-yl)benzenesulfonamide was obtained, yield: 15.8%; $^1H$ NMR (400 MHz, DMSO-$d_6$) δ 10.62 (s, 1H), 8.77 (s, 1H), 8.57 (s, 1H), 8.49-8.35 (m, 2H), 8.22 (d, *J* = 8.8 Hz, 1H), 7.75-7.62 (m, 1H), 7.41 (d, *J* = 15.6 Hz, 1H), 7.25 (t, *J* = 9.2 Hz, 2H), 6.81 (d, *J* = 15.6 Hz, 1H), 5.74-5.23 (m, 2H), 4.97-4.75 (m, 2H), 3.74 (s, 3H), 3.59-3.34 (m, 2H), 2.38 (s, 3H), 2.04-1.79 (m, 4H); ESI-MS(m/z): 636.0 $[M+H]^+$.

**Example 138**

**Preparation of (*S*,*E*)-2,6-difluoro-*N*-(2-methoxy-5-(4-(2-methyl-4-(4-oxopent-2-enoyl)piperazin-1-yl)pyrido[3,2-*d*] pyrimidin-6-yl)pyridin-3-yl)benzenesulfonamide**

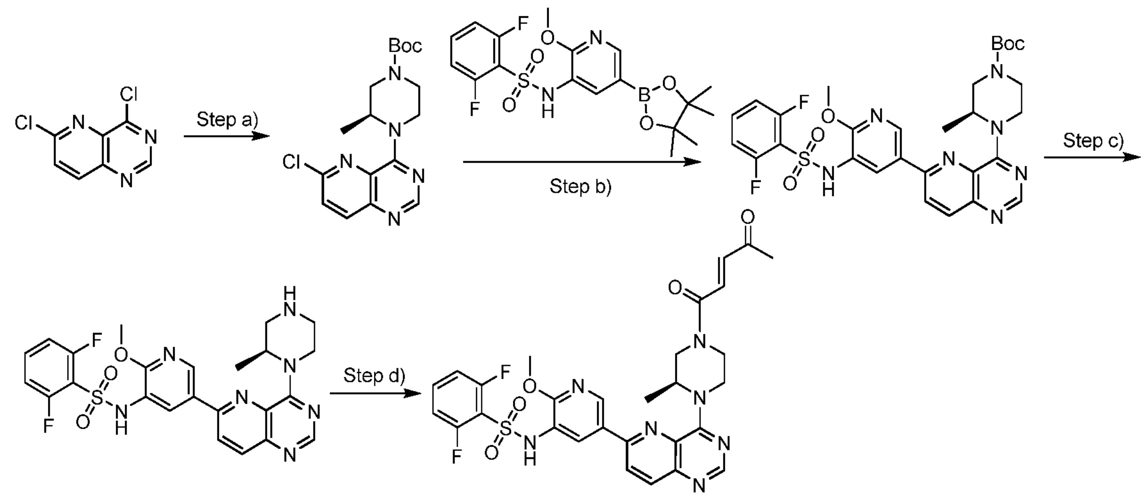


Step a): Preparation of tert-butyl (*S*)-4-(6-chloropyrido[3,2-*d*]pyrimidin-4-yl)-3-methylpiperazine-1-carboxylate

4,6-Dichloropyrido[3,2-*d*]pyrimidine (150 mg, 0.750 mmol), tert-butyl (*S*)-3-methylpiperazine-1-carboxylate (165 mg, 0.825 mmol) were dissolved in *N*,*N*-dimethylformamide (5 mL), with *N*,*N*-diisopropylethylamine (290 mg, 2.250 mmol) added. After the addition was completed, the reaction mixture was heated to 40°C and stirred for 4 h. Upon completion of the reaction, the reaction was quenched by adding water (50 mL). The reaction mixture was extracted with ethyl acetate (100 mL×2). The organic phases were combined, dried over anhydrous sodium sulfate and filtered. The filtrate was concentrated under reduced pressure. The residue was purified by silica gel column chromatography (Eluent: dichloromethane/methanol = 100/1 to 10/1). Tert-butyl (S)-4-(6-chloropyrido[3,2-*d*]pyrimidin-4-yl)-3-methylpiperazine-1-carboxylate was obtained, yield: 95.2%; ESI-MS(m/z): 364.2 $[M+H]^+$.

Step b): Preparation of tert-butyl (*S*)-4-(6-(5-((2,6-difluorophenyl)sulfonamido)-6-methoxypyridin-3-yl)pyrido[3,2-*d*]pyrimidin-4-yl)-3-methylpiperazine-1-carboxylate

Tert-butyl (*S*)-4-(6-chloropyrido[3,2-*d*]pyrimidin-4-yl)-3-methylpiperazine-1-carboxylate (260 mg, 0.714 mmol), 2,6-difluoro-*N*-(2-methoay-5-(4,4,5,5-tetramethyl-1,3,2-dioxaborolan-2-yl)pyridin-3-yl)benzenesulfonamide (365 mg, 0.857 mmol), $Pd(dppf)Cl_2$ (52 mg, 0.071 mmol) and cesium carbonate (698 mg, 2.142 mmol) were added to dioxane/water mixed solvent (10 mL, v/v = 4:1). After the addition was completed, in the presense of protective nitrogen, the reaction mixture was heated to 110°C and stirred for 2 h. Upon completion of the reaction, the reaction was quenched by adding water (50 mL). The reaction mixture was extracted with ethyl acetate (200 mL×2). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 100/1 to 10/1). Tert-butyl (*S*)-4-(6-(5-((2,6-difluorophenyl)sulfonamido)-6-methoxypyridin-3-yl)pyrido[3,2-*d*]pyrimidin-4-yl)-3-methylpiperazine-1-carboxylate was obtained, yield: 89.1%; ESI-MS(m/z): 628.2 $[M+H]^+$.

Step c): Preparation of (S)-4-(6-(5-((2,6-difluorophenyl)sulfonamido)-6-methoxypyridin-3-yl)pyrido[3,2-d]pyrimidin-4-yl)-3-methylpiperazine-1-carboxylate trifluoroacetate

Tert-butyl (*S*)-4-(6-(5-((2,6-difluorophenyl)sulfonamido)-6-methoxypyridin-3-yl)pyrido[3,2-*d*]pyrimidin-4-yl)-3-methylpiperazine-1-carboxylate (400 mg, 0.637 mmol) was dissolved in dichloromethane (6 mL). Under the ice bath condition, trifluoroacetic acid (1.5 mL) was slowly added dropwise. After the addition was completed, the reaction mixture was stirred at room temperature for 1 h. Upon completion of the reaction, the reaction mixture was concentrated under reduced pressure. Methyl tert-butyl ether was added to the crude product. The mixture was stirred to precipitate solid and filtered. (S)-4-(6-(5-((2,6-difluorophenyl)sulfonamido)-6-methoxypyridin-3-yl)pyrido[3,2-d]pyrimidin-4-yl)-3-methylpiperazine-1-carboxylate trifluoroacetate was obtained; ESI-MS(m/z): 528.5 $[M+H]^+$.

Step d): Preparation of (*S*,*E*)-2,6-difluoro-*N*-(2-methoxy-5-(4-(2-methyl-4-(4-oxopent-2-enoyl)piperazin-1-yl)pyrido[3,2-*d*]pyrimidin-6-yl)pyridin-3-yl)benzenesulfonamide

(S)-4-(6-(5-((2,6-difluorophenyl)sulfonamido)-6-methoxypyridin-3-yl)pyrido[3,2-d]pyrimidin-4-yl)-3-methylpiperazine-1-carboxylate trifluoroacetate (150 mg, 0.240 mmol) was dissolved in tetrahydrofuran (6 mL). The reaction

system was cooled to -78°C, with *N*,*N*-diisopropylethylamine (156 mg, 1.200 mmol), (*E*)-4-oxopent-2-enoic acid (30 mg, 0.264 mmol) and 50% $T_3P$ ethyl acetate solution (305 mg, 0.480 mmol) added successively. After the addition was completed, the reaction mixture was kept at -78°C and stirred for 1 h. Upon completion of the reaction, at -78°C, the reaction was quenched using saturated aqueous ammonium chloride solution (50 mL). The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). (*S*,*E*)-2,6-difluoro-*N*-(2-methoxy-5-(4-(2-methyl-4-(4-oxopent-2-enoyl)piperazin-1-yl)pyrido[3,2-*d*]pyrimidin-6-yl)pyridin-3-yl)benzenesulfonamide was obtained, yield: 15.8%; $^1$H NMR (400 MHz, DMSO-$d_6$) δ 10.61 (s, 1H), 8.79 (s, 1H), 8.57 (s, 1H), 8.49-8.31 (m, 2H), 8.22 (d, *J* = 8.8 Hz, 1H), 7.77-7.63 (m, 1H), 7.46 (dd, $J_1$ = 29.6 Hz, $J_2$ =16.0 Hz, 1H), 7.25 (t, *J* = 9.2 Hz, 2H), 6.75 (dd, $J_1$ = 15.6 Hz, $J_2$ = 3.6 Hz, 1H), 4.55-4.02 (m, 3H), 3.91-3.73 (m, 1H), 3.72 (s, 3H), 3.64-3.44 (m, 1H), 3.32-3.08 (m, 2H), 2.37 (s, 3H), 1.46-1.24 (m, 3H); ESI-MS(m/z): 624.0 [M+H]$^+$.

**Example 139**

**Preparation of (*E*)-2,6-difluoro-*N*-(2-methoxy-5-(4-(4-(4-oxopent-2-enoyl)piperazin-1-yl)pyrido[2,3-*d*]pyrimidin-6-yl)pyridin-3-yl)benzenesulfonamide**

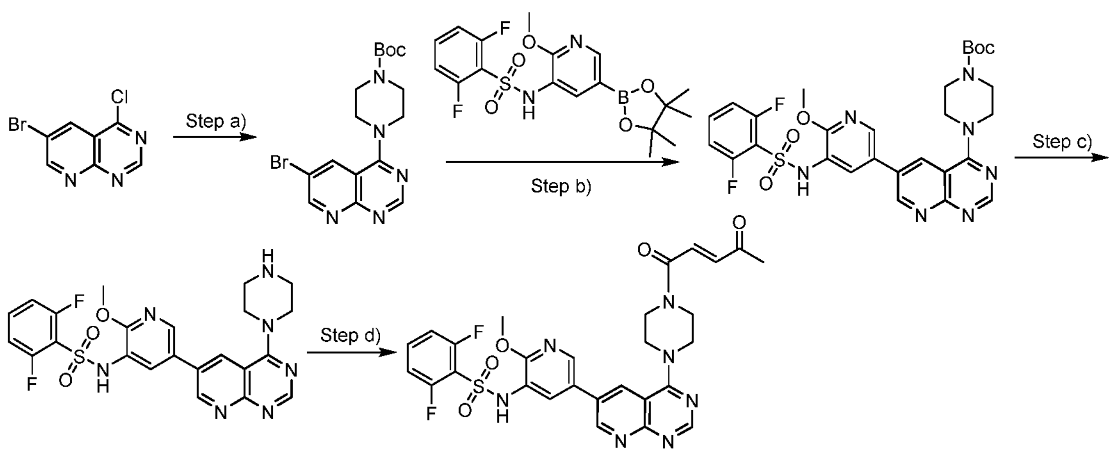


Referring to the preparation method of Example 131, (*E*)-2,6-difluoro-*N*-(2-methoxy-5-(4-(4-(4-oxopent-2-enoyl)piperazin-1-yl)pyrido[2,3-*d*]pyrimidin-6-yl)pyridin-3-yl)benzenesulfonamide was obtained, yield: 45.2%; $^1$H NMR (400 MHz, DMSO) δ 10.60 (s, 1H), 9.27 (s, 1H), 8.74 (s, 1H), 8.52 (s, 1H), 8.43 (s, 1H), 8.04 (s, 1H), 7.68-7.64 (m, 1H), 7.44 (d, *J* = 15.6 Hz, 1H), 7.23 (t, *J* = 18.4 Hz, 2H), 6.74 (d, *J* = 15.6 Hz, 1H), 4.02-3.98 (m, 4H), 3.90-3.88 (m, 2H), 3.81-3.78 (m, 2H),3.66 (s, 3H), 2.37 (s, 3H); ESI-MS (m/z): 610.0 [M+H]+.

**Example 140**

**Preparation of 2,4,6-trifluoro-*N*-(5-(4-(2-(2-fluoroacryloyl)-2,7-diazaspiro[3.5]nonan-7-yl)quinazoline-6-yl)-2-methoxypyridin-3-yl)benzenesulfonamide**

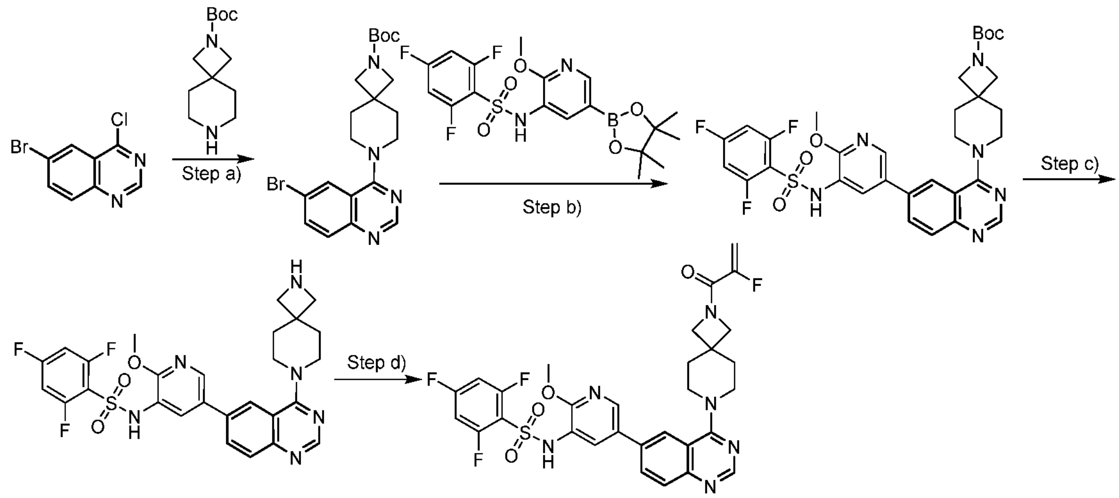


Step a): Preparation of tert-butyl 7-(6-bromoquinazolin-4-yl)-2,7-diazaspiro[3.5]nonane-2-carboxylate

6-Bromo-4-chloroquinazoline (1.5 g, 6.160 mmol) and tert-butyl 2,7-diazaspiro[3.5]nonane-2-carboxylate(1.4 g, 6.160 mmol) were dissolved in dimethyl sulfoxide (25 mL), with *N*,*N*-diisopropylethylamine (2.0 g, 15.349 mmol) added. After the addition was completed, the reaction mixture was heated to 50°C and stirred for 4 h. Upon completion of the reaction, the reaction was quenched by adding iced water (80 mL). The reaction mixture was extracted with ethyl acetate (60 mL×2). The organic phases were combined, dried over anhydrous sodium sulfate and filtered. The filtrate was concentrated under reduced pressure. The obtained crude product was ultrasonically dispersed into 10 mL petroleum ether. The reaction mixture was stirred at room temperature for 0.5 h and filtered. The solid was collected and dried to obtain tert-butyl 7-(6-bromoquinazolin-4-yl)-2,7-diazaspiro[3.5]nonane-2-carboxylate, yield: 99.9%; ESI-MS(m/z): 434.3 $[M+H]^+$.

Step b): Preparation of tert-butyl 7-(6-(6-methoxy-5-((2,4,6-trifluorophenyl)sulfonamido)pyridin-3-yl)quinazolin-4-yl)-2,7-diazaspiro [3 .5]nonane-2-carboxylate

Tert-butyl 7-(6-bromoquinazolin-4-yl)-2,7-diazaspiro[3.5]nonane-2-carboxylate (200 mg, 0.464 mmol), 2,4,6-trifluoro-*N*-(2-methoxy-5-(4,4,5,5-tetramethyl-1,3,2-nitroxyborolan-2-yl)pyridin-3-yl)benzenesulfonamide (206 mg, 0.464 mmol), $Pd(dppf)Cl_2$ (34 mg, 0.046 mmol), cesium carbonate (450 mg, 1.382 mmol), dioxane (5 mL) and water (0.5 mL) were added to the reaction flask. After the addition was completed, in the presense of protective nitrogen, the reaction mixture was kept at 100°C and stirred for 5 h. Upon completion of the reaction, the reaction mixture was cooled to room temperature, with the reaction quenched by adding saturated ammonium chloride (80 mL), extracted with ethyl acetate (80 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 5/1 to 1/2). Tert-butyl 7-(6-(6-methoxy-5 -((2,4,6-trifluorophenyl)sulfonamido)pyridin-3 -yl)quinazolin-4-yl)-2,7-diazaspiro [3.5]nonane-2-carboxylate was obtained, yield: 98.6%; ESI-MS(m/z): 671.2 $[M+H]^+$.

Step c): Preparation of *N*-(5-(4-(2,7-diazaspiro[3.5]nonan-7-yl)quinazolin-6-yl)-2-methoxypyridin-3-yl)-2,4,6-trifluorobenzenesulfonamide trifluoroacetate

Tert-butyl 7-(6-(6-methoxy-5-((2,4,6-trifluorophenyl)sulfonamido)pyridin-3-yl)quinazolin-4-yl)-2,7-diazaspiro[3.5]nonane-2-carboxylate (338 mg, 0.504 mmol) was dissolved in dichloromethane (9 mL). Under the ice bath condition, TFA (3 mL) was slowly added dropwise. After the addition was completed, the reaction mixture was stirred at room temperature for 1 h. Upon completion of the reaction, the reaction mixture was concentrated under reduced pressure. The obtained product was dispersed into 10 mL methyl tert-butyl ether. The reaction mixture was stirred at room temperature for 0.5 h and filtered. The solid was collected and dried to obtain *N*-(5-(4-(2,7-diazaspiro[3.5]nonan-7-yl)quinazolin-6-yl)-2-methoxypyridin-3-yl)-2,4,6-trifluorobenzenesulfonamide trifluoroacetate, yield: 88.1%; ESI-MS(m/z): 571.1 $[M+H]^+$.

Step d): Preparation of 2,4,6-trifluoro-*N*-(5-(4-(2-(2-fluoroacryloyl)-2,7-diazaspiro[3.5]nonan-7-yl)quinazoline-6-yl)-2-methoxypyridin-3-yl)benzenesulfonamide

*N*-(5-(4-(2,7-diazaspiro[3.5]nonan-7-yl)quinazolin-6-yl)-2-methoxypyridin-3-yl)-2,4,6-trifluorobenzenesulfonamide trifluoroacetate (100 mg, 0.154 mmol), 2-fluoroacrylic acid (16 mg, 0.182 mmol), *N,N*-diisopropylethylamine (97 mg, 0.753 mmol) were dissolved in tetrahydrofuran (2 mL). At -78°C, $T_3P$ (111 mg, 0.174 mmol, 50% wt%) was slowly added to the system. After the addition was completed, the reaction mixture was kept at -78°C and stirred for 0.5 h. Upon completion of the reaction, with 1 mL acetonitrile added, the reaction mixture was filtered and purified by Prep-HPLC (Method 2). 2,4,6-Trifluoro-N-(5-(4-(2-(2-fluoroacryloyl)-2,7-diazaspiro[3.5]nonan-7-yl)quinazoline-6-yl)-2-methoxypyridin-3-yl)benzenesulfonamide was obtained, yield: 26.7%; $^1$H NMR (400 MHz, DMSO-$d_6$) δ 10.68 (s, 1H), 8.62 (s, 1H), 8.49-8.41 (m, 1H), 8.12-8.00 (m, 3H), 7.89 (d, *J*= 8.4 Hz, 1H), 7.47-7.38 (m, 2H), 5.48 (dd, $J_1$ = 48.4 Hz, $J_2$ = 3.6 Hz, 1H), 5.29 (dd, $J_1$ = 16.4 Hz, $J_2$ = 3.6 Hz, 1H), 4.21-4.14 (m, 2H), 3.82-3.71 (m, 6H), 3.69 (s, 3H), 1.99-1.90 (m, 4H); ESI-MS(m/z): 643.0 [M+H]$^+$. Referring to the preparation method of Example 140 and using the corresponding raw materials, the compounds in the following examples were obtained.

| Number | Name | Structure | $^1$H NMR and MS |
|---|---|---|---|
| **Example 141** | (*E*)-2,4-difluoro-*N*-(2-methoxy-5-(4-(6-(4-oxopent-2-enoyl))-2,6-diazaspirocycle [3 .3]heptan-2-yl) quinazolin-6-yl) pyridin-3-yl) benzenesulfon amide | 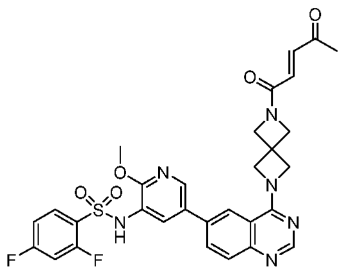 | $^1$H NMR (400 MHz, DMSO-$d_6$) δ 10.22 (s, 1H), 8.48 (s, 1H), 8.43-8.41 (m, 1H), 8.05-8.01 (m, 2H), 7.98-7.96 (m, 1H), 7.82-7.78 (m, 1H), 7.78-7.73 (m, 1H), 7.60-7.53 (m, 1H), 7.23-7.18 (m, 1H), 6.89 (d, *J* = 16.0 Hz, 1H), 6.75 (d, *J* = 16.0 Hz, 1H), 4.89-4.61 (m, 4H), 4.58 (s, 2H), 4.25 (s, 2H), 3.66 (s, 3H), 2.34 (s, 3H); ESI-MS(m/z): 621.0 [M+H]$^+$. |
| **Example 142** | (*E*)-2,4-difluoro-*N*-(2-methoxy-5-(4-(6-(4-oxopent-2-enoyl)-3,6-diazabicyclo[3.1. 1]heptan-3-yl) quinazolin-6-yl) pyridin-3-yl) benzenesulfon amide | 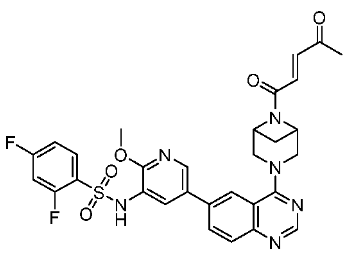 | $^1$H NMR (400 MHz, DMSO-$d_6$) δ 10.28 (s, 1H), 8.55-8.50 (m, 2H), 8.41 (s, 1H), 8.01-7.96 (m, 2H),7.85-7.75(m, 2H), 7.58-7.55 (m, 1H), 7.23-7.19 (m, 1H), 7.05 (d, *J* =16.0 Hz, 1H), 6.72 (d, *J* = 16.0 Hz, 1H), 5.03 (s, 1H), 4.61 (s, 1H), 4.51-4.45 (m, 3H), 4.37-4.33 (m , 1H), 3.68 (s, 3H), 2.75-2.73 (m, 1H), 2.34 (s, 3H), 1.72 (d, *J* =12.0 Hz, 1H); ESI-MS (m/z): 621.0 [M+H]$^+$. |
| **Example 144** | 2,4-difluoro-*N*-(2-methoxy-5-(4-(3-((*E*)-4-oxopent-2-enoyl)-3,8-diazabicyclo[3 2. 1]octan-8-yl) quinazolin-6-yl) pyridin-3-yl) benzenesulfon amide | 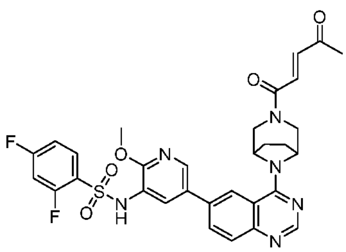 | $^1$H NMR (400 MHz, DMSO-$d_6$) δ 10.28 (s, 1H), 8.65 (s, 1H), 8.48 (s, 1H), 8.19-8.08 (m, 2H), 8.05 (s, 1H), 7.91 (d, *J* = 8 Hz, 1H), 7.78-7.75 (m, 1H), 7.56-7.53 (m, 1H), 7.43 (d, *J* = 16 Hz, 1H), 7.23-7.18 (m, 1H), 6.71 (d, *J* = 16 Hz, 1H), 4.98 (d, *J* = 12 Hz, 2H), 4.40 (d, *J* = 16 Hz, 1H), 4.10 (d, *J* = 19 Hz, 1H), 3.74 (d, *J* = 16 Hz, 1H), 3.69 (s, 3H), 3.28-3.25 (m, 1H), 2.35 (s, 3H), 1.99-1.87 (m, 2H), 1.80-1.75 (m, 1H), 1.66-1.61 (m, 1H); ESI-MS(m/z): 635.0 [M+H]$^+$. |

(continued)

| Number | Name | Structure | $^1$H NMR and MS |
|---|---|---|---|
| **Example 145** | 2,6-difluoro-*N*-(2-methoxy-5-(4-(8-((*E*)-4-oxopent-2-enoyl)-3,8-diazabicyclo[3 2. 1]octan-3-yl) quinazolin-6-yl) pyridin-3-yl) benzenesulfon amide | 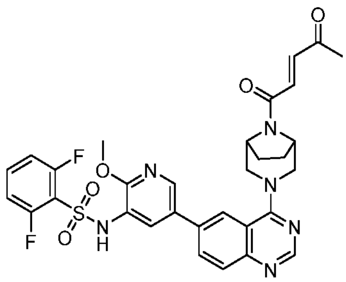 | $^1$H NMR (400 MHz, DMSO-$d_6$) δ 10.58 (s, 1H), 8.60 (s, 1H), 8.42 (s, 1H), 8.09-8.02 (m, 2H), 7.98 (s, 1H), 7.88 (d, *J* = 8.0 Hz, 1H), 7.73-7.64 (m, 1H), 7.41 (d, *J* = 12.0 Hz, 1H), 7.26 (t, *J* = 8.0 Hz, 2H), 6.84 (d, *J* = 16.0 Hz, 1H), 4.80-4.72 (m, 2H), 4.56-4.44 (m, 2H), 3.64 (s, 3H), 3.63-3.55 (m, 2H), 2.39 (s, 3H), 1.97-1.76 (m, 4H); ESI-MS(m/z): 635.0 [M+H]$^+$. |
| **Example 146** | 2,4-difluoro-*N*-(5-(4-(8-(2-fluoroacryloyl)-3,8-diazabicyclo [3 2. 1] octan-3-yl) quinazolin-6-yl)-2-methoxypyridin-3-yl) benzenesulfon amide | 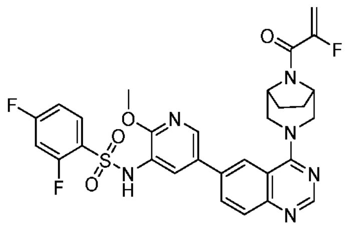 | $^1$H NMR (400 MHz, DMSO-$d_6$) δ 10.34 (s, 1H), 8.60 (s, 1H), 8.45 (s, 1H), 8.09-8.04 (m, 2H), 8.00-7.98 (m, 1H), 7.88 (d, *J* = 8.0 Hz, 1H), 7.80-7.73 (m, 1H), 7.60-7.53 (m, 1H), 7.24-7.18 (m, 1H), 5.52-5.41 (m, 1H), 5.40-5.36 (m, 1H), 4.72-4.60 (m, 2H), 4.51 (d, *J* = 16.0 Hz, 2H), 3.67 (s, 3H), 3.65-3.59 (m, 2H), 1.93-1.75 (m, 4H); ESI-MS(m/z): 611.0 [M+H]$^+$. |
| **Example 147** | *N*-(5-(4-((1-acryloylpyrrolidi n-3-yl)amino)quinazo lin-6-yl)-2-methoxypyridin-3-yl)-2,4-difluorobenzenes ulfonamide | 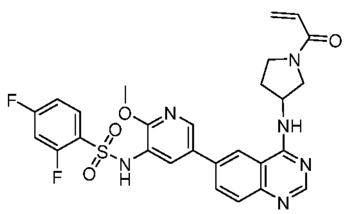 | $^1$H NMR (400 MHz, DMSO-$d_6$) δ 8.55-8.52 (m, 2H), 8.31-8.26 (m, 1H), 8.17 (s, 1H),7.91 (d, *J* =8.0 Hz, 1H), 7.84-7.76 (m, 3H), 7.40 (t, *J* =8.0 Hz, 1H), 7.16-7.11 (m, 1H), 6.68-6.56 (m, 1H), 6.19-6.13 (m, 1H), 5.71-5.65 (m, 1H), 4.92-4.77 (m, 1H), 4.08-4.04 (m, 1H), 3.88-3.50 (m, 7H), 2.39-2.22 (m, 1H), 2.20-2.03 (m, 1H); ESI-MS(m/z): 567.0 [M+H]$^+$. |
| **Example 148** | (*E*)-2,4-difluoro-*N*-(2-methoxy-5-(4-((1-(4-oxopent-2-enoyl) pyrrolidin-3-yl) amino)quinazo lin-6-yl)pyridin-3-yl) benzenesulfon amide | 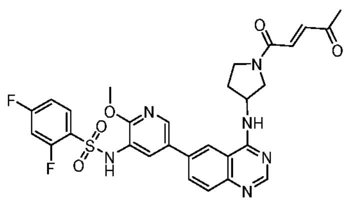 | $^1$H NMR (400 MHz, DMSO-$d_6$) δ 10.37 (s, 1H), 8.60 (s, 1H), 8.55 (d, *J* =4.0 Hz, 1H), 8.48 (s, 1H), 8.34-8.30 (s, 1H), 8.09-8.06 (m, 2H), 7.80 (dd, *J* =4.0 Hz, 8.0 Hz, 1H), 7.76-7.70 (m, 1H), 7.61-7.56 (m, 1H), 7.25-7.16 (m, 2H), 6.76 (t, *J* =16.0 Hz, 1H), 4.96-4.80 (m, 1H), 4.19-4.15 (m, 1H), 3.93-3.83 (m, 2H), 3.75-3.55 (m, 5H), 2.36 (d, *J* =8.0 Hz, 3H), 2.22-2.11 (m, 1H); ESI-MS(m/z): 609.0 [M+H]$^+$. |
| **Example 149** | 4-fluoro-*N*-(2-methoxy-5-(4-(8-((*E*)-4-oxopent-2-enoyl)-3,8-diazabicyclo [3 2. 1]octan-3-yl) quinazolin-6-yl) pyridin-3-yl) benzenesulfon amide | 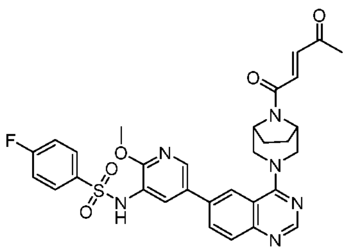 | $^1$H NMR (400 MHz, DMSO-$d_6$) δ 10.10 (s, 1H), 8.61 (s, 1H), 8.42 (s, 1H), 8.07-8.03 (m, 2H),7.96 (s, 1H), 7.89 (d, *J* =8.0 Hz, 1H), 7.85-7.80 (m, 2H), 7.44-7.39 (m, 3H), 6.84 (d, *J* =16.0 Hz, 1H), 4.80-4.75 (m, 2H), 4.55-4.46 (m, 2H), 3.68 (s, 3H), 2.63-3.57 (m, 2H), 2.40 (s, 3H), 1.97-1.91 (m, 1H),1.84-1.80 (m, 3H); ESI-MS(m/z): 617.0 [M+H]$^+$. |

(continued)

| Number | Name | Structure | $^1$H NMR and MS |
|---|---|---|---|
| **Example 150** | (*E*)-2-fluoro-*N*-(2-methoxy-5-(4-(2-(4-oxopent-2-enoyl)-2,7-diazaspiro[3.5]nonan-7-yl)quinazolin-6-yl)pyridin-3-yl) benzenesulfon amide | 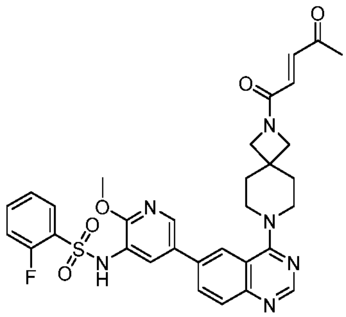 | $^1$H NMR (400 MHz, DMSO-$d_6$) δ 10.20 (s, 1H), 8.63 (s, 1H), 8.42 (d, *J* =4 Hz, 1H), 8.11-8.01 (m, 2H), 7.98 (d, *J* = 4 Hz, 1H), 7.89 (d, *J* = 8 Hz, 1H), 7.73-7.70 (m, 2H), 7.46-7.43 (m, 1H), 7.35-7.33(m, 1H), 6.93 (d, *J* = 16 Hz, 1H), 6.74 (d, *J* = 16 Hz, 1H), 4.15 (s, 2H), 3.84-3.70 (m, 6H), 3.67 (s, 3H), 2.35 (s, 3H), 1.96 (t, *J* = 8 Hz, 4H); ESI-MS(m/z): 631.0 [M+H]$^+$. |
| **Example 151** | 2-fluoro-*N*-(2-methoxy-5-(4-(8-((*E*)-4-oxopent-2-enoyl)-3,8-diazabicyclo[3 2. 1]octan-3-yl) quinazolin-6-yl) pyridin-3-yl) benzenesulfon amide | 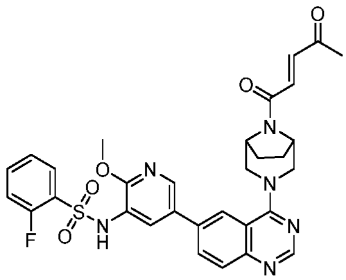 | $^1$H NMR (400 MHz, DMSO-$d_6$) δ 10.25 (s, 1H), 8.60 (s, 1H), 8.44 (s, 1H), 8.08-8.05 (m, 2H), 8.03 (s, 1H), 7.88 (d, *J* = 8 Hz, 1H), 7.74-7.69 (m, 2H), 7.46-7.33 (m, 3H), 6.85 (d, *J* = 16 Hz, 1H), 4.78-4.74 (m, 2H), 4.63-4.48 (m, 2H), 3.65 (s, 3H), 3.63-3.56 (m, 2H), 2.39 (s, 3H), 1.82-1.77 (m, 4H); ESI-MS(m/z): 617.0 [M+H]$^+$. |
| **Example 152** | (*S*,*E*)-2-fluoro-*N*-(2-methoxy-5-(4-(2-methyl-4-(4-oxopent-2-enoyl)piperazin-1-yl)quinazolin-6-yl) pyridin-3-yl) benzenesulfon amide | 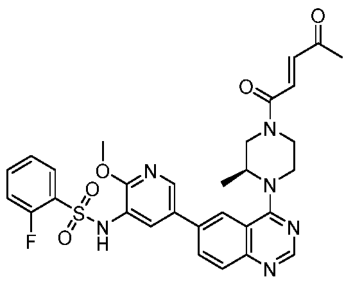 | $^1$H NMR (400 MHz, DMSO-$d_6$) δ 8.64 (s, 1H), 8.05 (s, 1H), 7.97-7.95 (m, 2H), 7.89-7.86 (m, 1H), 7.77-7.73 (m, 2H), 7.51-7.44 (m, 2H), 7.38 (d, *J* =16.0 Hz, 1H), 7.30-7.21 (m, 2H), 6.76 (dd, *J* =12.0 Hz, 16.0 Hz, 1H), 4.73-4.70 (m, 1H), 4.41-4.25 (m, 1H), 4.20-3.96 (m, 2H), 3.74 (s, 3H), 3.67-3.46 (m, 2H), 3.21-3.10 (m, 1H), 2.38 (d, *J* =4.0 Hz, 3H), 1.31-1.28 (m, 3H); ESI-MS(m/z): 605.0 [M+H]$^+$. |
| **Example 153** | (*S*,*E*)-4-fluoro-*N*-(2-methoxy-5-(4-(2-methyl-4-(4-oxopent-2-enoyl)piperazin-1-yl)quinazolin-6-yl) pyridin-3-yl) benzenesulfon amide | 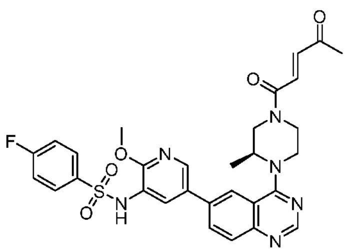 | $^1$H NMR (400 MHz, DMSO-$d_6$) δ 10.12 (s, 1H), 8.67 (s, 1H), 8.45-8.42 (m, 1H), 8.11-8.04 (m, 2H), 8.01-7.98 (m, 1H), 7.92 (d, *J* = 8.0 Hz, 1H), 7.85-7.80 (m, 2H), 7.53-7.43 (m, 1H), 7.43-7.36 (m, 2H), 6.80-6.71 (m, 1H), 4.85-4.71 (m, 1H), 4.45-3.97 (m, 3H), 3.68 (s, 3H), 3.65-3.47 (m, 2H), 3.23-3.12 (m, 1H), 2.37 (d, *J* = 8.0 Hz, 3H), 1.33 (t, *J* = 8.0 Hz, 3H); ESI-MS(m/z): 605.0 [M+H]$^+$. |
| **Example 154** | (*S*,*E*)-2,4,6-trifluoro-*N*-(2-methoxy-5-(4-(2-methyl-4-(4-oxopent-2-enoyl) piperazin-1-yl) quinazolin-6-yl) pyridin-3-yl) benzenesulfon amide | 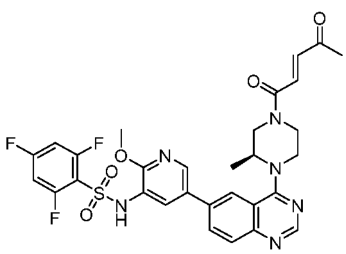 | $^1$HNMR (400 MHz, DMSO-$d_6$) δ 10.65 (s, 1H), 8.67 (s, 1H), 8.48 (s, 1H), 8.13-8.00 (m, 3H), 7.92 (d, *J* = 8 Hz, 1H), 7.53-7.33 (m, 3H), 6.75 (dd, $J_1$ = 16, $J_2$ = 8 Hz, 1H), 4.79 (m, 1H), 4.41-4.19 (m, 2H), 4.20-3.97 (m, 1H), 3.69 (s, 3H), 3.62-3.44 (m, 2H), 3.25-3.11 (m, 1H), 2.37 (s, 3H), 1.32 (t, *J* = 8 Hz, 3H); ESI-MS(m/z): 641.0 [M+H]$^+$. |

(continued)

| Number | Name | Structure | $^1$H NMR and MS |
|---|---|---|---|
| **Example 155** | 2,4,6-trifluoro-*N*-(2-methoxy-5-(4-(8-((*E*)-4-oxopent-2-enoyl))-3,8-diazabicyclo[3 2. 1]octyl-3-yl) quinazolin-6-yl) pyridin-3-yl) benzenesulfon amide | 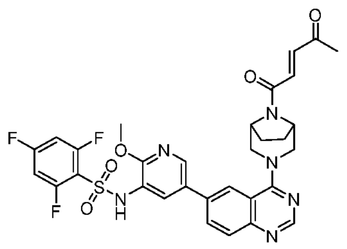 | $^1$H NMR (400 MHz, DMSO-$d_6$) δ 10.66 (s, 1H), 8.61 (s, 1H), 8.45 (s, 1H), 8.13-7.97 (m, 3H), 7.89 (d, *J* = 8.8 Hz, 1H), 7.47-7.35 (m, 3H), 6.84 (d, *J* = 15.6 Hz, 1H), 4.83-4.71 (m, 2H), 4.51 (dd, *J* = 26.8, 12.0 Hz, 2H), 3.72-3.55 (m, 5H), 2.40 (s, 3H), 2.00-1.74 (m, 4H); ESI-MS (m/z): 653.0 [M+H]$^+$. |
| **Example 156** | 2,4,6-trifluoro-*N*-(5-(4-(8-(2-fluoroacryloyl)-3,8-diazabicyclo[3 2. 1] octan-3-yl) quinazolin-6-yl)-2-methoxypyridin-3-yl) benzenesulfon amide | 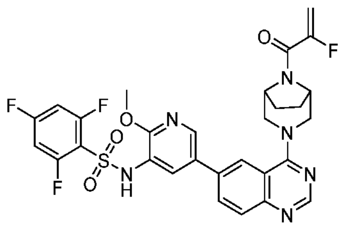 | $^1$H NMR (400 MHz, DMSO-$d_6$) δ 10.67 (s, 1H), 8.61 (s, 1H), 8.50 (s, 1H), 8.13-8.01 (m, 3H), 7.89 (d, *J* = 8.8 Hz, 1H), 7.47-7.37 (m, 2H), 5.47 (dd, *J* = 36.8, 3.6 Hz, 1H), 5.40-5.33 (m, 1H), 4.75-4.57 (m, 2H), 4.56-4.48 (m, 2H), 3.71-3.55 (m, 5H), 1.96-1.72 (m, 4H); ESI-MS(m/z): 629.0 [M+H]$^+$. |
| **Example 157** | (*E*)-2,4-difluoro-*N*-(2-methoxy-5-(4-(5-(4-oxopent-2-enoyl) hexahydro pyrrolo [3,4-*c*]pyrrol-2 (1*H*)-yl)quinazolin-6-yl)pyridin-3-yl) benzenesulfon amide | 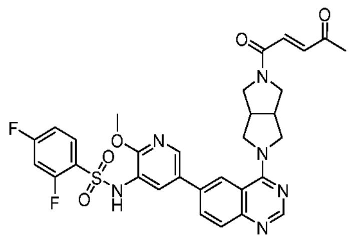 | $^1$H NMR (400 MHz, DMSO-$d_6$) δ 10.38 (s, 1H), 8.52-8.32 (m, 3H), 8.07-7.95 (m, 2H), 7.83-7.70 (m, 2H), 7.63-7.52 (m, 1H), 7.25-7.10 (m, 2H), 6.72 (d, *J* = 16.0 Hz, 1H), 4.31-4.17 (m, 2H), 4.04-3.89 (m, 3H), 3.79-3.70 (m, 2H), 3.66 (s, 3H), 3.53-3.42 (m, 1H), 3.21-3.02 (m, 2H), 2.33 (s, 3H); ESI-MS(m/z): 635.0 [M+H]$^+$. |
| **Example 158** | (*E*)-2,4-difluoro-*N*-(2-methoxy-5-(4-(5-(4-oxopent-2-enoyl)-2,5-diazabicyclo[2.2. 2]octan-2-yl) quinazolin-6-yl) pyridin-3-yl) benzenesulfon amide | 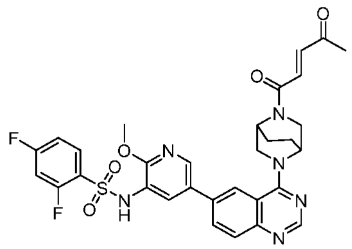 | $^1$H NMR (400 MHz, DMSO-$d_6$) δ 10.34 (s, 1H), 8.65 (s, 1H), 8.50-8.47 (m, 1H), 8.33 (d, *J* = 8.0 Hz, 1H), 8.13 (d, *J* = 16.0 Hz, 1H), 8.04-8.01 (m, 1H), 7.86 (d, *J* = 8.0 Hz, 1H), 7.79-7.72 (m, 1H), 7.61-7.54 (m, 1H), 7.40 (d, *J* = 16.0 Hz, 1H), 7.24-7.16 (m, 1H), 6.83-6.69 (m, 1H), 5.23 (s, 1H), 4.80-4.58 (m, 1H), 4.56-4.43 (m, 1H), 4.31-4.23 (m, 1H), 4.14-3.99 (m, 1H), 3.83-3.69 (m, 1H), 3.67-3.64 (m, 3H), 2.36-2.34 (m, 3H), 2.28-2.18 (m, 1H), 2.03-1.86 (s, 3H); ESI-MS(m/z): 635.0 [M+H]$^+$. |
| **Example 159** | (*E*)-2,4-difluoro-*N*-(5-(4-(4-(4-oxopent-2-enoyl) piperazin-1-yl) quinazolin-6-yl)-2-(2,2,2-trifluoroethoxy)p yridin-3-yl) benzenesulfon amide | 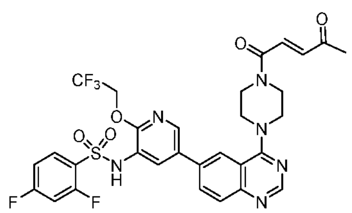 | $^1$H NMR (400 MHz, DMSO-$d_6$) δ 10.58 (s, 1H), 8.67 (s, 1H), 8.47 (s, 1H), 8.16-8.08 (m, 3H), 7.93 (d, *J* = 8.0 Hz, 1H), 7.84-7.77 (m, 1H), 7.50-7.42 (m, 2H), 7.22-7.15 (m, 1H), 6.77-6.70 (m, 1H), 4.94-4.86 (m, 2H), 3.93-3.86 (m, 6H), 3.83-3.78 (m, 2H), 2.37 (s, 3H); ESI-MS(m/z): 677.0 [M+H]$^+$. |

(continued)

| Number | Name | Structure | $^1$H NMR and MS |
|---|---|---|---|
| **Example 160** | (*E*)-*N*-(2-methoxy-5-(4-(4-(4-oxopent-2-enoyl)piperazin-1-yl) quinazolin-6-yl) pyridin-3-yl)-2,4-dimethylthiazole-5-sulfonamide | 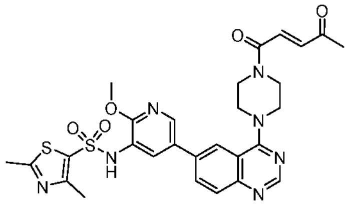 | $^1$H NMR (400 MHz, DMSO-$d_6$) δ 10.39 (s, 1H), 8.68 (s, 1H), 8.53 (d, *J* = 4.0 Hz, 1H), 8.14-8.09 (m, 2H), 8.04 (d, *J*= 4.0 Hz, 1H), 7.94 (d, J=8.0 Hz, 1H), 7.46 (d, *J* =16.0 Hz, 1H), 6.74 (d, *J* = 16.0 Hz, 1H), 3.92-3.80 (m, 8H),3.74 (s, 3H), 2.60 (s, 3H), 2.38 (s, 6H); ESI-MS(m/z): 608.0 [M+H]$^+$. |

**Example 161**

**Preparation of (*E*)-4-fluoro-*N*-(2-methoxy-5-(4-(2-(4-oxopent-2-enoyl)-2,7-diazaspiro[3.5]nonan-7-yl)quinazolin-6-yl)pyridin-3-yl)benzenesulfonamide**

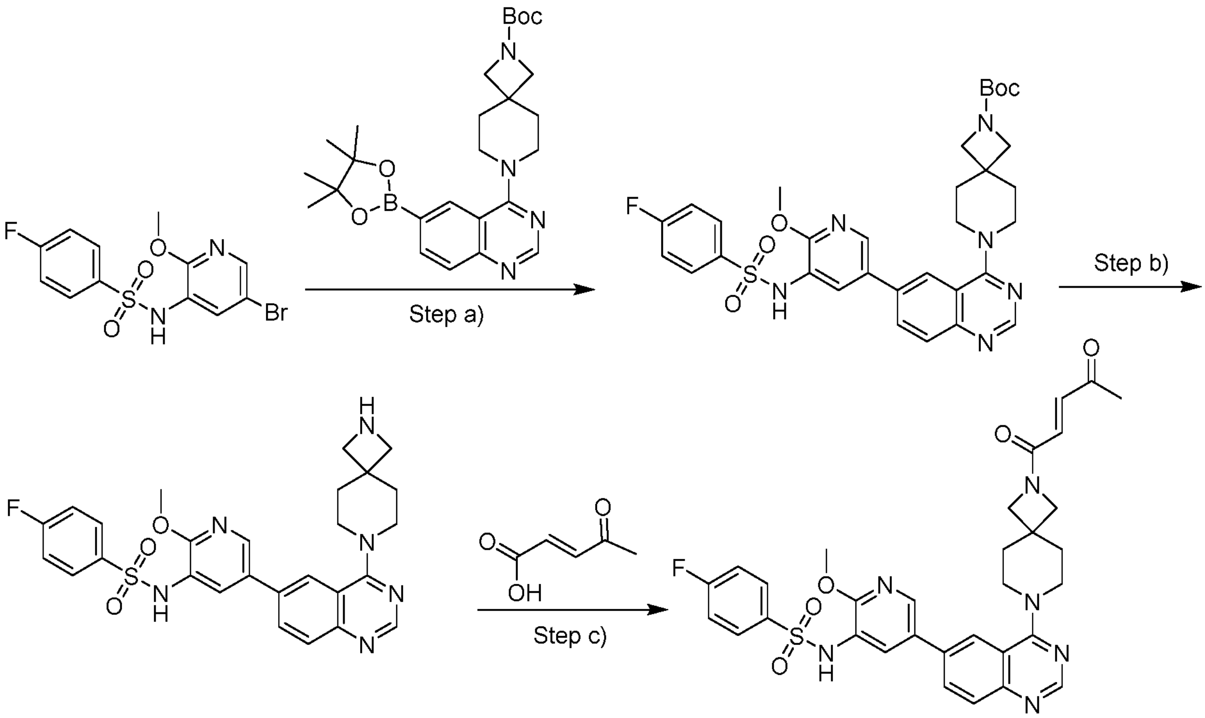


Step a): Preparation of tert-butyl 7-(6-(5-((4-fluorophenyl)sulfonamido)-6-methoxypyridin-3-yl)quinazolin-4-yl)-2,7-diazaspiro [3 .5]nonane-2-carboxylate

*N*-(5-bromo-2-methoxypyridin-3-yl)-4-fluorobenzenesulfonamide (100 mg, 0.277 mmol), tert-butyl 7-(6-(4,4,5,5-tetramethyl-1,3,2-dioxaborolan-2-yl)quinazolin-4-yl)-2,7-diazaspiro[3.5]nonane-2-carboxylate (133 mg, 0.277 mmol), Pd(dppf)$Cl_2$ (20 mg, 0.027mmol) and cesium carbonate (180 mg, 0.554 mmol) were added to dioxane/water mixed solvent (4.4 mL, v/v=10:1). After the addition was completed, in the presense of protective nitrogen, the reaction mixture was heated to 100°C and stirred for 3 h. Upon completion of the reaction, the reaction was quenched by adding water (10 mL). The reaction mixture was extracted by adding dichloromethane (10 mL×3). The organic phases were combined, dried over anhydrous sodium sulfate and filtered. The filtrate was concentrated under reduced pressure. The crude product obtained was purified by silica gel column chromatography (Eluent: dichloromethane/methanol=1/0 to 20/1). Tert-butyl 7-(6-(5-((4-fluorophenyl)sulfonamido)-6-methoxypyridin-3-yl)quinazolin-4-yl)-2,7-diazaspiro[3.5]nonane-2-carboxylate was obtained, yield: 63.9%; ESI-MS (m/z): 635.6 [M+H]$^+$.

Step b): Preparation of *N*-(5-(4-(2,7-diazaspiro[3.5]nonan-7-yl)quinazolin-6-yl)-2-methoxypyridin-3-yl)-4-fluorobenzenesulfonamide trifluoroacetate

Tert-butyl 7-(6-(5-((4-fluorophenyl)sulfonamido)-6-methoxypyridin-3-yl)quinazolin-4-yl)-2,7-diazaspiro[3.5]nonane-2-carboxylate (120 mg, 0.190 mmol) and dichloromethane (4 mL) were added to the reaction flask. With the reaction solution cooled to 0°C, trifluoroacetic acid (2 mL) was slowly added. After the addition was completed, the reaction mixture was naturally heated to room temperature and reacted for 0.5 h. Upon completion of the reaction, the reaction mixture was concentrated under reduced pressure. *N*-(5-(4-(2,7-diazaspiro[3.5]nonan-7-yl)quinazolin-6-yl)-2-methoxypyridin-3-yl)-4-fluorobenzenesulfonamide trifluoroacetate was obtained; ESI-MS (m/z): 535.2 $[M+H]^+$.

Step c): Preparation of (*E*)-4-fluoro-*N*-(2-methoxy-5-(4-(2-(4-oxopent-2-enoyl)-2,7-diazaspiro[3.5]nonan-7-yl)quinazolin-6-yl)pyridin-3-yl)benzenesulfonamide

*N*-(5-(4-(2,7-diazaspiro[3.5]nonan-7-yl)quinazolin-6-yl)-2-methoaypyridin-3-yl)-4-fluorobenzenesulfonamide trifluoroacetate (150 mg, 0.230 mmol) and tetrahydrofuran (2 mL) were added to the reaction flask. With the reaction solution cooled to -78°C, DIPEA (180 mg, 1.380 mmol), (*E*)-4-oxopent-2-enoic acid (26 mg, 0.230 mmol) and 50% $T_3P$ ethyl acetate solution (150 mg, 0.230 mmol) were added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). (*E*)-4-fluoro-*N*-(2-methoxy-5-(4-(2-(4-oxopent-2-enoyl)-2,7-diazaspiro[3.5]nonan-7-yl)quinazolin-6-yl)pyridin-3-yl)benzenesulfonamide was obtained, yield: 62.4%; $^1H$ NMR (400 MHz, DMSO-$d_6$) δ 10.04 (s, 1H), 8.64 (s, 1H), 8.40 (d, *J* = 4.0 Hz, 1H), 8.08-8.01 (m, 2H),7.96 (d, *J* = 4.0 Hz, 1H), 7.90 (d, *J* = 8.0 Hz, 1H), 7.85-7.81 (m, 2H), 7.45-7.39 (m, 2H), 6.93 (d, *J* = 16.0 Hz, 1H), 6.74 (d, *J* = 16.0 Hz, 1H), 4.15 (s, 2H), 3.80-3.70 (m, 9H), 2.35 (s, 3H), 1.98-1.95 (m, 4H); ESI-MS(m/z): 631.0 $[M+H]^+$.

Referring to the preparation method of Example 161 and using the corresponding raw materials, the compounds in the following examples were obtained.

| Number | Name | Structure | $^1H$ NMR and MS |
|---|---|---|---|
| **Example 162** | 3,5-difluoro-*N*-(2-methoxy-5-(4-(8-((*E*)-4-oxopent-2-enoyl)-3,8-diazabicyclo [3.2.1] octan-3-yl) quinazolin-6-yl) pyridin-3-yl)pyridine-4-sulfonamide | 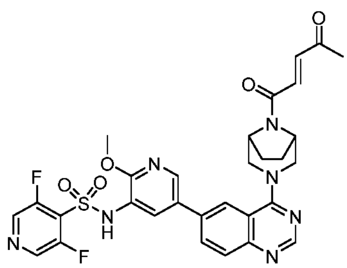 | $^1H$ NMR (400 MHz, DMSO-$d_6$) δ 11.20 (brs, 1H), 8.76 (s, 2H), 8.61 (s, 1H), 8.49 (s, 1H), 8.18-7.98 (m, 3H), 7.89 (d, *J* = 8.0 Hz, 1H), 7.41 (d, *J* = 16.0 Hz, 1H), 6.85 (d, *J* = 16.0 Hz, 1H), 4.83-4.71 (m, 2H), 4.59-4.45 (m, 2H), 3.69-3.50 (m, 5H), 2.40 (s, 3H), 1.98-1.74 (m, 4H); ESI-MS(m/z): 636.0 $[M+H]^+$. |
| **Example 163** | (*E*)-3,5-difluoro-*N*-(2-methoxy-5-(4-(2-(4-oxopent-2-enoyl)-2,7-diazaspiro [3.5] non an-7-yl)quinazolin-6-yl)pyridin-3-yl) pyridine-4-sulfonamide | 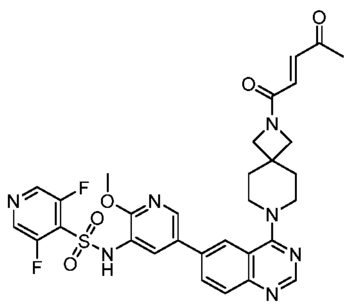 | $^1H$ NMR (400 MHz, DMSO-$d_6$) δ 11.20 (brs, 1H), 8.68 (s, 2H), 8.63 (s, 1H), 8.32 (s, 1H), 8.10-8.00 (m, 2H), 8.00-7.92 (m, 1H), 7.89 (d, *J* = 8.0 Hz, 1H), 6.94 (d, *J* = 16.0 Hz, 1H), 6.74 (d, *J* = 16.0 Hz, 1H), 4.15 (s, 2H), 3.80 (s, 2H), 3.78-3.67 (m, 4H), 3.65 (s, 3H), 2.35 (s, 3H), 2.02-1.86 (m, 4H); ESI-MS(m/z): 650.0 $[M+H]^+$. |

(continued)

| Number | Name | Structure | $^1$H NMR and MS |
|---|---|---|---|
| **Example 164** | (*S*,*E*)-3,5-difluoro-*N*-(2-methoxy-5-(4-(2-methyl-4-(4-oxopent-2-enoyl)piperazin-1-yl)quinazolin-6-yl) pyridin-3-yl)pyridine-4-sulfonamide | 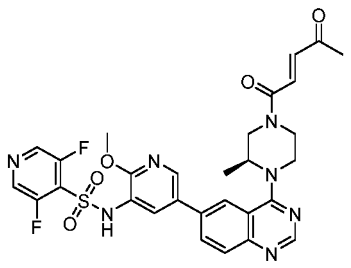 | $^1$H NMR (400 MHz, DMSO-$d_6$) δ 8.65 (s, 1H), 8.45 (s, 1H), 7.98-7.87 (m, 3H), 7.72 (d, *J* = 4.0 Hz, 1H), 7.45 (dd, $J_1$ = 48.0 Hz, $J_2$ = 16.0, 1H), 7.20-7.01 (m, 3H), 6.75 (dd, $J_1$ = 16.0 Hz, $J_2$ = 8.0.Hz, 1H), 4.81-4.67 (m, 1H), 4.50-3.94 (m, 3H), 3.74 (s, 3H), 3.69-3.45 (m, 2H), 3.25-3.09 (m, 1H), 2.38 (d, *J* = 4.0 Hz, 3H), 1.30 (t, *J* = 4.0 Hz, 3H); ESI-MS (m/z): 624.0 [M+H]$^+$. |
| **Example 165** | *(*E)-5-chloro-*N*-(2-methoxy-5-(4-(4-(4-oxopent-2-enoyl) piperazin-1-yl) quinazolin-6-yl) pyridin-3-yl)thiazole-2-sulfonamide | 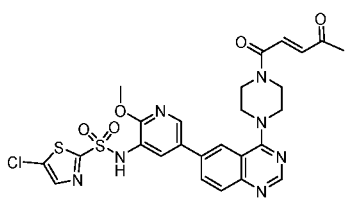 | $^1$H NMR (400 MHz, DMSO-$d_6$) δ 11.03 (s, 1H), 8.68 (s, 1H), 8.48 (s, 1H), 8.20-8.08 (m, 4H), 7.93 (d, *J* = 8.8 Hz, 1H), 7.44 (d, *J* = 15.6 Hz, 1H), 6.73 (d, *J* = 16.0 Hz, 1H), 3.97-3.77 (m, 8H), 3.76 (s, 3H), 2.36 (s, 3H); ESI-MS(m/z): 614.0 [M+H]$^+$. |

**Example 166**

**Preparation of (*E*)-*N*-(2,4-difluorophenyl)-2-methoxy-5-(4-(4-(4-oxopent-2-enoyl)piperazin-1-yl)quinazolin-6-yl)pyridine-3-sulfonamide**

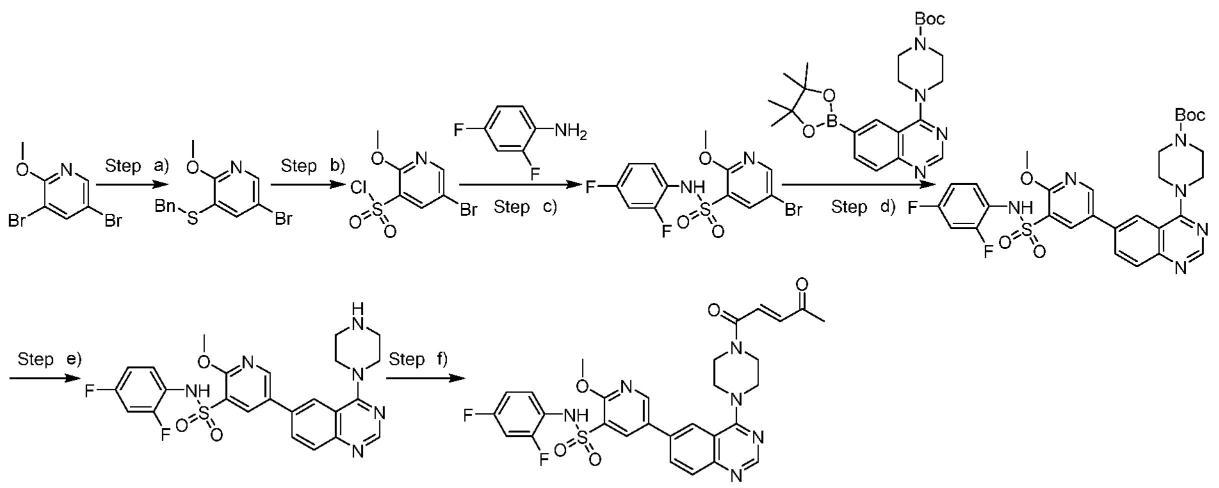


Step a): Preparation of 3-(benzylthio)-5-bromo-2-methoxypyridine

Sodium hydride (720 mg, 17.994 mmol) was added to three-necked bottle. In the presense of protective nitrogen, *N,N*-dimethylformamide (50 mL) was added to the system. The reaction system was cooled to 0°C, with benzylthiol (1.2 g, 14.994 mmol) slowly added, stirred and reacted for 10 min. Then with 3,5-dibromo-2-methoxypyridine (4.0 g, 14.994 mmol) added, the reaction mixture was kept at room temperature and reacted for 3 h. Upon completion of the reaction, the reaction solution was poured into iced water (45 mL) and extracted with ethyl acetate (80 mL). The organic phases were combined, washed with saturated sodium chloride (80 mL×3), dried over anhydrous sodium sulfate and filtered. The filtrate was concentrated under reduced pressure. The crude product obtained was purified by silica gel column chromatography (Eluent: petroleum ether/ethyl acetate = 1/0 to 10/1). 3-(Benzylthio)-5-bromo-2-methoxypyridine was

obtained, yield: 68.8%; $^1$H NMR (400 MHz, DMSO-$d_6$) δ 8.04 (d, *J* = 2.2 Hz, 1H), 7.77 (d, *J* = 2.2 Hz, 1H), 7.43-7.37 (m, 2H), 7.36-7.29 (m, 2H), 7.29-7.23 (m, 1H), 4.29 (s, 2H), 3.89 (s, 3H); ESI-MS(m/z): 309.9 [M+H]$^+$.

Step b): Preparation of 5-bromo-2-methoxypyridine-3-sulfonyl chloride

3-(Benzylthio)-5-bromo-2-methoxypyridine (600 mg, 1.934 mmol), acetic acid (6 mL) and water (2 mL) were added to the reaction flask, with N-chlorosuccinimide (1.0 g, 7.489 mmol) added in portions. After the addition was completed, the reaction mixture was kept at room temperature and reacted for 3 h. Upon completion of the reaction, the reaction mixture was concentrated under reduced pressure . The residue was dissolved by adding ethyl acetate (25 mL), washed with water (25 mL×3), dried over anhydrous sodium sulfate and filtered. The filtrate was concentrated under reduced pressure. 5-Bromo-2-methoxypyridine-3-sulfonyl chloride was obtained. The product can be used directly in the next reaction without purification.

Step c): Preparation of 5-bromo-*N*-(2,4-difluorophenyl)-2-methoxypyridine-3-sulfonamide

2,4-Difluoroaniline(248 mg, 1.921 mmol), 4-dimethylaminopyridine (24 mg, 0.194 mmol) and pyridine (456 mg, 5.764 mmol) were added to the reaction flask where 5-bromo-2-methoxypyridine-3-sulfonyl chloride (500 mg, crude product) was placed. In the presense of protective nitrogen, the reaction mixture was kept at room temperature and reacted for 15 h. Upon completion of the reaction, the reaction was quenched by adding saturated aqueous ammonium chloride solution (40 mL), and the reaction mixture was extracted with dichloromethane (30 mL×2). The organic phases were combined, dried over anhydrous sodium sulfate and filtered. The filtrate was concentrated under reduced pressure. The crude product obtained was slurried with petroleum ether/ethyl acetate mixed solvent (5 mL, v/v=3/1) and filtered. 5-Bromo-*N*-(2,4-difluorophenyl)-2-methoxypyridine-3-sulfonamide was obtained, two-step yield: 92.8%; ESI-MS(m/z): 378.9 [M+H]$^+$.

Step d): Preparation of tert-butyl 4-(6-(5-(*N*-(2,4-difluorophenyl)sulfamoyl)-6-methoxypyridin-3-yl)quinazolin-4-yl)piperazine-1-carboxylate

5-Bromo-*N*-(2,4-difluorophenyl)-2-methoxypyridine-3-sulfonamide (170 mg, 0.448 mmol), tert-butyl 4-(6-(4,4,5,5-tetramethyl-1,3,2-dioxaborolan-2-yl)quinazolin-4-yl)piperazine-1-carboxylate (200 mg, 0.454 mmol), Pd(dppf)$Cl_2$ (33 mg, 0.045 mmol), cesium carbonate (440 mg, 1.352 mmol) and dioxane (15 mL)/water (5 mL) mixed solvent were added to the reaction flask. After the addition was completed, in the presense of protective nitrogen, the reaction mixture was kept at 100°C and stirred for 5 h. Upon completion of the reaction, the reaction mixture was cooled to room temperature, with the reaction quenched by adding saturated ammonium chloride (30 mL), extracted with ethyl acetate (30 mL×2). The organic phases were combined, dried over anhydrous sodium sulfate and filtered. The filtrate was concentrated under reduced pressure. The crude product obtained was purified by silica gel column chromatography (Eluent: dichloromethane/methanol = 1/0 to 35/1). Tert-butyl 4-(6-(5-(*N*-(2,4-difluorophenyl)sulfamoyl)-6-methoxypyridin-3-yl)quinazolin-4-yl)piperazine-1-carboxylate was obtained, yield: 85.3%; ESI-MS(m/z): 613.5 [M+H]$^+$.

Step e): Preparation of *N*-(2,4-difluorophenyl)-2-methoxy-5-(4-(piperazin-1-yl)quinazolin-6-yl)pyridine-3-sulfonamide trifluoroacetate

Tert-butyl 4-(6-(5-(*N*-(2,4-difluorophenyl)sulfamoyl)-6-methoxypyridin-3-yl)quinazofin-4-yl)piperazine-1-carboxylate (331 mg, 0.540 mmol) was dissolved in dichloromethane (9 mL). Under the ice bath condition, TFA (3 mL) was slowly added dropwise. After the addition was completed, the reaction mixture was stirred at room temperature for 1 h. Upon completion of the reaction, the reaction mixture was concentrated under reduced pressure. The crude product obtained was purified by slurrying with methyl tert-butyl ether (10 mL) for 0.5 h, filtered and vacuum dried. *N*-(2,4-difluorophenyl)-2-methoxy-5-(4-(piperazin-1-yl)quinazolin-6-yl)pyridine-3-sulfonamide trifluoroacetate was obtained, yield: 81.4%; ESI-MS(m/z): 513.0 [M+H]$^+$.

Step f): Preparation of (*E*)-*N*-(2,4-difluorophenyl)-2-methoxy-5-(4-(4-(4-oxopent-2-enoyl)piperazin-1-yl)quinazolin-6-yl)pyridine-3-sulfonamide

*N*-(2,4-difluorophenyl)-2-methoxy-5-(4-(piperazin-1-yl)quinazolin-6-yl)pyridine-3-sulfonamide trifluoroacetate (100 mg, 0.160 mmol), (*E*)-4-oxopent-2-enoic acid (27 mg, 0.234 mmol), DIPEA (126 mg, 0.976 mmol) and tetrahydrofuran (2 mL) were added to the reaction flask. At -78°C, 50% 1-propylphosphonic anhydride ethyl acetate solution (149 mg, 0.234 mmol) was slowly added to the system. The reaction solution was kept at -78°C and reacted for 1 h. Upon completion of the reaction, the reaction solution was diluted by adding acetonitrile (1 mL) and purified by Prep-HPLC

(Method 2). (*E*)-*N*-(2,4-difluorophenyl)-2-methoxy-5-(4-(4-(4-oxopent-2-enoyl)piperazin-1-yl)quinazolin-6-yl)pyridine-3-sulfonamide was obtained, yield: 27.3%; $^1$H NMR (400 MHz, DMSO-$d_6$) δ 10.20 (s, 1H), 8.88 (d, *J* = 2.4 Hz, 1H), 8.66 (s, 1H), 8.30 (d, *J* = 2.8 Hz, 1H), 8.15 (d, *J* = 2.0 Hz, 1H), 8.09 (dd, *J* = 8.8, 2.0 Hz, 1H), 7.90 (d, *J* = 8.8 Hz, 1H), 7.45 (d, *J* = 16.0 Hz, 1H), 7.38-7.29 (m, 1H), 7.27-7.19 (m, 1H), 7.07-6.98 (m, 1H), 6.74 (d, *J* = 16.0 Hz, 1H), 4.01 (s, 3H), 3.95-3.74 (m, 8H), 2.37 (s, 3H); ESI-MS(m/z): 609.0 [M+H]$^+$.

**Example 167**

**Preparation of 2,4-difluoro-*N*-(5-(4-(4-(3-hydroxy-2-methylenebutanoyl)piperazin-1-yl)quinazolin-6-yl)-2-methoxypyridin-3-yl)benzenesulfonamide**

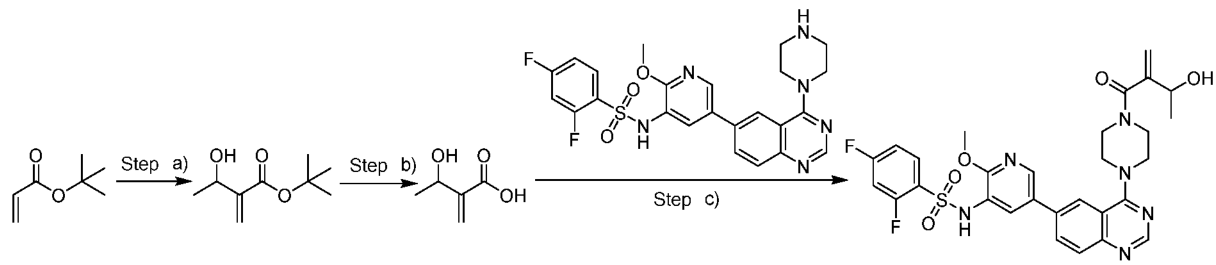


Step a): Preparation of tert-butyl 3-hydroxy-2-methylenebutanoate

Triethylenediamine (6.4 g, 57.056 mmol), dioxane (10 mL), water (10 mL) and tert-butyl acrylate (14.6 g, 113.672 mmol) were placed in sealed tube reactor. With acetaldehyde (22.5 mL, 113.672 mmol, 5 M tetrahydrofuran solution) added, the reaction solution was kept at room temperature and reacted for 48 h. Upon completion of the reaction, water (30 mL) and methyl tert-butyl ether (30 mL) were added to the reaction solution. After that, the aqueous phase was extracted with methyl tert-butyl ether (30 mL×2). The organic phases were combined, washed with saturated aqueous ammonium chloride solution (40 mL) and saturated sodium chloride (40 mL×2) successively, dried over anhydrous sodium sulfate and filtered. The filtrate was concentrated under reduced pressure. The crude product obtained was purified by silica gel column chromatography (Eluent: petroleum ether/ethyl acetate = 1/0 to 10/1). Tert-butyl 3-hydroxy-2-methylenebutanoate was obtained, yield: 17.4%; $^1$H NMR (400 MHz, $CDCl_3$) δ 6.11 (s, 1H), 5.72 (s, 1H), 4.64-4.51 (m, 1H), 1.52 (s, 9H), 1.37 (d, *J* = 6.8 Hz, 3H).

Step b): Preparation of 3-hydroxy-2-methylenebutanoic acid

Tert-butyl 3-hydroxy-2-methylenebutanoate (200 mg, 1.161 mmol) was dissolved in dichloromethane (3 mL). Under the ice bath condition, TFA (1 mL) was slowly added dropwise. After the addition was completed, the reaction mixture was stirred at room temperature for 1 h, then reacted for 0.5 h at 40°C. Upon completion of the reaction, the reaction mixture was concentrated under reduced pressure. The crude product obtained was dispersed in methyl tert-butyl ether (5 mL), concentrated under reduced pressure. 3-Hydroxy-2-methylenebutanoic acid was obtained, yield: 83.3%; $^1$H NMR (400 MHz, $CDCl_3$) δ 6.42 (s, 1H), 5.99 (s, 1H), 4.81-4.64 (m, 1H), 1.45 (d, *J* = 6.8 Hz, 3H).

Step c): Preparation of 2,4-difluoro-*N*-(5-(4-(4-(3-hydroxy-2-methylenebutanoyl)piperazin-1-yl)quinazolin-6-yl)-2-methoxypyridin-3-yl)benzenesulfonamide

2,4-Difluoro-*N*-(2-methoxy-5-(4-(piperazin-1-yl)quinazolin-6-yl)pyridin-3-yl)benzenesulfonamide trifluoroacetate (100 mg, 0.160 mmol), 3-hydroxy-2-methylenebutyric acid (28 mg, 0.241 mmol), DIPEA (129 mg, 1.000 mmol) and tetrahydrofuran (2 mL) were added to the reaction flask. At -78°C, $T_3P$ ethyl acetate solution (155 mg, 0.244 mmol, 50%) was slowly added to the system. The reaction solution was kept at -78°C and reacted fot 1 h. Upon completion of the reaction, the reaction solution was diluted by adding acetonitrile (1 mL), filtered and purified by Prep-HPLC (Method 2). 2,4-Difluoro-N-(5-(4-(4-(3-hydroay-2-methylenebutanoyl)piperazin-1-yl)quinazolin-6-yl)-2-methoxypyridin-3-yl)benzenesulfonamide was obtained, yield: 6.2%; $^1$H NMR (400 MHz, DMSO-$d_6$) δ 10.34 (s, 1H), 8.67 (s, 1H), 8.53-8.45 (m, 1H), 8.15-8.08 (m, 2H), 8.02 (d, *J* = 2.4 Hz, 1H), 7.91 (d, *J* = 8.8 Hz, 1H), 7.83-7.72 (m, 1H), 7.62-7.53 (m, 1H), 7.26-7.18 (m, 1H), 5.38 (s, 1H), 5.12-5.05 (m, 2H), 4.44-4.34 (m, 1H), 3.92-3.70 (m, 8H), 3.67 (s, 3H), 1.20 (d, *J* = 6.4 Hz, 3H); ESI-MS(m/z): 611.0 [M+H]$^+$.

**Example 168**

**Preparation of 2,4-difluoro-N-(2-methoxy-5-(4-(4-(2-methylene-3-oxobutanoyl)piperazin-1-yl)quinazolin-6-yl)pyridin-3-yl)benzenesulfonamide**

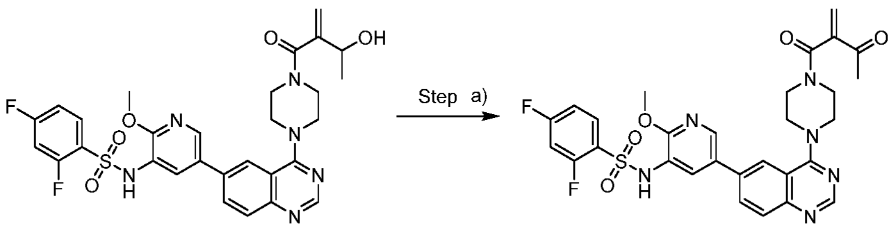


2,4-Difluoro-*N*-(5-(4-(4-(3-hydroxy-2-methylenebutanoyl)piperazin-1-yl)quinazolin-6-yl)-2-methoxypyridin-3-yl)benzenesulfonamide (70 mg, 0.115 mmol) was dissolved in anhydrous acetonitrile (15 mL). With manganese dioxide (2999 mg, 34.500 mmol) added, in the presense of protective nitrogen, the reaction mixture was heated to reflux, stirred and reacted for 10 h. Upon completion of the reaction, the reaction mixture was cooled to room temperature and filtered. The filtrate was concentrated under reduced pressure. The crude product obtained was purified by Prep-HPLC (Method 2). 2,4-Difluoro-*N*-(2-methoxy-5-(4-(4-(2-methylene-3-oxobutanoyl)piperazin-1-yl)quinazolin-6-yl)pyridin-3-yl)benzenesulfonamide was obtained, yield: 24.0%; $^1$H NMR (400 MHz, DMSO-$d_6$) δ 10.35 (s, 1H), 8.67 (s, 1H), 8.46 (s, 1H), 8.13-8.06 (m, 2H), 8.05-8.00 (m, 1H), 7.95-7.89 (m, 1H), 7.82-7.73 (m, 1H), 7.63-7.53 (m, 1H), 7.26-7.15 (m, 1H), 6.45 (s, 1H), 6.16 (s, 1H), 3.90-3.73 (m, 6H), 3.68 (s, 3H), 3.54-3.45 (m, 2H), 2.38 (s, 3H); ESI-MS(m/z): 609.0 [M+H]$^+$.

**Example 169**

**Preparation of (*E*)-2-chloro-4-fluoro-*N*-(2-methoxy-5-(4-(4-(4-oxopent-2-enoyl)piperazin-1-yl)quinazolin-6-yl)pyridin-3-yl)benzenesulfonamide**

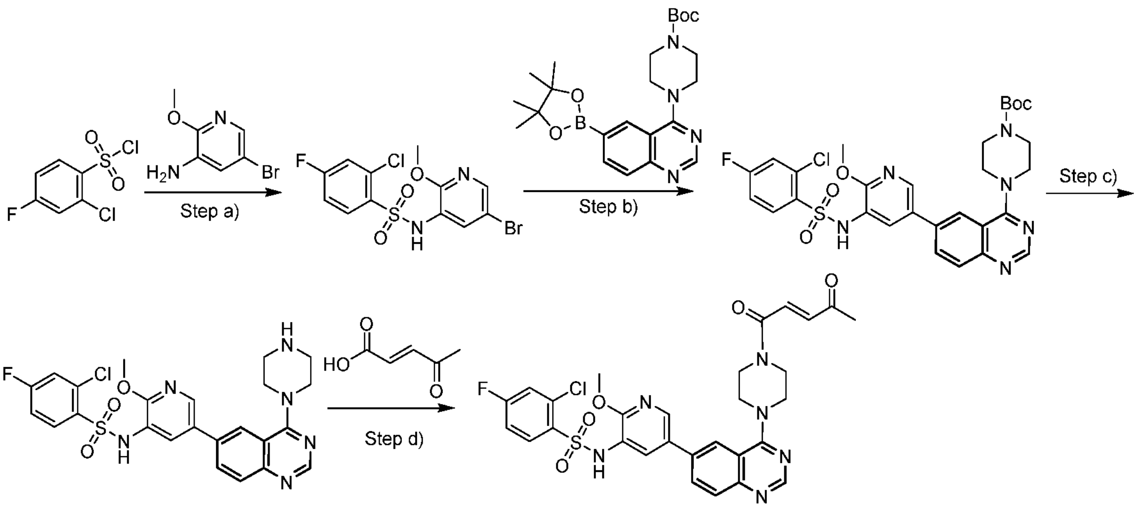


Step a): Preparation of *N*-(5-bromo-2-methoxypyridin-3-yl)-2-chloro-4-fluorobenzenesulfonamide

2-Chloro-4-fluorobenzenesulfonyl chloride (350 mg, 1.528 mmol), 5-bromo-2-methoxypyridin-3-amine (310 mg, 1.528 mmol), 4-dimethylaminopyridine (19 mg, 0.153 mmol) and dichloromethane (6 mL) were added to the reaction flask. The reaction mixture was stirred to mix and cooled to 0°C, pyridine (242 mg, 3.056 mmol) was added dropwise. After the addition was completed, the reaction mixture was kept at room temperature and reacted for 12 h. Upon completion of the reaction, the reaction was quenched by adding water (30 mL). The reaction mixture was extracted

with dichloromethane (60 mL×2). The organic phases were combined, dried over anhydrous sodium sulfate and filtered. The filtrate was concentrated under reduced pressure. The residue was purified by silica gel column chromatography (Eluent: petroleum ether/ethyl acetate = 5/1 to 1/1). *N*-(5-bromo-2-methoxypyridin-3-yl)-2-chloro-4-fluorobenzenesulfonamide was obtained, yield: 69.5%; ESI-MS (m/z): 396.9 $[M+H]^+$.

Step b): Preparation of tert-butyl 4-(6-(5-((2-chloro-4-fluorophenyl)sulfonamido)-6-methoxypyridin-3-yl)pyrido[3,2-*d*]pyrimidin-4-yl)piperazine-1-carboaylate

*N*-(5-bromo-2-methoxypyridin-3-yl)-2-chloro-4-fluorobenzenesulfonamide (210 mg, 0.531 mmol), tert-butyl 4-(6-(4,4,5,5-tetramethyl-1,3,2-dioxaborolan-2-yl)quinazolin-4-yl)piperazine-1-carboxylate (245 mg, 0.556 mmol), $Pd(dppf)Cl_2$ (39 mg, 0.053 mmol) and cesium carbonate (519 mg, 1.593 mmol), along with dioxane/water mixed solvent (5 mL, v/v = 5:1), were added to the reaction flask. After the addition was completed, in the presense of protective nitrogen, the reaction mixture was heated to reflux, stirred and reacted for 2 h. Upon completion of the reaction, the reaction was quenched by adding water (30 mL). The reaction mixture was extracted with ethyl acetate (60 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 2/1). Tert-butyl 4-(6-(5-((2-chloro-4-fluorophenyl)sulfonamido)-6-methoxypyridin-3-yl)pyrido[3,2-*d*]pyrimidin-4-yl)piperazine-1-carboxylate was obtained, yield: 65.9%; ESI-MS(m/z): 629.0 $[M+H]^+$.

Step c): Preparation of 2-chloro-4-fluoro-*N*-(2-methoxy-5-(4-(piperazin-1-yl)quinazolin-6-yl)pyridin-3-yl)benzenesulfonamide trifluoroacetate

Tert-butyl 4-(6-(5-((2-chloro-4-fluorophenyl)sulfonamido)-6-methoxypyridin-3-yl)pyrido[3,2-*d*]pyrimidin-4-yl)piperazine-1-carboxylate (220 mg, 0.350 mmol) was dissolved in dichloromethane (2 mL). Under the ice bath condition, trifluoroacetic acid (0.5 mL) was slowly added dropwise. After the addition was completed, the reaction mixture was stirred at room temperature for 1 h. Upon completion of the reaction, the reaction mixture was concentrated under reduced pressure. 2-chloro-4-fluoro-N-(2-methoxy-5-(4-(piperazin-1-yl)quinazolin-6-yl)pyridin-3-yl)benzenesulfonamide trifluoroacetate was obtained, yield: 94.7%; ESI-MS(m/z): 529.1 $[M+H]^+$.

Step d): Preparation of (*E*)-2-chloro-4-fluoro-*N*-(2-methoxy-5-(4-(4-(4-oxopent-2-enoyl)piperazin-1-yl)quinazolin-6-yl)pyridin-3-yl)benzenesulfonamide

2-chloro-4-fluoro-*N*-(2-methoxy-5-(4-(piperazin-1-yl)quinazolin-6-yl)pyridin-3-yl)benzenesulfonamide trifluoroacetate (90 mg, 0.140 mmol), (*E*)-4-oxopent-2-enoic acid (19 mg, 0.168 mmol) and tetrahydrofuran (2 mL) were added to the reaction flask. The reaction system was cooled to -78°C, with DIPEA (145 mg, 1.120 mmol) and 50% $T_3P$ ethyl acetate solution (178 mg, 0.28 mmol, 50%wt) added successively. After the addition was completed, the reaction mixture was kept at -78°C and stirred for 1 h. Upon completion of the reaction, at -78°C, the reaction was quenched by adding water (1 mL). The system was concentrated under reduced pressure at room temperature, diluted by adding acetonitrile (2 mL) and purified by Prep-HPLC (Method 2). (*E*)-2-chloro-4-fluoro-N-(2-methoxy-5-(4-(4-(4-oxopent-2-enoyl)piperazin-1-yl)quinazolin-6-yl)pyridin-3-yl)benzenesulfonamide was obtained, yield: 39.7% $^1H$ NMR (400 MHz, DMSO-$d_6$) δ 10.23 (s, 1H), 8.67 (s, 1H), 8.46-8.42 (m, 1H), 8.12-8.05 (m, 2H), 7.98-7.90 (m, 3H), 7.76-7.71 (m, 1H), 7.46 (d, *J* = 16.0 Hz, 1H), 7.39-7.33 (m, 1H), 6.75 (d, *J* = 16.0 Hz, 1H), 3.94-3.86 (m, 6H), 3.83-3.78 (m, 2H), 3.70 (s, 3H), 2.37 (s, 3H); ESI-MS(m/z): 625.0 $[M+H]^+$.

Referring to the preparation method of Example 169 and using the corresponding raw materials, the compounds in the following examples were obtained.

| Number | Name | Structure | $^1H$ NMR and MS |
|---|---|---|---|
| **Example 170** | (*E*)-*N*-(2-methoxy-5-(4-(4-(4-oxopent-2-enoyl)piperazin-1-yl) quinazolin-6-yl)pyridin-3-yl)methanesulfona mide | 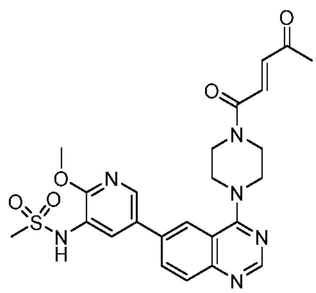 | $^1H$ NMR (400 MHz, DMSO-$d_6$) δ 9.41 (s, 1H), 8.67 (s, 1H), 8.47 (s, 1H), 8.15-8.13 (m, 2H), 8.01 (s, 1H), 7.93 (d, *J* = 8.8 Hz, 1H), 7.45 (d, *J* = 15.6 Hz, 1H), 6.73 (d, *J* = 15.6 Hz, 1H), 3.99 (s, 3H), 3.89-3.80 (m, 6H), 3.79-3.75 (m, 2H), 3.12 (s, 3H), 2.37 (s, 3H); ESI-MS(m/z): 511.0 $[M+H]^+$. |

(continued)

| Number | Name | Structure | $^1H$ NMR and MS |
|---|---|---|---|
| **Example 171** | (*E*)-2-chloro-4-fluoro-*N*-(2-methoxy-5-(4-(2-(4-oxopent-2-enoyl)-2,7-diazaspiro [3.5] non an-7-yl)quinazolin-6-yl)pyridin-3-yl)benzenesulfona mide | 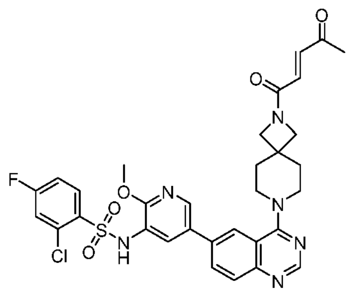 | $^1H$ NMR (400 MHz, DMSO-$d_6$) δ 10.17 (s, 1H), 8.63 (s, 1H), 8.41-8.37 (m, 1H), 8.07-8.01 (m, 2H), 7.99-7.92 (m, 2H), 7.89 (d, *J* = 8.8 Hz, 1H), 7.76-7.71 (m, 1H), 7.39-7.33 (m, 1H), 6.93 (d, *J* = 16.0 Hz, 1H), 6.75 (d, *J* = 16.0 Hz, 1H), 4.15 (s, 2H), 3.80 (s, 2H), 3.77-3.73 (m, 4H), 3.71 (s, 3H), 2.35 (s, 3H), 1.96 (t, *J* = 5.6 Hz, 4H); ESI-MS (m/z): 665.0 $[M+H]^+$. |
| **Example 172** | (*E*)-*N*-(2-methoxy-5-(4-(4-(4-oxopent-2-enoyl)piperazin-1-yl) quinazolin-6-yl)pyridin-3-yl)-3-methylpyridine-4-sulfonamide | 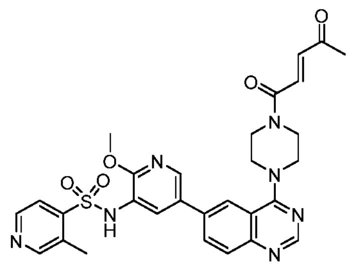 | $^1H$ NMR (400 MHz, DMSO-$d_6$) δ 10.46 (s, 1H), 8.71-8.68 (m, 2H), 8.58 (d, *J* = 4.0 Hz, 1H), 8.48 (s, 1H), 8.12-8.07 (m, 2H), 8.00 (s, 1H), 7.92 (d, *J* = 8.0 Hz, 1H), 7.56 (d, *J* = 8.0 Hz, 1H), 7.47 (d, *J* = 16.0 Hz, 1H), 6.74 (d, *J* = 16.0 Hz, 1H), 3.92-3.79 (m, 8H), 3.62 (s, 3H), 3.63 (s, 3H), 2.38 (s, 3H); ESI-MS(m/z): 588.0 $[M+H]^+$. |
| **Example 173** | (*S*,*E*)-*N*-(2-methoxy-5-(4-(2-methyl-4-(4-oxopent-2-enoyl) piperazin-1-yl)quinazolin-6-yl)pyridin-3-yl)-2,4-dimethylthiazole-5-sulfonamide | 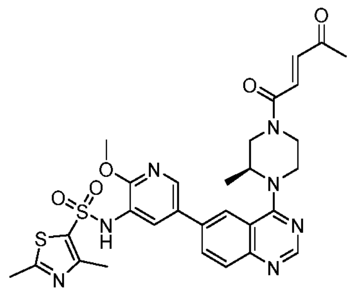 | $^1H$ NMR (400 MHz, DMSO-$d_6$) δ 10.40 (s, 1H), 8.68 (s, 1H), 8.51 (d, J =4.0 Hz, 1H), 8.12-8.04 (m, 3H), 7.94 (d, *J* =12.0 Hz, 1H), 7.45 (dd, $J_1$ = 40.0 Hz, $J_2$ = 12.0 Hz, 1H), 6.76 (dd, $J_1$ = 16.0 Hz, $J_2$ = 8.0 Hz, 1H), 4.82-4.76 (m, 1H), 4.44-4.19 (m, 3H), 3.74-3.49 (m, 5H), 3.24-3.14 (m, 1H), 2.60 (s, 3H), 2.38 (s, 6H), 1.36-1.32 (s, 3H); ESI-MS(m/z): 622.0 $[M+H]^+$. |
| **Example 174** | (*S*,*E*)-2-chloro-4-fluoro-*N*-(2-methoxy-5-(4-(2-methyl-4-(4-oxopent-2-enoyl)piperazin-1-yl) quinazolin-6-yl)pyridin-3-yl)benzenesulfona mide | 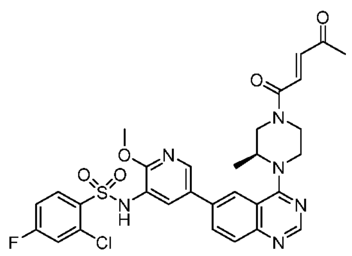 | $^1H$ NMR (400 MHz, DMSO-$d_6$) δ 10.11 (s, 1H), 8.66 (s, 1H), 8.45-8.41 (m, 1H), 8.09-8.02 (m, 2H), 7.98-7.89 (m, 3H), 7.73 (dd, $J_1$ = 8.8 Hz, $J_2$ = 2.4 Hz, 1H), 7.53-7.32 (m, 2H), 6.80-6.71 (m, 1H), 4.83-4.71 (m, 1H), 4.44-4.25 (m, 1H), 4.24-3.95 (m, 2H), 3.69 (s, 3H), 3.66-3.59 (m, 1H), 3.58-3.45 (m, 1H), 3.23-3.10 (m, 1H), 2.37 (d, *J* = 2.4 Hz, 3H), 1.32 (t, *J* = 6.8 Hz, 3H); ESI-MS(m/z): 639.0 $[M+H]^+$. |

(continued)

| Number | Name | Structure | $^1$H NMR and MS |
|---|---|---|---|
| **Example 175** | 2-chloro-4-fluoro-*N*-(2-methoxy-5-(4-(8-((*E*)-4-oxopent-2-enoyl)-3,8-diazabicyclo[3.2.1] octan-3-yl)quinazolin-6-yl) pyridin-3-yl) benzenesulfona mide | 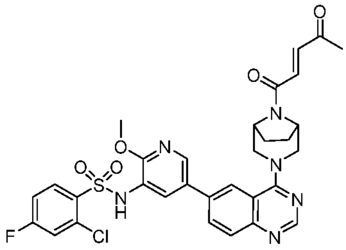 | $^1$H NMR (400 MHz, DMSO-$d_6$) δ 10.13 (s, 1H), 8.60 (s, 1H), 8.43-8.41 (m, 1H), 8.07-8.00 (m, 2H), 7.97-7.92 (m, 2H), 7.87 (d, *J* = 8.4 Hz, 1H), 7.73 (dd, $J_1$ = 8.4 Hz, $J_2$ = 2.4 Hz, 1H), 7.43-7.38 (m, 1H), 7.38-7.32 (m, 1H), 6.84 (d, *J* = 15.6 Hz, 1H), 4.80-4.72 (m, 2H), 4.55-4.44 (m, 2H), 3.69 (s, 3H), 3.59 (t, *J* = 11.2 Hz, 2H), 2.39 (s, 3H), 1.98-1.89 (m, 1H), 1.85-1.74 (m, 3H); ESI-MS(m/z): 651.0 [M+H]$^+$. |
| **Example 176** | *N*-(2-methoxy-5-(4-(8-((*E*)-4-oxopent-2-enoyl)-3,8-diazabicyclo [3.2.1] octan-3-yl) quinazolin-6-yl)pyridin-3-yl)-2,4-dimethylthiazole-5-sulfonamide | 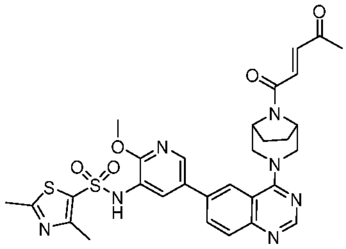 | $^1$H NMR (400 MHz, DMSO-$d_6$) δ 10.36 (s, 1H), 8.61 (s, 1H), 8.48 (s, 1H), 8.10-8.04 (m, 2H), 8.00 (s, 1H), 7.90 (d, *J* = 8.4 Hz, 1H), 7.42 (d, *J* = 15.6 Hz, 1H), 6.84 (d, *J* =15.6 Hz, 1H), 4.80-4.75 (m, 2H), 4.56-4.46 (m, 2H), 3.74 (s, 3H), 3.61 (t, *J* =13.2 Hz, 2H), 2.59(s, 3H), 2.40-2.38 (m, 6H), 1.95-1.92 (m, 1H), 1.85-1.81 (m, 3H); ESI-MS(m/z): 634.0 [M+H]$^+$. |
| **Example 177** | (*E*)-4-chloro-2-fluoro-*N*-(2-methoxy-5-(4-(4-(4-oxopent-2-enoyl) piperazin-1 - yl) quinazolin-6-yl)pyridin-3-yl)benzenesulfona mide | 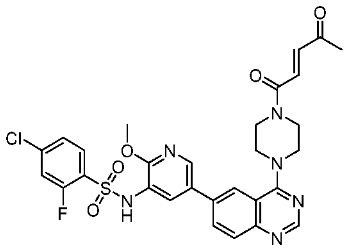 | $^1$H NMR (400 MHz, DMSO-$d_6$) δ 10.39 (s, 1H), 8.66 (s, 1H), 8.46-8.44 (m, 1H), 8.13-8.07 (m, 2H), 8.01-7.99 (m, 1H), 7.91 (d, *J* = 8.8 Hz, 1H), 7.77-7.69 (m, 2H), 7.47-7.40 (m, 2H), 6.74 (d, *J* = 16.0 Hz, 1H), 3.92-3.87 (m, 6H), 3.83-3.78 (m, 2H), 3.67 (s, 3H), 2.37 (s, 3H); ESI-MS(m/z): 625.0 [M+H]$^+$. |
| **Example 178** | (*E*)-*N*-(2-methoxy-5-(4-(2-(4-oxopent-2-enoyl)-2,7-diazaspiro [3.5] non an-7-yl) quinazolin-6-yl)pyridin-3 -yl)-2,4-dimethylthiazole-5-sulfonamide | 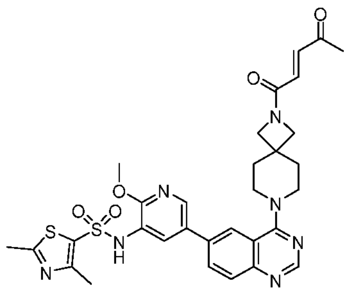 | $^1$H NMR (400 MHz, DMSO-$d_6$) δ 10.31 (s, 1H), 8.64 (d, *J* = 4.0 Hz, 1H), 8.42 (s, 1H), 8.08-7.89 (m, 4H), 6.96-6.91 (m, 1H), 6.77-6.72 (m, 1H), 4.15 (s, 2H), 3.80-3.72 (m, 9H), 2.59 (s, 3H), 2.38-2.34 (m, 6H), 1.99-1.96 (m, 4H); ESI-MS(m/z): 648.0 [M+H]$^+$. |

(continued)

| Number | Name | Structure | $^1$H NMR and MS |
|---|---|---|---|
| **Example 179** | (*E*)-4-(4-(6-(5-((2,4-difluorophenyl)sulfonamido)-6-methoxypyridin-3-yl)quinazolin-4-yl)piperazin-1-yl)-*N*-methyl-4-oxobut-2-enamide | 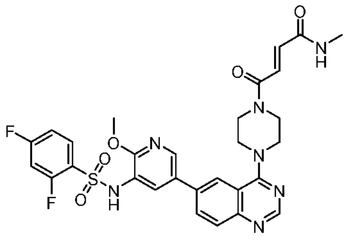 | $^1$H NMR (400 MHz, DMSO-$d_6$) δ 8.65 (s, 1H), 8.29 (s, 1H), 8.16-8.13 (m, 1H), 8.05-8.02 (m, 2H), 7.91-7.89 (m, 2H), 7.82-7.76 (m, 1H), 7.51-7.45 (m, 1H), 7.21-7.16 (m, 1H), 6.42 (d, *J* = 11.6 Hz, 1H), 6.05 (d, *J* = 11.6 Hz, 1H), 3.85-3.81 (m, 4H), 3.74-3.72 (m, 5H), 3.56-3.54 (m, 2H), 2.62 (d, *J* = 4.8 Hz, 3H); ESI-MS(m/z): 624.0 [M+H]$^+$. |
| **Example 180** | (*E*)-*N*-(5-(4-(4-(2-cyano-4,4-dimethylpent-2-enoyl)piperazin-1-yl)quinazolin-6-yl)-2-methoxypyridin-3-yl)-2,4-difluorobenzenesulfonamide | 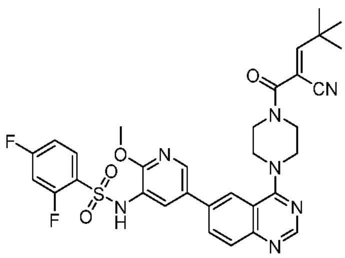 | $^1$H NMR (400 MHz, DMSO-$d_6$) δ 10.46 (s, 1H), 8.66 (s, 1H), 8.32 (s, 1H), 8.14-8.04 (m, 2H), 7.93-7.90 (m, 2H), 7.81-7.75 (m, 1H), 7.48 (t, *J* = 10.0 Hz, 1H), 7.20-7.15 (m, 1H), 6.91 (s, 1H), 3.90-3.88 (m, 4H), 3.77-3.70 (m, 7H), 1.24 (s, 9H); ESI-MS(m/z): 648.0 [M+H]$^+$. |
| **Example 181** | methyl (*E*)-3-((2,4-difluorophenyl)sulfonamido)-5-(4-(4-(4-oxopent-2-enoyl)piperazin-1-yl)quinazolin-6-yl)picolinate | 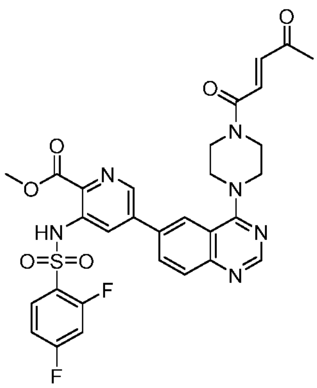 | $^1$H NMR (400 MHz, DMSO-$d_6$) δ 10.83 (br s, 1H), 8.83 (s, 1H), 8.69 (s, 1H), 8.27-8.08 (m, 3H), 8.00-7.89 (m, 2H), 7.58-7.48 (m, 1H), 7.45 (d, *J* = 16.0 Hz, 1H), 7.28-7.19 (m, 1H), 6.74 (d, *J* = 15.6 Hz, 1H), 4.10-3.79 (m, 8H), 3.79 (s, 3H), 2.36 (s, 3H); ESI-MS(m/z): 637.0 [M+H]$^+$. |
| **Example 182** | (*E*)-2,4-difluoro-*N*-(2-(methylthio)-5-(4-(4-(4-oxopent-2-enoyl)piperazin-1-yl)quinazolin-6-yl)pyridin-3-yl)benzenesulfona mide | 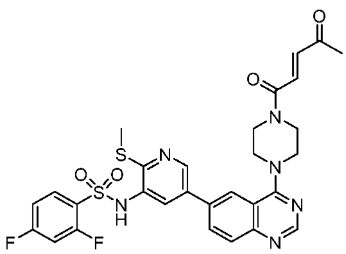 | $^1$H NMR (400 MHz, DMSO-$d_6$) δ 10.61 (s, 1H), 8.65 (s, 1H), 8.32 (s, 1H), 8.03 (s, 1H), 7.97-7.94 (m, 1H), 7.89 (d, *J* = 8.4 Hz, 1H), 7.82-7.76 (m, 1H), 7.62 (s, 1H), 7.45 (d, *J* = 16.0 Hz, 1H), 7.28 (s, 1H), 7.11-7.06 (m, 1H), 6.73 (d, *J* = 16.0 Hz, 1H), 3.89-3.79 (m, 8H), 2.37 (s, 3H), 2.35 (s, 3H); ESI-MS(m/z): 625.0 [M+H]$^+$. |
| **Example 183** | (*E*)-2,4-difluoro-*N*-(2-(hydroxymethyl)-5-(4-(4-(4-oxopent-2-enoyl)piperazin-1-yl)quinazolin-6-yl)pyridin-3-yl)benzenesulfona mide | 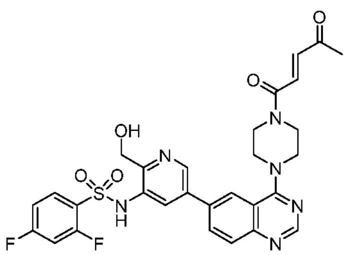 | $^1$H NMR (400 MHz, DMSO-$d_6$) δ 8.66 (s, 1H), 8.10 (d, *J* = 2.0 Hz, 1H), 7.99 (s, 1H), 7.97-7.86 (m, 2H), 7.84-7.78 (m, 1H), 7.70 (d, *J* = 2.0 Hz, 1H), 7.45 (d, *J* = 16.0 Hz, 1H), 7.26-7.16 (m, 1H), 7.11-7.01 (m, 1H), 6.73 (d, *J* = 16.0 Hz, 1H), 5.29 (t, *J* = 5.6 Hz, 1H), 4.54 (d, *J* = 5.6 Hz, 2H), 4.04-3.61 (m, 8H), 2.37 (s, 3H); ESI-MS(m/z): 609.0 [M+H]$^+$. |

(continued)

| Number | Name | Structure | $^1$H NMR and MS |
|---|---|---|---|
| **Example 184** | (*E*)-2-chloro-5-methoxy-*N*-(2-methoxy-5-(4-(4-(4-oxopent-2-enoyl) piperazin-1 - yl) quinazolin-6-yl)pyridin-3-yl)benzenesulfona mide | 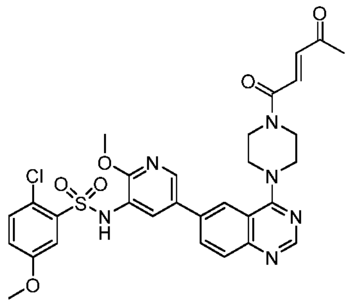 | $^1$H NMR (400 MHz, DMSO-$d_6$) δ 10.21 (s, 1H), 8.66 (s, 1H), 8.36 (s, 1H), 8.09-8.00 (m, 2H), 7.93-7.88 (m, 2H), 7.55 (d, *J* = 8.8 Hz, 1H), 7.48-7.42 (m, 2H), 7.18 (dd, $J_1$ = 8.8 Hz, $J_2$ = 3.2 Hz, 1H), 6.74 (d, *J* = 15.6 Hz, 1H), 3.91-3.85 (m, 6H), 3.82-3.77 (m, 2H), 3.76-3.73 (m, 6H), 2.37 (s, 3H); ESI-MS(m/z): 637.0 $[M+H]^+$. |
| **Example 185** | (*E*)-2-fluoro-4-methoxy-*N*-(2-methoxy-5-(4-(4-(4-oxopent-2-enoyl) piperazin-1- yl)quinazolin-6-yl)pyridin-3-yl) benzenesulfona mide | 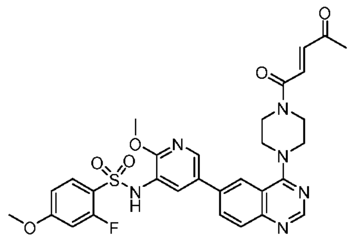 | $^1$H NMR (400 MHz, DMSO-$d_6$) δ 9.71 (s, 1H), 8.66 (s, 1H), 8.21 (s, 1H), 8.06-7.99 (m, 2H), 7.90 (d, *J* = 8.8 Hz, 1H), 7.84 (s, 1H), 7.64 (t, *J* = 8.8 Hz, 1H), 7.45 (d, *J* = 16.0 Hz, 1H), 7.00-6.93 (m, 1H), 6.84-6.79 (m, 1H), 6.74 (d, *J* = 16.0 Hz, 1H), 3.90-3.84 (m, 6H), 3.82-3.77 (m, 5H), 3.74 (s, 3H), 2.37 (s, 3H); ESI-MS(m/z): 621.0 $[M+H]^+$. |
| **Example 186** | (*E*)-2-fluoro-*N*-(2-methoxy-5-(4-(4-(4-oxopent-2-enoyl) piperazin-1- yl)quinazolin-6-yl)pyridin-3-yl)-6-methylbenzenesulf onamide | 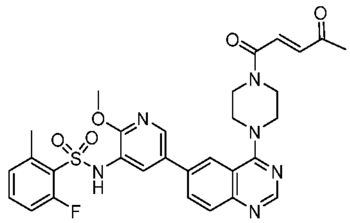 | $^1$H NMR (400 MHz, DMSO-$d_6$) δ 10.10 (br s, 1H), 8.67 (s, 1H), 8.48-8.38 (m, 1H), 8.11 (d, *J* = 2.0 Hz, 1H), 8.06 (d, *J* = 8.8 Hz, 1H), 7.99 (d, *J* = 2.4 Hz, 1H), 7.92 (d, *J* = 8.8 Hz, 1H), 7.57-7.40 (m, 2H), 7.27-7.13 (m, 2H), 6.74 (d, *J* = 15.6 Hz, 1H), 3.96-3.75 (m, 8H), 3.64 (s, 3H), 2.49 (s, 3H), 2.37 (s, 3H); ESI-MS(m/z): 605.0 $[M+H]^+$. |
| **Example 187** | (*E*)-4-fluoro-*N*-(2-methoxy-5-(4-(4-(4-oxopent-2-enoyl) piperazin-1- yl)quinazolin-6-yl)pyridin-3-yl) naphthalene-1-sulfonamide | 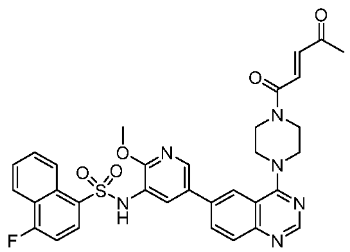 | $^1$H NMR (400 MHz, DMSO-$d_6$) δ 10.35 (br s, 1H), 8.81 (d, *J* = 8.4 Hz, 1H), 8.66 (s, 1H), 8.39-8.32 (m, 1H), 8.23-8.17 (m, 1H), 8.12-8.01 (m, 3H), 7.97-7.89 (m, 2H), 7.88-7.76 (m, 2H), 7.49-7.37 (m, 2H), 6.74 (d, *J* = 15.6 Hz, 1H), 3.92-3.75 (m, 8H), 3.28 (s, 3H), 2.37 (s, 3H); ESI-MS (m/z): 641.0 $[M+H]^+$. |
| **Example 188** | (*E*)-2,4-difluoro-*N*-(2-methyl-5-(4-(4-(4-oxopent-2-enoyl) piperazin-1- yl)quinazolin-6-yl)pyridin-3-yl) benzenesulfona mide | 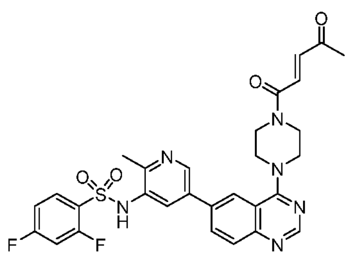 | $^1$H NMR (400 MHz, DMSO-$d_6$) δ 10.58 (s, 1H), 8.67 (s, 1H), 8.65 (s, 1H), 8.12-8.10 (m, 1H), 8.05-8.01 (m, 1H), 7.92 (d, *J* = 8.8 Hz, 1H), 7.85-7.77 (m, 2H), 7.56-7.49 (m, 1H), 7.45 (d, *J* = 16.0 Hz, 1H), 7.24-7.17 (m, 1H), 6.74 (d, *J* = 15.6 Hz, 1H), 3.92-3.86 (m, 6H), 3.82-3.77 (m, 2H), 2.37 (s, 3H), 2.32 (s, 3H); ESI-MS(m/z): 593.0 $[M+H]^+$. |

(continued)

| Number | Name | Structure | $^1H$ NMR and MS |
|---|---|---|---|
| **Example 189** | (*E*)-*N*-(2-cyano-5-(4-(4-(4-oxopent-2-enoyl)piperazin-1- yl) quinazolin-6-yl)pyridin-3-yl)-2,4-difluorobenzenesul fonamide | 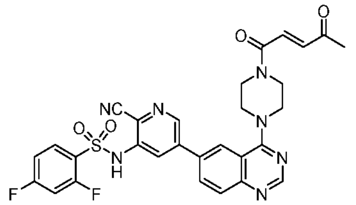 | $^1H$ NMR (400 MHz, DMSO-$d_6$) δ 8.79 (s, 1H), 8.55 (br s, 1H), 8.25 (d, *J* = 2.0 Hz, 1H), 8.11 (d, *J* = 8.4 Hz, 1H), 8.05 (d, *J* = 2.0 Hz, 1H), 7.94 (d, *J* = 8.8 Hz, 1H), 7.91-7.83 (m, 1H), 7.47-7.33 (m, 2H), 7.22-7.11 (m, 1H), 6.75 (d, *J* = 15.6 Hz, 1H), 4.18-4.03 (m, 4H), 3.97-3.86 (m, 2H), 3.84-3.77 (m, 2H), 2.37 (s, 3H); ESI-MS(m/z): 604.0 [M+H]$^+$. |
| **Example 190** | (*E*)-*N*-(2-ethoxy-5-(4-(4-(4-oxopent-2-enoyl)piperazin-1-yl) quinazolin-6-yl)pyridin-3-yl)-2-fluorobenzenesulfo namide | 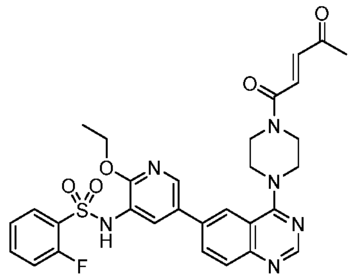 | $^1H$ NMR (400 MHz, DMSO-$d_6$) δ 8.66 (s, 1H), 8.27 (s, 1H), 8.11-7.97 (m, 2H), 7.90 (d, *J* = 8.8 Hz, 2H), 7.78-7.70 (m, 1H), 7.67-7.56 (m, 1H), 7.45 (d, *J* = 15.6 Hz, 1H), 7.41-7.34 (m, 1H), 7.33-7.19 (m, 1H), 6.74 (d, *J* = 15.6 Hz, 1H), 4.17 (dd, $J_1$ = 14.0 Hz, $J_2$ = 7.2 Hz, 2H), 4.03-3.60 (m, 8H), 2.37 (s, 3H), 1.15 (d, *J* = 7.2 Hz, 3H); ESI-MS(m/z): 605.0 [M+H]$^+$. |
| **Example 191** | (*E*)-2,4-difluoro-*N*-(2-(methoxy-$d_3$)-5-(4-(4-(4-oxopent-2-enoyl)piperazin-1-yl) quinazolin-6-yl)pyridin-3-yl)benzenesulfona mide | 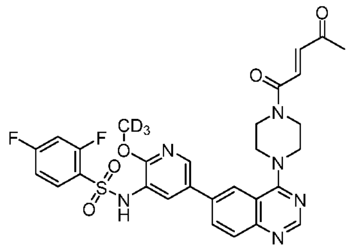 | $^1H$ NMR (400 MHz, DMSO-$d_6$) δ 10.28 (s, 1H), 8.66 (s, 1H), 8.39 (s, 1H), 8.17-8.02 (m, 2H), 8.00-7.85 (m, 2H), 7.82-7.72 (m, 1H), 7.60-7.48 (m, 1H), 7.45 (d, *J* = 15.6 Hz, 1H), 7.25-7.12 (m, 1H), 6.74 (d, *J* = 15.6 Hz, 1H), 4.01-3.68 (m, 8H), 2.37 (s, 3H); ESI-MS(m/z): 612.0 [M+H]$^+$. |
| **Example 192** | (*E*)-2,4-difluoro-*N*-(5-(4-(4-(4-oxopent-2-enoyl)piperazin-1-yl) quinazolin-6-yl)-2-(trifluoromethyl)py ridin-3-yl)benzenesulfonamide | 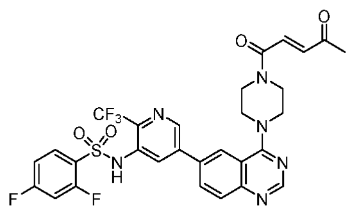 | $^1H$ NMR (400 MHz, DMSO-$d_6$) δ 8.87 (br s, 1H), 8.77 (s, 1H), 8.32 (s, 1H), 8.22-8.15 (m, 2H), 7.97 (d, *J* = 8.4 Hz, 1H), 7.88-7.79 (m, 1H), 7.53-7.38 (m, 2H), 7.24-7.17 (m, 1H), 6.75 (d, *J* = 15.6 Hz, 1H), 4.13-4.01 (m, 4H), 3.96-3.88 (m, 2H), 3.85-3.78 (m, 2H), 2.37 (s, 3H); ESI-MS(m/z): 647.0 [M+H]$^+$. |

**Example 193**

**Preparation of *N*-(5-(4-((2*R*,6*S*)-2,6-dimethyl-4-((*E*)-4-oxopent-2-enoyl)piperazin-1-yl)quinazolin-6-yl)-2-methoxypyridin-3-yl)-2-fluorobenzenesulfonamide**

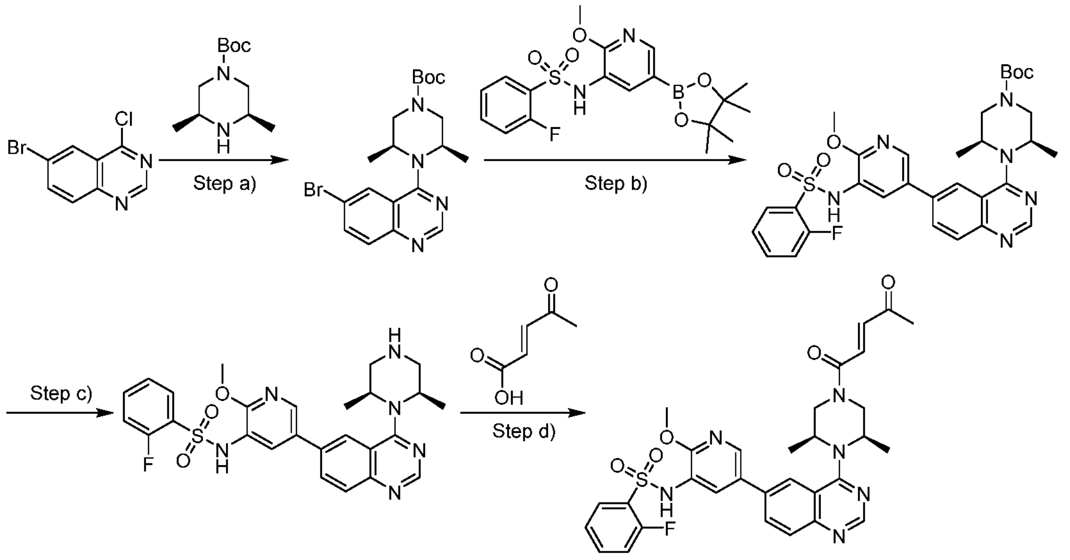


Step a): Preparation of tert-butyl (3*R*,5*S*)-4-(6-bromoquinazolin-4-yl)-3,5-dimethylpiperazine-1-carboxylate

6-Bromo-4-chloroquinazoline (500 mg, 2.053 mmol), tert-butyl (3*R*,5*S*)-3,5-dimethylpiperazine-1-carboxylate (880 mg, 4.106 mmol), sodium bicarbonate (517 mg, 6.159 mmol) and acetonitrile (20 mL) were added to the reaction flask. After the addition was completed, the reaction mixture was microwave heated to 110°C and reacted for 8 h. Upon completion of the reaction, the reaction was quenched by adding water (50 mL). The reaction mixture was extracted with ethyl acetate (100 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: dichloromethane/methanol=100/1 to 20/1). Tert-butyl (3*R*,5*S*)-4-(6-bromoquinazolin-4-yl)-3,5-dimethylpiperazine-1-carboxylatewas obtained, yield: 76.9%; ESI-MS(m/z): 421.1 $[M+H]^+$.

Step b): Preparation of tert-butyl (3A,5S)-4-(6-(5-((2-fluorophenyl)sulfonamido)-6-methoxypyridin-3-yl)quinazolin-4-yl)-3,5-dimethylpiperazine-1-carboxylate

Tert-butyl (3*R*,5*S*)-4-(6-bromoquinazolin-4-yl)-3,5-dimethylpiperazine-1-carboxylate (200 mg, 0.475 mmol), 2-fluoro-*N*-(2-methoxy-5-(4,4,5,5-tetramethyl-1,3,2-dioxaborolan-2-yl)pyridin-3-yl)benzenesulfonamide (194 mg, 0.475 mmol), Pd(dppf)$Cl_2$ (35 mg, 0.048 mmol) and cesium carbonate (464 mg, 1.425 mmol), dioxane/water mixed solvent (10 mL, v/v = 10:1) were added to the reaction flask. After the addition was completed, in the presense of protective nitrogen, the reaction mixture was heated to 100°C and reacted for 2 h. Upon completion of the reaction, the reaction was quenched by adding water (50 mL). The reaction mixture was extracted with ethyl acetate (100 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: dichloromethane/methanol=100/1 to 50/1). Tert-butyl (3*R*,5*S*)-4-(6-(5-((2-fluorophenyl)sulfonamido)-6-methoxypyridin-3-yl)quinazolin-4-yl)-3,5-dimethylpiperazine-1-carboxylate was obtained, yield: 64.5%; ESI-MS(m/z): 623.2 $[M+H]^+$.

Step c): Preparation of *N*-(5-(4-((2*R*,6*S*)-2,6-dimethylpiperazin-1-yl)quinazolin-6-yl)-2-methoxypyridin-3-yl)-2-fluorobenzenesulfonamide trifluoroacetate

Tert-butyl (3*R*,5*S*)-4-(6-(5-((2-fluorophenyl)sulfonamido)-6-methoxypyridin-3-yl)quinazolin-4-yl)-3,5-dimethylpiperazine-1-carboxylate (100 mg, 0.161 mmol), dichloromethane (4 mL) were added to the reaction flask, under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. *N*-(5-(4-((2*R*,6*S*)-2,6-dimethylpiperazin-1-yl)quinazolin-6-yl)-2-methoaypyridin-3-yl)-2-fluorobenzenesulfonamide trifluoroacetatewas obtained, yield: 90.4%; ESI-MS(m/z): 523.2 $[M+H]^+$.

Step d): Preparation of *N*-(5-(4-((2*R*,6*S*)-2,6-dimethyl-4-((*E*)-4-oxopent-2-enoyl)piperazin-1-yl)quinazolin-6-yl)-2-methoxypyridin-3-yl)-2-fluorobenzenesulfonamide

*N*-(5-(4-((2*R*,6*S*)-2,6-dimethylpiperazin-1-yl)quinazolin-6-yl)-2-methoxypyridin-3-yl)-2-fluorobenzenesulfonamide trifluoroacetate (60 mg, 0.094 mmol) and tetrahydrofuran (2 mL) were added to the reaction flask. The reaction system was cooled to -78°C, with DIPEA (73 mg, 0.564 mmol), (E)-4-oxopent-2-enoic acid (11 mg, 0.094 mmol) and 50% $T_3P$ ethyl acetate solution (120 mg, 0.188 mmol, 50%wt) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). *N*-(5-(4-((2*R*,6*S*)-2,6-dimethyl-4-((*E*)-4-oxopent-2-enoyl)piperazin-1-yl)quinazolin-6-yl)-2-methoxypyfidin-3-yl)-2-fluorobenzenesulfonamide was obtained, yield: 28.8%; $^1$H NMR (400 MHz, DMSO-$d_6$) δ 8.74 (s, 1H), 8.16-8.13 (m, 2H), 8.07-8.01 (m, 1H), 7.92 (d, *J* = 8.8 Hz, 1H), 7.87-7.79 (m, 1H), 7.77-7.71 (m, 1H), 7.60-7.51 (m, 1H), 7.44 (d, *J* = 16.0 Hz, 1H), 7.32 (t, *J* = 9.2 Hz, 1H), 7.24 (t, *J* = 7.6 Hz, 1H), 6.81 (d, *J* = 15.6 Hz, 1H), 4.61-4.47 (m, 2H), 3.97-3.74 (m, 3H), 3.73 (s, 3H), 3.47 (dd, $J_1$ = 13.2, $J_2$ = 4.0 Hz, 1H), 2.38 (s, 3H), 1.30 (d, *J* = 6.8 Hz, 6H).; ESI-MS(m/z): 619.0 [M+H]$^+$.

**Example 194**

**Preparation of (*E*)-2,4-difluoro-*N*-(5-(4-(4-(4-oxopent-2-enoyl)piperazin-1-yl)quinazolin-6-yl)-2-(trifluoromethoxy)pyridin-3-yl)benzenesulfonamide**

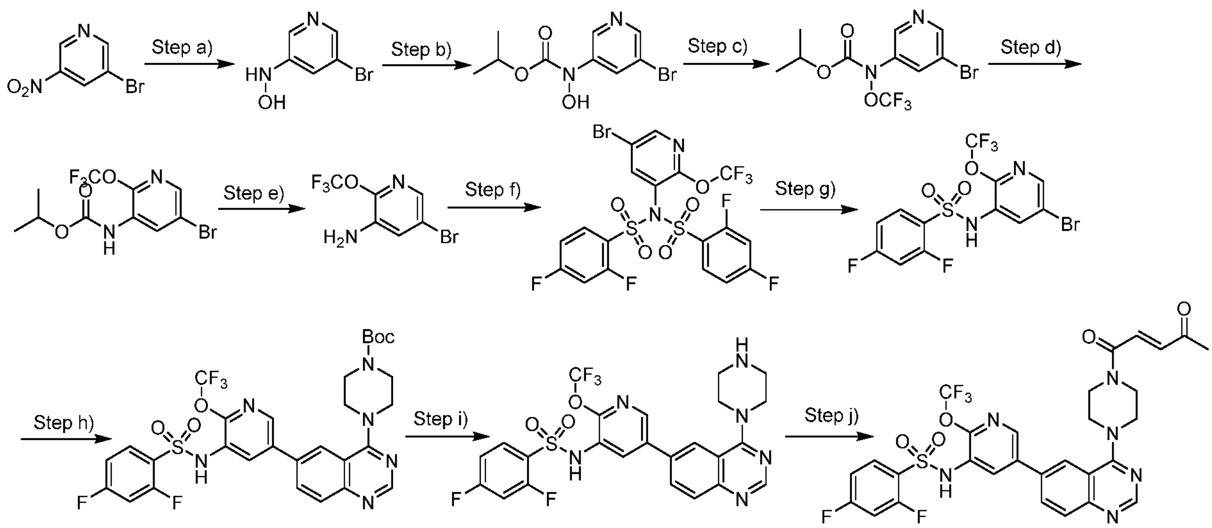


Step a): Preparation of *N*-(5-bromopyridin-3-yl)hydroaylamine

3-Bromo-5-nitropyridine (15 g, 0.074 mol) and tetrahydrofuran (150 mL) were added to the reaction flask, stirred to dissolve, with 10%Pd/C (3.93 g, water 55%). Hydrazine hydrate (6 g, 0.089 mol, 80% aqueous solution) was slowly added to the system dropwise. After the addition was completed, the reaction mixture was stirred at room temperature and reacted for 3h. Upon completion of the reaction, the reaction mixture was filtered. The filter cake was washed with methanol (100 mL×2), the filtrate was combined, concentrated under reduced pressure. The crude product obtained was purified by silica gel column chromatography (Eluent: dichloromethane/methanol=35/1). *N*-(5-bromopyridin-3-yl)hydroxylamine was obtained, yield: 76.8%; $^1$H NMR (400 MHz, DMSO-$d_6$) δ 8.81 (s, 1H), 8.73 (d, *J* = 2.0 Hz, 1H), 8.09 (d, *J* = 2.0 Hz, 1H), 8.04 (d, *J* = 2.4 Hz, 1H), 7.38-7.33 (m, 1H).

Step b): Preparation of isopropyl (5-bromopyridin-3-yl)(hydroxy)carbamate

*N*-(5-bromopyridin-3-yl)hydroxylamine (3000 mg, 15.872 mmol), sodium bicarbonate (1600 mg, 19.047 mmol), 4-dimethylaminopyridine (1939 mg, 15.872mmol) and tetrahydrofuran (70 mL) were added to three-necked bottle. In the presense of protective nitrogen, the reaction mixture was cooled to -78°C, with isopropyl chloroformate (2139 mg, 17.459 mmol) tetrahydrofuran (2 mL) solution slowly added. After the addition was completed, the reaction mixture was slowly heated to room temperature and stirred for 18 h. Upon completion of the reaction, the reaction was quenched by

adding water (50 mL). The reaction mixture was extracted with ethyl acetate (70 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=5/1 to 3/1). Isopropyl (5-bromopyridin-3-yl)(hydroxy)carbamate was obtained, yield: 28.3%; $^1$H NMR (400 MHz, $CDCl_3$) δ 8.75 (d, *J* = 2.4 Hz, 1H), 8.36 (d, *J* = 2.0 Hz, 1H), 8.22 (d, *J* = 2.0 Hz, 1H), 5.19-5.07 (m, 1H), 1.37 (d, *J* = 6.4 Hz, 6H); ESI-MS(m/z): 274.9 $[M+H]^+$.

Step c): Preparation of isopropyl (5-bromopyridin-3-yl)(trifluoromethoxy)carbamate

Isopropyl (5-bromopyridin-3-yl)(hydroxy)carbamate (1237 mg, 4.496 mmol) was dissolved in dichloromethane (60 mL), with 3,3-dimethyl-1-(trifluoromethyl)-1,2-benzoiodooxolane (1484 mg, 4.496 mmol) added. After the addition was completed, in the presense of protective nitrogen, the reaction mixture was stirred at room temperature and reacted for 15 h. Upon completion of the reaction, the reaction mixture was concentrated under reduced pressure. The crude product obtained was purified by silica gel column chromatography (Eluent: petroleum ether/ethyl acetate=10/1). Isopropyl (5-bromopyridin-3-yl)(trifluoromethoxy)carbamate was obtained, yield: 70.0%; $^1$H NMR (400 MHz, $CDCl_3$) δ 8.66-8.58 (m, 2H), 7.93 (d, *J* = 2.0 Hz, 1H), 5.16-5.05 (m, 1H), 1.34 (d, *J* = 6.4 Hz, 6H); ESI-MS(m/z): 343.1 $[M+H]^+$.

Step d): Preparation of isopropyl (5-bromo-2-(trifluoromethoxy)pyridin-3-yl)carbamate

Isopropyl (5-bromopyridin-3-yl)(trifluoromethoxy)carbamate (1350 mg, 3.935 mmol) and nitromethane (36 mL) were mixed. The reaction mixture was microwave heated to 160°C and stirred for 4 h. Upon completion of the reaction, the reaction mixture was concentrated under reduced pressure. The crude product obtained was purified by silica gel column chromatography (Eluent: petroleum ether/ethyl acetate=10/1). Isopropyl (5-bromo-2-(trifluoromethoxy)pyridin-3-yl)carbamatewas obtained, yield: 31.0%; $^1$H NMR (400 MHz, $CDCl_3$) δ 8.76 (br s, 1H), 7.96 (d, *J* = 2.4 Hz, 1H), 6.80 (s, 1H), 5.10-4.98 (m, 1H), 1.34 (d, *J* = 6.4 Hz, 6H); ESI-MS(m/z): 343.1 $[M+H]^+$.

Step e): Preparation of 5-bromo-2-(trifluoromethoxy)pyridin-3-amine

Isopropyl (5-bromo-2-(trifluoromethoxy)pyridin-3-yl)carbamate (420 mg, 1.224 mmol) and methanol (15 mL) and water (2 mL) were mixed, dissolved and added to the reaction flask, with lithium hydroxide monohydrate (206 mg, 4.896 mmol) added. After the addition was completed, in the presense of protective nitrogen, the reaction mixture was kept at 60°C, stirred and reacted for 15 h. Upon completion of the reaction, the reaction mixture was extracted by adding dichloromethane (30 mL) and water (20 mL). After that, the aqueous phase was extracted with dichloromethane (20 mL×2). The organic phases were combined, dried over anhydrous sodium sulfate and filtered. The filtrate was concentrated under reduced pressure. 5-Bromo-2-(trifluoromethoxy)pyridin-3-amine was obtained, yield: 78.5%; ESI-MS(m/z): 257.0 $[M+H]^+$.

Step f): Preparation of *N*-(5-bromo-2-(trifluoromethoxy)pyridin-3-yl)-*N*-((2.4-difluorophenyl)sulfonyl)-2,4-difluorobenzenesulfonamide

5-Bromo-2-(trifluoromethoxy)pyridin-3-amine (246 mg, 0.957 mmol) was dissolved in pyridine (9 mL), with 2,4-difluorobenzenesulfonyl chloride (1017 mg, 4.786 mmol) added. After the addition was completed, at 40°C, the reaction mixture was stirred and reacted for 18 h. Upon completion of the reaction, the reaction mixture was concentrated under reduced pressure. *N*-(5-bromo-2-(trifluoromethoxy)pyridin-3-yl)-*N*-((2,4-difluorophenyl)sulfonyl)-2,4-difluorobenzenesulfonamide was obtained. The product can be used directly in the next reaction without purification; ESI-MS(m/z): 609.0 $[M+H]^+$.

Step g): Preparation of *N*-(5-bromo-2-(trifluoromethoxy)pyridin-3-yl)-2,4-difluorobenzenesulfonamide

The crude product of *N*-(5-bromo-2-(trifluoromethoay)pyridin-3-yl)-*N*-((2,4-difluorophenyl)sulfonyl)-2,4-difluorobenzenesulfonamide (500 mg), tetrahydrofuran (6 mL) and water (6 mL) mixed solvent were added to the reaction flask, stirred to dissolve, with 6 M potassium hydroxide aqueous solution (6 mL) added. The reaction mixture was stirred at room temperature for 1 h. Upon completion of the reaction, the reaction mixture was extracted by adding dichloromethane (30 mL) and water (20 mL). After that, the aqueous phase was extracted with dichloromethane (20 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate=10/1). *N*-(5-bromo-2-(trifluoromethoxy)pyridin-3-yl)-2,4-difluorobenzenesulfonamide was obtained, yield: 70.1%; ESI-MS(m/z): 432.8 $[M+H]^+$.

Step h): Preparation of tert-butyl 4-(6-(5-((2,4-difluorophenyl)sulfonamido)-6-(trifluoromethoxy)pyridin-3-yl)quinazolin-4-yl)piperazine-1-carboxylate

*N*-(5-bromo-2-(trifluoromethoxy)pyridin-3-yl)-2,4-difluorobenzenesulfonamide (180 mg, 0.416 mmol), tert-butyl 4-(6-(4,4,5,5-tetramethyl-1,3,2-dioxaborolan-2-yl)quinazolin-4-yl)piperazine-1-carboxylate (202 mg, 0.458 mmol), Pd(dppf)$Cl_2$ (31 mg, 0.042 mmol) and cesium carbonate (406 mg, 1.248 mmol), dioxane (16 mL) and water (2 mL) were added to the reaction flask. After the addition was completed, in the presense of protective nitrogen, the reaction mixture was kept at 100°C and stirred for 3 h. Upon completion of the reaction, the reaction mixture was cooled to room temperature, the reaction was quenched by adding saturated ammonium chloride (80 mL). The reaction mixture was extracted with ethyl acetate (80 mL). The organic phases were combined, dried over anhydrous sodium sulfate and filtered. The filtrate was concentrated under reduced pressure. The crude product obtained was purified by silica gel column chromatography (Eluent: dichloromethane/methanol = 1/0 to 35/1). Tert-butyl 4-(6-(5-((2,4-difluorophenyl)sulfonamido)-6-(trifluoromethoxy)pyridin-3-yl)quinazolin-4-yl)piperazine-1-carboxylate was obtained, yield: 84.3%; ESI-MS(m/z): 667.5 $[M+H]^+$.

Step i): Preparation of 2,4-difluoro-*N*-(5-(4-(piperazin-1-yl)quinazolin-6-yl)-2-(trifluoromethoxy)pyridin-3-yl)benzenesulfonamide trifluoroacetate

Tert-butyl 4-(6-(5-((2,4-difluorophenyl)sulfonamido)-6-(trifluoromethoxy)pyndin-3-yl)quinazolin-4-yl)piperazine-1-carboxylate (347 mg, 0.521 mmol) was dissolved in dichloromethane (9 mL). Under the ice bath condition, TFA (3 mL) was slowly added dropwise. After the addition was completed, the reaction mixture was stirred at room temperature for 1 h. Upon completion of the reaction, the reaction mixture was concentrated under reduced pressure. The crude product obtained was dispersed in methyl tert-butyl ether (10 mL). The reaction mixture was stirred at room temperature for 0.5 h and filtered. The filter cake was dried to obtain 2,4-difluoro-*N*-(5-(4-(piperazin-1-yl)quinazolin-6-yl)-2-(trifluoromethoxy)pyridin-3-yl)benzenesulfonamide trifluoroacetate, yield: 96.5%; ESI-MS(m/z): 567.5 $[M+H]^+$.

Step j): Preparation of (*E*)-2,4-difluoro-N-(5-(4-(4-(4-oxopent-2-enoyl)piperazin-1-yl)quinazolin-6-yl)-2-(trifluoromethoxy)pyridin-3-yl)benzenesulfonamide

2,4-Difluoro-*N*-(5-(4-(piperazin-1-yl)quinazolin-6-yl)-2-(trifluoromethoxy)pyridin-3-yl)benzenesulfonamide trifluoroacetate (119 mg, 0.179 mmol), (E)-4-oxopent-2-enoic acid (22 mg, 0.197 mmol), DIPEA (116 mg, 0.895 mmol) were dissolved in tetrahydrofuran (2 mL). At -78°C, 1-propylphosphonic anhydride (171 mg, 0.269 mmol, 50% wt ethyl acetate solution) was slowly added to the system. After the addition was completed, the reaction mixture was kept at -78°C and stirred for 1 h. Upon completion of the reaction, the reaction was quenched by adding saturated ammonium chloride (40 mL), and the reaction mixture was extracted with dichloromethane (40 mL×2). The organic phases were combined, dried over anhydrous sodium sulfate and filtered. The filtrate was concentrated under reduced pressure. The crude product obtained was purified by Prep-HPLC (Method 2). (*E*)-2,4-difluoro-*N*-(5-(4-(4-(4-oxopent-2-enoyl)piperazin-1-yl)quinazolin-6-yl)-2-(trifluoromethoxy)pyridin-3-yl)benzenesulfonamide was obtained, yield: 29.6%; $^1$H NMR (400 MHz, DMSO-$d_6$) $\delta$ 10.96 (br s, 1H), 8.67 (s, 1H), 8.23-7.96 (m, 4H), 7.95-7.88 (m, 1H), 7.86-7.75 (m, 1H), 7.48-7.30 (m, 2H), 7.18-7.09 (m, 1H), 6.73 (d, *J* = 15.6 Hz, 1H), 3.92-3.76 (m, 8H), 2.36 (s, 3H); ESI-MS(m/z): 663.0 $[M+H]^+$.

**Example 195**

**Preparation of (*E*)-*N*-(2-(dimethylamino)-5-(4-(4-(4-oxopent-2-enoyl)piperazin-1-yl)quinazolin-6-yl)pyridin-3-yl)-2,4-difluorobenzenesulfonamide**

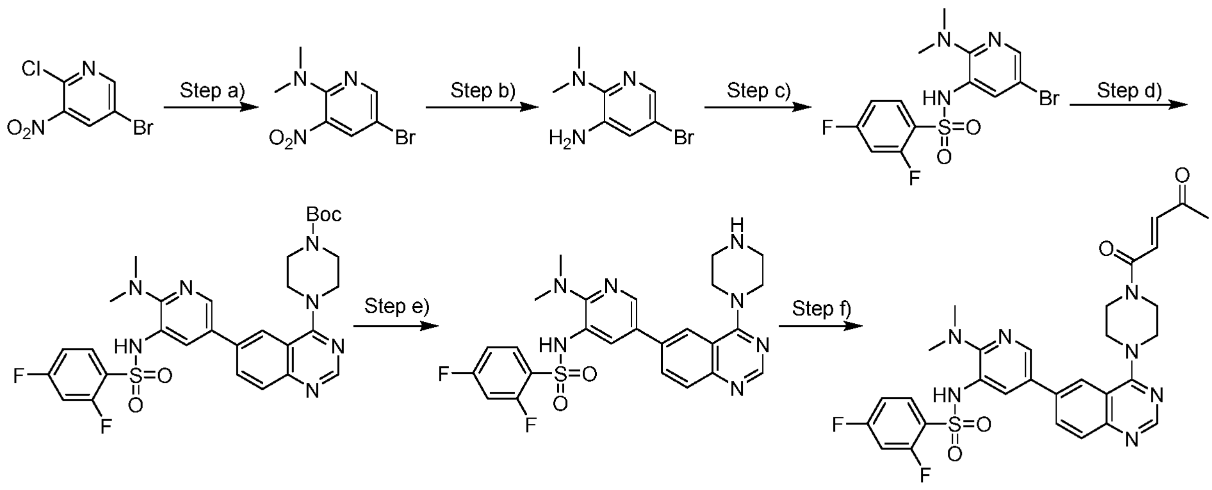


Step a): Preparation of 5-bromo-*N*,*N*-dimethyl-3-nitropyridin-2-amine

5-Bromo-2-chloro-3-nitropyridine (550 mg, 2.316 mmol), DIPEA (748 mg, 5.790 mmol) and *N,N*-dimethylformamide (30 mL) were added to the reaction flask, stirred to dissolve, with dimethylamine hydrochloride (283 mg, 3.474 mmol) added. After the addition was completed, the reaction mixture was heated to 100°C and reacted for 2 h. Upon completion of the reaction, the reaction was quenched by adding water (50 mL). The reaction mixture was extracted with ethyl acetate (150 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: petroleum ether/ethyl acetate = 10/1 to 5/1). 5-Bromo-*N*,*N*-dimethyl-3-nitropyridin-2-amine was obtained, yield: 87.7%; ESI-MS(m/z): 246.0 $[M+H]^+$.

Step b): Preparation of 5-bromo-$N^2$,$N^2$-dimethylpyridine-2,3-diamine

5-Bromo-*N*,*N*-dimethyl-3-nitropyridin-2-amine (500 mg, 2.032 mmol), iron powder (567 mg, 10.160 mmol) and ammonium chloride (109 mg, 2.032 mmol) were added to methanol and water mixed solvent (12.5 mL, v/v=4/1). After the addition was completed, the reaction mixture was heated to 60°C, stirred and reacted for 4h. Upon completion of the reaction, the reaction mixture was filtered. The filtrate was concentrated under reduced pressure, diluted by adding water (50 mL), extracted with dichloromethane (100 mL×3). The organic phases were combined, washed with saturated brine (50 mL), dried over anhydrous sodium sulfate and filtered. The filtrate was concentrated under reduced pressure. The crude product obtained was purified by silica gel column chromatography (Eluent: ethyl acetate/petroleum ether = 5/1 to 2/1). 5-Bromo-$N^2$,$N^2$-dimethylpyridine-2,3-diamine was obtained, yield: 91.1%; ESI-MS(m/z): 216.0 $[M+H]^+$.

Step c): Preparation of *N*-(5-bromo-2-(dimethylamino)pyridin-3-yl)-2,4-difluorobenzenesulfonamide

5-Bromo-$N^2$,$N^2$-dimethylpyridine-2,3-diamine (400 mg, 1.851 mmol) was dissolved in pyridine (10 mL), with 2,4-difluorobenzenesulfonyl chloride (590 mg, 2.777 mmol) added. The reaction mixture was kept at room temperature and reacted for 4 h. Upon completion of the reaction, the reaction was quenched by adding water (100 mL). The reaction mixture was extracted with ethyl acetate (100 mL). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 20/1 to 10/1). N-(5-bromo-2-(dimethylamino)pyridin-3-yl)-2,4-difluorobenzenesulfonamide was obtained, yield: 22.0%; ESI-MS(m/z): 392.0 $[M+H]^+$.

Step d): Preparation of tert-butyl 4-(6-(5-((2,4-difluorophenyl)sulfonamido)-6-(dimethylamino)pyridin-3-yl)quinazolin-4-yl)piperazine-1-carboxylate

N-(5-bromo-2-(dimethylamino)pyridin-3-yl)-2,4-difluorobenzenesulfonamide (160 mg, 0.408 mmol), tert-butyl 4-(6-(4,4,5,5-tetramethyl-1,3,2-dioxaborolan-2-yl)quinazolin-4-yl)piperazine-1-carboxylate (216 mg, 0.490 mmol), Pd(dppf)$Cl_2$ (30 mg, 0.041 mmol) and cesium carbonate (399 mg, 1.224 mmol) were added to dioxane (8 mL) and water (2 mL) mixed solvent. After the addition was completed, in the presense of protective nitrogen, the reaction mixture was heated to 110°C and reacted for 2 h. Upon completion of the reaction, the reaction was quenched by adding water (30

mL). The reaction mixture was extracted with ethyl acetate (50 mL). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/30 to 1/10). Tert-butyl 4-(6-(5-((2,4-difluorophenyl)sulfonamido)-6-(dimethylamino)pyridin-3-yl)quinazolin-4-yl)piperazine-1-carboxylate was obtained, yield: 76.3%; ESI-MS(m/z): 626.2 $[M+H]^+$.

Step e): Preparation of *N*-(2-(dimethylamino)-5-(4-(piperazin-1-yl)quinazofin-6-yl)pyridin-3-yl)-2,4-difluorobenzenesulfonamide trifluoroacetate

Tert-butyl 4-(6-(5-((2,4-difluorophenyl)sulfonamido)-6-(dimethylamino)pyridin-3-yl)quinazolin-4-yl)piperazine-1-carboxylate (200 mg, 0.320 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. N-(2-(dimethylamino)-5-(4-(piperazin-1-yl)quinazolin-6-yl)pyridin-3-yl)-2,4-difluorobenzenesulfonamide trifluoroacetate was obtained; ESI-MS(m/z): 526.2 $[M+H]^+$.

Step f): Preparation of (*E*)-*N*-(2-(dimethylamino)-5-(4-(4-(4-oxopent-2-enoyl)piperazin-1-yl)quinazofin-6-yl)pyridin-3-yl)-2,4-difluorobenzenesulfonamide

*N*-(2-(dimethylamino)-5-(4-(piperazin-1-yl)quinazofin-6-yl)pyridin-3-yl)-2,4-difluorobenzenesulfonamide trifluoroacetate (100 mg, 0.156 mmol) and tetrahydrofuran (5 mL) were added to the reaction flask. With the reaction system cooled to -78°C, DIPEA (102 mg, 0.780 mmol), (*E*)-4-oxopent-2-enoic acid (20 mg, 0.172 mmol) and 50% $T_3P$ ethyl acetate solution (204 mg, 0.312 mmol, 50%wt) were added successively. After the addition was completed, the reaction mixture was kept at -78°C and stirred for 1 h. Upon completion of the reaction, at -78°C, the reaction was quenched using saturated aqueous ammonium chloride solution (50mL). The reaction mixture was extracted by adding dichloromethane (100 mL). The organic phases were combined, washed with saturated brine (100 mL), dried over anhydrous sodium sulfate and filtered. The filtrate was concentrated under reduced pressure. The crude product obtained was purified by Prep-HPLC (Method 2). (*E*)-*N*-(2-(dimethylamino)-5-(4-(4-(4-oxopent-2-enoyl)piperazin-1-yl)quinazolin-6-yl)pyridin-3-yl)-2,4-difluorobenzenesulfonamide was obtained, yield: 24.0%; $^1H$ NMR (400 MHz, DMSO-$d_6$) δ 8.63 (s, 1H), 8.02-7.96 (m, 1H), 7.95-7.76 (m, 4H), 7.56 (d, *J* = 2.4 Hz, 1H), 7.46 (d, *J* = 15.6 Hz, 1H), 7.29-7.20 (m, 1H), 7.12-7.02 (m, 1H), 6.73 (d, *J* = 16.0 Hz, 1H), 3.95-3.72 (m, 8H), 2.94 (s, 6H), 2.37 (s, 3H); ESI-MS(m/z): 622.0 $[M+H]^+$.

**Example 196**

**Preparation of (*E*)-2,4-difluoro-*N*-(5-(8-fluoro-4-(4-(4-oxopent-2-enoyl)piperazin-1-yl)quinazoline-6-(yl)-2-methoxypyridin-3-yl)benzenesulfonamide**

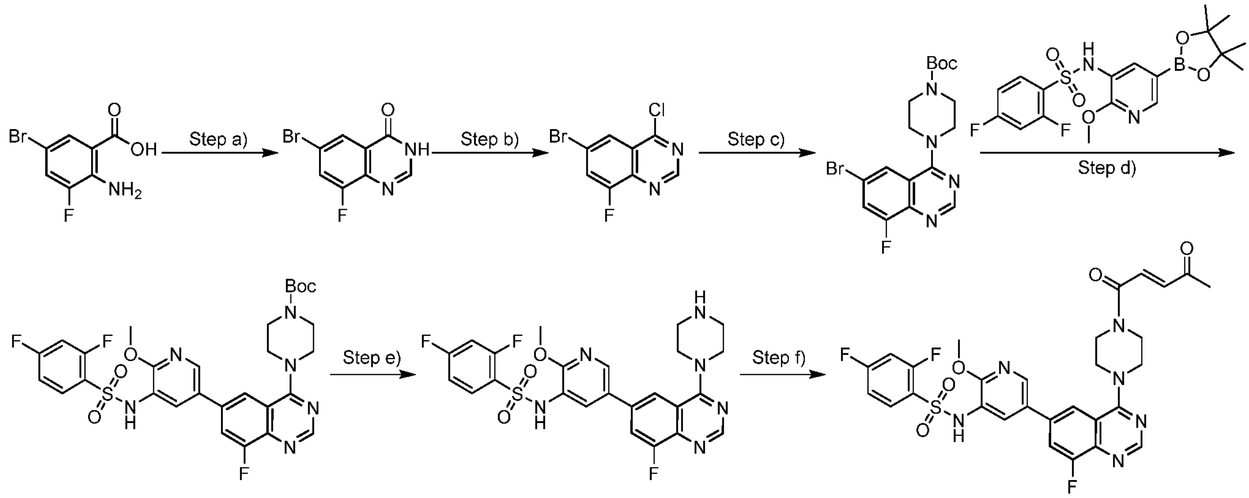

Step a): Preparation of 6-bromo-8-fluoroquinazolin-4(3*H*)-one

2-Amino-5-bromo-3-fluorobenzoic acid (500 mg, 2.137 mmol) was added to formamide (8 mL). The reaction mixture was microwave heated to 180°C and reacted for 1 h. Upon completion of the reaction, the reaction solution was directly filtered. The filter cake was washed by water (10 mL×3), dried to obtain 6-bromo-8-fluoroquinazolin-4(3*H*)-one, yield: 96.3%; ESI-MS(m/z): 243.0 $[M+H]^+$.

Step b): Preparation of 6-bromo-4-chloro-8-fluoroquinazoline

6-Bromo-8-fluoroquinazolin-4(3*H*)-one (500 mg, 2.057 mmol) was added to thionyl chloride (10 mL), with DMF (8 mg, 0.103 mmol) added. The reaction mixture was heated to reflux and reacted for 2 h. Upon completion of the reaction, the reaction solution was directly concentrated under reduced pressure. 6-Bromo-4-chloro-8-fluoroquinazoline was obtained, yield: 92.9%; ESI-MS(m/z): 261.0 $[M+H]^+$.

Step c): Preparation of tert-butyl 4-(6-bromo-8-fluoroquinazolin-4-yl)piperazine-1-carboxylate

6-Bromo-4-chloro-8-fluoroquinazoline (200 mg, 0.765 mmol), tert-butyl piperazine-1-carboxylate (210 mg, 1.128 mmol), DIPEA (562 mg, 4.325 mmol) were added to DMF (10 mL). The reaction mixture was heated to 60°C and reacted for 2 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/50). Tert-butyl 4-(6-bromo-8-fluoroquinazolin-4-yl)piperazine-1-carboxylate was obtained, yield: 95.2%; ESI-MS(m/z): 411.0 $[M+H]^+$.

Step d): Preparation of tert-butyl 4-(6-(5-((2,4-difluorophenyl)sulfonamido)-6-methoxypyridin-3-yl)-8-fluoroquinazolin-4-yl)piperazine-1-carboxylate

Tert-butyl 4-(6-bromo-8-fluoroquinazolin-4-yl)piperazine-1-carboxylate (300 mg, 0.729 mmol), 2,4-difluoro-*N*-(2-methoxy-5-(4,4,5,5-tetramethyl-1,3,2-dioxaborolan-2-yl)pyridin-3-yl)benzenesulfonamide (466 mg, 1.094 mmol), $Pd(dppf)Cl_2$ (53 mg, 0.073 mmol) and cesium carbonate (712 mg, 2.187 mmol) were added to dioxane/water mixed solvent (11 mL, v/v = 10:1). After the addition was completed, in the presense of protective nitrogen, the reaction mixture was heated to reflux and reacted for 3 h. Upon completion of the reaction, the reaction was quenched by adding water (50 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/30). Tert-butyl 4-(6-(5-((2,4-difluorophenyl)sulfonamido)-6-methoxypyridin-3-yl)-8-fluoroquinazolin-4-yl)piperazine-1-carboxylate was obtained, yield: 65.2%; ESI-MS(m/z): 631.2 $[M+H]^+$.

Step e): Preparation of 2,4-difluoro-*N*-(5-(8-fluoro-4-(piperazin-1-yl)quinazolin-6-yl)-2-methoaypyridin-3-yl)benzenesulfonamide trifluoroacetate

Tert-butyl 4-(6-(5-((2,4-difluorophenyl)sulfonamido)-6-methoxypyridin-3-yl)-8-fluoroquinazolin-4-yl)piperazine-1-carboxylate (300 mg, 0.476 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2,4-Difluoro-*N*-(5-(8-fluoro-4-(piperazin-1-yl)quinazolin-6-yl)-2-methoaypyridin-3-yl)benzenesulfonamide trifluoroacetate was obtained, yield: 97.1%; ESI-MS(m/z): 531.1 $[M+H]^+$.

Step f): Preparation of (*E*)-2,4-difluoro-N-(5-(8-fluoro-4-(4-(4-oxopent-2-enoyl)piperazin-1-yl)quinazoline-6-yl)-2- methoxypyridin-3-yl)benzenesulfonamide

2,4-Difluoro-*N*-(5-(8-fluoro-4-(piperazin-1-yl)quinazolin-6-yl)-2-methoaypyridin-3-yl)benzenesulfonamide trifluoroacetate (150 mg, 0.233 mmol) and tetrahydrofuran (5 mL) were added to the reaction flask. The reaction system was cooled to -78°C, with DIPEA (149 mg, 1.165 mmol), (*E*)-4-oxopent-2-enoic acid (29 mg, 0.256 mmol) and 50% $T_3P$ ethyl acetate solution (293 mg, 0.466 mmol, 50%wt) added successively. After the addition was completed, the reaction mixture was kept at -78°C and stirred for 1 h. Upon completion of the reaction, the reaction was quenched using saturated

aqueous ammonium chloride solution (50 mL) at -78°C. The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). (*E*)-2,4-difluoro-N-(5-(8-fluoro-4-(4-(4-oxopent-2-enoyl)piperazin-1-yl)quinazoline-6-yl)-2-methoxypyridin-3-yl)benzenesulfonamide was obtained, yield: 15.0%; $^1$H NMR (400 MHz, DMSO-$d_6$) $\delta$ 10.15 (brs, 1H), 8.68 (s, 1H), 8.35 (s, 1H), 8.04-7.88 (m, 3H), 7.82-7.72 (m, 1H), 7.54-7.41 (m, 2H), 7.22-7.12 (m, 1H), 6.74 (d, *J* = 16.0 Hz, 1H), 4.06-3.74 (m, 8H), 3.69 (s, 3H), 2.37 (s, 3H); ESI-MS(m/z): 627.0 [M+H]$^+$.

**Example 197**

**Preparation of (*E*)-2,4-Difluoro-*N*-(2-methoxy-5-(4-(1-(4-oxopent-2-enoyl)piperidin-4-yl)quinazoline-6-yl)pyridin-3-yl)benzenesulfonamide**

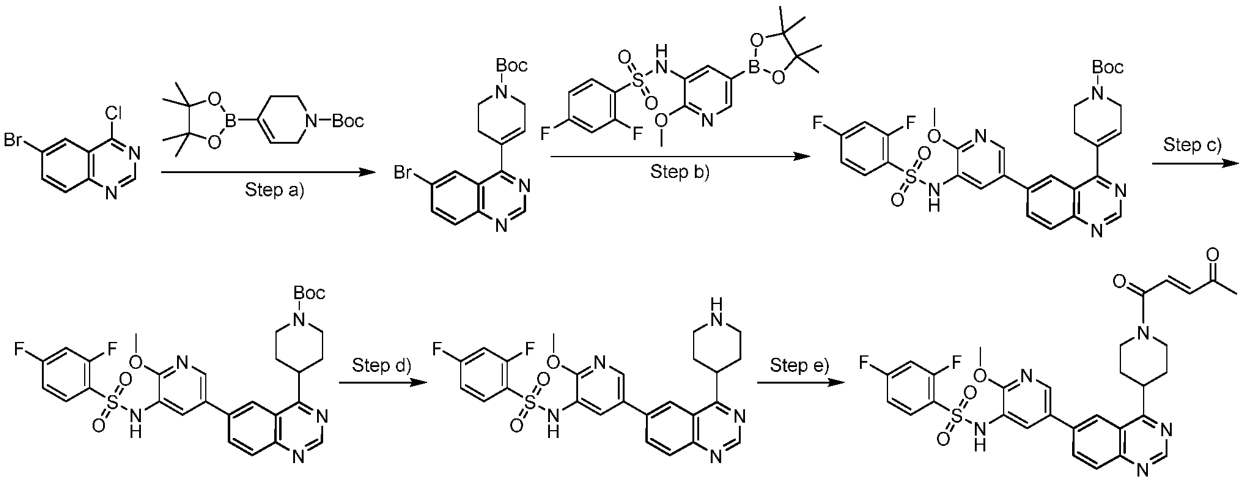


Step a): Preparation of tert-butyl 4-(6-bromoquinazolin-4-yl)-3,6-dihydropyridine-1(2*H*)-carboxylate

6-Bromo-4-chloroquinazoline (500 mg, 2.053 mmol), tert-butyl 4-(4,4,5,5-tetramethyl-1,3,2-dioxaborolan-2-yl)-3,6-dihydropyridine-1(2*H*)-carboxylate (634.81 mg, 2.053 mmol), Pd(dppf)$Cl_2$ (150 mg, 0.205 mmol), sodium carbonate (653 mg, 6.159 mmol), dioxane (25 mL) and water (7 mL) were added to the reaction flask. After the addition was completed, in the presense of protective nitrogen, the reaction mixture was heated to 45°C and stirred for 6 h. Upon completion of the reaction, the reaction mixture was cooled to room temperature and the reaction was quenched by adding saturated ammonium chloride (80 mL). The reaction mixture was extracted with ethyl acetate (80 mL×2). The organic phases were combined, dried over anhydrous sodium sulfate and filtered. The filtrate was concentrated under reduced pressure. The crude product obtained was purified by silica gel column chromatography (Eluent: dichloromethane/methanol = 1/0 to 35/1). Tert-butyl 4-(6-bromoquinazolin-4-yl)-3,6-dihydropyridine-1(2*H*)-carboxylate was obtained, yield: 48.0%; ESI-MS(m/z): 390.0 [M+H]$^+$.

Step b): Preparation of tert-butyl 4-(6-(5-((2,4-difluorophenyl)sulfonamido)-6-methoxypyridin-3-yl)quinazolin-4-y1)-3,6-dihydropyridine-1(2*H*)-carboxylate

Tert-butyl 4-(6-bromoquinazolin-4-yl)-3,6-dihydropyridine-1(2*H*)-carboxylate (256 mg, 0.656 mmol), 2,4-difluoro-*N*-(2-methoxy-5-(4,4,5,5-tetramethyl-1,3,2-dioxaborolan-2-yl)pyridin-3-yl)benzenesulfonamide (308 mg, 0.722 mmol), Pd(dppf)$Cl_2$ (48 mg, 0.066 mmol), cesium carbonate (640 mg, 1.968 mmol), dioxane (16 mL) and water (2 mL) were added to the reaction flask. After the addition was completed, in the presense of protective nitrogen, the reaction mixture was kept at 100°C and stirred for 3 h. Upon completion of the reaction, the reaction mixture was cooled to room temperature and the reaction was quenched by adding saturated ammonium chloride (80 mL). The reaction mixture was extracted with ethyl acetate (80 mL×2). The organic phases were combined, dried over anhydrous sodium sulfate and filtered. The filtrate was concentrated under reduced pressure. The crude product obtained was purified by silica gel column chromatography (Eluent: dichloromethane/methanol = 1/0 to 35/1). tert-butyl 4-(6-(5-((2,4-difluorophenyl)sulfonamido)-6-methoxypyridin-3-yl)quinazolin-4-yl)-3,6-dihydropyridine-1(2*H*)-carboxylate was obtained, yield: 90.0%;

ESI-MS(m/z): 610.6 $[M+H]^+$.

Step c): Preparation of tert-butyl 4-(6-(5-((2,4-difluorophenyl)sulfonamido)-6-methoxypyridin-3-yl)quinazolin-4-yl)piperidine-1-carboxylate

Tert-butyl 4-(6-(5-((2,4-difluorophenyl)sulfonamido)-6-methoxypyridin-3-yl)quinazolin-4-yl)-3,6-dihydropyridine-1(2*H*)-carboxylate (150 mg, 0.246 mmol) was dissolved in methanol (6 mL), with 10%Pd/C (150 mg, 55% water) and acetic acid (150 mg, 2.500 mmol) added. After the addition was completed, under hydrogen atmosphere, the reaction mixture was kept at 25°C and stirred for 5.5 h. Upon completion of the reaction, the reaction mixture was filtered. The filtrate was concentrated under reduced pressure. The crude product obtained was purified by silica gel column chromatography (Eluent: dichloromethane/methanol = 1/0 to 35/1). Tert-butyl 4-(6-(5-((2,4-difluorophenyl)sulfonamido)-6-methoxypyridin-3-yl)quinazolin-4-yl)piperidine-1-carboxylate was obtained, yield: 30.6%; ESI-MS(m/z): 612.0 $[M+H]^+$.

Step d): Preparation of 2,4-difluoro-N-(2-methoxy-5-(4-(piperidin-4-yl)quinazolin-6-yl)pyridin-3-yl)benzenesulfonamide trifluoroacetate

Tert-butyl 4-(6-(5-((2,4-difluorophenyl)sulfonamido)-6-methoxypyridin-3-yl)quinazolin-4-yl)piperidine-1-carboxylate (56 mg, 0.092 mmol) was dissolved in dichloromethane (3 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product obtained was dispersed in methyl tert-butyl ether (10 mL). The reaction mixture was stirred at room temperature for 0.5 h and filtered. The filter cake was vacuum dried to obtain 2,4-difluoro-*N*-(2-methoxy-5-(4-(piperidin-4-yl)quinazolin-6-yl)pyridin-3-yl)benzenesulfonamide trifluoroacetate, yield: 91.2%; ESI-MS(m/z): 512.0 $[M+H]^+$.

Step e): Preparation of (*E*)-2,4-Difluoro-*N*-(2-methoxy-5-(4-(1-(4-oxopent-2-enoyl)piperidin-4-yl)quinazoline-6-yl)pyridin-3-yl)benzenesulfonamide

2,4-Difluoro-*N*-(2-methoxy-5-(4-(piperidin-4-yl)quinazolin-6-yl)pyridin-3-yl)benzenesulfonamide trifluoroacetate (56 mg, 0.090 mmol), (E)-4-oxopent-2-enoic acid (12 mg, 0.099 mmol), DIPEA (58 mg, 0.450 mmol) were dissolved in tetrahydrofuran (2 mL). At -78°C, 1-propylphosphonic anhydride (86 mg, 0.135 mmol, 50% wt) was slowly added to the system. After the addition was completed, the reaction mixture was kept at -78°C and stirred for 1 h. Upon completion of the reaction, the reaction was quenched using saturated aqueous ammonium chloride solution (20 mL) at -78°C. The reaction mixture was extracted by adding dichloromethane (20 mL×2). The organic phases were combined, washed with saturated brine (20 mL), dried over anhydrous sodium sulfate and filtered. The filtrate was concentrated under reduced pressure. The crude product obtained was purified by Prep-HPLC (Method 2). (*E*)-2,4-Difluoro-*N*-(2-methoxy-5-(4-(1-(4-oxopent-2-enoyl)piperidin-4-yl)quinazoline-6-yl)pyridin-3-yl)benzenesulfonamide was obtained, yield: 21.0%; $^1H$ NMR (400 MHz, DMSO-$d_6$) δ 10.24 (brs, 1H), 9.14 (s, 1H), 8.54 (d, *J* = 2.4 Hz, 1H), 8.41 (s, 1H), 8.19-8.12 (m, 1H), 8.03 (d, *J* = 8.4 Hz, 1H), 7.97 (s, 1H), 7.76-7.66 (m, 1H), 7.49-7.37 (m, 2H), 7.17-7.09 (m, 1H), 6.63 (d, *J* = 15.6 Hz, 1H), 4.53 (d, *J* = 12.8 Hz, 1H), 4.24-4.11 (m, 2H), 3.63 (s, 3H), 3.38 (t, *J* = 12.8 Hz, 1H), 2.97 (t, *J* = 12.4 Hz, 1H), 2.30 (s, 3H), 1.97-1.66 (m, 4H); ESI-MS(m/z): 608.0 [M+H]+.

**Example 198**

**Preparation of (*E*)-2,4-difluoro-*N*-(2-methoxy-5-(4-(1-(4-oxopent-2-enoyl)-1,2,3,6-tetrahydropyridin-4-yl)quinazolin-6-yl)pyridin-3-yl)benzenesulfonamide**

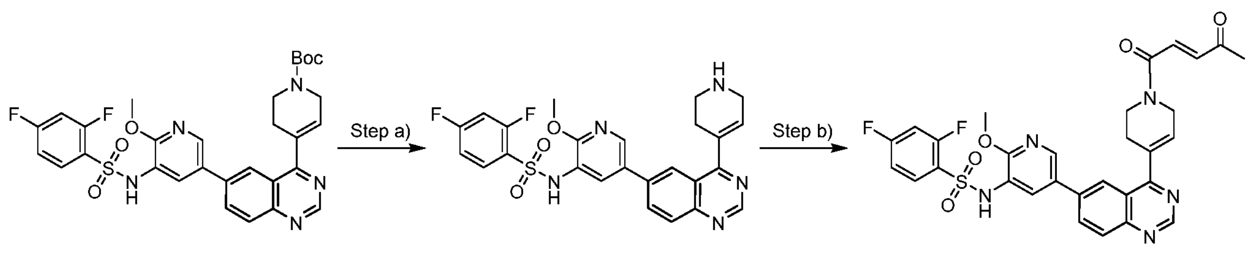

Step a): Preparation of 2,4-difluoro-*N*-(2-methoxy-5-(4-(1,2,3,6-tetrahydropyridin-4-yl)quinazolin-6-yl)pyfidine-3-benzenesulfonamide trifluoroacetate

tert-butyl 4-(6-(5-((2,4-difluorophenyl)sulfonamido)-6-methoxypyridin-3-yl)quinazolin-4-yl)-3,6-dihydropyridine-1(2*H*)-carboxylate (50 mg, 0.082 mmol) was dissolved in dichloromethane (3 mL), under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product obtained was dispersed in methyl tert-butyl ether (10 mL). The reaction mixture was stirred at room temperature for 0.5 h and filtered. The filter cake was dried to obtain 2,4-difluoro-*N*-(2-methoxy-5-(4-(1,2,3,6-tetrahydropyridin-4-yl)quinazolin-6-yl)pyridine-3-benzenesulfonamide trifluoroacetate, yield: 84.2%; ESI-MS(m/z): 510.1 $[M+H]^+$.

Step b): Preparation of (*E*)-2,4-difluoro-*N*-(2-methoxy-5-(4-(1-(4-oxopent-2-enoyl)-1,2,3,6-tetrahydropyridin-4-yl)quinazolin-6-yl)pyridin-3-yl)benzenesulfonamide

2,4-Difluoro-*N*-(2-methoxy-5-(4-(1,2,3,6-tetrahydropyfidin-4-yl)quinazolin-6-yl)pyridine-3-benzenesulfonamide trifluoroacetate (50 mg, 0.080 mmol), (*E*)-4-oxopent-2-enoic acid (12 mg, 0.104 mmol), DIPEA (52 mg, 0.400 mmol) were dissolved in tetrahydrofuran (2 mL). At -78°C, $T_3P$ (56 mg, 0.088 mmol, 50% wt) was slowly added to the system. After the addition was completed, the reaction mixture was kept at -78°C and stirred for 1 h. Upon completion of the reaction, at -78°C, the reaction was quenched using saturated aqueous ammonium chloride solution (20 mL). The reaction mixture was extracted by adding dichloromethane (20 mL×2). The organic phases were combined, washed with saturated brine (20 mL), dried over anhydrous sodium sulfate and filtered. The filtrate was concentrated under reduced pressure. The crude product obtained was purified by Prep-HPLC (Method 2). (*E*)-2,4-difluoro-*N*-(2-methoxy-5-(4-(1-(4-oxopent-2-enoyl)-1,2,3,6-tetrahydropyridin-4-yl)quinazolin-6-yl)pyridin-3-yl)benzenesulfonamide was obtained, yield: 11.4%; $^1$H NMR (400 MHz, DMSO-$d_6$) δ 10.32 (brs, 1H), 9.25 (s, 1H), 8.47-8.39 (m, 2H), 8.31-8.24 (m, 1H), 8.12 (d, *J* = 8.8 Hz, 1H), 8.04-7.98 (m, 1H), 7.84-7.70 (m, 1H), 7.58-7.41 (m, 2H), 7.23-7.14 (m, 1H), 6.74 (d, *J* = 16.0 Hz, 1H), 6.51-6.41 (m, 1H), 4.57-4.35 (m, 2H), 3.98-3.87 (m, 2H), 3.70 (s, 3H), 2.87-2.72 (m, 2H), 2.37 (d, *J* = 13.2 Hz, 3H); ESI-MS(m/z): 606.0 $[M+H]^+$.

Referring to the preparation method of Example 198 and using the corresponding raw materials, the compounds in the following examples were obtained.

| Number | Name | Structure | $^1$H NMR and MS |
|---|---|---|---|
| **Example 199** | (*E*)-*N*-(2-methoxy-5-(4-(1-(4-oxopent-2-enoyl)-1,2,3,6-tetrahydropyridin-4-yl)quinazolin-6-yl)pyridin-3-yl)-2,4-dimethylthiazole-5-sulfonamide | 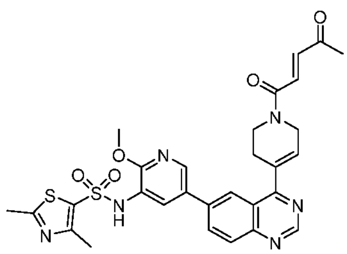 | $^1$H NMR (400 MHz, DMSO-$d_6$) δ 10.33 (brs, 1H), 9.25 (d, *J* = 1.6 Hz, 1H), 8.47-8.38 (m, 1H), 8.28-8.23 (m, 2H), 8.17-8.08 (m, 1H), 8.01-7.88 (m, 1H), 7.51 (dd, $J_1$ = 15.6 Hz, $J_2$ = 9.6 Hz, 1H), 6.73 (dd, $J_1$ = 15.6 Hz, $J_2$ = 9.6 Hz, 1H), 6.51-6.40 (m, 1H), 4.55 (d, *J* = 3.2 Hz, 1H), 4.40 (d, *J* = 3.2 Hz, 1H), 4.03-3.86 (m, 2H), 3.77 (s, 3H), 2.84-2.77 (m, 2H), 2.54 (s, 3H), 2.45-2.29 (m, 6H); ESI-MS(m/z): 605.0 $[M+H]^+$. |

(continued)

| Number | Name | Structure | $^1$H NMR and MS |
|---|---|---|---|
| **Example 200** | (*E*)-2-fluoro-*N*-(2-methoxy-5-(4-(1-(4-oxopent-2-enoyl)-1,2,3,6-tetrahydropyridin-4-yl) quinazolin-6-yl)pyridin-3-yl)benzenesulfona mide | 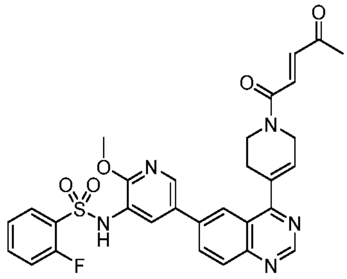 | $^1$H NMR (400 MHz, DMSO-$d_6$) δ 10.23 (s, 1H), 9.25 (s, 1H), 8.42-8.34 (m, 2H), 8.25-8.22 (m, 1H), 8.11 (d, *J* = 4.4 Hz, 1H), 7.95 (s, 1H), 7.76-7.69 (m, 1H), 7.62-7.48 (m, 2H), 7.44-7.37 (m, 1H), 7.27 (dd, $J_1$ = 15.2 Hz, $J_2$ = 7.6 Hz, 1H), 6.76-6.71 (m, 1H), 6.44 (d, *J* = 18.0 Hz, 1H), 4.54-4.38 (m, 2H), 3.96-3.89 (m, 2H), 3.69 (s, 3H), 2.84-2.77 (m, 2H), 2.39-2.34 (m, 3H); ESI-MS(m/z): 588.0 [M+H]$^+$. |
| **Example 201** | (*E*)-2,6-difluoro-*N*-(2-methoxy-5-(4-(1-(4-oxopent-2-enoyl)-1,2,3,6-tetrahydropyridin-4-yl) quinazolin-6-yl)pyridin-3-yl)benzenesulfona mide | 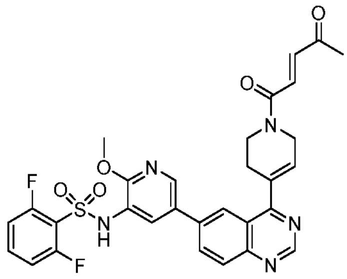 | $^1$H NMR (400 MHz, DMSO-$d_6$) δ 10.53 (s, 1H), 9.24 (s, 1H), 8.42 (dd, $J_1$ = 2.0 Hz, $J_2$ = 12.0 Hz, 1H), 8.26-8.20 (m, 2H), 8.12-8.09 (m, 1H), 7.96 (d, *J* = 12.8 Hz, 1H), 7.58-7.45 (m, 2H), 7.20-7.11 (m, 2H), 6.76-7.69 (m, 1H), 6.47-6.40 (m, 1H), 4.55-4.53 (m, 1H), 4.40-4.38 (m, 1H), 3.96-3.89 (m, 2H), 3.70 (d, *J* = 3.2 Hz, 3H), 2.84-2.77 (m, 2H), 2.39-2.36 (m, 3H); ESI-MS(m/z): 606.0 [M+H]$^+$. |
| **Example 202** | (*E*)-2-chloro-6-fluoro-*N*-(2-methoxy-5-(4-(1-(4-oxopent-2-enoyl)-1,2,3,6-tetrahydropyridin-4-yl) quinazolin-6-yl)pyridin-3-yl)benzenesulfona mide | 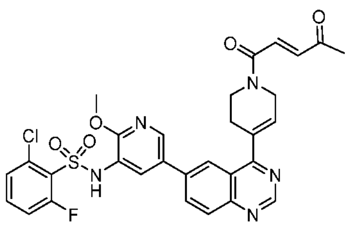 | $^1$H NMR (400 MHz, DMSO-$d_6$) δ 10.52 (s, 1H), 9.26 (d, *J* = 2.0 Hz, 1H), 8.54-8.47 (m, 1H), 8.47-8.41 (m, 1H), 8.33-8.23 (m, 1H), 8.13 (d, *J* = 8.8 Hz, 1H), 8.06 (dd, $J_1$ = 13.2 Hz, $J_2$ =2.4 Hz, 1H), 7.70-7.60 (m, 1H), 7.60-7.34 (m, 3H), 6.74 (d, *J* = 16.0 Hz, 1H), 6.53-6.41 (m, 1H), 4.56-4.36 (m, 2H), 3.98-3.86 (m, 2H), 3.67 (s, 3H), 2.88-2.73 (m, 2H), 2.37 (d, *J* = 16.0 Hz, 3H); ESI-MS (m/z): 622.0 [M+H]$^+$. |

**Example 203**

**Preparation of (*E*)-*N*-(2-methoxy-5-(4-(1-(4-oxopent-2-enoyl)piperidin-4-yl)quinazolin-6-yl)pyridin-3-yl)-2,4-dimethylthiazole-5-sulfonamide**

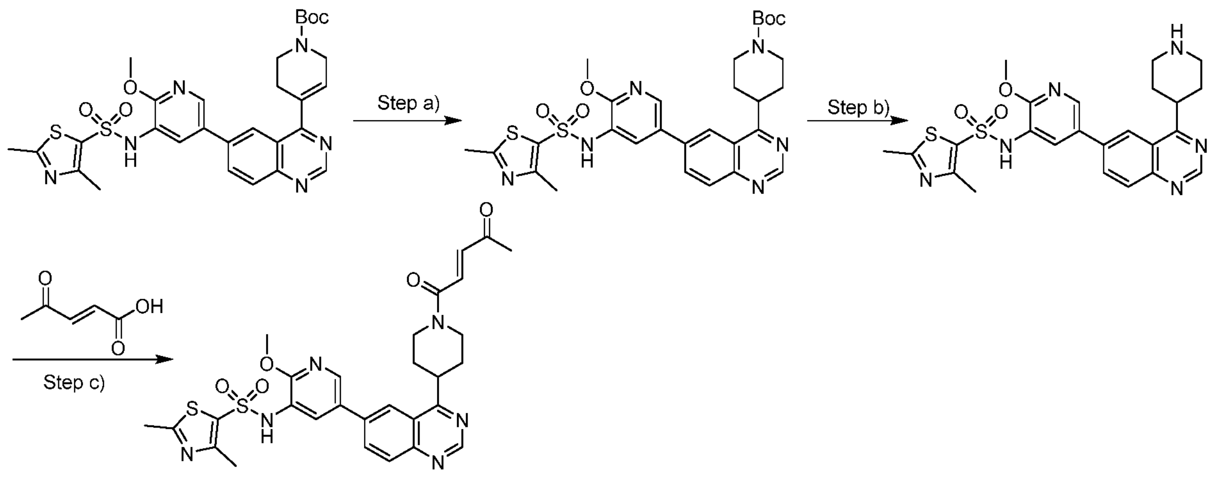


Step a): Preparation of tert-butyl 4-(6-(5-((2,4-dimethylthiazole)-5-sulfonamido)-6-methoxypyridin-3-yl)quinazolin-4-yl)piperidine-1-carboxylate

Tert-butyl 4-(6-(5-((2,4-dimethylthiazole)-5-sulfonamido)-6-methoxypyridin-3-yl)quinazolin-4-yl)-3,6-dihydropyridine-1(2*H*)-carboxylate (200 mg, 0.329 mmol) was added to methanol (10 mL). After that, glacial acetic acid (197 mg, 3.290 mmol) and 10% Pd/C (100 mg). After the addition was completed, under hydrogen atmosphere, the reaction mixture was kept at 30°C and reacted for 5 h. Upon completion of the reaction, the reaction mixture was filtered. The filtrate was concentrated under reduced pressure. The crude product obtained was purified by silica gel column chromatography (Eluent: dichloromethane/methanol=100/1 to 50/1). Tert-butyl 4-(6-(5-((2,4-dimethylthiazole)-5 -sulfonamido)-6-methoxypyridin-3 -yl)quinazolin-4-yl)piperidine-1 -carboxylate was obtained, yield: 39.4%; ESI-MS(m/z): 611.2 $[M+H]^+$.

Step b): Preparation of *N*-(2-methoay-5-(4-(piperidin-4-yl)quinazofin-6-yl)pyridin-3-yl)-2,4-dimethylthiazole-5-sulfonamide trifluoroacetate

Tert-butyl 4-(6-(5-((2,4-dimethylthiazole)-5-sulfonamido)-6-methoxypyridin-3-y1)quinazolin-4-yl)piperidine-1-carboxylate (50 mg, 0.082 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. *N*-(2-methoay-5-(4-(piperidin-4-yl)quinazolin-6-yl)pyridin-3-yl)-2,4-dimethylthiazole-5-sulfonamide trifluoroacetate was obtained, yield: 88.3%; ESI-MS(m/z): 511.1 $[M+H]^+$.

Step c): Preparation of (*E*)-*N*-(2-methoxy-5-(4-(1-(4-oxopent-2-enoyl)piperidin-4-yl)quinazolin-6-yl)pyridin-3-yl)-2,4-dimethylthiazole-5-sulfonamide

*N*-(2-methoay-5-(4-(piperidin-4-yl)quinazolin-6-yl)pyridin-3-yl)-2,4-dimethylthiazole-5-sulfonamide trifluoroacetate (30 mg, 0.048 mmol) and tetrahydrofuran (2 mL) were added to the reaction flask. The reaction system was cooled to -78°C, with *N*,*N*-diisopropylethylamine (11 mg, 0.288 mmol), (*E*)-4-oxopent-2-enoic acid (6 mg, 0.048 mmol) and 50% $T_3P$ ethyl acetate solution (61 mg, 0.096 mmol, 50%wt) added successively. After the addition was completed, the reaction mixture was kept at -78°C and stirred for 1 h. Upon completion of the reaction, the reaction solution was diluted by adding acetonitrile (1 mL), purified by Prep-HPLC (Method 2) and freeze dried. (*E*)-*N*-(2-methoxy-5-(4-(1-(4-oxopent-2-enoyl)piperidin-4-yl)quinazolin-6-yl)pyfidin-3-yl)-2,4-dimethylthiazole-5-sulfonamide was obtained, yield: 19.5%; $^1H$ NMR (400 MHz, DMSO-$d_6$) δ 10.27 (br s, 1H), 9.20 (s, 1H), 8.53 (s, 1H), 8.29 (s, 1H), 8.19-8.16 (m, 1H), 8.10 (d, *J* = 8.7 Hz, 1H), 7.91 (s, 1H), 7.50 (d, *J* = 15.6 Hz, 1H), 6.70 (d, *J* = 15.6 Hz, 1H), 4.66-4.56 (m, 1H), 4.29-4.18 (m, 2H), 3.77 (s, 3H), 3.53-3.40 (m, 1H), 3.10-3.00 (m, 1H), 2.55 (s, 3H), 2.40 (s, 3H), 2.37 (s, 3H), 2.02-1.77 (m, 4H); ESI-MS(m/z): 607.0 $[M+H]^+$.

Referring to the preparation method of Example 203 and using the corresponding raw materials, the compounds in the following examples were obtained.

| Number | Name | Structure | $^1$H NMR and MS |
|---|---|---|---|
| **Example 204** | (*E*)-2-fluoro-*N*-(2-methoxy-5-(4-(1-(4-oxopent-2-enoyl) piperidin-4-yl) quinazolin-6-yl)pyridin-3-yl)benzenesulfonamide | 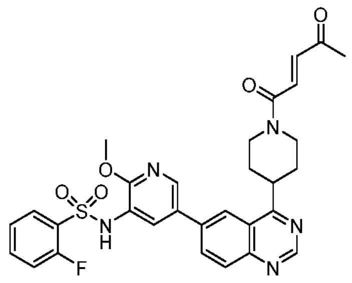 | $^1$H NMR (400 MHz, DMSO-$d_6$) δ 9.18 (s, 1H), 8.45 (s, 1H), 8.06 (s, 3H), 7.80-7.76 (m, 2H), 7.51-7.48 (m, 2H), 7.26-7.21 (m, 2H), 6.69 (d, *J* = 15.6 Hz, 1H), 4.61-4.58 (m, 1H), 4.27-4.14 (m, 2H), 3.76 (s, 3H), 3.51-3.44 (m, 1H), 3.06-2.97 (m, 1H), 2.37 (s, 3H), 2.01-1.88 (m, 3H), 1.83-1.74 (m, 1H); ESI-MS(m/z): 590.0 [M+H]$^+$. |
| **Example 205** | (*E*)-2,6-difluoro-*N*-(2-methoxy-5-(4-(1-(4-oxopent-2-enoyl) piperidin-4-yl) quinazolin-6-yl)pyridin-3-yl)benzenesulfonamide | 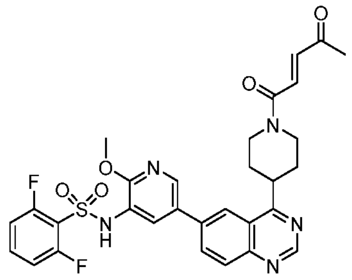 | $^1$H NMR (400 MHz, DMSO-$d_6$) δ 10.61 (s, 1H), 9.21 (s, 1H), 8.60 (s, 1H), 8.42 (s, 1H), 8.22 (d, *J* = 8.8 Hz, 1H), 8.13-8.03 (m, 2H), 7.63 (s, 1H), 7.42 (d, *J* = 16.0 Hz, 1H), 7.24-7.19 (m, 2H), 6.70 (d, *J* = 16.0 Hz, 1H), 4.62-4.59 (m, 1H), 4.27-4.24 (m, 2H), 3.68 (s, 3H), 3.48-3.41 (m, 1H), 3.06-3.00 (m, 1H), 2.37 (s, 3H), 2.01-1.78 (m, 4H); ESI-MS(m/z): 608.0 [M+H]$^+$. |
| **Example 206** | (*E*)-2-chloro-6-fluoro-*N*-(2-methoxy-5-(4-(1-(4-oxopent-2-enoyl)piperidin-4-yl) quinazolin-6-yl)pyridin-3-yl)benzenesulfonamide | 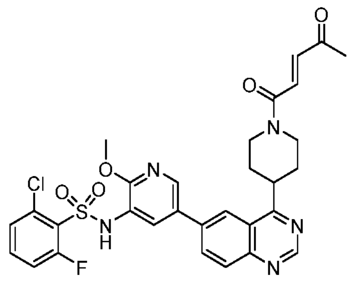 | $^1$H NMR (400 MHz, DMSO-$d_6$) δ 9.20 (s, 1H), 8.56 (s, 1H), 8.35 (br s, 1H), 8.19 (d, *J* = 8.8 Hz, 1H), 8.09 (d, *J* = 8.8 Hz, 1H), 7.96 (s, 1H), 7.60-7.29 (m, 4H), 6.70 (d, *J* = 15.6 Hz, 1H), 4.60 (d, *J* = 12.4 Hz, 1H), 4.31-4.16 (m, 2H), 3.69 (s, 3H), 3.44 (t, *J* = 12.8 Hz, 1H), 3.09-2.97 (m, 1H), 2.37 (s, 3H), 2.05-1.72 (m, 4H); ESI-MS(m/z): 624.0 [M+H]$^+$. |

**Example 207**

**Preparation of 2,4-difluoro-*N*-(2-methoxy-5-(4-(4-(vinylsulfonyl)piperazin-1-yl)quinazolin-6-yl)pyridin-3-yl)benzenesulfonamide**

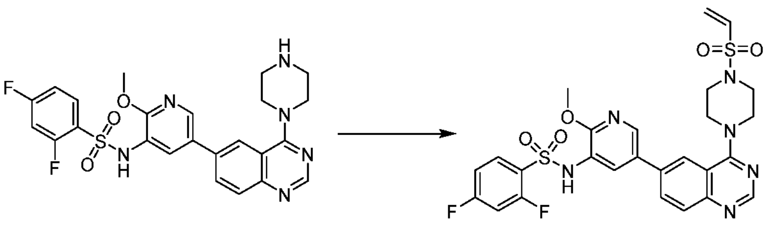

2,4-difluoro-*N*-(2-methoxy-5-(4-(piperazin-1-yl)quinazolin-6-yl)pyridin-3-yl)benzenesulfonamide trifluoroacetate (100 mg, 0.160 mmol) and tetrahydrofuran (5 mL) were added to the reaction flask. At 0°C, 4A molecular sieve (200 mg) was added. After that, triethylamine (81 mg, 0.800 mmol) was slowly added to the system. In the presense of protective nitrogen, 2-chloroethane-1-sulfonyl chloride (20 mg, 0.124 mmol) tetrahydrofuran solution (0.5 mL) was added dropwise. After the addition was completed, the reaction mixture was kept at 0°C and reacted for 1 h. Upon completion of the reaction, the reaction was quenched by adding saturated ammonium chloride (15 mL), and the reaction mixture was extracted with dichloromethane (15 mL×2). The organic phases were combined, dried over anhydrous sodium

sulfate and filtered. The filtrate was concentrated under reduced pressure. The crude product obtained was purified by Prep-HPLC (Method 2). 2,4-Difluoro-*N*-(2-methoxy-5-(4-(4-(vinylsulfonyl)piperazin-1-yl)quinazolin-6-yl)pyridin-3-yl)benzenesulfonamide was obtained, yield: 38.2%; $^1$H NMR (400 MHz, DMSO-$d_6$) δ 10.35 (s, 1H), 8.69 (s, 1H), 8.45 (s, 1H), 8.15-8.06 (m, 2H), 8.03-7.98 (m, 1H), 7.93 (d, *J* = 8.4 Hz, 1H), 7.82-7.73 (m, 1H), 7.56 (t, *J* = 9.6 Hz, 1H), 7.21 (dd, *J* = 8.8, 2.4 Hz, 1H), 6.93-6.82 (m, 1H), 6.24-6.13 (m, 2H), 3.96-3.80 (m, 4H), 3.69 (s, 3H), 3.32-3.27 (m, 4H); ESI-MS(m/z): 503.1 [M+H]$^+$.

**Example 208**

**Preparation of (*E*)-*N*-(2-(difluoromethoxy)-5-(4-(4-(4-oxopent-2-enoyl)piperazin-1-yl)quinazoline-6-yl)pyridin-3-yl)-2,4-difluorobenzenesulfonamide**

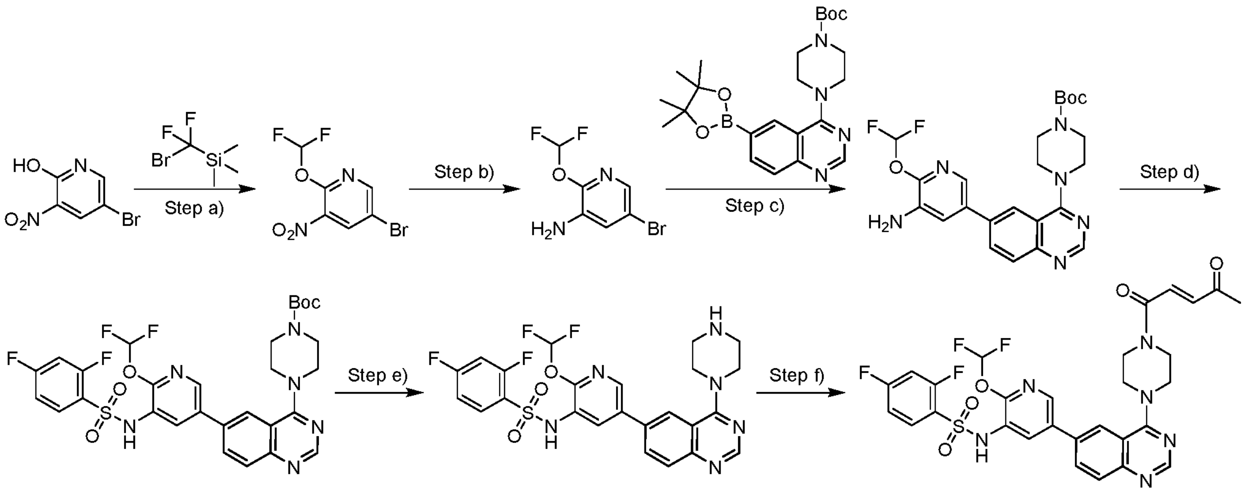


Step a): Preparation of 5-bromo-2-(difluoromethoxy)-3-nitropyridine

5-Bromo-3-nitropyridin-2-ol (1.0 g, 4.566 mmol) and sodium carbonate (532 mg, 5.023 mmol) were added to acetonitrile (30 mL). The reaction mixture was heated to 60°C and reacted for 15 min, with (bromodifluoromethyl)trimethylsilane (1.4 g, 6.849 mmol) slowly added to the reaction solution. After the addition was completed, the reaction mixture was heated to 75°C and reacted for 4 h. Upon completion of the reaction, the reaction was quenched by adding water (30 mL). The reaction mixture was extracted with ethyl acetate (60 mL×2). The organic phases were combined, dried over anhydrous sodium sulfate and filtered. The filtrate was concentrated under reduced pressure. The residue was purified by silica gel column chromatography (Eluent: petroleum ether/ethyl acetate=3/1 to 1/1). 5-Bromo-2-(difluoromethoxy)-3-nitropyridine was obtained, yield: 28.5%; ESI-MS (m/z): 268.9 [M+H]$^+$.

Step b): Preparation of 5-bromo-2-(difluoromethoxy)pyridin-3-amine

5-Bromo-2-(difluoromethoxy)-3-nitropyridine (650 mg, 2.416 mmol) and Pt/C (700 mg, 0.179mmol, 5%wt%) and ethyl acetate (10mL) were added to the reaction flask. Under hydrogen atmosphere, the reaction mixture was kept at room temperature and reacted for 4 h. Upon completion of the reaction, the reaction solution was filtered. The filtrate was concentrated under reduced pressure. 5-Bromo-2-(difluoromethoxy)pyridin-3-amine was obtained, yield: 74.5%; ESI-MS(m/z): 239.0 [M+H]$^+$.

Step c): Preparation of tert-butyl 4-(6-(5-amino-6-(difluoromethoxy)pyridin-3-yl)quinazolin-4-yl)piperazine-1-carboxylate

5-Bromo-2-(difluoromethoxy)pyridin-3-amine (200 mg, 0.837 mmol), tert-butyl 4-(6-(4,4,5,5-tetramethyl-1,3,2-dioxaborolan-2-yl)quinazolin-4-yl)piperazine-1-carboxylate (442 mg, 1.004 mmol), Pd(dppf)$Cl_2$ (61 mg, 0.084 mmol), cesium carbonate (821 mg, 2.511mmol), 1,4-dioxane (10 mL) and water (2 mL) were added to the reaction flask, in the presense of protective nitrogen, the reaction mixture was heated to reflux, stirred and reacted for 2 h. Upon completion of the reaction, the reaction solution was concentrated under reduced pressure. The residue was purified by silica gel column chromatography (Eluent: dichloromethane/methanol = 20/1 to 10/1). Tert-butyl 4-(6-(5-amino-6-(difluoromethoxy)pyridin-3-yl)quinazolin-4-yl)piperazine-1-carboxylate was obtained, yield: 86.0%; ESI-MS(m/z): 473.6 $[M+H]^+$.

Step d): Preparation of tert-butyl 4-(6-(6-(difluoromethoxy)-5-((2,4-difluorophenyl)sulfonamido)pyridin-3-yl)quinazolin-4-yl)piperazine-1-carboxylate

Tert-butyl 4-(6-(5-amino-6-(difluoromethoxy)pyridin-3-yl)quinazolin-4-yl)piperazine-1-carboxylate (360 mg, 0.762 mmol) and 2,4-difluorobenzenesulfonyl chloride (162 mg, 0.914 mmol) were dissolved in pyridine (8 mL). The reaction mixture was kept at room temperature and reacted overnight. Upon completion of the reaction, the reaction was quenched by adding water (50 mL). The reaction mixture was extracted by adding dichloromethane (100 mL). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate=3/1 to 1/1). Tert-butyl 4-(6-(6-(difluoromethoxy)-5-((2,4-difluorophenyl)sulfonamido)pyridin-3-yl)quinazolin-4-yl)piperazine-1-carboaylate was obtained, yield: 54.7%; ESI-MS(m/z): 649.6 $[M+H]^+$.

Step e): Preparation of *N*-(2-(difluoromethoxy)-5-(4-(piperazin-1-yl)quinazolin-6-yl)pyridin-3-yl)-2,4-difluorobenzenesulfonamide trifluoroacetate

Tert-butyl 4-(6-(6-(difluoromethoxy)-5-((2,4-difluorophenyl)sulfonamido)pyridin-3-yl)quinazolin-4-yl)piperazine-1-carboxylate (270 mg, 0.416 mmol) was dissolved in dichloromethane (4 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. *N*-(2-(difluoromethoxy)-5-(4-(piperazin-1-yl)quinazolin-6-yl)pyridin-3-yl)-2,4-difluorobenzenesulfonamide trifluoroacetate was obtained; ESI-MS(m/z): 549.1 $[M+H]^+$.

Step f): Preparation of (*E*)-*N*-(2-(difluoromethoxy)-5-(4-(4-(4-oxopent-2-enoyl)piperazin-1-yl)quinazoline-6-yl)pyridin-3-yl)-2,4-difluorobenzenesulfonamide

*N*-(2-(difluoromethoxy)-5-(4-(piperazin-1-yl)quinazolin-6-yl)pyridin-3-yl)-2,4-difluorobenzenesulfonamide trifluoroacetate (150 mg, 0.226 mmol) and tetrahydrofuran (5 mL) were added to the reaction flask. The reaction system was cooled to -78°C, with DIPEA (149 mg, 1.130 mmol), (E)-4-oxopent-2-enoic acid (29 mg, 0.249 mmol) and 50% $T_3P$ ethyl acetate solution (293 mg, 0.452 mmol, 50%wt) added successively. After the addition was completed, the reaction mixture was kept at -78°C and stirred for 1 h. Upon completion of the reaction, the reaction was quenched using saturated aqueous ammonium chloride solution (50mL) at -78°C. The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). (*E*)-*N*-(2-(difluoromethoxy)-5-(4-(4-(4-oxopent-2-enoyl)piperazin-1-yl)quinazoline-6-(yl)pyridin-3-yl)-2,4-difluorobenzenesulfonamide was obtained, yield: 13.8%; $^1H$ NMR (400 MHz, DMSO-$d_6$) $\delta$ 11.73 (s, 1H), 8.65 (s, 1H), 8.17-8.03 (m, 1H), 7.99-7.82 (m, 4H), 7.65-7.52 (m, 1H), 7.44 (d, *J* = 15.6 Hz, 2H), 7.36-7.27 (m, 1H), 7.19-7.09 (m, 1H), 6.73 (d, *J* = 16.0 Hz, 1H), 4.02-3.72 (m, 8H), 2.36 (s, 3H); ESI-MS(m/z): 645.0 $[M+H]^+$.

**Example 209**

**Preparation of (*E*)-3-fluoro-4-(*N*-(2-methoxy-5-(4-(4-(4-oxopent-2-enoyl)piperazin-1-yl)quinazolin-6-yl)pyridin-3-yl)sulfamoyl)benzoic acid**

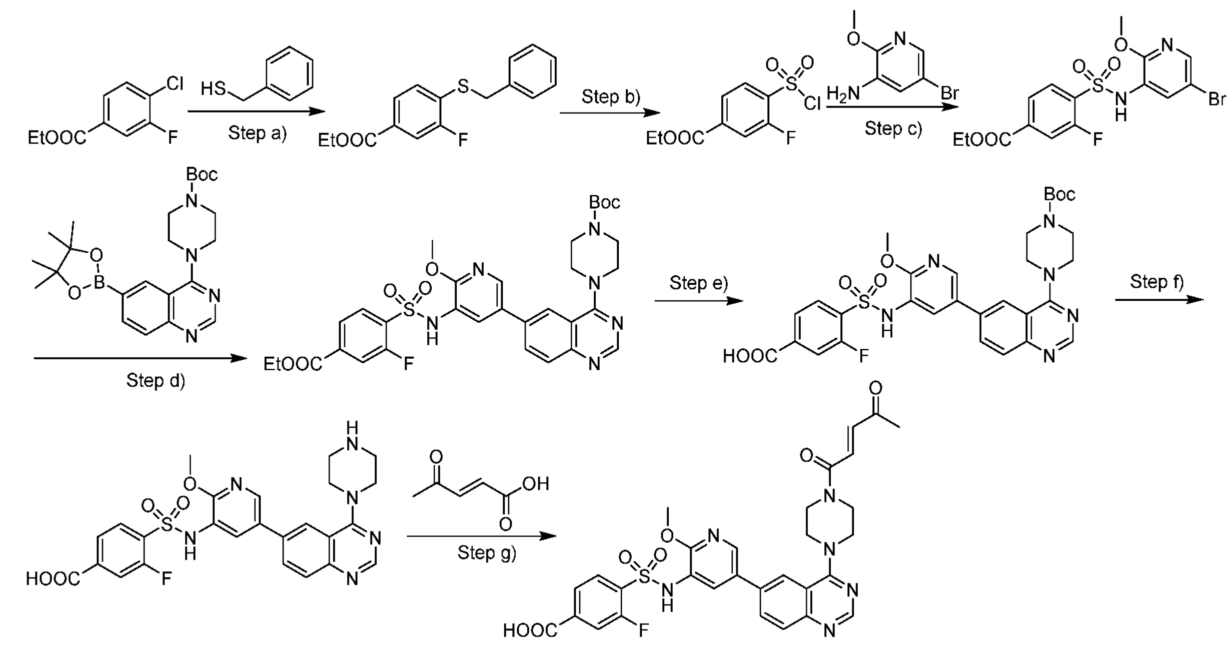


Step a): Preparation of ethyl 4-(benzylthio)-3-fluorobenzoate

Ethyl 4-chloro-3-fluorobenzoate (1.0 g, 4.936 mmol), cesium carbonate (1.61 g, 4.936 mmol) and DMF (10 mL) were added to the reaction flask, with benzylthiol (736 mg, 5.923 mmol) added. After the addition was completed, the reaction mixture was stirred at room temperature and reacted overnight. Upon completion of the reaction, the reaction was quenched by adding water (50 mL), and the reaction mixture was extracted with dichloromethane (150 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: ethyl acetate/petroleum ether = 1/20 to 1/5). Ethyl 4-(benzylthio)-3-fluorobenzoate was obtained, yield: 59.3%; $^1$H NMR (400 MHz, DMSO-$d_6$) δ 7.73 (dd, $J_1$ = 8.0, $J_2$ = 1.6 Hz, 1H), 7.67-7.57 (m, 2H), 7.43-7.39 (m, 2H), 7.35-7.25 (m, 3H), 4.37 (s, 2H), 4.30 (q, $J$ = 7.2 Hz, 2H), 1.31 (t, $J$ = 7.2 Hz, 3H).

Step b): Preparation of ethyl 4-(chlorosulfonyl)-3-fluorobenzoate

Ethyl 4-(benzylthio)-3-fluorobenzoate (400 mg, 1.378 mmol) was added to acetonitrile (8 mL). After that, glacial acetic acid (414 mg, 6.890 mmol) and water (2 mg, 0.138 mmol) were added to the system. After the addition was completed, under the ice bath condition, 1,3-dichloro-5,5-dimethylhydantoin (543 mg, 2.756 mmol) was added. After the addition was completed, the reaction mixture was kept under the ice bath condition and reacted for 2 h. Upon completion of the reaction, the reaction was quenched by adding water (50 mL), and the reaction mixture was extracted with dichloromethane (100 mL×2). The organic phases were combined, washed with saturated brine (50 mL), dried over anhydrous sodium sulfate and filtered. The filtrate was concentrated under reduced pressure. Ethyl 4-(chlorosulfonyl)-3-fluorobenzoate was obtained, yield: 97.8%.

Step c): Preparation of ethyl 4-(N-(5-bromo-2-methoxypyridin-3-yl)sulfamoyl)-3-fluorobenzoate

Ethyl 4-(chlorosulfonyl)-3-fluorobenzoate (180 mg, 0.675 mmol), 5-bromo-2-methoxypyridin-3-amine (137 mg, 0.675 mmol), pyridine (160 mg, 2.025 mmol), 4-dimethylaminopyridine (8 mg, 0.068 mmol) were added to dichloromethane (100 mL). After the addition was completed, the reaction mixture was kept at room temperature and reacted overnight. Upon completion of the reaction, the reaction was quenched by adding water (50 mL), and the reaction mixture was extracted with dichloromethane (100 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: methanol/dichloromethane = 1/100 to 1/30). Ethyl 4-(N-(5-bromo-2-methoxypyridin-3-yl)sulfamoyl)-3-fluorobenzoate was obtained, yield: 45.7%; ESI-MS(m/z): 433.0 [M+H]$^+$.

Step d): Preparation of tert-butyl 4-(6-(5-((4-(ethoxycarbonyl)-2-fluorophenyl)sulfonamido)-6-methoxypyridin-3-yl)quinazolin-4-yl)piperazine-1-carboxylate

Ethyl 4-(*N*-(5-bromo-2-methoxypyridin-3-yl)sulfamoyl)-3-fluorobenzoate (200 mg, 0.462 mmol), tert-butyl 4-(6-(4,4,5,5-tetramethyl-1,3,2-dioxaborolan-2-yl)quinazolin-4-yl)piperazine-1-carboxylate (203 mg, 0.462 mmol), Pd(dppf)$Cl_2$ (34 mg, 0.046 mmol) and cesium carbonate (452 mg, 1.386 mmol) were added to dioxane/water mixed solvent (5 mL, v/v = 10: 1). After the addition was completed, in the presense of protective nitrogen, the reaction mixture was heated to reflux, stirred and reacted for 3 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/30). Tert-butyl 4-(6-(5-((4-(ethoxycarbonyl)-2-fluorophenyl)sulfonamido)-6-methoxypyridin-3 -yl)quinazolin-4-yl)piperazine-1 -carboxylate was obtained, yield: 59.6%; ESI-MS(m/z): 667.2 $[M+H]^+$.

Step e): Preparation of 4-(*N*-(5-(4-(4-(tert-butoaycarbonyl)piperazin-1-yl)quinazofin-6-yl)-2-methoaypyridin-3-yl)sulfamoyl)-3-fluorobenzoic acid

Tert-butyl4-(6-(5-((4-(ethoxycarbonyl)-2-fluorophenyl)sulfonamido)-6-methoxypyridin-3-yl)quinazolin-4-yl)piperazine-1-carboxylate (200 mg, 0.300 mmol) was added to methanol (10 mL), then with sodium hydroxide solution (4 mL, 2M) added. After the addition was completed, the reaction mixture was kept at room temperature and reacted overnight. Upon completion of the reaction, the reaction mixture was concentrated under reduced pressure to remove methanol. The residue was diluted by adding water (30 mL). The impurities were extracted by adding ethyl acetate (50 mL), and the aqueous phase was adjusted to pH = 3 with dilute hydrochloric acid solution (2M) to precipitate solids. The solution was filtered and the filter cake was collected and vacuum dried to obtain 4-(*N*-(5-(4-(4-(tert-butoxycarbonyl)piperazin-1-yl)quinazolin-6-yl)-2-methoxypyridin-3-yl)sulfamoyl)-3-fluorobenzoic acid, yield: 48.3%; ESI-MS(m/z): 639.2 $[M+H]^+$.

Step f): Preparation of 3-fluoro-4-(*N*-(2-methoxy-5-(4-(piperazin-1-yl)quinazolin-6-yl)pyfidin-3-yl)sulfamoyl)benzoic acid trifluoroacetate

4-(*N*-(5-(4-(4-(tert-butoxycarbonyl)piperazin-1yl)quinazolin-6-yl)-2-methoxypyridin-3-yl)sulfamoyl)-3-fluorobenzoic acid (60 mg, 0.094 mmol) was dissolved in dichloromethane (4 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 3-Fluoro-4-(*N*(2-methoxy-5-(4-(piperazin-1-yl)quinazofin-6-yl)pyridin-3-yl)sulfamoyl)benzoic acid trifluoroacetate was obtained, yield: 91.2%; ESI-MS(m/z): 539.1 $[M+H]^+$.

Step g): Preparation of (*E*)-3-fluoro-4-(*N*-(2-methoxy-5-(4-(4-(4-oxopent-2-enoyl)piperazin-1-yl)quinazolin-6-yl)pyridin-3-yl)sulfamoyl)benzoic acid

3-Fluoro-4-(*N*-(2-methoxy-5-(4-(piperazin-1-yl)quinazolin-6-yl)pyridin-3-yl)sulfamoyl)benzoic acid trifluoroacetate (40 mg, 0.061 mmol) and tetrahydrofuran (2 mL) were added to the reaction flask. The reaction system was cooled to -78°C, with DIPEA (47 mg, 0.366 mmol), (E)-4-oxopent-2-enoic acid (7 mg, 0.061 mmol) and 50% $T_3P$ ethyl acetate solution (78 mg, 0.122 mmol, 50%wt) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). (E)-3-fluoro-4-(N-(2-methoxy-5-(4-(4-(4-oxopent-2-enoyl)piperazin-1-yl)quinazolin-6-yl)pyridin-3-yl)sulfamoyl)benzoic acid was obtained, yield: 10.5%; $^1$H NMR (400 MHz, DMSO-$d_6$) δ 8.66 (s, 1H), 8.14 (s, 1H), 8.12-8.01 (m, 2H), 7.96-7.87 (m, 2H), 7.85-7.69 (m, 3H), 7.45 (d, *J* = 16.0 Hz, 1H), 6.73 (d, *J* = 16.0 Hz, 1H), 4.02-3.72 (m, 8H), 3.67 (s, 3H), 2.37 (s, 3H); ESI-MS(m/z): 635.0 $[M+H]^+$.

**Example 210**

**Preparation of (*E*)-3,5-difluoro-*N*-(2-methoxy-5-(4-(4-(4-oxopent-2-enoyl)piperazin-1-yl)pyrido[3,2-*d*]pyrimidin-6-yl)pyridin-3-yl)pyridine-4-sulfonamide**

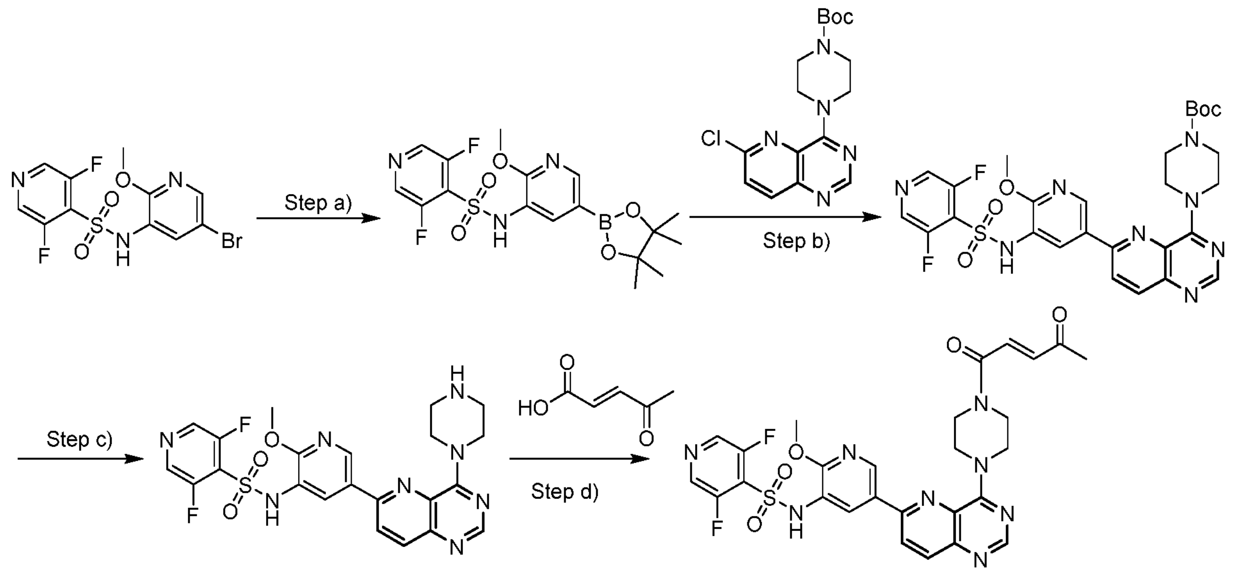


Step a): Preparation of 3,5-difluoro-*N*-(2-methoxy-5-(4,4,5,5-tetramethyl-1,3,2-dioxaborolan-2-yl)pyfidine-3-yl)pyridine-4-sulfonamide

N-(5-bromo-2-methoxypyridin-3-yl)-3,5-difluoropyridine-4-sulfonamide (100 mg, 0.263 mmol), pinacol diborate (90 mg, 0.354 mmol), Pd(dppf)$Cl_2$ (19 mg, 0.026 mmol) and potassium acetate (77 mg, 0.789 mmol) were added to 1,4-dioxane (10 mL). After the addition was completed, in the presense of protective nitrogen, the reaction mixture was heated to 90°C and reacted for 2 h. Upon completion of the reaction, the reaction was quenched by adding water (50 mL). The reaction mixture was extracted with ethyl acetate (100 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: dichloromethane/methanol=100/1 to 50/1). 3,5-Difluoro-*N*-(2-methoxy-5-(4,4,5,5-tetramethyl-1,3,2-dioxaborolan-2-yl)pyridine-3-yl)pyridine-4-sulfonamide was obtained, yield: 91.2%; ESI-MS(m/z): 428.1 $[M+H]^+$.

Step b): Preparation of tert-butyl 4-(6-(5-((3,5-difluoropyridine)-4-sulfonamido)-6-methoxypyridin-3-yl)pyrido [3,2-*d*]pyrimidin-4-yl)piperidine-1-carboaylate

3,5-Difluoro-*N*-(2-methoxy-5-(4,4,5,5-tetramethyl-1,3,2-dioxaborolan-2-yl)pyridine-3-yl)pyridine-4-sulfonamide (100 mg, 0.234 mmol), tert-butyl 4-(6-chloropyrido[3,2-*d*]pyrimidin-4-yl)piperazine-1-carboaylate (82 mg, 0.234 mmol), Pd(dppf)$Cl_2$ (17 mg, 0.023 mmol) and cesium carbonate (229 mg, 0.702 mmol) were added to 1,4-dioxane/water mixed solvent (5 mL, v/v = 10:1). After the addition was completed, in the presense of protective nitrogen, the reaction mixture was heated to 100°C and stirred for 2 h. Upon completion of the reaction, the reaction was quenched by adding water (50 mL). The reaction mixture was extracted with ethyl acetate (100 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: dichloromethane/methanol = 100/1 to 50/1). Tert-butyl 4-(6-(5-((3,5-difluoropyridine)-4-sulfonamido)-6-methoaypyridin-3-yl)pyrido[3,2-*d*]pyrimidin-4-yl)piperidine-1-carboaylate was obtained, yield: 78.6%; ESI-MS(m/z): 615.2 $[M+H]^+$.

Step c): Preparation of 3,5-difluoro-*N*-(2-methoxy-5-(4-(pipemzin-1-yl)pyrido[3,2-*d*]pyriniidin-6-yl)pyridin-3-yl)pyridine-4-sulfonamide trifluoroacetate

tert-butyl 4-(6-(5-((3,5-difluoropyridine)-4-sulfonamido)-6-methoxypyridin-3-yl)pyrido[3,2-*d*]pyrimidin-4-yl)piperidine-1-carboxylate (70 mg, 0.114 mmol) was dissolved in dichloromethane (4 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 3 ,5-difluoro-*N*-(2-methoxy-5-(4-(piperazin-1-yl)pyrido[3,2-*d*]pyrimidin-6-yl)pyridin-3-y1)pyridine-4-sulfonamide trifluoroacetate trifluoroacetate was obtained, yield: 90.6%; ESI-MS(m/z): 515.1 $[M+H]^+$.

Step d): Preparation of (*E*)-3,5-difluoro-*N*-(2-methoxy-5-(4-(4-(4-oxopent-2-enoyl)piperazin-1-yl)pyrido[3,2-*d*]pyrimidin-6-yl)pyridin-3-yl)pyridine-4-sulfonamide

3,5-Difluoro-*N*-(2-methoay-5-(4-(piperazin-1-yl)pyrido[3,2-*d*]pyrimidin-6-yl)pyridin-3-yl)pyridine-4-sulfonamide trifluoroacetate (50 mg, 0.080 mmol) and tetrahydrofuran (2 mL) were added to the reaction flask. The reaction system was cooled to -78°C, with DIPEA (62 mg, 0.480 mmol), (*E*)-4-oxopent-2-enoic acid (9 mg, 0.080 mmol) and 50% $T_3P$ ethyl acetate solution (102 mg, 0.160 mmol, 50%wt) added successively. After the addition was completed, the reaction mixture was kept at -78°C and stirred for 1 h. Upon completion of the reaction, the reaction solution was diluted by adding acetonitrile (1 mL) and purified by Prep-HPLC (Method 2). (*E*)-3,5-difluoro-N-(2-methoxy-5-(4-(4-(4-oxopent-2-enoyl)piperazin-1-yl)pyrido[3,2-d]pyrimidin-6-yl)pyridin-3-yl)pyridine-4-sulfonamide was obtained, yield: 16.1%; $^1$H NMR (400 MHz, DMSO-$d_6$) δ 11.20 (brs, 1H), 8.73-8.40 (m, 4H), 8.38-8.26 (m, 2H), 8.18 (d, *J* = 9.2 Hz, 1H), 7.47 (d, *J* = 16.0 Hz, 1H), 6.72 (d, *J* = 15.6 Hz, 1H), 4.78-4.22 (m, 4H), 4.01-3.76 (m, 4H), 3.72 (s, 3H), 2.37 (s, 3H); ESI-MS(m/z): 611.0 [M+H]$^+$.

Referring to the preparation method of Example 210 and using the corresponding raw materials, the compounds in the following examples were obtained.

| Number | Name | Structure | $^1$H NMR and MS |
|---|---|---|---|
| **Example 211** | 2,6-difluoro-*N*-(5-(4-(4-(2-fluoroacryloyl)pipe razin-1-yl)pyrido [3,2-*d*] pyrimidin-6-yl)-2-methoxypyridin-3-yl) benzenesulfona mide | 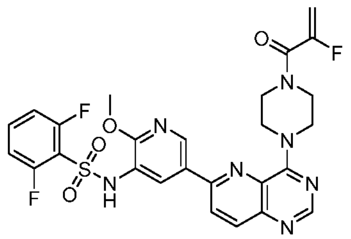 | $^1$**H** NMR (400 MHz, DMSO-$d_6$) δ 10.63 (s, 1H), 8.80-8.77 (m, 1H), 8.56 (s, 1H), 8.45-8.39 (m, 2H), 8.21 (d, *J* = 8.8 Hz, 1H), 7.73-7.65 (m, 1H), 7.25 (t, *J* = 9.2 Hz, 2H), 5.37-5.20 (m, 2H), 4.50 (s, 4H), 3.82 (s, 4H), 3.72 (s, 3H); ESI-MS(m/z): 586.0 [M+H]$^+$. |
| **Example 212** | (*E*)-2,6-difluoro-*N*-(2-methoxy-5-(4-(4-(4-oxohex-2-enoyl) piperazin-1-yl)pyrido [3,2-*d*]pyrimidin-6-yl)pyridin-3-yl)benzenesulfona mide | 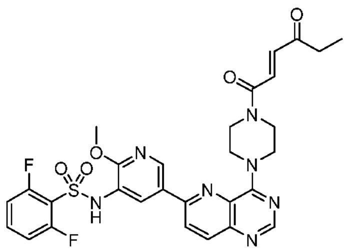 | $^1$H NMR (400 MHz, DMSO-$d_6$) δ 10.62 (s, 1H), 8.75 (s, 1H), 8.56 (s, 1H), 8.40 (d, *J* = 8.4 Hz, 2H), 8.20 (d, J = 8.8 Hz, 1H), 7.72-7.65 (m, 1H), 7.24 (t, *J* = 18.8 Hz, 2H), 6.58 (d, *J* = 12.0 Hz, 1H), 6.44 (d, *J* = 15.6 Hz, 1H), 4.47 (s, 4H),3.74 (s, 2H), 3.73 (s, 3H),3.65-3.62 (m, 2H), 2.60-2.54 (m, 2H), 0.94 (t, *J* = 14.4 Hz, 3H); ESI-MS (m/z): 624.0 [M+H]$^+$. |
| **Example 213** | (*E*)-2,6-difluoro-*N*-(5-(4-(4-(4-fluorobut-2-enoyl)piperazin-1-yl) pyrido [3,2-*d*]pyrimidine-6-yl)-2-methoxypyridin-3-yl)benzenesulfona mide | 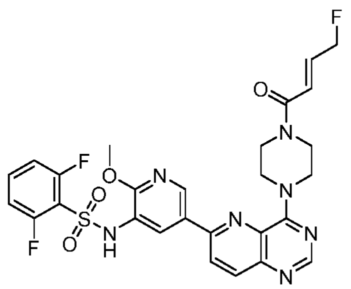 | $^1$H NMR (400 MHz, DMSO-$d_6$) δ 10.64 (s, 1H), 8.61 (d, *J* = 44.4 Hz, 2H), 8.39-8.33 (m, 2H), 8.19 (d, *J* = 8.8 Hz, 1H), 7.66-7.57 (m, 1H), 7.19 (t, *J* = 9.2 Hz, 2H), 6.87-6.71 (m, 2H), 5.22-5.17 (m, 1H), 5.09-5.06 (m, 1H), 4.47 (s, 4H), 3.89-3.76 (m, 4H), 3.73 (s, 3H); ESI-MS(m/z): 600.0 [M+H]$^+$. |
| **Example 214** | *N*-(5-(4-(4-acryloylpiperazin-1-yl) pyrido [3,2-*d*]pyrimidin-6-yl)-2-methoxypyridin-3-yl)-2,6-difluorobenzenesul fonamide | 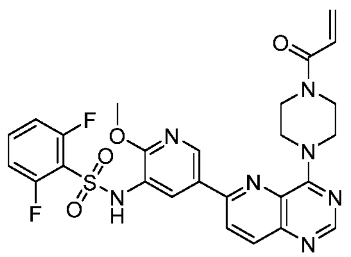 | $^1$H NMR (400 MHz, DMSO-$d_6$) δ 10.65 (s, 1H), 8.80-8.77 (m, 1H), 8.56 (s, 1H), 8.43-8.39 (m, 2H), 8.20 (d, *J* = 8.8 Hz, 1H), 7.73-7.64 (m, 1H), 7.25 (t, *J* = 9.2 Hz, 2H), 6.83 (dd, $J_1$ = 16.8 Hz, $J_2$ =10.4 Hz, 1H), 6.21-6.15 (m, 1H), 5.77-5.72 (m, 1H), 4.47 (s, 4H), 3.88-3.77 (m, 4H), 3.72 (s, 3H); ESI-MS(m/z): 568.0 [M+H]$^+$. |

(continued)

| Number | Name | Structure | $^1$H NMR and MS |
|---|---|---|---|
| **Example 215** | (*E*)-2,6-difluoro-*N*-(2-methoxy-5-(4-(4-(4-methoxybut-2-enoyl) piperazin-1-yl)pyridino [3,2-*d*]pyrimidin-6-yl) pyridin-3-yl) benzenesulfona mide | 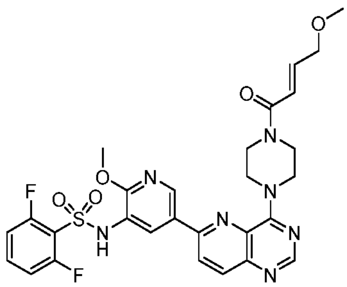 | $^1$H NMR (400 MHz, DMSO-$d_6$) δ 10.63 (s, 1H), 8.76(s, 1H), 8.55(s, 1H), 8.40 (d, *J* = 10.0 Hz, 2H), 8.20 (d, *J* = 8.8 Hz, 1H), 7.74-7.59 (m, 1H), 7.24 (t, *J* = 9.2 Hz, 2H), 6.71 (m, 2H), 4.45 (m, 4H), 4.08 (m, 2H), 3.82 (m, 4H), 3.73 (s, 3H), 3.29 (s, 3H); ESI-MS(m/z): 612.2 [M+H]$^+$. |
| **Example 216** | (*E*)-2,6-difluoro-*N*-(2-methoxy-5-(4-(4-(4,4,4-trifluorobut-2-enoyl) piperazin-1-yl)pyridino [3,2-*d*]pyrimidin-6-yl) pyridin-3-yl) benzenesulfona mide | 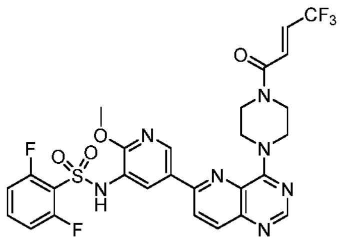 | $^1$H NMR (400 MHz, DMSO-$d_6$) δ 10.62 (s, 1H), 8.73 (s, 1H), 8.56 (s, 1H), 8.38 (d, *J* = 8.8 Hz, 2H), 8.20 (d, *J* = 8.8 Hz, 1H), 7.68-7.63 (m, 1H), 7.40 (d, *J* = 15.6 Hz, 1H), 7.22 (t, *J* = 18.8 Hz, 2H), 6.87-6.78 (m, 1H), 4.49 (s, 4H), 3.88 (s, 2H), 3.81 (s, 2H),3.73 (s, 3H); ESI-MS (m/z): 636.0 [M+H]$^+$. |
| **Example 217** | (*E*)-2,5-difluoro-*N*-(2-methoxy-5-(4-(4-(4-oxopent-2-enoyl) piperazin-1-yl)pyrido [3,2-*d*]pyrimidin-6-yl)pyridin-3-yl)benzenesulfona mide | 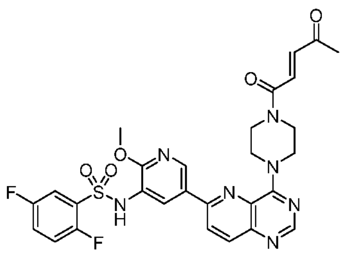 | $^1$H NMR (400 MHz, DMSO-$d_6$) δ 10.31 (s, 1H), 8.62 (d, *J*=4.8 Hz, 1H), 8.55 (s, 1H), 8.38-8.34 (m, 2H), 8.18 (d, *J* = 8.8 Hz, 1H), 7.52-7.41 (m, 4H), 6.72 (d, *J* = 16.0 Hz, 1H), 4.72-4.26 (m, 4H), 3.95-3.92 (m, 2H), 3.83-3.77 (m, 5H), 2.36 (s, 3H); ESI-MS(m/z): 610.0 [M+H]$^+$. |
| **Example 218** | (*E*)-2,3-difluoro-*N*-(2-methoxy-5-(4-(4-(4-oxopent-2-enoyl) piperazin-1-yl)pyrido [3,2-*d*]pyrimidin-6-yl)pyridin-3-yl)benzenesulfona mide | 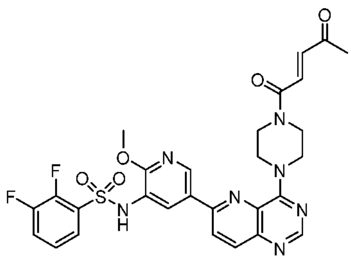 | $^1$H NMR (400 MHz, DMSO-$d_6$) δ10.59 (brs, 1H), 8.76 (s, 1H), 8.57 (s, 1H), 8.44-8.36 (m, 2H), 8.21 (d, *J* = 8.8 Hz, 1H), 7.77-7.66 (m, 1H), 7.54-7.42 (m, 2H), 7.36-7.27 (m, 1H), 6.72 (d, *J* = 15.6 Hz, 1H), 4.64-4.37 (m, 4H), 3.98-3.79 (m, 4H), 3.73 (s, 3H), 2.36 (s, 3H); ESI-MS(m/z): 610.0 [M+H]$^+$. |
| **Example 219** | (*E*)-2,4,6-trifluoro-*N*-(2-methoxy-5-(4-(4-(4-oxopent-2-enoyl) piperazin-1-yl)pyrido [3,2-*d*]pyrimidin-6-yl)pyridin-3-yl)benzenesulfona mide | 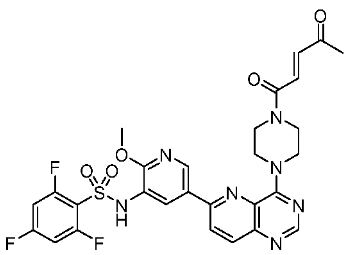 | $^1$H NMR (400 MHz, DMSO-$d_6$) δ 10.67 (s, 1H), 8.61 (s, 1H), 8.55 (s, 1H), 8.36-8.32 (m, 2H), 8.18 (d, *J* = 8.8 Hz, 1H), 7.46 (d, *J* = 16.0 Hz, 1H), 7.28 (t, *J* = 9.6, 2H), 6.72 (d, *J* = 16.0 Hz, 1H), 4.66-4.35 (m, 4H), 3.96-3.90 (m, 2H), 3.83-3.79 (m, 2H), 3.77 (s, 3H), 2.36 (s, 3H); ESI-MS(m/z): 628.0 [M+H]$^+$. |

(continued)

| Number | Name | Structure | $^1$H NMR and MS |
|---|---|---|---|
| **Example 220** | (*E*)-3-fluoro-*N*-(2-methoxy-5-(4-(4-(4-oxopent-2-enoyl) piperazin-1-yl)pyrido [3,2-*d*]pyrimidin-6-yl)pyridin-3-yl)pyridine-4-sulfonamide | 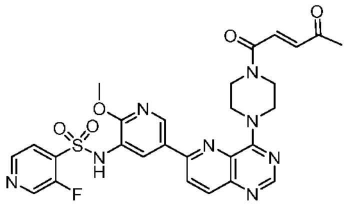 | $^1$H NMR (400 MHz, DMSO-$d_6$) δ 10.83 (brs, 1H), 8.80 (s, 1H), 8.71 (s, 1H), 8.60-8.52 (m, 2H), 8.42-8.31 (m, 2H), 8.20 (d, *J* = 9.2 Hz, 1H), 7.69-7.64 (m, 1H), 7.47 (d, *J* = 15.6 Hz, 1H), 6.72 (d, *J* = 16.0 Hz, 1H), 4.69-4.30 (m, 4H), 3.98-3.77 (m, 4H), 3.71 (s, 3H), 2.36 (s, 3H); ESI-MS(m/z): 593.0 [M+H]$^+$. |
| **Example 221** | (*E*)-2-fluoro-*N*-(2-methoxy-5-(4-(4-(4-oxopent-2-enoyl) piperazin-1-yl)pyrido [3,2-*d*]pyrimidin-6-yl)pyridin-3-yl)-4-(trifluoromethyl)benzenesulfonamide | 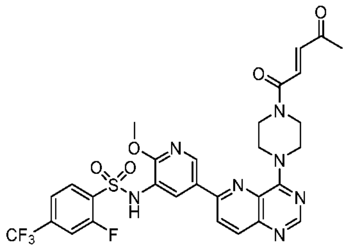 | $^1$H NMR (400 MHz, DMSO-$d_6$) δ 10.59 (s, 1H), 8.78-8.73 (m, 1H), 8.56 (s, 1H), 8.43-8.38 (m, 2H), 8.20 (d, J = 8.8 Hz, 1H), 7.98 (d, *J* = 10.0 Hz, 1H), 7.92 (t, *J* = 7.6 Hz, 1H), 7.73-7.69 (m, 1H), 7.46 (d, *J* = 15.6 Hz, 1H), 6.72 (d, *J* = 15.6 Hz, 1H), 4.50 (s, 4H), 3.96-3.90 (m, 2H), 3.85-3.80 (m, 2H), 3.69 (s, 3H), 2.35 (s, 3H); ESI-MS(m/z): 660.0 [M+H]$^+$. |
| **Example 222** | (*E*)-*N*-(2-methoxy-5-(4-(4-(4-oxopent-2-enoyl)piperazin-1-yl) pyrido [3,2-*d*]pyrimidin-6-yl)pyridin-3-yl)-2,4-dimethylthiazole-5-sulfonamide | 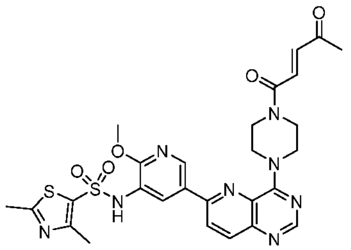 | $^1$H NMR (400 MHz, DMSO-$d_6$) δ 10.41 (s, 1H), 8.66 (s, 1H), 8.56 (s, 1H), 8.39-8.36 (m, 2H), 8.20 (d, *J* = 8.8 Hz, 1H), 7.47 (d, *J* = 16.0 Hz, 1H), 6.72 (d, *J* =15.6 Hz, 1H), 4.80-4.20 (m, 4H), 3.95-3.93 (m, 2H), 3.84-3.80 (m, 5H), 2.53 (s, 3H), 2.38 (s, 3H), 2.36 (s, 3H); ESI-MS (m/z): 609.0 [M+H]$^+$. |
| **Example 223** | (*E*)-*N*-(2-methoxy-5-(4-(4-(4-oxopent-2-enoyl)piperazin-1-yl) pyrido [3,2-*d*]pyrimidin-6-yl)pyridin-3-yl)-4-methylpyridine-3-sulfonamide | 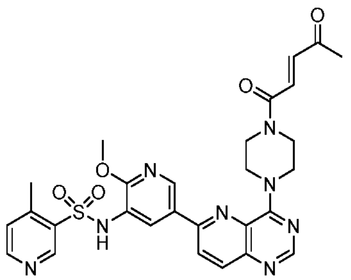 | $^1$H NMR (400 MHz, DMSO-$d_6$) δ 10.41 (s, 1H), 8.71 (s, 1H), 8.64 (s, 1H), 8.59-8.51 (m, 2H), 8.41-8.34 (m, 2H), 8.19 (d, *J* = 8.8 Hz, 1H), 7.49 (d, *J* = 15.6 Hz, 1H), 7.42 (d, *J* = 5.2 Hz, 1H), 6.73 (d, *J* = 15.6 Hz, 1H), 4.75-4.28 (m, 4H), 4.01-3.80 (m, 4H), 3.73 (s, 3H), 2.67 (s, 3H), 2.35 (s, 3H); ESI-MS(m/z): 589.0 [M+H]$^+$. |
| **Example 224** | (*E*)-3,5-dichloro-*N*-(2-methoxy-5-(4-(4-(4-oxopent-2-enoyl) piperazin-1-yl)pyrido [3,2-*d*]pyrimidin-6-yl)pyridin-3-yl)pyridine-4-sulfonamide | 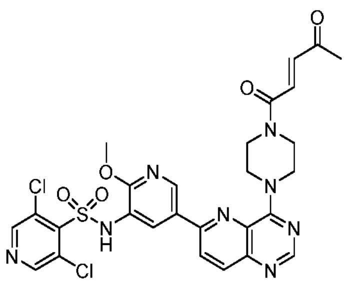 | $^1$H NMR (400 MHz, DMSO-$d_6$) δ 10.98 (brs, 1H), 8.73 (s, 2H), 8.69-8.62 (m, 1H), 8.56 (s, 1H), 8.36 (d, *J* = 8.8 Hz, 1H), 8.28 (d, *J* = 2.4 Hz, 1H), 8.20 (d, *J* = 8.8 Hz, 1H), 7.47 (d, *J* = 16.0 Hz, 1H), 6.73 (d, *J* = 15.6 Hz, 1H), 4.76-4.24 (m, 4H), 4.01-3.77 (m, 4H), 3.65 (s, 3H), 2.36 (s, 3H); ESI-MS(m/z): 643.0 [M+H]$^+$. |

(continued)

| Number | Name | Structure | $^1$H NMR and MS |
|---|---|---|---|
| **Example 225** | (*E*)-2-chloro-6-fluoro-*N*-(2-methoxy-5-(4-(4-(4-oxopent-2-enoyl) piperazin-1-yl)pyrido [3,2-d]pyrimidin-6-yl)pyridin-3-yl)benzenesulfona mide | 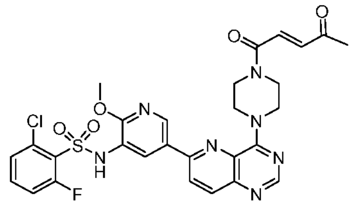 | $^1$H NMR (400 MHz, DMSO-$d_6$) δ 10.27 (brs, 1H), 8.57-8.49 (m, 2H), 8.34-8.25 (m, 2H), 8.18 (d, *J* = 8.8 Hz, 1H), 7.55-7.43 (m, 2H), 7.38 (d, J = 8.0 Hz, 1H), 7.33-7.24 (m, 1H), 6.72 (d, *J* = 16.0 Hz, 1H), 4.68-4.25 (m, 4H), 4.00-3.88 (m, 2H), 3.86-3.78 (m, 2H), 3.75 (s, 3H), 2.36 (s, 3H); ESI-MS(m/z): 626.0 [M+H]$^+$. |
| **Example 226** | (*E*)-2,4-difluoro-*N*-(5-(4-(4-(4-oxopent-2-enoyl)piperazin-1-yl) pyrido [3,2-*d*]pyrimidin-6-yl)-2-(trifluoromethoxy) pyridin-3-yl) benzenesulfona mide | 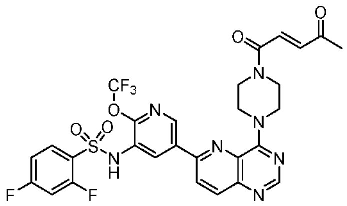 | $^1$H NMR (400 MHz, DMSO-$d_6$) δ 11.02 (br s, 1H), 8.76-8.50 (m, 3H), 8.48-8.35 (m, 1H), 8.24 (d, *J* = 8.8 Hz, 1H), 7.84-7.72 (m, 1H), 7.50-7.37 (m, 2H), 7.20-7.09 (m, 1H), 6.72 (d, *J* = 16.0 Hz, 1H), 4.70-4.35 (m, 4H), 4.00-3.90 (m, 2H), 3.86-3.77 (m, 2H), 2.35 (s, 3H); ESI-MS(m/z): 664.0 [M+H]$^+$. |
| **Example 227** | (*E*)-*N*-(2-ethoxy-5-(4-(4-(4-oxopent-2-enoyl)piperazin-1-yl) pyrido [3,2-*d*]pyrimidin-6-yl)pyridin-3-yl)-2-fluorobenzenesulfo namide | 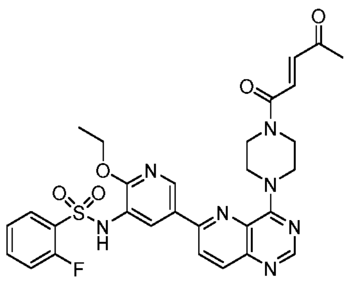 | $^1$H NMR (400 MHz, DMSO-$d_6$) δ 8.66-8.51 (m, 2H), 8.39-8.31 (m, 2H), 8.18 (d, *J* = 8.8 Hz, 1H), 7.76-7.67 (m, 1H), 7.65-7.55 (m, 1H), 7.47 (d, *J* = 16.0 Hz, 1H), 7.36 (t, *J* = 9.2 Hz, 1H), 7.25 (t, *J* = 7.6 Hz, 1H), 6.72 (d, *J* = 15.6 Hz, 1H), 4.51 (d, *J* = 39.8 Hz, 4H), 4.25-4.21 (m, 2H), 3.88 (d, *J* = 47.6 Hz, 4H), 2.36 (s, 3H), 1.18 (d, J = 6.8 Hz, 3H); ESI-MS(m/z): 606.0 [M+H]$^+$. |
| **Example 228** | (*R*,*E*)-2-fluoro-*N*-(2-methoxy-5-(4-(3-methyl-4-(4-oxopent-2-enoyl) piperazin-1-yl)pyrido[3,2-*d*]pyrimidin-6-yl)pyridin-3-yl)benzenesulfona mide | 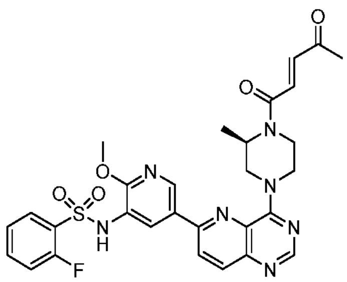 | $^1$H NMR (400 MHz, DMSO-$d_6$) δ 10.32 (s, 1H), 8.79-8.77 (m, 1H), 8.55 (s, 1H), 8.43-8.38 (m, 2H), 8.20 (d, *J* = 8.8 Hz, 1H), 7.74-7.66 (m, 2H), 7.48-7.40 (m, 2H), 7.31 (t, *J* = 8.0 Hz, 1H), 6.77-6.66 (m, 1H), 5.42 (s, 2H), 4.84-4.57 (m, 1H), 4.43-4.13 (m, 1H), 3.83-3.76 (m, 1H), 3.73 (s, 3H), 3.57-3.37 (m, 2H), 2.36-2.33 (m, 3H), 1.27-1.14 (m, 3H); ESI-MS(m/z): 606.0 [M+H]$^+$. |

(continued)

| Number | Name | Structure | $^1$H NMR and MS |
|---|---|---|---|
| **Example 229** | (*E*)-2-fluoro-*N*-(2-methoxy-5-(4-(4-(4-oxopent-2-enoyl) piperazin-1-yl)pyrido [3,2-*d*]pyrimidin-6-yl)pyridin-3-yl)-6-methylbenzenesulf onamide | 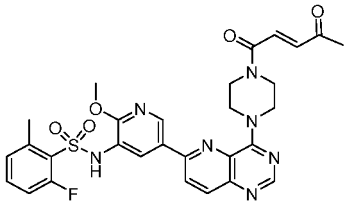 | $^1$H NMR (400 MHz, DMSO-$d_6$) δ 10.22 (s, 1H), 8.78 (d, *J* = 2.4 Hz, 1H), 8.57 (s, 1H), 8.45 (d, *J* = 2.0 Hz, 1H), 8.41 (d, *J* = 8.8 Hz, 1H), 8.22 (d, *J* = 8.8 Hz, 1H), 7.56-7.42 (m, 2H), 7.25-7.12 (m, 2H), 6.73 (d, *J* = 15.6 Hz, 1H), 4.51 (br s, 4H), 3.96-3.88 (m, 2H), 3.86-3.79 (m, 2H), 3.70 (s, 3H), 2.50 (s, 3H), 2.36 (s, 3H); ESI-MS(m/z): 606.0 [M+H]$^+$. |

**Example 230**

**Preparation of (*E*)-*N*-(2-methoxy-5-(4-(1-(4-oxopent-2-enoyl)piperidin-4-yl)pyrido[3,2-*d*]pyrimidin-6-yl)pyridin-3-yl)-2,4-dimethylthiazol-5-sulfonamide**

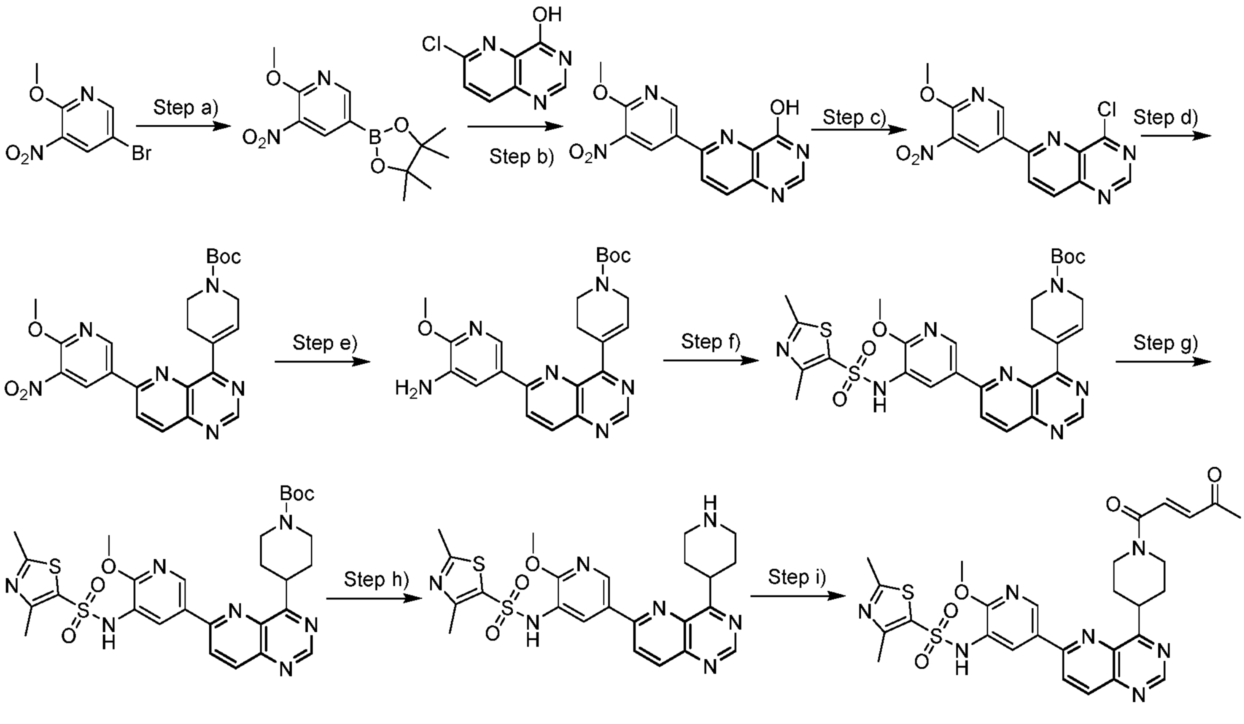


Step a): Preparation of 2-methoxy-3-nitro-5-(4,4,5,5-tetramethyl-1,3,2-dioxaborolan-2-yl)pyridine

5- Bromo-2-methoxy-3-nitropyridine (2.0 g, 8.583 mmol), pinacol diborate (2.6 g, 10.300 mmol), Pd(dppf)$Cl_2$ (628 mg, 0.858 mmol) and potassium acetate (2.5 g, 25.749 mmol) were added to 1,4-dioxane (30 mL). After the addition was completed, in the presense of protective nitrogen, the reaction mixture was heated to 110°C and reacted for 2 h. Upon completion of the reaction, the reaction was quenched by adding water (50 mL). The reaction mixture was extracted with ethyl acetate (150 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: petroleum ether/ethyl acetate=5/1 to 1/1). 2-Methoxy-3-nitro-5-(4,4,5,5-tetramethyl-1,3,2-dioxaborolan-2-yl)pyridine was obtained, yield: 91.7%; ESI-MS(m/z): 281.3 [M+H]$^+$.

Step b): Preparation of 6-(6-methoxy-5-nitropyridin-3-yl)pyrido[3,2-*d*]pyrimidin-4-ol

2-Methoxy-3-nitro-5-(4,4,5,5-tetramethyl-1,3,2-dioxaborolan-2-yl)pyridine (1.2 g, 4.284 mmol), 6-chloropyrido[3,2-d]pyrimidin-4-ol (856 mg, 4.712 mmol), Pd(dppf)$Cl_2$ (313 mg, 0.428 mmol) and cesium carbonate (4.2 g, 12.852 mmol) were added to dioxane/water mixed solvent (50 mL, v/v = 9: 1). After the addition was completed, in the presense of protective nitrogen, the reaction mixture was heated to 100°C, stirred and reacted for 3 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane=1/100 to 1/30). 6-(6-Methoxy-5-nitropyridin-3-yl)pyrido[3,2-*d*]pyrimidin-4-ol was obtained, yield: 62.5%; ESI-MS(m/z): 300.3 $[M+H]^+$.

Step c): Preparation of 4-chloro-6-(6-methoxy-5-nitropyfidin-3-yl)pyrido[3,2-d]pyrimidine

6-(6-Methoxy-5-nitropyridin-3-yl)pyrido[3,2-*d*]pyrimidin-4-ol (850 mg, 2.841 mmol) was added to thionyl chloride (30 mL), then with DMF (21 mg, 0.284 mmo) added. After the addition was completed, the reaction mixture was kept at 100°C and reacted overnight. Upon completion of the reaction, the reaction solution was concentrated under reduced pressure, under the ice bath condition, adjusted to pH = 7-8 with saturated aqueous sodium bicarbonate solution and extracted with dichloromethane (100 mL×3). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane=1/100 to 1/30). 4-Chloro-6-(6-methoxy-5-nitropyridin-3-yl)pyrido[3,2-*d*]pyrimidine was obtained, yield: 51.0%; ESI-MS(m/z): 318.3 $[M+H]^+$.

Step d): Preparation of tert-butyl 4-(6-(6-methoxy-5-nitropyridin-3-yl)pyrido[3,2-*d*]pyrimidin-4-yl)-3,6-dihydropyridine-1(2*H*)-carboxylate

4-Chloro-6-(6-methoxy-5-nitropyridin-3-yl)pyrido[3,2-d]pyrimidine (460 mg, 1.448 mmol), tert-butyl 4-(4,4,5,5-tetramethyl-1,3,2-dioxaborolan-2-yl)-3,6-dihydropyridine-1(2*H*)-carboxylate (537 mg, 1.738 mmol) and Pd(dppf)$Cl_2$ (106 mg, 0.145 mmol) were added to dioxane (10 mL), in the presense of protective nitrogen, cesium carbonate (1.4 g, 4.344 mmol) was dissolved in water (2mL) and slowly added to the reaction solution. After the addition was completed, the reaction mixture was stirred at room temperature and reacted for 6 h. Upon completion of the reaction, the reaction mixture was diluted by adding water (30 mL) and extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane=1/100 to 1/10). Tert-butyl 4-(6-(6-methoxy-5-nitropyridin-3-yl)pyrido[3,2-*d*]pyrimidin-4-yl)-3,6-dihydropyridine-1(2*H*)-carboxylate was obtained, yield: 89.2%; ESI-MS(m/z): 465.1 $[M+H]^+$.

Step e): Preparation of tert-butyl 4-(6-(5-amino-6-methoxypyridin-3-yl)pyrido[3,2-*d*]pyrimidin-4-y1)-3,6-dihydropyridine-1(2*H*)-carboxylate

Tert-butyl 4-(6-(6-methoxy-5-nitropyridin-3 -yl)pyrido[3,2-*d*]pyrimidin-4-yl)-3,6-dihydropyridine-1(2*H*)-carboxylate (600 mg, 1.292 mmol), iron powder (720 mg, 12.920 mmol) and ammonium chloride (69 mg, 1.292 mmol) were added to methanol and water mixed solvent (25 mL, v/v = 4:1). After the addition was completed, the reaction mixture was heated to 60°C, stirred and reacted for 4 h. Upon completion of the reaction, the reaction mixture was filtered. The filtrate was diluted by adding water (40 mL), extracted with dichloromethane (100 mL×3). The organic phases were combined, washed with saturated brine (50 mL), dried over anhydrous sodium sulfate and filtered. The filtrate was concentrated under reduced pressure. The crude product obtained was purified by silica gel column chromatography (Eluent: methanol/dichloromethane= 1/100 to 1/10). Tert-butyl 4-(6-(5-amino-6-methoxypyridin-3-yl)pyrido[3,2-*d*]pyrimidin-4-yl)-3,6-dihydropyridine-1(2*H*)-carboxylate was obtained, yield: 82.0%; ESI-MS(m/z): 435.3 $[M+H]^+$.

Step f): Preparation of tert-butyl 4-(6-(5-((2,4-dimethylthiazole)-5-sulfonamido)-6-methoxypyridin-3-yl)pyrido[3,2-*d*]pyrimidin-4-yl)-3,6-dihydropyridine-1(2*H*)-carboxylate

Tert-butyl 4-(6-(5-amino-6-methoxypyridin-3 -yl)pyrido [3,2-*d*]pyrimidin-4-yl)-3,6-dihydropyridine-1(2*H*)-carboxylate (430 mg, 0.990 mmol) was dissolved in pyridine (10 mL) and 2,4-dimethylthiazole-5-sulfonyl chloride (419 mg, 1.980 mmol) was added to the mixture. The reaction mixture was kept at room temperature and reacted for 4 h. Upon

completion of the reaction, the reaction was quenched by adding water (100 mL). The reaction mixture was extracted with ethyl acetate (100 mL×2). The organic phases were combined, washed with saturated brine (100 mL), dried over anhydrous sodium sulfate and filtered. The filtrate was concentrated under reduced pressure. The crude product obtained was purified by silica gel column chromatography (Eluent: dichloromethane/methanol = 20/1 to 10/1). Tert-butyl 4-(6-(5-((2,4-dimethylthiazole)-5-sulfonamido)-6-methoxypyridin-3-yl)pyrido[3,2-*d*]pyrimidin-4-yl)-3,6-dihydropyfidine-1(2*H*)-carboxylate was obtained, yield: 38.1%; ESI-MS(m/z): 610.3 $[M+H]^+$.

Step g): Preparation of tert-butyl 4-(6-(5-((2,4-dimethylthiazole)-5-sulfonamido)-6-methoxypyridin-3-yl)pyrido[3,2-*d*]pyrimidin-4-yl)piperazine-1-carboaylate

Tert-butyl 4-(6-(5-((2,4-dimethylthiazole)-5-sulfonamido)-6-methoaypyridin-3-yl)pyrido[3,2-*d*]pyrimidin-4-yl)-3,6-dihydropyridine-1(2*H*)-carboxylate (180 mg, 0.295 mmol), 10%Pd/C (100 mg), glacial acetic acid (18 mg, 0.295 mmol) and methanol (10 mL) were added to the reaction flask. Under hydrogen atmosphere, the reaction mixture was kept at 30°C and reacted for 6 h. Upon completion of the reaction, the reaction mixture was filtered. The filtrate was concentrated under reduced pressure. The crude product obtained was purified by silica gel column chromatography (Eluent: dichloromethane/methanol=20/1 to 10/1). Tert-butyl 4-(6-(5-((2,4-dimethylthiazole)-5 -sulfonamido)-6-methoxypyridin-3 -yl)pyrido [3,2-*d*]pyrimidin-4-yl)piperazine-1-carboxylate was obtained, yield: 55.2%; ESI-MS(m/z): 612.1 $[M+H]^+$.

Step h): Preparation of *N*-(2-methoxy-5-(4-(piperidin-4-yl)pyrido[3,2-*d*]pyrimidin-6-yl)pyridin-3-yl)-2,4-dimethylthiazol-5-sulfonamide trifluoroacetate

Tert-butyl 4-(6-(5-((2,4-Dimethylthiazole)-5-sulfonamido)-6-methoxypyridin-3-yl)pyrido[3,2-*d*]pyrimidin-4-yl)piperazine-1-carboxylate (100 mg, 0.163 mmol) was dissolved in dichloromethane (4 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. *N*-(2-methoxy-5-(4-(piperidin-4-yl)pyrido[3,2-*d*]pyrimidin-6-yl)pyridin-3-yl)-2,4-dimethylthiazol-5-sulfonamide trifluoroacetate was obtained; ESI-MS(m/z): 512.5 $[M+H]^+$.

Step i): Preparation of (*E*)-*N*-(2-methoxy-5-(4-(1-(4-oxopent-2-enoyl)piperidin-4-yl)pyrido[3,2-*d*]pyrimidin-6-yl)pyridin-3-yl)-2,4-dimethylthiazol-5-sulfonamide

*N*-(2-methoxy-5-(4-(piperidin-4-yl)pyrido[3,2-*d*]pyrimidin-6-yl)pyridin-3-yl)-2,4-dimethylthiazol-5-sulfonamide trifluoroacetate (100 mg, 0.160 mmol) and tetrahydrofuran (4 mL) were added to the reaction flask. The reaction system was cooled to -78°C, with DIPEA (103 mg, 0.800 mmol), (E)-4-oxopent-2-enoic acid (20 mg, 0.176 mmol) and 50% $T_3P$ ethyl acetate solution (204 mg, 0.320 mmol, 50%wt) added successively. After the addition was completed, the reaction mixture was kept at -78°C and stirred for 1 h. Upon completion of the reaction, the reaction was quenched using saturated aqueous ammonium chloride solution (50mL) at -78°C. The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). (E)-N-(2-methoxy-5-(4-(1-(4-oxopent-2-enoyl)piperidin-4-yl)pyrido[3,2-dJpyrimidin-6-yl)pyridin-3-yl)-2,4-dimethylthiazol-5-sulfonamide was obtained, yield: 17.2%; $^1H$ NMR (400 MHz, DMSO-$d_6$) δ 10.49 (brs, 1H), 9.27 (s, 1H), 8.89 (s, 1H), 8.68-8.58 (m, 2H), 8.47 (d, *J* = 8.8 Hz, 1H), 7.50 (d, *J* = 16.0 Hz, 1H), 6.70 (d, *J* = 15.6 Hz, 1H), 4.64 (d, *J* = 12.8 Hz, 1H), 4.57-4.46 (m, 1H), 4.30 (d, *J* = 13.6 Hz, 1H), 3.84 (s, 3H), 3.50-3.38 (m, 1H), 3.12-2.98 (m, 1H), 2.55 (s, 3H), 2.42 (s, 3H), 2.37 (s, 3H), 2.17-2.05 (m, 2H), 1.99-1.75 (m, 2H); ESI-MS(m/z): 608.0 $[M+H]^+$.

Referring to the preparation method of Example 230 and using the corresponding raw materials, the compounds in the following examples were obtained.

| Number | Name | Structure | $^1$H NMR and MS |
|---|---|---|---|
| **Example 231** | (*E*)-2,4,6-trifluoro-*N*-(2-methoxy-5-(4-(1-(4-oxopent-2-enoyl)piperidin-4-yl) pyrido [3,2-*d*] pyrimidin-6-yl)pyridin-3-yl)benzenesulfona mide | 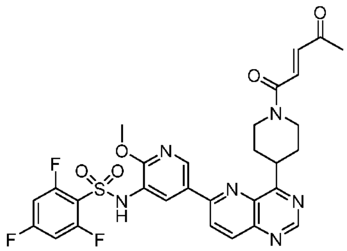 | $^1$H NMR (400 MHz, DMSO-$d_6$) δ 10.74 (brs, 1H), 9.25 (s, 1H), 8.81 -8.60 (m, 1H), 8.60-8.32 (m, 3H), 7.50 (d, *J* = 15.6 Hz, 1H), 7.40-7.04 (m, 2H), 6.70 (d, *J* = 15.6 Hz, 1H), 4.66-4.58 (m, 1H), 4.54-4.48 (m, 1H), 4.29-4.25 (m, 1H), 3.80 (s, 3H), 3.43 (t, *J* = 13.2 Hz, 1H), 3.05-2.97 (m, 1H), 2.37 (s, 3H), 2.12-2.03 (m, 2H), 1.93-1.76 (m, 2H); ESI-MS(m/z): 627.0 [M+H]$^+$. |
| **Example 232** | (*E*)-2-fluoro-*N*-(2-methoxy-5-(4-(1-(4-oxopent-2-enoyl) piperidin-4-yl)pyrido [3,2-*d*]pyrimidin-6-yl) pyridin-3-yl) benzenesulfona mide | 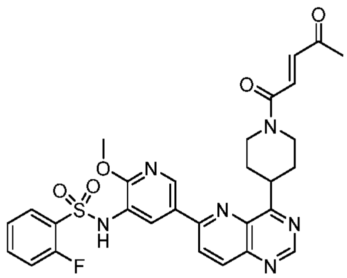 | $^1$H NMR (400 MHz, DMSO-$d_6$) δ 10.27 (brs, 1H), 9.27 (s, 1H), 8.81 (s, 1H), 8.65-8.35 (m, 3H), 7.76 (t, *J* = 7.6 Hz, 1H), 7.70-7.56 (m, 1H), 7.51 (d, *J* = 15.6 Hz, 1H), 7.39 (t, *J* = 9.6 Hz, 1H), 7.28 (t, *J* = 7.6 Hz, 1H), 6.70 (d, *J* = 16.0 Hz, 1H), 4.64 (d, *J* = 13.2 Hz, 1H), 4.48 (t, *J* = 11.6 Hz, 1H), 4.29 (d, *J* = 13.6 Hz, 1H), 3.77 (s, 3H), 3.42 (t, *J* = 12.8 Hz, 1H), 3.02 (t, *J* = 12.8 Hz, 1H), 2.37 (s, 3H), 2.16-2.01 (m, 2H), 2.00-1.88 (m, 1H), 1.85-1.72 (m, 1H); ESI-MS(m/z): 591.2 [M+H]$^+$. |
| **Example 233** | (*E*)-2,6-difluoro-*N*-(2-methoxy-5-(4-(1-(4-oxopent-2-enoyl) piperidin-4-yl)pyrido [3,2-*d*]pyrimidin-6-yl) pyridin-3-yl) benzenesulfona mide | 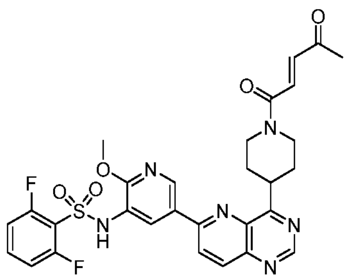 | $^1$H NMR (400 MHz, DMSO-$d_6$) δ 10.67 (s, 1H), 9.27 (s, 1H), 8.86 (s, 1H), 8.63-8.56 (m, 2H), 8.48 (d, *J* = 9.2 Hz, 1H), 7.68-7.59 (m, 1H), 7.50 (d, *J* = 15.6 Hz, 1H), 7.22 (t, *J* = 9.2 Hz, 2H), 6.70 (d, *J* = 15.6 Hz, 1H), 4.62 (d, *J* = 13.2 Hz, 1H), 4.54-4.45 (m, 1H), 4.28 (d, *J* = 13.2 Hz, 1H), 3.76 (s, 3H), 3.47-3.38 (m, 1H), 3.06-2.97 (m, 1H), 2.37 (s, 3H), 2.08 (d, *J* = 13.2 Hz, 2H), 1.97-1.88 (m, 1H),1.83-1.74(m, 1H); ESI-MS(m/z): 609.0 [M+H]$^+$. |
| **Example 234** | (*E*)-2,4-difluoro-*N*-(2-methoxy-5-(4-(1-(4-oxopent-2-enoyl) piperidin-4-yl)pyrido [3,2-*d*]pyrimidin-6-yl) pyridin-3-yl) benzenesulfona mide | 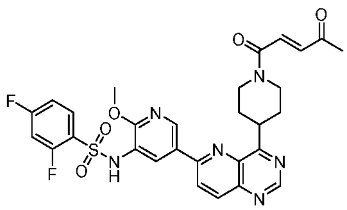 | $^1$H NMR (400 MHz, DMSO-$d_6$) δ 10.36 (s, 1H), 9.27 (s, 1H), 8.81 (s, 1H), 8.61-8.51 (m, 2H), 8.47 (d, *J* = 8.8 Hz, 1H), 7.86-7.74 (m, 1H), 7.55-7.42 (m, 2H), 7.22-7.12 (m, 1H), 6.70 (d, *J* = 15.6 Hz, 1H), 4.68-4.59 (m, 1H), 4.54-4.42 (m, 1H), 4.34-4.23 (m, 1H), 3.79 (s, 3H), 3.48-3.37 (m, 1H), 3.08-2.94 (m, 1H), 2.37 (s, 3H), 2.14-2.04 (m, 2H), 1.99-1.86 (m, 1H), 1.86-1.71 (m, 1H); ESI-MS(m/z): 609.0 [M+H]$^+$. |

(continued)

| Number | Name | Structure | $^1$H NMR and MS |
|---|---|---|---|
| **Example 235** | (*E*)-2-chloro-6-fluoro-*N*-(2-methoxy-5-(4-(1-(4-oxopent-2-enoyl)piperidin-4-yl) pyrido [3,2-*d*] pyrimidin-6-yl)pyridin-3-yl)benzenesulfonamide | 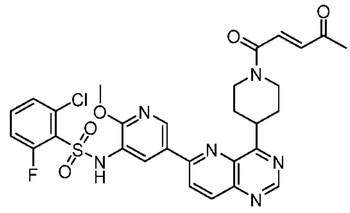 | $^1$H NMR (400 MHz, DMSO-$d_6$) δ 10.57 (s, 1H), 9.28 (s, 1H), 8.97 (d, *J* = 2.0 Hz, 1H), 8.67 (d, *J* = 2.4 Hz, 1H), 8.63 (d, *J* = 9.2 Hz, 1H), 8.50 (d, *J* = 8.8 Hz, 1H), 7.64 (dd, *J* = 8.0, 5.2 Hz, 1H), 7.57-7.46 (m, 2H), 7.46-7.27 (m, 1H), 6.71 (d, *J* = 16.0 Hz, 1H), 4.63 (d, *J* = 13.2 Hz, 1H), 4.55-4.41 (m, 1H), 4.28 (d, *J* = 13.6 Hz, 1H), 3.76 (s, 3H), 3.49-3.37 (m, 1H), 3.11-2.94 (m, 1H), 2.37 (s, 3H), 2.19-2.01 (m, 2H), 1.99-1.87 (m, 1H), 1.85-1.73 (m, 1H); ESI-MS(m/z): 625.0 [M+H]$^+$. |

**Example 236**

**Preparation of (*E*)-2,4,6-trifluoro-*N*-(2-methoxy-5-(4-(1-(4-oxopent-2-enoyl)-1,2,3,6-tetrahydropyridin-4-yl)pyrido[3,2-d]pyrimidin-6-yl)pyridin-3-yl)benzenesulfonamide**

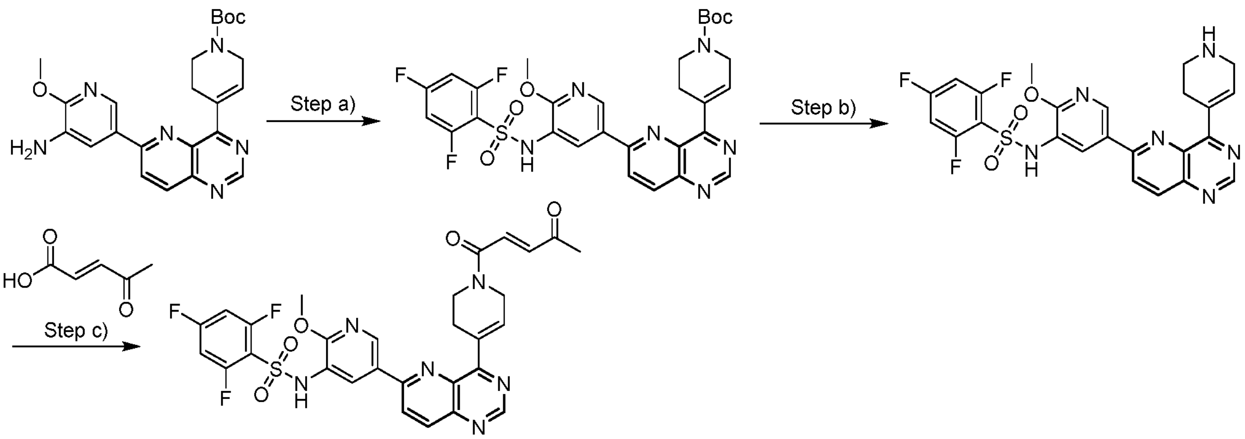


Step a): Preparation of tert-butyl 4-(6-(6-methoxy-5-((2,4,6-trifluorophenyl)sulfonamido)pyridin-3-yl)pyrido[3,2-*d*]pyrimidin-4-yl)-3,6-dihydropyridine-1(2*H*)-carboaylate

Tert-butyl 4-(6-(5-amino-6-methoxypyridin-3-yl)pyrido[3,2-*d*]pyrimidin-4-yl)-3,6-dihydropyridine-1(2*H*)-carboxylate (90 mg, 0.207 mmol) was added to pyridine (3 mL), then with 2,4,6-trifluorobenzenesulfonyl chloride (143 mg, 0.621 mmol) added. After the addition was completed, the reaction mixture was kept at room temperature and reacted for 2 h. Upon completion of the reaction, the reaction was quenched by adding water (30 mL). The reaction mixture was extracted with ethyl acetate (50 mL×2). The organic phases were combined, washed with saturated brine (50 mL×3), dried over anhydrous sodium sulfate and filtered. The filtrate was concentrated under reduced pressure. The crude product obtained was purified by silica gel column chromatography (Eluent: ethyl acetate/petroleum ether = 1/10 to 1/1). Tert-butyl 4-(6-(6-methoxy-5-((2,4,6-trifluorophenyl)sulfonamido)pyridin-3-yl)pyrido[3,2-*d*]pyrimidin-4-yl)-3,6-dihydropyridine-1(2*H*)-carboxylate was obtained, yield: 85.5%; ESI-MS(m/z): 629.2 [M+H]$^+$.

Step b): Preparation of 2,4,6-trifluoro-*N*-(2-methoxy-5-(4-(1,2,3,6-tetrahydropyridin-4-yl)pyrido[3,2-*d*]pyrimidin-6-yl)pyridin-3 -yl)benzenesulfonamide trifluoroacetate

Tert-butyl 4-(6-(6-methoxy-5-((2,4,6-trifluorophenyl)sulfonamido)pyridin-3-yl)pyrido[3,2-*d*]pyrimidin-4-yl)-3,6-dihydropyridine-1(2*H*)-carboxylate (100 mg, 0.159 mmol) was dissolved in dichloromethane (4 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2,4,6-Trifluoro-*N*-(2-methoxy-5-(4-(1,2,3,6-tetrahydropyridin-4-yl)pyrido[3,2-*d*]pyrimidin-6-yl)pyridin-3-yl)benzenesulfonamide trifluoroacetate was obtained, yield: 85.2%; ESI-MS(m/z): 529.1 $[M+H]^+$.

Step c): Preparation of (*E*)-2,4,6-trifluoro-*N*-(2-methoxy-5-(4-(1-(4-oxopent-2-enoyl)-1,2,3,6-tetrahydropyridin-4-yl)pyrido[3,2-*d*]pyrimidin-6-yl)pyridin-3-yl)benzenesulfonamide

2,4,6-trifluoro-*N*-(2-methoxy-5-(4-(1,2,3,6-tetrahydropyridin-4-yl)pyrido[3,2-*d*]pyrimidin-6-yl)pyridin-3-yl)benzenesulfonamide trifluoroacetate (60 mg, 0.093 mmol) and tetrahydrofuran (2 mL) were added to the reaction flask. The reaction system was cooled to -78°C, with DIPEA (72 mg, 0.558 mmol), (*E*)-4-oxopent-2-enoic acid (11 mg, 0.093 mmol) and 50% $T_3P$ ethyl acetate solution (118 mg, 0.186 mmol, 50%wt) added successively. After the addition was completed, the reaction mixture was kept at -78°C and stirred for 1 h. Upon completion of the reaction, the reaction solution was diluted by adding acetonitrile (1 mL), purified by Prep-HPLC (Method 2) and freeze dried. (*E*)-2,4,6-trifluoro-*N*-(2-methoxy-5-(4-(1-(4-oxopent-2-enoyl)-1,2,3,6-tetrahydropyridin-4-yl)pyrido[3,2-*d*]pyrimidin-6-yl)pyridin-3-yl)benzenesulfonamide was obtained, yield: 13.8%; $^1$H NMR (400 MHz, DMSO-$d_6$) δ 9.24 (d, *J* = 5.2 Hz, 1H), 8.40-8.36 (m, 2H), 8.28-8.25 (m, 1H), 7.97-7.88 (m, 1H), 7.87-7.79 (m, 1H), 7.56 (dd, $J_1$ = 36.4 Hz, $J_2$ = 16.0 Hz, 1H), 7.03-6.98 (m, 2H), 6.67 (dd, $J_1$ = 16.0 Hz, $J_2$ = 12.4 Hz, 1H), 4.71 (d, *J* = 3.6 Hz, 1H), 4.45 (d, *J* = 3.2 Hz, 1H), 3.92-3.83 (m, 2H), 3.82 (d, *J* = 1.6 Hz, 3H), 3.10-3.00 (m, 1H), 2.93-2.82 (m, 1H), 2.39 (d, *J* = 9.6 Hz, 3H); ESI-MS(m/z): 625.0 $[M+H]^+$.

Referring to the preparation method of Example 236 and using the corresponding raw materials, the compounds in the following examples were obtained.

| Number | Name | Structure | $^1$H NMR and MS |
|---|---|---|---|
| **Example 237** | (*E*)-*N*-(2-methoxy-5-(4-(1-(4-oxopent-2-enoyl)-1,2,3,6-tetrahydropyridin-4-yl) pyrido [3,2-*d*]pyrimidin-6-yl)pyridin-3-yl)-2,4-dimethylthiazole-5-sulfonamide | 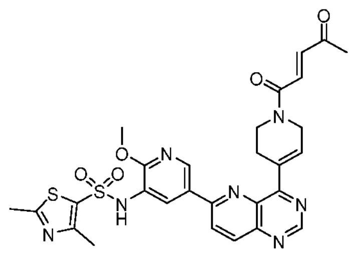 | $^1$H NMR (400 MHz, DMSO-$d_6$) δ 10.49 (brs, 1H),, 9.32-9.25 (m, 1H), 8.86-8.77 (m, 1H), 8.65-8.57 (m, 2H), 8.50-8.44 (m, 1H), 7.79-7.74 (m, 1H), 7.60-7.44 (m, 1H), 6.75-6.65 (m, 1H), 4.69-4.62 (m, 1H), 4.53-4.46 (m, 1H), 3.96-3.87 (m, 2H), 3.84 (s, 3H), 3.07 (s, 1H), 2.94 (s, 1H), 2.56-2.52 (m, 3H), 2.42-2.35 (m, 6H); ESI-MS (m/z): 606.0 $[M+H]^+$. |
| **Example 238** | (*E*)-2-fluoro-*N*-(2-methoxy-5-(4-(1-(4-oxopent-2-enoyl)-1,2,3,6-tetrahydropyridin-4-yl) pyrido [3,2-*d*]pyrimidin-6-yl)pyridin-3-yl) benzenesulfona mide | 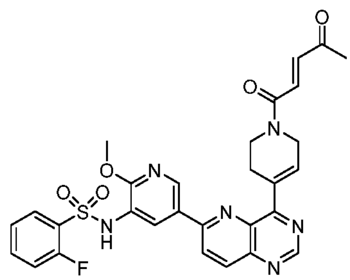 | $^1$H NMR (400 MHz, DMSO-$d_6$) δ 9.24 (d, *J* = 3.2 Hz, 1H), 8.48-8.46 (m, 1H), 8.39-8.36 (m, 2H), 8.22 (s, 1H), 7.81-7.71 (m, 2H), 7.55 (dd, $J_1$ = 39.2, $J_2$ = 15.6 Hz, 1H), 7.43-7.34 (m, 1H), 7.20-7.08 (m, 2H), 6.67 (dd, $J_1$ = 9.2 Hz, $J_2$ = 7.2 Hz, 1H), 4.72 (d, *J* = 3.6 Hz, 1H), 4.48 (d, *J* = 3.2 Hz, 1H), 3.92-3.86 (m, 2H), 3.83 (d, *J* = 4.0 Hz, 3H), 3.12-3.06 (m, 1H), 2.94-2.88 (m, 1H), 2.38 (d, *J* = 9.6 Hz, 3H); ESI-MS(m/z): 589.0 $[M+H]^+$. |

(continued)

| Number | Name | Structure | $^1$H NMR and MS |
|---|---|---|---|
| **Example 239** | (*E*)-2,6-difluoro-*N*-(2-methoxy-5-(4-(1-(4-oxopent-2-enoyl)-1,2,3,6-tetrahydropyridin-4-yl) pyrido [3,2-*d*]pyrimidin-6-yl)pyridin-3-yl) benzenesulfona mide | 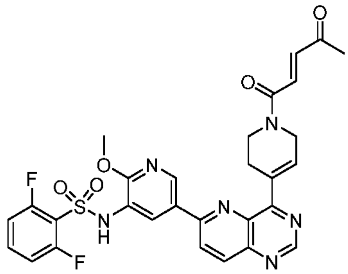 | $^1$H NMR (400 MHz, DMSO-$d_6$) δ 10.59 (s, 1H), 9.28 (s, 1H), 8.66 (d, *J* = 20.8 Hz, 1H), 8.57-8.49 (m, 2H), 8.47-8.42 (m, 1H), 7.83-7.76 (m, 1H), 7.60-7.45 (m, 2H), 7.18-7.08 (m, 2H), 6.70 (dd, $J_1$ = 15.6 Hz, $J_2$ = 7.2 Hz, 1H), 4.69-4.63 (m, 1H), 4.49-4.44 (m, 1H), 3.93-3.84 (m, 2H), 3.79-3.76 (m, 3H), 3.04 (s, 1H), 2.90 (s, 1H), 2.38 (d, *J* = 11.2 Hz, 3H); ESI-MS(m/z): 607.0 $[M+H]^+$. |
| **Example 240** | (*E*)-2,4-difluoro-*N*-(2-methoxy-5-(4-(1-(4-oxopent-2-enoyl)-1,2,3,6-tetrahydropyridin-4-yl) pyrido [3,2-*d*]pyrimidin-6-yl)pyridin-3-yl) benzenesulfona mide | 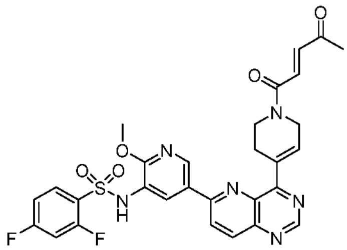 | $^1$H NMR (400 MHz, DMSO-$d_6$) δ 10.43 (s, 1H), 9.26 (d, *J* = 2.0 Hz, 1H), 8.56-8.35 (m, 4H), 7.85-7.70 (m, 2H), 7.54 (d, *J* = 16.0 Hz, 1H), 7.29 (s, 1H), 7.04 (d, *J* = 8.0 Hz, 1H), 6.68 (d, *J* = 16.0 Hz, 1H), 4.70 (s, 1H), 4.48 (d, *J* = 3.6 Hz, 1H), 3.95-3.84 (m, 2H), 3.82 (d, *J* = 3.2 Hz, 3H), 3.07 (s, 1H), 2.91 (s, 1H), 2.39 (d, *J* = 11.2 Hz, 3H); ESI-MS(m/z): 607.1 $[M+H]^+$. |
| **Example 241** | (*E*)-2-chloro-6-fluoro-*N*-(2-methoxy-5-(4-(1-(4-oxopent-2-enoyl)-1,2,3,6-tetrahydropyridin-4-yl) pyrido [3,2-*d*]pyrimidin-6-yl)pyridin-3-yl) benzenesulfona mide | 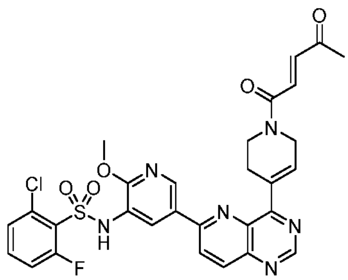 | $^1$H NMR (400 MHz, DMSO-$d_6$) δ 10.54 (br s, 1H), 9.34-9.26 (m, 1H), 8.90-8.82 (m, 1H), 8.65-8.57 (m, 2H), 8.48 (d, *J* = 9.2 Hz, 1H), 7.79-7.70 (m, 1H), 7.65-7.42 (m, 3H), 7.41-7.32 (m, 1H), 6.77-6.68 (m, 1H), 4.67-4.44 (m, 2H), 3.96-3.85 (m, 2H), 3.76 (s, 3H), 3.09-2.89 (m, 2H), 2.37 (d, *J* = 12.4 Hz, 3H); ESI-MS(m/z): 623.0 $[M+H]^+$. |

**Example 242**

**Preparation of (*E*)-2,4-difluoro-*N*-(2-methoxy-5-(7-methyl-4-(4-(4-oxopent-2-enoyl)piperazin-1-yl)quinazolin-6-yl)pyridin-3-yl)benzenesulfonamide**

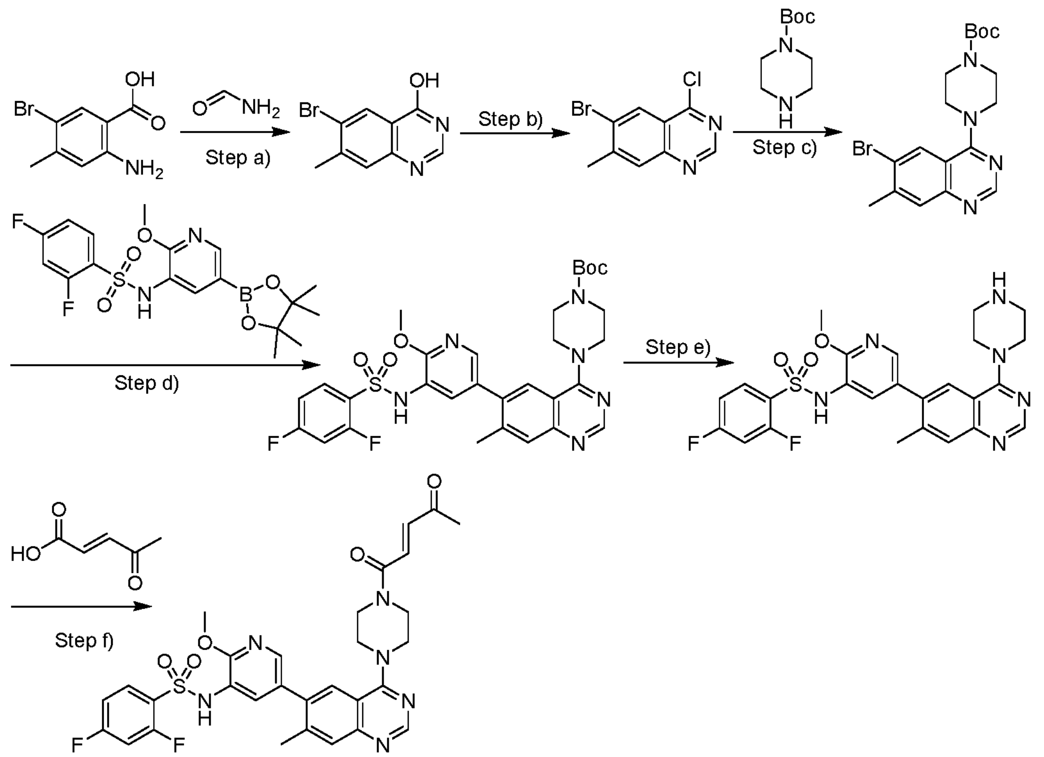


Step a): Preparation of 6-bromo-7-methylquinazolin-4-ol

2-Amino-5-bromo-4-methylbenzoic acid (1 g, 4.347 mmol) and formamide (8 mL) were added to the reaction flask. After the addition was completed, in the presense of protective nitrogen, the reaction mixture was heated to 180°C, stirred and reacted for 8 h. Upon completion of the reaction, the reaction mixture was cooled to room temperature, with reaction quenched by adding water (30 mL). The mixture was stirred for another 0.5 h at room temperature and filtered. The filter cake was dried to obtain 6-bromo-7-methylquinazolin-4-ol, yield: 79.1%; ESI-MS (m/z): 241.1 $[M+H]^+$.

Step b): Preparation of 6-bromo-4-chloro-7-methylquinazoline

6-Bromo-7-methylquinazolin-4-ol (823 mg, 3.443 mmol), thionyl chloride (10 mL) and *N*,*N*-dimethylformamide (0.1 mL) were added to the reaction flask. After the addition was completed, the reaction mixture was heated to 110°C, stirred and reacted for 2 h. Upon completion of the reaction, the reaction mixture was cooled to room temperature, concentrated under reduced pressure to remove thionyl chloride. The crude product was dissolved with dichloromethane (20 mL), with reaction quenched by adding iced water (50 mL), adjusted to pH=7-8 with saturated sodium bicarbonate solution and extracted with dichloromethane (50 mL × 3). The organic phases were combined, dried over anhydrous sodium sulfate and filtered. The filtrate was concentrated under reduced pressure. 6-Bromo-4-chloro-7-methylquinazoline was obtained, The product can be used directly in the next reaction without further purification; ESI-MS (m/z): 259.0 $[M+H]^+$.

Step c): Preparation of tert-butyl 4-(6-bromo-7-methylquinazolin-4-yl)piperazine-1-carboxylate

6-Bromo-4-chloro-7-methylquinazoline (850 mg, 3.301 mmol), tert-butyl piperazine-1-carboxylate (922 mg, 4.952 mmol), *N*,*N*-diisopropylethylamine (1.28 g, 9.903 mmol) and *N*,*N*-dimethylformamide (10 mL) were added to the reaction flask. After the addition was completed, the reaction mixture was heated to 50°C, stirred and reacted for 1 h. Upon completion of the reaction. The reaction was quenched by adding water (50 mL) and the reaction mixture was extracted with ethyl acetate (50 mL×3). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 10/1 to 4/1). Tert-butyl 4-(6-bromo-7-methylquinazolin-4-yl)piperazine-1-carboxylate was obtained, yield: 56.5%; ESI-MS (m/z): 407.1 $[M+H]^+$.

Step d): Preparation of tert-butyl 4-(6-(5-((2,4-difluorophenyl)sulfonamido)-6-methoxypyridin-3-yl)-7-methylquinazolin-4-yl)piperazine-1-carboxylate

Tert-butyl 4-(6-bromo-7-methylquinazolin-4-yl)piperazine-1-carboxylate (150 mg, 0.368 mmol), 2,4-difluoro-N-(2-methoxy-5-(4,4,5,5-tetramethyl-1,3,2-dioxaborolan-2-yl)pyridin-3-yl)benzenesulfonamide (188 mg, 0.442 mmol), Pd(dppf)$Cl_2$ (54 mg, 0.074 mmol), cesium carbonate (240 mg, 0.736 mmol), 1,4-dioxane (5 mL) and water (0.5 mL) were added to the reaction flask. After the addition was completed, in the presense of protective nitrogen, the reaction mixture was heated to 110°C and stirred for 3h. Upon completion of the reaction, the reaction mixture was cooled to room temperature and filtered with diatomaceous earth. The filtrate was concentrated under reduced pressure. The crude product obtained was purified by silica gel column chromatography (Eluent: petroleum ether/ethyl acetate = 5/1 to 1/1). Tert-butyl 4-(6-(5-((2,4-difluorophenyl)sulfonamido)-6-methoxypyridin-3-yl)-7-methylquinazolin-4-yl)piperazine-1-carboxylate was obtained, yield: 73.3%; ESI-MS (m/z): 627.3 $[M+H]^+$.

Step e): Preparation of 2,4-difluoro-*N*-(2-methoxy-5-(7-methyl-4-(piperazin-1-yl)quinazolin-6-yl)pyridin-3-yl)benzenesulfonamide trifluoroacetate

Tert-butyl 4-(6-(5-((2,4-difluorophenyl)sulfonamido)-6-methoxypyridin-3-yl)-7-methylquinazolin-4-yl)piperazine-1-carboxylate (170 mg, 0.271 mmol), trifluoroacetic acid (1.5 mL) and dichloromethane (5 mL) were added to the reaction flask. The reaction mixture was kept at room temperature and stirred for 1 h. Upon completion of the reaction, the reaction mixture was concentrated under reduced pressure. The crude product was slurried by adding methyl tert-butyl ether (5 mL), filtered and dried to obtain 2,4-difluoro-*N*-(2-methoxy-5-(7-methyl-4-(piperazin-1-yl)quinazolin-6-yl)pyridin-3-yl)benzenesulfonamide trifluoroacetate, yield: 92.5%; ESI-MS (m/z): 527.2 $[M+H]^+$. Step f): Preparation of (*E*)-2,4-difluoro-*N*-(2-methoxy-5-(7-methyl-4-(4-(4-oxopent-2-enoyl)piperazin-1-yl)quinazolin-6-yl)pyridin-3-yl)benzenesulfonamide 2,4-Difluoro-*N*-(2-methoxy-5-(7-methyl-4-(piperazin-1-yl)quinazolin-6-yl)pyridin-3-yl)benzenesulfonamide trifluoroacetate (110 mg, 0.172 mmol), (E)-4-oxopent-2-enoic acid (22 mg, 0.189 mmol) and tetrahydrofuran (2 mL) were added to the reaction flask. The reaction system was cooled to -78 to , with DIPEA (156 mg, 1.204 mmol) and 50% $T_3P$ ethyl acetate solution (164 mg, 0.258 mmol, 50%wt) added successively. After the addition was completed, the reaction mixture was kept at -78°C and stirred for 0.5 h. Upon completion of the reaction, the reaction was quenched by adding water (1 mL) into the system. The system was concentrated under reduced pressure at room temperature. The residue was diluted by adding acetonitrile (1 mL) and purified by Prep-HPLC (Method 2). (E)-2,4-difluoro-N-(2-methoay-5-(7-methyl-4-(4-(4-oxopent-2-enoyl)piperazin-1-yl)quinazolin-6-yl)pyridin-3-yl)benzenesulfonamide was obtained, yield: 58.9%; $^1H$ NMR (400 MHz, DMSO-$d_6$) δ 10.30 (s, 1H), 8.63 (s, 1H), 8.02 (s, 1H), 7.81-7.73 (m, 3H), 7.62 (s, 1H), 7.52 (t, *J* = 10.0 Hz, 1H), 7.43 (d, *J* = 15.6 Hz, 1H), 7.24-7.17 (m, 1H), 6.71 (d, *J* = 16.0 Hz, 1H), 3.87-3.81 (m, 6H), 3.77-3.73 (m, 2H), 3.71 (s, 3H), 2.36 (s, 3H), 2.32 (s, 3H); ESI-MS(m/z): 623.0 $[M+H]^+$.

**Example 243**

**Preparation of (*E*)-2,4-difluoro-*N*-(2-methoxy-5-(4-(4-(4-oxopent-2-enoyl)piperazin-1-yl)-8-(trifluoromethyl)quinazolin-6-yl)pyridin-3-yl)benzenesulfonamide**

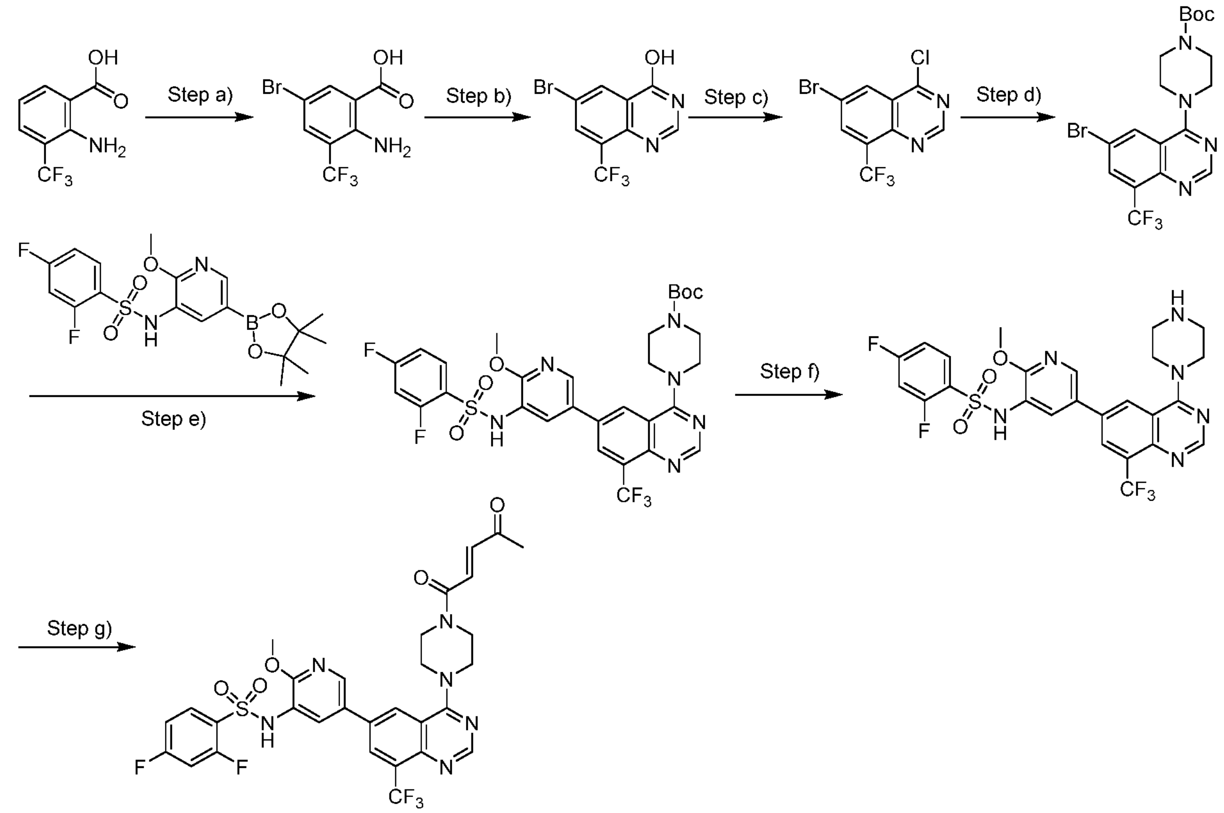


Step a): Preparation of 2-amino-5-bromo-3-(trifluoromethyl)benzoic acid

2-Amino-3-(trifluoromethyl)benzoic acid (2.0 g, 9.749 mmol), *N*,*N*-dimethylformamide (20 mL) were added to the reaction flask. In the presense of protective nitrogen, *N*-bromosuccinimide (1.7 g, 9.749 mmol) was slowly added to the system. After the addition was completed, the reaction mixture was kept at room temperature and reacted for 3 h. Upon completion of the reaction, the reaction solution was poured into water and filtered after a large amount of solid precipitationappeared. The filter cake was washed by water (50 mL×2) and dried. 2-Amino-5-bromo-3-(trifluoromethyl)benzoic acid was obtained, yield: 79.4%; ESI-MS(m/z): 282.0 $[M-H]^-$.

Step b): Preparation of 6-bromo-8-(trifluoromethyl)quinazolin-4-ol

2-Amino-5-bromo-3-(trifluoromethyl)benzoic acid (2.2 g, 7.746 mmol), formamide (20 mL) were added to the reaction flask. The reaction mixture was placed in microwave reactor, heated to 180°C and reacted for 1 h. Upon completion of the reaction, the reaction mixture was cooled to room temperature and filtered after solid precipitation appeared. The filter cake was washed by water (10mL×3) and dried. 6-Bromo-8-(trifluoromethyl)quinazolin-4-ol was obtained, yield: 83.7%; ESI-MS(m/z): 292.9 $[M+H]^+$.

Step c): Preparation of 6-bromo-4-chloro-8-(trifluoromethyl)quinazoline

6-Bromo-8-(trifluoromethyl)quinazolin-4-ol (1.9 g, 6.483 mmol), thionyl chloride (40mL), *N,N*-dimethylformamide (24 mg, 0.324 mmol) were added to the reaction flask. After the addition was completed, the reaction mixture was heated to 100°C and reacted for 2 h. Upon completion of the reaction, the reaction mixture was concentrated under reduced pressure. 6-Bromo-4-chloro-8-(trifluoromethyl)quinazoline was obtained, yield: 99.0%; ESI-MS(m/z): 311.0 $[M+H]^+$.

Step d): Preparation of tert-butyl 4-(6-bromo-8-(trifluoromethyl)quinazolin-4-yl)piperazine-1-carboxylate

6-Bromo-4-chloro-8-(trifluoromethyl)quinazoline (2 g, 6.421 mmol), *N*,*N*-dimethylformamide (20 mL), DIPEA (2.5 g, 19.263 mmol) and tert-butyl piperazine-1-carboxylate (1.3 g, 7.063 mmol) were added to the reaction flask. After the addition was completed, the reaction mixture was heated to 40°C and reacted for 3 h. Upon completion of the reaction, the reaction was quenched by adding water (50 mL) and the reaction mixture was extracted with ethyl acetate (150

mL×2). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate = 10/1 to 2/1). Tert-butyl 4-(6-bromo-8-(trifluoromethyl)quinazolin-4-yl)piperazine-1-carboxylate was obtained, yield: 94.6%; ESI-MS(m/z): 461.2 $[M+H]^+$.

Step e): Preparation of tert-butyl 4-(6-(5-((2,4-difluorophenyl)sulfonamido)-6-methoxypyridin-3-yl)-8-(trifluoromethyl)quinazolin-4-yl)piperazine-1-carboxylate

Tert-butyl 4-(6-bromo-8-(trifluoromethyl)quinazolin-4-yl)piperazine-1-carboxylate (200 mg, 0.434 mmol), 2,4-difluoro-*N*-(2-methoxy-5-(4,4,5,5-tetramethyl-1,3,2-dioxaborolan-2-yl)pyridin-3-yl)benzenesulfonamide (222 mg, 0.521 mmol), Pd(dppf)$Cl_2$ (31 mg, 0.043 mmol) cesium carbonate (424 mg, 1.302 mmol) and dioxane/water mixed solvent (10 mL, v/v = 4:1) were added to the reaction flask. After the addition was completed, in the presense of protective nitrogen, the reaction mixture was heated to 100°C, stirred and reacted for 3 h. Upon completion of the reaction, the reaction was quenched by adding water (30 mL). The reaction mixture was extracted with ethyl acetate (50 mL×2). The organic phases were combined, washed with saturated brine (30 mL), dried over anhydrous sodium sulfate and filtered. The filtrate was concentrated under reduced pressure. The crude product obtained was purified by silica gel column chromatography (Eluent: methanol/dichloromethane = 1/100 to 1/10). Tert-butyl 4-(6-(5-((2,4-difluorophenyl)sulfonamido)-6-methoxypyridin-3-yl)-8-(trifluoromethyl)quinazolin-4-yl)piperazine-1-carboxylate was obtained, yield: 67.8%; ESI-MS(m/z): 681.5 $[M+H]^+$.

Step f): Preparation of 2,4-difluoro-N (2-methoay-5-(4-(piperazin-1-yl)-8-(trifluoromethyl)quinazolin-6-yl)pyridin-3 -yl)benzenesulfonamide trifluoroacetate

Tert-butyl 4-(6-(5-((2,4-difluorophenyl)sulfonamido)-6-methoxypyridin-3-yl)-8-(trifluoromethyl)quinazolin-4-yl)piperazine-1-carboxylate (200 mg, 0.294 mmol) and dichloromethane (4 mL) were added to the reaction flask. Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure, then with methyl tert-butyl ether (10 mL) added, stirred at room temperature for 0.5 h and filtered after solid precipitation appeared. 2,4-Difluoro-*N*-(2-methoxy-5-(4-(piperazin-1-yl)-8-(trifluoromethyl)quinazolin-6-yl)pyridin-3-yl)benzenesulfonamide trifluoroacetate was obtained; ESI-MS (m/z): 581.2 $[M+H]^+$.

Step g): Preparation of (*E*)-2,4-difluoro-*N*-(2-methoxy-5-(4-(4-(4-oxopent-2-enoyl)piperazin-1-yl)-8-(trifluoromethyl)quinazolin-6-yl)pyridin-3-yl)benzenesulfonamide

2,4-Difluoro-*N*-(2-methoxy-5-(4-(piperazin-1-yl)-8-(trifluoromethyl)quinazolin-6-yl)pyridin-3-yl)benzenesulfonamide trifluoroacetate (150 mg, 0.162 mmol) and tetrahydrofuran (6 mL) were added to the reaction flask. The reaction system was cooled to -78°C, with DIPEA (105 mg, 0.810 mmol), (E)-4-oxopent-2-enoic acid (20 mg, 0.178 mmol) and 50% $T_3P$ ethyl acetate solution (206 mg, 0.324 mmol, 50%wt) added successively. After the addition was completed, the reaction mixture was kept at -78°C and stirred for 1 h. Upon completion of the reaction, at -78°C, the reaction was quenched using saturated aqueous ammonium chloride solution (50mL). The reaction mixture was extracted by adding dichloromethane (100 mL×2). The organic phases were combined, washed with saturated brine (100 mL), dried over anhydrous sodium sulfate and filtered. The filtrate was concentrated under reduced pressure. The crude product obtained was purified by Prep-HPLC (Method 2). (E)-2,4-difluoro-N-(2-methoxy-5-(4-(4-(4-oxopent-2-enoyl)piperazin-1-yl)-8-(trifluoromethyl)quinazolin-6-yl)pyridin-3-yl)benzenesulfonamide was obtained, yield: 38.9%; $^1$H NMR (400 MHz, DMSO-$d_6$) δ 10.31 (s, 1H), 8.73 (s, 1H), 8.45-8.26 (m, 3H), 7.97 (s, 1H), 7.85-7.70 (m, 1H), 7.61-7.37 (m, 2H), 7.26-7.08 (m, 1H), 6.74 (d, *J* = 16.0 Hz, 1H), 4.15-3.75 (m, 8H), 3.70 (s, 3H), 2.37 (s, 3H); ESI-MS(m/z): 677.0 $[M+H]^+$.

**Example 244**

**Preparation of (*E*)-2,4-difluoro-*N*-(1-(methyl-$d_3$)-2-oxo-5-(4-(4-(4-oxopent-2-enoyl)piperazin-1-yl)quinazolin-6-yl)-1,2-dihydropyridin-3-yl)benzenesulfonamide**

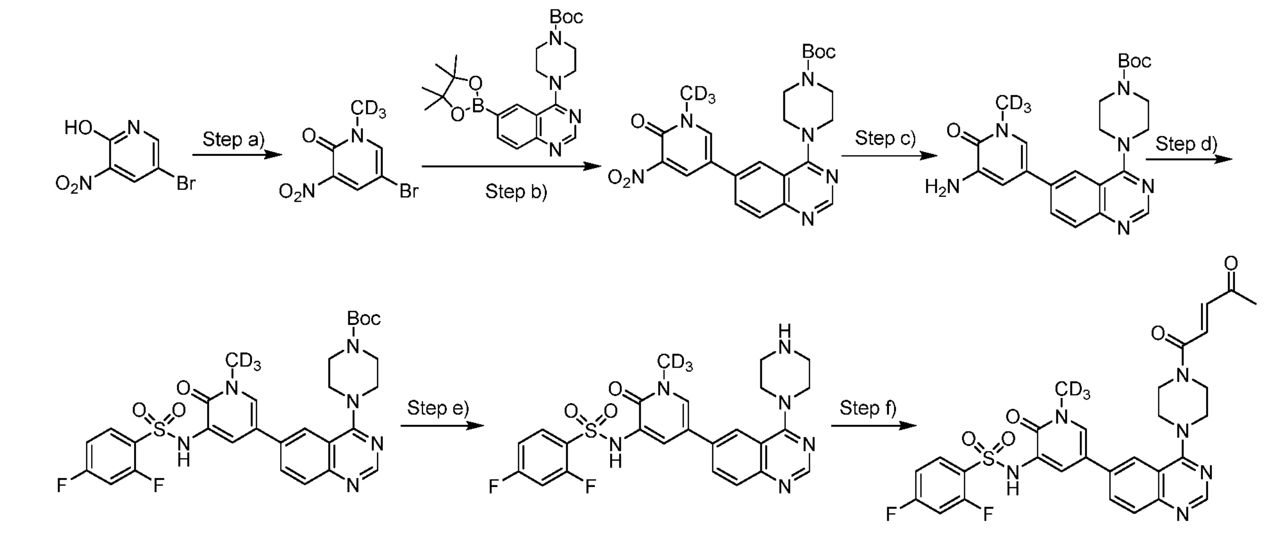


Step a): Preparation of 5-bromo-1-(methyl- *d*$_3$)-3-nitropyridin-2(1*H*)-one

Sodium hydride (73 mg, 1.827 mmol) and *N*,*N*-dimethylformamide (6 mL) were added to the reaction flask. The reaction mixture was cooled to 0°C, with 5-bromo-3-nitropyridin-2-ol (200 mg, 0.913 mmol) added dropwise. After the addition was completed, the reaction mixture was kept at 0°C, stirred and reacted for 30 min, with deuterated methyl iodide (265 mg, 1.827 mmol) added dropwise. After the addition was completed, the reaction mixture was stirred at room temperature and reacted for 2 h. Upon completion of the reaction, the reaction was quenched by adding water (3 mL). The reaction mixture was extracted with ethyl acetate (10 mL×3). The organic phases were combined, washed with water (5 mL×3), dried over anhydrous sodium sulfate and filtered. The filtrate was concentrated under reduced pressure. The crude product obtained was purified by silica gel column chromatography (Eluent: petroleum ether/ethyl acetate = 10/1 to 3/1). 5-Bromo-1-(methyl- *d*$_3$)-3-nitropyridin-2(1*H*)-one was obtained, yield: 41.6%; ESI-MS(m/z): 236.1 $[M+H]^+$.

Step b): Preparation of tert-butyl 4-(6-(1-(methyl-d$_3$)-5-nitro-6-oxo-1,6-dihydropyridin-3-yl)quinazolin-4-yl)piperazine-1-carboxylate

5-Bromo-1-(methyl-*d*$_3$)-3-nitropyridin-2(1*H*)-one (100 mg, 0.424 mmol), tert-butyl 4-(6-(4,4,5,5-tetramethyl-1,3,2-dioxaborolan-2-yl)quinazolin-4-yl)piperazine-1-carboxylate (187 mg, 0.424 mmol), Pd(dppf)$Cl_2$ (62 mg, 0.085 mmol), cesium carbonate (276 mg, 0.848 mmol) and dioxane/water mixed solvent (3.3 mL, v/v = 10:1) were added to the reaction flask. After the addition was completed, in the presense of protective nitrogen, the reaction mixture was heated to 100°C, stirred and reacted for 3 h. Upon completion of the reaction, the reaction was quenched by adding water (3 mL) and the reaction mixture was extracted with dichloromethane (10 mL×3). The organic phases were combined, dried over anhydrous sodium sulfate and filtered. The filtrate was concentrated under reduced pressure. The crude product obtained was purified by silica gel column chromatography (Eluent: dichloromethane/methanol = 1/0 to 10/1). Tert-butyl 4-(6-(1-(methyl-*d*$_3$)-5-nitro-6-oxo-1,6-dihydropyridin-3-yl)quinazolin-4-yl)piperazine-1-carboxylate was obtained, yield: 74.1%; ESI-MS(m/z): 470.3 $[M+H]^+$.

Step c): Preparation of tert-butyl 4-(6-(5-amino-1-(methyl-d$_3$)-6-oxo-1,6-dihydropyridin-3-yl)quinazolin-4-yl)piperazine-1-carboxylate

Tert-butyl 4-(6-(1-(methyl-*d*$_3$)-5-nitro-6-oxo-1,6-dihydropyridin-3-yl)quinazolin-4-yl)piperazine-1-carboxylate (100 mg, 0.213 mmol), iron powder (59 mg, 1.065 mmol), ammonium chloride (2 mg, 0.043 mmol) and ethanol/water mixed solvent (4 mL, v/v = 3:1) were added to the reaction flask. After the addition was completed, in the presense of protective nitrogen, the reaction mixture was heated to 80°C and stirred for 3 h. Upon completion of the reaction, the reaction mixture was filtered. The filtrate was concentrated under reduced pressure. The residue was diluted by adding water (3 mL) and extracted with dichloromethane (5 mL×3). The organic phases were combined, dried over anhydrous sodium sulfate and filtered. The filtrate was concentrated under reduced pressure. Tert-butyl 4-(6-(5-amino-1-(methyl-*d*$_3$)-6-oxo-1,6-dihydropyridin-3-yl)quinazolin-4-yl)piperazine-1-carboxylate was obtained, yield: 74.5%; ESI-MS(m/z): 440.0 $[M+H]^+$.

Step d): Preparation of tert-butyl 4-(6-(5-((2,4-difluorophenyl)sulfonamido)-1-(methyl-*$d_3$*)-6-oxo-1,6-dihydropyridin-3 -yl)quinazolin-4-yl)piperazine-1-carboxylate

Tert-butyl 4-(6-(5-amino-1-(methyl-*$d_3$*)-6-oxo-1,6-dihydropyridin-3-yl)quinazolin-4-yl)piperazine-1-carboxylate (80 mg, 0.182 mmol) and 2,4-difluorobenzenesulfonyl chloride (39 mg, 0.182 mmol) were dissolved in pyridine (2 mL). The reaction mixture was heated to 70°C, stirred and reacted for 1 h. Upon completion of the reaction, the reaction mixture was concentrated under reduced pressure. The concentrate was diluted by adding water (3 mL) and extracted with dichloromethane (5 mL×3). The organic phases were combined, washed with water (5 mL×3), dried over anhydrous sodium sulfate and filtered. The filtrate was concentrated under reduced pressure. The crude product obtained was purified by silica gel column chromatography (Eluent: dichloromethane/methanol = 1/0 to 10/1). Tert-butyl 4-(6-(5-((2,4-difluorophenyl)sulfonamido)-1-(methyl-*$d_3$*)-6-oxo-1,6-dihydropyridin-3-yl)quinazolin-4-yl)piperazine-1-carboxylate was obtained, yield: 21.4%; ESI-MS(m/z): 616.2 $[M+H]^+$.

Step e): Preparation of 2,4-difluoro-*N*-(1-(methyl-$d_3$)-2-oxo-5-(4-(piperazin-1-yl)quinazolin-6-yl)-1,2-dihydropyridin-3-yl)benzenesulfonamide trifluoroacetate

Tert-butyl 4-(6-(5-((2,4-difluorophenyl)sulfonamido)-1-(methyl-$d_3$)-6-oxo-1,6-dihydropyridin-3-yl)quinazolin-4-yl)piperazine-1-carboxylate (30 mg, 0.049 mmol) was dissolved in dichloromethane (2 mL). Under the ice bath condition, trifluoroacetic acid (1 mL) was slowly added dropwise. After the addition was completed, the reaction mixture was stirred at room temperature for 1 h. Upon completion of the reaction, the reaction mixture was concentrated under reduced pressure. 2,4-Difluoro-*N*-(1-(methyl-*$d_3$*)-2-oxo-5-(4-(piperazin-1-yl)quinazofin-6-yl)-1,2-dihydropyridin-3-yl)benzenesulfonamide trifluoroacetate was obtained; ESI-MS (m/z): 516.4 $[M+H]^+$.

Step f): Preparation of (*E*)-2,4-difluoro-*N*-(1-(methyl-*$d_3$*)-2-oxo-5-(4-(4-(4-oxopent-2-enoyl)piperazin-1-yl)quinazolin-6-yl)-1,2-dihydropyridin-3-yl)benzenesulfonamide

2,4-Difluoro-*N*-(1-(methyl-*$d_3$*)-2-oxo-5-(4-(piperazin-1-yl)quinazolin-6-yl)-1,2-dihydropyridin-3-yl)benzenesulfonamide trifluoroacetate (300 mg, 0.477 mmol) and tetrahydrofuran (4 mL) were added to the reaction flask. The reaction system was cooled to -60°Cwith DIPEA (308 mg, 2.383 mmol), (*E*)-4-oxopent-2-enoic acid (54 mg, 0.477 mmol) and 50% $T_3P$ ethyl acetate solution (304 mg, 0.477 mmol) added successively. After the addition was completed, the reaction mixture was kept at -60°C and stirred for 1 h. Upon completion of the reaction, the reaction system was diluted by adding acetonitrile (1 mL) and purified by Prep-HPLC (Method 2). (*E*)-2,4-difluoro-*N*-(1-(methyl-$d_3$)-2-oxo-5-(4-(4-(4-oxopent-2-enoyl)piperazin-1-yl)quinazofin-6-yl)-1,2-dihydropyridin-3-yl)benzenesulfonamide was obtained, yield: 23.9%; $^1H$ NMR (400 MHz, DMSO-*$d_6$*) δ 8.64 (s, 1H), 7.97-7.86 (m, 5H), 7.63 (s, 1H), 7.49-7.37 (m, 2H), 7.20-7.15 (m, 1H), 6.74 (d, *J* = 18.8 Hz, 1H), 3.88-3.80 (m, 8H), 2.37 (s, 3H); ESI-MS(m/z): 612.0 $[M+H]^+$.

**Example 245**

**Preparation of (*E*)-2,4-difluoro-*N*-(2-methoxy-5-(4-(4-(4-oxopent-2-enoyl)piperazin-1-yl)pyrido[3,4-d]pyrimidin-6-yl)pyridin-3-yl)benzenesulfonamide**

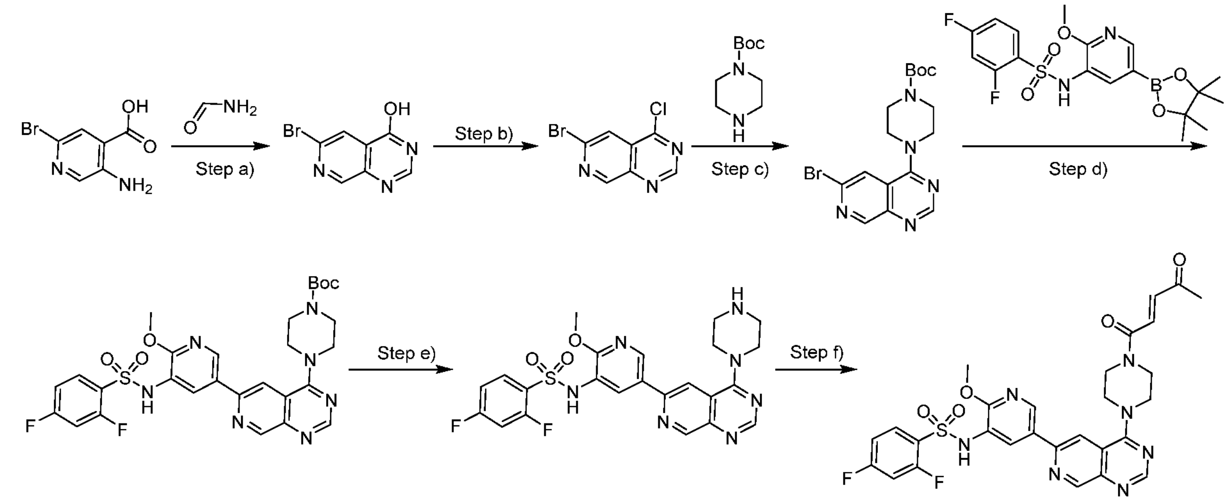


Step a): Preparation of 6-bromopyrido[3,4-d]pyrimidin-4-ol

5-Amino-2-bromoisonicotinic acid (1 g, 4.608 mmol) and formamide (10 mL) were added to the reaction flask. After the addition was completed, in the presense of protective nitrogen, the reaction mixture was heated to 170 °C and stirred for 6 h. Upon completion of the reaction, the reaction mixture was cooled to room temperature and the resultant precipitation was filtered. The filter cake was washed with water (20 mL) and dried *in vacuo.* 6-Bromopyrido[3,4-*d*]pyrimidin-4-ol was obtained, yield: 68.2%; $^1H$ NMR (400 MHz, DMSO-$d_6$) δ 12.73 (s, 1H), 8.88 (s, 1H), 8.25 (s, 1H), 8.10 (s, 1H).

Step b): Preparation of 6-bromo-4-chloropyrido[3,4-*d*]pyrimidine

6-Bromopyrido[3,4-*d*]pyrimidin-4-ol (350 mg, 1.548 mmol), thionyl chloride (10 mL) and *N,N*-dimethylformamide (0.1 mL) were added to the reaction flask. After the addition was completed, the reaction mixture was heated to 120°C and stirred for 12 h. Upon completion of the reaction, the reaction mixture was cooled to room temperature, concentrated under reduced pressure to remove thionyl chloride. 6-Bromo-4-chloropyrido[3,4-*d*]pyrimidine was obtained; ESI-MS (m/z): 246.0 $[M+H]^+$.

Step c): Preparation of tert-butyl 4-(6-bromopyrido[3,4-*d*]pyrimidin-4-yl)piperazine-1-carboxylate

6-Bromo-4-chloropyrido[3,4-*d*]pyrimidine (300 mg, 1.227 mmol), tert-butyl piperazine-1-carboxylate (240 mg, 1.288 mmol), DIPEA (634 mg, 4.908mmol) and DMF (5 mL) were added to the reaction flask. After the addition was completed, the reaction mixture was heated to 55°C and stirred for 1 h. Upon completion of the reaction, the reaction was quenched by water (50 mL). The reaction mixture was extracted with ethyl acetate (50 mL×3). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 10/1 to 4/1). Tert-butyl 4-(6-bromopyrido[3,4-*d*]pyrimidin-4-yl)piperazine-1-carboxylate was obtained, yield: 82.7%; ESI-MS (m/z): 394.1 $[M+H]^+$.

Step d): Preparation of tert-butyl 4-(6-(5-((2,4-difluorophenyl)sulfonamido)-6-methoxypyridin-3-yl)pyrido[3,4-*d*]pyrimidin-4-yl)piperazine-1-carboxylate

Tert-butyl 4-(6-bromopyrido[3,4-*d*]pyrimidin-4-yl)piperazine-1-carboxylate (200 mg, 0.507 mmol), 2,4-difluoro-*N*-(2-methoxy-5-(4,4,5,5-tetramethyl-1,3,2-dioxaborolan-2-yl)pyridin-3-yl)benzenesulfonamide (238 mg, 0.558 mmol), Pd(dppf)$Cl_2$ (37 mg, 0.051 mmol), cesium carbonate (496 mg, 1.521 mmol), 1,4-dioxane (4 mL) and water (1 mL) were added to the reaction flask. After the addition was completed, in the presense of protective nitrogen, the reaction mixture was heated to 100°C and stirred for 1.5 h. Upon completion of the reaction, the reaction mixture was cooled to room temperature and filtered with diatomaceous earth. The filtrate was concentrated under reduced pressure. The crude product obtained was purified by silica gel column chromatography (Eluent: dichloromethane/methanol = 100/1 to 10/1). Tert-butyl 4-(6-(5-((2,4-difluorophenyl)sulfonamido)-6-methoxypyridin-3-yl)pyrido[3,4-*d*]pyrimidin-4-yl)piperazine-1-carboxylate was obtained, yield: 80.4%; ESI-MS (m/z): 614.3 $[M+H]^+$.

Step e): Preparation of 2,4-difluoro-*N*-(2-methoay-5-(4-(piperazin-1-yl)pyrido[3,4-*d*]pyrimidin-6-yl)pyridin-3-yl)benzenesulfonamide trifluoroacetate

Tert-butyl 4-(6-(5-((2,4-difluorophenyl)sulfonamido)-6-methoaypyridin-3-yl)pyrido[3,4-*d*]pyrimidin-4-yl)piperazine-1-carboxylate (250 mg, 0.407 mmol) and dichloromethane (3 mL) were added to the reaction flask. At room temperature, trifluoroacetic acid (1 mL) was slowly added dropwise and the mixture was stirred for 1 h. Upon completion of the reaction, the reaction mixture was concentrated under reduced pressure. The crude product was slurried by adding appropriate amount of methyl tert-butyl ether and filtered. The filter cake was dried. 2,4-Difluoro-*N*-(2-methoxy-5-(4-(piperazin-1-yl)pyrido[3,4-d]pyrimidin-6-yl)pyridin-3-yl)benzenesulfonamide trifluoroacetate was obtained; ESI-MS (m/z): 514.0 $[M+H]^+$.

Step f): Preparation of (*E*)-2,4-difluoro-N-(2-methoxy-5-(4-(4-(4-oxopent-2-enoyl)piperazin-1-yl)pyrido[3,4-*d*]pyrimidin-6-yl)pyridin-3-yl)benzenesulfonamide

2,4-Difluoro-*N*-(2-methoxy-5-(4-(piperazin-1-yl)pyrido[3,4-*d*]pyrimidin-6-yl)pyridin-3-yl)benzenesulfonamide trifluoroacetate (100 mg, 0.159 mmol) and (*E*)-4-oxopent-2-enoic acid (20 mg, 0.175 mmol) and tetrahydrofuran (2.5 mL) were added to the reaction flask. The reaction system was cooled to -78°C, with 50% $T_3P$ ethyl acetate solution (152 mg, 0.239 mmol) and DIPEA (164 mg, 1.272 mmol) added successively. After the addition was completed, the reaction mixture was kept at -78°C and stirred for 0.5 h. Upon completion of the reaction, At -78°C, the reaction was quenched using saturated aqueous ammonium chloride solution (20 mL). The reaction mixture was extracted by adding dichloromethane (30 mL×2). The organic phases were combined, washed with saturated brine (20 mL), dried over anhydrous sodium sulfate and filtered. The filtrate was concentrated under reduced pressure. The crude product obtained was purified by Prep-HPLC (Method 2). (*E*)-2,4-difluoro-N-(2-methoxy-5-(4-(4-(4-oxopent-2-enoyl)piperazin-1-yl)pyrido[3,4-d]pyrimidin-6-yl)pyridin-3-yl)benzenesulfonamide was obtained, yield: 28.6%; $^1H$ NMR (400 MHz, DMSO-$d_6$) δ 10.32 (s, 1H), 9.28 (s, 1H), 8.82-8.79 (m, 1H), 8.76 (s, 1H), 8.39 (d, *J* = 2.4 Hz, 1H), 8.29 (s, 1H), 7.79-7.71 (m, 1H), 7.61-7.54 (m, 1H), 7.44 (d, *J* = 15.6 Hz, 1H), 7.25-7.18 (m, 1H), 6.75 (d, *J* = 15.6 Hz, 1H), 4.13-4.05 (m, 4H), 3.95-3.89 (m, 2H), 3.84-3.78 (m, 2H), 3.69 (s, 3H), 2.37 (s, 3H); ESI-MS(m/z): 610.0 $[M+H]^+$.

**Example 246**

**Preparation of (*E*)-*S*-(2-methoxy-5-(4-(4-(4-oxopent-2-enoyl)piperazin-1-yl)quinazolin-6-yl)pyridin-3-yl)2,4-difluorobenzenesulfonothioate**

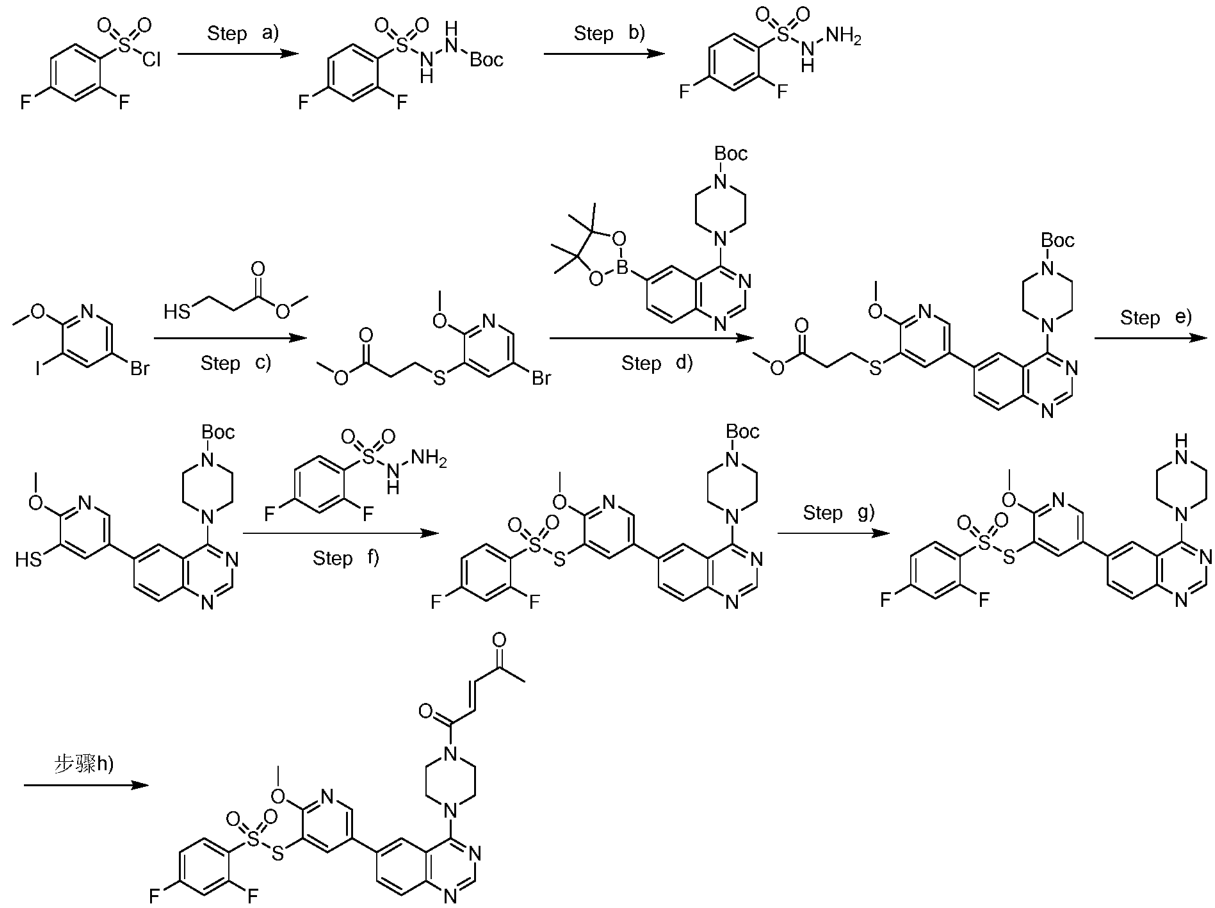


Step a): Preparation of tert-butyl 2-((2,4-difluorophenyl)sulfonyl)hydrazine-1-carboxylate

Tert-butyl carbazate (5.0 g, 0.038 mol), pyridine (15.0 g, 0.190 mol) and dichloromethane (60 mL) were added to the reaction flask, with 2,4-difluorobenzenesulfonyl chloride (8.5 g, 0.040 mol) slowly added dropwise. After the addition was completed, the reaction mixture was kept at room temperature and reacted for 15 h. Upon completion of the reaction, the reaction was quenched by adding saturated aqueous ammonium chloride solution (100 mL) and the reaction mixture was extracted with dichloromethane (80 mL×2). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 1/0 to 10/1). Tert-butyl 2-((2,4-difluorophenyl)sulfonyl)hydrazine-1-carboxylate was obtained, yield: 49.6%; $^1$H NMR (400 MHz, Methanol-$d_4$) δ 7.97-7.82 (m, 1H), 7.25-7.02 (m, 2H), 1.28 (s, 9H).

Step b): Preparation of 2,4-difluorobenzenesulfonohydrazide

Tert-butyl 2-((2,4-difluorophenyl)sulfonyl)hydrazine-1-carboxylate (4.8 g, 0.016 mol) was dissolved in 4 M HCl ethyl acetate (80 mL). The reaction mixture was stirred at room temperature for 1 h. Upon completion of the reaction, the reaction mixture was concentrated under reduced pressure. The crude product obtained was suspended in tetrahydrofuran (15 mL), with saturated aqueous sodium bicarbonate solution (50 mL) and ethyl acetate (50 mL) added, the aqueous phase was extracted with ethyl acetate (60 mL×2). The organic phases were combined, dried over anhydrous sodium sulfate and filtered. The filtrate was concentrated under reduced pressure. 2,4-Difluorobenzenesulfonohydrazide was obtained, yield: 87.8%; $^1$H NMR (400 MHz, DMSO-$d_6$) δ 8.67 (s, 1H), 7.92-7.78 (m, 1H), 7.59-7.46 (m, 1H), 7.36-7.22 (m, 1H), 4.33 (br s, 2H).

Step c): Preparation of methyl 3-((5-bromo-2-methoxypyridin-3-yl)thio)propanoate

5-Bromo-3-iodo-2-methoxypyridine (1000 mg, 3.186 mmol), methyl 3-mercaptopropanoate (345 mg, 2.867 mmol), XantPhos (184 mg, 0.319 mmol), Pd(OAc)$_2$ (72 mg, 0.319 mmol) and DIPEA (1235 mg, 9.558 mmol) were added to the reaction flask, with dioxane (80 mL) added. After the addition was completed, in the presense of protective nitrogen,

the reaction mixture was heated to 50°C and stirred for 2 h. Upon completion of the reaction, the reaction mixture was cooled to room temperature, the reaction was quenched by adding saturated ammonium chloride (50 mL). The reaction mixture was extracted with ethyl acetate (50 mL×3). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 1/0 to 10/1). Methyl 3-((5-bromo-2-methoxypyridin-3-yl)thio)propanoate was obtained, yield: 78.3%; ESI-MS(m/z): 305.9 $[M+H]^+$.

Step d): Preparation of tert-butyl 4-(6-(6-methoxy-5-((3-methoxy-3-oxopropyl)thio)pyridin-3-yl)quinazolin-4-yl)piperazine-1-carboxylate

Methyl 3-((5-bromo-2-methoxypyridin-3-yl)thio)propanoate (939 mg, 3.067 mmol), tert-butyl 4-(6-(4,4,5,5-tetramethyl-1,3,2-dioxaborolan-2-yl)quinazolin-4-yl)piperazine-1-carboxylate (1485 mg, 3.374 mmol), $Pd(dppf)Cl_2$ (224 mg, 0.307 mmol) and sodium bicarbonate (773 mg, 9.201 mmol) were added to the reaction flask, with dioxane (60 mL) and water (0.5 mL) added. After the addition was completed, in the presense of protective nitrogen, the reaction mixture was heated to 60°C and stirred for 15 h. Upon completion of the reaction, the reaction mixture was cooled to room temperature, the reaction was quenched by adding saturated ammonium chloride (150 mL). The reaction mixture was extracted with ethyl acetate (150 mL×3). The organic phases were combined, dried over anhydrous sodium sulfate and filtered. The filtrate was concentrated under reduced pressure. The crude product obtained was purified by silica gel column chromatography (Eluent: petroleum ether/ethyl acetate = 1/0 to 1/2). Tert-butyl 4-(6-(6-methoxy-5-((3-methoxy-3-oxopropyl)thio)pyridin-3-yl)quinazolin-4-yl)piperazine-1-carboxylate was obtained, yield: 29.3%; ESI-MS(m/z): 540.3 $[M+H]^+$.

Step e): Preparation of tert-butyl 4-(6-(5-mercapto-6-methoxypyridin-3-yl)quinazolin-4-yl)piperazine-1-carboxylate

Tert-butyl 4-(6-(6-methoxy-5-((3-methoxy-3-oxopropyl)thio)pyridin-3-yl)quinazolin-4-yl)piperazine-1-carboxylate (485 mg, 0.899 mmol) was dissolved in tetrahydrofuran (20 mL), with potassium tert-butoxide (345 mg, 3.595 mmol) added. After the addition was completed, the reaction mixture was stirred at room temperature for 0.5 h. Upon completion of the reaction, the reaction was quenched by adding saturated ammonium chloride (20 mL), and the reaction mixture was extracted with dichloromethane (20 mL×3). The organic phases were combined, dried over anhydrous sodium sulfate and filtered. The filtrate was concentrated under reduced pressure. Tert-butyl 4-(6-(5-mercapto-6-methoxypyridin-3-yl)quinazolin-4-yl)piperazine-1-carboxylate was obtained. The product can be used directly in the next reaction without purification; ESI-MS(m/z): 454.0 $[M+H]^+$.

Step f): Preparation of tert-butyl 4-(6-(5-(((2,4-difluorophenyl)sulfonyl)thio)-6-methoxypyridin-3-yl)quinazolin-4-yl)piperazine-1-carboxylate

Tert-butyl 4-(6-(5-mercapto-6-methoxypyridin-3-yl)quinazolin-4-yl)piperazine-1-carboxylate (408 mg, crude), 2,4-difluorobenzenesulfonohydrazide (282 mg, 1.356 mmol), sodium iodide (68 mg, 0.452 mmol) were added to the reaction flask, with acetonitrile (15 mL) added. Tert-butyl peroxide (204 mg, 2.260 mmol) was slowly added dropwise to the reaction system. After the addition was completed, in the presense of protective nitrogen, the reaction mixture was stirred at room temperature for 15 h. Upon completion of the reaction, the reaction mixture was directly concentrate under reduced pressure. The crude product obtained was purified by silica gel column chromatography (Eluent: dichloromethane/methanol = 1/0 to 33/1). Tert-butyl 4-(6-(5-(((2,4-difluorophenyl)sulfonyl)thio)-6-methoxypyridin-3-yl)quinazolin-4-yl)piperazine-1-carboxylate was obtained, two-step yield: 45.0%; ESI-MS(m/z): 630.5 $[M+H]^+$.

Step g): Preparation of *S*-(2-methoxy-5-(4-(piperazin-1-yl)quinazolin-6-yl)pyridin-3-yl) 2,4-difluorobenzenesulfonothioate trifluoroacetate

Tert-butyl 4-(6-(5-(((2,4-difluorophenyl)sulfonyl)thio)-6-methoxypyridin-3-yl)quinazolin-4-yl)piperazine-1-carboxylate (150 mg, 0.238 mmol) was dissolved in dichloromethane (9 mL). Under the ice bath condition, TFA (3 mL) was slowly added dropwise. After the addition was completed, the reaction mixture was stirred at room temperature for 1 h. Upon completion of the reaction, the reaction mixture was concentrated under reduced pressure. The crude product obtained was dispersed in 10 mL methyl tert-butyl ether, stirred at room temperature for 0.5 h. The solid was collected and dried. *S*-(2-methoxy-5-(4-(piperazin-1-yl)quinazolin-6-yl)pyridin-3-yl) 2,4-difluorobenzenesulfonothioate trifluoroacetate was obtained, yield: 98.9%; ESI-MS(m/z): 530.1 $[M+H]^+$.

Step h): Preparation of (*E*)-*S*-(2-methoxy-5-(4-(4-(4-oxopent-2-enoyl)piperazin-1-yl)quinazolin-6-yl)pyridin-3-yl) 2,4-difluorobenzenesulfonothioate

*S*-(2-methoxy-5-(4-(piperazin-1-yl)quinazolin-6-yl)pyridin-3-yl) 2,4-difluorobenzenesulfonothioate trifluoroacetate (150 mg, 0.233 mmol), (*E*)-4-oxopent-2-enoic acid (30 mg, 0.257 mmol), DIPEA (150 mg, 1.165 mmol) were placed to the reaction flask, with tetrahydrofuran (6 mL) added. At -78°C, $T_3P$ (193 mg, 0.303 mmol, 50% wt ethyl acetate solution) was slowly added to the system. After the addition was completed, the reaction mixture was kept at -78°C and stirred for 1 h. Upon completion of the reactionthe reaction was quenched by adding saturated ammonium chloride (40 mL) and the reaction mixture was extracted with dichloromethane (40 mL×2). The organic phases were combined, dried over anhydrous sodium sulfate and filtered. The filtrate was concentrated under reduced pressure. The crude product obtained was purified by Prep-HPLC (Method 2). (*E*)-*S*-(2-methoxy-5-(4-(4-(4-oxopent-2-enoyl)piperazin-1-yl)quinazolin-6-yl)pyridin-3-yl) 2,4-difluorobenzenesulfonothioate was obtained, yield: 25.2%; $^1$H NMR (400 MHz, DMSO-$d_6$) δ 8.85 (d, *J* = 2.4 Hz, 1H), 8.77 (s, 1H), 8.48 (d, *J* = 2.4 Hz, 1H), 8.28 (br s, 1H), 8.22 (d, *J* = 8.8 Hz, 1H), 7.94 (d, *J* = 8.8 Hz, 1H), 7.81-7.70 (m, 1H), 7.64-7.54 (m, 1H), 7.44 (d, *J* = 16.0 Hz, 1H), 7.28-7.19 (m, 1H), 6.76 (d, *J* = 15.6 Hz, 1H), 4.16-4.02 (m, 4H), 3.96-3.87 (m, 2H), 3.86-3.77 (m, 2H), 3.60 (s, 3H), 2.37 (s, 3H); ESI-MS(m/z): 626.0 $[M+H]^+$.

**Example 247**

**Preparation of (*E*)-2,4-difluoro-*N*-(2-methoxy-5-(4-(1-(4-oxopent-2-enoyl)pyrrolidin-3-yl)quinazolin-6-yl)pyridin-3-yl)benzenesulfonamide**

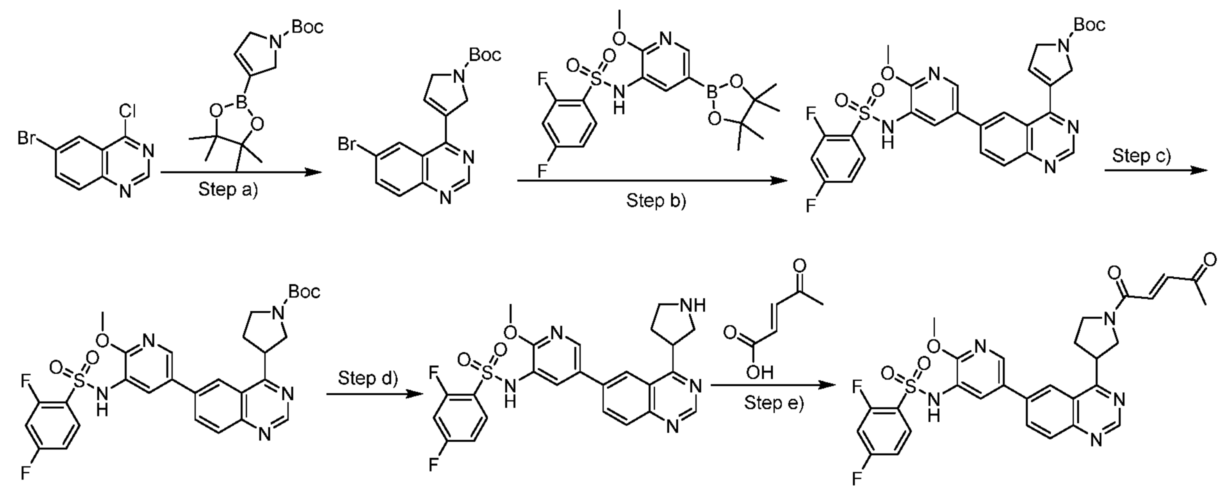


Step a): Preparation of tert-butyl 3-(6-bromoquinazolin-4-yl)-2,5-dihydro-1*H*-pyrrole-1-carboxylate

6-Bromo-4-chloroquinazoline (500 mg, 2.053 mmol), tert-butyl 3-(4,4,5,5-tetramethyl-1,3,2-dioxaborolan-2-yl)-2,5-dihydro-1*H*-pyrrole-1-carboxylate (606 mg, 2.053 mmol), Pd(dppf)$Cl_2$ (150 mg, 0.205 mmol) and cesium carbonate (2.01 g, 6.159 mmol) were added to 1,4-dioxane/water mixed solvent (20 mL, v/v = 10/1). After the addition was completed, in the presense of protective nitrogen, the reaction mixture was kept at room temperature and reacted overnight. Upon completion of the reaction, the reaction was quenched by adding water (50 mL). The reaction mixture was extracted with ethyl acetate (80 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: methanol/dichloromethane = 1/100 to 1/10). Tert-butyl 3-(6-bromoquinazolin-4-yl)-2,5-dihydro-1*H*-pyrrole-1-carboxylate was obtained, yield: 67.5%; ESI-MS(m/z): 376.1 $[M+H]^+$.

Step b): Preparation of tert-butyl 3-(6-(5-((2,4-difluorophenyl)sulfonamido)-6-methoxypyridin-3-yl)quinazolin-4-yl)-2,5-dihydro-1*H*-pyrrole-1-carboxylate

Tert-butyl 3-(6-bromoquinazolin-4-yl)-2.5-dihydro-1*H*-pyrrole-1-carboxylate (300 mg, 0.797 mmol), 2,4-difluoro-*N*-(2-methoxy-5-(4,4,5,5-tetramethyl-1,3,2-dioxaborolan-2-yl)pyridin-3-yl)benzenesulfonamide (340 mg, 0.797 mmol), Pd(dppf)$Cl_2$ (59 mg, 0.080 mmol) and cesium carbonate (779 mg, 2.391 mmol) were added to 1,4-dioxane/water mixed

solvent (10 mL, v/v = 10/1). After the addition was completed, in the presense of protective nitrogen, the reaction mixture was heated to 100°C and reacted for 3 h. Upon completion of the reaction, the reaction was quenched by adding water (50 mL). The reaction mixture was extracted with ethyl acetate (100 mL×2). The organic phases were combined, washed with saturated brine (50 mL), dried over anhydrous sodium sulfate and filtered. The filtrate was concentrated under reduced pressure. The crude product obtained was purified by silica gel column chromatography (Eluent: dichloromethane/methanol = 100/1 to 10/1). Tert-butyl 3-(6-(5-((2,4-difluorophenyl)sulfonamido)-6-methoxypyridin-3-yl)quinazolin-4-yl)-2.5-dihydro-1*H*-pyrrole-1-carboxylate was obtained, yield: 74.3%; ESI-MS(m/z): 596.2 $[M+H]^+$.

Step c): Preparation of tert-butyl 3-(6-(5-((2,4-difluorophenyl)sulfonamido)-6-methoxypyridin-3-yl)quinazolin-4-yl)pyrrolidine-1-carboxylate

Tert-butyl 3-(6-(5-((2,4-difluorophenyl)sulfonamido)-6-methoaypyridin-3-yl)quinazolin-4-yl)-2,5-dihydro-1*H*-pyrrole-1-carboxylate (200 mg, 0.336 mmol), 10%Pd/C (100 mg), glacial acetic acid (0.2 mL) were added to methanol (20 mL), with hydrogen purged three times, under hydrogen atmosphere, the reaction mixture was kept at 30°C and react for 3 h. Upon completion of the reaction, the reaction solution was filtered. The filtrate was concentrated under reduced pressure. The residue was diluted by adding water (30 mL) and extracted with ethyl acetate (50 mL×2). The organic phases were combined, washed with saturated brine (30 mL), dried over anhydrous sodium sulfate and filtered. The filtrate was concentrated under reduced pressure. Tert-butyl 3-(6-(5-((2,4-difluorophenyl)sulfonamido)-6-methoxypyridin-3-yl)quinazolin-4-yl)pyrrolidine-1-carboxylate was obtained, yield: 86.3%; ESI-MS(m/z): 598.2 $[M+H]^+$.

Step d): Preparation of 2,4-difluoro-*N*-(2-methoxy-5-(4-(pyrrolidin-3-yl)quinazolin-6-yl)pyridin-3-yl)benzenesulfonamide trifluoroacetate

Tert-butyl 3-(6-(5-((2,4-difluorophenyl)sulfonamido)-6-methoxypyridin-3-yl)quinazolin-4-yl)pyrrolidine-1-carboxylate (150 mg, 0.251mmol) was dissolved in dichloromethane (4 mL). Under the ice bath condition, TFA (1 mL) was slowly added dropwise. After the addition was completed, the reaction mixture was stirred at room temperature for 1 h. Upon completion of the reaction, the reaction mixture was concentrated under reduced pressure. The crude product was slurried with methyl tert-butyl ether (5 mL) to precipitate solid and filtered. 2,4-Difluoro-*N*-(2-methoxy-5-(4-(pyrrolidin-3-yl)quinazolin-6-yl)pyridin-3-yl)benzenesulfonamide trifluoroacetate was obtained; ESI-MS(m/z): 498.2 $[M+H]^+$.

Step e): Preparation of (*E*)-2,4-difluoro-*N*-(2-methoxy-5-(4-(1-(4-oxopent-2-enoyl)pyrrolidin-3-yl)quinazolin-6-yl)pyridin-3-yl)benzenesulfonamide

2,4-Difhioro-*N*-(2-methoxy-5-(4-(pyrrolidin-3-yl)quinazolin-6-yl)pyridin-3-yl)benzenesulfonamide trifluoroacetate (80 mg, 0.131 mmol) was dissolved in tetrahydrofuran (2 mL). The reaction system was cooled to -78°C, with DIPEA (102 mg, 0.786 mmol), (*E*)-4-oxopent-2-enoic acid (15 mg, 0.131 mmol) and 50% $T_3P$ ethyl acetate solution (167 mg, 0.262 mmol, 50%wt) added successively. After the addition was completed, the reaction mixture was kept at -78°C and reacted for 1 h. Upon completion of the reaction, the reaction solution was diluted by adding acetonitrile (1 mL), purified by Prep-HPLC (Method 2) and freeze dried. (*E*)-2,4-difluoro-*N*-(2-methoxy-5-(4-(1-(4-oxopent-2-enoyl)pyrrolidin-3-yl)quinazolin-6-yl)pyridin-3-yl)benzenesulfonamide was obtained, yield: 18.0%; $^1H$ NMR (400 MHz, DMSO-$d_6$) δ 10.34 (br s, 1H), 9.24 (d, $J$ = 5.2 Hz, 1H), 8.67-8.56 (m, 2H), 8.33-8.29 (m, 1H), 8.14-8.10 (m, 1H), 7.79-7.74 (m, 1H), 7.60-7.54 (m, 1H), 7.24 (dd, $J_1$ = 15.6, $J_2$ = 4.4 Hz, 1H), 7.21-7.15 (m, 1H), 6.77 (dd, $J_1$ = 15.6, $J_2$ = 13.2 Hz, 2H), 4.85-4.74 (m, 1H), 4.33-4.18 (m, 1H), 4.09-3.84 (m, 3H), 3.73-3.68 (m, 1H), 3.67 (d, $J$ = 2.0 Hz, 3H), 3.64-3.57 (m, 1H), 2.36 (d, $J$ = 18.0 Hz, 3H).; ESI-MS(m/z): 594.0 $[M+H]^+$.

**Example 248**

**Preparation of (*Z*)-2,4-difluoro-*N*-(2-methoxy-5-(4-(4-(5,5,5-trifluoro-4-oxopent-2-enoyl)piperazin-1-yl)quinazolin-6-yl)pyridin-3-yl)benzenesulfonamide**

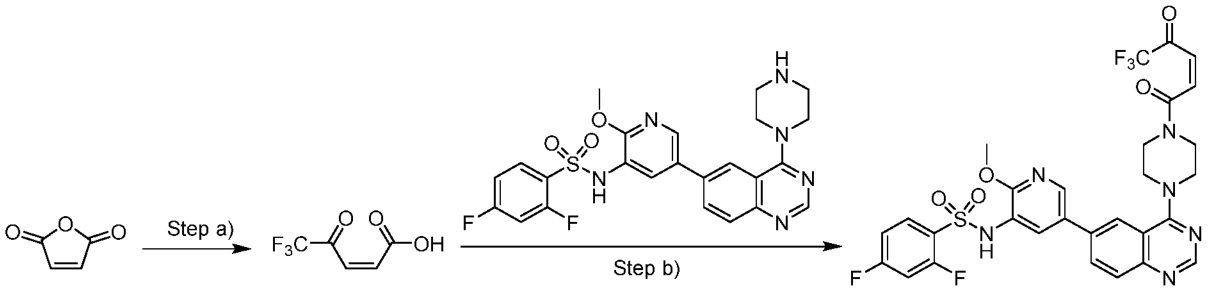


Step a): Preparation of (Z)-5,5,5-trifluoro-4-oxopent-2-enoic acid

In the presense of protective nitrogen, furan-2,5-dione (5.0 g, 0.051 mol) and cesium fluoride (9.3 g, 0.061 mol) was suspended in acetonitrile (60 mL) at 0°C. (Trifluoromethyl)trimethylsilane (8.7 g, 0.061 mol) was slowly added dropwise to the system. After the addition was completed, in the presense of protective nitrogen, the reaction mixture was kept at 0°C and stirred for 1 h. Upon completion of the reaction, at 0°C, the reaction was quenched by adding 2 M aqueous sodium hydroxide solution (60 mL) and the reaction solution was extracted with methyl tert-butyl ether (100 mL×2). The organic was discareded. The aqueous phase was adjusted to pH = 1 with 40% sulfuric acid and extracted with dichloromethane (50 mL×10). The organic phases were combined, washed with saturated brine (100 mL), dried over anhydrous sodium sulfate and filtered. The filtrate was concentrated under reduced pressure. The crude product obtained was purified by silica gel column chromatography (Eluent: petroleum ether/ethyl acetate = 1/0 to 2/1). (Z)-5,5,5-trifluoro-4-oxopent-2-enoic acid was obtained, yield: 14.0%; $^1$H NMR (400 MHz, DMSO-$d_6$) δ 9.45 (s, 1H), 7.82 (d, *J* = 5.6 Hz, 1H), 6.75 (d, *J* = 5.6 Hz, 1H); ESI-MS(m/z): 167.0 [M-H]$^-$.

Step b): Preparation of (*Z*)-2,4-difluoro-*N*-(2-methoxy-5-(4-(4-(5,5,5-trifluoro-4-oxopent-2-enoyl)piperazin-1-yl)quinazolin-6-yl)pyridin-3-yl)benzenesulfonamide

2,4-Difluoro-*N*-(2-methoxy-5-(4-(piperazin-1-yl)quinazolin-6-yl)pyridin-3-yl)benzenesulfonamide trifluoroacetate (100 mg, 0.160 mmol), (*Z*)-5,5,5-trifluoro-4-oxopent-2-enoic acid (27 mg, 0.160 mmol), *N*,*N*-diisopropylethylamine (83 mg, 0.640 mmol) were added to DMF (5 mL). After the addition was completed, under the ice bath condition, HATU (61 mg, 0.160 mmol) was added. The reaction mixture was kept under the ice bath condition and reacted for 1 h. Upon completion of the reaction, the reaction was quenched by adding water (30 mL), and the reaction mixture was extracted with dichloromethane (50 mL×2). The organic phases were combined, washed with saturated brine (30 mL), dried over anhydrous sodium sulfate and filtered. The filtrate was concentrated under reduced pressure. The crude product obtained was purified by Prep-HPLC (Method 2). (*Z*)-2,4-difluoro-*N*-(2-methoxy-5-(4-(4-(5,5,5-trifluoro-4-oxopent-2-enoyl)piperazin-1-yl)quinazolin-6-yl)pyridin-3-yl)benzenesulfonamide was obtained, yield: 23.5%; $^1$H NMR (400 MHz, DMSO-$d_6$) δ 10.29 (s, 1H), 8.66 (s, 1H), 8.45-8.32 (m, 1H), 8.15-8.00 (m, 3H), 8.00-7.93 (m, 1H), 7.91 (d, *J* = 9.2 Hz, 1H), 7.82-7.72 (m, 1H), 7.58-7.45 (m, 1H), 7.24-7.15 (m, 1H), 6.79 (d, *J* = 5.6 Hz, 1H), 3.97-3.75 (m, 4H), 3.69 (s, 3H), 3.15-2.93 (m, 4H); ESI-MS(m/z): 663.0 [M+H]$^+$.

**Bioactivity testing**

The positive reference used in the present disclosure of PI3Kα is the compound with following structure (Alpelisib, NVP-BYL-719):

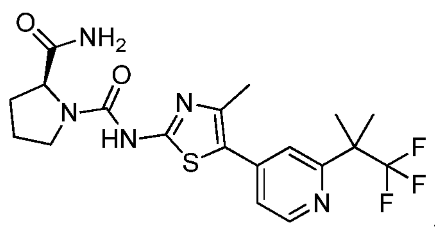

.

The positive reference used in the present disclosure of PI3Kδ is the compound with following structure (Linperlisib, YY-20394):

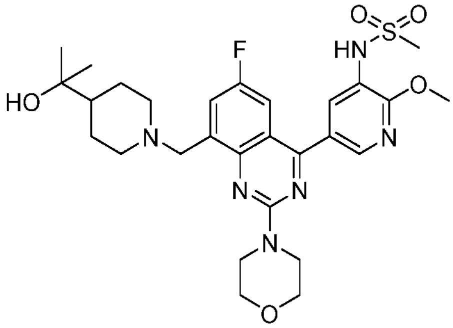

,

or the compound with following structure (Parsaclisib, IBI-376):

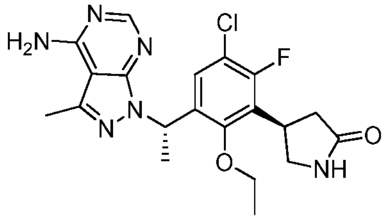

.

The positive reference used in the present disclosure of G12C is the compound with following structure (AMG-510):

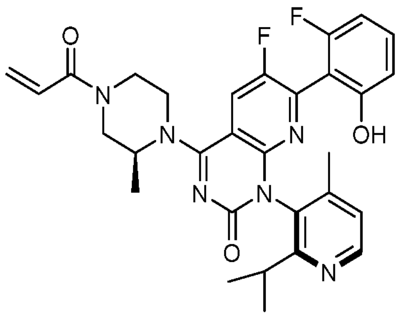

.

**Test Example 1: Inhibiton of PI3Kα Efficacy assay**

The ADP-Glo™ kinase assay was used to assess the inhibition of PI3Kα with our compounds. The utilized proteins and assay reagents were obtained from the ADP-Glo™ Kinase Assay Kit (Promega). Initially, a 2 mM stock solution of the test compound (dissolved in DMSO) was serially diluted in five-fold concentrations using DMSO to obtain eight working solutions 1 (200×). Subsequently, each of the eight working solutions 1 was further diluted in a 20-fold gradient by adding 5 μL into 95 μL of ddHzO, resulting in eight working solutions 2 (10×). In a 384-well white flat-bottom plate, 2 μL of 2.5× PI3Kα kinase solution and 1 μL of working solution 2 (10×) were added to each well and mixed, followed by a 15-minute incubation at room temperature (RT). Subsequently, 2 μL of 2.5× PI and ATP solution mixture were added to each well, mixed, and incubated at RT for 60 minutes. Then, 5 μL of ADP-Glo (containing 10 mM MgCh) reagent was added to each well, mixed, and incubated at RT for 40 minutes. Finally, 10 μL of the Kinase Detection Reagent was added to each well, mixed, and incubated at RT for 40 minutes. Luminescence values were recorded using a multi-mode microplate reader with luminescence channel settings, and the inhibitory rate was calculated using the following formula:

$$\%\ Inhibition\ rate = [\text{(Negative - test compound) / ( Negative -Blank)}]*100$$

Data were analyzed using GraphPad Prism with log(inhibitor) vs. response-Variable Slope(four parameters) fitting calculations for $IC_{50}$ values. (Note: The negative control refers to the group without inhibitor, and the blank control

refers to the group without enzyme.) The data were presented in Tables 1 to 4.

**Test Example 2: Inhibiton of PI3Kδ Efficacy assay**

The ADP-Glo™ kinase assay was used to assess the inhibition of PI3Kδ with our compounds. The utilized proteins and assay reagents were obtained from the ADP-Glo™ Kinase Assay Kit (Promega). Initially, a 2 mM stock solution of the test compound (dissolved in DMSO) was serially diluted in five-fold concentrations using DMSO to obtain eight working solutions 1 (200×). Subsequently, each of the eight working solutions 1 was further diluted in a 20-fold gradient by adding 5 μL into 95 μL of $ddH_2O$, resulting in eight working solutions 2 (10×). In a 384-well white flat-bottom plate, 2 μL of 2.5× PI3Kδ kinase solution and 1 μL of working solution 2 (10×) were added to each well and mixed, followed by a 15-minute incubation at room temperature (RT). Subsequently, 2 μL of 2.5× PI and ATP solution mixture were added to each well, mixed, and incubated at RT for 60 minutes. Then, 5 μL of ADP-Glo (containing 10 mM $MgCl_2$) reagent was added to each well, mixed, and incubated at RT for 40 minutes. Finally, 10 μL of the Kinase Detection Reagent was added to each well, mixed, and incubated at RT for 40 minutes. Luminescence values were recorded using a multi-mode microplate reader with luminescence channel settings, and the inhibitory rate was calculated using the following formula:

$$\%\ Inhibition\ rate = [\ (\text{Negative - test compound})\ /\ (\ \text{Negative -Blank})]*100$$

Data were analyzed using GraphPad Prism with log(inhibitor) vs. response-Variable Slope(four parameters) fitting calculations for $IC_{50}$ values. (Note: The negative control refers to the group without inhibitor, and the blank control refers to the group without enzyme.) The data were presented in Tables 1 to 4.

Table 1: Inhibitory activity of compounds against PI3Kα and PI3Kδ

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 1 | 0.26 | 2.35 | Example 22 | 11.6 | 82.8 |
| Example 2 | 7.3 | 41.8 | Example 24 | 2.9 | 6.3 |
| Example 4 | 9.5 | 12.0 | Example 25 | 26.9 | 126.6 |
| Example 6 | 1.5 | 10.9 | Example 26 | 44.6 | 265.1 |
| Example 7 | 1.4 | 12.0 | Example 27 | 1.8 | 27.3 |
| Example 8 | 0.34 | 17.9 | Example 28 | 1.8 | 49.7 |
| Example 9 | 0.56 | 9.05 | Example 29 | 41.3 | 160.0 |
| Example 10 | 0.44 | 16.7 | Example 30 | 430.4 | 1835 |
| Example 11 | 0.64 | 9.2 | Example 31 | 148.6 | 335.6 |
| Example 12 | 9.3 | 77.4 | Example 32 | 2264 | >5000 |
| Example 13 | 11.3 | 85.3 | Example 33 | 12.6 | 68.7 |
| Example 14 | 9.5 | 62.1 | Example 34 | 1.4 | 20.8 |

(continued)

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 15 | 10.2 | 130.4 | Example 35 | 5.8 | 116.9 |
| Example 16 | 0.26 | 28.5 | Example 36 | 21.5 | 139.6 |
| Example 17 | 2.3 | 31.0 | Example 37 | 1.3 | 20.1 |
| Example 18 | 1.2 | 12.5 | Example 38 | 1.5 | 14.7 |
| Example 19 | 4.7 | 57.8 | Example 41 | 7.6 | 10.1 |
| Example 20 | 7.4 | 211.8 | Alpelisib | 18.7 | N.T. |
| Example 21 | 5.8 | 58.2 | Linperlisib | N.T. | 18.0 |
| Note: N.T. represents not tested. | | | | | |

Table 2: Inhibitory activity of compounds against PI3Kα and PI3Kδ

| Example | PI3Kα | PI3Kδ | Example | PI3Kα | PI3Kδ |
|---|---|---|---|---|---|
| Number | inhibitory activity ($IC_{50}$, nM) | inhibitory activity ($IC_{50}$, nM) | Number | inhibitory activity ($IC_{50}$, nM) | inhibitory activity ($IC_{50}$, nM) |
| Example 42 | 1.5 | 16.5 | Example 89 | 2.2 | 8.6 |
| Example 43 | 2.5 | 12.9 | Example 90 | 4.0 | 23.7 |
| Example 44 | 4.3 | 17.5 | Example 91 | 2.6 | 61.5 |
| Example 45 | 2.8 | 38.6 | Example 92 | 6.4 | 51.1 |
| Example 46 | 1.7 | 36.3 | Example 93 | 6.0 | 72.9 |
| Example 47 | 2.2 | 25.2 | Example 94 | 11.0 | 56.7 |
| Example 48 | 0.63 | 13.2 | Example 95 | 414.1 | N.T. |
| Example 49 | 2.6 | 14.9 | Example 96 | 1.0 | 171.0 |
| Example 50 | 0.61 | 11.4 | Example 97 | 1.4 | 14.1 |
| Example 51 | 5.6 | 19.2 | Example 98 | 9.8 | N.T. |
| Example 52 | 9.1 | N.T. | Example 99 | 6.0 | 7.9 |

(continued)

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 53 | 4.4 | N.T. | Example 100 | 0.54 | N.T. |
| Example 54 | 2.5 | N.T. | Example 101 | 9.9 | 33.6 |
| Example 55 | 1.2 | 43.5 | Example 102 | 1.2 | 32.8 |
| Example 56 | 4.4 | 37.2 | Example 103 | 0.95 | 32.2 |
| Example 57 | 1.9 | 33.4 | Example 104 | 3.8 | 32.2 |
| Example 58 | 3.6 | 8.3 | Example 105 | 8.6 | 38.5 |
| Example 59 | 3.3 | 13.1 | Example 106 | 117.4 | 116.9 |
| Example 60 | 3.9 | 13.5 | Example 107 | 125.6 | 794.0 |
| Example 61 | 5.2 | N.T. | Example 108 | 161.3 | 267.6 |
| Example 62 | 11.8 | 12.5 | Example 109 | 3.5 | 48.9 |
| Example 63 | 6.2 | N.T. | Example 110 | 3.5 | N.T. |
| Example 64 | 16.7 | 14.6 | Example 111 | 8.8 | 23.2 |
| Example 65 | 3.9 | 53.0 | Example 112 | 5.0 | 23.9 |
| Example 66 | 13.8 | 14.8 | Example 113 | 2.9 | 11.5 |
| Example 67 | 9.8 | 13.4 | Example 114 | 1.4 | 28.5 |
| Example 68 | 1.4 | 27.7 | Example 115 | 1.9 | N.T. |
| Example 69 | 2.1 | 15.5 | Example 116 | 1.8 | 27.0 |
| Example 70 | 8.8 | 35.0 | Example 117 | 1.5 | N.T. |
| Example 71 | 2.1 | 31.5 | Example 118 | 1.9 | 19.7 |
| Example 72 | 5.9 | 24.3 | Example 119 | 2.8 | 11.8 |
| Example 73 | 2.5 | 16.5 | Example 120 | 3.0 | 11.5 |

(continued)

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 74 | 1.8 | 23.2 | Example 121 | 111.3 | N.T. |
| Example 75 | 0.78 | 31.2 | Example 122 | 8.1 | 4.7 |
| Example 76 | 7.5 | 25.3 | Example 123 | 9.1 | 31.4 |
| Example 77 | 3.9 | N.T. | Example 124 | 4.4 | 61.4 |
| Example 79 | 909.8 | 903 | Example 125 | 5.0 | 17.0 |
| Example 82 | 89.9 | 75.6 | Example 126 | 130.7 | 52.7 |
| Example 83 | 6.2 | 17.6 | Example 127 | 1.9 | 31.7 |
| Example 84 | 0.83 | 7.6 | Example 128 | 21.7 | N.T. |
| Example 85 | 2.8 | N.T. | Example 129 | 106.9 | 141.5 |
| Example 86 | 0.31 | N.T. | Example 130 | 2.6 | 152 |
| Example 87 | 2.4 | N.T. | Alpelisib | 11.0 | N.T. |
| Example 88 | 2.6 | 11.8 | Parsaclisib | N.T. | 8.0 |

Table 3: Inhibitory activity of compounds against PI3Kα and PI3Kδ

| Example Number | PI3Kα inhibitory activity ($IC_{50}$, nM) | PI3Kδ inhibitory activity ($IC_{50}$, nM) |
|---|---|---|
| Example 131 | 1.6 | 13.7 |
| Example 132 | 3.6 | 29.2 |
| Example 133 | 5.4 | 20.4 |
| Alpelisib | 10.0 | N.T. |
| Parsaclisib | N.T. | 6.0 |

Table 4: Inhibitory activity of compounds against PI3Kα and PI3Kδ

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 140 | 0.43 | N.T. | Example 196 | 1.7 | N.T. |
| Example 141 | 0.30 | N.T. | Example 197 | 4.1 | 8.6 |

(continued)

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 142 | 0.57 | N.T. | Example 198 | 1.1 | 13.3 |
| Example 144 | 3.2 | 13.4 | Example 199 | 1.5 | 12.0 |
| Example 145 | 0.44 | 23.8 | Example 200 | 2.3 | 9.7 |
| Example 146 | 2.1 | N.T. | Example 201 | 3.8 | 11.8 |
| Example 147 | 1.6 | N.T. | Example 202 | 1.9 | 27.9 |
| Example 148 | 9.2 | N.T. | Example 203 | 2.4 | 10.4 |
| Example 149 | 3.3 | N.T. | Example 204 | 3.3 | 17.0 |
| Example 150 | 0.11 | 10.7 | Example 205 | 1.3 | 9.0 |
| Example 151 | 5.9 | N.T. | Example 206 | 3.9 | 15.0 |
| Example 152 | 0.28 | 15.5 | Example 207 | 3.2 | N.T. |
| Example 153 | 0.73 | 12.7 | Example 208 | 30.6 | N.T. |
| Example 154 | 0.54 | 13.8 | Example 209 | 0.57 | N.T. |
| Example 155 | 0.30 | 53.1 | Example 210 | 0.54 | 5.9 |
| Example 156 | 0.50 | N.T. | Example 211 | 1.7 | N.T. |
| Example 157 | 1.3 | N.T. | Example 212 | 3.8 | N.T. |
| Example 158 | 3.0 | 34.6 | Example 213 | 9.8 | N.T. |
| Example 159 | 11.8 | 91.7 | Example 214 | 2.1 | N.T. |
| Example 160 | 2.0 | 152 | Example 215 | 2.4 | N.T. |
| Example 161 | 0.82 | 9.9 | Example 216 | 1.0 | N.T. |
| Example 162 | 2.4 | N.T. | Example 217 | 0.41 | 10.6 |
| Example 163 | 0.25 | N.T. | Example 218 | 2.6 | 10.8 |
| Example 164 | 2.8 | N.T. | Example 219 | 0.91 | 11.6 |

(continued)

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 165 | 1.2 | 12.0 | Example 220 | 1.1 | 11.4 |
| Example 166 | 16.1 | 22.3 | Example 221 | 2.2 | N.T. |
| Example 167 | 2.2 | 11.8 | Example 222 | 0.49 | 11.7 |
| Example 168 | 6.2 | 17.2 | Example 223 | 1.4 | N.T. |
| Example 169 | 3.8 | 18.9 | Example 224 | 1.1 | 23.8 |
| Example 170 | 7.0 | 17.0 | Example 225 | 0.50 | 8.9 |
| Example 171 | 12.9 | 40.1 | Example 226 | 1.4 | N.T. |
| Example 172 | 4.1 | 11.3 | Example 227 | 2.4 | N.T. |
| Example 173 | 2.2 | 16.5 | Example 228 | 2.8 | 15.9 |
| Example 174 | 3.5 | 17.1 | Example 229 | 3.0 | N.T. |
| Example 175 | 6.0 | 162 | Example 230 | 2.6 | 4.7 |
| Example 176 | 2.6 | 13.9 | Example 231 | 3.6 | 16.1 |
| Example 177 | 5.0 | 19.1 | Example 232 | 2.0 | 14.6 |
| Example 178 | 20.5 | 9.2 | Example 233 | 4.0 | 31.3 |
| Example 179 | 0.37 | N.T. | Example 234 | 4.2 | 20.0 |
| Example 180 | 1.8 | N.T. | Example 235 | 0.3 | 2.38 |
| Example 181 | 0.73 | 20.4 | Example 236 | 1.7 | N.T. |
| Example 182 | 0.65 | N.T. | Example 237 | 5.2 | N.T. |
| Example 183 | 5.3 | N.T. | Example 238 | 3.1 | N.T. |
| Example 184 | 0.81 | 162 | Example 239 | 6.4 | N.T. |
| Example 185 | 1.6 | N.T. | Example 240 | 5.0 | N.T. |
| Example 186 | 2.4 | N.T. | Example 241 | 0.27 | 2.96 |

(continued)

| Example Number | PI3Kα inhibitory activity ($IC_{50}$, nM) | PI3Kδ inhibitory activity ($IC_{50}$, nM) | Example Number | PI3Kα inhibitory activity ($IC_{50}$, nM) | PI3Kδ inhibitory activity ($IC_{50}$, nM) |
|---|---|---|---|---|---|
| Example 187 | 4.6 | N.T. | Example 242 | 282.7 | N.T. |
| Example 188 | 3.3 | N.T. | Example 243 | 838.1 | N.T. |
| Example 189 | 3.1 | N.T. | Example 244 | 270.0 | N.T. |
| Example 190 | 9.5 | N.T. | Example 245 | 14.9 | N.T. |
| Example 191 | 6.4 | 16.7 | Example 246 | 2.24 | 13.77. |
| Example 192 | 9.3 | N.T. | Example 247 | 0.53 | 3.33 |
| Example 193 | 2.8 | N.T. | Example 248 | 2.3 | N.T. |
| Example 194 | 2.8 | N.T. | Alpelisib | 8.0 | N.T. |
| Example 195 | 2.4 | 13.5 | Parsaclisib | N.T. | 4.0 |

Alpelisib, Linperlisib/Parsaclisib were known as PI3Kα inhibitors and PI3Kδ inhibitors, respectively. From the above data, it can be observed that the compounds of the present disclosure demonstrate significant inhibitory effects on PI3Kα and PI3Kδ. Some of the compounds exhibit superior activity compared to the positive control compounds and can be utilized as PI3K inhibitors. They hold promising prospects for wide application in the field of diseases mediated by PI3K proteins, such as cancer.

**Test Example 3 : Enzymatic activity of compounds against $KRAS^{G12C}$ in vitro ($KRAS^{G12C}$-SOS1 Binding testing method)**

The HTRF $KRAS^{G12C}$/SOS1 Binding Assay was used to measure the interaction between $KRAS^{G12C}$ and SOS1 proteins, and evaluate the enzymatic activity of compounds against $KRAS^{G12C}$. The utilized proteins and assay reagents were obtained from the $KRAS^{G12C}$-SOS1 binding assay kits (Cisbio). Initially, a 2 mM stock solution of the test compound (dissolved in DMSO) was serially diluted in five-fold concentrations using DMSO to obtain eight working solutions 1 (200×). Subsequently, each of the eight working solutions 1 was further diluted in a 20-fold gradient by adding 5 μL into 95 μL of Diluent buffer, mixed completely with vortex mixer, resulting in eight working solutions 2 (10×). In a 384-well white flat-bottom plate, 4 μL of Tag2-$KRAS^{G12C}$ solution, 2 μL of working solution 2 (10×) and 4 μL of Tag1-SOS1 solution were sequentially added to each well and mixed, followed by a 15-minute incubation at room temperature (RT). Subsequently, 5 μL of Anti-tag1-$Tb^{3+}$ stock solution and 5μL of Antitag2-XI,665 were added to each well, mixed, and incubated at 4°C for 3 hours. Remove the plate and read on an HTRF® compatible reader, with the excitation light wavelength set at 337nm and readings recorded at 620nm and 665nm. The data results were presented as the ratio of the 665nm signal value to the 620nm signal value for each well, calculated as follows: Ratio = $10^4$ × (665nm signal value) / (620nm signal value). The inhibition rate was calculated using the following formula:

$$\%\ Inhibition\ rate = [(\mathrm{Ratio}_{negative} - \mathrm{Ratio}_{compound}) / (\mathrm{Ratio}_{negative} - \mathrm{Ratio}_{Blank})] \times 100$$

Data were analyzed using GraphPad Prism with log(inhibitor) vs. response-Variable Slope (four parameters) fitting calculations for $IC_{50}$ values. (Note: The negative control refers to the group without inhibitor, and the blank control refers to the group without enzyme.) The data were presented in Table 5.

Table 5: Inhibitory activity of compounds on KRAS-G12C/SOS1 binding

| Compound | Kras G12C binding assay ($IC_{50}$, nM) | Compound | Kras G12C binding assay ($IC_{50}$, nM) |
|---|---|---|---|
| Example 6 | 323 | Example 161 | 283.9 |
| Example 8 | 221.5 | Example 165 | 362.2 |
| Example 13 | 96.71 | Example 168 | 546.3 |
| Example 15 | 557.8 | Example 169 | 249.2 |
| Example 17 | 313 | Example 171 | 441.3 |
| Example 20 | 239.4 | Example 172 | 622.0 |
| Example 22 | 149.0 | Example 173 | 198.9 |
| Example 34 | 521.1 | Example 174 | 422.7 |
| Example 38 | 807.1 | Example 175 | 287.5 |
| Example 42 | 109.0 | Example 176 | 113.3 |
| Example 46 | 166.5 | Example 177 | 246.2 |
| Example 49 | 225.3 | Example 178 | 213.3 |
| Example 51 | 941.6 | Example 179 | 147.2 |
| Example 56 | 470.9 | Example 184 | 365.0 |
| Example 58 | 278.1 | Example 191 | 143.0 |
| Example 84 | 267.7 | Example 195 | 158.4 |
| Example 88 | 218.9 | Example 197 | 271.0 |
| Example 89 | 403.6 | Example 198 | 133.4 |
| Example 91 | 173.2 | Example 199 | 176.2 |
| Example 92 | 369.0 | Example 200 | 133.3 |
| Example 93 | 344.4 | Example 201 | 140.3 |
| Example 94 | 188.8 | Example 202 | 142.2 |
| Example 97 | 101.2 | Example 203 | 419.2 |
| Example 99 | 188.4 | Example 204 | 306.1 |
| Example 101 | 197.9 | Example 205 | 369.1 |
| Example 102 | 140.6 | Example 206 | 188.3 |
| Example 103 | 202.7 | Example 210 | 130.7 |
| Example 104 | 168.6 | Example 217 | 207.4 |
| Example 109 | 498.9 | Example 218 | 291.8 |
| Example 111 | 364.5 | Example 219 | 151.5 |
| Example 116 | 1573 | Example 220 | 274.0 |
| Example 122 | 145.2 | Example 223 | 210.3 |
| Example 124 | 729.6 | Example 224 | 187.5 |
| Example 131 | 200.0 | Example 225 | 109.5 |
| Example 132 | 317.8 | Example 228 | 293.8 |
| Example 133 | 250.6 | Example 230 | 493.1 |
| Example 144 | 389.9 | Example 231 | 462.5 |

(continued)

| Compound | Kras G12C binding assay ($IC_{50}$, nM) | Compound | Kras G12C binding assay ($IC_{50}$, nM) |
|---|---|---|---|
| Example 145 | 200.2 | Example 232 | 269.2 |
| Example 150 | 236.1 | Example 233 | 203.6 |
| Example 152 | 174.8 | Example 234 | 292.0 |
| Example 153 | 210.0 | Example 235 | 112.6 |
| Example 154 | 183.4 | Example 241 | 454.5 |
| Example 155 | 461.8 | Example 246 | 159.0 |
| Example 158 | 190.6 | Example 247 | 277.1 |
| Example 160 | 168.9 | / | / |

From the above data, it can be seen that the compounds of the present disclosure also have obvious inhibitory effects on KRAS G12C and have broad application prospects in disease-related fields such as cancer mediated by KRAS G12C protein.

**Test Example 4: Determination of the inhibitory effect on proliferation activity of KRAS G12C mutant cell line NCI-H358 cells**

The CellTiter-Glo® assay was used to detect the number of viable cells, in order to evaluate the anti-proliferation efficacy of compounds. NCI-H358 cells in logarithmic growth phase were collected and seeded in a clear-bottom 96-well plate at a density of $4\times10^3$ cells per well in 80μL of medium, and then incubated overnight at 37°C with 5% $CO_2$. The compounds were serially diluted 5-fold in DMSO to obtain 8 concentrations, and then further diluted with RPMI-640 (10% FBS) cell culture medium to obtain the working solutions (5×). 20μL of each working solution was added to the cell culture supernatant in each well, and the plate was further incubated for 3 days at 37°C with 5% $CO_2$. The plate was then equilibrated with CellTiter-Glo® reagent at room temperature (25°C) for 10-30 minutes. After aspirating 50μL of the medium, 50μL of CellTiter-Glo® reagent was added. The cells were thoroughly mixed with the CellTiter-Glo® reagent using a microplate shaker for 2 minutes, and then incubated at room temperature for 10 minutes. The 96-well plate was placed in a multi-mode microplate reader to record the luminescence values (RLU).

The inhibition rate was calculated through the following equation:

$$\%\ Inhibition\ rate = \left[\frac{\overline{\text{Ratio}}_{Negative\ reference} - \overline{\text{Ratio}}_{Tested\ compound}}{\overline{\text{Ratio}}_{Negative\ reference}}\right] * 100$$

Data were analyzed using GraphPad Prism for $IC_{50}$ values (Note: The negative control refers to the group without inhibitor). The data were presented in Table 6.

**Test Example 5: Determination of the anti-proliferation activity against the MIA Paca-2 cells cell line (KRAS G12C mutant)**

The number of viable cells was determined using the CellTiter-Glo® assay to evaluate the anti-proliferation efficacy of compounds. MIA PaCa-2 cells in logarithmic growth phase were collected and seeded in a clear-bottom 384-well plate at a density of 600 cells per well in 15 μL of medium, and then incubated overnight at 37°C with 5% $CO_2$. The compounds were serially diluted 4-fold in DMSO to obtain 9 concentrations, and then further diluted with DMEM (10% FBS + 2.5% horse serum + 1% penicillin/streptomycin) cell culture medium to obtain the working solutions (2×). 15 μL of each working solution was added to the cell culture supernatant in each well, and the cells were further incubated for 3 days at 37°C with 5% $CO_2$. The plate was then equilibrated with CellTiter-Glo® reagent at room temperature (25°C) for 30 minutes. After adding 25 μL of CellTiter-Glo® reagent, the cells were thoroughly mixed with the CellTiter-Glo® reagent using a microplate shaker for 2 minutes, and then incubated at room temperature for at least 30 minutes. The 384-well plate was placed in a multi-mode microplate reader to record the luminescence values (RLU).

The inhibition rate was calculated through the following equation:

$$\% \, Inhibition \, rate = \left[\frac{\overline{\text{Ratio}}_{Negative\ reference} - \overline{\text{Ratio}}_{Tested\ compound}}{\overline{\text{Ratio}}_{Negative\ reference}}\right] * 100$$

Data were analyzed using GraphPad Prism for $IC_{50}$ values (Note: The negative control refers to the group without inhibitor). The data were presented in Table 7.

Table 6: Inhibitory activity of compounds on H358 cells (G12C mutation)

| Example Number | NCI-H358 ($IC_{50}$, nM) | Example Number | NCI-H358 ($IC_{50}$, nM) |
|---|---|---|---|
| Example 1 | 21.0 | Example 128 | 278.2 |
| Example 2 | 485.0 | Example 130 | 442.2 |
| Example 4 | 494.0 | Example 131 | 5.2 |
| Example 5 | 5387 | Example 132 | 4.0 |
| Example 6 | 18.0 | Example 133 | 4.1 |
| Example 7 | 44.0 | Example 140 | 7.3 |
| Example 8 | 91.2 | Example 141 | 80.6 |
| Example 9 | 349.6 | Example 142 | 350.8 |
| Example 10 | 24.6 | Example 144 | 47.8 |
| Example 11 | 101.7 | Example 145 | 36.2 |
| Example 12 | 50.6 | Example 146 | 82.1 |
| Example 13 | 73.3 | Example 147 | 790.3 |
| Example 14 | 232.9 | Example 148 | 637.8 |
| Example 15 | 19.9 | Example 149 | 90.7 |
| Example 16 | 35.2 | Example 150 | 16.4 |
| Example 17 | 34.6 | Example 151 | 80.3 |
| Example 18 | 60.1 | Example 152 | 29.8 |
| Example 19 | 22.0 | Example 153 | 59.8 |
| Example 20 | 26.2 | Example 154 | 16.7 |
| Example 21 | 27.0 | Example 155 | 40.3 |
| Example 22 | 23.7 | Example 156 | 29.7 |
| Example 24 | 123.2 | Example 157 | 128.3 |
| Example 25 | 288.4 | Example 158 | 55.2 |
| Example 26 | 141.7 | Example 159 | 171.7 |
| Example 33 | 333.3 | Example 160 | 13.0 |
| Example 35 | 899.9 | Example 161 | 43.4 |
| Example 36 | 278.1 | Example 162 | 141.4 |
| Example 41 | 4293 | Example 163 | 189.9 |
| Example 42 | 79.0 | Example 164 | 156.4 |
| Example 43 | 27.9 | Example 165 | 36.5 |
| Example 44 | 41.6 | Example 166 | 344.0 |
| Example 45 | 163.7 | Example 167 | 36.5 |
| Example 46 | 29.9 | Example 168 | 31.2 |

(continued)

| Example Number | NCI-H358 ($IC_{50}$, nM) | Example Number | NCI-H358 ($IC_{50}$, nM) |
|---|---|---|---|
| Example 47 | 34.3 | Example 169 | 5.7 |
| Example 48 | 52.6 | Example 170 | 61.6 |
| Example 49 | 32.0 | Example 171 | 5.7 |
| Example 50 | 31.1 | Example 172 | 68.1 |
| Example 51 | 48.7 | Example 173 | 6.8 |
| Example 53 | 520.0 | Example 174 | 15.8 |
| Example 54 | 10.4 | Example 175 | 22.3 |
| Example 55 | 39.5 | Example 176 | 27.0 |
| Example 56 | 69.7 | Example 177 | 36.1 |
| Example 57 | 29.9 | Example 178 | 25.3 |
| Example 58 | 18.1 | Example 179 | 276.4 |
| Example 59 | 30.6 | Example 180 | 114.0 |
| Example 60 | 48.2 | Example 181 | 49.7 |
| Example 61 | 291.8 | Example 182 | 228.1 |
| Example 62 | 15.1 | Example 184 | 73.8 |
| Example 63 | 19.6 | Example 185 | 69.4 |
| Example 64 | 12.6 | Example 186 | 25.4 |
| Example 65 | 25.1 | Example 187 | 139.7 |
| Example 66 | 13.5 | Example 188 | 529.6 |
| Example 67 | 24.2 | Example 190 | 111.7 |
| Example 68 | 861.4 | Example 191 | 202 |
| Example 69 | 60.7 | Example 192 | 782.4 |
| Example 70 | 58.4 | Example 193 | 52.7 |
| Example 71 | 75.0 | Example 194 | 728.3 |
| Example 72 | 14.4 | Example 195 | 43.9 |
| Example 73 | 34.3 | Example 196 | 186.1 |
| Example 74 | 26.5 | Example 197 | 14.0 |
| Example 75 | 285.0 | Example 198 | 122 |
| Example 76 | 3416 | Example 199 | 12.0 |
| Example 77 | 220.7 | Example 200 | 10.9 |
| Example 80 | 784.4 | Example 201 | 7.2 |
| Example 81 | 1089 | Example 202 | 5.2 |
| Example 83 | 458.8 | Example 203 | 8.5 |
| Example 84 | 13.0 | Example 204 | 25.2 |
| Example 85 | 213.1 | Example 205 | 14.3 |
| Example 86 | 271.4 | Example 206 | 8.0 |
| Example 87 | 130.7 | Example 207 | 40.0 |
| Example 88 | 80.9 | Example 208 | 1059 |

(continued)

| Example Number | NCI-H358 ($IC_{50}$, nM) | Example Number | NCI-H358 ($IC_{50}$, nM) |
|---|---|---|---|
| Example 89 | 81.4 | Example 210 | 53.4 |
| Example 90 | 54.5 | Example 211 | 12.8 |
| Example 91 | 17.8 | Example 212 | 15.2 |
| Example 92 | 16.7 | Example 213 | 14.3 |
| Example 93 | 18.0 | Example 214 | 12.6 |
| Example 94 | 62.5 | Example 215 | 10.0 |
| Example 95 | 575.8 | Example 216 | 56.0 |
| Example 96 | 35.8 | Example 217 | 27.0 |
| Example 97 | 13.7 | Example 218 | 25.6 |
| Example 98 | 8.5 | Example 219 | 7.3 |
| Example 99 | 9.6 | Example 220 | 61.5 |
| Example 100 | 154.7 | Example 221 | 42.9 |
| Example 101 | 11.6 | Example 222 | 35.1 |
| Example 102 | 73.0 | Example 223 | 135.4 |
| Example 103 | 83.0 | Example 224 | 88.1 |
| Example 104 | 742 | Example 225 | 13.5 |
| Example 107 | 405.4 | Example 226 | 248.2 |
| Example 109 | 63.8 | Example 227 | 83.5 |
| Example 110 | 155.4 | Example 228 | 132 |
| Example 111 | 15.8 | Example 229 | 24.0 |
| Example 112 | 72.2 | Example 230 | 6.9 |
| Example 113 | 58.0 | Example 231 | 3.7 |
| Example 114 | 34.6 | Example 232 | 8.7 |
| Example 115 | 46.5 | Example 233 | 5.0 |
| Example 116 | 95.9 | Example 234 | 5.5 |
| Example 117 | 37.0 | Example 236 | 19.5 |
| Example 118 | 79.2 | Example 237 | 58.6 |
| Example 119 | 67.9 | Example 238 | 65.8 |
| Example 120 | 94.9 | Example 239 | 55.0 |
| Example 122 | 47.9 | Example 240 | 48.7 |
| Example 123 | 212 | Example 243 | 719.6 |
| Example 124 | 19.7 | Example 245 | 496.9 |
| Example 125 | 97.2 | Example 248 | 115.4 |
| Example 127 | 222 | / | / |

Table 7: Inhibitory activity of compounds on MIA Paca-2 cells

| Example Number | MIA PaCa-2 ($IC_{50}$, nM) | Example Number | MIA PaCa-2 ($IC_{50}$, nM) |
|---|---|---|---|
| Example 1 | 98 | Example 173 | 9.2 |
| Example 2 | 485 | Example 174 | 15.6 |
| Example 4 | 494 | Example 175 | 22.8 |
| Example 6 | 17.1 | Example 176 | 29.3 |
| Example 7 | 32.3 | Example 177 | 25.9 |
| Example 8 | 73.6 | Example 178 | 43.1 |
| Example 10 | 83.2 | Example 179 | 87.8 |
| Example 13 | 144.4 | Example 180 | 45.2 |
| Example 15 | 71.1 | Example 181 | 56.2 |
| Example 17 | 87.9 | Example 182 | 201.6 |
| Example 20 | 64.1 | Example 184 | 60.2 |
| Example 22 | 92.1 | Example 185 | 103.6 |
| Example 34 | 254.1 | Example 186 | 52.9 |
| Example 38 | 59.8 | Example 187 | 173.3 |
| Example 42 | 283.5 | Example 188 | 865.3 |
| Example 46 | 91.9 | Example 190 | 108.2 |
| Example 49 | 110 | Example 191 | 54.22 |
| Example 51 | 50.7 | Example 192 | 827.1 |
| Example 58 | 26.2 | Example 193 | 114.9 |
| Example 70 | 40.1 | Example 194 | 782.1 |
| Example 84 | 245.0 | Example 195 | 93.3 |
| Example 88 | 333.1 | Example 196 | 154.6 |
| Example 89 | 510.7 | Example 197 | 21.3 |
| Example 90 | 51.9 | Example 198 | 8.7 |
| Example 91 | 30.7 | Example 199 | 41.1 |
| Example 92 | 31.0 | Example 200 | 40.5 |
| Example 93 | 39.4 | Example 201 | 31.9 |
| Example 94 | 65.9 | Example 202 | 11.6 |
| Example 96 | 90.0 | Example 203 | 51.4 |
| Example 97 | 109.2 | Example 204 | 114.8 |
| Example 99 | 16.7 | Example 205 | 78.9 |
| Example 101 | 11.6 | Example 206 | 21.0 |
| Example 102 | 142.5 | Example 207 | 73.3 |
| Example 103 | 344.5 | Example 208 | 1035 |
| Example 104 | 138.7 | Example 210 | 47.7 |
| Example 109 | 94.2 | Example 211 | 10.4 |
| Example 111 | 44.4 | Example 212 | 14.0 |
| Example 116 | 106.7 | Example 213 | 16.4 |

(continued)

| Example Number | MIA PaCa-2 ($IC_{50}$, nM) | Example Number | MIA PaCa-2 ($IC_{50}$, nM) |
|---|---|---|---|
| Example 122 | 105.8 | Example 214 | 6.3 |
| Example 123 | 84.1 | Example 215 | 6.3 |
| Example 124 | 58.4 | Example 216 | 47.7 |
| Example 131 | 25.0 | Example 217 | 21.1 |
| Example 132 | 16.1 | Example 218 | 21.5 |
| Example 133 | 12.0 | Example 219 | 5.8 |
| Example 134 | 19.6 | Example 220 | 58.5 |
| Example 135 | 9.1 | Example 221 | 60.0 |
| Example 136 | 6.6 | Example 222 | 29.1 |
| Example 137 | 6.8 | Example 223 | 94.4 |
| Example 138 | 10.5 | Example 224 | 79.3 |
| Example 144 | 58.7 | Example 225 | 15.6 |
| Example 145 | 77.2 | Example 226 | 357.4 |
| Example 150 | 58.5 | Example 227 | 76.2 |
| Example 152 | 68.8 | Example 228 | 34.0 |
| Example 153 | 56.7 | Example 229 | 40.8 |
| Example 154 | 28.8 | Example 230 | 242 |
| Example 155 | 77.6 | Example 231 | 132 |
| Example 158 | 139.8 | Example 232 | 17.7 |
| Example 159 | 357.4 | Example 233 | 142 |
| Example 160 | 48.4 | Example 234 | 14.9 |
| Example 161 | 67.8 | Example 236 | 34.6 |
| Example 165 | 250.5 | Example 237 | 71.6 |
| Example 166 | 368.9 | Example 238 | 79.5 |
| Example 167 | 111.7 | Example 239 | 70.2 |
| Example 168 | 48.5 | Example 240 | 55.2 |
| Example 169 | 31.7 | Example 243 | 392.7 |
| Example 170 | 118.0 | Example 245 | 826.9 |
| Example 171 | 12.6 | Example 248 | 44.1 |
| Example 172 | 171.2 | / | / |

From the data in Tables 6-7, it can be seen that the compounds of the present disclosure have strong anti-cell proliferation activity against KRAS G12C mutated tumor cells.

**Test Example 6: Generation of NCI-H358-AMGR drug-resistant cell line**

NCI-H358 cells were seeded and cultured at 50 to 70% confluence in six-well plate with RPMI-640 (10% FBS) medium at 37°C with 5% $CO_2$. AMG510 was added for induction at three starting concentrations of 1nM, 10nM and 50nM, and the medium containing the same concentration of AMG510 was replaced every 3 days. Cells were passaged once they reached confluence. After every two passages at a given concentration of drug, the concentration was increased in a 3-fold increment until final concentrations of 1 μM for AMG510 was achieved. The inhibitory effects of AMG510 on

both drug-resistant and parental cell lines were compared, and successful generation of the NCI-H358-AMGR drug-resistant cell line was confirmed when the inhibition rate at the maximum dose (10μM) was <50%.

NCI-H358-AMGR cells in logarithmic growth phase were collected and seeded into a clear-bottom 96-well plate at a density of $4\times10^3$ cells per well in 80μL of medium, and then incubated overnight at 37°C with 5% $CO_2$. The compounds were serially diluted 5-fold in DMSO to obtain 8 concentrations, and then further diluted with RPMI-640 (10% FBS) cell culture medium to obtain the working solutions (5×). 20μL of each working solution was added to the cell culture supernatant in each well, and the plate was further incubated for 3 days at 37°C with 5% $CO_2$. The plate was then equilibrated with CellTiter-Glo® reagent at room temperature (25°C) for 10-30 minutes. After aspirating 50μL of the medium, 50μL of CellTiter-Glo® reagent was added. The cells were thoroughly mixed with the CellTiter-Glo® reagent using a microplate shaker for 2 minutes, and then incubated at room temperature for 10 minutes. The 96-well plate was placed in a multi-mode microplate reader to record the luminescence values (RLU).

The inhibitory rate was calculated using the following formula:

$$\%\ Inhibition\ rate = \left[\frac{\overline{\text{Ratio}}_{Negative\ reference} - \overline{\text{Ratio}}_{Tested\ \text{compound}}}{\overline{\text{Ratio}}_{Negative\ reference}}\right] * 100$$

Data were analyzed using GraphPad Prism for $IC_{50}$ values (Note: The negative control refers to the group without inhibitor). The data were presented in Table 8.

Table 8: NCI-H358-AMGR drug-resistant cell proliferation inhibitory activity test

| Compound | NCI-H358-AMGR Proliferation IC50 (nM) | Compound | NCI-H358-AMGR Proliferation IC50 (nM) |
|---|---|---|---|
| AMG-510 | > 10000 | Example 168 | 239.5 |
| Example 1 | 26.96 | Example 169 | 36.2 |
| Example 6 | 15.58 | Example 170 | 379.5 |
| Example 7 | 41.69 | Example 171 | 41.5 |
| Example 8 | 81.6 | Example 172 | 450.7 |
| Example 10 | 86.6 | Example 173 | 54.7 |
| Example 15 | 62.1 | Example 174 | 28.6 |
| Example 17 | 53.3 | Example 175 | 58.1 |
| Example 20 | 48.7 | Example 176 | 76.1 |
| Example 22 | 47.9 | Example 177 | 48.9 |
| Example 38 | 51.1 | Example 178 | 66.2 |
| Example 42 | 132.3 | Example 181 | 283.5 |
| Example 46 | 27.8 | Example 184 | 134.7 |
| Example 49 | 45.5 | Example 185 | 131.1 |
| Example 51 | 61.0 | Example 186 | 43.9 |
| Example 56 | 116.5 | Example 187 | 372.6 |
| Example 58 | 100.1 | Example 191 | 36.4 |
| Example 70 | 74.5 | Example 193 | 21.6 |
| Example 84 | 639.4 | Example 195 | 92.3 |
| Example 90 | 56.2 | Example 197 | 38.4 |
| Example 91 | 83.9 | Example 198 | 22.1 |
| Example 92 | 110.7 | Example 199 | 21.7 |
| Example 93 | 69.3 | Example 200 | 6.5 |

(continued)

| Compound | NCI-H358-AMGR Proliferation IC50 (nM) | Compound | NCI-H358-AMGR Proliferation IC50 (nM) |
|---|---|---|---|
| Example 94 | 65.2 | Example 201 | 5.3 |
| Example 99 | 65.2 | Example 202 | 9.6 |
| Example 101 | 28.3 | Example 203 | 5.4 |
| Example 102 | 516.0 | Example 204 | 47.1 |
| Example 109 | 112.8 | Example 205 | 15.2 |
| Example 111 | 117.8 | Example 206 | 9.5 |
| Example 116 | 232.2 | Example 210 | 286.9 |
| Example 122 | 127.6 | Example 217 | 32.1 |
| Example 123 | 117.9 | Example 218 | 13.5 |
| Example 124 | 85.5 | Example 219 | 15.9 |
| Example 131 | 19.1 | Example 220 | 206.8 |
| Example 144 | 43.1 | Example 223 | 85.2 |
| Example 145 | 223.4 | Example 224 | 219.5 |
| Example 150 | 112.0 | Example 225 | 34.9 |
| Example 152 | 62.1 | Example 226 | 358.8 |
| Example 153 | 100.2 | Example 228 | 40.3 |
| Example 154 | 61.6 | Example 229 | 30.0 |
| Example 155 | 163.5 | Example 230 | 26.7 |
| Example 158 | 154.7 | Example 231 | 21.6 |
| Example 159 | 360.7 | Example 232 | 44.0 |
| Example 160 | 69.5 | Example 233 | 52.0 |
| Example 161 | 145.4 | Example 234 | 58.2 |
| Example 165 | 170.8 | Example 236 | 44.7 |
| Example 167 | 73.0 | | |

**Test Example 7: Generation of MIA PaCa-2-AMGR drug-resistant cell line**

MIA PaCa-2 cells were seeded and cultured at 50 to 70% confluence in six-well plate with DMEM (10%FBS+2.5% horse serum) medium at 37°C with 5% $CO_2$. AMG510 was added for induction at three starting concentrations of 1nM, 10nM and 50nM, and the medium containing the same concentration of AMG510 was replaced every 3 days. Cells were passaged once they reached confluence. After every two passages at a given concentration of drug, the concentration was increased in a 3-fold increment until final concentrations of 1 μM for AMG510 was achieved. The inhibitory effects of AMG510 on both drug-resistant and parental cell lines were compared, and successful generation of the MIA PaCa-2-AMGR drug-resistant cell line was confirmed when the inhibition rate at the maximum dose (10μM) was <50%.

MIA PaCa-2-AMGR cells in logarithmic growth phase were collected and seeded into a clear-bottom 96-well plate at a density of $4\times10^3$ cells per well in 80μL of medium, and then incubated overnight at 37°C with 5% $CO_2$. The compounds were serially diluted 5-fold in DMSO to obtain 8 concentrations, and then further diluted with DMEM (10%FBS+2.5% horse serum) cell culture medium to obtain the working solutions (5×). 20μL of each working solution was added to the cell culture supernatant in each well, and the plate was further incubated for 3 days at 37°C with 5% $CO_2$. The plate was then equilibrated with CellTiter-Glo® reagent at room temperature (25°C) for 10-30 minutes. After aspirating 50μL of the medium, 50μL of CellTiter-Glo® reagent was added. The cells were thoroughly mixed with the CellTiter-Glo® reagent using a microplate shaker for 2 minutes, and then incubated at room temperature for 10 minutes. The 96-well plate was placed in a multi-mode microplate reader to record the luminescence values (RLU).

The inhibitory rate was calculated using the following formula:

$$\% \, Inhibition\, rate = \left[\frac{\overline{\text{Ratio}}_{Negative\, reference} - \overline{\text{Ratio}}_{Tested\text{ compound}}}{\overline{\text{Ratio}}_{Negative\, reference}}\right] * 100$$

Data were analyzed using GraphPad Prism for $IC_{50}$ values (Note: The negative control refers to the group without inhibitor). The data were presented in Table 9.

Table 9: MIA PaCa2-AMGR drug-resistant cell proliferation inhibitory activity test

| Compound | MIA PaCa-2-AMGR proliferation inhibitory ($IC_{50}$, nM) | Compound | MIA PaCa-2-AMGR proliferation inhibitory ($IC_{50}$, nM) |
|---|---|---|---|
| AMG-510 | > 10000 | Example 168 | 191.5 |
| Example 1 | 36.0 | Example 169 | 54.4 |
| Example 6 | 49.2 | Example 170 | 182.7 |
| Example 7 | 222.4 | Example 171 | 32.9 |
| Example 8 | 249.5 | Example 172 | 256.3 |
| Example 10 | 225.8 | Example 173 | 62.6 |
| Example 13 | 433.1 | Example 174 | 26.3 |
| Example 15 | 243.2 | Example 175 | 36.7 |
| Example 17 | 106 | Example 176 | 48.6 |
| Example 20 | 111.4 | Example 177 | 36.6 |
| Example 22 | 76.7 | Example 178 | 34.0 |
| Example 38 | 215.5 | Example 181 | 117.9 |
| Example 42 | 185.4 | Example 184 | 175.5 |
| Example 46 | 77.4 | Example 185 | 45.7 |
| Example 49 | 62.4 | Example 186 | 31.3 |
| Example 51 | 181.3 | Example 187 | 223.7 |
| Example 56 | 115.5 | Example 191 | 40.8 |
| Example 59 | 141.4 | Example 193 | 66.2 |
| Example 70 | 139.2 | Example 195 | 81.1 |
| Example 90 | 105.9 | Example 197 | 15.6 |
| Example 91 | 79.8 | Example 198 | 10.7 |
| Example 92 | 56.9 | Example 199 | 19.1 |
| Example 93 | 114.0 | Example 200 | 4.3 |
| Example 94 | 126.4 | Example 201 | 11.1 |
| Example 99 | 72.9 | Example 202 | 13.0 |
| Example 101 | 35.9 | Example 203 | 16.7 |
| Example 102 | 908.0 | Example 204 | 45.8 |
| Example 109 | 110.9 | Example 205 | 32.9 |
| Example 111 | 90.1 | Example 206 | 6.9 |
| Example 116 | 462.3 | Example 210 | 97.4 |
| Example 122 | 273.0 | Example 217 | 21.4 |

(continued)

| Compound | MIA PaCa-2-AMGR proliferation inhibitory ($IC_{50}$, nM) | Compound | MIA PaCa-2-AMGR proliferation inhibitory ($IC_{50}$, nM) |
|---|---|---|---|
| Example 123 | 328.0 | Example 218 | 16.1 |
| Example 124 | 83.9 | Example 219 | 6.4 |
| Example 131 | 29.6 | Example 220 | 212.1 |
| Example 144 | 56.6 | Example 223 | 63.1 |
| Example 145 | 112.9 | Example 224 | 144.1 |
| Example 150 | 55.1 | Example 225 | 26.0 |
| Example 152 | 52.1 | Example 226 | 424.5 |
| Example 153 | 49.7 | Example 228 | 13.5 |
| Example 154 | 32.2 | Example 229 | 31.3 |
| Example 155 | 111.9 | Example 230 | 35.3 |
| Example 158 | 135.6 | Example 231 | 3.6 |
| Example 159 | 226.4 | Example 232 | 9.0 |
| Example 160 | 53.8 | Example 233 | 16.7 |
| Example 161 | 55.3 | Example 234 | 5.3 |
| Example 165 | 173.1 | Example 236 | 28.8 |
| Example 195 | 280.0 | / | / |

From the data in Table 9, it can be observed that the compounds of the present disclosure exhibit potent inhibitory effects against KRAS G12C inhibitor-resistant tumor cell lines (e.g., resistant to AMG-510) and can be used to overcome drug resistance to KRAS G12C inhibitors.

Although the embodiments of the present disclosure have been shown and described, it will be understood by those of ordinary skill in the art that various changes, modifications, substitutions and variations can be made to these embodiments without departing from the principles and spirit of the disclosure. The scope of the disclosure is defined by the appended claims and their equivalents.

## Claims

1. A compound of formula I″″ ,or a tautomer, meso isomer, racemate, enantiomer, diastereomer or mixture thereof, or a pharmaceutically acceptable hydrate, solvate or salt thereof:

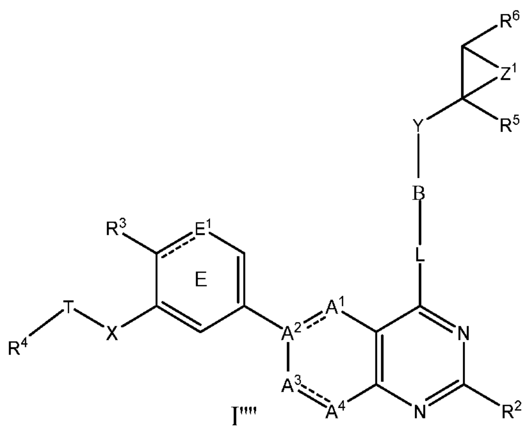

I''''

Wherein,

T is selected from S(O)z, C=O, $CH_2$ or $NHS(O)_2$;
X is selected from $NR^1$, S or S(O)z;
$A^1$ is selected from CH, $CH_2$, C=O or N, $A^2$ is selected from C, CH or N, $A^3$ is selected from $CR^8$, $CR^9R^{10}$ or N, $A^4$ is selected from $CR^{29}$, $CR^9R^{10}$ or N;
$Z^1$ is a bond or O;
$E^1$ is $NR^{11}$, CH or $CH_2$;
B is

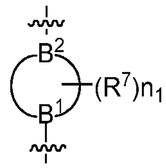

,

the ring where $B^1$, $B^2$ are located is a 5- to 10-membered nitrogen-containing heterocycloalkyl or a 5- to 10-membered nitrogen-containing heterocycloalkenyl, wherein the cycloalkyl or cycloalkenyl is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;
or, B is

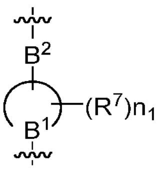

,

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;
or, B is

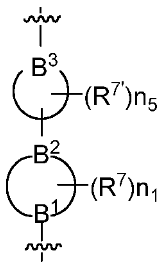

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13''}$ or N, and at least one of $B^1$ and $B^2$ is N; the ring where $B^3$ is located is a 4- to 10-membered nitrogen-containing heterocycloalkyl and is a monocyclic ring, spirocyclic ring or bridged cyclic ring, $B^3$ is selected from $CR^{12'}$ or N;
$B^1$ is connected to L;
L is selected from a bond or $NR^{15}$;
Y is selected from C=O or $S(O)_2$;
$R^5$, $R^6$ are independently selected from hydrogen, halogen, cyano, alkyl, aliphatic heterocyclyl, aryl, heteroaryl, - $OR^a$, $-NR^bR^c$, $-C(O)R^{16}$, $-S(O)R^{16}$, $-S(O)_2R^{16}$, $-P(O)R^{16}R^{17}$, $-C(O)NR^bR^c$ or $-C(O)OR^a$, wherein the alkyl, aliphatic heterocyclyl, aryl, heteroaryl are optionally substituted by one or more $R^{18}$;
each occurrence of $R^{18}$ is independently selected from halogen, cyano, alkyl, cycloalkyl, aliphatic heterocyclyl, $-OR^a$, $-NR^bR^c$, $-C(O)R^{16}$, $-C(O)NR^bR^c$ or $-C(O)OR^a$;
$R^1$, $R^{11}$, $R^{14}$, $R^{15}$ are independently selected from hydrogen, alkyl, cycloalkyl, aliphatic heterocyclyl or $-C(O)R^{19}$, wherein the alkyl, cycloalkyl, aliphatic heterocyclyl are optionally substituted by one or more substituents selected

from the group consisting of: halogen, cyano, hydroxy, amino, alkyl, cycloalkyl and aliphatic heterocyclyl;
$n_1$, $n_5$ are independently selected from 0, 1, 2, 3 or 4;
each occurrence of $R^{12}$, $R^{12'}$, $R^{13}$, $R^{13'}$, $R^{13''}$, $R^7$, $R^{7'}$ is independently selected from hydrogen, halogen, cyano, alkyl, cycloalkyl, aliphatic heterocyclyl, aryl, heteroaryl, $-OR^n$, $-NR^oR^p$, $-C(O)R^{20}$, $-C(O)NR^oR^p$ or $-C(O)OR^n$, wherein alkyl, cycloalkyl, aliphatic heterocyclyl, aryl, heteroaryl are optionally substituted by one or more $R^{21}$;
each occurrence of $R^{21}$ is independently selected from halogen, cyano, alkyl, cycloalkyl, aliphatic heterocyclyl, - $OR^n$, $-NR^oR^p$, $-C(O)R^{20}$, $-C(O)NR^oR^p$ or $-C(O)OR^n$;
or $Z^1$ is a bond, $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicyclyl, or $R^6$, $R^{14}$ together with the fragment to which $R^6$, $R^{14}$ are attached form a 4- to 9-membered aliphatic heterocyclyl, or $R^5$, $R^6$ together with the atoms to which $R^5$, $R^6$ are attached form a 6- to 10-membered aryl or 5- to 10-membered heteroaryl, wherein the cycloalkenyl, alicyclic heterocyclyl, alicyclyl, aryl and heteroaryl are optionally substituted by one or more substituents selected from the group consisting of: halogen, cyano, hydroxy, amino, alkyl, cycloalkyl and alicyclic heterocyclyl;
$R^2$ is selected from hydrogen, halogen, cyano, alkyl, cycloalkyl, alicyclic heterocyclyl, aryl, heteroaryl, $-OR^d$, - $NR^eR^f$, $-C(O)R^{22}$, $-C(O)R^eR^f$ or $-C(O)OR^d$, wherein the alkyl, cycloalkyl, alicyclic heterocyclyl, aryl, heteroaryl are optionally substituted by one or more $R^{23}$;
each occurrence of $R^{23}$ is independently selected from halogen, cyano, alkyl, cycloalkyl, aliphatic heterocyclyl, - $OR^d$, $-NR^eR^f$, $-C(O)R^{22}$, $-C(O)NR^eR^f$ or $-C(O)OR^d$;
$R^3$ is selected from hydrogen, halogen, cyano, alkyl, cycloalkyl, aliphatic heterocyclyl, aryl, heteroaryl, $-OR^g$, $-SR^g$, $-NR^hR^i$, $-C(O)R^{24}$, $-C(O)R^hR^i$ or $-C(O)OR^g$, when the '-----' in ring E is absent, $R^3$ can also be a carbonyl group that together formed by the connected carbon atom, wherein alkyl, cycloalkyl, aliphatic heterocyclyl, aryl, heteroaryl are optionally substituted by one or more $R^{25}$;
each occurrence of $R^{25}$ is independently selected from halogen, cyano, alkyl, cycloalkyl, aliphatic heterocyclyl, $-OR^g$, $-NR^hR^i$, $-C(O)R^{24}$, $-C(O)NR^hR^i$ or $-C(O)OR^g$;
$R^4$ is selected from alkyl, cycloalkyl, aliphatic heterocyclyl, aryl or heteroaryl, wherein the alkyl, cycloalkyl, aliphatic heterocyclyl, aryl, heteroaryl are optionally substituted by one or more $R^{26}$;
each occurrence of $R^{26}$ is independently selected from halogen, cyano, alkyl, cycloalkyl, aliphatic heterocyclyl, aryl, heteroaryl, $-OR^j$, $-NR^kR^m$, $-C(O)R^{27}$, $-C(O)NR^kR^m$ or $-C(O)OR^j$, wherein the alkyl, cycloalkyl, aliphatic heterocyclyl, aryl, heteroaryl are optionally substituted by one or more $R^{28}$;
each occurrence of $R^{28}$ is independently selected from halogen, cyano, alkyl, cycloalkyl, aliphatic heterocyclyl, $-OR^j$, $-NR^kR^m$, $-C(O)R^{27}$, $-C(O)NR^kR^m$ or $-C(O)OR^j$;
each occurrence of $R^8$, $R^9$, $R^{10}$, $R^{29}$ is independently selected from hydrogen, halogen, cyano, alkyl, cycloalkyl, aliphatic heterocyclyl, aryl, heteroaryl, $-OR^q$, $-NR^rR^s$, $-C(O)R^{30}$, $-C(O)NR^rR^s$ or $-C(O)OR^q$; wherein the alkyl, cycloalkyl, aliphatic heterocyclyl, aryl, heteroaryl are optionally substituted by one or more $R^{31}$;
When the '-----' in ring E is a bond, $R^{11}$ is absent;
each occurrence of $R^{31}$ is independently selected from halogen, cyano, alkyl, cycloalkyl, aliphatic heterocyclyl, $-OR^q$, $-NR^rR^s$, $-C(O)R^{30}$, $-C(O)NR^rR^s$ or $-C(O)OR^q$;
each occurrence of $R^a$, $R^b$, $R^c$, $R^d$, $R^e$, $R^f$, $R^g$, $R^h$, $R^i$, $R^j$, $R^k$, $R^m$, $R^n$, $R^o$, $R^p$, $R^q$, $R^r$, $R^s$ is independently selected from H, alkyl, cycloalkyl, aliphatic heterocyclyl or $-C(O)R^{32}$; wherein the alkyl, cycloalkyl, alicyclic heterocyclyl are optionally substituted by one or more substituents selected from the group consisting of: halogen, cyano, hydroxy, amino, alkyl, cycloalkyl and alkyl substituted or unsubstituted aliphatic heterocyclyl;
each occurrence of $R^{16}$, $R^{17}$, $R^{19}$, $R^{20}$, $R^{22}$, $R^{24}$, $R^{27}$, $R^{30}$, $R^{32}$ is independently selected from H, alkyl, cycloalkyl or alicyclic heterocyclyl, wherein the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl.

2. A compound of formula I''', or a tautomer, meso isomer, racemate, enantiomer, diastereomer or mixture thereof, or a pharmaceutically acceptable hydrate, solvate or salt thereof:

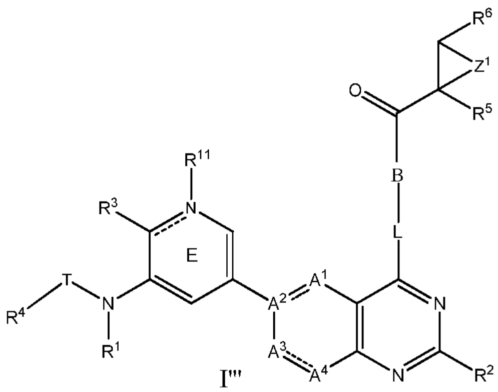

wherein,

T is selected from S(O)z, C=O, $CH_2$ or $NHS(O)_2$;
$A^1$ is selected from CH, $CH_2$, C=O or N, $A^2$ is selected from C, CH or N, $A^3$ is selected from $CR^8$, $CR^9R^{10}$ or N, $A^4$ is selected from $CR^{29}$, $CR^9R^{10}$ or N;
$Z^1$ is a bond or O;
B is

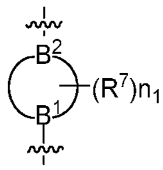

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;
or, B is

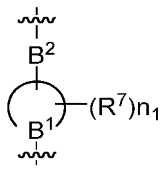

,

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;
or, B is

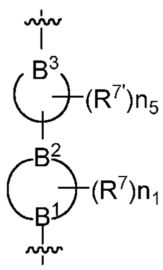

,

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13''}$ or N, and at least one of $B^1$ and $B^2$ is N; the ring where $B^3$ is located is a 4- to 10-membered nitrogen-containing heterocycloalkyl and is a monocyclic ring, spirocyclic ring or bridged cyclic ring, $B^3$ is selected from $CR^{12'}$ or N;

$B^1$ is connected to L;

L is selected from a bond or $NR^{15}$;

$R^5$, $R^6$ are independently selected from hydrogen, halogen, cyano, alkyl, aliphatic heterocyclyl, aryl, heteroaryl, - $OR^a$, $-NR^bR^c$, $-C(O)R^{16}$, $-S(O)R^{16}$, $-S(O)_2R^{16}$, $-P(O)R^{16}R^{17}$, $-C(O)NR^bR^c$ or $-C(O)OR^a$, wherein the alkyl, aliphatic heterocyclyl, aryl, heteroaryl are optionally substituted by one or more $R^{18}$;

each occurrence of $R^{18}$ is independently selected from halogen, cyano, alkyl, cycloalkyl, aliphatic heterocyclyl, $-OR^a$, $-NR^bR^c$, $-C(O)R^{16}$, $-C(O)NR^bR^c$ or $-C(O)OR^a$;

$R^1$, $R^{11}$, $R^{14}$, $R^{15}$ are independently selected from hydrogen, alkyl, cycloalkyl, aliphatic heterocyclyl or $-C(O)R^{19}$, wherein the alkyl, cycloalkyl, aliphatic heterocyclyl are optionally substituted by one or more substituents selected from the group consisting of: halogen, cyano, hydroxy, amino, alkyl, cycloalkyl and aliphatic heterocyclyl;

$n_1$, $n_5$ are independently selected from 0, 1, 2, 3 or 4;

each occurrence of $R^{12}$, $R^{12'}$, $R^{13}$, $R^{13'}$, $R^{13''}$, $R^7$, $R^{7'}$ is independently selected from hydrogen, halogen, cyano, alkyl, cycloalkyl, aliphatic heterocyclyl, aryl, heteroaryl, $-OR^n$, $-NR^oR^p$, $-C(O)R^{20}$, $-C(O)NR^oR^p$ or $-C(O)OR^n$, wherein alkyl, cycloalkyl, aliphatic heterocyclyl, aryl, heteroaryl are optionally substituted by one or more $R^{21}$;

each occurrence of $R^{21}$ is independently selected from halogen, cyano, alkyl, cycloalkyl, aliphatic heterocyclyl, - $OR^n$, $-NR^oR^p$, $-C(O)R^{20}$, $-C(O)NR^oR^p$ or $-C(O)OR^n$;

or $Z^1$ is a bond, $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicycyl, or $R^6$, $R^{14}$ together with the fragment to which $R^6$, $R^{14}$ are attached form a 4- to 9-membered aliphatic heterocyclyl, or $R^5$, $R^6$ together with the atoms to which $R^5$, $R^6$ are attached form a 6- to 10-membered aryl or 5- to 10-membered heteroaryl, wherein the cycloalkenyl, alicyclic heterocyclyl, alicyclyl, aryl and heteroaryl are optionally substituted by one or more substituents selected from the group consisting of: halogen, cyano, hydroxy, amino, alkyl, cycloalkyl and alicyclic heterocyclyl;

$R^2$ is selected from hydrogen, halogen, cyano, alkyl, cycloalkyl, alicyclic heterocyclyl, aryl, heteroaryl, $-OR^d$, - $NR^eR^f$, $-C(O)R^{22}$, $-C(O)R^eR^f$ or $-C(O)OR^d$, wherein alkyl, cycloalkyl, alicyclic heterocyclyl, aryl, heteroaryl are optionally substituted by one or more $R^{23}$;

each occurrence of $R^{23}$ is independently selected from halogen, cyano, alkyl, cycloalkyl, aliphatic heterocyclyl, $-OR^d$, $-NR^eR^f$, $-C(O)R^{22}$, $-C(O)NR^eR^f$ or $-C(O)OR^d$;

$R^3$ is selected from hydrogen, halogen, cyano, alkyl, cycloalkyl, aliphatic heterocyclyl, aryl, heteroaryl, $-OR^g$, - $NR^hR^i$, $-C(O)R^{24}$, $-C(O)R^hR^i$ or $-C(O)OR^g$, when the '-----' in ring E is absent, $R^3$ can also be the carbonyl group that together formed by the connected carbon atom, wherein the alkyl, cycloalkyl, aliphatic heterocyclyl, aryl, heteroaryl are optionally substituted by one or more $R^{25}$;

each occurrence of $R^{25}$ is independently selected from halogen, cyano, alkyl, cycloalkyl, aliphatic heterocyclyl, $-OR^g$, $-NR^hR^i$, $-C(O)R^{24}$, $-C(O)NR^hR^i$ or $-C(O)OR^g$;

$R^4$ is selected from alkyl, cycloalkyl, aliphatic heterocyclyl, aryl or heteroaryl, wherein the alkyl, cycloalkyl, aliphatic heterocyclyl, aryl, heteroaryl are optionally substituted by one or more $R^{26}$;

each occurrence of $R^{26}$ is independently selected from halogen, cyano, alkyl, cycloalkyl, aliphatic heterocyclyl, aryl, heteroaryl, $-OR^j$, $-NR^kR^m$, $-C(O)R^{27}$, $-C(O)NR^kR^m$ or $-C(O)OR^j$, wherein the alkyl, cycloalkyl, aliphatic heterocyclyl, aryl, heteroaryl are optionally substituted by one or more $R^{28}$;

each occurrence of $R^{28}$ is independently selected from halogen, cyano, alkyl, cycloalkyl, aliphatic heterocyclyl, -ORi, $-NR^kR^m$, $-C(O)R^{27}$, $-C(O)NR^kR^m$ or $-C(O)OR^j$;

each occurrence of $R^8$, $R^9$, $R^{10}$, $R^{29}$ is independently selected from hydrogen, halogen, cyano, alkyl, cycloalkyl, aliphatic heterocyclyl, aryl, heteroaryl, $-OR^q$, $-NR^rR^s$, $-C(O)R^{30}$, $-C(O)NR^rR^s$ or $-C(O)OR^q$; wherein the alkyl, cycloalkyl, aliphatic heterocyclyl, aryl, heteroaryl are optionally substituted by one or more $R^{31}$;

When the '-----' in ring E is a bond, $R^{11}$ is absent;

each occurrence of $R^{31}$ is independently selected from halogen, cyano, alkyl, cycloalkyl, aliphatic heterocyclyl, $-OR^q$, $-NR^rR^s$, $-C(O)R^{30}$, $-C(O)NR^rR^s$ or $-C(O)OR^q$;

each occurrence of $R^a$, $R^b$, $R^c$, $R^d$, $R^e$, $R^f$, $R^g$, $R^h$, $R^i$, $R^j$, $R^k$, $R^m$, $R^n$, $R^o$, $R^p$, $R^q$, $R^r$, $R^s$ is independently selected from H, alkyl, cycloalkyl, aliphatic heterocyclyl or $-C(O)R^{32}$; wherein the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl;

each occurrence of $R^{16}$, $R^{17}$, $R^{19}$, $R^{20}$, $R^{22}$, $R^{24}$, $R^{27}$, $R^{30}$, $R^{32}$ is independently selected from H, alkyl, cycloalkyl or alicyclic heterocyclyl, wherein the alkyl, cycloalkyl, alicyclic heterocyclyl are optionally substituted by one or more substituents selected from the group consisting of: halogen, cyano, hydroxy, amino, alkyl, cycloalkyl and aliphatic heterocyclyl.

**3.** The compound according to claim 1 or 2, wherein

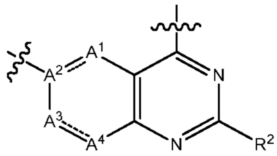

is selected from the following groups:

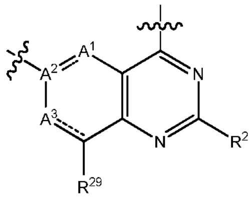,
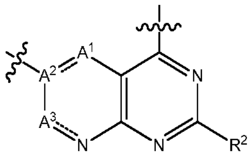,
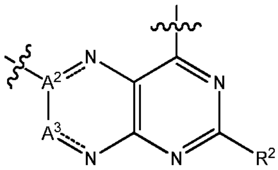,
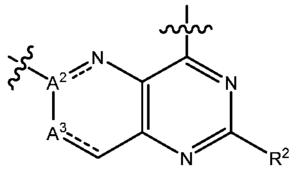;

preferably,

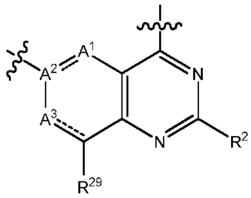

is selected from the following groups:

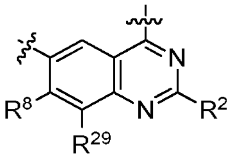
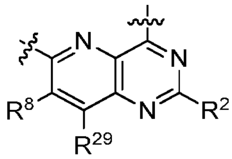
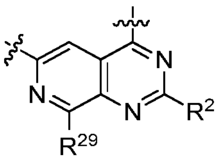
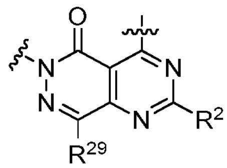
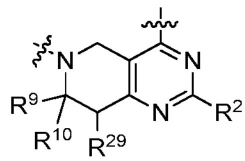

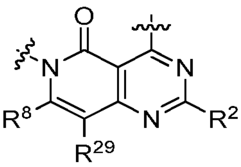,

preferably

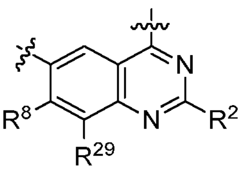,
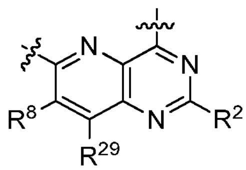,
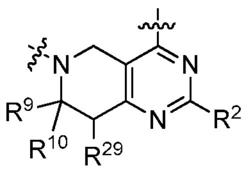,

more preferably

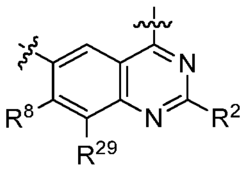

.

4. The compound according to any one of claims 1 to 3, wherein the compound has a structure shown in formula I', or a tautomer, meso isomer, racemate, enantiomer, diastereomer or mixture thereof, or a pharmaceutically acceptable hydrate, solvate or salt thereof:

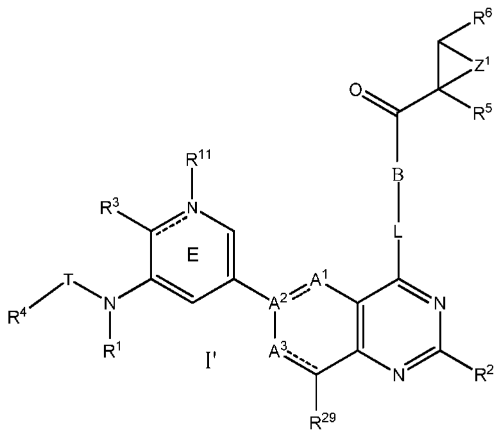

I'

Wherein,

T is selected from S(O)z, C=O, $CH_2$ or $NHS(O)_2$;
$A^1$ is selected from CH, $CH_2$, C=O or N, $A^2$ is selected from C or N, $A^3$ is selected from $CR^8$, $CR^9R^{10}$ or N;
$Z^1$, B, L, $R^1$, $R^2$, $R^3$, $R^4$, $R^5$, $R^6$, $R^{11}$, ring E, $R^{29}$ are each defined as the compound of formula I'''' or I'''.

5. The compound according to any one of claims 1 to 4, wherein the compound has a structure shown in formula I, or a tautomer, meso isomer, racemate, enantiomer, diastereomer or mixture thereof, or a pharmaceutically acceptable hydrate, solvate or salt thereof:

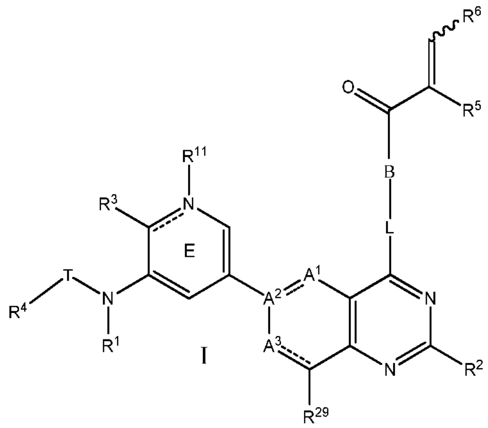

I

wherein,

T is selected from S(O)z or C=O;
B is

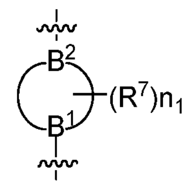

the ring where $B^1$, $B^2$ are located is a 5- to 10-membered nitrogen-containing heterocycloalkyl and is a monocyclic ring, spirocyclic ring or bridged cyclic ring, $B^1$ is selected from $CR^{12}$ or N, $B^2$ is selected from $CR^{13}$ or N, and at least one of $B^1$ and $B^2$ is N;
or, B is

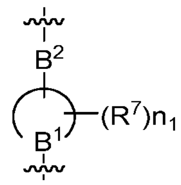

the ring where $B^1$ is located is a 4- to 8-membered nitrogen-containing monoheterocycloalkyl, $B^1$ is selected from $CR^{12}$ or N, $B^2$ is selected from $CR^{13}R^{13'}$ or $NR^{14}$;
$B^1$ is connected to L;
$R^5$, $R^6$ are independently selected from hydrogen, halogen, cyano, alkyl, aliphatic heterocyclyl, aryl, heteroaryl, - $C(O)R^{16}$, $-S(O)R^{16}$, $-S(O)_2R^{16}$, $-P(O)R^{16}R^{17}$, $-C(O)NR^bR^c$ or $-C(O)OR^a$, wherein the alkyl, aliphatic heterocyclyl, aryl, heteroaryl are optionally substituted by one or more $R^{18}$;
or $R^5$, $R^6$ together with the atoms to which $R^5$, $R^6$ are attached form a 4- to 9-membered cycloalkenyl, or $R^5$, $R^{14}$ together with the fragment to which $R^5$, $R^{14}$ are attached form a 4- to 9-membered aliphatic heterocyclyl, or $R^6$, $R^{13}$ together with the fragment to which $R^6$, $R^{13}$ are attached form a 4- to 9-membered cycloalkenyl, or $R^5$, $R^{13}$ together with the fragment to which $R^5$, $R^{13}$ are attached form a 4- to 9-membered alicyclyl, or $R^6$, $R^{14}$ together with the fragment to which $R^6$, $R^{14}$ are attached form a 4- to 9-membered aliphatic heterocyclyl;
$A^1$, $A^2$, $A^3$, L, $R^1$, $R^2$, $R^3$, $R^4$, $R^7$, $R^{11}$, $R^{16}$, $R^{17}$, $R^a$, $R^b$, $R^c$, $R^{12}$, $R^{13}$, $R^{13'}$, $R^{14}$, $R^{18}$, ring E, $R^{29}$, $n_1$ are each defined the same as the compound of formula I'.

6. The compound according to any one of claims 1 to 4, wherein the compound has a structure shown in formula I", or a tautomer, meso isomer, racemate, enantiomer, diastereomer or mixture thereof, or a pharmaceutically acceptable hydrate, solvate or salt thereof:

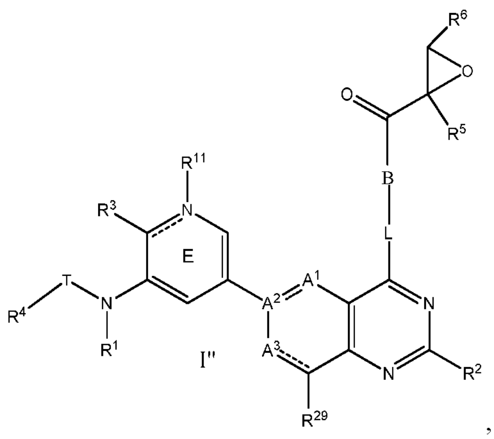

wherein, T is selected from S(O)z or C=O;
$A^1$, $A^2$, $A^3$, L, B, $R^1$, $R^2$, $R^3$, $R^4$, $R^5$, $R^6$, $R^{11}$, ring E, $R^{29}$ are each defined as the compound of formula I'.

7. The compound according to any one of claims 1 to 3, wherein the compound has a structure shown in formula (I'''

-1) or formula (I'''-2), or a tautomer, meso isomer, racemate, enantiomer, diastereomer or mixture thereof, or a pharmaceutically acceptable hydrate, solvate or salt thereof:

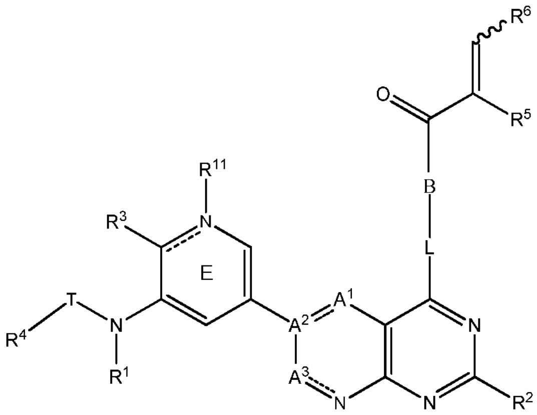

I'''-1

,

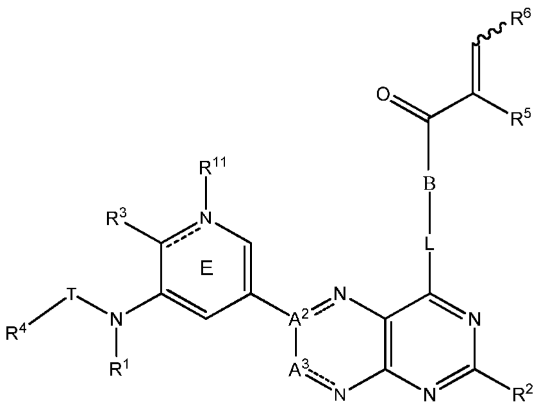

I'''-2

,

wherein, T is selected from S(O)z or C=O.

**8.** The compound according to any one of claims 1 to 7, wherein L is a bond, $B^1$ is N; or L is $NR^{15}$, $B^1$ is $CR^{12}$; further, when B is

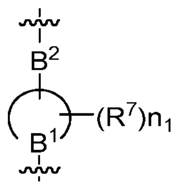

,

$B^1$ is N, $B^2$ is $NR^{14}$.

**9.** The compound according to any one of claims 1 to 8, wherein B is

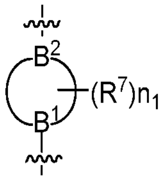

,

the ring where $B^1$, $B^2$ are located is a 6- to 9-membered nitrogen-containing heterocycloalkyl or a 6- to 9-membered nitrogen-containing heterocycloalkenyl, the cycloalkyl or cycloalkenyl is a monocyclic ring or spirocyclic ring; or, B is

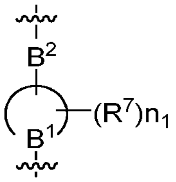

,

the ring where $B^1$ is located is a 4- to 6-membered nitrogen-containing monoheterocycloalkyl;

further, B is

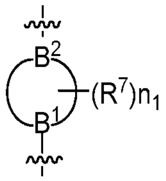

,

the ring where $B^1$, $B^2$ are located is a 5- to 6-membered nitrogen-containing monoheterocycloalkyl, 6-membered nitrogen-containing monoheterocycloalkenyl or 8- to 9-membered nitrogen-containing spiroheterocycloalkyl; or B is

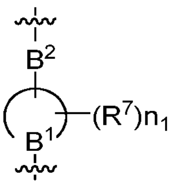

,

the ring where $B^1$ is located is a 4-membered nitrogen-containing monoheterocycloalkyl;
further,

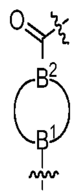

or

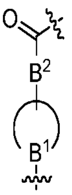

is selected from the following groups:

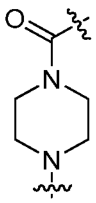 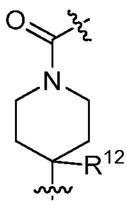 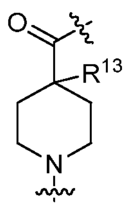 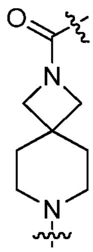 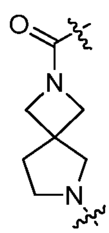 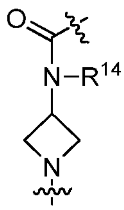 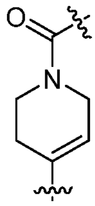 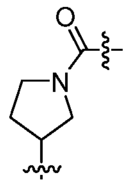 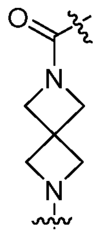 ;

$R^{12}$ is selected from hydrogen, halogen, C1~C6 alkyl, preferably hydrogen, halogen or C1~C3 alkyl, more preferably hydrogen;

$R^{13}$ is selected from hydrogen, halogen, C1~C6 alkyl, preferably hydrogen, halogen or C1~C3 alkyl, or $R^{13}$ and $R^6$ together with the fragment to which $R^{13}$ and $R^6$ are attached form a 4- to 6-membered cycloalkenyl, preferably $R^{13}$ and $R^6$ together with the fragment to which $R^{13}$ and $R^6$ are attached form a 5-membered cycloalkenyl;

$R^{14}$ is selected from hydrogen, C1~C6 alkyl, C3-C6 cycloalkyl, preferably hydrogen or C1~C3 alkyl, wherein the alkyl, cycloalkyl are optionally substituted by one or more substituents selected from the group consisting of: halogen, cyano, hydroxy, amino, C1-C6 alkyl, C3-C6 cycloalkyl and 3- to 6-membered aliphatic heterocyclyl, or $R^{14}$ and $R^5$ together with the fragment to which $R^{14}$ and $R^5$ are attached form a 4- to 6-membered aliphatic heterocyclyl, preferably $R^{14}$ and $R^5$ together with the fragment to which $R^{14}$ and $R^5$ are attached form a 5-membered aliphatic heterocyclyl;

further,

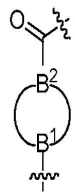

is selected from

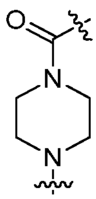 , 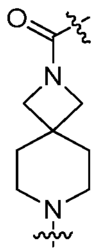 , 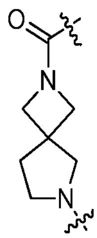 or 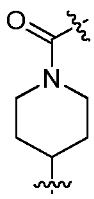 ;

preferably

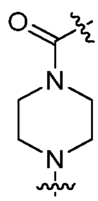 , 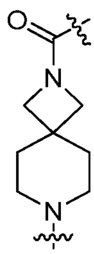 ,

or

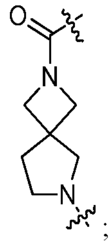

more preferably,

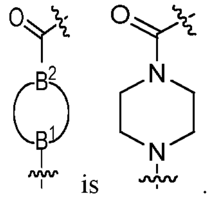

10. The compound according to any one of claims 1 to 8, wherein B is

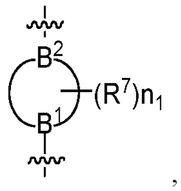

the ring where $B^1$, $B^2$ are located is a 6- to 9-membered nitrogen-containing bridged heterocycloalkyl, preferably 7- to 8-membered nitrogen-containing bridged heterocycloalkyl, more preferably 8-membered nitrogen-containing bridged heterocycloalkyl;

or B is

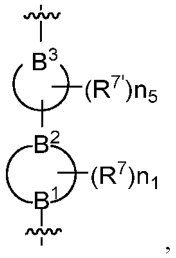

the ring where $B^1$, $B^2$ are located is a 5- to 8-membered nitrogen-containing heterocycloalkyl and is a monocyclic ring, spirocyclic ring or bridged cyclic ring, preferably 5- to 8-membered nitrogen-containing monoheterocycloalkyl, more preferably 6- to 7-membered nitrogen-containing monoheterocycloalkyl; the ring where $B^3$ is located is a 4- to 8-membered nitrogen-containing heterocycloalkyl and is a monocyclic ring, spirocyclic ring or bridged cyclic ring, preferably 4- to 8-membered nitrogen-containing monoheterocycloalkyl, more preferably 4- to 6-membered nitrogen-containing monoheterocycloalkyl;
further, $B^1$ is selected from $CR^{12}$ or N, $B^2$ is selected from $CR^{13''}$ or N, moreover at least one of $B^1$ and $B^2$ is N; $B^3$ is selected from $CR^{12}$ or N; $R^{12}$, $R^{12'}$, $R^{13''}$ are independently selected from hydrogen, halogen, C1~C6 alkyl, preferably hydrogen, halogen or C1-C3 alkyl, more preferably hydrogen;
further, each of $B^1$, $B^2$, $B^3$ is N;
further,

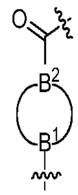

is

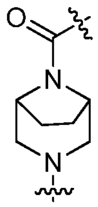, 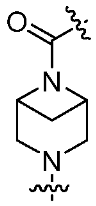, 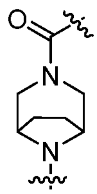, 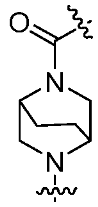, 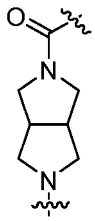; 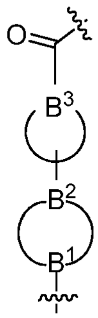 is 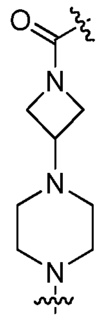.

11. The compound according to any one of claims 1 to 10, wherein the compound has a structure shown in formula II or formula III, or a tautomer, meso isomer, racemate, enantiomer, diastereomer or mixture thereof, or a pharmaceutically acceptable hydrate, solvate or salt thereof:

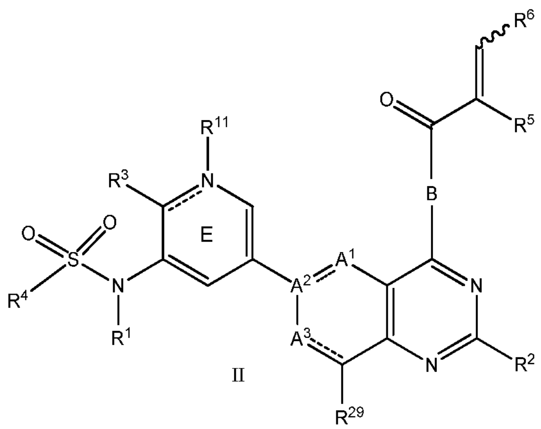

II

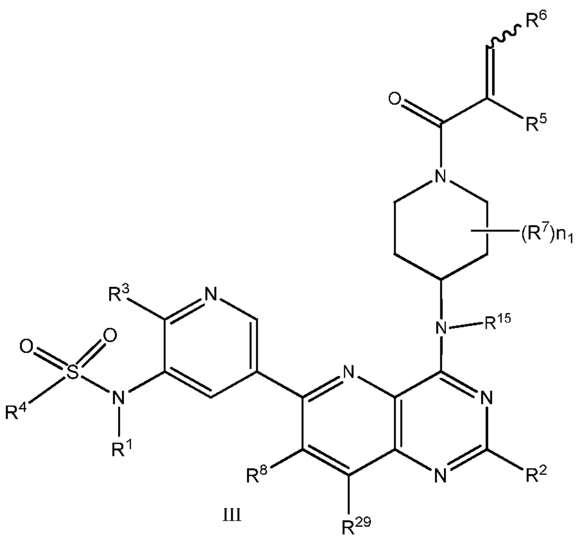

III

in formula II,

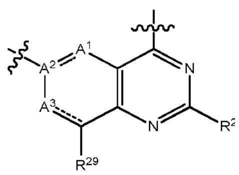

is not

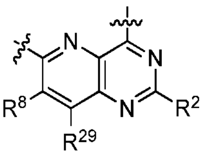

,

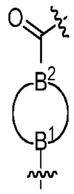

is not

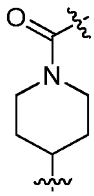

;

preferably, in formula II, when

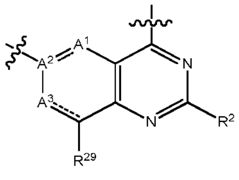

is

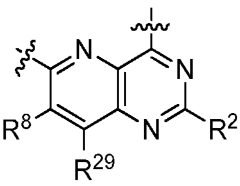,
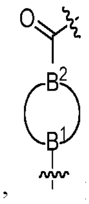

preferably

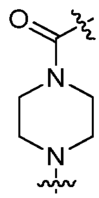,
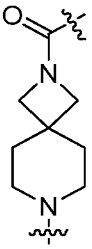
or
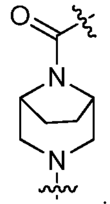.

12. The compound according to any one of claims 1 to 10, wherein the compound has a structure shown in formula IV or formula V, or a tautomer, meso isomer, racemate, enantiomer, diastereomer or mixture thereof, or a pharmaceutically acceptable hydrate, solvate or salt thereof:

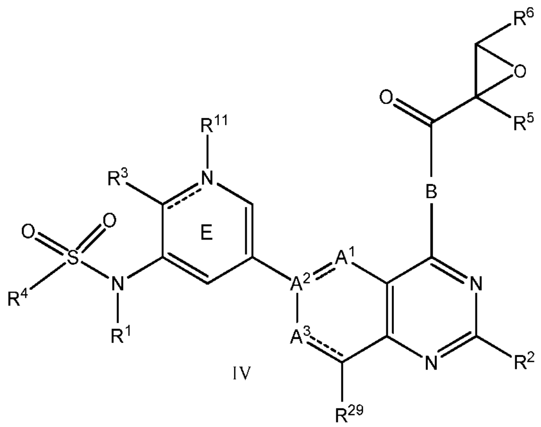

IV

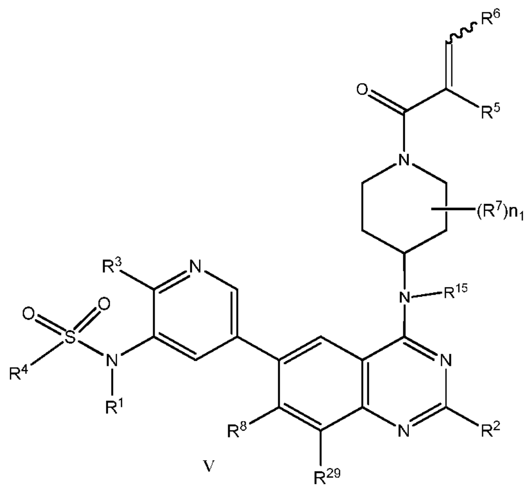

V

.

13. The compound according to any one of claims 1 to 12, wherein $n_1$ is selected from 0, 1, 2 or 3, preferably 0, 1 or 2, more preferably 0 or 1;

preferably, $n_5$ is selected from 0, 1, 2 or 3, preferably 0, 1 or 2, more preferably 0 or 1.

**14.** The compound according to any one of claims 1 to 13, wherein $R^1$, $R^{11}$, $R^{15}$ are independently selected from hydrogen, halogen, C1~C6 alkyl or C3-C6 cycloalkyl, wherein the alkyl, cycloalkyl are optionally substituted by one or more substituents selected from the group consisting of: halogen, cyano, hydroxy, amino, C1-C6 alkyl, C3-C6 cycloalkyl and 3- to 6-membered aliphatic heterocyclyl; when the '----- ' in ring E is a bond, $R^{11}$ is absent; further, $R^1$, $R^{11}$, $R^{15}$ are independently selected from hydrogen, halogen, C1-C3 alkyl, wherein the alkyl or cycloalkyl are optionally substituted by one or more substituents selected from the group consisting of: halogen, cyano, hydroxy, amino, C1-C3 alkyl and C3-C6 cycloalkyl;

further, $R^1$, $R^{15}$ are independently selected from hydrogen or methyl, preferably hydrogen;
$R^{11}$ is selected from hydrogen or methyl, preferably methyl, when the '-----' in ring E is a bond, $R^{11}$ is absent.

**15.** The compound according to any one of claims 11 to 14, wherein the compound has a structure shown in formula II', formula III', formula IV' or formula V', or a tautomer, meso isomer, racemate, enantiomer, diastereomer or mixture thereof, or a pharmaceutically acceptable hydrate, solvate or salt thereof:

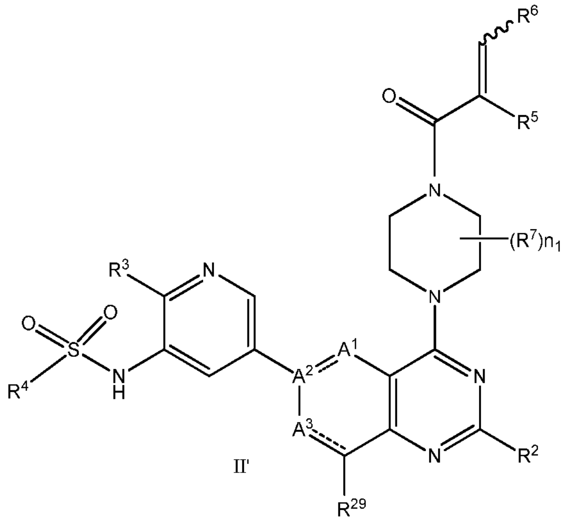

II'

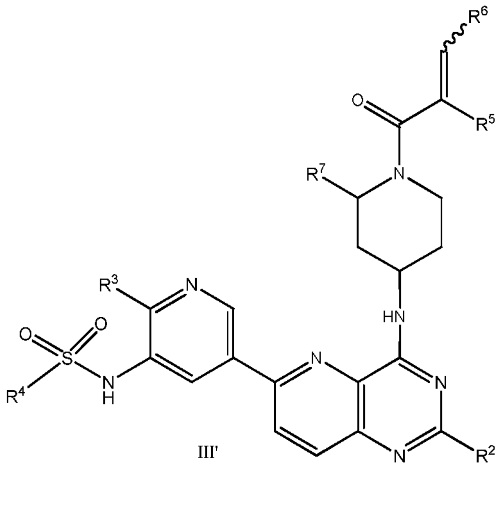

III'

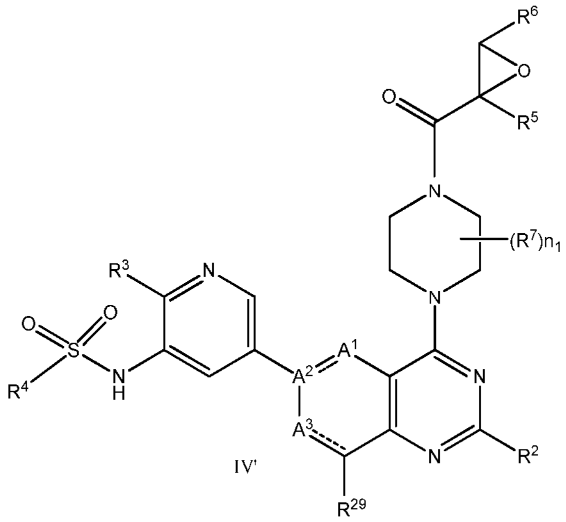

IV'

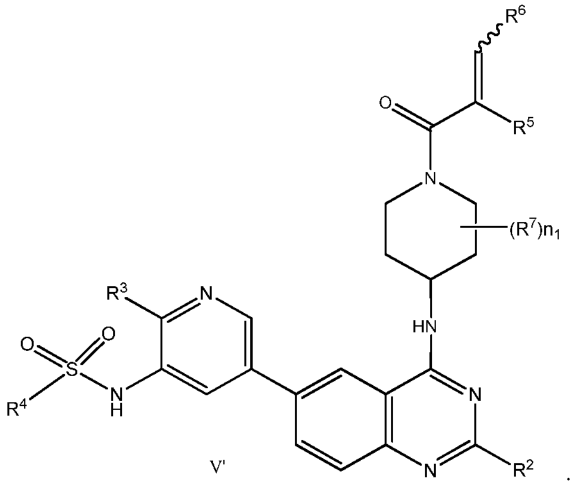

V'

.

**16.** The compound according to any one of claims 1 to 10, wherein the compound has a structure shown in formula VI, VII, VIII or IX, or a tautomer, meso isomer, racemate, enantiomer, diastereomer or mixture thereof, or a pharmaceutically acceptable hydrate, solvate or salt thereof:

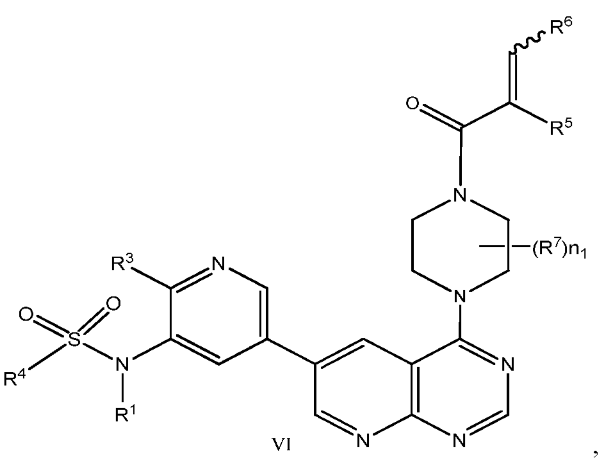

VI

,

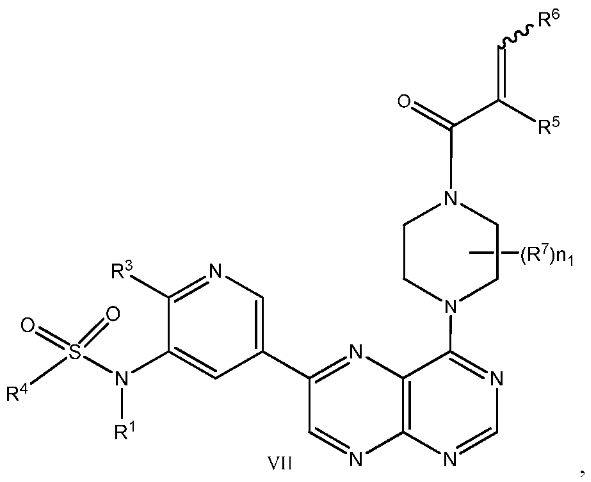

VII

,

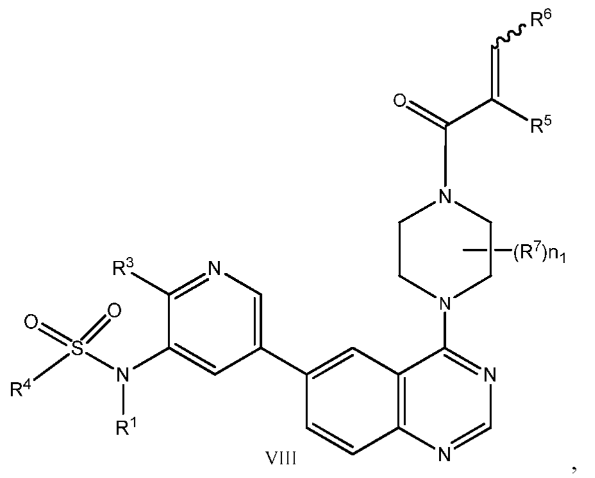

VIII ,

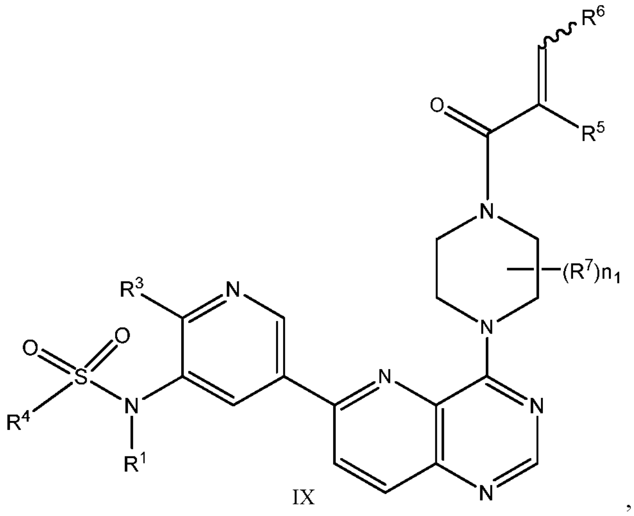

IX ,

wherein $n_1$ is selected from 0, 1, 2 or 3, preferably 0, 1 or 2, more preferably 0 or 1.

**17.** The compound according to any one of claims 1 to 16, wherein $R^5$, $R^6$ are independently selected from hydrogen, halogen, C1~C6 alkyl, C3~C6 cycloalkyl, 3- to 6-membered aliphatic heterocyclyl, 6- to 10-membered aryl, 5- to 10-membered heteroaryl, $-C(O)R^{16}$, $-S(O)R^{16}$, $-S(O)_2R^{16}$, $-P(O)R^{16}R^{17}$, $-C(O)NR^bR^c$ or $-C(O)OR^a$, wherein the alkyl, cycloalkyl, aliphatic heterocyclyl, aryl, heteroaryl are optionally substituted by one or more $R^{18}$;

each occurrence of $R^{18}$ is independently selected from halogen, cyano, C1-C6 alkyl, C3-C6 cycloalkyl, $-OR^a$ or $-NR^bR^c$;

or $R^5$, $R^6$ together with the atoms to which $R^5$, $R^6$ are attached form

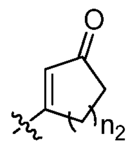

,

$n_2$ is selected from 0, 1, 2, 3 or 4;

or $R^5$, $R^{14}$ together with the fragment to which $R^5$, $R^{14}$ are attached form

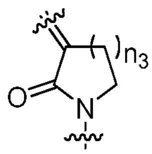

,

$n_3$ is selected from 0, 1, 2, 3 or 4;

or $R^6$, $R^{13}$ together with the fragment to which $R^6$, $R^{13}$ are attached form

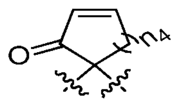

,

$n_4$ is selected from 0, 1, 2, 3 or 4;

further, $R^5$, $R^6$ are independently selected from hydrogen, halogen, C1~C3 alkyl, 5- to 6-membered heteroaryl, - C(O)$R^{16}$, -S(O)$_2R^{16}$ or -P(O)$R^{16}R^{17}$, wherein the alkyl, heteroaryl are optionally substituted by one or more $R^{18}$; each occurrence of $R^{18}$ is independently selected from halogen, C1~C3 alkyl, -$OR^a$ or -$NR^bR^c$;

or $R^5$, $R^6$ together with the atoms to which $R^5$, $R^6$ are attached form

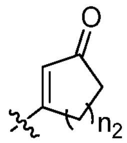

,

$n_2$ is selected from 1, 2 or 3;

or $R^5$, $R^{14}$ together with the fragment to which $R^5$, $R^{14}$ are attached form

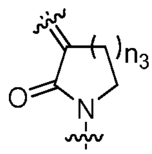

,

$n_3$ is selected from 1, 2 or 3;

or $R^6$, $R^{13}$ together with the fragment to which $R^6$, $R^{13}$ are attached form

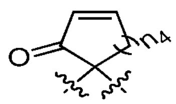

,

$n_4$ is selected from 1, 2 or 3;

further, $R^5$, $R^6$ are independently selected from H, F, -C(O)$CH_3$, -C(O)$CH_2CH_2CH_3$, -$CH_2N(CH_3)_2$, -$CH_2F$, - S(O)$_2CH_3$, -P(O)$(CH_3)_2$ or

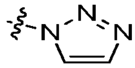

;

or $R^5$, $R^6$ together with the atoms to which $R^5$, $R^6$ are attached form

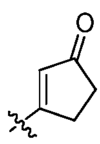 or 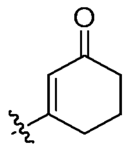 ,

or $R^5$, $R^{14}$ together with the fragment to which $R^5$, $R^{14}$ are attached form

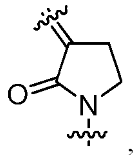 ,

or $R^6$, $R^{13}$ together with the fragment to which $R^6$, $R^{13}$ are attached form

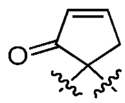 ;

further, $R^5$, $R^6$ are independently selected from H, F, $-C(O)CH_3$, $-C(O)CH_2CH_2CH_3$, $-CH_2F$ or

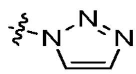 ;

further, $R^5$ is selected from H or F, $R^6$ is selected from H, $-C(O)CH_3$, $-C(O)CH_2CH_2CH_3$, $-CH_2F$ or

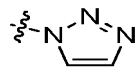 ;

further, the configuration of

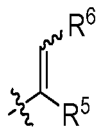

is

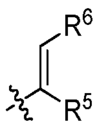 ;

preferably, $R^5$, $R^6$ are independently selected from hydrogen, halogen, C1-C6 alkyl, C3-C6 cycloalkyl, 3- to 6-membered aliphatic heterocyclyl, 6- to 10-membered aryl, 5- to 10-membered heteroaryl, $-C(O)R^{16}$, $-S(O)R^{16}$, $-S(O)_2R^{16}$, $-P(O)R^{16}R^{17}$, $-C(O)NR^bR^c$, $-C(O)OR^a$, $-OR^a$ or $-NR^bR^c$, wherein the alkyl, cycloalkyl, aliphatic heterocyclyl, aryl, heteroaryl are optionally substituted by one or more $R^{18}$;

each occurrence of $R^{18}$ is independently selected from halogen, cyano, C1-C6 alkyl, C3-C6 cycloalkyl, $-OR^a$ or $-NR^bR^c$;

or $Z^1$ is a bond, $R^5$, $R^6$ together with the atoms to which $R^5$, $R^6$ are attached form a 6- to 10-membered aryl,

wherein the aryl is optionally substituted by one or more substituents selected from the group consisting of: halogen, cyano, hydroxy, amino, C1-C6 alkyl, C3-C6 cycloalkyl and 3- to 6-membered aliphatic heterocyclyl;
further, $R^5$, $R^6$ are independently selected from hydrogen, halogen, C1-C3 alkyl, 5- to 6-membered heteroaryl, - $C(O)R^{16}$, $-S(O)_2R^{16}$, $-P(O)R^{16}R^{17}$, $-C(O)NR^bR^c$, $-C(O)OR^a$ or $-OR^a$, wherein the alkyl, heteroaryl are optionally substituted by one or more $R^{18}$;
further, $R^5$, $R^6$ are independently selected from hydrogen, halogen, C1~C3 alkyl, 5- to 6-membered heteroaryl, - $C(O)R^{16}$, $-S(O)_2R^{16}$, $-P(O)R^{16}R^{17}$, $-C(O)OR^a$ or $-OR^a$, wherein the alkyl, heteroaryl are optionally substituted by one or more $R^{18}$;
each occurrence of $R^{18}$ is independently selected from halogen, C1~C3 alkyl, $-OR^a$ or $-NR^bR^c$;
or $Z^1$ is a bond, $R^5$, $R^6$ together with the atoms to which $R^5$, $R^6$ are attached form a phenyl, wherein the phenyl is optionally substituted by 1~2 following substituents: halogen, cyano, hydroxy, amino, C1~C3 alkyl;
further, $R^5$, $R^6$ are independently selected from hydrogen, halogen, C1-C3 alkyl, $-C(O)R^{16}$, $-S(O)_2R^{16}$, $-P(O)R^{16}R^{17}$, -C(O)O-C1~C3 alkyl, -O-C1~C3 alkyl, 1H-1,2,3-triazolyl or oxazolyl, wherein the alkyl, 1H-1,2,3-triazolyl, oxazolyl are optionally substituted by 1~3 $R^{18}$;
each occurrence of $R^{18}$ is independently selected from halogen, C1-C3 alkyl, $-OR^a$ or $-NR^bR^c$;
or $Z^1$ is a bond, $R^5$, $R^6$ together with the atoms to which $R^5$, $R^6$ are attached form a phenyl substituted by one hydroxy;
further, $R^5$, $R^6$ are independently selected from H, F, CN, $-C(CH_3)_3$, $-CH(OH)CH_3$, $-C(O)CF_3$, $-C(O)CH_2CH_3$, - $C(O)CH_3$, $-C(O)NHCH_3$, $-C(O)CH_2CH_2CH_3$, $-CH_2N(CH_3)_2$, $-CH_2F$, $-S(O)_2CH_3$, $-P(O)(CH_3)_2$,

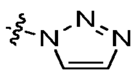
,

$CHCH_3CH_3$, $-CH_2OCH_3$, $CF_3$,

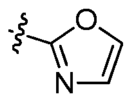
,

$-C(O)OCH_3$ or $-OCH_3$;
or $Z^1$ is a bond, $R^5$, $R^6$ together with the atoms to which $R^5$, $R^6$ are attached form

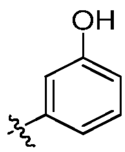
;

further, $R^5$ is selected from H, F, CN, $-CH(CH_3)_2$, $-CH(OH)CH_3$, $-CH_2OCH_3$, $-OCH_3$ or $-C(O)CH_3$, $R^6$ is selected from H, $-C(CH_3)_3$, $-C(O)CH_3$, $-C(O)CH_2CH_2CH_3$, $-C(O)NHCH_3$, $-CH_2N(CH_3)_2$, $-CH_2F$,

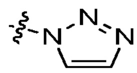
,

$-CH_2OCH_3$, $CF_3$,

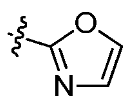
,

$-C(O)OCH_3$, $-P(O)(CH_3)$, $-S(O)_2CH_3$, $-C(O)CF_3$ or $-C(O)CH_2CH_3$;
further, $Z^1$ is a bond; $R^5$ is H; $R^6$ is selected from H, $-C(CH_3)_3$, $-C(O)CH_3$, $-C(O)CH_2CH_2CH_3$, $-C(O)NHCH_3$, - $CH_2N(CH_3)_2$, $-CH_2F$,

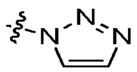,

$-CH_2OCH_3$, $CF_3$,

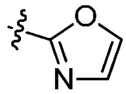,

$-C(O)OCH_3$, $-P(O)(CH_3)$, $-S(O)_2CH_3$ or $-C(O)CF_3$; preferably, $R^6$ is selected from H and $-C(O)CH_3$; further preferably,

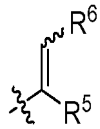

is

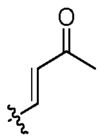.

**18.** The compound according to any one of claims 1 to 17, wherein each occurrence of $R^a$, $R^b$, $R^c$ is independently selected from H, C1-C6 alkyl or $-C(O)R^{13}$, wherein the alkyl is optionally substituted by one or more substituents selected from the group consisting of: halogen, cyano, hydroxy, amino and C1~C6 alkyl;

further, each occurrence of $R^a$, $R^b$, $R^c$ is independently selected from H or C1-C3 alkyl, wherein the alkyl is optionally substituted by one or more of the following substituents: halogen, C1-C3 alkyl;
further, each occurrence of $R^a$, $R^b$, $R^c$ is independently selected from H or C1~C3 alkyl, preferably H or methyl; more preferably methyl.

**19.** The compound according to any one of claims 1 to 18, wherein $R^2$ is selected from hydrogen, halogen, cyano, C1~C6 alkyl, C3-C6 cycloalkyl, $-OR^d$ or $-NR^eR^f$, wherein the alkyl, cycloalkyl are optionally substituted by one or more $R^{14}$, each occurrence of $R^{14}$ is independently selected from halogen, cyano, C1~C6 alkyl, C3~C6 cycloalkyl, - $OR^d$ or $-NR^eR^f$;

further, $R^2$ is selected from hydrogen, halogen, cyano, C1~C3 alkyl, $-OR^d$ or $-NR^eR^f$, wherein the alkyl is optionally substituted by one or more $R^{14}$, each occurrence of $R^{14}$ is independently selected from halogen, cyano, C1~C3 alkyl, $-OR^d$ or $-NR^eR^f$;
further, $R^2$ is hydrogen.

**20.** The compound according to any one of claims 1 to 19, wherein each occurrence of $R^d$, $R^e$, $R^f$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino, C1-C6 alkyl, C3-C6 cycloalkyl and alkyl substituted or unsubstituted C3-C6 heterocycloalkyl;

further, each occurrence of $R^d$, $R^e$, $R^f$ is independently selected from H or C1-C3 alkyl, wherein the alkyl is optionally substituted by one or more substituents selected from the group consisting of: halogen, C1-C3 alkyl and alkyl substituted or unsubstituted 5-membered nitrogen-containing heterocycloalkyl;
further, each occurrence of $R^d$, $R^e$, $R^f$ is independently selected from H or C1-C3 alkyl, wherein the alkyl is optionally substituted by one or more of the following substituents: halogen, C1-C3 alkyl,

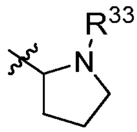

,

$R^{33}$ is selected from H or C1~C6 alkyl, preferably C1~C3 alkyl, more preferably methyl.

**21.** The compound according to any one of claims 1 to 20, wherein $R^3$ is selected from hydrogen, halogen, cyano, C1~C6 alkyl, C3~C6 cycloalkyl, 3- to 6-membered aliphatic heterocyclyl, $-OR^g$, $-C(O)OR^g$, $-SR^g$ or $-NR^hR^i$, when the '-----' in ring E is absent, $R^3$ can also be a carbonyl group that together formed by the connected carbon atom, wherein the alkyl, cycloalkyl, aliphatic heterocyclyl are optionally substituted by one or more $R^{25}$, each occurrence of the $R^{25}$ is independently selected from halogen, cyano, C1-C6 alkyl, C3-C6 cycloalkyl, $-OR^g$ or $-NR^hR^i$;

further, $R^3$ is selected from hydrogen, halogen, cyano, C1-C6 alkyl, $-OR^g$, $-C(O)OR^g$, $-SR^g$ or $-NR^hR^i$, when the '-----' in ring E is absent, $R^3$ can also be a carbonyl group that together formed by the connected carbon atom, wherein the alkyl is optionally substituted by one or more halogen or -OH;
further, $R^3$ is selected from hydrogen, halogen, C1~C3 alkyl, $-OR^g$ or $-NR^hR^i$, when the '-----' in ring E is absent, $R^3$ can also be a carbonyl group that together formed by the connected carbon atom, wherein the alkyl is optionally substituted by one or more $R^{25}$, each occurrence of $R^{25}$ is independently selected from halogen, cyano, C1-C3 alkyl, $-OR^g$ or $-NR^hR^i$;
further, $R^3$ is selected from hydrogen, F, Cl, Br, methyl, $-OR^g$ or $-NR^hR^i$, when the '-----' in ring E is absent, $R^3$ forms carbonyl group together with the connected carbon atom;
further, $R^3$ is selected from hydrogen, Cl, methyl or $-OR^g$, when the '-----' in ring E is absent, $R^3$ forms carbonyl group together with the connected carbon atom;
further, $R^3$ is selected from hydrogen, Cl or $-OR^g$;
further, $R^3$ is selected from hydrogen, Cl, $-CF_3$, $-OCH_3$, $-OCH_2CH_3$, $-OCF_3$, $-OCHF_2$, $-N(CH_3)_2$, $-CH_3$, -CHzOH, $-OCH_2CF_3$, -OH, $-NHCH_3$, $-SCH_3$, $-OCD_3$, -CN or $-C(O)OCH_3$;
further, $R^3$ is selected from hydrogen, $-OCH_3$, $-OCH_2CH_3$, $-N(CH_3)_2$, -CHzOH, $-OCH_2CF_3$, $-SCH_3$, $-OCD_3$, -CN or - $C(O)OCH_3$; preferably, $R^3$ is $-OCH_3$.

**22.** The compound according to any one of claims 1 to 21, wherein each occurrence of $R^g$, $R^h$, $R^i$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino and C1~C6 alkyl;

further, each occurrence of $R^g$, $R^h$, $R^i$ is independently selected from H, C1-C3 alkyl, cyclopropyl or cyclopentyl, wherein the alkyl, cyclopropyl are optionally substituted by one or more of the following substituents: halogen, C1~C3 alkyl;
further, each occurrence of $R^g$, $R^h$, $R^i$ is independently selected from H, methyl, cyclopropyl or cyclopentyl, preferably methyl.

**23.** The compound according to any one of claims 1 to 22, wherein each occurrence of $R^g$, $R^h$, $R^i$ is independently selected from H, methyl, cyclopropyl, cyclopentyl, ethyl, trifluoromethyl, trifluoroethyl, difluoromethyl or deuterated methyl;
preferably, each occurrence of $R^g$, $R^h$, $R^i$ is independently selected from H, methyl, cyclopropyl, cyclopentyl or ethyl;
preferably, each occurrence of $R^g$, $R^h$, $R^i$ is independently selected from H, methyl, cyclopropyl or ethyl.

**24.** The compound according to any one of claims 1 to 23, wherein $R^8$, $R^9$, $R^{10}$, $R^{29}$ are independently selected from hydrogen, halogen, cyano, C1~C6 alkyl, C3~C6 cycloalkyl, $-OR^q$ or $-NR^rR^s$, wherein the alkyl, cycloalkyl are optionally substituted by one or more $R^{31}$;

each occurrence of $R^{31}$ is independently selected from halogen, cyano, C1~C6 alkyl, C3~C6 cycloalkyl, $-OR^q$, - $NR^rR^s$, $-C(O)R^{15}$, $-C(O)NR^rR^s$ or $-C(O)OR^q$;
further, $R^8$, $R^9$, $R^{10}$, $R^{29}$ are independently selected from hydrogen, halogen, cyano, C1~C3 alkyl or $-OR^q$, wherein the alkyl, cycloalkyl are optionally substituted by one or more $R^{31}$;
each occurrence of $R^{31}$ is independently selected from halogen, C1~C3 alkyl or $-OR^q$;
further, $R^8$, $R^{29}$ are independently selected from hydrogen, F, Cl, Br, C1~C3 alkyl, halogenated C1~C3 alkyl, -CN or $-OR^q$, preferably hydrogen, Cl or OH;

$R^9$, $R^{10}$ is hydrogen;

further, $R^8$ is selected from hydrogen, Cl or $-CH_3$, preferably hydrogen; $R^{29}$ is selected from hydrogen, OH, -CN, F or $-CF_3$, preferably, $R^{29}$ is selected from hydrogen, OH, -CN or F; more preferably $R^{29}$ is hydrogen.

**25.** The compound according to any one of claims 1 to 24, wherein each occurrence of $R^q$, $R^r$, $R^s$ is independently selected from H, C1~C6 alkyl or C3~C6 cycloalkyl, wherein the alkyl, cycloalkyl are optionally substituted by one or more of the following substituents: halogen, cyano, hydroxy, amino, C1~C6 alkyl;
further, each occurrence of $R^q$, $R^r$, $R^s$ is independently selected from H or C1~C3 alkyl, preferably H.

**26.** The compound according to any one of claims 1 to 25, wherein $R^4$ is selected from C1~C6 alkyl, C3~C9 cycloalkyl, 3- to 6-membered heterocycloalkyl, 6- to 10-membered aryl or 5- to 10-membered heteroaryl, wherein the alkyl, cycloalkyl, heterocycloalkyl, aryl, heteroaryl are optionally substituted by one or more $R^{26}$;

each occurrence of $R^{26}$ is independently selected from halogen, cyano, C1~C6 alkyl, C3~C6 cycloalkyl, $-OR^j$, - $NR^kR^m$, $-C(O)R^{27}$, $-C(O)NR^kR^m$ or $-C(O)OR^j$, wherein the alkyl, cycloalkyl are optionally substituted by one or more $R^{28}$;

each occurrence of $R^{28}$ is independently selected from halogen, cyano, alkyl, cycloalkyl, $-OR^j$ or $-NR^kR^m$;

further, $R^4$ is selected from C1~C3 alkyl, C3~C6 cycloalkyl, phenyl, 5- to 6-membered nitrogen-containing heteroaryl or 5- to 6-membered sulphur-containing heteroaryl, wherein the alkyl, cycloalkyl, aryl, nitrogen-containing heteroaryl, sulphur-containing heteroaryl are optionally substituted by 1~3 $R^{26}$;

each occurrence of $R^{26}$ is independently selected from halogen, cyano, C1~C3 alkyl, C3~C6 cycloalkyl, $-OR^j$ or - $NR^kR^m$;

further, $R^4$ is selected from C1~C3 alkyl, C3~C6 cycloalkyl, phenyl, naphthyl, 5- to 6-membered nitrogen-containing heteroaryl or 5- to 6-membered sulphur-containing heteroaryl, wherein the alkyl, cycloalkyl, aryl, nitrogen-containing heteroaryl, sulphur-containing heteroaryl are optionally substituted by 1~3 $R^{26}$;

each occurrence of $R^{26}$ is independently selected from halogen, cyano, C1~C3 alkyl, halogenated C1~C3 alkyl, - O(C1~C3 alkyl) or -C(O)OH;

further, $R^4$ is selected from C1~C3 alkyl, cyclohexyl, phenyl, pyridyl, thienyl, naphthyl, pyrrolyl or thiazolyl, wherein the alkyl, cyclohexyl, phenyl, pyridyl, thienyl, naphthyl, pyrrolyl, thiazolyl are optionally substituted by 1~2 $R^{26}$;

each occurrence of $R^{26}$ is independently selected from F, Cl, Br, C1~C3 alkyl or $-OR^j$, preferably F, Cl, methyl or methoxy;

each occurrence of $R^{26}$ is independently selected from halogen, cyano, C1~C3 alkyl, C3~C6 cycloalkyl, $-OR^j$ or - $NR^kR^m$, wherein the alkyl, cycloalkyl are optionally substituted by 1~3 $R^{28}$;

each occurrence of $R^{28}$ is independently selected from halogen, cyano, C1~C3 alkyl, $-OR^j$ or $-NR^kR^m$;

further, each occurrence of $R^{26}$ is independently selected from F, Cl, Br, cyano, unsubstituted C1~C3 alkyl or C1~C3 alkyl substituted by 1 to 3 halogen, $-OR^j$, $-C(O)OR^j$, more preferably F, Cl, methyl, methoxy, cyano, trifluoromethyl or -COOH;

further, $R^4$ is selected from C1~C3 alkyl, cyclohexyl, phenyl, pyrrolyl or thiazolyl, wherein the alkyl, cyclohexyl, phenyl, pyrrolyl, thiazolyl are optionally substituted by 1~2 $R^{26}$;

each occurrence of $R^{26}$ is independently selected from F, Cl, Br, C1~C3 alkyl or $-OR^j$, preferably F, Cl, methyl or methoxy;

further, $R^4$ is selected from methyl, cyclohexyl,

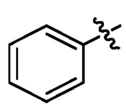 , 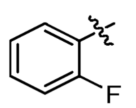 , 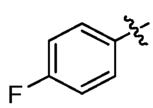 , 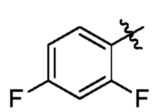 ,

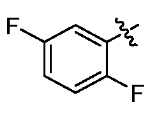 , 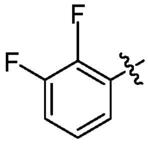 , 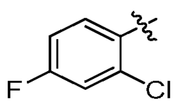 , 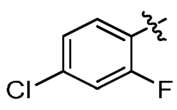 , 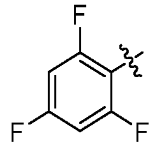 , 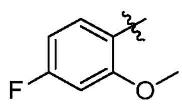 ,

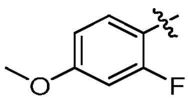, 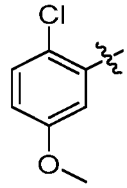, 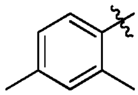, 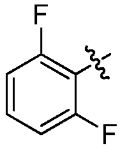, 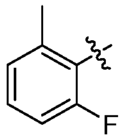, 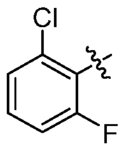, 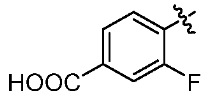,

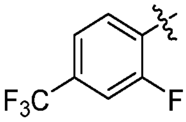, 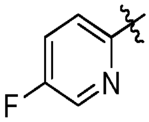, 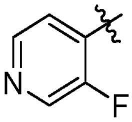, 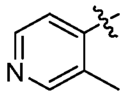, 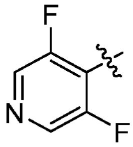, 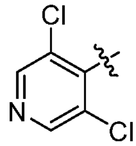, 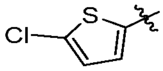,

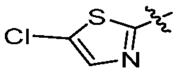, 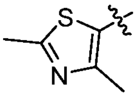 or 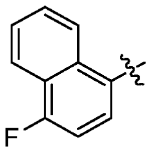,

preferably methyl, cyclohexyl,

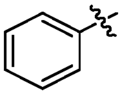, 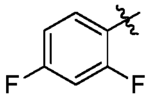,

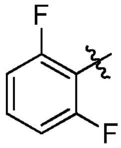 or 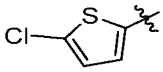,

more preferably

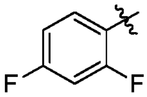 or 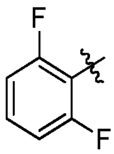;

preferably, $R^4$ is selected from

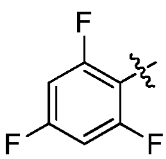, 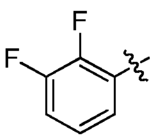, 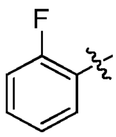, 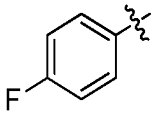, 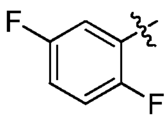,

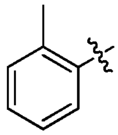, 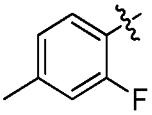, 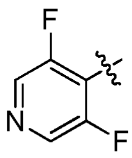 or 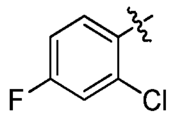;

preferably, $R^4$ is selected from

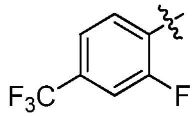, 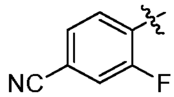 or 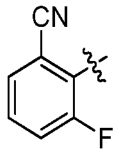.

**27.** The compound according to claim 26, wherein the $R^4$ is selected from naphthyl or pyrrolyl, wherein the naphthyl, pyrrolyl are optionally substituted by 1~3 $R^{26}$; $R^{26}$ is as defined in claim 24 or 26;

further, wherein the $R^4$ is selected from unsubstituted following group or following group substituted by 1 to 3 $R^{26}$:

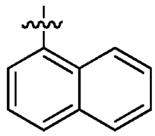, 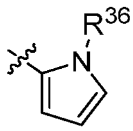,

$R^{36}$ is selected from H or C1~C6 alkyl, preferably C1~C3 alkyl, more preferably methyl;
further, wherein the $R^4$ is selected from

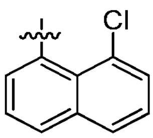 or 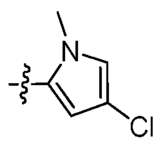.

**28.** The compound according to any one of claims 1 to 27, wherein, each occurrence of $R^j$, $R^k$, $R^m$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino and C1~C6 alkyl; further, each occurrence of $R^j$, $R^k$, $R^m$ is independently selected from H or C1~C3 alkyl, preferably C1~C3 alkyl, more preferably methyl.

**29.** The compound according to any one of claims 1 to 28, wherein each occurrence of $R^7$ is independently selected from halogen, cyano, C1~C6 alkyl, $-OR^n$, $-NR^oR^p$, $-C(O)R^{20}$, $-C(O)NR^oR^p$ or $-C(O)OR^n$, wherein the alkyl is optionally substituted by one or more $R^{21}$;

each occurrence of $R^{21}$ is independently selected from halogen, cyano, C1~C6 alkyl, $-OR^n$, $-NR^oR^p$, $-C(O)R^{20}$, - $C(O)NR^oR^p$ or $-C(O)OR^n$;
further, each occurrence of $R^7$ is independently selected from halogen, cyano, C1~C3 alkyl, $-OR^n$ or $-NR^oR$, wherein the alkyl is optionally substituted by one or more $R^{21}$;
each occurrence of $R^{21}$ is independently selected from halogen, cyano, C1~C3 alkyl, $-OR^n$ or $-NR^oR^p$;
further, each occurrence of $R^7$ is independently selected from C1~C3 alkyl, wherein the alkyl is optionally substituted by one or more $R^{21}$;
each occurrence of $R^{21}$ is independently selected from halogen, cyano or C1~C3 alkyl, preferably cyano;
further, $R^7$ is selected from hydrogen, methyl or $-CH_2CN$;
further,

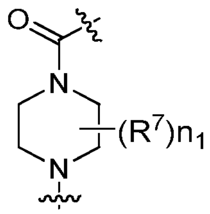

is selected from

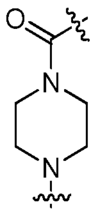 , 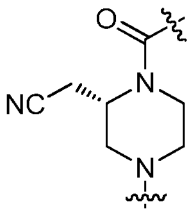 , 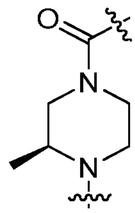 , 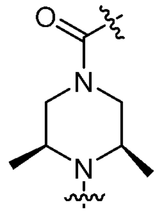

or

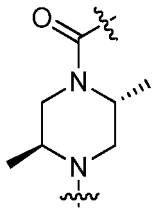 ;

preferably,

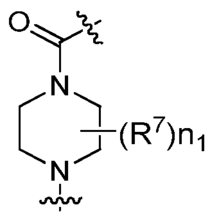

is

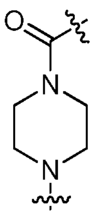 .

**30.** The compound according to any one of claims 1 to 29, wherein each occurrence of $R^{7'}$ is independently selected from halogen, cyano, C1~C6 alkyl, $-OR^n$, $-NR^oR^p$, $-C(O)R^{20}$ or $-C(O)OR^n$, wherein the alkyl is optionally substituted by 1~3 $R^{21}$;

each occurrence of $R^{21}$ is independently selected from halogen, cyano, C1~C6 alkyl, $-OR^n$ or $-NR^oR^p$;
further, each occurrence of $R^{7'}$ is independently selected from halogen, cyano, C1~C3 alkyl, $-OR^n$ or $-NR^oR$, wherein the alkyl is optionally substituted by 1~2 $R^{21}$;
each occurrence of $R^{21}$ is independently selected from halogen, cyano, C1~C3 alkyl, $-OR^n$ or $-NR^oR^p$;

further, $n_5$ is selected from 0, 1, 2 or 3, preferably 0, 1 or 2, more preferably 0 or 1.

**31.** The compound according to any one of claims 1 to 30, wherein each occurrence of $R^n$, $R^o$, $R^p$ is independently selected from H, C1~C6 alkyl or C3~C6 cycloalkyl, wherein the alkyl, cycloalkyl are optionally substituted by one or more substituents selected from the group consisting of: halogen, cyano, hydroxy, amino and C1~C6 alkyl; further, each occurrence of $R^n$, $R^o$, $R^p$ is independently selected from H or C1~C3 alkyl.

**32.** The compound according to any one of claims 1 to 31, wherein each occurrence of $R^{16}$, $R^{17}$, $R^{19}$, $R^{20}$, $R^{22}$, $R^{24}$, $R^{27}$, $R^{30}$, $R^{32}$ is independently selected from H or C1~C6 alkyl, wherein the alkyl is optionally substituted by one or more substituents selected from the group consisting of: halogen, cyano, hydroxy, amino and C1~C6 alkyl; further, each occurrence of $R^{16}$, $R^{17}$, $R^{19}$, $R^{20}$, $R^{22}$, $R^{24}$, $R^{27}$, $R^{30}$, $R^{32}$ is independently selected from H or C1~C3 alkyl, wherein the alkyl is optionally substituted by one or more substituents selected from the group consisting of:

halogen and hydroxy, amino, C1~C3 alkyl;
further, each occurrence of $R^{16}$, $R^{17}$, $R^{19}$, $R^{20}$, $R^{22}$, $R^{24}$, $R^{27}$, $R^{30}$, $R^{32}$ is independently selected from C1~C3 alkyl, preferably methyl or propyl.

**33.** The compound according to any one of claims 1 to 32, wherein the compound has a structure shown in formula II, or a tautomer, meso isomer, racemate, enantiomer, diastereomer or mixture thereof, or a pharmaceutically acceptable hydrate, solvate or salt thereof:

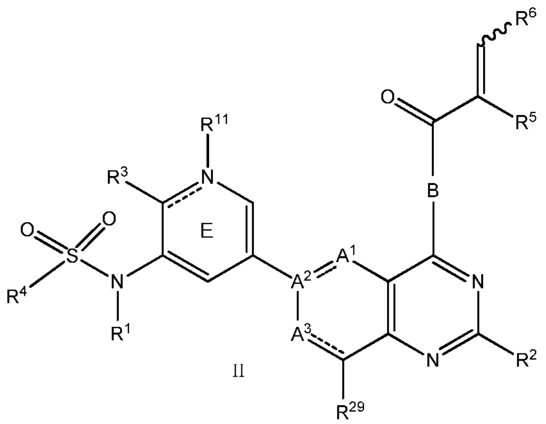

II

wherein

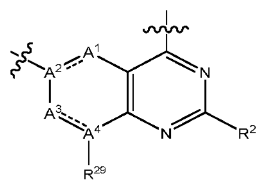

is

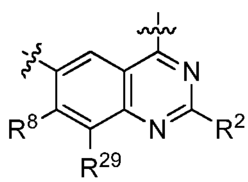

;

B is

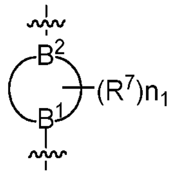

,

the ring where $B^1$, $B^2$ are located is a 6- to 9-membered nitrogen-containing heterocycloalkyl or a 6- to 9-membered nitrogen-containing heterocycloalkenyl, wherein the cycloalkyl or cycloalkenyl is a monocyclic ring, spirocyclic ring or bridged cyclic ring;

$R^1$ is H;
$R^2$ is H;
the '-----' in ring E represemts '-----' is a bond or absent, when '-----' is a bond, $R^{11}$ is absent, ring E is

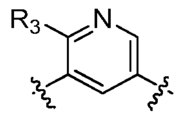

;

when '-----' is absent, ring E is

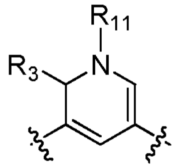

;

when $R^{11}$ exists, $R^{11}$ is selected from hydrogen or methyl;
$R^8$ is selected from hydrogen, Cl or $-CH_3$; preferably $R^8$ is hydrogen;
$R^{29}$ is selected from hydrogen, OH, -CN, F or $-CF_3$; preferably $R^{29}$ is hydrogen;
$R^3$ is selected from hydrogen, halogen, cyano, C1~C6 alkyl, $-OR^g$, $-C(O)OR^g$, $-SR^g$ or $-NR^hR^i$, when the '-----' in ring E is absent, $R^3$ can also be a carbonyl group that together formed by the connected carbon atom, wherein the alkyl is optionally substituted by one or more halogen or -OH;
each occurrence of $R^g$, $R^h$, $R^i$ is independently selected from H, methyl, cyclopropyl, cyclopentyl or ethyl; preferably methyl;
$R^4$ is selected from C1~C3 alkyl, C3~C6 cycloalkyl, phenyl, naphthyl, 5- to 6-membered nitrogen-containing heteroaryl or 5- to 6-membered sulphur-containing heteroaryl, wherein the alkyl, cycloalkyl, aryl, nitrogen-containing heteroaryl, sulphur-containing heteroaryl are optionally substituted by 1~3 $R^{26}$;
each occurrence of $R^{26}$ is independently selected from halogen, cyano, C1~C3 alkyl, halogenated C1~C3 alkyl, - O(C1~C3 alkyl) or -C(O)OH;
$R^5$, $R^6$ are independently selected from hydrogen, halogen, C1~C3 alkyl, 5- to 6-membered heteroaryl, $-C(O)R^{16}$, $-S(O)_2R^{16}$, $-P(O)R^{16}R^{17}$, $-C(O)NR^bR^c$, $-C(O)OR^a$ or $-OR^a$, wherein the alkyl, heteroaryl are optionally substituted by one or more $R^{18}$;
$R^{16}$, $R^{17}$ are independently selected from C1~C3 alkyl;
each occurrence of $R^a$, $R^b$, $R^c$ is independently selected from H and C1~C3 alkyl;
each occurrence of $R^{18}$ is independently selected from halogen, C1~C3 alkyl, $-OR^a$ or $-NR^bR^c$;
each occurrence of $R^7$ is independently selected from C1~C3 alkyl, wherein the alkyl is optionally substituted by one or more substituents independently selected from halogen, cyano or C1~C3 alkyl;
$n_1$ is selected from 0 or 1;
preferably,

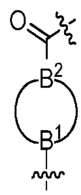

is selected from the following group:

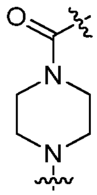, 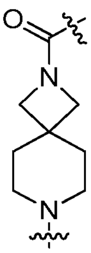, 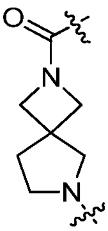, 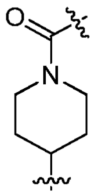, 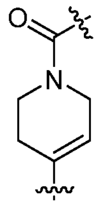 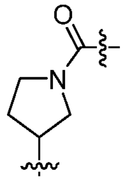, 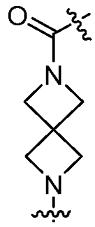, 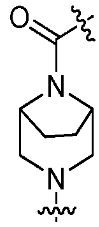, 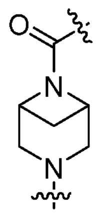, 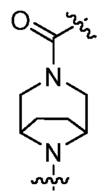,

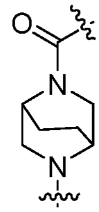 or 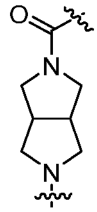.

**34.** The compound according to any one of claims 1 to 33, wherein the compound has a structure shown in formula VIII, or a tautomer, meso isomer, racemate, enantiomer, diastereomer or mixture thereof, or a pharmaceutically acceptable hydrate, solvate or salt thereof:

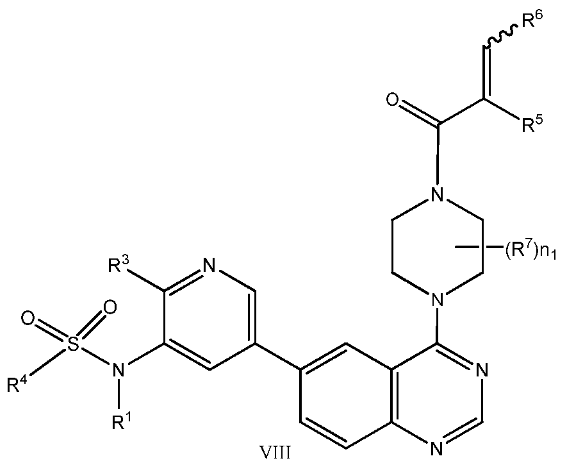

VIII

Wherein,

$R^1$ is H;
$R^3$ is selected from hydrogen, halogen, cyano, C1~C6 alkyl, $-OR^g$, $-C(O)OR^g$, $-SR^g$ or $-NR^hR^i$, wherein the alkyl is optionally substituted by one or more halogen or -OH;
each occurrence of $R^g$, $R^h$, $R^i$ is independently selected from H, methyl, cyclopropyl, cyclopentyl or ethyl; preferably methyl;
$R^4$ is selected from C1~C3 alkyl, cyclohexyl, phenyl, pyridyl, thienyl, naphthyl, pyrrolyl or thiazolyl, wherein alkyl, cyclohexyl, phenyl, pyridyl, thienyl, naphthyl, pyrrolyl, thiazolyl are optionally substituted by 1~2 $R^{26}$;
each occurrence of $R^{26}$ is independently selected from F, Cl, Br or cyano, unsubstituted C1~C3 alkyl or C1~C3 alkyl substituted by 1 to 3 halogen, $-OR^j$, $-C(O)OR^j$;
$R^j$ is selected from H and C1~C3 alkyl;
$R^5$, $R^6$ are independently selected from hydrogen, halogen, C1~C3 alkyl, 5- to 6-membered heteroaryl, $-C(O)R^{16}$, $-S(O)_2R^{16}$, $-P(O)R^{16}R^{17}$, $-C(O)NR^bR^c$, $-C(O)OR^a$ or $-OR^a$, wherein the alkyl, heteroaryl are optionally substituted by one or more $R^{18}$;
$R^{16}$, $R^{17}$ is independently selected from C1~C3 alkyl;
each occurrence of $R^{18}$ is independently selected from halogen, C1~C3 alkyl, $-OR^a$ or $-NR^bR^c$;
each occurrence of $R^a$, $R^b$, $R^c$ is independently selected from H and C1~C3 alkyl;
each occurrence of $R^7$ is independently selected from C1~C3 alkyl, wherein the alkyl is optionally substituted by one or more substituents independently selected from halogen, cyano or C1~C3 alkyl;

$n_1$ is selected from 0 or 1.

**35.** The compound according to claim 34 or a tautomer, meso isomer, racemate, enantiomer, diastereomer or mixture thereof, or a pharmaceutically acceptable hydrate, solvate or salt thereof:
wherein,

$R^1$ is H;
$R^3$ is selected from hydrogen, Cl, $-CF_3$, $-OCH_3$, $-OCH_2CH_3$, $-OCF_3$, $-OCHF_2$, $-N(CH_3)_2$, $-CH_3$, $-CH_2OH$, $-OCH_2CF_3$, -OH, $-NHCH_3$, $-SCH_3$, $-OCD_3$, -CN or$-C(O)OCH_3$;
$R^4$ is selected from methyl, cyclohexyl,

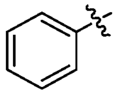, 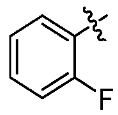, 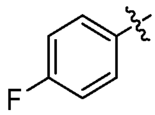, 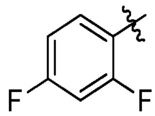, 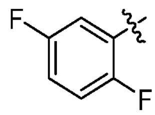,

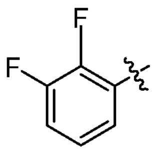, 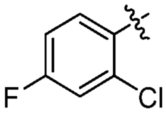, 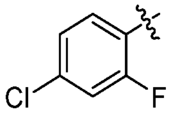, 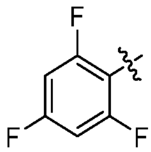, 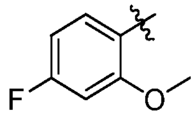, 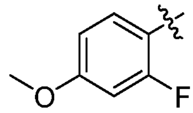, 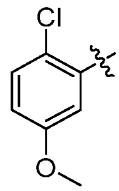,

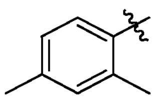, 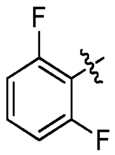, 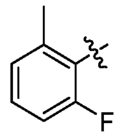, 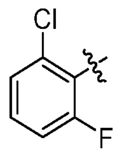, 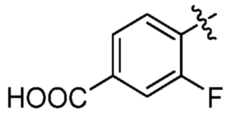, 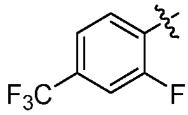, 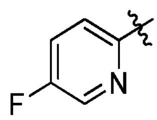,

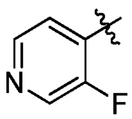, 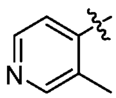, 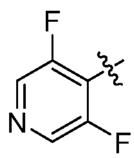, 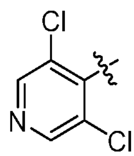 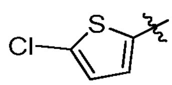, 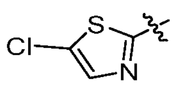, 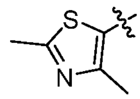

or

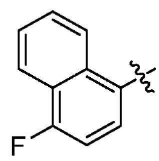;

$R^5$ is selected from H, F, CN, $-CH(CH_3)_2$, $-CH(OH)CH_3$, $-CH_2OCH_3$, $-OCH_3$ or $-C(O)CH_3$;
$R^6$ is selected from H, $-C(CH_3)_3$, $-C(O)CH_3$, $-C(O)CH_2CH_2CH_3$, $-C(O)NHCH_3$, $-CH_2N(CH_3)_2$, $-CH_2F$,

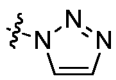, -

$CH_2OCH_3$, $CF_3$,

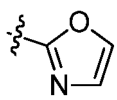
,

$-C(O)OCH_3$, $-P(O)(CH_3)$, $-S(O)_2CH_3$, $-C(O)CF_3$ or $-C(O)CH_2CH_3$;
$R^7$ is selected from hydrogen, methyl or $-CH_2CN$;
$n_1$ is selected from 0 or 1.

**36.** The following compounds, or a tautomer, meso isomer, racemate, enantiomer, diastereomer or mixture thereof, or a pharmaceutically acceptable hydrate, solvate or salt thereof:

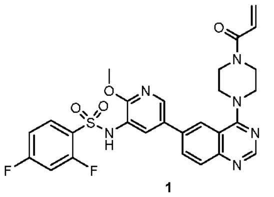
1

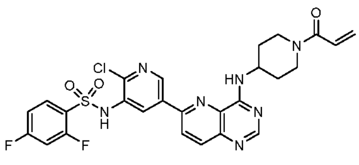
2

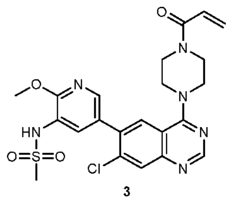
3

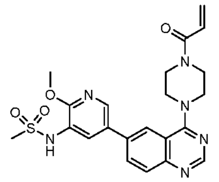
4

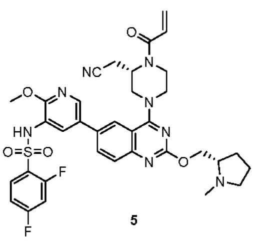
5

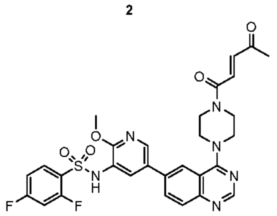
6

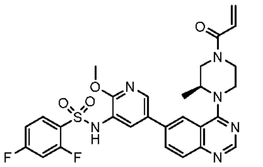
7

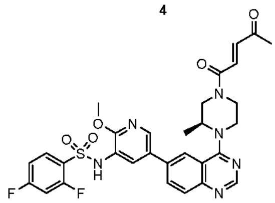
8

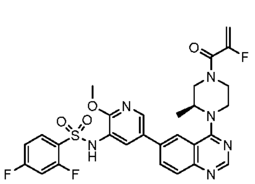
9

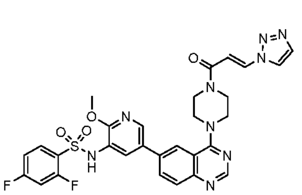
10

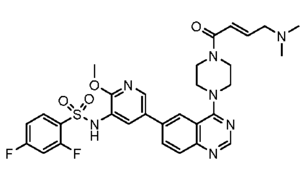
11

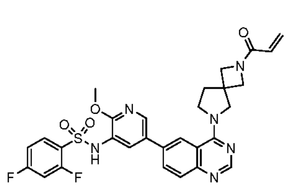
12

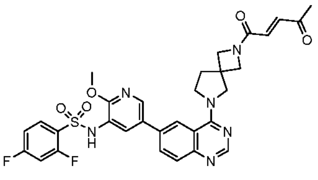
13

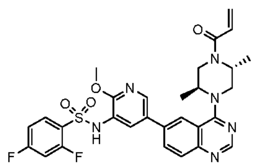
14

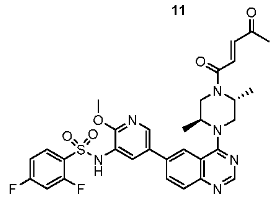
15

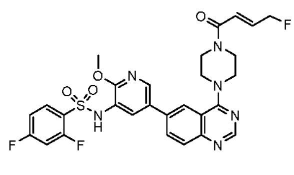
16

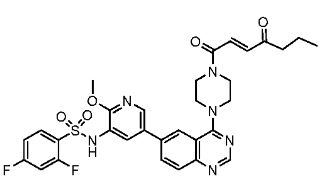
17

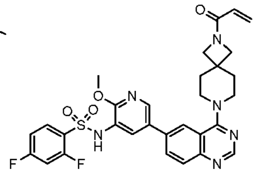
18

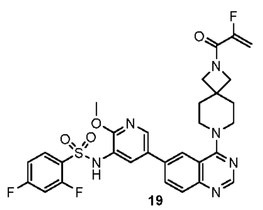
19

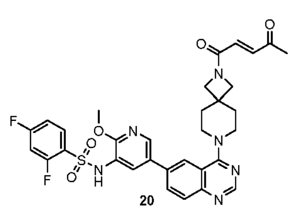
20

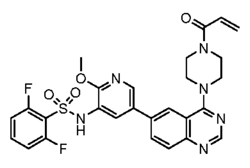
21
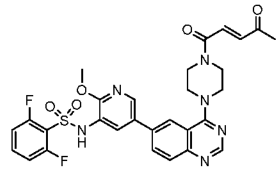
22
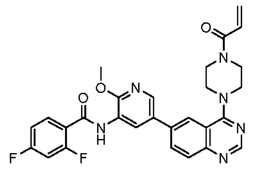
23
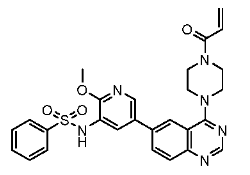
24
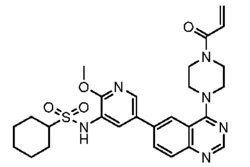
25
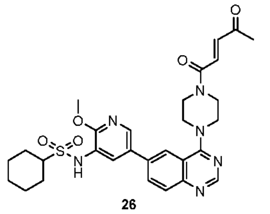
26
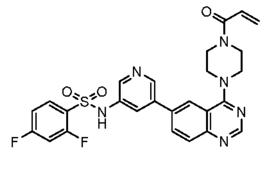
27
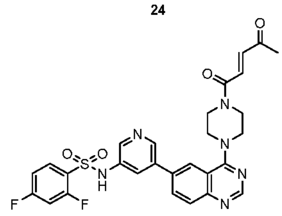
28
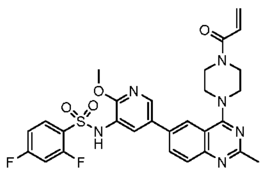
29
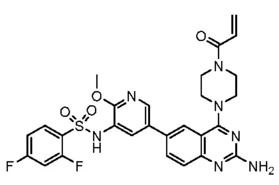
30
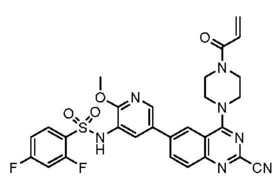
31
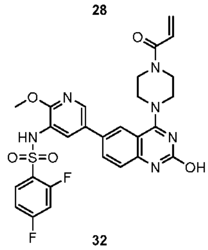
32
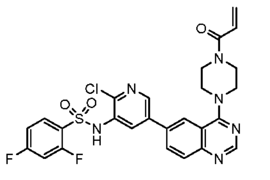
33
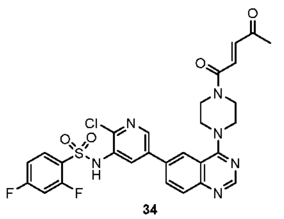
34
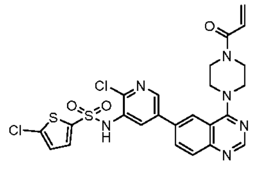
35
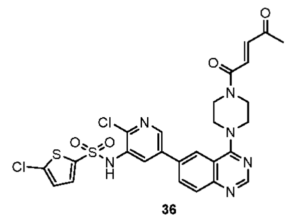
36
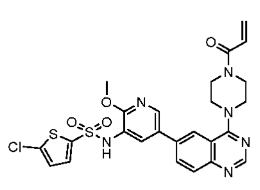
37
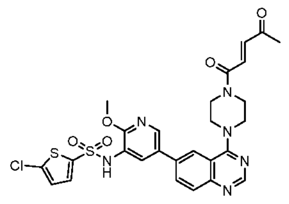
38
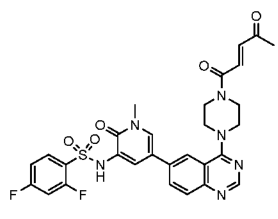
39
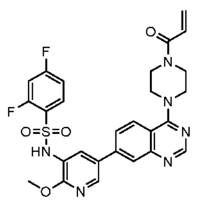
40
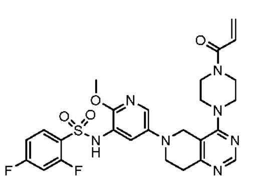
41
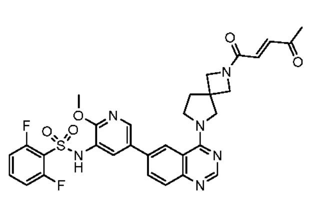
42
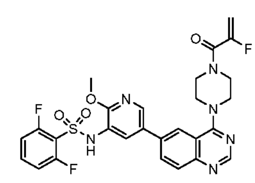
43
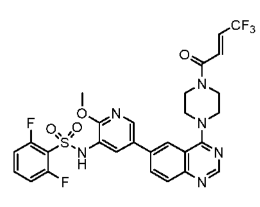
44
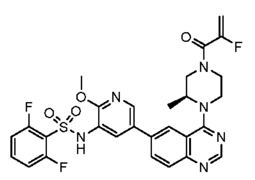
45
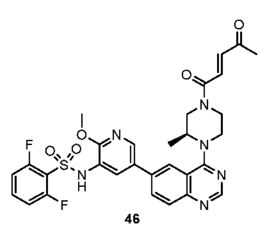
46
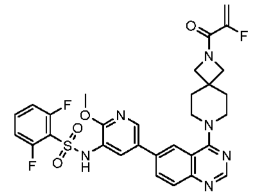
47
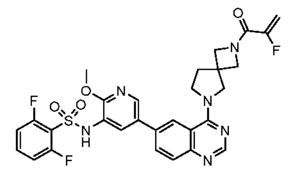
48

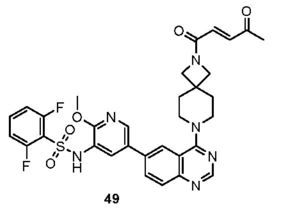
49

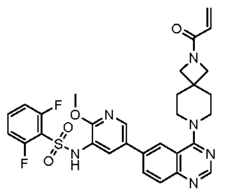
50

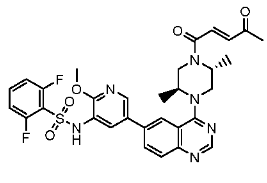
51

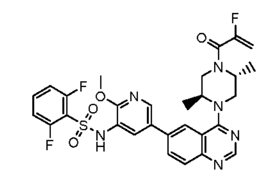
52

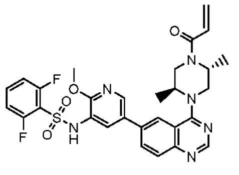
53

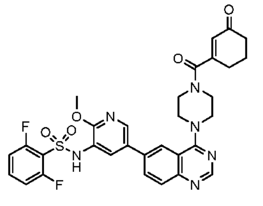
54

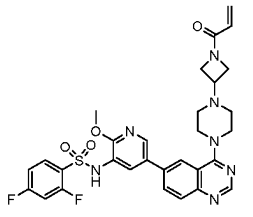
55

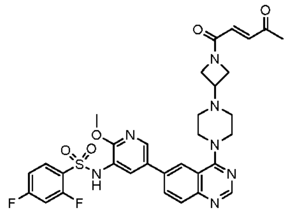
56

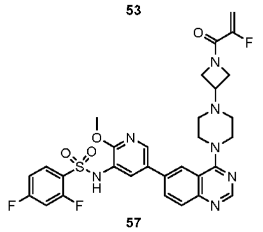
57

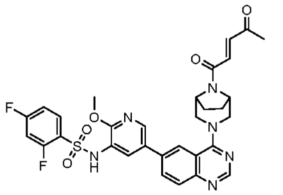
58

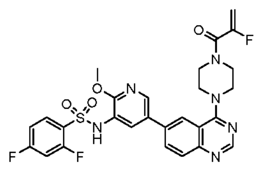
59

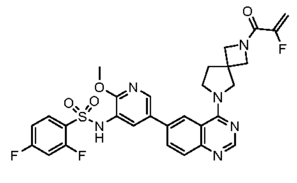
60

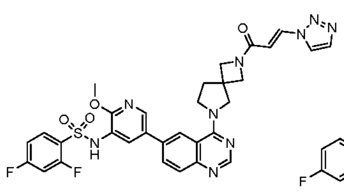
61

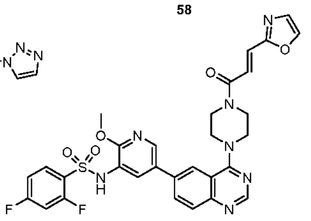
62

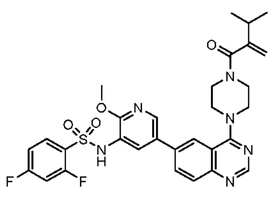
63

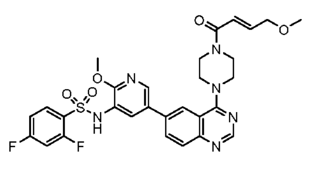
64

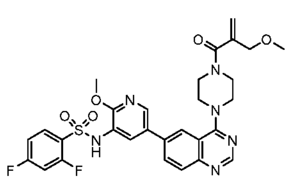
65

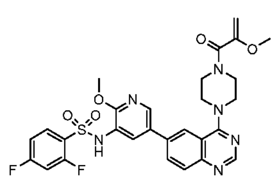
66

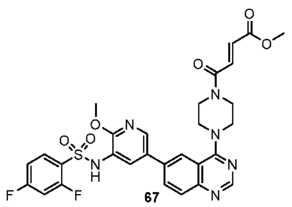
67

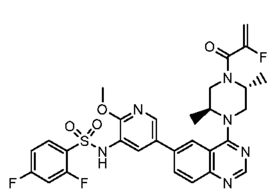
68

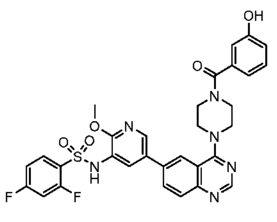
69

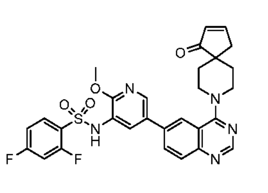
70

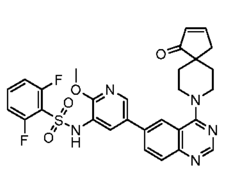
71

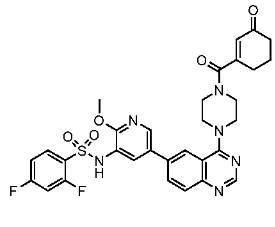
72

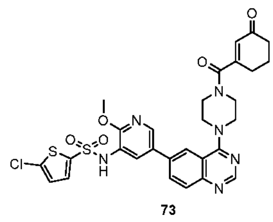
73

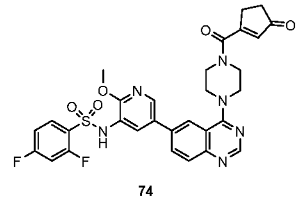
74

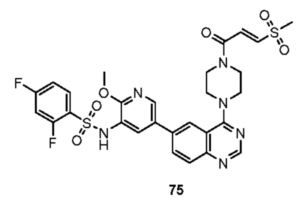
75

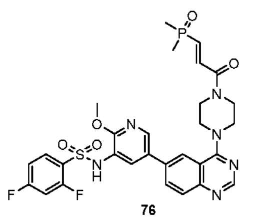
76

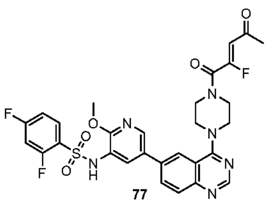
77

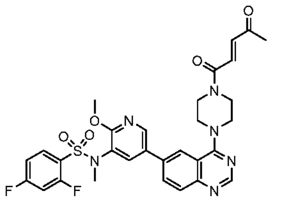
78

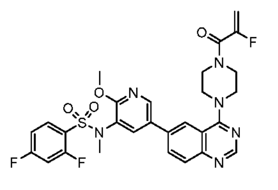
79

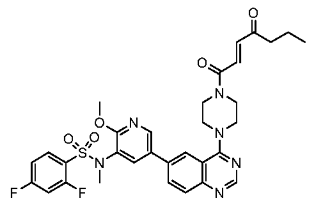
80

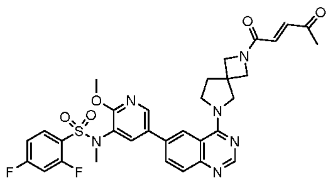
81

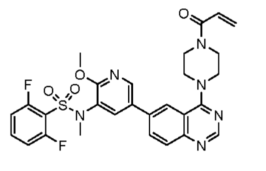
82

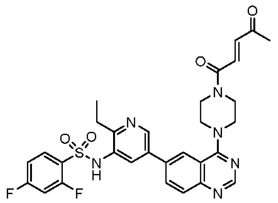
83

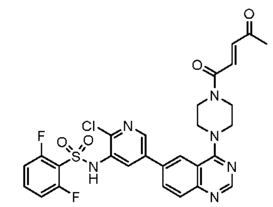
84

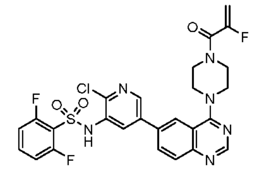
85

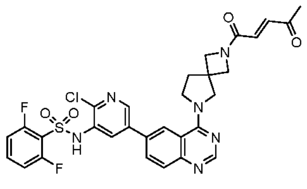
86

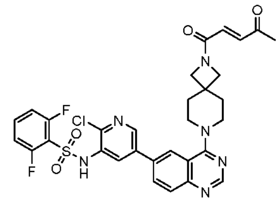
87

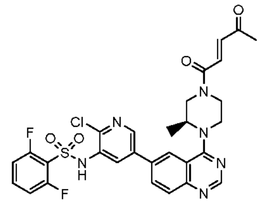
88

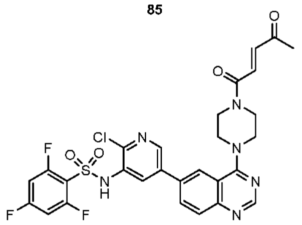
89

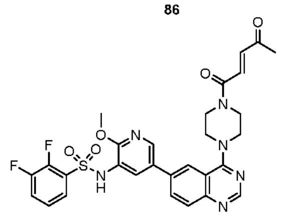
90

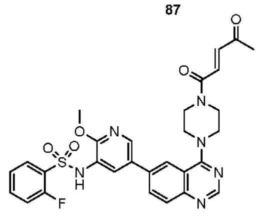
91

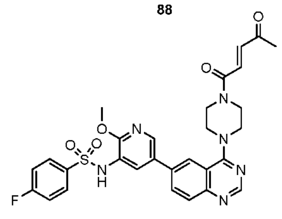
92

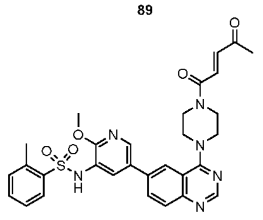
93

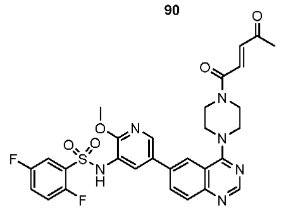
94

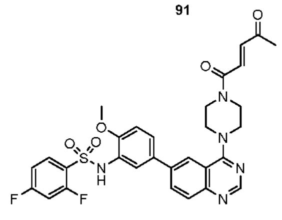
95

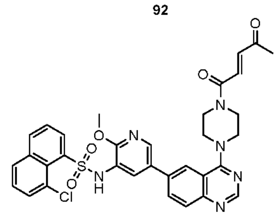
96

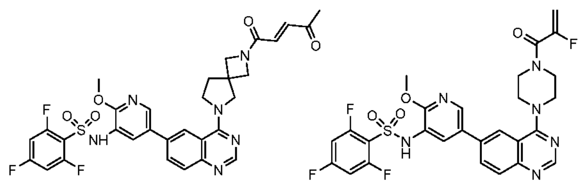
97

98

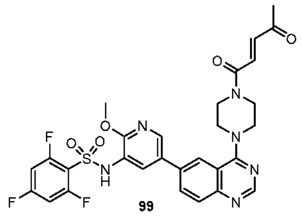
99

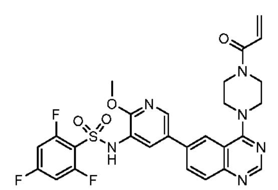
100

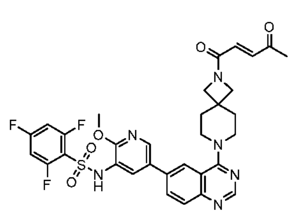

101

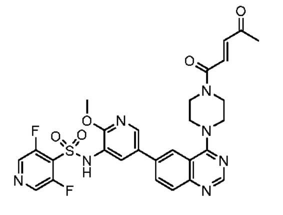

102

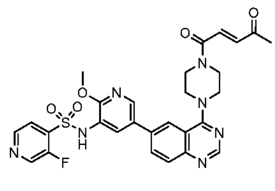

103

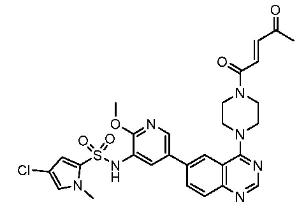

104

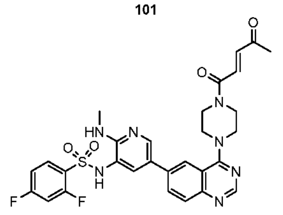

105

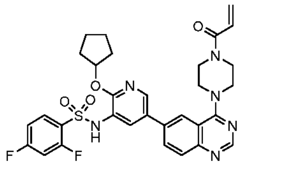

106

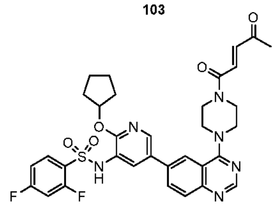

107

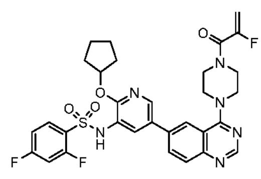

108

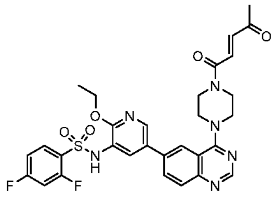

109

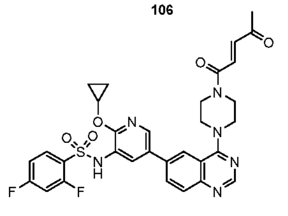

110

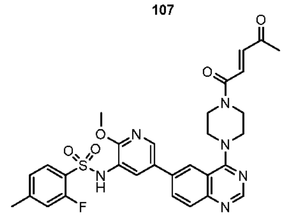

111

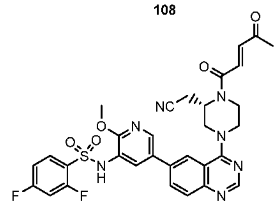

112

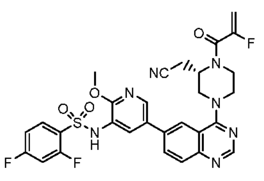

113

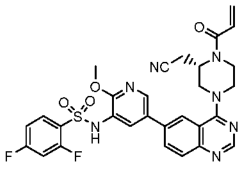

114

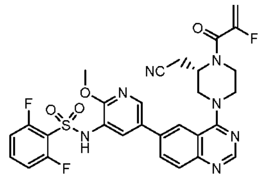

115

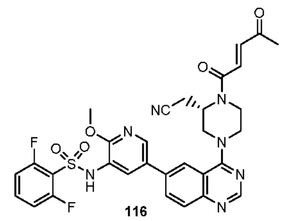

116

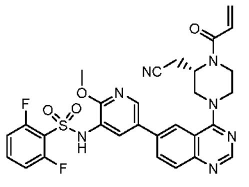

117

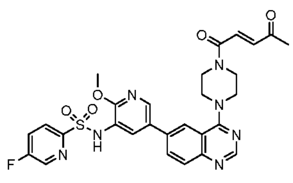

118

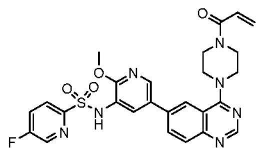

119

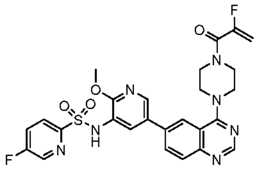

120

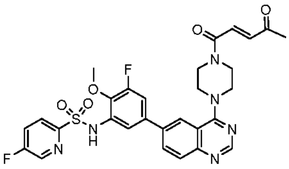

121

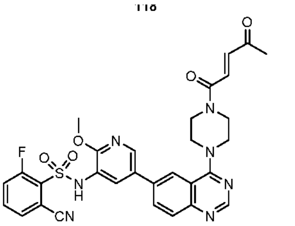

122

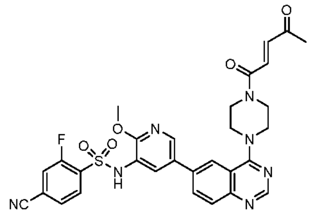

123

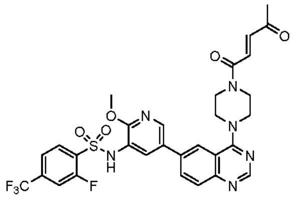

124

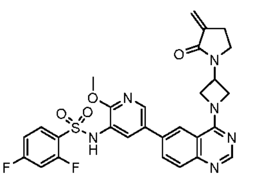

125

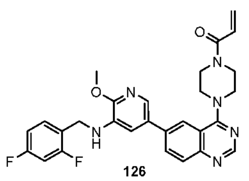

126

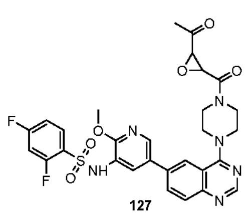

127

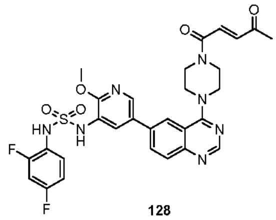

128

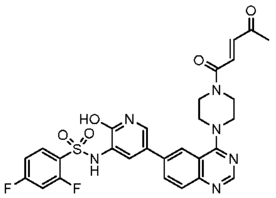
129

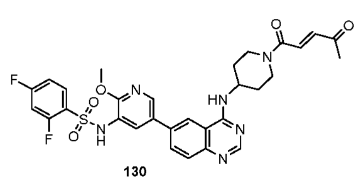
130

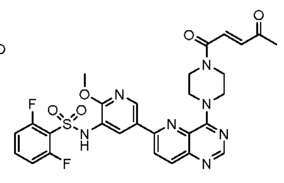
131

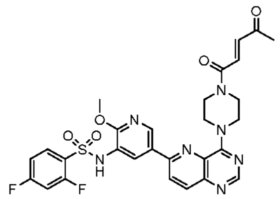
132

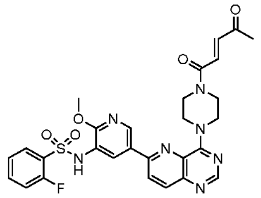
133

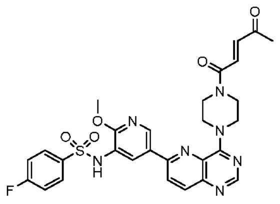
134

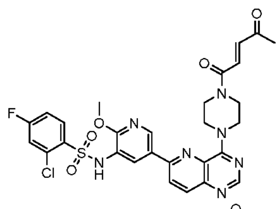
135

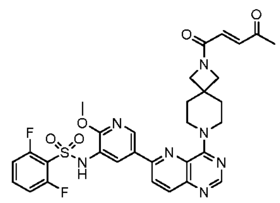
136

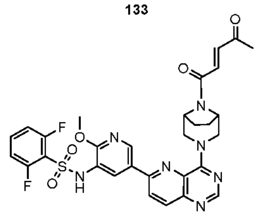
137

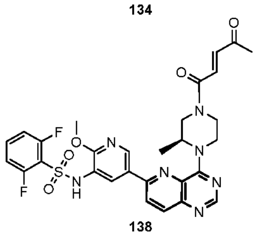
138

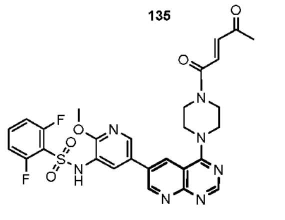
139

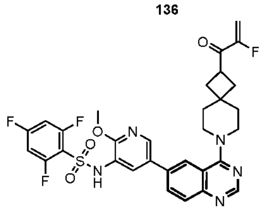
140

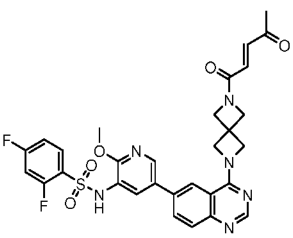
141

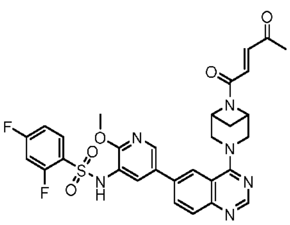
142

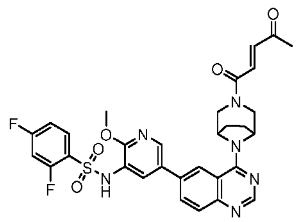
144

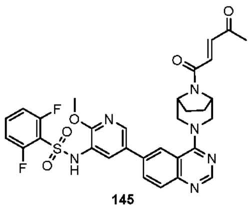
145

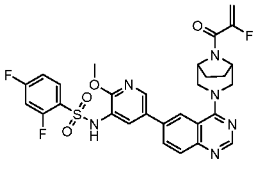
146

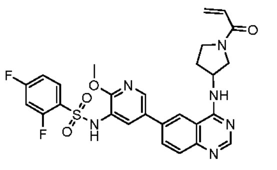
147

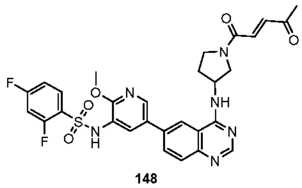
148

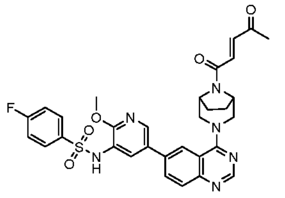
149

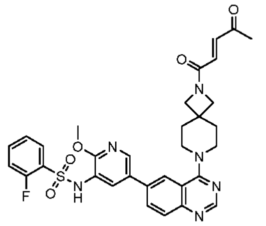
150

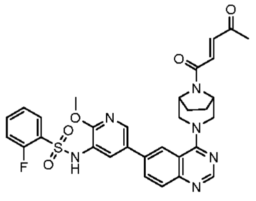
151

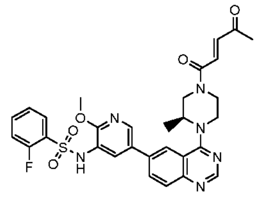
152

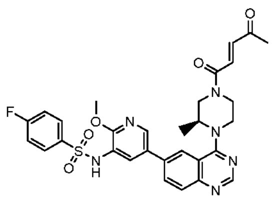
153

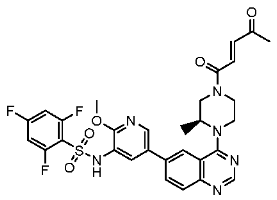
154

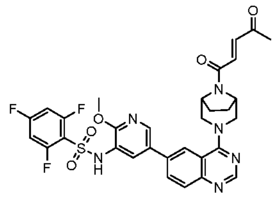
155

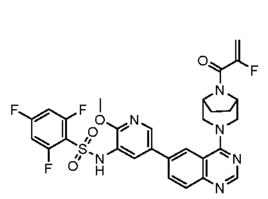
156

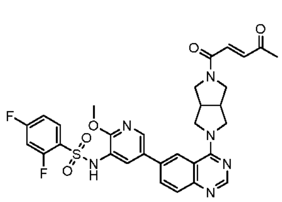
157

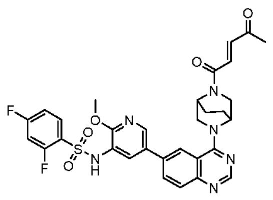
158

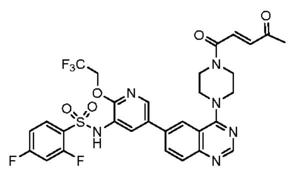
159

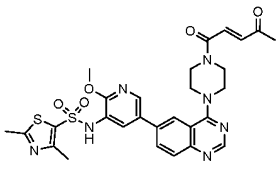
160

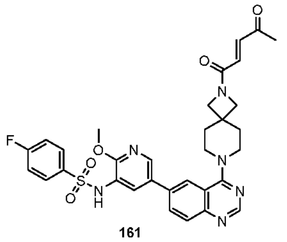
161

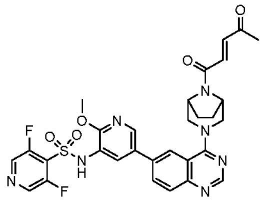
162

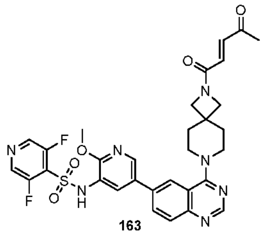
163

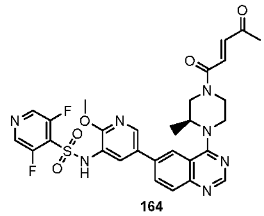
164

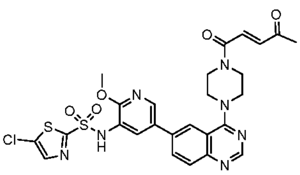
165

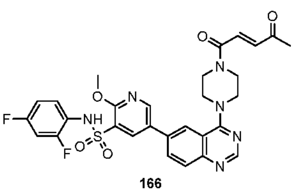
166

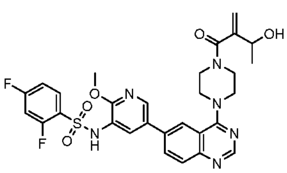
167

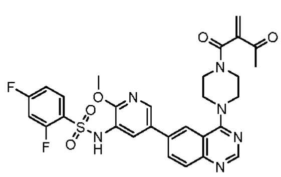
168

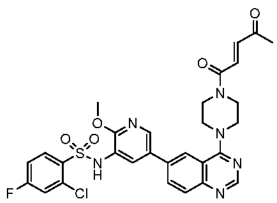
169

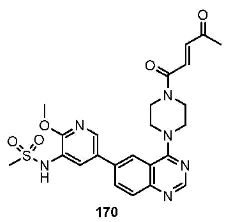
170

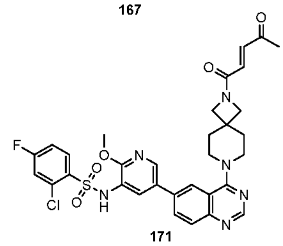
171

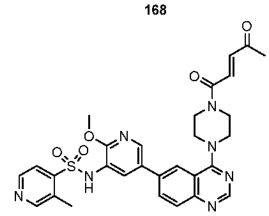
172

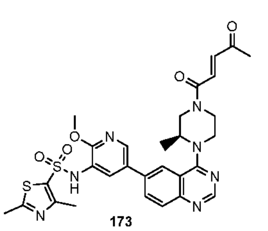
173

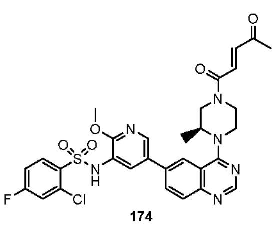
174

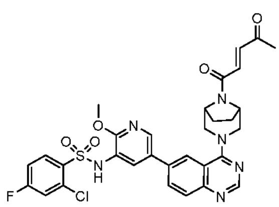
175

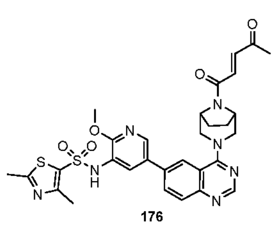
176

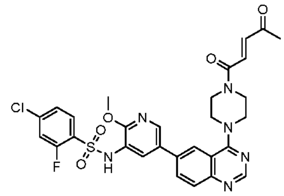
177

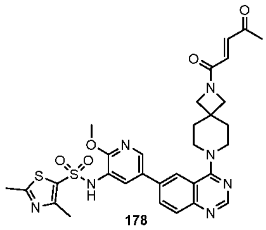
178

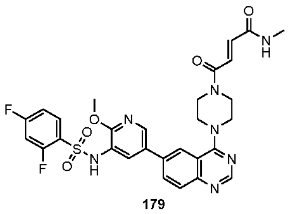
179

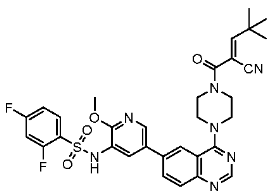
180

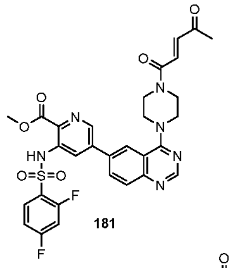
181

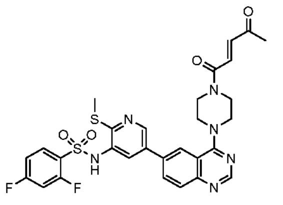
182

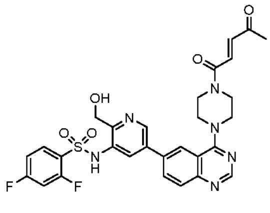
183

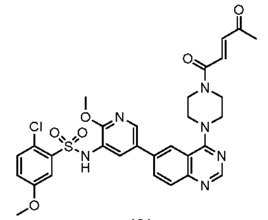
184

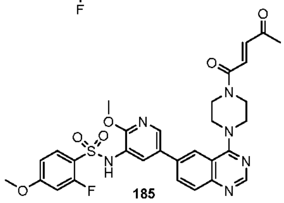
185

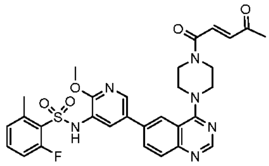
186

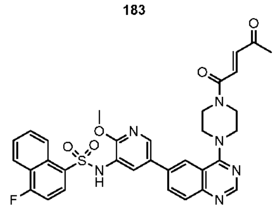
187

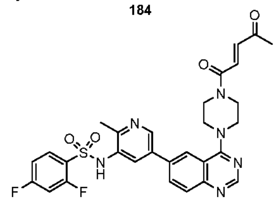
188

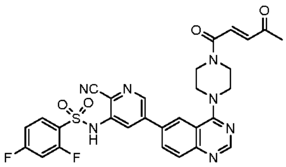
189

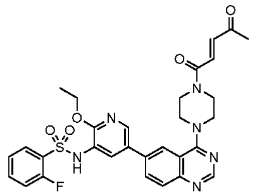
190

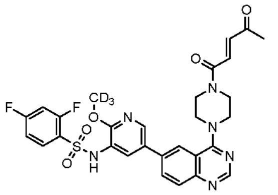
191

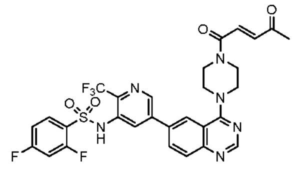
192

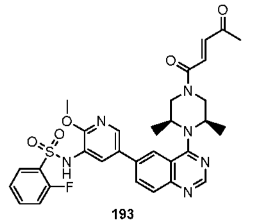
193

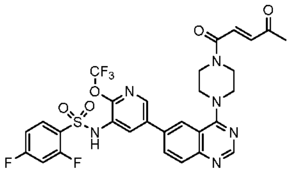
194

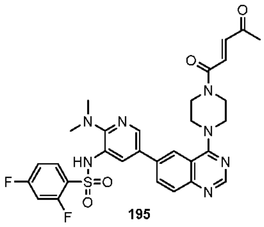
195

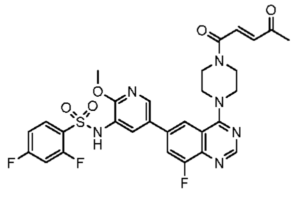
196

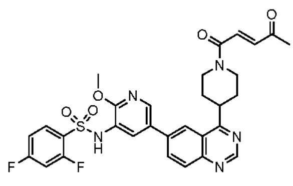
197

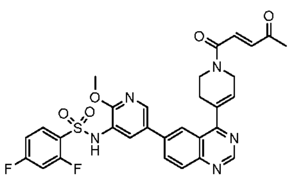
198

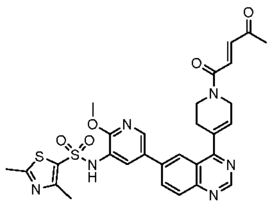
199

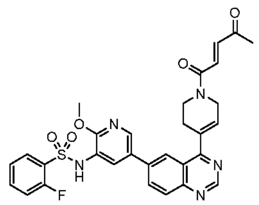
200

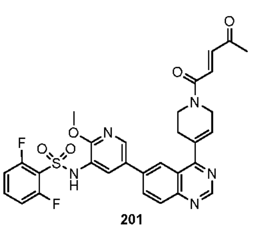
201

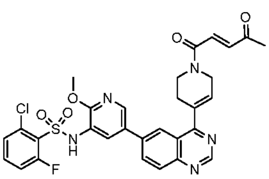
202

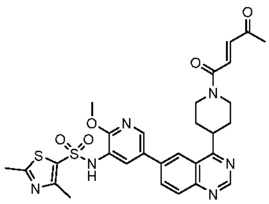
203

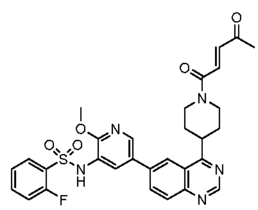
204

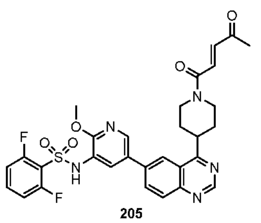
205

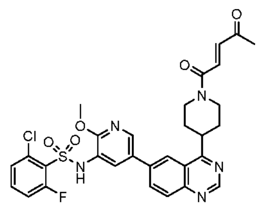
206

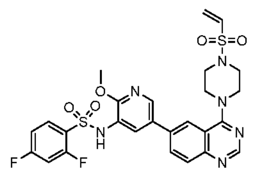
207

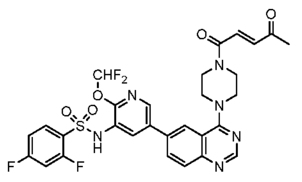
208

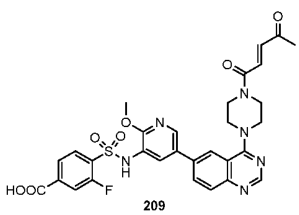
209

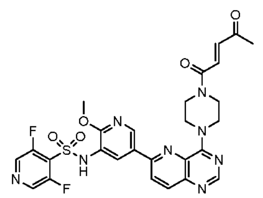
210

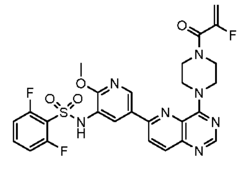
211

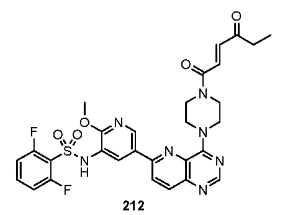
212

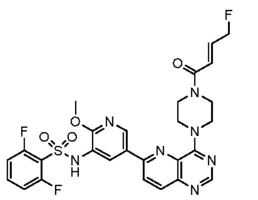
213

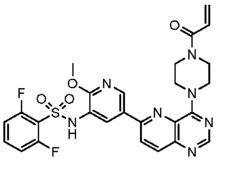
214

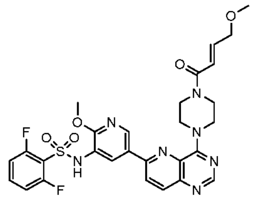
215

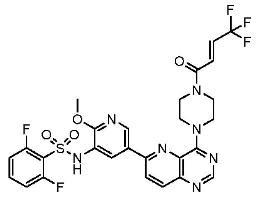
216

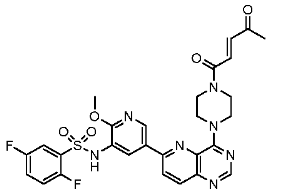
217

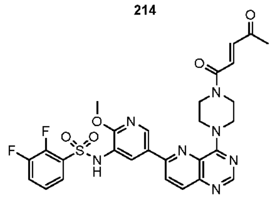
218

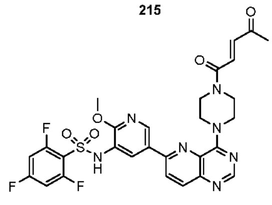
219

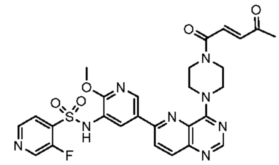
220

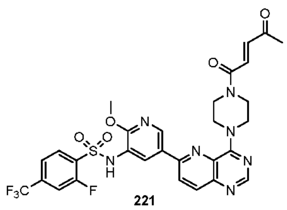
221

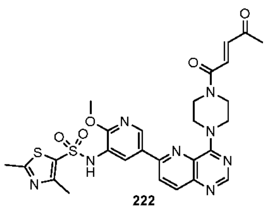
222

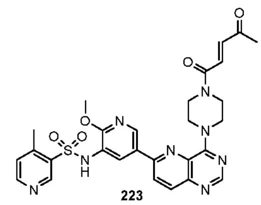
223

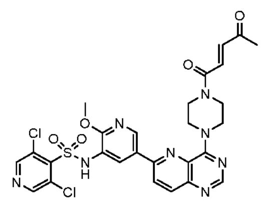
224

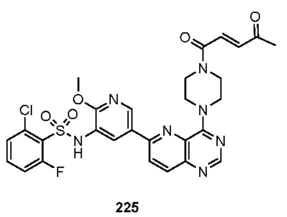
225

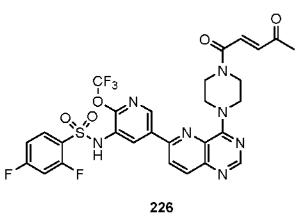
226

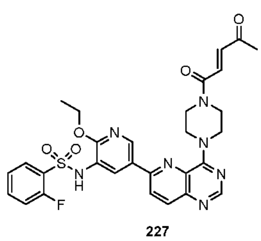
227

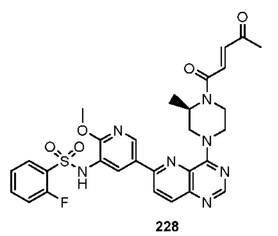
228

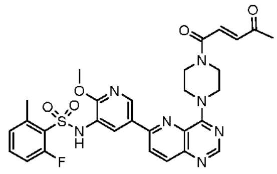
229

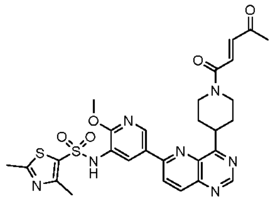
230

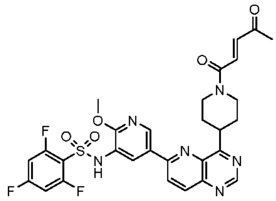
231

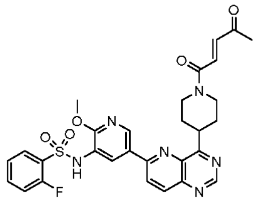
232

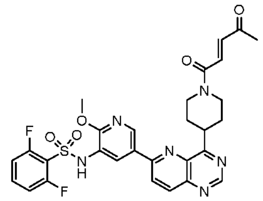
233

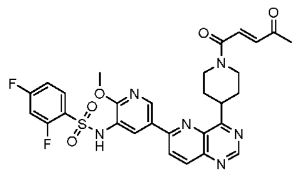
234

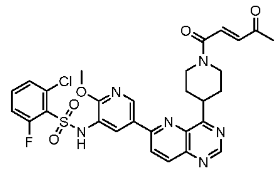
235

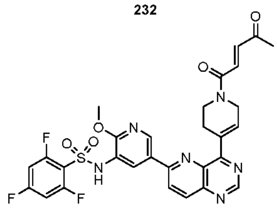
236

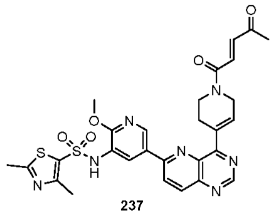
237

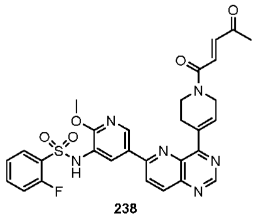
238

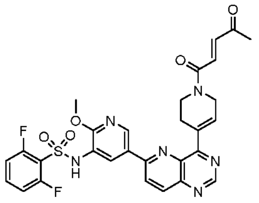
239

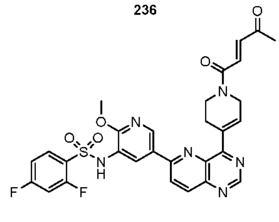
240

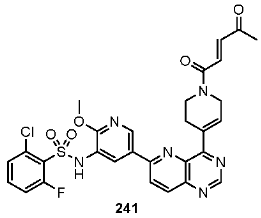
241

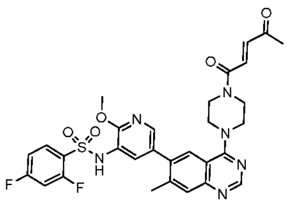
242

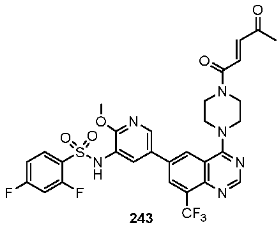
243

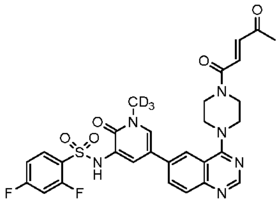
244

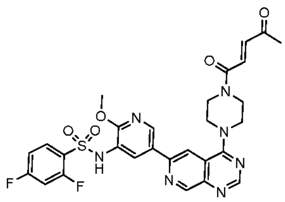
245

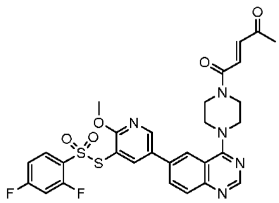
246

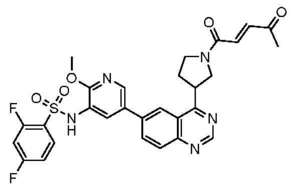
247

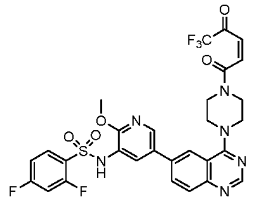
248

**37.** A pharmaceutical composition, wherein the active ingredient of the pharmaceutical composition is selected from one or a combination of two or more of the compound of any one of claims 1 to 36 ,or a tautomer, meso isomer, racemate, enantiomer, diastereomer or mixture thereof, or a pharmaceutically acceptable hydrate, solvate or salt thereof.

**38.** Use of the compound of any one of claims 1 to 36 ,or a tautomer, meso isomer, racemate, enantiomer, diastereomer or mixture thereof, or a pharmaceutically acceptable hydrate, solvate or salt thereof or the pharmaceutical composition of claim 37 in the preparation of KRAS inhibitors and/or PI3K inhibitors;

further, wherein the KRAS inhibitors is selected from KRAS G12C inhibitors, KRAS G12V inhibitors, KRAS G12D inhibitors or KRAS G12S inhibitors, preferably KRAS G12C inhibitors;
wherein the PI3K inhibitors is PI3K$\alpha$ inhibitors and/or PI3K$\delta$ inhibitors.

**39.** Use of the compound of any one of claims 1 to 36 ,or a tautomer, meso isomer, racemate, enantiomer, diastereomer or mixture thereof, or a pharmaceutically acceptable hydrate, solvate or salt thereof or the pharmaceutical composition of claim 37 in the preparation of a medicament for the treatment of a disease mediated by KRAS and/or PI3K;

further, wherein the disease mediated by KRAS and/or PI3K comprises a disease mediated by one or more of KRAS G12C, PI3Kα, and PI3Kδ;
preferably, wherein the disease is cancer or an autoimmune disease;
preferably, wherein the cancer is selected from: non-small cell lung cancer, lung cancer, pancreatic cancer, ovarian cancer, bladder cancer, prostate cancer, chronic myelogenous leukemia, colorectal cancer, brain cancer, liver cancer, kidney cancer, stomach cancer, breast cancer, triple negative breast cancer, skin cancer, melanin Cancer, head and neck cancer, bone cancer, cervical cancer, pelvic cancer, vaginal cancer, oral cancer, lymphoma, blood cancer, esophageal cancer, urethra cancer, nasal cavity cancer.

**40.** Use of the compound of any one of claims 1 to 36, or a tautomer, meso isomer, racemate, enantiomer, diastereomer or mixture thereof, or a pharmaceutically acceptable hydrate, solvate or salt thereof or the pharmaceutical composition of claim 37 in the preparation of a medicament for the treatment of a disease that is resistant to anticancer agents;

further, wherein the anticancer agent is selected from KRAS G12C inhibitors, KRAS G12V inhibitors, KRAS G12D inhibitors, KRAS G12S inhibitors, preferably KRAS G12C inhibitors;
further, wherein the KRAS G12C inhibitors is selected from AMG-510, MRTX-849, preferably AMG-510.

**41.** Use of the compound of any one of claims 1 to 36 ,or a tautomer, meso isomer, racemate, enantiomer, diastereomer or mixture thereof, or a pharmaceutically acceptable hydrate, solvate or salt thereof or the pharmaceutical composition of claim 37 in the preparation of a medicament for the treatment of a disease causing overexpression of PI3K protein and/or KRAS G12C protein.

**42.** Use of the compound of any one of claims 1 to 36 ,or a tautomer, meso isomer, racemate, enantiomer, diastereomer or mixture thereof, or a pharmaceutically acceptable hydrate, solvate or salt thereof or the pharmaceutical composition of claim 37 in the preparation of medicament for the treatment of a disease caused by overexpression of PI3K protein and/or KRAS G12C protein.

## INTERNATIONAL SEARCH REPORT

International application No.
**PCT/CN2022/120154**

**A. CLASSIFICATION OF SUBJECT MATTER**

C07D 401/14(2006.01)i；C07D 239/72(2006.01)i；C07D 471/04(2006.01)i；C07D 403/14(2006.01)i；A61K 31/517(2006.01)i；A61P 35/00(2006.01)i

According to International Patent Classification (IPC) or to both national classification and IPC

**B. FIELDS SEARCHED**

Minimum documentation searched (classification system followed by classification symbols)

C07D，A61K，A61P

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, STN-REGISTRY, STN-CAPLUS, STN-MARPAT: 四川汇宇, 陈寿军, 强晓明, 丁兆, 熊勇, 王海波, 刘科, 柳明登, KARS, PI3K, 抑制, 吡啶, 癌, 肿瘤, inhibit+, +pyridin+, +sulf+, +sulph+, cancer, tumor, stn结构检索, stn structure search

**C. DOCUMENTS CONSIDERED TO BE RELEVANT**

| Category* | Citation of document, with indication, where appropriate, of the relevant passages | Relevant to claim No. |
|---|---|---|
| X | WO 2016044772 A1 (ARAXES PHARMA L.L.C.) 24 March 2016 (2016-03-24)<br>abstract, and claims 1, 29, 34 and 38-46, and description, pp. 4, 61-62 and 213, and table 1 | 1-42 |
| X | US 2018118757 A1 (ARAXES PHARMA L.L.C.) 03 May 2018 (2018-05-03)<br>abstract, and claims 1, 11, 23, 82 and 91-95, and description, paragraphs [0121]-[0140], and table 1 | 1-42 |
| A | CN 106488910 A (ARAXES PHARMA L.L.C.) 08 March 2017 (2017-03-08)<br>abstract, and claims 1 and 82-95, and description, table 1, and pp. 139-141 | 1-42 |
| A | CN 103987699 A (NOVARTIS AG) 13 August 2014 (2014-08-13)<br>abstract, and claims 1 and 10-15, and description, pp. 60, 65 and 68 | 1-42 |
| A | CN 104302647 A (CMG PHARMACEUTICAL CO., LTD.) 21 January 2015 (2015-01-21)<br>abstract, and claims 1and 7-11, and description, pp. 6-7 and 43-77 | 1-42 |
| A | CN 110022878 A (THE REGENTS OF THE UNIVERSITY OF MICHIGAN) 16 July 2019 (2019-07-16)<br>abstract, and claims 1-18, and description, embodiment 1 | 1-42 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 November 2022** | **15 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/120154**

**C. DOCUMENTS CONSIDERED TO BE RELEVANT**

| Category* | Citation of document, with indication, where appropriate, of the relevant passages | Relevant to claim No. |
|---|---|---|
| A | WO 2014160177 A2 (EXELIXIS, INC.) 02 October 2014 (2014-10-02) abstract, and claims 1 and 46-49, and description, table 1, and pp. 4 and 61-62 | 1-42 |
| A | WO 2017070256 A2 (ARAXES PHARMA L.L.C.) 27 April 2017 (2017-04-27) abstract, and claims 4 and 13, and description, pp. 3 and 49, and tables 2-3 | 1-42 |

Form PCT/ISA/210 (second sheet) (January 2015)

**INTERNATIONAL SEARCH REPORT**
**Information on patent family members**

International application No.
**PCT/CN2022/120154**

| Patent document cited in search report | | | Publication date (day/month/year) | Patent family member(s) | | | Publication date (day/month/year) |
|---|---|---|---|---|---|---|---|
| WO | 2016044772 | A1 | 24 March 2016 | ES | 2835729 | T3 | 23 June 2021 |
| | | | | JP | 2017528488 | A | 28 September 2017 |
| | | | | TW | 201625305 | A | 16 July 2016 |
| | | | | US | 2016166571 | A1 | 16 June 2016 |
| | | | | UY | 36307 | A | 29 April 2016 |
| | | | | JP | 2022168089 | A | 04 November 2022 |
| | | | | US | 2019262342 | A1 | 29 August 2019 |
| | | | | EP | 3193851 | A1 | 26 July 2017 |
| | | | | JO | 3556 | B1 | 05 July 2020 |
| US | 2018118757 | A1 | 03 May 2018 | CR | 05.02.2016 | A | 20 July 2016 |
| | | | | US | 2022242875 | A1 | 04 August 2022 |
| | | | | JO | 3805 | B1 | 31 January 2021 |
| | | | | US | 2020102321 | A1 | 02 April 2020 |
| | | | | TW | 201524959 | A | 01 July 2015 |
| | | | | US | 2015239900 | A1 | 27 August 2015 |
| | | | | US | 2021188869 | A1 | 24 June 2021 |
| | | | | UY | 35781 | A | 29 May 2015 |
| | | | | EA | 201991884 | A2 | 28 February 2020 |
| CN | 106488910 | A | 08 March 2017 | NI | 201600049 | A | 20 May 2016 |
| | | | | NO | 20160646 | A1 | 19 April 2016 |
| | | | | EP | 3055290 | A1 | 17 August 2016 |
| | | | | CA | 2926328 | A1 | 16 April 2015 |
| | | | | BR | 112016008016 | A2 | 12 September 2017 |
| | | | | EA | 201690752 | A1 | 29 July 2016 |
| | | | | NZ | 719076 | A | 26 November 2021 |
| | | | | PH | 12016500538 | A1 | 13 June 2016 |
| | | | | EP | 3636639 | A1 | 15 April 2020 |
| | | | | AU | 2014331794 | A1 | 21 April 2016 |
| | | | | IL | 244699 | D0 | 21 April 2016 |
| | | | | SG | 11201602662 Y | A | 30 May 2016 |
| | | | | KR | 20160076519 | A | 30 June 2016 |
| | | | | MX | 2016004360 | A | 19 August 2016 |
| | | | | ZA | 201602245 | B | 25 September 2019 |
| | | | | UA | 119971 | C2 | 10 September 2019 |
| | | | | WO | 2015054572 | A1 | 16 April 2015 |
| | | | | JP | 2016532656 | A | 20 October 2016 |
| CN | 103987699 | A | 13 August 2014 | US | 2014249139 | A1 | 04 September 2014 |
| | | | | WO | 2013057711 | A1 | 25 April 2013 |
| | | | | JP | 2014530851 | A | 20 November 2014 |
| | | | | EP | 2768813 | A1 | 27 August 2014 |
| CN | 104302647 | A | 21 January 2015 | US | 2014113915 | A1 | 24 April 2014 |
| | | | | KR | 20150005551 | A | 14 January 2015 |
| | | | | EP | 2828258 | A1 | 28 January 2015 |
| | | | | US | 2012238587 | A1 | 20 September 2012 |
| | | | | WO | 2013141586 | A1 | 26 September 2013 |
| | | | | JP | 2015512896 | A | 30 April 2015 |
| CN | 110022878 | A | 16 July 2019 | WO | 2018085674 | A1 | 11 May 2018 |
| | | | | CA | 3042697 | A1 | 018 |
| | | | | AU | 2017354019 | A1 | 23 May 2019 |

Form PCT/ISA/210 (patent family annex) (January 2015)

**INTERNATIONAL SEARCH REPORT**
**Information on patent family members**

International application No.
**PCT/CN2022/120154**

| Patent document cited in search report | Publication date (day/month/year) | Patent family member(s) | Publication date (day/month/year) |
|---|---|---|---|
| | | KR 20190089860 A | 31 July 2019 |
| | | US 2020078360 A1 | 12 March 2020 |
| | | JP 2019537604 A | 26 December 2019 |
| | | EP 3534905 A1 | 11 September 2019 |
| WO 2014160177 A2 | 02 October 2014 | None | |
| WO 2017070256 A2 | 27 April 2017 | US 2017131278 A1 | 11 May 2017 |
| | | EP 3364977 A2 | 29 August 2018 |
| | | JP 2018533939 A | 22 November 2018 |
| | | US 2018246102 A1 | 30 August 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016164675 A
- WO 2016168540 A
- WO 2018119183 A
- WO 2018206539 A1
- WO 2020216190 A1


### Non-patent literature cited in the description


- *Nat Rev Drug Discov,* 2014, vol. 13, 828-851
- *Nature,* 2013, vol. 503, 548-551
- *Science,* 2016, vol. 351, 604-608
- *Cancer Discov,* 2016, vol. 6, 316-29
- **MATTHEW, R. JANES ; YI LIU et al.** *Cell,* 2018, vol. 172, 578-589
- **XIE, J.Y et al.** *Nature,* 2020, vol. 577, 421-425
- **ADACHI, Y et al.** *Clin Cancer Res,* 08 September 2020